(19) Europäisches Patentamt
European Patent Office
Office européen des brevets

(11) EP 2 815 749 A1

(12) **EUROPEAN PATENT APPLICATION**

(43) Date of publication:
**24.12.2014 Bulletin 2014/52**

(51) Int Cl.:
***A61K 31/45*** *(2006.01)*

(21) Application number: **14172555.6**

(22) Date of filing: **16.06.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **20.06.2013 EP 13173120**
**20.06.2013 EP 13173118**
**25.07.2013 EP 13178100**
**25.07.2013 EP 13178101**
**29.08.2013 EP 13182294**
**29.08.2013 EP 13182299**
**06.09.2013 EP 13183449**
**24.10.2013 EP 13190172**
**24.10.2013 EP 13190171**

(71) Applicant: **IP Gesellschaft für Management mbH**
**50931 Köln (DE)**

(72) Inventor: **Trinius, Frank**
**50931 Köln (DE)**

(54) **Solid form of 4-amino-2-(2,6-dioxopiperidine-3-yl)isoindoline-1,3-dione having specified X-ray diffraction pattern**

(57) The invention relates to a packaging comprising one or more administration units comprising a solid form of 4-amino-2-(2,6-dioxopiperidine-3-yl)isoindoline-1,3-dione.

EP 2 815 749 A1

## Description

CROSSREFERENCES

The present application claims priorities of European patent application no. 13173120.0, filed on 20 June 2013; European patent application no. 13173118.4, filed on 20 June 2013; European patent application no. 13178100.7, filed on 25 July 2013; European patent application no. 13178101.5, filed on 25 July 2013; European patent application no. 13182294.2, filed on 29 August 2013; European patent application no. 13182299.1, filed on 29 August 2013; European patent application no. 13183449.1, filed on 06 September 2013; European patent application no. 13190172.0, filed on 24 October 2013; and European patent application no. 13190171.2, filed on 24 October 2013.

BACKGROUND ARTS

Many pharmaceuticals are marketed without any packaging. In various countries, small drug shops are a common source of medicine where stocking of prescription-only medicines and unpackaged tablets is the norm (Goodman C et al., Health Policy Plan. 2007 Nov;22(6):393-403). Furthermore, self-medication is common practice in various countries. For example, the most frequently proposed drugs to treat male urethritis are: ampicillin 250-mg capsules (44%); oxytetracyline 250-mg capsules (24%); and cotrimoxazole 450-mg tablets (12%), and these drugs are frequently sold unpackaged (Sow PS et al., Int J Infect Dis. 2002 Jun;6(2):108-12).

In Germany and other European countries, numerous pharmaceuticals are marketed in packaging wherein every single administration unit is individually packaged in a single packaging. Examples include but are not limited to Frubiase® Calcium (Boehringer Ingelheim) marketed as a liquid administration unit that is individually packaged in a glass ampoule; Orthomol Immun® Direktgranulat (Orthomol) marketed as administration unit in granulate form that is individually packaged in a bag; Orthomol Vital m® (Orthomol) marketed as a liquid administration unit that is individually packaged in a bottle having a lid containing an individually packaged tablet; Vitasprint® (Pfizer) and Celestamine® (MSD Sharp & Dohme) each marketed as a liquid administration unit that is individually packaged in a bottle; Alka-Seltzer® (Bayer) marketed as effervescent tablet that is individually packaged in a bag; Aspirin® Complex (Bayer), Aspiring Effect (Bayer), Helmex® (Infectopharm), Monuril® Granulat (Pierre Fabre Pharma) each marketed as administration unit in granulate form that individually packaged in a bag; ellaOne® (HRA Pharma), Levonelle® (Bayer Schering Pharma), Pidana® (HRA Pharma), Fungata® (Pfizer), and Canesten® Gyn once (Bayer) each marketed as a single tablet that is individually packaged in a blister. Furthermore, numerous single-to-use syringes are marketed individually packaged.

It is an object of the invention to provide administration units containing drugs that have advantages compared to conventional administration units and conventional drugs, respectively. It is also an object of the invention to provide packaging containing administration units containing drugs that have advantages compared to conventional packaging. These objects have been achieved by the present invention.

SUMMARY OF THE INVENTION

The present invention relates to different drugs (active pharmaceutical ingredients) and certain forms of said drugs, such as stereoisomers, salts and/or polymorphs thereof. The present invention covers various aspects that are numbered (i), (ii), (iii), (iv), (v), (vi), (vii), and (viii). Aspects (i), (ii), (iii), (iv), (v), (vi), (vii), and (viii) are separate from one another.

The present invention relates to an administration unit comprising (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug71}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}; a packaging comprising one or more of such administration units; a method for manufacturing such an administration unit; a method for manufacturing such a packaging; and particles of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug71}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}.

Certain embodiments of the invention are also disclosed in WO2013/088335, WO2013/087546, WO2013/109514, WO2013/126326, WO2013/126394, WO2013/130402, WO2013/158121, and WO2013/156772, respectively. Any embodiment disclosed in any of these references is incorporated herein by reference as a preferred embodiment of the invention.

GENERAL DEFINITIONS

To avoid unnecessary repetitions, the description refers to the following abbreviations the meaning of which is defined hereinafter:

Aspect (i) of the present invention relates to 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate, also known as cabazitaxel, which has the following structure of formula (I):

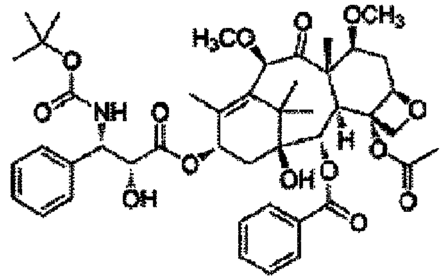

(I),

abbreviated as '{drug1}'. In particular, the present invention relates to crystalline ethyl acetate solvate form of cabazitaxel, abbreviated as '{drug1a}'; to crystalline ethyl acetate solvate form of cabazitaxel characterized by an X-Ray Powder Diffraction (XRPD) pattern having the peaks at 8.7, 10.1, 13.8, 14.1 and 14.8 ± 0.2 degrees 2-theta, abbreviated as '{drug1b}'; and to crystalline ethyl acetate solvate form of cabazitaxel characterized by an X-Ray Powder Diffraction (XRPD) pattern having the peaks at 7.5, 7.9, 8.7, 10.1, 10.2, 12.6, 12.9, 13.8, 14.1 and 14.8 ± 0.2 degrees 2-theta, abbreviated as '{drug1c}'. {drug1a}, {drug1b} and {drug1c} are specific forms falling within the definition of {drug1}. Aspect (i) of the invention is separate from aspects (ii), (iii), (iv), (v), (vi), (vii), and (viii) of the invention. Thus, all embodiments of {drug1a}, {drug1b}, and {drug1c}, respectively, are only related to {drug1}, but neither to {drug2}, nor to {drug3}, nor to {drug4}, nor to {drug5}, nor to {drug6}, nor to {drug7}, nor to {drug8}.
Aspect (ii) of the present invention relates to compositon X, namely a pharmaceutical composition comprising a polymer polyvinylpyrrolidone (PVP) or copovidone, a compound according to formula (IIA),

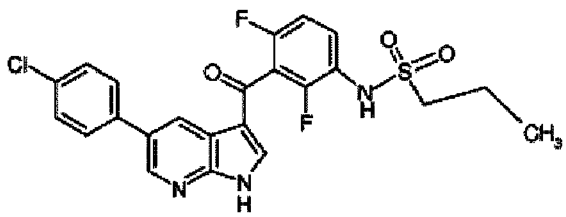

(IIA)

or a compound according to formula (IIB),

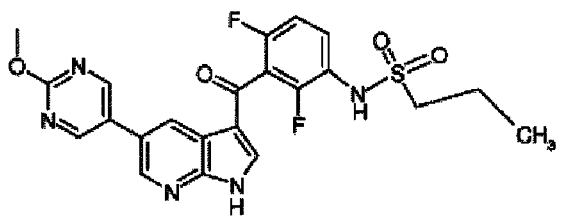

(IIB)

and, optionally, a surfactant and/or hydroxypropyl methylcellulose-acetate succinate, abbreviated as '{drug2a}'. In particular, the present invention relates to a solid dispersion of composition X, abbreviated as '{drug2a}'. {drug2a} is a specific form falling within the definition of {drug2}. Aspect (ii) of the invention is separate from aspects (i), (iii),

(iv), (v), (vi), (vii) and (viii) of the invention. Thus, all embodiments of {drug2a} are only related to {drug2}, but neither to {drug1}, nor to {drug3}, nor to {drug4}, nor to {drug5}, nor to {drug6}, nor to {drug7}, nor to {drug8}.

Aspect (iii) of the present invention relates to phosphate salt or nitrate salt of 4-(R)-(6,7-dihydro-5H-pyrrolo[1,2-c]imidazol-5-yl)-3-fluoro-benzonitrile according to Formula (III)

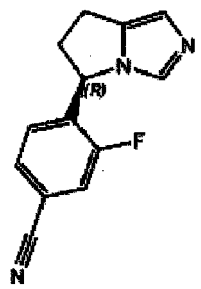

(III),

abbreviated as '{drug3}'. In particular, the present invention relates to crystalline form of phosphate salt of 4-(R)-(6,7-dihydro-5H-pyrrolo[1,2-c]imidazol-5-yl)-3-fluoro-benzonitrile, abbreviated as '{drug3a}'; to crystalline Form A of phosphate salt of 4-(R)-(6,7-dihydro-5H-pyrrolo[1,2-c]imidazol-5-yl)-3-fluoro-benzonitrile, abbreviated as '{drug3b}'; to crystalline Form B of phosphate salt of 4-(R)-(6,7-dihydro-5H-pyrrolo[1,2-c]imidazol-5-yl)-3-fluoro-benzonitrile, abbreviated as '{drug3c}'; to crystalline Form C of phosphate salt of 4-(R)-(6,7-dihydro-5H-pyrrolo[1,2-c]imidazol-5-yl)-3-fluoro-benzonitrile, abbreviated as '{drug3d}'; to crystalline Form D of phosphate salt of 4-(R)-(6,7-dihydro-5H-pyrrolo[1,2-c]imidazol-5-yl)-3-fluoro-benzonitrile, abbreviated as '{drug3e}'; to crystalline Form E of phosphate salt of 4-(R)-(6,7-dihydro-5H-pyrrolo[1,2-c]imidazol-5-yl)-3-fluoro-benzonitrile, abbreviated as '{drug3f}'; to crystalline Form F of phosphate salt of 4-(R)-(6,7-dihydro-5H-pyrrolo[1,2-c]imidazol-5-yl)-3-fluoro-benzonitrile, abbreviated as '{drug3g}'; to crystalline Form G of phosphate salt of 4-(R)-(6,7-dihydro-5H-pyrrolo[1,2-c]imidazol-5-yl)-3-fluoro-benzonitrile, abbreviated as '{drug3h}'; to crystalline Form H of phosphate salt of 4-(R)-(6,7-dihydro-5H-pyrrolo[1,2-c]imidazol-5-yl)-3-fluoro-benzonitrile, abbreviated as '{drug3i}'; and to crystalline form of nitrate salt of 4-(R)-(6,7-dihydro-5H-pyrrolo[1,2-c]imidazol-5-yl)-3-fluoro-benzonitrile, abbreviated as '{drug3j}'. {drug3a}, {drug3b}, {drug3c}, {drug3d}, {drug3e}, {drug3f}, {drug3g}, {drug3h}, {drug3i}, and {drug3j} are specific forms falling within the definition of {drug3}. Aspect (iii) of the invention is separate from aspects (i), (ii), (iv), (v), (vi), (vii), and (viii) of the invention. Thus, all embodiments of {drug3a}, {drug3b}, {drug3c}, {drug3d}, {drug3e}, {drug3f}, {drug3g}, {drug3h}, {drug3i}, and {drug3j}, respectively, are only related to {drug3}, but neither to {drug1}, nor to {drug2}, nor to {drug4}, nor to {drug5}, nor to {drug6}, nor to {drug7}, nor to {drug8}.

Aspect (iv) of the present invention relates to 4-amino-2-(2,6-dioxopiperidine-3-yl)isoindoline-1,3-dione according to formula (IV)

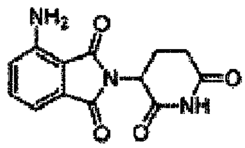

(IV),

abbreviated as '{drug4}'. In particular, the present invention relates to a solid form of 4-amino-2-(2,6-dioxopiperidine-3-yl)isoindoline-1,3-dione, abbreviated as '{drug4a}'; to crystalline Form A of 4-amino-2-(2,6-dioxopiperidine-3-yl)isoindoline-1,3-dione, abbreviated as '{drug4b}'; and to an amorphous Forms of 4-amino-2-(2,6-dioxopiperidine-3-yl)isoindoline-1,3-dione, abbreviated as '{drug4c}'. {drug4a}, {drug4b}, and {drug4c} are specific forms falling within the definition of {drug4}. Aspect (iv) of the invention is separate from aspects (i), (ii), (iii), (v), (vi), (vii), and (viii) of the invention. Thus, all embodiments of {drug4a}, {drug4b}, and {drug4c}, respectively, are only related to {drug4}, but neither to {drug1}, nor to {drug2}, nor to {drug3}, nor to {drug5}, nor to {drug6}, nor to {drug7}, nor to {drug8}.

Aspect (v) of the present invention relates to 3-(4-nitro-1-oxoisoindolin-2-yl)piperidine-2,6-dione according to formula (V)

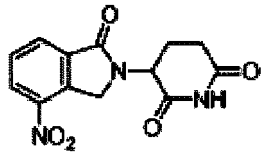

(V),

abbreviated as '{drug5}'. In particular, the present invention relates to a solid form of 3-(4-nitro-1-oxoisoindolin-2-yl)piperidine-2,6-dione, abbreviated as '{drug5a}'; to crystalline Form A of 3-(4-nitro-1-oxoisoindolin-2-yl)piperidine-2,6-dione, abbreviated as '{drug5b}'; to crystalline Form B of 3-(4-nitro-1-oxoisoindolin-2-yl)piperidine-2,6-dione, abbreviated as '{drug5c}'; and to crystalline Form C of 3-(4-nitro-1-oxoisoindolin-2-yl)piperidine-2,6-dione, abbreviated as '{drug5d}'. {drug5a}, {drug5b}, {drug5c}, and {drug5d} are specific forms falling within the definition of {drug5}. Aspect (v) of the invention is separate from aspects (i), (ii), (iii), (iv), (vi), (vii), and (viii) of the invention. Thus, all embodiments of {drug5a}, {drug5b}, {drug5c}, and {drug5d}, respectively, are only related to {drug5}, but neither to {drug1}, nor to {drug2}, nor to {drug3}, nor to {drug4}, nor to {drug6}, nor to {drug7}, nor to {drug8}.

Aspect (vi) of the present invention relates to N-(5S,6S,9R)-5-amino-6-(2,3-10-dffluorophenyl)-6,7,8,9-tetrahydro-5H-cyclohepta[b]pyridin-9-yl-4-(2-oxo-2,3-dihydro-1H-imidazo[4,5-b]pyridin-1-yl)piperidine-1-carboxylate according to formula (VI)

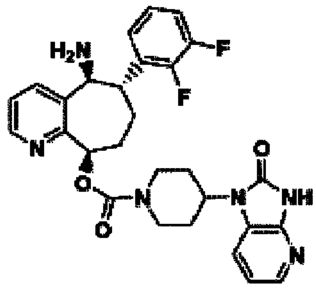

(VI),

abbreviated as '{drug6}'. In particular, the present invention relates to a hemisulfate salt of N-(5S,6S,9R)-5-amino-6-(2,3-10-difluorophenyl)-6,7,8,9-tetrahydro-5H-cyclohepta[b]pyridin-9-yl-4-(2-oxo-2,3-dihydro-1H-imidazo[4,5-b]pyridin-1-yl)piperidine-1-carboxylate, abbreviated as '{drug6a}'; to crystalline Form HI.5-1 of hemisulfate salt of N-(5S,6S,9R)-5-amino-6-(2,3-10-difluorophenyl)-6,7,8,9-tetrahydro-5H-cyclohepta[b]pyridin-9-yl-4-(2-oxo-2,3-dihydro-1H-imidazo[4,5-b]pyridin-1-yl)piperidine-1-carboxylate, abbreviated as '{drug6b}'; and to crystalline Form P33 of hemisulfate salt of N-(5S,6S,9R)-5-amino-6-(2,3-10-difluorophenyl)-6,7,8,9-tetrahydro-5H-cyclohepta[b]pyridin-9-yl-4-(2-oxo-2,3-dihydro-1H-imidazo[4,5-b]pyridin-1-yl)piperidine-1-carboxylate, abbreviated as '{drug6c}'. {drug6a}, {drug6b} and {drug6c} are specific forms falling within the definition of {drug6}. Aspect (vi) of the invention is separate from aspects (i), (ii), (iii), (iv), (v), (vii), and (viii) of the invention. Thus, all embodiments of {drug6a}, {drug6b} and {drug6c}, respectively, are only related to {drug6}, but neither to {drug1}, nor to {drug2}, nor to {drug3}, nor to {drug4}, nor to {drug5}, nor to {drug7}, nor to {drug8}.

Aspect (vii) of the present invention relates to N-[2,4-bis(1,1-dimethylethyl)-5-hydroxyphenyl]-1,4-dihydro-4-oxoquinoline-3-carboxamide according to formula (VII)

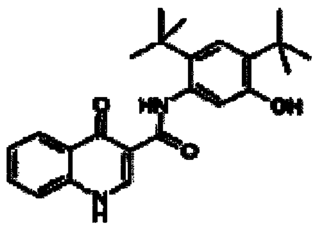

(VII),

abbreviated as {drug7}. In particular, the present invention relates to crystalline solvates form of N-[2,4-bis(1,1-dimethylethyl)-5-hydroxyphenyl]-1,4-dihydro-4-oxoquinoline-3-carboxamide, abbreviated as '{drug7a}'; to crystalline Form D of N-[2,4-bis(1,1-dimethylethyl)-5-hydroxyphenyl]-1,4-dihydro-4-oxoquinoline-3-carboxamide, abbreviated as '{drug7b}'; to crystalline Form E of N-[2,4-bis(1,1-dimethylethyl)-5-hydroxyphenyl]-1,4-dihydro-4-oxoquinoline-3-carboxamide, abbreviated as '{drug7c}'; to crystalline Form F of N-[2,4-bis(1,1-dimethylethyl)-5-hydroxyphenyl]-l,4-dihydro-4-oxoquinoline-3-carboxamide, abbreviated as '{drug7d}'; to crystalline Form G of N-[2,4-bis(1,1-dimethylethyl)-5-hydroxyphenyl]-1,4-dihydro-4-oxoquinoline-3-carboxamide, abbreviated as '{drug7e}'; to crystalline Form H of N-[2,4-bis(1,1-dimethylethyl)-5-hydroxyphenyl]-1,4-dihydro-4-oxoquinoline-3-carboxamide, abbreviated as '{drug7f}'; to crystalline Form I of N-[2,4-bis(1,1-dimethylethyl)-5-hydroxyphenyl]-1,4-dihydro-4-oxoquinoline-3-carboxamide, abbreviated as '{drug7g}'; to crystalline Form J of N-[2,4-bis(1,1-dimethylethyl)-5-hydroxyphenyl]-1,4-dihydro-4-oxoquinoline-3-carboxamide, abbreviated as '{drug7h}'; to crystalline Form K of N-[2,4-bis(1,1-dimethylethyl)-5-hydroxyphenyl]-1,4-dihydro-4-oxoquinoline-3-carboxamide, abbreviated as '{drug7i}'; to crystalline Form L of N-[2,4-bis(1,1-dimethylethyl)-5-hydroxyphenyl]-1,4-dihydro-4-oxoquinoline-3-carboxamide, abbreviated as '{drug7j}'; to crystalline Form M of N-[2,4-bis(1,1-dimethylethyl)-5-hydroxyphenyl]-1,4-dihydro-4-oxoquinoline-3-carboxamide, abbreviated as '{drug7k}'; to crystalline Form N of N-[2,4-bis(1,1-dimethylethyl)-5-hydroxyphenyl]-1,4-dihydro-4-oxoquinoline-3-carboxamide, abbreviated as '{drug71}'; to crystalline Form O of N-[2,4-bis(1,1-dimethylethyl)-5-hydroxyphenyl]-1,4-dihydro-4-oxoquinoline-3-carboxamide, abbreviated as '{drug7m}'; to crystalline Form P of N-[2,4-bis(1,1-dimethylethyl)-5-hydroxyphenyl]-1,4-dihydro-4-oxoquinoline-3-carboxamide, abbreviated as '{drug7n}'; to crystalline Form Q of N-[2,4-bis(1,1-dimethylethyl)-5-hydroxyphenyl]-1,4-dihydro-4-oxoquinoline-3-carboxamide, abbreviated as '{drug7o}'; to crystalline Form R of N-[2,4-bis(1,1-dimethylethyl)-5-hydroxyphenyl]-1,4-dihydro-4-oxoquinoline-3-carboxamide, abbreviated as '{drug7p}'; to crystalline Form S of N-[2,4-bis(1,1-dimethylethyl)-5-hydroxyphenyl]-1,4-dihydro-4-oxoquinoline-3-carboxamide, abbreviated as '{drug7q}'; to crystalline Form T of N-[2,4-bis(1,1-dimethylethyl)-5-hydroxyphenyl]-1,4-dihydro-4-oxoquinoline-3-carboxamide, abbreviated as '{drug7r}'; to crystalline Form W of N-[2,4-bis(1,1-dimethylethyl)-5-hydroxyphenyl]-1,4-dihydro-4-oxoquinoline-3-carboxamide, abbreviated as '{drug7s}'; and to Hydrate B of N-[2,4-bis(1,1-dimethylethyl)-5-hydroxyphenyl]-1,4-dihydro-4-oxoquinoline-3-carboxamide, abbreviated as '{drug7t}'. {drug7a}, {drug7b}, {drug7c}, {drug7d}, {drug7e}, {drug7f} , {drug7g}, {drug7h}, {drug7i}, {drug7j}, {drug7k}, {drug71}, {drug7m}, {drug7n}, {drug7o}, {drug7p}, {drug7q}, {drug7r}, {drug7s}, and {drug7t} are specific forms falling within the definition of {drug7}. Aspect (vii) of the invention is separate from aspects (i), (ii), (iii), (iv), (v), (vi), and (viii) of the invention. Thus, all embodiments of {drug7a}, {drug7b}, {drug7c}, {drug7d}, {drug7e}, {drug7f}, {drug7g}, {drug7h}, {drug7i}, {drug7j}, {drug7k}, {drug71}, {drug7m}, {drug7n}, {drug7o}, {drug7p}, {drug7q}, {drug7r}, {drug7s}, and {drug7t}, respectively, are only related to {drug7}, but neither to {drug1}, nor to {drug2}, nor to {drug3}, nor to {drug4}, nor to {drug5}, nor to {drug6}, nor to {drug8}.

Aspect (viii) of the present invention relates to (S)-4-amino-N-(1-(4-chlorophenyl)-3-hydroxypropyl)-1-(7H-pyrrolo[2,3-d]pyrimidin-4-yl)piperidine-4-carboxamide according to general formula (VIII)

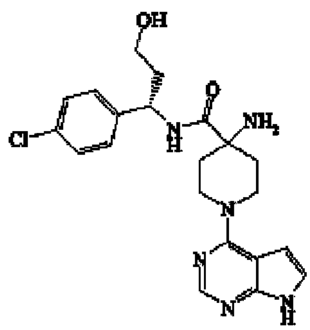

(VIII),

abbreviated as {drug8}. In particular, the present invention relates to a solid form of (S)-4-amino-N-(1-(4-chlorophenyl)-3-hydroxypropyl)-1-(7H-pyrrolo[2,3-d]pyrimidin-4-yl)piperidine-4-carboxamid, abbreviated as {drug8a}); to Form A of (S)-4-amino-N-(1-(4-chlorophenyl)-3-hydroxypropyl)-1-(7H-pyrrolo[2,3-d]pyrimidin-4-yl)piperidine-4-carboxamid, abbreviated as {drug8b}; to Form B of (S)-4-amino-N-(1-(4-chlorophenyl)-3-hydroxypropyl)-1-(7H-pyrrolo[2,3-d]pyrimidin-4-yl)piperidine-4-carboxamid, abbreviated as {drug8c}; and to Form C of (S)-4-amino-N-(1-(4-chlorophenyl)-3-hydroxypropyl)-1-(7H-pyrrolo[2,3-d]pyrimidin-4-yl)piperidine-4-carboxamid, abbreviated as {drug8d}. {drug8a}, {drug8b}, {drug8c} and {drug8d} are specific forms falling within the definition of {drug8}. Aspect (viii) of the invention is separate from aspects (i), (ii), (iii), (iv), (v), (vi), and (vii) of the invention. Thus, all embodiments of {drug8a}, {drug8b}, {drug8c} and {drug8d}, respectively, are only related to {drug8}, but neither to {drug1}, nor to {drug2}, nor to {drug3}, nor to {drug4}, nor to {drug5}, nor to {drug6}, nor to {drug7}.

Summarizing therefore the following abbreviations have the following meanings:

Aspect (i):

{drug1} = cabazitaxel, which has the following structure of formula (I):

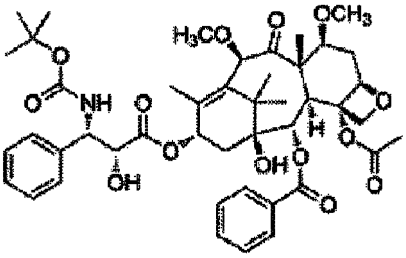

(I);

{drug1a} = crystalline ethyl acetate solvate form of cabazitaxel;
{drug1b} = crystalline ethyl acetate solvate form of cabazitaxel characterized by an X-Ray Powder Diffraction (XRPD) pattern having the peaks at 8.7, 10.1, 13.8, 14.1 and 14.8 ± 0.2 degrees 2-theta; and
{drug1c} = crystalline ethyl acetate solvate form of cabazitaxel characterized by an X-Ray Powder Diffraction (XRPD) pattern having the peaks at 7.5, 7.9, 8.7, 10.1, 10.2, 12.6, 12.9, 13.8, 14.1 and 14.8 ± 0.2 degrees 2-theta.

Aspect (ii):

{drug2} = compositon X, namely a pharmaceutical composition comprising a polymer polyvinylpyrrolidone (PVP) or copovidone, a compound according to formula (IIA),

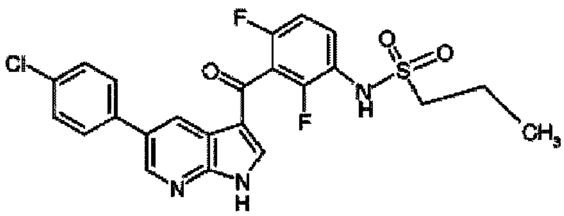

(IIA)

or a compound according to formula (IIB),

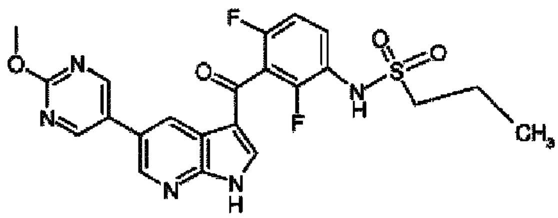

(IIB)

and, optionally, a surfactant and/or hydroxypropyl methylcellulose-acetate succinate; and
{drug2a} = solid dispersion of composition X.

Aspect (iii):

{drug3} = phosphate salt or nitrate salt of 4-(R)-(6,7-dihydro-5H-pyrrolo[1,2-c]imidazol-5-yl)-3-fluoro-benzonitrile according to Formula (III)

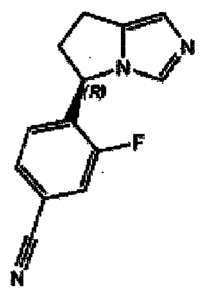

(III);

{drug3a} = crystalline form of phosphate salt of 4-(R)-(6,7-dihydro-5H-pyrrolo[1,2-c]imidazol-5-yl)-3-fluoro-benzonitrile;
{drug3b} = crystalline Form A of phosphate salt of 4-(R)-(6,7-dihydro-5H-pyrrolo[1,2-c]imidazol-5-yl)-3-fluoro-benzonitrile;
{drug3c} = crystalline Form B of phosphate salt of 4-(R)-(6,7-dihydro-5H-pyrrolo[1,2-c]imidazol-5-yl)-3-fluoro-benzonitrile;
{drug3d} = crystalline Form C of phosphate salt of 4-(R)-(6,7-dihydro-5H-pyrrolo[1,2-c]imidazol-5-yl)-3-fluoro-benzonitrile;
{drug3e} = crystalline Form D of phosphate salt of 4-(R)-(6,7-dihydro-5H-pyrrolo[1,2-c]imidazol-5-yl)-3-fluoro-benzonitrile;
{drug3f} = crystalline Form E of phosphate salt of 4-(R)-(6,7-dihydro-5H-pyrrolo[1,2-c]imidazol-5-yl)-3-fluoro-benzonitrile;
{drug3g} = crystalline Form F of phosphate salt of 4-(R)-(6,7-dihydro-5H-pyrrolo[1,2-c]imidazol-5-yl)-3-fluoro-benzonitrile;
{drug3h} = crystalline Form G of phosphate salt of 4-(R)-(6,7-dihydro-5H-pyrrolo[1,2-c]imidazol-5-yl)-3-fluoro-benzonitrile;
{drug3i} = crystalline Form H of phosphate salt of 4-(R)-(6,7-dihydro-5H-pyrrolo[1,2-c]imidazol-5-yl)-3-fluoro-benzonitrile; and
{dug3j} = crystalline form of nitrate salt of 4-(R)-(6,7-dihydro-5H-pyrrolo[1,2-c]imidazol-5-yl)-3-fluoro-benzonitrile.

Aspect (iv):

{drug4} = 4-amino-2-(2,6-dioxopiperidine-3-yl)isoindoline-1,3-dione according to formula (IV)

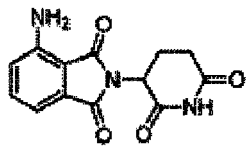

(IV);

{drug4a} = solid form of 4-amino-2-(2,6-dioxopiperidine-3-yl)isoindoline-1,3-dione;
{drug4b} = Form A of 4-amino-2-(2,6-dioxopiperidine-3-yl)isoindoline-1,3-dione; and
{drug4c} = amorphous Forms of 4-amino-2-(2,6-dioxopiperidine-3-yl)isoindoline-1,3-dione.

Aspect (v):

{drug5} = 3-(4-nitro-1-oxoisoindolin-2-yl)piperidine-2,6-dione dione according to formula (V)

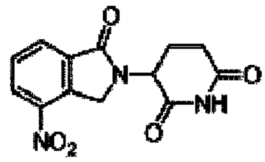

(V);

{drug5a} = solid form of 3-(4-nitro-1-oxoisoindolin-2-yl)piperidine-2,6-dione;
{drug5b} = crystalline Form A of 3-(4-nitro-1-oxoisoindolin-2-yl)piperidine-2,6-dione;
{drug5c} = crystalline Form B of 3-(4-nitro-1-oxoisoindolin-2-yl)piperidine-2,6-dione; and
{drug5d} = crystalline Form C of 3-(4-nitro-1-oxoisoindolin-2-yl)piperidine-2,6-dione.

Aspect (vi):

{drug6} = N-(5S,6S,9R)-5-amino-6-(2,3-10-difluorophenyl)-6,7,8,9-tetrahydro-5H-cyclohepta[b]pyridin-9-yl-4-(2-oxo-2,3-dihydro-1H-imidazo[4,5-b]pyridin-1-yl)piperidine-1-carboxylate;
{drug6a} = hemisulfate salt of N-(5S,6S,9R)-5-amino-6-(2,3-10-difluorophenyl)-6,7,8,9-tetrahydro-5H-cyclohepta[b]-pyridin-9-yl-4-(2-oxo-2,3-diliydro-1H-imidazo[4,5-b]pyridin-1-yl)piperidine-1-carboxylate;
{drug6b} = crystalline Form HI.5-1 of hemisulfate salt of N-(5S,6S,9R)-5-amino-6-(2,3-10-difluorophenyl)-6,7,8,9-tetrahydro-5H-cyclohepta[b]pyridin-9-yl-4-(2-oxo-2,3-dihydro-1H-imidazo[4,5-b]pyridin-1-yl)pipexidine-1-carboxylate;
{drug6c} = crystalline Form P33 of hemisulfate salt of N-(5S,6S,9R)-5-amino-6-(2,3-10-difluorophenyl)-6,7,8,9-tetrahydro-5H-cyclohepta[b]pyridin-9-yl-4-(2-oxo-2,3-dihydro-1H-itnidazo[4,5-b]pyridin-1-yl)pipexidine-1-carboxylate.

Aspect (vii):

{drug7} = N-[2,4-bis(1,1-dimethylethyl)-5-hydroxyphenyl]-1,4-dihydro-4-oxoquinoline-3-carboxamide according to formula (VII)

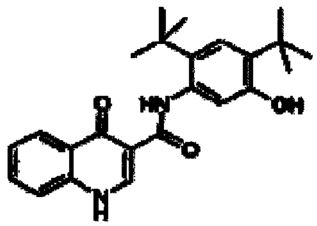

(VII);

{drug7a} = crystalline solvates form of N-[2,4-bis(1,1-dimethylethyl)-5-hydroxyphenyl]-1,4-dihydro-4-oxoquinoline-3-carboxamide;
{drug7b} = crystalline Form D of N-[2,4-bis(1,1-dimethylethyl)-5-hydroxyphenyl]-1,4-dihydro-4-oxoquinoline-3-carboxamide;
{drug7c} = crystalline Form E of N-[2,4-bis(1,1-dimethylethyl)-5-hydroxyphenyl]-1,4-dihydro-4-oxoquinoline-3-carboxamide;
{drug7d} = crystalline Form F of N-[2,4-bis(1,1-dimethylethyl)-5-hydroxyphenyl]-1,4-dihydro-4-oxoquinoline-3-carboxamide;
{drug7e} = crystalline Form G of N-[2,4-bis(1,1-dimethylethyl)-5-hydroxyphenyl]-1,4-dihydro-4-oxoquinoline-3-carboxamide;
{drug7f} = crystalline Form H of N-[2,4-bis(1,1-dimethylethyl)-5-hydroxyphenyl]-1,4-dihydro-4-oxoquinoline-3-carboxamide;
{drug7g} = crystalline Form I of N-[2,4-bis(1,1-dimethylethyl)-5-hydroxyphenyl]-1,4-dihydro-4-oxoquinoline-3-carboxamide;
{drug7h} = crystalline Form J of N-[2,4-bis(1,1-dimethylethyl)-5-hydroxyphenyl]-1,4-dihydro-4-oxoquinoline-3-carboxamide;
{drug7i} = crystalline Form K of N-[2,4-bis(1,1-dimethylethyl)-5-hydroxyphenyl]-1,4-dihydro-4-oxoquinoline-3-carboxamide;

{drug7j} = crystalline Form L of N-[2,4-bis(1,1-dimethylethyl)-5-hydroxyphenyl]-1,4-dihydro-4-oxoquinoline-3-carboxamide;

{drug7k} = crystalline Form M of N-[2,4-bis(1,1-dimethylethyl)-5-hydroxyphenyl]-1,4-dihydro-4-oxoquinoline-3-carboxamide;

{drug71} = crystalline Form N of N-[2,4-bis(1,1-dimethylethyl)-5-hydroxyphenyl]-1,4-dihydro-4-oxoquinoline-3-carboxamide;

{drug7m} = crystalline Form O of N-[2,4-bis(1,1-dimethylethyl)-5-hydroxyphenyl]-1,4-dihydro-4-oxoquinoline-3-carboxamide;

{drug7n} = crystalline Form P of N-[2,4-bis(1,1-dimethylethyl)-5-hydroxyphenyl]-1,4-dihydro-4-oxoquinoline-3-carboxamide;

{drug7o} = crystalline Form Q of N-[2,4-bis(1,1-dimethylethyl)-5-hydroxyphenyl]-1,4-dihydro-4-oxoquinoline-3-carboxamide;

{drug7p} = crystalline Form R of N-[2,4-bis(1,1-dimethylethyl)-5-hydroxyphenyl]-1,4-dihydro-4-oxoquinoline-3-carboxamide;

{drug7q} = crystalline Form S of N-[2,4-bis(1,1-dimethylethyl)-5-hydroxyphenyl]-1,4-dihydro-4-oxoquinoline-3-carboxamide;

{drug7r} = crystalline Form T of N-[2,4-bis(1,1-dimethylethyl)-5-hydroxyphenyl]-1,4-dihydro-4-oxoquinoline-3-carboxamide;

{drug7s} = crystalline Form W of N-[2,4-bis(1,1-dimethylethyl)-5-hydroxyphenyl]-1,4-dihydro-4-oxoquinoline-3-carboxamide; and

{drug7t} = Hydrate B of N-[2,4-bis(1,1-dimethylethyl)-5-hydroxyphenyl]-1,4-dihydro-4-oxoquinoline-3-carboxamide.

Aspect (viii):

{drug8} = (S)-4-amino-N-(1-(4-chlorophenyl)-3-hydroxypropyl)-1-(7H-pyrrolo[2,3-d]pyrimidin-4-yl)piperidine-4-carboxamide according to formula (VIII)

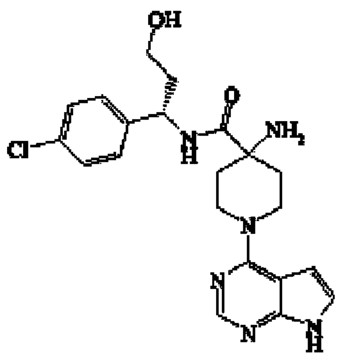

(VIII);

{drug8a} = solid form of (S)-4-amino-N-(1-(4-chlorophenyl)-3-hydroxypropyl)-1-(7H-pyrrolo[2,3-d]pyrimidin-4-yl)-piperidine-4-carboxamid;

{drug8b} = Form A of (S)-4-amino-N-(1-(4-chlorophenyl)-3-hydroxypropyl)-1-(7H-pyrrolo[2,3-d]pyrimidin-4-yl)-piperidine-4-carboxamid;

{drug8c} = Form B of (S)-4-amino-N-(1-(4-chlorophenyl)-3-hydroxypropyl)-1-(7H-pyrrolo[2,3-d]pyrimidin-4-yl)-piperidine-4-carboxamid; and

{drug8d} = Form C of (S)-4-amino-N-(1-(4-chlorophenyl)-3-hydroxypropyl)-1-(7H-pyrrolo[2,3-d]pyrimidin-4-yl)-piperidine-4-carboxamid.

SPECIFIC INORMATION CONCERNING ASPECT (I) OF THE INVENTION

Aspect (i) of the invention relates to an administration unit comprising crystalline ethyl acetate solvate form of cabazitaxel The invention relates to a solid form of cabazitaxel, namely to crystalline ethyl acetate solvate form of cabazitaxel, which is useful for treating in the prevention and/or treatment of cancers, e.g. prostate cancer. Crystalline ethyl acetate solvate form of cabazitaxel is described in WO2013/088335, which is incorporated by reference. Cabazitaxel

as decribed in WO2013/088335 is 4-alpha-acetoxy-2-alpha-benzoyloxy-5-beta,20-epoxy-1-beta-hydroxy-7-beta,10-beta-dimethoxy-9-oxo-13-taxen-13-alpha-yl(2R,3S)-3-tert-butoxycarbonylamino-2-hydroxy-3-phenylpropionate, which has the following structure of formula (I):

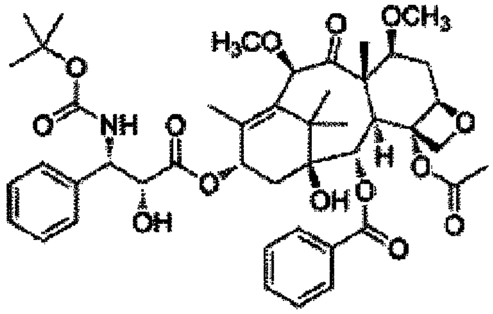

(I)

As used herein, crystalline ethyl acetate solvate form of cabazitaxel is a crystalline form of cabazitaxel as described in WO2013/088335 and which is cited hereinafter: The present invention relates to a novel crystalline form of cabazitaxel ({drug1}) or 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate. The invention also relates to the process for the preparation of said novel crystalline form, pharmaceutical compositions and medicament comprising it, and its therapeutic use in the prevention and/or treatment of cancers, more particularly prostate cancer. The 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3 S)-3-tert-butoxycarbonylamino-2-hydroxy-3-phenylpropionate, which has the following structure of formula (I):

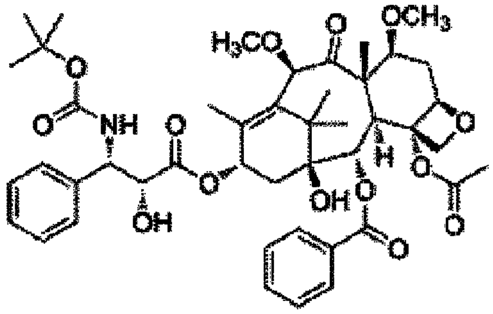

(I)

exhibits notable anticancer properties and is particularly interesting for preventing and/or treating prostate cancers. Prostate cancer affects a large proportion of the male population worldwide, it is the most frequently occurring cancer in men after lung cancer. The use of 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate in the treatment of prostate cancer is known and described in WO 2011/051894. The process for the preparation of 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate is described more particularly in WO 96/30355. Finally, crystalline acetone solvate form of 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate is known and described in WO 2005/028462. Crystalline ethanol solvates, anhydrous and hydrated forms of this compound are also known and described in WO 2009/115655. The present invention is concerned with the obtaining of a new active crystalline form of such a compound.

Indeed, it is known that the identification of new crystalline forms of active principle useful for preventing and/or treating cancers may be particularly interesting. It is also known that the ability of a substance to exist in more than one crystal form is defined as polymorphism and its different crystal forms are called polymorphs. In general, polymorphism is due to the ability of a compound to change its molecular conformation or to form different inter- and/or intra-molecular interactions, particularly hydrogen bonds, which is reflected in different atom arrangements in the crystal lattice of different polymorphs. Thus, polymorphs of a compound can differ notably from each other by different energies in their crystal

lattices and, therefore, generally have specific physical properties in the solid state such as crystal morphology, density, melting point, colour, chemical and physical stability, hygroscopy, solubility, dissolution rate, granular properties... In other words, polymorphic forms of the same compound can exhibit different behaviors in terms of formulation, therapeutic activity and chemical and physical stability. Unexpectedly, the 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate can exist in a crystalline ethyl acetate solvate form ({drug1a}). Thus, the present invention provides a novel crystalline ethyl acetate solvate form of the 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate. Advantageously, this crystalline ethyl acetate solvate form of the cabazitaxel is obtained in higher purity than the acetone solvate form, as illustrated in the examples. Further, this new crystalline ethyl acetate solvate form of the cabazitaxel may be stored for a long time without the need for specific conditions to prevent a premature vaporization of the solvent ethyl acetate contrary to the prior crystalline forms having solvent like acetone or ethanol. This slower desolvatation over time compared to the crystalline ethanol solvate form and crystalline acetone solvate form is also an advantage for the handling. In particular, the crystalline ethyl acetate solvate form has the following characteristics: X-Ray Powder Diffraction (XRPD) pattern having the peaks at 8.7, 10.1, 13.8, 14.1 and 14.8 ± 0.2 degrees 2-theta ({drug1b}). More particularly, said crystalline ethyl acetate solvate form has the following characteristics: X-Ray Powder Diffraction (XRPD) pattern having the peaks at 7.5, 7.9, 8.7, 10.1, 10.2, 12.6, 12.9, 13.8, 14.1 and 14.8 ± 0.2 degrees 2-theta ({drug1c}). More particularly, the novel crystalline ethyl acetate solvate form of the 4a-acetoxy-2a-benzoyloxy-5 P,20-epoxy- 1 P-hydroxy-7P, 10P-dimethoxy-9-oxo-11-taxen- 13a-yl(2R,3S)-3-tert-butoxycarbonylamino-2-hydroxy-3-phenylpropionate having the above defined characteristics, is defined as form A. Another aspect of the present invention is the process for the preparation of said crystalline ethyl acetate solvate form of 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate. Thus, the present invention is directed to a particular process for preparing said crystalline ethyl acetate solvate form of 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate, said process comprising at least the following steps: - having the 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate in solution in an organic solvent (for example acetone or methylene chloride) at room temperature; - making a change of solvent to ethyl acetate at atmospheric or under reduced pressure; - maintaining the so-formed solution in ethyl acetate on continued stirring, and - recovering said crystalline ethyl acetate solvate form of 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate. Said process is particularly advantageous over those disclosed in the prior art since it provides a crystalline ethyl acetate solvate form of 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate in good yields and with good chemical purity. The present invention also relates to said crystalline ethyl acetate solvate form of 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate as a medicament. In a further aspect, the present invention provides said crystalline ethyl acetate solvate form of 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate for its use for preventing and/or treating cancers.

In a preferred aspect, the invention provides a crystalline ethyl acetate solvate form of 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate as herein defined substantially free of impurities. By "substantially free", it is meant that the crystalline ethyl acetate solvate form comprises less than 2% of impurities, preferably less than 1.5% of impurities, and more preferably less than 0.9% of impurities. In a another preferred embodiment, said crystalline ethyl acetate solvate form of 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate, has a X-Ray Powder Diffraction (XRPD) diagram exhibiting characteristic lines located at 8.7, 10.1, 13.8, 14.1 and 14.8 ± 0.2 degrees 2-theta. More particularly said crystalline ethyl acetate solvate form of 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate, has a X-Ray Powder Diffraction (XRPD) diagram exhibiting characteristic lines located at 7.5, 7.9, 8.7, 10.1, 10.2, 12.6, 12.9, 13.8, 14.1 and 14.8 ± 0.2 degrees 2-theta (see Figure 1 of WO2013/088335). More particularly the novel crystalline ethyl acetate solvate form of the 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate having the above defined characteristics is defined as form A. ± 0.2 degrees 2-theta. The ethyl acetate content was determined by Gas Chromatography (GC). The obtained value is of about 9.5% m/m, an ethyl acetate mole per cabazitaxel mole. As stated previously, another aspect of the present invention is the process for the

preparation of the crystalline ethyl acetate solvate form of 4-alpha-acetoxy-2-alpha-benzoyloxy-5-beta,20-epoxy-1-beta-hydroxy-7-beta, 10-beta-dimethoxy-9-oxo-11-taxen-13-alpha-yl-(2R,3S)-3-tert-butoxycarbonylamino-2-hydroxy-3-phenylpropionate. Said process for preparing the crystalline ethyl acetate solvate form may comprise the following steps: - having the 4-alpha-acetoxy-2-alpha-benzoyloxy-5-beta,20-epoxy-1-beta-hydroxy-7 -beta, 10-beta-dimethoxy-9-oxo-11-taxen-13-alpha-yl(2R,3S)-3-tert-butoxycarbonylamino-2-hydroxy-3-phenylpropionate in solution in an organic solvent (for example acetone or methylene chloride) at room temperature; - making a change of solvent to ethyl acetate at atmospheric or under reduced pressure; - maintaining the so-formed solution in ethyl acetate on continued stirring, and - recovering said crystalline ethyl acetate solvate form of 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate. Regarding the step 1, the solution may be prepared by dissolving crude 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate in six volumes of acetone at room temperature. The change of solvent to ethyl acetate can be made under reduced pressure or at atmospheric pressure depending of the boiling point of the solvent used, preferably at constant volume under reduced pressure (for example 80 mbar at about 14°C with acetone). To maintain the so-formed solution in ethyl acetate in step 3, the slurry volume can be adjusted to 10 volumes by casting of ethyl acetate. Finally, regarding the step 4, to obtain the crystalline ethyl acetate solvate form of cabazitaxel, the slurry may be filtered and the resulting cake washed with ethyl acetate. This cake may be dried under vacuum at 38°C for 15H. As states previously, said process is particularly advantageous over those disclosed in the prior art since it provides a crystalline ethyl acetate solvate form of 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate in good yields and with good chemical purity. By "good yield' in the present invention, it is meant that said ethyl acetate solvate form is obtained in a yield higher than or equal to 80%. As used herein a "good chemical purity" is a purity which is higher than or equal to 99%. The present invention is also directed to a medicament comprising said crystalline ethyl acetate solvate form of 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate. Thus, the present invention also relates to a pharmaceutical composition comprises said crystalline ethyl acetate solvate form of 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate and also at least one pharmaceutically acceptable excipient. All components of the present compositions must be pharmaceutically acceptable. As used herein, a "pharmaceutically acceptable" component is one that is suitable for use with humans and/or other animals without undue adverse side effects (such as toxicity, irritation and allergic response) commensurate with a reasonable benefit/risk ratio. The compositions of the present invention are generally administered to patients, which include, but are not limited to, mammals, for example, humans, by conventional routes known in the art. The present invention further relates to the use of the ethyl acetate solvate crystalline form according to the invention, as a medicament. The present invention further relates to the use of the ethyl acetate solvate crystalline form according to the invention, as a medicament in the prevention and/or treatment of cancers. For example, the cancer may be prostate cancer.

Figure 1 of WO2013/088335: XRPD pattern ($\lambda_{Cu}$= 1.5406 A) of ethyl acetate solvate form of cabazitaxel.

The examples that follow describe the preparation of crystalline ethyl acetate solvate form of cabazitaxel and its purity. These examples are not limiting, and serve merely to illustrate the present invention.

Example 1: Preparation of crystalline ethyl acetate solvate form of cabazitaxel: The crude 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate has been dissolved in six volumes of acetone, at room temperature. The change of solvent to ethyl acetate under reduced pressure and at constant volume has been made at 80 mbar and T = 14.4°C. The slurry volume has been adjusted to 10 volumes by casting of ethyl acetate. After two hours under continued stirring, the slurry has been filtered and the resulting cake washed with ethyl acetate. Said cake has been dried under vacuum at 38°C for 15H.

Example 2: Purity of the ethyl acetate solvate form of cabazitaxel: Cabazitaxel synthesized by the conventional process (crystallized as acetone solvate in acetone/water, see WO2005/028462) and enriched with impurities has been purified by formation of the ethyl acetate solvate form. Results are compared with the cabazitaxel purified by the conventional process. The obtained results are summarized in the following table.

| ***Impurities*** | ***Starting cabazitaxel*** | ***Purified cabazitaxel with AcOEt crystallization treatment*** | ***Purified cabazitaxel with conventional process (crystallized in acetone/water)*** |
|---|---|---|---|
| ***Total*** | **1.52 %** | **0.71 %** | **0.98 %** |
| ***p-anisaldéhyde*** | 0.02 % | Undetected | Undetected |

(continued)

| Impurities | Starting cabazitaxel | Purified cabazitaxel with AcOEt crystallization treatment | Purified cabazitaxel with conventional process (crystallized in acetone/water) |
|---|---|---|---|
| Impurity A | 0.02 % | 0.01 % | 0.01 % |
| Impurity B | 0.16 % | 0.14 % | 0.10 % |
| Impurity C | 0.21 % | 0.18 % | 0.20 % |
| Impurity D | 0.02 % | Undetected | Undetected |
| Impurity E | 0.17 % | 0.12 % | 0.11 % |
| Impurities F+G | 0.18 % | 0.05 % | 0.14 % |
| Impurity H | 0.25 % | 0.10 % | 0.12 % |
| Impurity I | 0.34 % | 0.12 % | 0.24 % |
| Impurity J | 0.21 % | 0.05 % | 0.07 % |

These results show that ethyl acetate treatment has a higher purifying power. More particularly, cabazitaxel treated with ethyl acetate has less impurities F+G and I. Said crystalline ethyl acetate solvate form according to the invention was characterized by X-Ray Powder Diffraction (XRPD) and Gas Chromatography (GC) as shown below. a) X-Ray Powder Diffraction (XRPD): Experimental diagram is recorded at ambient conditions on a PANalytical X'Pert Pro MPD powder diffractometer using the Bragg-Brentano (vertical Θ-2Θ) configuration) parafocusing geometry coupled with a X'Celerator detector. A sealed copper anode X-ray tube is used, running at 45 kV and 40 mA levels. An incident beam monochromator (Johansson type: a symmetrically cut curved germanium (111) crystal) produces pure Cu $K_a$ radiation ($\lambda$ = 1.54060 A). A thin layer of the product is deposited on a single-crystal silicon wafer, cut out according to Si (510) crystallographic orientation that, by systematic extinction, impedes any Bragg reflection. In order to bring more crystallites into the diffraction position and thus reduce the influence of particle statistics on the measurements, a sample spinner is used. The spinner rotation speed is set at 1 revolution per second. The angular range extends from 1.5 to 40° in 2Θ, with a 0.017° step size in 2Θ. A counting time of 500 seconds per step was used. Figure 1 of WO2013/088335 shows XRPD pattern obtained for ethyl acetate solvate form of cabazitaxel. Characteristic peaks at 7.5, 7.9, 8.7, 10.1, 10.2, 12.6, 12.9, 13.8, 14.1 and 14.8 ± 0.2 degrees 2-theta are observed in the pattern of crystalline ethyl acetate solvate form of cabazitaxel. b) Gas chromatography: The content of ethyl acetate has been determined by Gas Chromatography (GC) on a Rtx®-200 column (fused silica). The obtained value is of about 9.5% m/m, an ethyl acetate mole per cabazitaxel mole.

Very preferred embodiments are the claims of WO2013088335, as cited and incorparated hereinafter as items 1 to 9:

1. Crystalline ethyl acetate solvate form of 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate. 2. Crystalline form of 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate according to item 1, characterized by X-Ray Powder Diffraction diagram exhibiting characteristic lines located at 8.7, 10.1, 13.8, 14.1 and 14.8 ± 0.2 degrees 2-theta. 3. Crystalline form of 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate according to item 1, characterized by X-Ray Powder Diffraction diagram exhibiting characteristic lines located at 7.5, 7.9, 8.7, 10.1, 10.2, 12.6, 12.9, 13.8, 14.1 and 14.8 ± 0.2 degrees 2-theta. 4. Medicament, characterized in that it comprises a crystalline form according to any one of items 1 to 3. 5. Pharmaceutical composition, characterized in that it comprises a crystalline form according to any one of items 1 to 3, and also at least one pharmaceutically acceptable excipient. 6. Crystalline form according to any one of items 1 to 3, for its use as a medicament. 7. Crystalline form according to any one of items 1 to 3 for use according to claim 6, wherein the medicament is for use in the prevention and/or treatment of cancers. 8. Crystalline form according to any one of items 1 to 3 for use according to claim 7, wherein the cancer is prostate cancer. 9. Process for the preparation of the crystalline form according to any one of items 1 to 3, characterized in that it comprises at least the following steps: - having the 4-alpha-acetoxy-2-alpha-benzoyloxy-5-beta,20-epoxy-1-beta-hydroxy-7-beta,10-beta-dimethoxy-9-oxo-ll-taxen-13-alpha-yl(2R,3S)-3-tert-butoxycarbonylamino-2-hydroxy-3-phenylpropionate in

solution in an organic solvent at room temperature; - making a change of solvent to ethyl acetate at atmospheric or under reduced pressure; - maintaining the so-formed solution in ethyl acetate on continued stirring, and - recovering said crystalline ethyl acetate solvate form of 4-alpha-acetoxy-2-alpha-benzoyloxy-5-beta,20-epoxy-1-beta-hydroxy-7-beta,10-beta-dimethoxy-9-oxo-11-taxen-13-alpha-yl(2R,3S)-3-tert-butoxycarbonylamino-2-hydroxy-3-phenylpropionate. The invention relates to an administration unit comprising crystalline ethyl acetate solvate form of cabazitaxel.

SPECIFIC INORMATION CONCERNING ASPECT (II) OF THE INVENTION

Aspect (ii) of the invention relates to an administration unit comprising solid dispersion of composition X ({drug2a}). The invention relates to a solid form of compositon X, namely to solid dispersion of composition X ({drug2a}), which is useful for treating cancer, in particular melanoma. Compositon X is described in WO2013/087546 and is a pharmaceutical composition comprising a polymer polyvinylpyrrolidone (PVP) or copovidone, a compound according to formula (IIA),

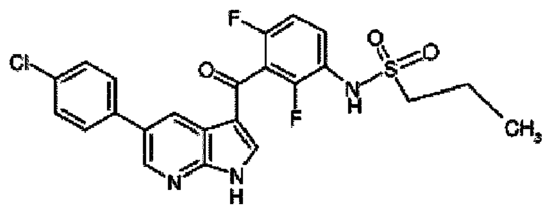

(IIA)

or a compound according to formula (IIB),

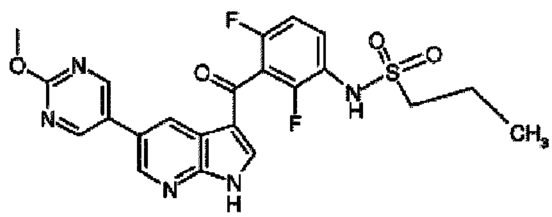

(IIB)

and, optionally, a surfactant and/or hydroxypropyl methylcellulose-acetate succinate. A solid dispersion of composition X ({drug2a}) is described in WO2013087546, which is incorporated by reference. As used herein, solid dispersion of composition X ({drug2a}) is a form of compositon X as described in WO2013087546 and further described hereinfater. The present invention relates to a pharmaceutical composition comprising a solid dispersion of a drug. In the composition, the drug is in substantially amorphous form. The present invention relates to a pharmaceutical composition comprising a solid dispersion comprising a compound of formula (IIA),

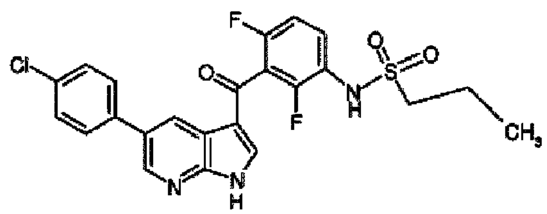

or a compound according to formula (IIB),

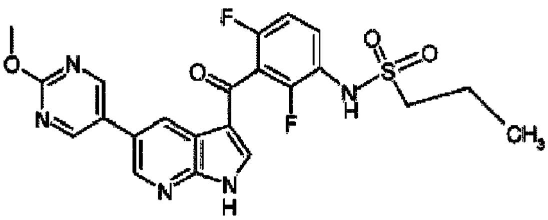

a polymer that is polyvinylpyrrolidone (PVP) or copovidone, and, optionally, a surfactant and/or HPMC-AS. The present invention also relates to a method of treating or ameliorating cancer comprising administering to a subject in need of such treatment a therapeutically effective amount of a composition of the present invention.

Figure 1 of WO2013/087546: 2-stage non-sink dissolution test results for Formulations 48A, 48B and 48C. Figure 2 of WO2013/087546: X-ray diffraction patterns for Formulation 49A and 49C.

The present invention provides a pharmaceutical composition comprising a solid dispersion comprising a Drug (as defined below) and a polymer. As used herein, the term "substantially in amorphous form" means that greater than 50%, or greater than 55%, or greater than 60%, or greater than 65%, or greater than 70%, or greater than 75%, or greater than 80%, or greater than 85%, or greater than 90%, or greater than 95% of the Drug is present in amorphous form. As used herein, the term "solid dispersion" means any solid composition having at least two components, for example a Drug and a polymer. As used herein, the term "molecularly dispersed" refers to the random distribution of a Drug with a polymer. As used herein, the term "solid molecular complex" refers to a solid dispersion that includes a Drug molecularly dispersed within a matrix formed by a polymer (hereafter, a "polymer matrix"). As used herein, the term "immobilized", with reference to the immobilization of a Drug within a polymer matrix, means that the molecules of a Drug interact with the molecules of the polymer in such a way that the molecules of the Drug are held in the aforementioned matrix and prevented from crystal nucleation due to lack of mobility. For example, the polymer may prevent intramolecular hydrogen bonding or weak dispersion forces between two or more Drug molecules. As used herein, "Drug" refers to either Compound IIA or Compound IIB (both defined below). Both Compound IIA and Compound IIB are Raf kinase inhibitors. As such, they are useful in treating or ameliorating cancer.

"Compound IIA", as used herein, refers to propane- 1- sulfonic acid {3-[5-(4-chloro-phenyl)-1H-pyrrolo[2,3-b] pyridine-3-carbonyl]-2,4-difluoro-phenyl}-amide. This drug has the following structure

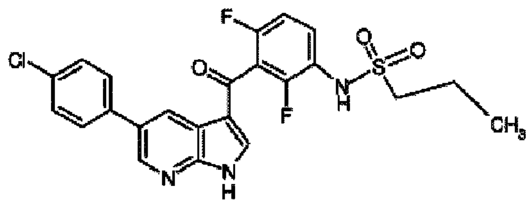

"Compound IIB", as used herein, refers to propane-1-sulfonic acid {2,4-difluoro-3-[5-(2-methoxy-pyrimidin-5-yl)-1H-pyrrolo[2,3-b]pyridine-3-carbonyl]-phenyl-amide. This drug has the following structure.

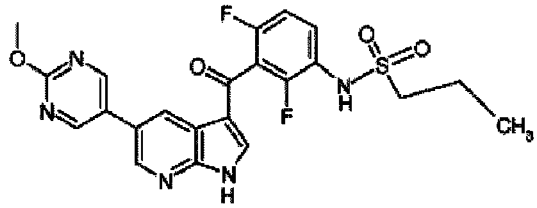

It has been found that choice of polymer has a significant effect on AUC and Cmax achieved in vivo (see Example 8). In an embodiment, the polymer is polyvinylpyrrolidone (PVP) or copovidone. In a particular embodiment, the polymer is PVP. In another particular embodiment, the polymer is copovidone. Copovidone (available from BASF and ISP) is a hydrophilic copolymer of 1-vinyl-2-pyrrolidone and vinyl acetate in the mass proportion of 6:4. Copovidone is capable of forming a stable solid dispersion with the Drug which retains the Drug in amorphous form for up to eight hours in the physiological relevant fluid, thus improving its bioavailability upon administration. In addition to the above, copovidone is a non-ionic polymer that has pH independent solubility in the physiological pH range (1.5- 7.5). As a result, a solid dispersion formed using copovidone is capable of releasing the Drug throughout the GI tract, thus allowing for

improved absorption of the Drug. In an embodiment, the Drug is molecularly dispersed in the aforementioned polymer. In an embodiment, the solid dispersion is a solid molecular complex of Compound IIA or Compound IIB and said polymer. In an embodiment, the Drug is immobilized within a matrix formed by said polymer. In an embodiment, the composition comprises a solid dispersion wherein the Drug is present in an amount of from about 1% to about 50%, from about 1% to about 40%, or from about 1% to about 30% by weight of the solid dispersion. In an embodiment, the solid dispersion has a single glass transition temperature higher than about 50°C, preferably above 100°C. In an embodiment, the composition comprises a solid dispersion comprising a polymer wherein the polymer is present in an amount of from about 50% to about 98.8%, from about 60% to about 98.8%, or from about 70% to about 98.8% by weight of the solid dispersion. In an embodiment, the solid dispersion is prepared using a hot melt extrusion process (see, e.g., Ghebre-Sellassie, I. and C. Martin, Pharmaceutical Extrusion Technology, Marcel Dekker, 2003). In such a process, the components of the solid dispersion are blended and extruded at high temperature. In an embodiment, the composition comprises Compound IIA molecularly dispersed in copovidone. In an embodiment, the solid dispersion is a solid molecular complex of Compound IIA and copovidone. In an embodiment, Compound IIA is immobilized within a matrix formed by copovidone. In an embodiment, the composition comprises a solid dispersion wherein Compound IIA is present in an amount of from about 1% to about 40% by weight of the solid dispersion and copovidone is present in an amount of from about 60% to about 98.8% by weight of the solid dispersion. In an embodiment, the composition comprises a solid dispersion wherein Compound IIA is present in an amount of from about 1% to about 40% by weight of the solid dispersion and copovidone is present in an amount of from about 60% to about 98.8% by weight of the solid dispersion. In an embodiment, the solid dispersion comprising Compound IIA and copovidone is prepared using a hot melt extrusion process (see, e.g., Ghebre-Sellassie, I. and C. Martin, Pharmaceutical Extrusion Technology, Marcel Dekker, 2003). In an embodiment, the composition comprises Compound IIB molecularly dispersed in copovidone. In an embodiment, the solid dispersion is a solid molecular complex of Compound IIB and copovidone. In an embodiment, Compound IIB is immobilized within a matrix formed by said copovidone. In an embodiment, the composition comprises a solid dispersion wherein Compound IIB is present in an amount of from about 1% to about 50% by weight of the solid dispersion and copovidone is present in an amount of from about 50% to about 98.8% by weight of the solid dispersion. In an embodiment, the solid dispersion comprising Compound IIB and copovidone is prepared using a hot melt extrusion process (see, e.g., Ghebre-Sellassie, I. and C. Martin, Pharmaceutical Extrusion Technology, Marcel Dekker, 2003). In an embodiment of the present invention, the composition further comprises a flow enhancer. In a particular embodiment, the flow enhancer is colloidal silica (also designated herein as colloidal silicon dioxide). The flow enhancer may, for example, be present in the composition in an amount of up to about 5% by weight of the composition, or up to about 3% by weight of the composition. It has been found that compositions comprising colloidal silicon dioxide exhibit improved stability and improved AUC and Cmax as compared with the composition that did not contain colloidal silicon dioxide (see Example 6). Melt extrusion formulations exhibit the advantages of good bioavailability and solid state stability. In addition, there are manufacturing advantages to using melt extrusion formulations. It is desirable to develop melt extrusion formulations that also have the advantages of lower dose to achieve sufficient therapeutic effect, low bulk density, high surface area, enhanced drug loading with lower polymer loading, good solubility and excellent physico-chemical properties. For patient compliance, development of a higher strength dosage form such as a tablet is desirable. Solid dispersion formulations known in the art require a high usage of polymer which may impart undesirable binder effects on tablets, thus slowing tablet disintegration. While disintegrants may be added, the addition of additional excipients may have a negative effect on tablet compaction. It is advantageous to develop other solid dispersion formulation tablets with fast disintegration and good tablet compaction. It has been found that, in embodiments comprising Compound IIA as the Drug, the addition of a surfactant as a component of certain solid dispersion allows for improved dissolution of Compound IIA from a solid dispersion. Accordingly, an embodiment of the present invention is a composition comprising a solid dispersion which comprises Compound IIA, a polymer that is PVP or copovidone, and a surfactant. In an embodiment of the present invention, the surfactant is selected from the group consisting of sodium lauryl sulfate (SLS), glycerol monostearate, dioctyl sodium succinate (DOSS), and mixtures thereof. In an embodiment, the surfactant is SLS. In another embodiment, the surfactant is glycerol monostearate. In yet another embodiment, the surfactant is DOSS. In certain embodiments, the surfactant is present in an amount of up to about 10% by weight of the solid dispersion, or up to about 5% by weight of the solid dispersion, or from about 1% to about 2% by weight of the solid dispersion. In a particular embodiment, the composition comprises a solid dispersion which comprises Compound IIA, copovidone and DOSS. In a more particular embodiment, DOSS is present in an amount of from about 1% to about 2% by weight of the solid dispersion. It has been found that, in embodiments of the present invention wherein the Drug is Compound IIB and the polymer is copovidone, the addition of hydroxypropyl methylcellulose - acetate succinate (HPMC-AS) in the solid dispersion allows for improved disintegrating properties for the resulting dosage form. HPMC-AS of various grades may be used, including HPMC-AS, LF; HPMC-AS, M; HPMC-AS, HF; and HPMC-AS, HG. An embodiment of the present invention is a composition comprising a solid dispersion which comprises Compound IIB, copovidone and HPMC-AS. In another embodiment, the solid dispersion comprises Compound IIB, copovidone and HPMC-AS, LF. In another embodiment, the solid dispersion comprisies Compound IIB, copovidone and HPMC-AS, HF. In yet another embodiment, the solid dispersion comprises

Compounnd II, copovidone and HPMC-AS, HG. In such embodiments, It has been found that the ratio of the copovidone to HPMC-AS used in the solid dispersion is of critical importance. In an embodiment, the ratio is from about 15:85 to about 50:50. In another embodiment, the ratio is from about 15:85 to about 40:60. In a particular embodiment, the ratio is about 35:65. In another particular embodiment, the ratio is about 20:80. In an embodiment, the present invention relates to a pharmaceutical composition comprising a solid dispersion comprising a Drug, a polymer that is polyvinylpyrrolidone (PVP) or copovidone, and, optionally, a surfactant and/or HPMC-AS. In addition to the above, the present invention contemplates to use of additional components in the present composition. Plasticizers, for example PEG-400 and poloxamer (which also serves as a surfactant), may be used. In addition, disintegrants, for example sodium starch glycolate, Polypasdone XL, and croscarmellose sodium may be used. Further lubricants such as magnesium stearate may be used. In addition to the above, the present invention relates to a method of treating or ameliorating cancer comprising administering to a subject in need of such treatment a therapeutically effective amount of a composition of the present invention. In a particular embodiment, the cancer is melanoma. Thus, the present invention also relates to the compositions disclosed herein for use as medicaments, in particular as medicaments for the treatment of cancer, more specifically melanoma.

Example 1: This example describes a formulation of the present invention comprising Compound IIA. The contents of the formulation were as follows.

| | Wt. % |
|---|---|
| Compound IIA | 21.5 |
| PVP (Povidone K-90) | 51.6 |
| PEG-400 | 12.9 |
| Poloxamer | 10 |
| Sodium Starch Glycolate | 3 |
| Colloidal Silicon Dioxide (Aerosil 200) | 1 |

The formulation was prepared using the HME process (Ghebre-Sellassie, I. and C. Martin, Pharmaceutical Extrusion Technology, Marcel Dekker, 2003). Compound IIA, PVP and PEG 400 were mixed and the blend was extruded at 160°C. The resulting extrudates were milled by hand. Poloxamer, sodium starch glycolate and colloidal silicon dioxide were added externally to the milled extrudate and blended together to achieve a homogeneous blend. The blend was filled into hard gelatin capsules.

Example 2: For comparison, a formulation containing Compound IIA in stable crystalline form was prepared. Stable Crystalline Formulation:

| | Wt. % |
|---|---|
| Compound IIA | 54.5 |
| ProSolv® (JRS Pharma) | 33 |
| Poloxamer | 10 |
| Sodium Starch Glycolate | 1 |
| Magnesium Stearate | 1 |
| Colloidal Silicon Dioxide (Aerosil 200) | 0.5 |

This formulation was prepared by a dry blending method (Lachman et al., The Theory and Practice of Industrial Pharmacy, Lea & Febiger, 1986). All the components were blended for a suitable time and the resulting dry blend was filled into hard gelatin capsules.

Example 3: Also for comparison, a formulation containing Compound IIA in stable crystalline form dissolved in a lipid-based vehicle (the Lipid Formulation) was also prepared. Lipid Formulation:

| | Wt. % |
|---|---|
| Compound IIA | 10 |

(continued)

| | Wt. % |
|---|---|
| Labrosol® (Gattefosse) | 46.8 |
| Gelucire® (Gattefosse) | 21.6 |
| Vitamin E Tocopherol Glycol Succinate (Vitamin E - TPGS) | 21.6 |

This formulation was prepared by dispersing Compound IIA with Labrosol® (Gattefosse), Gelucire® (Gattefosse) and Vitamin E-TPGS in a mortar and pestle. The resulting lipid suspension was then filled into hard gelatin capsules.

Example 4: A single dose oral PK study using the formulations of Examples 2 and 3 and the solid dispersion formulation of Example 1 was conducted in Female Beagle Dogs using cross over design. All the formulations were dosed at 50 mg/ kg dose level. It has been found that, when Compound IIA was administered as a solid dispersion (the Example 1 formulation), it exhibited significantly higher bioavailability compared to when Compound IIA was administered in either the formulations of Examples 2 or 3 wherein Compound IIA was in crystalline form. Table 1: Comparison of Dog PK data - Solid Dispersion vs. Crystalline:

| Formulation | Form of Compound IIA | AUC/dose (ng.h/mL) | Cmax/dose (ng/mL) |
|---|---|---|---|
| Example 2 Formulation | Crystalline | 8-10 | 0.6-1 |
| Example 3 Formulation | Crystalline | 20-24 | 4.5-5.2 |
| Example 1 Formulation | Amorphous | 535-560 | 90-115 |

Example 5: The miscibility of Compound IIA in various polymers at constant temperature was analyzed. Compound IIA and polymer were mixed to produce a blend that was 10% by weight Compound IIA and 90% by weight polymer. The homogeneous blend was extruded using a Haake® MiniLab bench-top extruder. The feed rate was constant between 1- 2 g/ min and screw speed was set at 100 RPM. The blends were extruded at two different temperatures: 160 and 200°C respectively. The extrudates were classified as miscible, partially immiscible, immiscible as per PXRD patterns and visual observations. It has been found that Compound IIA has higher solubility/ miscibility in copovidone compared to other polymers when melt extruded at 160°C (Table 2). Table 2: Miscibility studies for Compound IIA:

| Polymer with 10% Compound IIA | Miscibility @ 160°C | Miscibility @ 200°C |
|---|---|---|
| Povidone K 30 | Partially immiscible | Miscible |
| Copovidone | Miscible | Miscible |
| Povidone K 90 | Partially immiscible | Miscible |
| Polyvinyl acetate phthalates | Partially immiscible | Polymer degradation |
| Eudragit E 100 | Immiscible | Immiscible |
| Hypromellose | Partially Immiscible | Partially immiscible |
| Hypromellose-AS | Partially Immiscible | Polymer degradation |
| Poloxamer | Immiscible | Polymer degradation |

Example 6: Two formulations, one without and one with colloidal silicon dioxide (Examples 6a and 6b, respectively) were produced as follows:

| | 6a Wt. % | 6b Wt. % |
|---|---|---|
| Compound IIA | 25 | 24 |
| Povidone | 60 | 59 |
| Glyceryl Monostearate | 15 | 15 |
| Aerosil® 200 (colloidal silicon dioxide) | 0 | 2 |

The formulations were processed using Leistriz Micro 18 lab scale extruder at a constant feed rate of 10-15 g/min, screw speed of 150 rpm and processing temperature in the range of 160- 185°C. Upon extrusion, the extrudates were milled into fine powder and filled into hard gelatin capsule for testing and evaluation purpose. Both formulations showed glass transition temperature in the range of 110 -120°C and amorphous PXRD pattern. Both formulations provided similar in vitro release profile. The formulation containing colloidal silicon dioxide was found to be stable for up to 4 hours under normal conditions and also had improved AUC and Cmax as compared with the formulation that did not contain colloidal silicon dioxide (see Table 3). Table 3: AUC and Cmax for Formulations 6a and b:

| | 6a | 6b |
|---|---|---|
| Motor load % | 95-100 | 95-100 |
| Cmax/Dose (ng/ml/mg/kg) | 135-200 | 342-370 |
| AUC/Dose (ng*Hours/mL/mg/kg) | 700-2000 | 1500-3600 |

Example 7: The following evaluation showed that the addition of glyceryl monostearate improved the processability of the formulation (Table 5). Table 5: Solid dispersion formulations with or without glyceryl monostearate:

| Example | 7a % (w/w) | 7b % (w/w) | 7c % (w/w) |
|---|---|---|---|
| Compound IIA | 10 | 10 | 10 |
| Povidone | | | 85 |
| Copovidone | 85 | 90 | |
| Glyceryl Monostearate | 5 | | 5 |
| Processibility (% motor load) | 50-70 | 90-95 | 40-50 |

Example 8: It has been also found that choice of surfactant and polymer also have significant effect on AUC and Cmax. The solid dispersion formulation below containing copovidone and sodium lauryl sulfate provided higher AUC and Cmax compared to the solid dispersion formulation containing povidone and glycerol monostearate (see Table 6). Table 6: AUC and Cmax for Formulations 8a, b and c (Ingredients in %(w/w)):

| Example | 8a | 8b | 8c |
|---|---|---|---|
| Compound IIA | 25 | 20 | 20 |
| Povidone | 58 | | |
| Copovidone | | 74 | 78 |
| Glyceryl Monostearate | 15 | 5 | |
| Sodium Lauryl Sulfate | | | 1 |
| Colloidal Silicon (Aerosil 200) | 2 | 1 | 1 |
| Total (%w/w) | 100 | 100 | 100 |
| Cmax/Dose (ng/ml/mg/kg) | 342-370 | 500-850 | 600-1050 |
| AUC/Dose (np*Hours/mL/mp/kp) | 1500-3600 | 2780-4780 | 3540-7560 |

Examples 9 - 11: Compared to solubilizers such as SLS that also provided higher bioavailability from melt extrudates, tablets containing DOSS provided a better in vitro release, suggesting DOSS surprisingly functions as release modifier. Table 7: Formulations 9, 10 and 11 (Ingredients in mg/tablet) and dissolution:

| Examples | 9 | 10 | 11 |
|---|---|---|---|
| Compound IIA | 200 | 200 | 200 |
| Copovidone | 584 | 584 | 576 |

(continued)

| Examples | 9 | 10 | 11 |
|---|---|---|---|
| Colloidal Silicon Dioxide | 8 | 8 | 8 |
| Sodium Lauryl Sulfate | 8 | | |
| Dioctyl Sodium Sulfosucccinate (DOSS) | | 8 | 16 |
| Tablet weight | 800 | 800 | 800 |
| Extrusion temperature (°C) | 160-185 | 160-185 | 160-185 |
| Feed rate (g/ min) | 10-20 | 10-20 | 10-20 |
| Screw speed (RPM) | 150-200 | 150-200 | 150-200 |
| PXRD pattern | Amorphous | Amorphous | Amorphous |
| Dissolution (D60 min) | ~10% | ~50% | ~50% |
| Dissolution (D 180) | ~20% | 100% | 100% |
| Compression - hardness (25 kN) | ~120 N | ~145N | ~140 N |

Examples 12 to 18: The method of addition of solubilizers in the solid dispersion formulation has significant effect on dissolution rate and drug recovery. Intragranular addition of docusate sodium 85% (Dioctyl sodium sulfosuccinate containing 15% sodium benzoate) provided higher dissolution rate and recovery.

Table 8:

| Ingredient [%w/w] | | | | | | | |
|---|---|---|---|---|---|---|---|
| Examples | 12 | 13 | 14 | 15 | 16 | 17 | 18 |
| Intragranular Excipients | | | | | | | |
| Compound IIA | 20 | 25 | 15 | 27 | 20 | 20 | 20 |
| Copovidone | 76.5 | 71.9 | 81.9 | 70 | 75.4 | 75 | 75.5 |
| Docusate sodium 85% | 0.1 | 0.4 | 0.1 | 0.4 | 1 | 2 | 0.5 |
| Colloidal Silicon Dioxide | 0.1 | 0.1 | 0.1 | 0.1 | 0.2 | 0.3 | 0.3 |
| Extragranular Excipients | | | | | | | |
| Colloidal Silicon Dioxide | 0.1 | 0.1 | 0.2 | 0.1 | 0.2 | 0.1 | 0.2 |
| Glyceryl behenate | 0.8 | 0.5 | 0.2 | 0.5 | 0.8 | 0.2 | 0.5 |
| Opadry II | 2.4 | 2 | 2.5 | 1.9 | 2.4 | 2.4 | 3 |

Example 19: This example describes a formulation of the present invention comprising Compound IIA.

| | [mg/tablet] |
|---|---|
| Intragranular Excipients | |
| Compound IIA | 240.0 |
| Copovidone[1] | 940.0 |
| Docusate sodium 85%[1,2] | 10.0 |
| Colloidal Silicon Dioxide[1] | 10.0 |
| Extragranular Excipients | |
| Colloidal Silicon Dioxide | 2.0 |
| Glyceryl behenate | 8.0 |

(continued)

| | |
|---|---|
| Extragranular Excipients | |
| Kernal weight | 1210.0 |
| Coating composition | |
| Opadry II Pink[3] | 30.0 |
| Total tablet Weight | 1240.0 |
| [1]. These four ingredients were the components of the powder mixture which was processed (extruded) through the Leistritz extruder. [2]. Dioctyl sodium sulfo succinate containing 15% sodium benzoate. [3]. Complete coating system. | |

Compound IIA, copovidone, docusate sodium 85% and colloidal silicon dioxide were blended and extruded using Leistriz Micro 18 lab scale extruder. The feed rate was constant between 10-15 g/ min and screw speed was set at 150 RPM. The processing temperature was set in between 160- 185°C. The extrudates were milled and external components- colloidal silicon dioxide and glycerol behenate- were added and blended for 15 min using suitable powder blender. The blend was compressed into tablet with hardness in the rage of 110 to 180 N hardness. The tablets were coated with Opadry II pink complete coating system.

Example 20: This example describes a formulation of the present invention comprising Compound IIB. The contents of the formulation were as follows:

| | Wt. % |
|---|---|
| Compound IIB | 15.1 |
| Copovidone (Kollidon 64) | 20.8 |
| HPMC-AS, LF | 38.8 |
| Colloidal Silicon Dioxide (Aerosil 200) | 1.8 |
| Microcrystalline cellulose (Avicel PH 102) | 15.0 |
| Polyplasdone XL | 5.0 |
| Croscarmellose sodium (AcDiSol) | 3.0 |
| Magnesium Stearate | 0.5 |

The formulation was prepared using the HME process (Ghebre-Sellassie, I. and C. Martin, Pharmaceutical Extrusion Technology, Marcel Dekker, 2003). Compound IIB, copovidone and HPMC-AS were mixed and the blend was extruded at 160 0 C. The resulting extrudates were milled by hand. Colloidal sodium dioxide, microcrystalline cellulose, Polyplasdone XL, croscarmellose sodium, and magnesium stearate were added externally to the milled extrudate and blended together to achieve a homogeneous blend.

Examples 21 to 32a: The following are additional compositions comprising Compound IIB wherein Compound IIB contained in amorphous form. The amounts are expressed in wt of the composition.

Table 9:

| Example | Compound II | Copovidone | HPMC-AS | Covpovidone/ HPMC-AS ratio | Additional Components |
|---|---|---|---|---|---|
| | | | | | |
| 21 | 25 | 74 | none | 1000 | 1%SLS |
| 22 | 25 | 55.5 | 18.5 | 75/25 | 1% SLS |
| 23 | 20 | 40 | 40 | 50/50 | no SLS |
| 24 | 20 | 39.5 | 39.5 | 50/50 | 1%SLS |

(continued)

| Example | Compound II | Copovidone | HPMC-AS | Covpovidone/ HPMC-AS ratio | Additional Components |
|---|---|---|---|---|---|
| 25 | 20 | 39.9 | 39.9 | 50/50 | 0.2% SLS |
| 26 | 20 | 70 | none | 100/0 | 10% Cremophor |
| 27 | 20 | 79 | none | 100/0 | 1% DOSS |
| 28 | 20 | 97 | 97 | 50/50 | 5%Cramophor, 1% DOSS |
| 29 | 20 | 39 | 39 | 50/50 | 1% DOSS |
| 30 | 20 | 31 | 47 | 40/60 | 1% DOSS, 1% silica |
| 31 | 20 | 97 | 37 | 50/50 | 5% Span, 1% silica |
| 32a | 10 | none | 87 | 0/100 | 2% DOSS, 1% silica |

Examples 32b to 47: The following are additional compositions comprising Compound IIB wherein Compound IIB is contained in amorphous form. The amounts are expressed in wt % of the composition. With the exception of Example 43, each composition was loaded into tablets which were 75.5% by weight of tablet was the composition. The tablets formed using the composition of Examples 36 and 41 showed no disintegration. The tablets formed using the compositions of example 32b to 35, 37 to 40, 42, and 44 to 47 showed disintegration. For Example 43, tablets containing the composition at 60% to 75% by weight showed no disintegration.

Table 10:

| Example | % drug | % Copovidone | % HPMC-AS | Copovidone HPMC-AS ratio |
|---|---|---|---|---|
| 32b | 20 | 31.6 | 47.4 | 40/60 |
| 33 | 20 | 23.7 | 55.3 | 30/70 |
| 34 | 20 | 27.6 | 51.4 | 35/65 |
| 35 | 20 | 31.6 | 47.4 | 40/60 |
| 36 | 20 | 51.4 | 27.6 | 65/35 |
| 37 | 15 | 29.4 | 54.6 | 35/65 |
| 38 | 20 | 27.6 | 51.4 | 35/65 |
| 39 | 25 | 29.6 | 44.4 | 40/60 |
| 40 | 25 | 37 | 37 | 50/50 |
| 41 | 40 | 59 | none | 100/0 |
| 42 | 30 | 34.5 | 34.5 | 50/50 |
| 43 | 40 | 59 | none | 100/0 |
| 44 | 20 | 39.5 | 39.5 | 50/50 |
| 45 | 25 | 37 | 37 | 50/50 |
| 46 | 25 | 29.6 | 44.4 | 40/60 |
| 47 | 30 | 34.5 | 34.5 | 50/50 |

Example 48: This example describes formulations of the present invention utilizing different grades of HPMCAS and polymeric ratios prepared by hot melt extrusion. The compositions of the formulations are presented in Table 11. Formulation 48 A was prepared by tumble blending of drug and colloidal silicon dioxide, followed by delumping using a rotary impeller mill with a 0.055" screen and final blending with polymeric excipients. Melt extrusion was conducted using a Leistritz 18-mm twin screw co-rotating extruder in a 20: 1 configuration with 3 mm die at a processing temperature of 175°C. Formulations 48B and 48C were manufactured by tumble blending drug and polymeric excipients prior to melt extrusion. Melt extrusion was conducted using a Haake Minilab conical twin screw extruder maintained at a temperature of 175°C.

Table 11 - Compound IIB Melt Extruded Formulations Expressed as a Percentage of Total Extrudate Amount:

| Material | Formulation 48A | Formulation 48B | Formulation 48C |
|---|---|---|---|
| Compound IIB | 20.00 | 20.0 | 20.0 |
| Copovidone | 27.65 | 16.0 | 16.0 |
| HPMCAS-LF | 51.35 | - | - |
| HPMCAS-MF | - | 64.0 | - |
| HPMCAS-HF | - | - | 64.0 |
| Colloidal Silicon Dioxide | 1.00 | - | - |

Following extrusion, all dispersions were milled, screened to a fine powder having a size approximately less than 250 microns and tested for dissolution performance under non-sink conditions applying a 2-stage dissolution test. Dissolution profiles for each formulation, tested as powder containing 250 mg equivalent of Compound IIB, were monitored using a fiber-optic probe and USP apparatus II 6-vessel dissolution assembly implementing a pH change methodology. First stage media was pH 2 simulated gastric fluid without enzyme at a total volume of approximately 500 ml. The second stage media was a biorelevant FaSSIF media at pH 6.5, obtained by adding concentrate to the acidic volume of the first stage to achieve a total volume of approximately 1000 ml. Profiles for each formulation, presented in Figure 1 of WO2013/087546, show greater levels of drug in solution than the crystalline solubilities of Compound IIB. Melt extruded solid dispersions of Formulation A and Formulation C were also administered to beagle dogs (n = 6) as a 75 mg/ml total solids oral suspension in a pH 4.0 2.0% hydroxypropyl cellulose vehicle at a dose of 75 mg API/kg. The pharmacokinetic measurements of Compound IIB are presented in Table 12.

Table 12 -Pharmacokinetic Measurements of Compound IIB at 75 mg/kg in Beagle Dogs. Data Presented as Mean Value + Standard Deviation:

| Metric | Formulation 48A | Formulation 48C |
|---|---|---|
| AUC0-24 | 244,000 ± 165,000 | 352,000 ± 258,000 |
| Cmax | 24,000 ± 9,100 | 39,200 ±14,900 |

Example 49: This example describes formulations of the present invention utilizing differing Copovidone:HPMCAS-HF ratios to increase the amount of Compound IIB contained within the dispersion in a substantially amorphous state when prepared by hot melt extrusion at 175°C. The compositions of each formulation are presented in 13 along with critical product and process attributes. Formulations 49A, 49B, 49C and 49D were manufactured by tumble blending drug and polymeric excipients prior to melt extrusion. Melt extrusion was conducted using a Haake Minilab conical twin screw extruder maintained at a temperature of 175°C and screw speed of 360 rpm. The appearance of a transparent amber glass from the die exit was used to identify amorphous materials which were confirmed by x-ray diffraction testing performed on milled powder samples of solid dispersion. Representative diffraction patterns for Formulation 49A and Formulation 49C are shown in Figure 2 of WO2013/087546.

Table 13 - Compound IIB Hot Melt Extruded Formulation, Process and Product Attributes:

| Metric | Formulation 49A | Formulation 49B | Formulation 49C | Formulation 49D |
|---|---|---|---|---|
| FORMULATION | | | | |
| Compound IIB | 20.00 | 25.0 | 30.0 | 35.0 |
| Copovidone | 16.00 | 15.0 | 35.0 | 32.5 |
| HPMCAS-HF | 64.00 | 60.0 | 35.0 | 32.5 |
| MANUFACTURING | | | | |
| Temp. [°C] | 175 | 175 | 175 | 175 |
| Appearance | Clear Glass | Opaque | Clear Glass | Opaque |
| XRD | Amorphous | Not-Tested | Amorphous | Not-Tested |

Especially preferred embodiments are the claims of WO2013087546 which are repeated as items 1 to 22 hereinafter:

1. A pharmaceutical composition comprising a solid dispersion comprising a polymer polyvinylpyrrolidone (PVP) or copovidone, a compound according to formula (IIA),

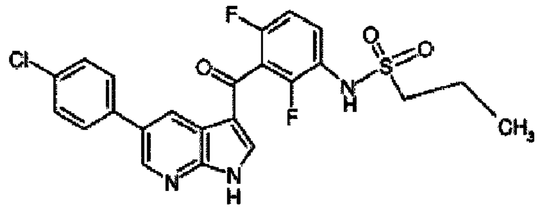

or a compound according to formula (IIB),

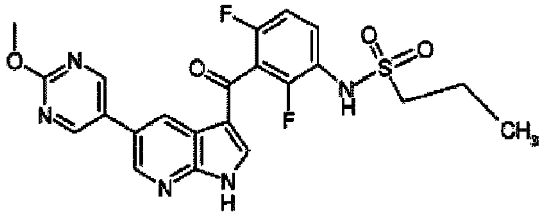

and, optionally, a surfactant and/or hydroxypropyl methylcellulose -acetate succinate. 2. A composition according to item 1 wherein said compound is molecularly dispersed in said polymer. 3. A composition according to item s 1 or 2 wherein said solid dispersion is a solid molecular complex of said compound and said polymer. 4. A composition according to item 3 wherein said compound is immobilized within a matrix formed by said polymer. 5. A composition according to any one of items 1 to 4 wherein said polymer is copovidone. 6. A composition according to any one of items 1 to 5 wherein said compound is present in an amount of from about 1% to about 50% by weight of the solid dispersion. 7. A composition according to any one of items 1 to 6 wherein said polymer is present in an amount of from about 50% to about 98.8% by weight of the solid dispersion. 8. A composition according to any one of items 1 to 7 wherein said solid dispersion is prepared using a hot melt extrusion process. 9. A composition according to any one of items 1 to 8 further comprising a flow enhancer. 10. A composition according to item 9 wherein said flow enhancer is colloidal silicone. 11. A composition according to item 9 or 10 wherein said flow enhancer is present in an amount of up to about 5% by weight of the composition. 12. A composition according to any one of items 1 to 11 wherein said polymer is copovidone and said solid dispersion comprises a surfactant. 13. A composition according to item 12 wherein said surfactant is selected from the group consisting of sodium lauryl sulfate (SLS), glycerol monostearate, dioctyl sodium succinate (DOSS), and mixtures thereof. 14. A composition according to item 12 or 13 wherein said surfactant is dioctyl sodium succinate. 15. A composition according to any one of items 12 to 14 wherein said surfactant is present in an amount of up to about 10% by weight of said solid dispersion. 16. A composition according to any one of items 1 to 15 wherein said compound is a compound of formula (IIA). 17. A composition according to any one of items 1 to 15 wherein said compound is a compound of formula (IIB). 18. A composition according to any one of items 1 to 11 wherein said compound is a compound of formula (IIB) and said polymer is copovidone and said solid dispersion comprises hydroxypropyl methylcellulose - acetate succinate. 19. A composition according to item 18 wherein said copovidone and said hydroxypropyl methylcellulose - acetate succinate are present in the solid dispersion in a ratio of from about 15:85 to about 40:60, respectively. 20. A composition according to any one of items 1 to 19 for use as medicament. 21. A composition according to any one of items 1 to 19 for use as medicament for the treatment of cancer, in particular melanoma. 22. The novel methods, compositions and uses substantially as described herein.

SPECIFIC INORMATION CONCERNING ASPECT (III) OF THE INVENTION

Aspect (iii) of the invention relates to an administration unit comprising crystalline form of phosphate salt ({drug3a}) or crystalline form of of nitrate salt of 4-(R)-(6,7-dihydro-5H-pyrrolo[1,2-c]imidazol-5-yl)-3-fluoro-benzonitrile. The invention relates to a solid form of phosphate salt or of nitrate salt of 4-(R)-(6,7-dihydro-5H-pyrrolo[1,2-c]imidazol-5-yl)-3-fluoro-benzonitrile, namely to crystalline form of phosphate salt or crystalline form of of nitrate salt of 4-(R)-(6,7-dihydro-5H-pyrrolo[1,2-c]imidazol-5-yl)-3-fluoro-benzonitrile, which is useful for treating Cushing's disease. Crystalline form of phosphate salt or crystalline form of of nitrate salt of 4-(R)-(6,7-dihydro-5H-pyrrolo[1,2-c]imidazol-5-yl)-3-fluoro-benzonitrile is described in WO2013/109514, which is incorporated by reference. Phosphate salt and nitrate salt of

4-(R)-(6,7-dihydro-5H-pyrrolo[1,2-c]imidazol-5-yl)-3-fluoro-benzonitrile is a salt of 6,7-dihydro-5H-pyrrolo[1,2-c]imidazol-5-yl-3-fluoro-benzonitrile, its 4-(R)-enantiomer with Formula (III)

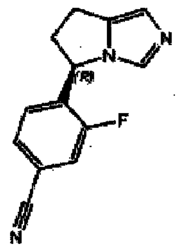

(III).

As used herein, crystalline form of phosphate salt ({drug3a}) or crystalline form of nitrate salt of 4-(R)-(6,7-dihydro-5H-pyrrolo[1,2-c]imidazol-5-yl)-3-fluoro-benzonitrile is a crystalline form of phosphate salt ({drug3a}) or crystalline form of nitrate salt of 4-(R)-(6,7-dihydro-5H-pyrrolo[1,2-c]imidazol-5-yl)-3-fluoro-benzonitrile characterized and described in WO2013/109514 and repeated hereinafter: The present invention relates to specific salts and salt forms of 4-(R)-(6,7-Dihydro-5H-pyrrolo[1,2-c]imidazol-5-yl)-3-fluoro-benzonitrile (also referred to as "Compound (III)" within this application), these salts and salt forms for use in methods of treatment of diseases and conditions, the use of these salts and salt forms in the manufacture of medicaments for the treatment of diseases and conditions, methods of treatment comprising administering said salts or salt forms to a mammal in need thereof in therapeutically effective amounts for treating a disease of condition affecting said mammal, pharmaceutical formulations comprising said salts or salt forms. The compound 6,7-dihydro-5H-pyrrolo[1,2-c]imidazol-5-yl-3-fluoro-benzonitrile, its 4-(R)-enantiomer with Formula (III)

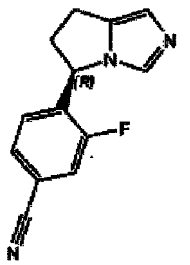

and its manufacture are described in WO 2007/024945 A1, among many other compounds disclosed therein. It is known to be active as inhibitor of aldosterone synthase and aromatase. For example, in a clinical proof-of-concept study in patients with primary aldosteronism efficiency of 4-(R)-(6,7-Dihydro-5H-pyrrolo[1,2-c]imidazol-5-yl)-3-fluoro-benzonitrile free base in inhibition of aldosterone synthase could be shown (see e.g. Amar L. et al., Hypertension 56(5), 831-838 Nov. 2010 (Epub 2010 Sep). No specific salts of the compounds mentioned in said patent application are disclosed. Compound (III) in the form of free base has a quite low melting point of about 111.5°C which constitutes a certain risk regarding physical stability of the compound during manufacture, storage and processing to pharmaceutical formulations. Thus there was a need to find specific processing conditions or specific forms of this compound that allow for stable processing. Surprisingly it was difficult to obtain salt of Compound (III). About 20 various different salt forming agents were evaluated with Compound (III), but only 3 salts were formed: Phosphate salt, nitrate salt and hydrochloride salt. Other salt forming agents did not produce solid material at all or not in any useful quantity or quality. The phosphate salt shows the most superior properties. It can be isolated and manufactured better and thus is advantageous over the free base compound, with additional properties such as a higher melting point over the free base and other salts, lower hygroscopicity and improved aqueous solubility. The nitrate salt also shows comparable advantages. The invention, in a first aspect, relates to a phosphate salt of Compound (III), especially in crystalline forms, particularly in crystalline Form A as described below. The term "phosphate salt" as used in the present application, refers to the acid addition salt of Compound (III) with phosphoric acid. More particularly, it refers to the dihydrogen-phosphate of Compound (III) protonated once, that is, wherein Compound (III) is protonated once and thus each molecule carries a single positive charge, while the counterion is $H_2P0_4^-$). The second aspect of the invention relates to a nitrate salt of Compound (III), especially in crystalline forms. In further aspects the invention relates to the salts or salt forms mentioned herein in the manufacture of medicaments for the treatment of diseases and conditions, to said salts or salt forms (especially in the form of pharmaceutical compositions) for use in the treatment of diseases and conditions, to methods of treatment comprising

administering said salts or salt forms to a mammal in need thereof in therapeutically effective amounts for treating a disease of condition affecting said mammal, pharmaceutical formulations comprising said salts or salt forms. Thus, in one further aspect the invention relates to a pharmaceutical composition that includes (an especially prophylactically or therapeutically effective amount of) a phosphate salt of Compound (III) and one or more pharmaceutically acceptable excipients, in particular for use in the treatment of conditions or diseases described herein, especially conditions or diseases mediated by aldosterone synthase or aromatase. The pharmaceutical compositions of this aspect of the invention may be formulated, e.g., for oral administration. In accordance with yet another aspect, the invention provides a process for making a nitrate salt or a phosphate salt, especially in crystalline form, respectively, of Compound (III), the process comprising: (a) providing a solution of Compound (III) in either a protic or an aprotic polar solvent, especially in an aqueous- alcoholic or alcoholic solution (alcoholic especially referring to hydroxy-$C_{1-7}$alkanes) (where said solution may have a temperature in the range e.g. from 0°C to the boiling point of the solution, e.g. up to 90°C, e.g. in the range from 10 to 50°C); (b) adding nitric acid or phosphoric acid; and (c) isolating the formed crystalline form of a nitrate salt or crystalline form of a phosphate salt ({drug3a}) of Compound (III); where if desired one obtainable (then educt) salt can be converted into the other salt by addition of the corresponding acid contributing the other anion to a solution of the educt salt. Variants of this process are provided and included as embodiments of the invention, e.g. in which seed crystals of the respective nitrate salt or phosphate salt of Compound (III) are added, the solvent is at least partially removed (e.g. by evaporation), the temperature is lowered to improve crystallization or on which anti-solvents are added to improve precipitation, or any combinations of such process measures. This process may also be a step (e.g. the final purification step) in the manufacture of the respective salt, e.g. at the end of the process described for the manufacture of the free base in WO 2007/024945 A1, and the corresponding general method of manufacture is incorporated by reference herein and applied specifically to the preparation of the compound Compound (III). Forms B, C, D, E, F, G and H of the phosphate salt may be obtained e.g. as described in the Examples, where the temperature is room temperature (23°C) ± 10°C and the solvents may be replaced by corresponding solvents.

In one first particular embodiment, the invention relates to a phosphate salt of Compound (III), in particular with a molar ratio of Compound (III) to phosphate of about 1 : 1, in particular in crystalline form.

In one special embodiment, the invention relates to a crystalline phosphate salt ({drug3a}) of Compound (III) having a melting point that is at least 50°C, at least 55°C or at least 85°C higher than that of the free base. In one embodiment, the phosphate salt ({drug3a}) of Compound (III) has a melting temperature of at least 170°C or at least 197°C. In one embodiment, the phosphate salt ({drug3a}) of Compound (III) has a melting temperature between 205°C to 214°C, between 206°C to 213°C, between 208°C to 213°C, between 209°C to 212°C or between 209°C to 211 °C. The melting temperatures herein, if not described otherwise, are obtained using thermogravimetry/differential thermal analysis (TG/DTA). TG/DTA is determined using a Seiko EXSTAR TG/DTA 6000, temperature range room temperature to 250°C, scan rate 10°C/min, nitrogen flow 100 ml/min.

In another special embodiment, the invention relates to "Form A" ({drug3b}) of the crystalline phosphate salt of Compound (III), especially with an XRPD showing at least one, more preferably two, three, four, five, six, seven, eight, nine, ten, 11, 12, 13, 14, 15, 16 or all of the following peaks, given as angle of refraction 2-theta (Θ) values, obtainable as described in the Examples, where each peak may vary by ± 1 or ± 0.5, in particular ± 0.2 degrees: <u>6.0</u>, 10.0, 12.1, <u>12.9,</u> 14.0, 14.5, <u>15.5</u>, <u>16.0, 16.3, 17.5,</u> 18.2, 18.4, <u>19.7, 20.4, 22.1, 24.3, 29.2,</u> with the underlined peaks defining a preferred embodiment and all peaks defining a more preferred embodiment. In one embodiment, the "Form A" ({drug3b}) of the crystalline phosphate salt of Compound (III) has a melting temperature of 210±5°C, 210±2°C, 210±1°C or 210±0.5°C or 210.2°C. In one embodiment, the invention relates to Form A ({drug3b}) with an XRPD showing at least one, more preferably two, three, four, five, six, seven, eight or all of the following peaks, given as angle of refraction 2-theta (Θ) values, obtainable as described in the Examples, where each peak may vary by ± 1 or ± 0.5, in particular ± 0.2 degrees: 6.0, 12.9, 15.5, 16.0, 16.3, 19.7,20.4,22.1, 24.3,29.2. In one embodiment, the two largest peaks of Form A ({drug3b}) in the XRPD diagram have a relative intensity of 1 to 0.5 to 0.7, especially of 1 to 0.55 to 0.65, more especially of 0.57 to 0.61, e.g. of 1 to 0.59 (obtainable by integration of each of the peaks in the XRPD diagrams). In a particular embodiment the larger peak is at a 2-theta (Θ) value of 6.0 ±1 or ±0.5, in particular ± 0.2 degrees and the smaller peak at a 2-theta (Θ) value of 19.7 ±1 or ± 0.5, in particular ± 0.2 degrees, respectively. Another embodiment relates to Form A ({drug3b}) with an XRPD showing at least one or all of the following peaks, given as angle of refraction 2-theta (Θ) values, obtainable as described in the Examples, where each peak may vary by ± 1 or ±0.5, in particular ± 0.2 degrees: 12.9 <u>16.3</u> and <u>20.4</u> degrees. Preferred is the phosphate salt showing an XRPD as shown in Figure 1-A of WO2013/109514. A specific embodiment of the invention relates to "Form A" ({drug3b}) of the crystalline phosphate salt of Compound (III), having a melting point between 209 and 212°C.

In one embodiment, the invention relates to a "Form B" ({drug3c}) of crystalline phosphate salt of Compound (III) with an XRPD showing at least one, more preferably two, three, four, five, six, seven, eight, nine, ten, 11, 12, 13, 14, 15, 16, 17, 18 or all of the following peaks, given as angle of refraction 2-theta (Θ) values, obtainable as described in the Examples, where each peak may vary by ± 1 or ±0.5, in particular ± 0.2 degrees: 6.0, 9.8, <u>11.8</u>, 12.7, 12.9, 14.0, 14.3, <u>15.2</u>, 16.0, 16.3, 17.5, 18.0, 18.4, <u>19.3</u>, 19.7, 20.1, 22.1, 24.3, 29.2, with the underlined peaks defining a preferred

embodiment, all peaks defining a more preferred embodiment. More preferred is the crystalline phosphate salt Form B ({drug3c}) showing an XRPD as shown in Figure 1-B of WO2013/109514. An embodiment of the invention relates to "Form B" ({drug3c}) of the crystalline phosphate salt of Compound (III), having a melting point of 209±5°C, for example between 207 and 211 °C, e.g. of 209.0°C. In one embodiment, the invention relates to a "Form C" ({drug3d}) of crystalline phosphate salt of Compound (III) with an XRPD showing at least one, more preferably two, three, four, five, six, seven, eight, nine, ten, 11, 12, 13, 14, 15, 16, 17 or all of the following peaks, given as angle of refraction 2-theta (Θ) values, obtainable as described in the Examples, where each peak may vary by ± 1 or ±0.5, in particular ± 0.2 degrees: 6.0, 10.0, 11.9, 12.9, 14.0, 14.5, 15.5, 16.0, 16.3, 17.8, 18.2, 18.4, 19.3, 19.7, 20.1, 22.1, 24.3, 29.2, with the underlined peaks defining a preferred embodiment, all peaks defining a more preferred embodiment.. More preferred is the crystalline phosphate salt Form C ({drug3d}) showing an XRPD as shown in Figure 1-C of WO2013/109514. An embodiment of the invention relates to "Form C" ({drug3d}) of the crystalline phosphate salt of Compound (III), having a melting point of 191 ±5°C, for example between 189 and 193°C, e.g. of 190.7°C

In one embodiment, the invention relates to a "Form D" ({drug3e}) of crystalline phosphate salt of Compound (III), with an XRPD showing at least one, more preferably two, three, four, five, six, seven, eight, nine, ten, 11, 12, 13, 14, 15, 16, 17 or all of the following peaks/given as angle of refraction 2-theta (Θ) values, obtainable as described in the Examples, where each peak may vary by ± 1 or ±0.5, in particular ± 0.2 degrees: 6.2, 10.0, 12.4, 12.9, 13.3, 14.8, 15.5, 16.0, 16.6, 17.5, 18.2, 18.4, 19.3, 19.7, 20.2, 22.1, 24.3, 29.2, with the underlined peaks defining a preferred embodiment, all peaks defining a more preferred embodiment.. More preferred is the crystalline phosphate salt Form D ({drug3e}) showing an XRPD as shown in Figure 1-D of WO2013/109514. An embodiment of the invention relates to "Form D" ({drug3e}) of the crystalline phosphate salt of Compound (III), having a melting point of 211±5°C, for example between 208 and 213°C, e.g. of 210.8°C.

In one embodiment, the invention relates to a "Form E" ({drug3f}) of crystalline phosphate salt of Compound (III), with an XRPD showing at least one, more preferably two, three, four, five, six, seven, eight, nine, ten, 11, 12, 13, 14, 15, 16, 17 or all of the following peaks, given as angle of refraction 2-theta (Θ) values, obtainable as described in the Examples, where each peak may vary by ± 1 or ±0.5, in particular ± 0.2 degrees: 6.4, 10.3, 12.1, 12.9, 13.3, 14.5, 15.2, 16.0, 16.8, 17.5, 18.2, 18.4, 19.3, 19.7, 20.1, 22.1, 24.3, 29.2, with the underlined peaks defining a preferred embodiment, all peaks defining a more preferred embodiment.. More preferred is the crystalline phosphate salt Form E ({drug3f}) showing an XRPD as shown in Figure 1-E of WO2013/109514. An embodiment of the invention relates to "Form E" ({drug3f}) of the crystalline phosphate salt of Compound (III), having a melting point of 214±5°C, for example between 211 and 216°C, e.g. of 213.6°C

In one embodiment, the invention relates to a "Form F" ({drug3g}) of crystalline phosphate salt of Compound (III), with an XRPD showing at least one, more preferably two, three, four, five, six, seven, eight, nine, ten, 11, 12, 13, 14, 15, 16, 17 or all of the following peaks, given as angle of refraction 2-theta (Θ) values, obtainable as described in the Examples, where each peak may vary by ± 1 or ±0.5, in particular ± 0.2 degrees: 6.0, 10.0, 12.1, 12.9, 13.8. 14.0, 15.5, 16.0, 16.6, 17.5, 18.2, 18.4, 19.1, 19.7, 20.1, 22.1, 24.3, 29.2, with the underlined peaks defining a preferred embodiment, all peaks defining a more preferred embodiment.. More preferred is the crystalline phosphate salt Form F ({drug3g}) showing an XRPD as shown in Figure 1-F of WO2013/109514. An embodiment of the invention relates to "Form F" ({drug3g}) of the crystalline phosphate salt of Compound (III), having a melting point of 201 ±5°C, for example between 199 and 203°C, e.g. of 201.1°C

In one embodiment, the invention relates to a "Form G" ({drug3h}) of crystalline phosphate salt of Compound (III), with an XRPD showing at least one, more preferably two, three, four, five, six, seven, eight, nine, ten, 11, 12, 13, 14, 15, 16, or all of the following peaks, given as angle of refraction 2-theta (Θ) values, obtainable as described in the Examples, where each peak may vary by ± 1 or +0.5, in particular ± 0.2 degrees: 6.0, 12.1, 13.1, 14.0, 14.5, 15.5, 15.8, 16.3, 17.5, 18.2, 18.4, 19.3, 19.7, 20.1, 22.1, 24.3,29.2, with the underlined peaks defining a preferred embodiment, all peaks defining a more preferred embodiment.. More preferred is the crystalline phosphate salt Form G ({drug3h}) showing an XRPD as shown in Figure 1-G of WO2013/109514. An embodiment of the invention relates to "Form G" ({drug3h}) of the crystalline phosphate salt of Compound (III), having a melting point of 180±5°C, for example between 177 and 183°C, e.g. of 179.8°C

In one embodiment, the invention relates to a "Form H" ({drug3i}) of crystalline phosphate salt of Compound (III), with an XRPD showing at least one, more preferably two, three, four, five, six, seven, eight, nine, ten, 11, 12, 13, 14, 15, 16, or all of the following peaks, given as angle of refraction 2-theta (Θ) values, obtainable as described in the Examples, where each peak may vary by ± 1 or ±0.5, in particular ± 0.2 degrees: 6.0, 10.2, 12.1, 13.1, 14.0, 14.5, 15.5, 16.2, 16.5, 17.5, 18.2, 18.4, 19.3, 19.7, 20.1, 22.2, 24.3, 29.5, with the underlined peaks defining a preferred embodiment, all peaks defining a more preferred embodiment. More preferred is the crystalline phosphate salt Form H ({drug3i}) showing an XRPD as shown in Figure 1-H of WO2013/109514. An embodiment of the invention relates to "Form H" ({drug3i}) of the crystalline phosphate salt of Compound (III), having a melting point of 208±5°C, for example between 206 and 210°C, e.g. of 208.0°C.

In one embodiment, the invention relates to a nitrate salt of Compound (III), in particular with a molar ratio of

Compound (III) to nitrate of about 1:1, in particular in crystalline Form ({drug3j}). In one special embodiment, the invention relates to a crystalline nitrate salt ({drug3j}) of Compound (III) having a melting point that is at least 40°C higher than that of the free base, especially (using TG/DTA as described above) between 160 and 163°C. In another special embodiment, the invention relates to a crystalline nitrate salt ({drug3j}) of Compound (III), with an XRPD showing at least one, more preferably two, three, four, five, six, seven, eight, nine, ten, 11, 12, 13, 14, 15, 16, 17 or all of the following peaks, given as angle of refraction 2-theta (Θ) values, obtainable as described in the Examples, where each peak may vary by ± 1 or ±0.5, in particular ± 0.2 degrees: 7.4, 10.6, 12.5, 15.0, 15.7,17.5, 18.0, 18.7, 20.4, 21.2, 22.8, 24.6, 26.5, 28.2, 28.8, 29.9, 31.0, 31.5, with the underlined peaks defining a preferred embodiment, all peaks defining a more preferred embodiment. Preferred is the nitrate salt showing an XRPD as shown in Figure 2 of WO2013/109514.

A specific embodiment of the invention relates to a crystalline nitrate salt ({drug3j}) of Compound (III) showing the XRPD characteristics above and a melting point between 160 and 163°C using TG/DTA. The compound 6,7-dihydro-5H-pyrrolo[1,2-c]imidazol-5-yl-3-fluoro-benzonitrile as free base) and especially its enantiomers, especially 4-(R)-(6,7-dihydro-5H-pyrrolo[1,2-c]imi-dazol-5-yl)-3-fluoro-benzonitrile, can be manufactured as described in published inter-national patent application WO 2007/024945 which is incorporated by reference herein (see especially Table 2, and Chiral Resolution).

"About" where used means especially ± 10%, ± 5% or ± 3% (referring to the given numeric value, respectively), if not indicated otherwise. In each of the invention embodiments, "about" can be deleted. "Prophylactically or therapeutically effective amount" means the amount of a compound that, when administered for treating or preventing a disease or disorder mentioned above or below, is sufficient to effect such treatment or prevention for the disease or disorder, prophylactic especially refers to the prevention of the onset or recurrence or ameliorating the onset or recurrence of such disease or disorder, therapeutic especially refers to the amelioration or complete suppression of one or more symptoms up to the cure of such disease or disorder. The "prophylactically or therapeutically effective amount" will vary depending on the salt(s) or salt form(s) used, the disease or disorder and its severity and the age, weight, etc., of the patient to be treated. The term "pharmaceutical composition" is intended to encompass a product comprising the active ingredient(s), optionally at least one pharmaceutically acceptable excipients. The pharmaceutical compositions of the present invention encompass any composition made by admixing the active ingredient, additional active ingredient(s) and pharmaceutically acceptable excipients. The term "pharmaceutical composition" is also intended to comprise a combination product, comprising a salt or salt form according to any one of claims 1 to 12, in combination with one or more other therapeutic agents (pharmaceutically active compounds) and preferably at least one pharmaceutically acceptable excipient. Other therapeutic agents include at least one or two or more selected from the following groups: (i) angiotensin II receptor antagonist or a pharmaceutically acceptable salt thereof, (ii) HMG-Co-A reductase inhibitor or a pharmaceutically acceptable salt thereof, (iii) angiotensin converting enzyme (ACE) Inhibitor or a pharmaceutically acceptable salt thereof, (iv) calcium channel blocker (CCB) or a pharmaceutically acceptable salt thereof, (v) dual angiotensin converting enzyme/neutral endopeptidase (ACE/NEP) inhibitor or a pharmaceutically acceptable salt thereof, (vi) endothelin antagonist or a pharmaceutically acceptable salt thereof, (vii) renin inhibitor or a pharmaceutically acceptable salt thereof, (viii) diuretic or a pharmaceutically acceptable salt thereof, (ix) an ApoA-1 mimic; (x) an anti-diabetic agent; (xi) an obesity-reducing agent; (xii) an aldosterone receptor blocker; (xiii) an endothelin receptor blocker; (xiv) a CETP inhibitor; (xv) an inhibitor of Na-K-ATPase membrane pump; (xvi) a beta-adrenergic receptor blocker or an alpha-adrenergic receptor blocker; (xvii) a neutral endopeptidase (NEP) inhibitor; and (xviii) an inotropic agent. Such compounds are e.g. defined in WO 2007/024945 A1 and are included here by reference. Also where not specifically mentioned, the therapeutic agent(s) may be in the free (non-salt) form or in the form of a pharmaceutically acceptable salt, respectively. A compound of the present invention may be administered either simultaneously, before or after the other therapeutic agent (active ingredient), either separately by the same or different route of administration or together in the same pharmaceutical formulation. Thus the combination product may comprise the salt or salt form according to the invention in one, one or more other therapeutic agents in a separate formulation, but in the form of a kit of parts for the simultaneous or chronically staggered administration, or in one fixed combination. Thus, the combinations as described above can be administered to a subject via simul-taneous, separate or sequential administration (use). Simultaneous administration (use) can take place in the form of one fixed combination with two or three or more active ingredients, or by simultaneously administering two or three or more compounds that are formulated independently. Sequential administration(use) preferably means administration of one (or more) compounds or active ingredients of a combination at one time point, other compounds or active ingredients at a different time point, that is, in a chronically staggered manner, preferably such that the combination shows more efficiency than the single compounds administered independently (especially showing synergism). Separate administration (use) preferably means administration of the compounds or active ingredients of the combination independently of each other at different time points, preferably meaning that two, or three or more compounds are administered such that no overlap of measurable blood levels of both compounds are present in an overlapping manner (at the same time). Also combinations of two or three or more of sequential, separate and simultaneous administrations are possible, preferably such that the combination compound-drugs show a joint therapeutic effect that exceeds the effect found when the combination compound-drugs are used independently at time intervals so large

that no mutual effect on their therapeutic efficiency can be found, a synergistic effect being especially preferred. Alternatively, the pharmaceutical compositions contain a therapeutically effective amount of a salt or salt form of Compound (III) of the invention as defined above or below, either alone or in a combination with one or more therapeutic agents, e.g., each at an effective therapeutic dose as reported in the art, selected from the group consisting of an anties-trogen; an anti-androgen; a gonadorelin agonist; a topoisomerase I inhibitor; a topoiso-merase II inhibitor; a microtubule active agent; an alkylating agent; an anti-neoplastic anti-metabolite; a platin compound; a compound targeting/decreasing a protein or lipid kinase activity or a protein or lipid phosphatase activity, a anti-angiogenic compound; a compound which induces cell differentiation processes; monoclonal antibodies; a cyclo-oxygenase inhibitor; a bisphosphonate; a heparanase inhibitor; a biological response modifier; an inhibitor of Ras oncogenic isoforms; a telomerase inhibitor; a protease inhibitor, a matrix metalloproteinase inhibitor, a methionine aminopeptidase inhibitor; a proteasome inhibitor; agents which target, decrease or inhibit the activity of Flt-3; an HSP90 inhibitor; antiproliferative antibodies; an HDAC inhibitor; a compound which targets, decreases or inhibits the activity/function of serine/theronine mTOR kinase; a somatostatin receptor antagonist; an anti-leukemic compound; tumor cell damaging approaches; an EDG binder; a ribonucleotide reductase inhibitor; an S-adenosylmethi-onine decarboxylase inhibitor; a monoclonal antibody of VEGF or VEGFR; photodynamic therapy; an Angiostatic steroid; an implant containing corticosteroids; an AT1 receptor antagonist; and an ACE inhibitor.

Additionally, the present invention provides: - a pharmaceutical composition or combination of the present invention for use as a medicament; - the use of a pharmaceutical composition or combination of the present invention for the delay of progression and/or treatment of a disorder or disease mediated by aldo-sterone synthase, or responsive to inhibition of aldosterone synthase, or characterized by abnormal activity or expression of aldosterone synthase. - the use of a pharmaceutical composition or combination of the present invention for the delay of progression and/or treatment of a disorder or disease mediated by aromatase, or responsive to inhibition of aromatase, or characterized by abnormal activity or expression of aromatase. - the use of a pharmaceutical composition or combination of the present invention for the delay of progression and/or treatment of a disorder or disease selected from hypokalemia, hypertension, congestive heart failure, atrial fibrillation, renal failure, in particular, chronic renal failure, restenosis, sleep apnoea, atherosclerosis, syndrome X, obe-sity, nephropathy, post-myocardial infarction, coronary heart diseases, increased formation of collagen, fibrosis such as cardiac or myocardiac fibrosis and remodeling following hypertension and endothelial dysfunction.

The salt(s) or salt form(s) of the present invention are useful as aldosterone synthase inhibitors (see e.g. WO 2007/024945 incorporated by reference herein regarding the diseases and disorders related to aldosterone synthase). In particular, the salt(s) or salt form(s) of the present invention as aldosterone synthase inhibitors are useful for treatment of a disorder or disease characterized by abnormal aldosterone synthase activity. Preferably, the salt(s) or salt form(s) of the present invention are also useful for treatment of a disorder or disease selected from hypokalemia, hypertension, congestive heart failure, atrial fibrillation, renal failure, in particular, chronic renal failure, restenosis, sleep apnoea, atherosclerosis, syndrome X, obesity, nephropathy, post-myocardial infarction, coronary heart diseases, inflammation, increased formation of collagen, fibrosis such as cardiac or myocardiac fibrosis and remodeling following hypertension and endothelial dysfunction. The disorder or disease to be treated with a salt or salt form according to the inventions is especially selected from hypertension (essential or resistant), primary aldosteronism congestive heart failure and acute heart failure. Further diseases or disorders of specific interest are selected from the group consisting of heart failure, cachexia, acute coronary syndrome, chronic stress syndrome, Cushing’s disease, Cushing’s syndrome, metabolic syndrome or hypercortisolemia. In a very preferred embodiment the disease is Cushing’s disease. "Protic or an aprotic solvents" may be selected from aqueous or non-aqueous solvents or solvent mixtures, e.g. comprising one or more selected from water, $C_{1-7}$alkanols, $C_{1-8}$ketones, $C_{1-7}$alkanediols, $C_{1-7}$alkylnitriles, $C_{17}$alkyl-$C_{2-7}$alkanoates, with methanol, acetone, propylene glykol, ethyl acetate, or especially ethanol being especially preferred. "Anti-solvent" is a solvent which when added to an existing solution of a substance reduces the solubility of the substance. In the case of the present salts, anti-solvents are especially less polar (more hydrophilic) solvents than those used for dissolving Compound A before the addition of nitric acid or phosphoric acid, e.g. organic solvents that are at least to a certain extent (e.g. at least up to 10 vol %) miscible with water. Throughout this specification, where the plural forms "salts" or "salt foms" are used, this also includes a single salt or a single salt form.

The Figures of WO2013/109514show the XRDP of the following salts and salt forms of Compound (III): Figure 1-A of WO2013/109514shows the X-ray powder diffraction pattern of the initial phosphate salt Form A ({drug3b}). Figure 1-B of WO2013/109514shows the X-ray powder diffraction pattern of phosphate salt Form B ({drug3c}). Figure 1-C of WO2013/109514shows the X-ray powder diffraction pattern of phosphate salt Form C ({drug3d}). Figure 1-D of WO2013/109514shows the X-ray powder diffraction pattern of phosphate salt Form D ({drug3e}). Figure 1-E of WO2013/109514shows the X-ray powder diffraction pattern of phosphate salt Form E ({drug3f}). Figure 1-F of WO2013/109514shows the X-ray powder diffraction pattern of phosphate salt Form F ({drug3g}). Figure 1-G of WO2013/109514shows the X-ray powder diffraction pattern of phosphate salt Form G ({drug3h}). Figure 1-H of WO2013/109514shows the X-ray powder diffraction pattern of phosphate salt Form H ({drug3i}). Figure 2 of WO2013/109514shows the X-ray powder diffraction pattern of nitrate salt ({drug3j}). Figure 3 of WO2013/109514shows

the X-ray powder diffraction pattern of chloride salt.

The following Examples illustrate the invention without limiting its scope, though they also form specific embodiments of the invention. Abbreviations used: IPA isopropyl alcohol, RH relative humidity, XRDP X-ray powder diffraction pattern. If not mentioned otherwise, all reactions are carried out at room temperature (21 to 24°C, e.g. 23°C).

Example 1: Phosphate salt of 4-(R)-(6,7-dihydro-5H-pyrrolo[1.2-climidazol-5-yl)-3-fluoro-benzonitrile (Form A) ({drug3b}): 2 g of free base was dissolved in a 40 ml ethanol and 1 equivalent phosphoric acid was added over the course of several minutes. After addition, solids were collected by filtration. The solids were dried at 22°C under nitrogen flow. About 1.8g were collected. The molar ratio of phosphate to 4-(R)-(6,7-dihydro-5H-pyrrolo[1,2-c]imidazol-5-yl)-3-fluoro-benzonitrile in the obtained salt is 1:1. In water, the phosphate salt was soluble at the 0.1% target concentration and stable for 2 days at 50°C. The free base remained largely insoluble. It converted from a free flo- wing solid into an oily material within a short time after contact with water, and remained as such for the 2 day period at 50°C. Hygroscopicity: Sorption/desorption isotherms were measured using the VT1100 humidity microbalance (VTI Corporation, Hialeah, FL, USA). Measurements were carried out at 25°C. Samples were dried under N2 flow at 25°C. The hygroscopicity of the phosphate salt was found to be only 0% water uptake at 5 % RH and 0.9 % water uptake at 75 % relative humidity. Determination of solubility: Excess solids were equilibrated in each solvent for over 24 hours at 25°C ± 0.1. Concentration in supernatant was measured by gravimetry for organic solvents and by HPLC from aqueous solvents and propylene glycol. The following results were obtained for the phosphate salt in comparison with the free base:

Table 1-1 Solubility profile (mg/ml)

| Solvent | Free base | Phosphate salt |
|---|---|---|
| pH 1 | > 50 | > 50 |
| pH 6.8 | 28.6 | > 50 |
| Water | 7.2 | > 50 |
| Ethanol | > 50 | 5.8 |
| Acetone | > 50 | 1.5 |
| Propylene glycol | 34.4 | 1.5 |
| Ethyl Acetate | > 50 | n.d. |

This shows that the solubility of the phosphate salt is comparably low in non-aqueous solvents which are thus anti-solvents for the salt, thus making it possible to achieve good precipitation and thus good yields and good purity. On the other hand, the solubility in water is better than that for the free base which is advantageous for providing oral or parenteral formulations. The melting point was determined by TG/DTA as described above and was determined to be 210.2°C. Determination of the XRDP: Parameters and device used: XRPD-method, Instrument D8; Bruker (Karlsruhe, Germany)Irradiation CuKa (40 kV, 40 mA), Scan time 4 minutes (2 minutes per frame), Scan range 3° - 40° (2 theta value). The XRDP obtained for this first phosphate form (Form A) ({drug3b}) is shown in Figure 1-A. The following table shows the corresponding angle of refraction 2-Theta values (in degrees [°]) for the important peaks:

Table 1-3, Form A

| Measured 2-Theta (degrees) | Peak |
|---|---|
| 6.0 | 1 |
| 10.0 | 2 |
| 12.1 | 3 |
| 12.9 | 4 |
| 14.0 | 5 |
| 14.5 | 6 |
| 15.5 | 7 |
| 16.0 | 8 |
| 16.3 | 9 |
| 17.5 | 10 |
| 18.2 | 11 |

(continued)

| Measured 2-Theta (degrees) | Peak |
|---|---|
| 18.4 | 12 |
| 19.7 | 13 |
| 20.4 | 14 |
| 22.1 | 15 |
| 24.3 | 16 |

Example 2: Nitrate salt of 4-(R)-(6,7-dihydro-5H-pyrrolori,2-climidazol-5-yl)-3-fluoro-benzonitrile ({drug3j}): 2 g of 4-(R)-6,7-dihydro-5H-pyrrolo[1,2-c]imidazol-5-yl-3-fluoro-benzonitrile free base was dissolved in 40 ml ethanol and 1 equivalent nitric acid (same molar amounts as mo-lar amount of free base) was added over the course of several minutes. After addition, solids were collected by filtration. The sample was dried at 21°C to 23°C by dry nitrogen flow. The amount recovered was 1.7 g. The resulting 4-(R)-(6,7-dihydro-5H-pyrrolo[1,2-c]imidazol-5-yl)-3-fluoro-benzonitrile nitrate salt has a 1 :1 molar ratio of base to chloride. It shows the XRPD represented in Figure 2 of WO2013/109514. The hygroscopicity (measured under the conditions mentioned in Example 1) of the nitrate salt of 4-(R)-(6,7-dihydro-5H-pyrrolo[1,2-c]imidazol-5-yl)-3-fluoro-benzonitrile was found to be 0 % water uptake at 5 % RH and 0.2 % at 75 % RH. In water, the nitrate salt was soluble at the 0.1 % target concentration and stable for 2 days at 50°C. The free base remained largely insoluble It converted from a free flowing solid into an oily material within a short time after contact with water, and remained as such for the 2 day period at 50°C. Solubility was determined as shown for Example 1 and the following results were obtained:

Table 2-2 Solubility profile (mg/ml):

| Solvent | Free base | nitrate salt |
|---|---|---|
| pH 1 | > 50 | > 50 |
| pH 6.8 | 28.6 | 21.8 |
| Water | 7.2 | 38.2 |
| Ethanol | > 50 | 4.2 |
| Acetone | > 50 | 2.8 |
| Propylene glycol | 34.4 | 13.0 |
| Ethyl Acetate | > 50 | 1.5 |

Using the TG/DCA method described above, the melting point of the nitrate was determined to be between 160 and 163°C. The following angle of refraction peaks (2 Theta in degrees) are found in the XRPD (using the method described in Example 1), also providing their approximate intensity:

Table 2-2:

| No. | 2 Theta | Intensity | No. | 2-Theta | Intensity |
|---|---|---|---|---|---|
| 1 | 7.4 | 93 | 10 | 21.2 | 54 |
| 2 | 10.6 | 41 | 11 | 22.8 | 45 |
| 3 | 12.5 | 23 | 12 | 24.6 | 51 |
| 4 | 15.0 | 18 | 13 | 26.5 | 92 |
| 5 | 15.7 | 35 | 14 | 28.2 | 56 |
| 6 | 17.5 | 42 | 15 | 28.8 | 33 |
| 7 | 18.0 | 64 | 16 | 29.9 | 55 |
| 8 | 18.7 | 78 | 17 | 31.0 | 23 |
| 9 | 20.4 | 137 | 18 | 31.5 | 29 |

Example 3: Further Forms B to H of 4-(R)-(6,7-dihvdro-5H-pyrrolon,2-climidazol-5-yl)-3-fluoro-benzonitrile phosphate salt The following salt modifications B to H were obtained as described below: *Preparation of phosphate salt Modification B ({drug3c}):* Form A ({drug3b}) of 4-(R)-(6,7-dihydro-5H-pyrrolo[1,2-c]imidazol-5-yl)-3-fluoro-benzonitrile

phosphate salt was equilibrated in IPA for 72 hours at 50°C. Sample was collected by filtration and allowed to air dry. A melting temperature of Form B ({drug3c}) of 209.0°C was determined by TG/DTA. *Preparation of phosphate salt Modification C (fdrug3d}):* 30mg of 4-(R)-(6,7-dihydro-5H-pyrrolo[1,2-c]imidazol-5-yl)-3-fluoro-benzonitrile phosphate salt was dissolved in water at high concentration then chilled to 5°C. Ethanol was added to precipitate Form C ({drug3d}). Sample was collected by filtration and allowed to air dry. A melting temperature of Form C ({drug3d})of 206.4°C was determined by TG/DTA. *Preparation of phosphate salt Modification D ({drug3e})*: 30 mg of 4-(R)-(6,7-dihydro-5H-pyrrolo[1,2-c]imidazol-5-yl)-3-fluoro-benzonitrile phosphate salt was dissolved in methanol at 50°C and Form D({drug3e}) is precipitated by the addition of acetone. Sample was collected by filtration and allowed to air dry. A melting temperature of Form D ({drug3e}) of 210.8°C was determined by TG/DTA. *Preparation of phosphate salt Modification E ({drug3f})*: 20 mg of 4-(R)-(6,7-dihydro-5H-pyrrolo[1,2-c]imidazol-5-yl)-3-fluoro-benzonitrile free base was dissolved in 2-propanol/water solution (4:1) at room temperature. An equal molar amount of phosphoric acid was added to yield Form E ({drug3f}). Sample was collected by filtration and allowed to air dry. A melting temperature of Form E ({drug3f}) of 199.2°C was determined by TG/DTA. *Preparation of phosphate salt Modification F ({drug3g})*: 20 mg of 4-(R)-(6,7-dihydro-5H-pyrrolo[1,2-c]imidazol-5-yl)-3-fluoro-benzonitrile free base was dissolved in methyl i-butyl ketone at (4:1) at 50°C. An equal molar amount of phosphoric acid was added to yield Form F ({drug3g}). Sample was collected by filtration and allowed to air dry. A melting temperature of Form F ({drug3g}) of 201°C was determined by TG/DTA. *Preparation ofphosphate salt Modification G ({drug3h})*: 20 mg 4-(R)-(6,7-dihydro-5H-pyrrolo[1,2-c]imidazol-5-yl)-3-fluoro-benzonitrile free base was dissolved in methanol (+20% water) at 5°C. An equal molar amount of phosphoric acid was added to yield Form G ({drug3h}). Sample was collected by filtration and allowed to air dry. A melting temperature of Form G ({drug3h}) of 170.8°C was determined by TG/DTA. *Preparation of phosphate salt Modification H ({drug3i})*: 20 mg 4-(R)-(6,7-dihydro-5H-pyrrolo[1,2-c]imidazol-5-yl)-3-fluoro-benzonitrile free base was dissolved in ethanol (+20% water) at 50°C. An equal molar amount of phosphoric acid was added to yield Form H ({drug3i}) after the solution is chilled to room temperature. Sample was collected by filtration and allowed to air dry. A melting temperature of Form H({drug3i}) of 208.0°C was determined by TG/DTA.

The following Table 3-1 shows the angle of refraction 2-Theta values (in degrees) for the XRDPs of Figure 1-A to 1-H of WO2013/109514 for Forms A to G in comparison to those for Form A ({drug3b}), with characteristic peaks for each form in comparison to Form A ({drug3b}) being underlined:

Table 3-1

| A<br>**Measured 2-Theta** | **Relative intensity (1%)** | B | F | C | D | E | G | H |
|---|---|---|---|---|---|---|---|---|
| 6.0 | 100 | 6.0 | 6.0 | 6.0 | 6.2 | **6.4** | 6.0 | 6.0 |
| 10.0 | 11 | 9.8 | 10.0 | 10.0 | 10.0 | 10.3 | | 10.2 |
| 12.1 | 14 | **11.8** | 12.1 | **11.9** | **12.4** | 12.1 | 12.1 | 12.1 |
| | | 12.7 | | | | | | |
| 12.9 | 21 | 12.9 | 12.9 | 12.9 | 12.9 | 12.9 | 13.1 | 13.1 |
| | | | **13.8** | | **13.3** | | | |
| 14.0 | 10 | 14.0 | 14.0 | 14.0 | | 13.3 | 14.0 | 14.0 |
| 14.5 | 12 | 14.3 | | 14.5 | **14.8** | 14.5 | 14.5 | 14.5 |
| 15.5 | 18 | **15.2** | 15.5 | 15.5 | 15.5 | 15.2 | 15.5 | 15.5 |
| 16.0 | 17 | 16.0 | 16.0 | 16.0 | 16.0 | 16.0 | 15.8 | 16.2 |
| 16.3 | 20 | 16.3 | **16.6** | 16.3 | **16.6** | **16.8** | 16.3 | 16.5 |
| 17.5 | 10 | 17.5 | 17.5 | **17.8** | 17.5 | 17.5 | 17.5 | 17.5 |
| 18.2 | 13 | 18.0 | 18.2 | 18.2 | 18.2 | 18.2 | 18.2 | 18.2 |
| 18.4 | 12 | 18.4 | 18.4 | 18.4 | 18.4 | 18.4 | 18.4 | 18.4 |
| | | **19.3** | **19.1** | **19.3** | **19.3** | **19.3** | **19.3** | **19.3** |
| 19.7 | 59 | 19.7 | 19.7 | 19.7 | 19.7 | 19.7 | 19.7 | 19.7 |
| 20.4 | 20 | 20.1 | **20.1** | **20.1** | 20.2 | **20.1** | **20.1** | **20.1** |
| 22.1 | 44 | 22.1 | 22.1 | 22.1 | 22.1 | 22.1 | 22.1 | 22.2 |
| 24.3 | 26 | 24.3 | 24.3 | 24.3 | 24.3 | 24.3 | 24.3 | 24.3 |
| 29.2 | 23 | 29.2 | 29.2 | 29.2 | 29.2 | 29.2 | 29.2 | 29.5 |

Thus it can be shown that in addition to Form A ({drug3b}) (Example 1) further polymorphs (Forms) of Compound

(III) phosphate salt can be found.

Comparison Example: HCl salt of 4-(R)-(6,7-dihydro-5H-pyrrolof1,2-climidazol-5-yl)-3-fluoro-benzonitrile: 2 g of 4-(R)-(6,7-dihydro-5H-pyrrolo[1,2-c]imidazol-5-yl)-3-fluoro-benzonitrile free base was dissolved in 40 ml ethanol and 1 equivalent HCl (equal molar amount of HCl to the molar amount of the free base) was added over the course of several minutes. Since the system did not produce crystallization, the solvent was evaporated. The solids were suspended in methyl tertbutyl ether for 2 hours at room temperature. The solids were collected by filtration. The solids were dried at 22°C under nitrogen flow. About 1.6g were collected. The resulting 4-(R)-(6,7-dihydro-5H-pyrrolo[1,2-c]imidazol-5-yl)-3-fluoro-benzonitrile HCl salt has a 1:1 molar ratio of base to chloride. It showed the XRPD shown in Figure 3 of WO2013/109514. The following peaks (in 2 Theta) were found in the XRPD (using the method described in Example 1):

| No. | Position | Intensity | No. | Position | Intensity |
|---|---|---|---|---|---|
| 1 | 13.3 | 36 | 13 | 24.1 | 33 |
| 2 | 14.1 | 51 | 14 | 24.3 | 37 |
| 3 | 14.7 | 19 | 15 | 24.5 | 24 |
| 4 | 16.2 | 18 | 16 | 25.7 | 20 |
| 5 | 16.5 | 17 | 17 | 25.9 | 13 |
| 6 | 16.7 | 24 | 18 | 26.6 | 52 |
| 7 | 20.6 | 15 | 19 | 26.9 | 37 |
| 8 | 21.0 | 15 | 20 | 27.6 | 17 |
| 9 | 22.0 | 14 | 21 | 28.0 | 26 |
| 10 | 23.0 | 25 | 22 | 28.4 | 40 |
| 11 | 23.1 | 32 | 23 | 29.0 | 15 |
| 12 | 23.5 | 53 | 24 | 29.7 | 14 |

In contrast to both the phosphate and the nitrate salts, the HCl salt showed excessive hygroscopicity, as is shown in the following table: Moisture gain (%) comparison between Forms (r.h. = relative humidity):

| r.h.% | free base | Nitrate | Phosphate | HCl |
|---|---|---|---|---|
| 0 | 0.0 | 0.0 | 0.0 | 0.0 |
| 5 | 0.0 | 0.0 | 0.0 | 0.0 |
| 25 | 0.1 | 0.1 | 0.1 | 0.5 |
| 50 | 0.2 | 0.2 | 0.1 | 0.2 |
| 75 | 0.3 | 0.2 | 0.2 | 0.2 |
| 85 | 0.5 | 0.2 | 0.3 | 3.3 |
| 95 | 1.0 | 0.2 | 0.8 | 23.6 |

It will be apparent to those skilled in the art, that many modifications, both to materials, and methods, may be practiced with out departing from the purpose and interest of this invention.

SPECIFIC INORMATION CONCERNING ASPECT (IV) OF THE INVENTION

Aspect (iv) of the invention relates to an administration unit comprising solid form of 4-amino-2-(2,6-dioxopiperidine-3-yl)isoindoline-1,3-dione ({drug4}). The invention relates to a solid form of 4-amino-2-(2,6-dioxopiperidine-3-yl)isoindoline-1,3-dione, namely to solid form of 4-amino-2-(2,6-dioxopiperidine-3-yl)isoindoline-1,3-dione, which is useful for treating multiple myeloma, myeloproliferative disease, anemia, scleroderma, amyloidosis. Solid form of 4-amino-2-(2,6-dioxopiperidine-3-yl)isoindoline-1,3-dione is described in WO2013/126326, which is incorporated by reference. As used herein, solid form of 4-amino-2-(2,6-dioxopiperidine-3-yl)isoindoline-1,3-dione is a solid form of 4-amino-2-(2,6-dioxopiperidine-3-yl)isoindoline-1,3-dione characterized by / obtained preferred as described in WO2013/126326, which are repeated heienafter: Provided herein are solid forms comprising 4-amino-2-(2,6-dioxopiperidine-3-yl)isoindoline-1,3-dione. Pharmaceutical compositions comprising the solid forms and methods of use for treating, preventing, and managing various disorders are also provided herein. The identification and selection of a solid form of a pharmaceutical compound are complex, given that a change in solid form may affect a variety of physical and chemical properties, which may provide benefits or drawbacks in processing, formulation, stability, bioavailability, storage, handling (e.g., shipping),

among other important pharmaceutical characteristics. Useful pharmaceutical solids include crystalline solids and amorphous solids, depending on the product and its mode of admini tration. Amoiphous solids are characterized by a lack of long-range structural order, whereas crystalline solids are characterized by structural periodicity. The desired class of pharmaceutical solid depends upon the specific application; amorphous solids are sometimes selected on the basis of, e.g., an enhanced dissolution profile, while crystalline solids may be desirable for properties such as, e.g., physical or chemical stability (see, e.g., S. R. Vippagunta et at, Adv. Drug. Deliv. Rev., (2001) 48:3-26; L. Y-a, Adv. Drug. Deliv. Rev., (2001) 48:27-42). Whether crystalline or amorphous, solid forms of a pharmaceutical compound include single-component and multiple-component solids. Single-component solids consist essentially of the pharmaceutical compound or active ingredient in the absence of other compounds. Variety among single-component crystalline materials may potentially arise from the phenomenon of polymorphism, wherein multiple three-dimensional arrangements exist for a particular pharmaceutical compound (see, e.g., S. R. Byrn ei al, Solid State Chemistry of Drugs, (1999) SSCI, West Lafayette). The importance of discovering polymorphs was underscored by the case of Ritonavir™, an HIV protease inhibitor that was formulated as soft gelatin capsules. About two years after the product was launched, the unanticipated precipitation of a new, less soluble polymorph in the formulation necessitated the withdrawal of the product from the market until a more consistent formulation could be developed (see S. R. Chcmburkar et al. Org. Process Res. Dev., (2000) 4:413-417). Additional diversity among the potential solid forms of a pharmaceutical compound may arise from the possibility of multiple-component solids. Crystalline solids comprising two or more ionic species may be termed salts (see, e.g.. Handbook of Pharmaceutical Salts: Properties, Selection and Use, P. H. Stahl and C. G. Wermuth, Eds., (2002), Wiley, Weinheim). Additional types of multiple-component solids that may potentially offer other property improvements for a pharmaceutical compound or salt thereof include, e.g., hydrates, solvates, co-crystals and clathrates, among others (see, e.g., 8. R. Bym el al, Solid State Chemistry of Drugs, (1999) SSCI, West Lafayette). Moreover, multiple-component crystal forms may potentially be susceptible to polymorphism, wherein a given multiple-component composition may exist in more than one three-dimensional crystalline arrangement. The variety of possible solid forms creates potential di versity in physical and chemical properties for a given pharmaceutical compound. The discovery and selection of solid forms are of great importance in the development of an effective, stable and marketable pharmaceutical product.

Pomalidomide, which was previously referred to as CC-4047, and has a chemical name of 4-amino-2-(2,6-dioxopiperidine-3-yl)isoindoline-1,3-dione. Pomalidomide is a compound that inhibits, for example, LPS induced monocyte T Fa, IL-Iβ, IL-12, IL-6, MIP-1, MCP-1, GM-CSF, G-CSF, and COX-2 production, and may be used in treating various disorders. See, e.g., U.S. Patent Nos. 5,635,517, 6,316,471, 6,476,052, 7,393,863, 7,629,360, and 7,863,297; and U.S. Patent Application Publication Nos. 2.005/0143420, 2006/0166932, 2006/0188475, 2007/0048327, 2007/0066512, 2007/0155791, 2008/0051431, 2008/0317708, 2009/0087407, 2009/0088410, 2009/01317385, 2009/0148853, 2009/0232776, 2009/0232796, 2010/0098657, 2010/0099711, and 2011 /0184025, the entireties of which are incorporated herein by reference. The compound is also known to co-stimulate the activation of T-cells. Pomalidomide has direct anti-myeloma tumoricidal activity, immunomodulatory activities and inhibits stromal cell support for multiple myeloma tumor ceil growth. Specifically, pomalidomide inhibits proliferation and induces apoptosis of hematopoietic tumor cells. Id. Additionally, pomalidomide inhibits the proliferation of lenaiidomide-resistant multiple myeloma cell lines and synergizes with dexamethasone in both lenalidomide-sensitive and lenalidomide-resistant cell lines to induce tumor cell apoptosis. Pomalidomide enhances T cell- and natural killer (NK) cell-mediated immunity, and inhibits production of pro-inflammatory cytokines (e.g., TNF-ct and XL-6) by monocytes. Pomalidomide also inhibits angiogenesis by blocking the migration and adhesion of endothelial cells. Due to its diversified pharmacological properties, pomalidomide is useful in treating, preventing, and/or managing various diseases or disorders. Pomalidomide and methods of synthesizing the compound are described, e.g., in U.S. Patent Nos. 5,635,517, 6,335,349, 6,316,471, 6,476,052, 7,041,680, 7,709,502, and 7,994,327; and U.S. Patent Application Publication Nos. 2006/0178402 and 2011/0224440; the entireties of which are incorporated herein by reference. Citation of any references in this Section is not to be construed as an admission that such references are prior art to the present application.

Provided herein are solid forms (e.g., crystal forms or amorphous forms, or mixtures thereof) comprising pomalidomide, or pharmaceutically acceptable salts, stereoisomers, solvates (including, hydrates), co-crystals, prodrugs, or elathrates thereof. Also provided are methods of preparing, isolating, and characterizing the solid forms. Also provided herein are pharmaceutical compositions and single unit dosage forms, which comprise one or more solid forms provided herein.

Also provided herein are methods of treating and managing various diseases or disorders. The methods comprise administering to a. patient in need of such treatment or management a therapeutically effective amount of a solid form provided herein. Also provided herein are methods of preventing various diseases and disorders, which comprise administering to a patient in need of such prevention a prophylactically effective amount of a solid form provided herein. The various diseases and disorders include, but are not limited to: cancer, including hematologic cancer or solid tumor, for example, multiple myeloma, leukemia, lymphoma, sarcoma, prostate cancer, or small cell lung cancer; scleroderma; amyloidosis; pain; myelofibrosis; myeloproliferative disease, for example, myelofibrosis with myeloid metaplasia (MMM);

myelodysplastic syndromes; diffuse systemic sclerosis; macular degeneration; a skin disease; a pulmonary disorder; an asbestos-related disorder; a parasitic disease; an immunodeficiency disorder; a CNS disorder; a CNS injury; atherosclerosis; hemoglobinopathy; anemia, for example, sickle ceil anemia; an inflammatory disease; an autoimmune disease; a viral disease; a genetic disease; an allergic disease; a bacterial disease; an ocular neovascular disease; a choroidal neovascular disease; a retina neovascular disease; and rubeosis.

Figure 1 of WO2013/126326 provides a representative X-ray Powder Diffraction (XRPD) pattern of one embodiment of a solid form of pomalidomide. Figure 2 of WO2013/126326 provides a representative XRPD pattern of one embodiment of a solid form of pomalidomide. Figure 3 of WO2013/126326 provides a representative XRPD pattern of one embodiment of a solid form of pomalidomide. Figure 4 of WO2013/126326 provides a representative XRPD pattern of one embodiment of a solid form of pomalidomide. Figure 5 of WO2013/126326 provides a representative XRPD pattern of one embodiment of a solid form of pomalidomide. Figure 6 of WO2013/126326 provides a representative XRPD pattern of one embodiment of a solid form of pomalidomide. Figure 7 of WO2013/126326 provides a birefringence image (at 5x magnification) of representative crystals of one embodiment of a solid form of pomalidomide. Figure 8 of WO2013/126326 provides a birefringence image (at 5x magnification) of representative crystals of one embodiment of a solid form of pomalidomide. Figure 9 of WO2013/126326 provides a birefringence image (at 5x magnification) of representative crystals of one embodiment of a solid form of pomalidomide. Figure 10 of WO2013/126326 provides a representative Differential Scanning Calorimetry (DSC) thermogram of one embodiment of a solid form of pomalidomide. Figure 11 of WO2013/126326 provides a representative DSC thermogram of one embodiment of a solid form of pomalidomide. Figs. 12a and 12b of WO2013/126326 provide representative DSC cycHne thermograms of embodiments of solid forms of pomalidomide. Figure 13 of WO2013/126326 provides a representative DSC thermogram of one embodiment of a solid form of pomalidomide. Figure 14 of WO2013/126326 provides a representative DSC thermogram of one embodiment of a solid form of pomalidomide. Figure 15 of WO2013/126326 provides a representative DSC thermogram of one embodiment of a solid form of pomalidomide. Figure 16 of WO2013/126326 provides a representative DSC thermogram of one embodiment of a solid form of pomalidomide. Figure 17 of WO2013/126326 provides a representative Thermal Gravimetric Analysis (TGA) thermogram of one embodiment of a solid form of pomalidomide. Figure 18 of WO2013/126326 provides a representati e XRPD pattern of one embodiment of an amorphous form of pomalidomide. Figure 19 of WO2013/126326 provides one embodiment of a representative single X-ray structure of pomalidomide. Figure 20 of WO2013/126326 provides one embodiment of a representative single X-ray structure of pomalidomide. Figure 21 of WO2013/126326 provides a simulated XRPD pattern, based on the single X-ray data of pomalidomide. Figure 22 of WO2013/126326 provides a representative infrared (IR) spectrum of one embodiment of a solid form of pomalidomide. Figure 23 of WO2013/126326 provides a dynamic vapor sorption (DVS) isotherm plot of one embodiment of a solid form of pomalidomide. Figure 24 of WO2013/126326 provides a DVS isotherm plot of one embodiment of a solid form of pomalidomide. Figure 25 of WO2013/126326 provides a representative XRPD pattern of one embodiment of a solid form of pomalidomide. Figure 26 of WO2013/126326 pro vides a representative XRPD pattern of one embodiment of a solid form of pomalidomide. Figure 27 of WO2013/126326 provides a representative XRPD pattern of one embodiment of a solid form of pomalidomide after compression test.

As used herein, and in the specification and the accompanying claims, the indefinite articles "a" and "an" and the definite article "the" include plural as well as single referents, unless the context clearly indicates otherwise. As used herein, and unless otherwise specified, the compound referred to herein by the name pomalidomide, 4-amino-2-(2,6-dioxopiperidine-3-yl)isoindoliRe-1,3-dione, or CC-4047, corresponds to chemical structure (IV), depicted below. In certain embodiments, the term pomalidomide, 4-amino-2-(2,6-dioxopiperidine-3-yl)isoindoline-1,3-dione, or CC-4047 may be used herein to refer to either a free base form or an ionized form of a compound of formula (IV) (e.g., the molecule is protonated at one or more basic centers).

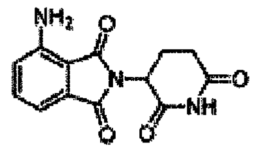

(IV)

Unless otherwise specified, the terms "solid form," "solid forms," and related terms, when used herein to refer to pomalidomide, refer to a pliysical form comprising pomalidomide, which is not predominantly in a liquid or a gaseous state. As used herein, the terms "solid form" and "solid forms" encompass semi-solids. Solid forms may be crystalline, amorphous, partially crystalline, partially amorphous, or mixtures of forms. A "single-component" solid form comprising pomalidomide consists essentially of pomalidomide. A "multiple-component" solid form comprising pomalidomide comprises a significant quantity of one or more additional species, such as ions and/or molecules, within the solid form. For

example, in particular embodiments, a crystalline multiple-component solid form comprising pomalidomide further comprises one or more species non-covalenily bonded at regular positions in the crystal lattice. Unless otherwise specified, the term "crystalline" and related terms used herein, when used to describe a substance, component, product, or form, mean that the substance, component, product, or form is substantially crystalline, for example, as determined by X-ray diffraction, (see, e.g., Remington 's Pharmaceutical Sciences, 18th ed., Mack Publishing, Easton PA, 173 (1990); The United States Pharmacopeia, 23rd ed., 1843-1844 (1995)). Unless otherwise specified, the term "crystal form," "crystal forms," and related terms herein refer to crystalline modifications comprising a given substance, including single-component crystal forms and multiple-component crystal forms, and including, but not limited to, polymorphs, solvates, hydrates, co-crystals, other molecular complexes, salts, solvates of salts, hydrates of salts, co-crystals of salts, and other molecular complexes of salts, and polymorphs thereof. In some embodiments, a crystal form of a substance may be substantially free of amorphous forms and/or other crystal forms. In other embodiments, a crystal form of a substance may contain less than about 1%, 2%, 3%, 4%, 5%, 10%, 15%, 20%, 25%, 30%, 35%, 40%, 45%, or 50% of one or more amorphous form(s) and/or other crystal form(s) on a weight basis. Crystal forms of a substance may be obtained by a number of methods. Such methods include, but are not limited to, melt recrystallization, melt cooling, solvent recrystallization, recrystallization in confined spaces such as, e.g., in nanopores or capillaries, recrystallization on surfaces or templates such as, e.g., on polymers, recrystallization in the presence of additives, such as, e.g., co-crystal counter-molecules, desolvation, dehydration, rapid evaporation, rapid cooling, slow cooling, vapor diffusion, sublimation, grinding, and solvent-drop grinding. Unless otherwise specified, the terms "polymorph," "polymorphic form," "polymorphs," "polymorphic forms," and related terms herein refer to two or more crystal forms that consist essentially of the same molecule, molecules or ions. Different polymorphs may have different physical properties, such as, for example, melting temperatures, heats of fusion, solubilities, dissolution rates, and/or vibrational spectra as a result of a different arrangement or conformation of the molecules or ions in the crystal lattice. The differences in physical properties exhibited by polymorphs may affect pharmaceutical parameters, such as storage stability, compressibility and density (important in formulation and product manufacturing), and dissolution rate (an important factor in bioavailability). Differences in stability can result from changes in chemical reactivity (e.g., differential oxidation, such that a dosage form discolors more rapidly when comprised of one polymorph than when comprised of another polymorph) or mechanical changes (e.g., tablets crumble on storage as a kinetically favored polymorph converts to thermodynamically a more stable polymorph) or both (e.g., tablets of one polymorph are more susceptible to breakdown at high humidity). As a result of solubility/dissolution differences, in the extreme case, some polymorphic transitions may result in lack of potency or, at the other extreme, toxicity. In addition, the physical properties of the crystal may be important in processing; for example, one polymorph might be more likely to form solvates or might be difficult to filter and wash free of impurities (e.g., particle shape and size distribution might be different between polymorphs). Unless otherwise specified, the term "co-crystal," as used herein, refers to a crystal form of a substance which contains at least one additional ingredient. The substance and the additional ingredient(s) interact through non-covalent forces in a crystal lattice. Unless otherwise specified, the term "amorphous," "amorphous form," and related terms used herein mean that the substance, component, or product referred to is not substantially crystalline as determined by X-ray diffraction. In certain embodiments, an amorphous form of a substance may be substantially free of crystal forms. In other embodiments, an amorphous form of a substance may contain less than about 1%, 2%, 3%, 4%, 5%, 10%, 15%, 20%, 25%, 30%, 35%, 40%, 45% or 50% of one or more crystal forms on a weight basis. In other embodiments, an amorphous form of a substance may comprise additional components or ingredients (for example, an additive, a polymer, or an excipient that may serve to further stabilize the amorphous form). In some embodiments, amorphous form may be a solid solution. Amorphous forms of a substance can be obtained by a number of methods. Such methods include, but are not limited to, heating, melt cooling, rapid melt cooling, solvent evaporation, rapid solvent evaporation, desolvation, sublimation, grinding, ball-milling, cryo-grinding, spray drying, and freeze drying. As used herein, and unless otherwise specified, the terms "about" and "approximately," when used in connection with doses, amounts, or weight percents of ingredients of a composition or a dosage form, mean a dose, amount, or weight percent that is recognized by one of ordinary skill in the art to provide a pharmacological effect equivalent to that obtained from the specified dose, amount, or weight percent. In certain embodiments, the terms "about" and "approximately," when used in this context, contemplate a dose, amount, or weight percent within 30%, within 20%, within 15%, within 10%, or within 5%, of the specified dose, amount, or weight percent. Techniques for characterizing crystal forms and amorphous forms include, but are not limited to, thermal gravimetric analysis (TGA), differential scanning caiorimetry (DSC), X-ray powder diffractometry (XRPD), single-crystal X-ray diffractometry, vibrational spectroscopy, e.g., infrared (IR) and Raman spectroscopy, solid-state and solution nuclear magnetic resonance (NMR) spectroscopy, optical microscopy, hot stage optical microscopy, scanning electron microscopy (SEM), electron crystallography and quantitative analysis, particle size analysis (PSA), surface area analysis, solubility studies, and dissolution studies. As used herein, and unless otherwise specified, the terms "about" and "approximately," when used in connection with a numeric value or range of values which is provided to characterize a particular solid form, e.g., a specific temperature or temperature range, such as, for example, that describes a melting, dehydration, desolvation, or glass transition temperature; a mass change, such as, for example, a mass change as a function of temperature or

humidity; a solvent or water content, in terms of, for example, mass or a percentage; or a peak position, such as, for example, in analysis by, for example, IR or Raman spectroscopy or XRPD; indicate that the value or range of values may deviate to an extent deemed reasonable to one of ordinary skill in the art while still describing the solid form. In certain embodiments, the terms "about" and "approximately," when used in this context, indicate that the numeric value or range of values may vary within 30%, 20%, 15%, 10%, 9%, 8%, 7%, 6%, 5%, 4%, 3%, 2%, 1.5%, 1%, 0.5%, or 0.25% of the recited value or range of values. For example, in some embodiments, the value of an XRPD peak position may vary by up to ±0.2 degrees two theta while still describing the particular XRPD peak. As used herein, and unless otherwise specified, a crystalline or amorphous form that is "pure," i.e., substantially free of other crystalline or amorphous forms, contains less than about 10% by weight of one or more other crystalline or amorphous forms, less than about 5% by weight of one or more other crystalline or amorphous forms, less than about 3% by weight of one or more other crystalline or amorphous forms, or less than about 1% by weight of one or more other crystalline or amorphous forms. As used herein, and unless otherwise specified, a solid form that is "substantially physically pure" is substantially free from other solid forms. In certain embodiments, a crystal form that is substantially physically pure contains less than about 50%, 45%, 40%, 35%, 30%, 25%, 20%, 15%, 10%, 9%, 8%, 7%, 6%, 5%, 4%, 3%, 2%, 1%, 0.5%, 0.4%, 0.3%, 0.2%, 0.1%, 0.05%, or 0.01% of one or more other solid forms on a weight basis. The detection of other solid forms can be accomplished by any method apparent to a person of ordinary skill in the art, including, but not limited to, diffraction analysis, thermal analysis, elemental combustion analysis and/or spectroscopic analysis. As used herein, and unless otherwise specified, a solid form that is "substantially chemically pure" is substantially free from other chemical compounds (i.e., chemical impurities). In certain embodiments, a solid form that is substantially chemically pure contains less than about 50%, 45%, 40%, 35%, 30%, 25%, 20%, 15%, 10%, 9%, 8%, 7%, 6%, 5%, 4%, 3%, 2%, 1%, 0.5%, 0.4%, 0.3%, 0.2%, 0.1 %, 0.05%, or 0.01% of one or more other chemical compounds on a weight basis. The detection of other chemical compounds can be accomplished by any method apparent to a person of ordinary skill in the art, including, but not limited to, methods of chemical analysis, such as, e.g., mass spectrometry analysis, spectroscopic analysis, thermal analysis, elemental combustion analysis and/or chromatographic analysis. As used herein, and unless otherwise indicated, a chemical compound, solid form, or composition that is "substantially free" of another chemical compound, solid form, or composition means that the compound, solid form, or composition contains, in certain embodiments, less than about 50%, 45%, 40%, 35%, 30%, 25%, 20%, 15%, 10%, 9%, 8%, 7%, 6%, 5%, 4%, 3%, 2%, 1%, 0.5%, 0.4%, 0.3%, 0.2% 0.1%, 0.05%, or 0.01% by weight of the other compound, solid form, or composition. As used herein, and unless otherwise specified, the term "pharmaceutically acceptable salts" refers to salts prepared from pharmaceutically acceptable, relatively non-toxic acids, including inorganic acids and organic acids, in some embodiments, suitable acids include, but are not limited to, acetic, benzenesulfonic, benzoic, camphorsulfonic, carbonic, citric, dihydrogenphosphoric, ethenesulfonic, fumaric, galactunoric, gluconic, glucuronic, glutamic, hydrobromic, hydrochloric, hydriodic, isobutyric, isethionic, lactic, maleic, malic, malonic, mandelic, methanesulfonic, monohydrogencarbonic, monohydrogen-phosphoric, monohydrogensulfuric, mucic, nitric, pamoic, pantothenic, phosphoric, phthalic, propionic, suberic, succinic, sulfuric, tartaric, toluenesulfonic acid (including / oluenesulfonie, zn-ioluenesulfonic, and o-toluenesulfonic acids), and the like (see, e.g., S. M. Berge et al, J. Pharm. Set, 66: 1-19 (1977); and Handbook of Pharmaceutical Salts: Properties, Selection and Use, P. H. Stahl and C. G. Wermuth, Eds., (2002), Wiley, Weinheim). In some embodiments, suitable acids are strong acids (e.g., with pKa less than about 1), including, but not limited to, hydrochloric, hydrobromic, sulfuric, nitric, methanesulfonic, benzene sulfonic, toluene sulfonic, naphthalene sulfonic, naphthalene disuifonic, pyridine-sulfonic, or other substituted sulfonic acids. Also included are salts of other relatively non-toxic compounds that possess acidic character, including amino acids, such as aspartic acid and the like, and other compounds, such as aspirin, ibuprofen, saccharin, and the like. Acid addition salts can be obtained by contacting the neutral form of a compound with a sufficient amount of the desired acid, either neat or in a suitable solvent. As solids, salts can exist in crystalline or amorphous forms, or mixtures thereof. Salts can also exist in polymorphic forms. Unless otherwise specified, the terms "solvate" and "solvated," as used herein, refer to a solid form of a substance which contains solvent. The terms "hydrate" and "hydrated" refer to a solvate wherein the solvent is water. "Polymorphs of solvates" refer to the existence of more than one solid form for a particular solvate composition. Similarly, "polymorphs of hydrates" refer to the existence of more than one solid form for a particular hydrate composition. The term "desolvated solvate," as used herein, refers to a solid form of a. substance which can be made by removing the solvent from a solvate. The terms "solvate" and "solvated," as used herein, can also refer to a solvate of a salt, co-crystal, or molecular complex. The terms "hydrate" and "hydrated," as used herein, can also refer to a hydrate of a salt, co-crystal, or molecular complex. As used herein, and unless otherwise specified, the terms "treat," "treating" and "treatment" refer to the eradication or amelioration of a disease or disorder, or of one or more symptoms associated with the disease or disorder. In certain embodiments, the terms refer to minimizing the spread or worsening of the disease or disorder resulting from the administration of one or more prophylactic or therapeutic agents to a subject with such a disease or disorder. In some embodiments, the terms refer to the administration of a compound provided herein, with or without other additional active agent, after the onset of symptoms of a particular disease. As used herein, and unless otherwise specified, the terms "prevent", "preventing" and "prevention" refer to the prevention of the onset, recurrence or spread of a disease or disorder, or of one or more symptoms thereof. In certain embodiments, the terms

refer to the treatment with or administration of a compound provided herein, with or without other additional active compound, prior to the onset of symptoms, particularly to patients at risk of a disease or disorder provided herein. The terms encompass the inhibition or reduction of a symptom of a particular disease. Patients with familial history of a disease in particular are candidates for preventive regimens in certain embodiments. In addition, patients who have a history of recurring symptoms are also potential candidates for the prevention. In this regard, the term "prevention" may be interchangeably used with the term "prophylactic treatment". As used herein, and unless otherwise specified, the terms "manage", "managing" and "management" refer to preventing or slowing the progression, spread, or worsening of a disease or disorder, or of one or more symptoms thereof. Often, the beneficial effects that a subject derives from a prophylactic and/or therapeutic agent do not result in a cure of the disease or disorder. In this regard, the term "managing" encompasses treating a. patient who had suffered from the particular disease in an attempt to prevent or minimize the recurrence of the disease or one or more symptoms thereof. As used herein, and unless otherwise specified, a "therapeutically effective amount" of a compound is an amount sufficient to provide a therapeutic benefit in the treatment or management of a disease or disorder, or to delay or minimize one or more symptoms associated with the disease or disorder. A therapeutically effective amount of a compound means an amount of therapeutic agent, alone or in combination with other therapies, that provides a therapeutic benefit in the treatment or management of the disease or disorder. The term "therapeutically effective amount" can encompass an amount that improves overall therapy, reduces or avoids symptoms or causes of disease or disorder, or enhances the therapeutic efficacy of another therapeutic agent. As used herein, and unless otherwise specified, a "prophylactically effective amount" of a compound is an amount sufficient to prevent a disease or disorder, or one or more symptoms thereof, or prevent the recurrence of the disease or disorder, or one or more symptoms thereof. A prophylactically effective amount of a compound means an amount of therapeutic agent, alone or in combination with other agents that provide a prophylactic benefit in the prevention of the disease or disorder. The term "prophylactically effective amount" can encompass an amount that improves overall prophylaxis or enhances the prophylactic efficacy of another prophylactic agent. Unless otherwise specified, the term "composition" as used herein is intended to encompass a product comprising the specified ingredient(s) (and in the specified amountis), if indicated), as well as any product which results, directly or indirectly, from combination of the specified ingredient(s) in the specified amouni(s). By "pharmaceutically acceptable," it is meant a diluent, excipient, or carrier in a formulation must be compatible with the other ingredient(s) of the formulation and not deleterious to the recipient thereof. Unless otherwise specified, the term "therapeutically and prophylactically effective amount" refers to the amount of the subject solid form that will elicit the biological or medical response of a tissue, system, animal or human that is being sought by the researcher, veterinarian, medical doctor or other clinician or that is sufficient to prevent development of or alleviate to some extent one or more of the symptoms of the disease being treated. Unless otherwise specified, the term "subject" is defined herein to include animals, such as mammals, including, but not limited to, primates (e.g., humans), cows, sheep, goats, horses, dogs, cats, rabbits, rats, mice, and the like. In specific embodiments, the subject is a human. As used herein, and unless otherwise specified, the terms "symptom of a hemoglobinopathy" and "symptom of anemia" mean any physiological or biological symptom associated with any hemoglobinopathy or anemia, including, but not limited to, dizziness, shortness of breath, loss of consciousness, tiredness, weakness, hemolysis, pains associated with abnormal hemoglobin, reduced erythrocyte counts (i.e., reduced hematocrit), a reduced ability of a given volume of blood to cany oxygen, as compared with a volume of normal blood, deformities of erythrocytes visible under a microscope, etc. The terms also include negative psychological symptoms such as depression, low self-esteem, perception of illness, perception of limited physical capability, etc. The compounds provide herein may also contain an unnatural proportion of an atomic isotope at one or more of the atoms that constitute such a compound. For example, the compound may be radiolabeled with radioactive isotopes, such as for example tritium (H), iodine-125 ($^{125}I$) sulfur-35 ($^{35}S$), or carbon-14 ($^{14}C$). Radiolabeled compounds are useful as therapeutic agents, e.g., cancer therapeutic agents, research reagents, e.g., binding assay reagents, and diagnostic agents, e.g., in vivo imaging agents. All isotopic variations of the compounds provided herein, whether radioactive or not, are intended to be encompassed herein. In certain embodiments, a compound provided herein contains unnatural proportion(s) of one or more isotopes, including, but not limited to, hydrogen ($^{1}H$), deuterium ($^{2}H$), tritium ($^{3}H$), carbon-11 ($^{11}C$), carbon-12 (12C), carbon-13 ($^{13}C$), carbon-14 ($^{14}C$), nitrogen-13 ($^{13}N$), nitrogen-14 ($^{14}N$), nitrogen-15 ($^{15}N$), oxygen-14 ($^{14}O$), oxygen-15 ($^{15}O$), oxygen-16 ($^{16}O$), oxygen-17 ($^{17}O$), oxygen- 8 ($^{18}O$), fluorine-17 ($^{17}F$), fluorine-18 ($^{18}F$), phosphorus-31 ($^{31}P$), phosphorus-32 ($^{32}P$), phosphorus-33 ($^{33}P$), sulfur-32 ($^{32}S$), sulfur-33 ($^{33}S$), sulfur-34 ($^{34}S$), sulfur-35 ($^{35}S$), sulfur-36 ($^{36}S$), chlorine-35 ($^{35}Cl$), chlorine-36 ($^{36}Cl$), chlorine-37 ($^{37}Cl$), bromine-79 ($^{79}Br$), bromine-81 ($^{81}Br$), iodine-123 ($^{123}I$), iodine-125 ($^{125}I$), iodine- 127 ($^{127}I$), iodine-129 ($^{129}I$), and iodine-131 ($^{131}I$). In certain embodiments, a compound provided herein contains unnatural proportion(s) of one or more isotopes in a stable form, that is, nonradioactive, including, but not limited to, hydrogen ($^{1}H$), deuterium ($^{2}H$), carbon-12 ($^{12}C$), carbon-13 ($^{13}C$), nitrogen-14 ($^{14}N$), nitrogen-15 ($^{15}N$), oxygen-16 ($^{16}O$), oxygen-17 ($^{17}O$), oxygen-18 ($^{18}O$), fluorine-17 ($^{17}F$), phosphorus-31 ($^{31}P$), sulfur-32 ($^{32}S$), sulfur-33 ($^{33}S$), sulfur-34 ($^{34}S$), suifur-36 ($^{36}S$), chlorine-35 ($^{31}Cl$), chlorine-37 ($^{37}Cl$), bromine-79 ($^{79}Br$), bromine-81 ($^{81}Br$), and iodine-127 ($^{127}I$). In certain embodiments, a compound pro vided herein contains unnatural proportion(s) of one or more isotopes in an unstable form, that is radioactive, including, but not limited to, tritium ($^{3}H$), carbon-11 ($^{11}C$), carbon-14 ($^{14}C$), nitrogen-13 ($^{13}N$), oxygen-14 ($^{14}O$), oxygen-15 ($^{15}O$),

fluorine-18 ($^{18}F$), phosphorous-32 ($^{32}P$), phosphorus-33 ($^{33}P$), sulfur-35 ($^{35}S$), chlorine-36 ($^{36}Cl$), iodine-123 ($^{123}I$), iodine-125 ($^{125}I$), iodine-129 ($^{129}I$), and iodine-131 ($^{131}I$). in certain embodiments, in a compound as provided herein, any hydrogen can be $^{1}H$, for example, or any carbon can be $^{13}C$, for example, or any nitrogen can be $^{15}N$, for example, or any oxygen can be $^{18}O$, for example, where feasible according to the judgment of one of skill. In certain embodiments, a compound provided herein contains unnatural proportions of deuterium (D). In exemplary embodiments, provided herein are isotopologues of pomalidomide, as disclosed in U.S. Provisional Application No. 61/500,053, filed June 22, 2011, which is incorporated by reference herein in its entirety. In one embodiment, provided herein are solid forms (e.g., crystal forms, amorphous forms, or mixtures thereof) of isotopologues of pomalidomide provided herein. Unless otherwise specified, to the extent that there is a discrepancy between a depicted chemical structure of a compound provided herein and a chemical name of a compound provided herein, the chemical structure shall control.

In one embodiment, provided herein are single-component or multiple-component solid forms (e.g., crystal forms, amorphous forms, or mixtures thereof) comprising pomalidomide, e.g., a solid form of pomalidomide or a pharmaceutically acceptable salt, solvate, hydrate, co-crystal, stereoisomer, prodrug, or clathrate thereof. In one embodiment, provided herein are solid forms (e.g., crystal forms, amorphous forms, or mixtures thereof) of a free base of pomalidomide, or a solvate, hydrate, co-crystal, stereoisomer, prodrug, or clathrate thereof. Pomalidomide can be synthesized or obtained according to a method known in the literature or based upon the teachings herein, including the methods described in detail in the examples herein. In one embodiment, pomalidomide can be prepared according to methods described in, for example, U.S. Patent Nos. 5,635,517, 6,335,349, 6,316,471, 6,476,052, 7,041,680, 7,709,502, and 7,994,327; and U.S. Patent Application Publication Nos. 2006/0178402 and 2011/0224440; the entireties of which are incorporated herein by reference. In one embodiment, solid forms provided herein may be a crystal form or an amorphous form or mixtures thereof (e.g., mixtures of crystal forms, or mixtures of crystal and amorphous forms), which comprises pomalidomide or a pharmaceutically acceptable salt, solvate, hydrate, co-crystal, stereoisomer, prodrug, or clathraie thereof. In one embodiment, provided herein is a crystal form comprising pomalidomide or a pharmaceutically acceptable salt, solvate, hydrate, co-crystal, stereoisomer, prodrug, or clathraie thereof. In one embodiment, provided herein is an amorphous form comprising pomalidomide or a pharmaceutically acceptable salt, solvate, hydrate, co-crystal, stereoisomer, prodrug, or elathratc thereof. In one embodiment, provided herein is a mixture comprising two or more crystal forms of pomalidomide or a pharmaceutically acceptable salt, solvate, hydrate, co-crystal, stereoisomer, prodrug, or clathraie thereof. In one embodiment, provided herein is a mixture comprising a crystal form and an amorphous form of pomalidomide or a pharmaceutically acceptable salt, solvate, hydrate, co-crystal, stereoisomer, prodrug, or clathrate thereof. Solid forms comprising pomalidomide include: single-component and multiple-component forms, crystal forms comprising pomalidomide, amorphous forms comprising pomalidomide, mixtures of crystal forms comprising pomalidomide, mixtures of crystal and amorphous forms comprising pomalidomide, including, but not limited to, polymorphs, salts, solvates, hydrates, co-crystals, stereoisomers, prodrugs, and clathrates of pomalidomide. In one embodiment, provided herein is an unsolvated solid form comprising pomalidomide. In one embodiment, provided herein is an anhydrous solid form comprising pomalidomide. In one embodiment, provided herein is an unsolvated crystal form comprising pomalidomide. In one embodiment, provided herein is an anhydrous crystal form comprising pomalidomide. In one embodiment, provided herein is an unsolvated amorphous form comprising pomalidomide. In one embodiment, provided herein is an anhydrous amorphous form comprising pomalidomide. In one embodiment, provided herein is a solvated solid form comprising pomalidomide. In one embodiment, provided herein is a hydrated solid form comprising pomalidomide (e.g., a hydrate having a stoichiometric or non-stoichiometric amount of water). Irs one embodiment, provided herein is a hydrated form of pomalidomide, including, but not limited to, a hemihydrate, a monohydrate, a dihydrate, a trihydrate, and the like. In one embodiment, the hydrated form is substantially crystalline. In one embodiment, the hydrated form is substantially amorphous. In one embodiment, the anhydrous form is substantially crystalline. In one embodiment, the anhydrous form is substantially amorphous. Solid forms provided herein can be prepared by the methods described herein, or by techniques, including, but not limited to, heating, cooling, freeze drying, spray drying, lyophilization, quench cooling the melt, rapid solvent evaporation, slow solvent evaporation, solvent recrystallization, antisolvent addition, slurry recrystallization, crystallization from the melt, desolvation, recrystallization in confined spaces, such as, e.g., in nanopores or capillaries, recrystallization on surfaces or templates, such as, e.g., on polymers, recrystallization in the presence of additives, such as, e.g., co-crystal counter-molecules, desolvation, dehydration, rapid cooling, slow cooling, exposure to solvent and/or water, drying, including, e.g., vacuum drying, vapor diffusion, sublimation, grinding (including, e.g., cryo-grinding and solvent-drop grinding), microwave-induced precipitation, sonication-mduced precipitation, laser-induced precipitation, and precipitation from a supercritical fluid. The particle size of the resulting solid forms, which can vary (e.g., from nanometer dimensions to millimeter dimensions), can be controlled, e.g., by varying crystallization conditions, such as, e.g., the rate of crystallization and/or the crystallization solvent system, or by particle-size reduction techniques, e.g., grinding, milling, micronizing, or sonication. In another embodiment, provided herein are compositions comprising one or more solid form(s) comprising pomalidomide, for example, a solid form of pomalidomide or a pharmaceutically acceptable salt, solvate, hydrate, co-crystal, stereoisomer, prodrug, or clathrate thereof. Also provided herein are compositions comprising: (i) one or more solid form(s) provided herein (e.g., one or more crystal forms, one

or more amorphous forms, and mixtures thereof), and (ii) other active ingredient(s). Also provided herein are methods of using these compositions in the treatment, prevention, or management of conditions and disorders including, but not limited to: cancer, including hematologic cancer or solid tumor, for example, multiple myeloma, leukemia, lymphoma, sarcoma, prostate cancer, or small cell lung cancer; scleroderma; amyloidosis; pain; myelofibrosis; myeloproliferative disease, e.g., MMM; myelodysplastic syndromes; diffuse systemic sclerosis; macular degeneration; a skin disease; a pulmonary disorder; an asbestos-related disorder; a parasitic disease; an immunodeficiency disorder; a CNS disorder; a CNS injury; atherosclerosis; hemoglobinopathy; anemia, e.g., sickle cell anemia; an inflammatory disease; an autoimmune disease; a viral disease; a genetic disease; an allergic disease; a bacterial disease; an ocular neovascular disease; a choroidal neovascular disease; a retina neovascular disease; and mbeosis.

While not intending to be bound by any particular theory, certain solid forms provided herein exhibit physical properties, e.g., stability, solubility and/or dissolution rate, appropriate for use in clinical and therapeutic dosage forms. Moreover, while not wishing to be bound by any particular theory, certain solid forms provided herein exhibit physical properties, e.g., crystal morphology, compressibility and/or hardness, suitable for manufacture of a solid dosage form. In some embodiments, such properties can be determined using techniques such as X-ray diffraction, microscopy, IR spectroscopy and thermal analysis, as described herein and known in the art.

Certain embodiments herein provide solid forms comprising pomalidomide. In one embodiment, provided herein is a solid form of pomalidomide that is substantially crystalline. In one embodiment, provided herein is a crystal form of pomalidomide. In one embodiment, provided herein is a solid form of pomalidomide comprising crystalline pomalidomide. In one embodiment, provided herein is a solid form of pomalidomide comprising an amorphous form of pomalidomide. In one embodiment, provided herein is a solid form of pomalidomide comprising a crystal form and an amorphous form of pomalidomide. In one embodiment, provided herein is a solid form of pomalidomide comprising two or more crystal forms of pomalidomide. In certain embodiments, provided herein are solid forms of pomalidomide that can be designated herein as Form A. Provided herein are various embodiments, preparations, or modifications of Form A. In certain embodiments, Form A can be obtained by crystallization from certain solvent systems, for example, solvent systems comprising one or more of the following solvents or solvent combinations: acetonitrile, methyl ethyl ketone (MEK), tetrahydrofuran (THF), and tetrahydrofura ieptane. Other examples of solvent systems are provided herein elsewhere. In certain embodiments, a solid form provided herein (e.g.. Form A) can be obtained by slurry crystallization, evaporation crystallization, cooling crystallization, and precipitation crystallization. In certain embodiments, slurry crystallization is effected by adding solvent or solvent mixtures to a solid substrate, and the slurry is stirred, and optionally heated to various temperatures. In certain embodiments, the slurry is heated at about 25°C, about 50°C, about 80°C, or about 100°C. In certain embodiments, upon heating and cooling, the residual solvents of the slurry can be removed by wicking, or other suitable methods, such as filtration, centrifugation, or decantation, and the crystals can be dried in air or under vacuum. In certain embodiments, evaporation crystallization is effected by adding a solvent or solvent mixture to a solid substrate, and allowing the solvent or solvent mixture to evaporate under ambient conditions. In certain embodiments, the residual solvent can be removed by wicking, or other suitable methods, such as filtration, centrifugation, or decantation, and the crystals can be dried in air or under vacuum. In certain embodiments, precipitation crystallization is effected by adding a solvent or solvent mixture to a solid substrate, and subsequently adding an anti-solvent. In certain embodiments, the resultant mixture stands for a period of time, e.g., overnight, and under certain conditions, for example at room temperature. In certain embodiments, the residual solvent can be removed by wicking, or other suitable methods, such as filtration, centrifugation, or decantation, and the crystals can be dried in air or under vacuum. In certain embodiments, cooling crystallization is effected by adding a solvent or solvent mixture to a solid substrate at elevated temperature, and allowing the resultant mixture to stand for a period of time at a reduced temperature. In certain embodiments, the elevated temperature is, for example, about 30°C, about 40°C, about 50°C, about 60°C, about 70°C, or about 80°C. In certain embodiments, the reduced temperature is, for example, about 15°C, about 10°C, about 5°C, about 0°C, about -5°C, about -10°C, about -15°C, or about -20°C. The residual solvent can be removed by wicking, or other suitable methods, such as filtration, centrifugation, or decantation, and the crystals can be dried in air or under vacuum. In certain embodiments, a solid form provided herein, e.g., Form A, is substantially crystalline, as indicated by, e.g., X-ray powder diffraction measurements. In one embodiment, representative XRPD patterns of various embodiments of Form A are provided in FIGS, 1-6 and 25-27. In one embodiment, provided herein is a crystalline pomalidomide ({drug4b}) having an XRPD pattern corresponding substantially to the representative XRPD pattern depicted in Figure 1 of WO2013/126326. In one embodiment, provided herein is a crystalline pomalidomide ({drug4b}) having an XRPD pattern corresponding substantially to the representative XRPD pattern depicted in Figure 2 of WO2013/126326. In one embodiment, provided herein is a crystalline pomalidomide ({drug4b}) having an XRPD pattern corresponding substantially to the representative XRPD pattern depicted in Figure 3 of WO2013/126326. In one embodiment, provided herein is a crystalline pomalidomide ({drug4b}) having an XRPD pattern corresponding substantially to the representative XRPD pattern depicted in Figure 4 of WO2013/126326. In one embodiment, provided herein is a crystalline pomalidomide ({drug4b}) having an XRPD pattern corresponding substantially to the representative XRPD pattern depicted in Figure 5 of WO2013/126326. In one embodiment, provided herein is a crystalline pomalidomide ({drug4b}) having an XRPD pattern corresponding substantially to the representative XRPD pattern depicted in Figure 6 of WO2013/126326. In one embodiment, provided herein is a crystalline pomalidomide ({drug4b}) having an XRPD pattern corresponding substantially to the representative XRPD pattern depicted in Figure 25 of WO2013/126326. In one embodiment, provided herein is a crystalline pomalidomide ({drug4b}) having an XRPD pattern corresponding substantially to the representative XRPD pattern depicted in Figure 26 of WO2013/126326. In one embodiment, provided herein is a crystalline pomalidomide ({drug4b}) having an XRPD pattern corresponding substantially to the representative XRPD pattern depicted in Figure 27 of WO2013/126326. In some embodiments, provided herein is a crystalline pomalidomide ({drug4b}) characterized by one or more XRPD peaks (e.g., one, two, three, four, five, six, seven, eight, nine, ten, or greater than ten peaks) selected from peaks located at the following approximate positions: 12.1, 14.0, 16.2, 17.4, 18.4, 19.3, 20.1, 23.0, 24.4, 24.8, 25.6, 26.9, 28.1, and 29.2 degrees 20. In some embodiments, provided herein is a crystalline pomalidomide ({drug4b}) characterized by any 1, 2, 3, 4, 5, 6, 7, 8, 9, 10, or more of XRPD peaks selected from peaks located at the following approximate positions: 12.1, 14.0, 16.2, 17.4, 18.4, 19.3, 20.1, 23.0, 24.4, 24.8, 25.6, 26.9, 28.1, and 29.2 degrees 2 Θ. In some embodiments, provided herein is a crystalline pomalidomide ({drug4b}) characterized by one or more XRPD peaks (e.g., one, two, three, four, five, six, seven, eight, nine, ten, or greater than ten peaks) selected from peaks located at the following approximate positions: 12.15, 14.0, 16.25, 17.4, 1 8.45, 19.25, 20.05, 22.95, 24.4, 24.85, 25.6, 26.95, 28.05, and 29.2 degrees 2.0. In some embodiments, provided herein is a crystalline pomalidomide ({drug4b}) characterized by any 1, 2, 3, 4, 5, 6, 7, 8, 9, 10, or more of XRPD peaks selected from peaks located at the following approximate positions: 12.15, 14.0, 16.25, 17.4, 18.45, 19.2,5, 20.05, 22.95, 24.4, 24.85, 25.6, 26.95, 28.05, and 29.2 degrees 20. in some embodiments, provided herein is a crystalline pomalidomide ({drug4b}) characterized by one or more XRPD peaks (e.g., one, two, three, four, five, six, seven, or eight peaks) selected from peaks located at the following approximate positions: 12, 1, 14.0, 17.4, 18.4, 24.4, 24.8, 25.6, and 28.1 degrees 20, In some embodiments, provided herein is a crystalline pomalidomide ({drug4b}) characterized by one or more XRPD peaks (e.g., one, two, three, four, five, six, seven, or eight peaks) selected from peaks located at the following approximate positions: 12.15, 14.0, 17,4, 18,45, 24.4, 24.85, 25.6, and 28,05 degrees 20. In some embodiments, provided herein is a crystalline pomalidomide ({drug4b}) characterized by one or more XRPD peaks (e.g., one, two, three, four, five, six, seven, or eight peaks) selected from peaks located at the following approximate positions: 12.1, 14.0, 17.4, 18.4, 23.0, 24.4, 25.6, and 28.1 degrees 20. In some embodiments, provided herein is a crystalline pomalidomide ({drug4b}) characterized by one or more XRPD peaks (e.g., one, two, three, four, five, six, seven, or eight peaks) selected from peaks located at the following approximate positions: 12.15, 14.0, 17.4, 18,45, 22.95, 24.4, 25.6, and 28,05 degrees 20. In some embodiments, provided herein is a crystalline pomalidomide ({drug4b}) characterized by one or more XRPD peaks (e.g., one, two, three, four, five, six, or seven peaks) selected from peaks located at the following approximate positions: 12.1, 14.0, 17.4, 18.4, 24.4, 25.6, and 28,1 degrees 20. In some embodiments, provided herein is a crystalline pomalidomide ({drug4b}) characterized by one or more XRPD peaks (e.g., one, two, three, four, five, six, or seven peaks) selected from peaks located at the following approximate positions: 12.15, 14.0, 17.4, 18.45, 24.4, 25.6, and 28.05 degrees 20. In some embodiments, provided herein is a crystalline pomalidomide ({drug4b}) characterized by one or more XRPD peaks (e.g., one, two, three, four, five, six, or seven peaks) selected trom peaks located at the following approximate positions: 12.1, 14.0, 17.4, 18.4, 23.0, 24.4, and 25.6 degrees 20. In some embodiments, provided herein is a crystalline pomalidomide ({drug4b}) characterized by one or more XRPD peaks (e.g., one, two, three, four, five, six, or seven peaks) selected from peaks located at the following approximate positions: 12.15, 14.0, 17.4, 18.45, 22.95, 24.4, and 25.6 degrees 20. In some embodiments, provided herein is a crystalline pomalidomide ({drug4b}) characterized by one or more XRPD peaks (e.g., one, two, three, or four peaks) selected from peaks located at the following approximate positions: 12.1, 17.4, 24.4, and 25.6 degrees 20. In some embodiments, provided herein is a crystalline pomalidomide ({drug4b}) characterized by one or more XRPD peaks (e.g., one, two, three, or four peaks) selected from peaks located at the following approximate positions: 12.15, 17.4, 24.4, and 25.6 degrees 20. In some embodiments, provided herein is a crystalline pomalidomide ({drug4b}) characterized by one or more XRPD peaks (e.g., one, two, three, or four peaks) selected from peaks located at the following approximate positions: 12.1, 17.4, 25.6, and 28.1 degrees 20, In some embodiments, provided herein is a crystalline pomalidomide ({drug4b}) characterized by one or more XRPD peaks (e.g., one, two, three, or four peaks) selected from peaks located at the following approximate positions: 12.15, 17.4, 25,6, and 28.05 degrees 20. In some embodiments, provided herein is a crystalline pomalidomide ({drug4b}) characterized by one or more XRPD peaks (e.g., one, two, or three peaks) selected from peaks located at the following approximate positions: 12.1, 17.4, and 25.6 degrees 20. In some embodiments, provided herein is a crystalline pomalidomide ({drug4b}) characterized by one or more XRPD peaks (e.g., one, two, or three peaks) selected from peaks located at the following approximate positions: 12.15, 17.4, and 25.6 degrees 20. In some embodiments, provided herein is a crystalline pomalidomide ({drug4b}) characterized by one or more XRPD peaks (e.g., one, two, or three peaks) selected from peaks located at the following approximate positions: 12.1, 17.4, and 24.4 degrees 20. In some embodiments, provided herein is a crystalline pomalidomide ({drug4b}) characterized by one or more XRPD peaks (e.g., one, two, or three peaks) selected from peaks located at the following approximate positions: 12.15, 17.4, and 24.4 degrees 20. In certain embodiments, provided herein is a crystalline pomalidomide ({drug4b}) characterized by XRPD peaks located at the following approximate positions: 12, 17, and 26 degrees 20. In

certain embodiments, provided herein is a crystalline pomalidomide ({drug4b}) characterized by XRPD peaks located at the following approximate positions: 12.1, 17.4, and 25.6 degrees 20. In certain embodiments, provided herein is a crystalline pomalidomide ({drug4b}) characterized by XRPD peaks located at the following approximate positions: 14, 18, and 24 degrees 20. In certain embodiments, provided herein is a crystalline pomalidomide ({drug4b}) characterized by XRPD peaks located at the following approximate positions: 14, 18, and 25 degrees 20. In certain embodiments, provided herein is a crystalline pomalidomide ({drug4b}) characterized by XRPD peaks located at the following approximate positions: 14.0, 18.4, and 24.4 degrees 20. In certain embodiments, provided herein is a crystalline pomalidomide ({drug4b}) characterized by XRPD peaks located at the following approximate positions: 14.0, 18.4, and 24.8 degrees 20. In certain embodiments, provided herein is a crysialline pomalidomide characterized by XRPD peaks located at the following approximate positions: 14, 18, 24, and 25 degrees 20. In certain embodiments, provided herein is a crystalline pomalidomide ({drug4b}) characterized by XRPD peaks located at the following approximate positions: 14.0, 18,4, 24.4, and 24.8 degrees 20. In certain embodiments, provided herein is a crystalline pomalidomide ({drug4b}) characterized by XRPD peaks located at the following approximate positions: 12, 14, 17, 18, 24, and 26 degrees 20. In certain embodiments, provided herein is a crystalline pomalidomide characterized by XRPD peaks located at the following approximate positions: 12, 1.4, 17, 18, 25, and 26 degrees 20. In certain embodiments, provided herein is a crystalline pomalidomide ({drug4b}) characterized by XRPD peaks located at the following approximate positions: 12.1, 14.0, 17.4, 18.4, 24.4, and 25.6 degrees 20. In certain embodiments, provided herein is a crystalline pomalidomide ({drug4b}) characterized by XRPD peaks located at the following approximate positions: 12.1, 14.0, 17.4, 18.4, 24.8, and 25.6 degrees 20. In certain embodiments, provided herein is a crysialline pomalidomide characterized by XRPD peaks located at the following approximate positions: 12.1, 14.0, 17.4, 1 8.4, 24.4, 24.8, and 25.6 degrees 20. In certain embodiments, provided herein is a crystalline pomalidomide ({drug4b}) characterized by XRPD peaks located at the following approximate positions: 12.1, 14.0, 17.4, 1 8.4, 24.4, 24.8, 25,6, and 28.1 degrees 20. In certain embodiments, provided herein is a crystalline pomalidomide ({drug4b}) characterized by XRPD peaks located at the following approximate positions: 12.1,14.0,17.4, 18.4, 20.1, 23.0, 24.4, 24.8, 25.6, 28.1, and 29.2 degrees 2Θ. In one embodiment, provided herein is a crystalline pomalidomide ({drug4b}) characterized by one or more XRPD peaks (e.g., one, two, three, four, five, six, seven, eight, nine, ten, or greater than ten peaks) selected from peaks located at the approximate positions (e.g., ± 0.2 degrees 20) as listed in Table I.

Table 1:

| Peak No. | Approximate Peak Position (°2θ) | Peak Intensity |
|---|---|---|
| 1 | 12.15 | 121.995 |
| 2 | 14.00 | 93.465 |
| 3 | 16.25 | 57.345 |
| 4 | 17.40 | 160.614 |
| 5 | 18.45 | 101.439 |
| 6 | 19.25 | 40.401 |
| 7 | 20.05 | 48.110 |
| 8 | 22.95 | 56.442 |
| 9 | 24.40 | 108.833 |
| 10 | 24.85 | 95.511 |
| 11 | 25.60 | 223.093 |
| 12 | 26.95 | 52.851 |
| 13 | 28.05 | 118.675 |
| 14 | 29.20 | 64.837 |

Certain embodiments of Form A exhibit an XRPD pattern corresponding to the representative XRPD of any one of Figures 1, 2, 3, 4, 5, 6, 25, 26, and 27 of WO2013/126326. In one embodiment, provided herein is a crystalline pomalidomide (e.g., Form A) having a particular crystal morphology. In one embodiment, birefringence images depicting certain pomalidomide crystals are provided in Figures 7-9 of WO2013/126326. In one embodiment, birefringence images depicting various embodiments of Form A are provided in Figures 7-9 of WO2013/126326. In one embodiment, pomalidomide crystals have a morphology substantially similar to that of the crystals depicted in any one of Figures 7, 8, and 9 of WO2013/126326. In some embodiments, pomalidomide crystals are of approximately the same size as the crystals depicted in any one of Figures 7, 8, and 9 of WO2013/126326. In one embodiment, provided herein is a crystalline pomalidomide ({drug4b}) having thermal characteristics corresponding substantially to the representative thermal characteristics depicted in Figures 10, 11, 12, 13, 14, 15, 16, and 17 of WO2013/126326. In one embodiment, representative thermal characteristics of various embodiments of Form A are provided in Figures 10, 11, 12, 13, 14,15, 16, and 17 of WO2013/126326. In one embodiment, provided herein is a crystalline pomalidomide ({drug4b}) having a DSC thermograms corresponding substantially to the representative DSC thermograms depicted in Figures 10, 11, 12, 13, 14, 5, and 16 of WO2013/126326. In one embodiment, representative DSC thermograms of various embodiments of Form A are presented in Figures 10, 11, 12, 13, 14, 15, and 16 of WO2013/126326. In one embodiment, provided herein is a crystalline pomalidomide ({drug4b}) having a DSC thermogram comprising an endothermic event with a maximum at about 320°C when heated from approximately 25°C to approximately 350°C (e.g., a DSC thermogram comprising an endotherm with a maximum at about 317.60 CC, about 31 8.62°C, about 319.16°C, about 319.35°C, about 31 9.87°C, about 320,02°C, about 320.08°C, about 320,46 CC, or about 320.83°C). In one embodiment, provided herein is a crystalline pomalidomide ({drug4b}) having a DSC thermogram comprising an endothermic event with a maximum at about.31 8°C (e.g., when heated from approximately 25°C to approximately 350°C). In one embodiment, provided herein is a crystalline pomalidomide ({drug4b}) having a DSC thermogram comprising an endothermic event with a maximum at about 319°C (e.g., when heated from approximately 25°C to approximately 350°C). In one embodiment, provided herein is a crystalline pomalidomide ({drug4b}) having a DSC thermogram comprising an endothermic event with a maximum at about 320°C (e.g., when heated from approximately 25°C to approximately 350°C). In one embodiment, provided herein is a crystalline pomalidomide ({drug4b}) having a DSC thermogram comprising an endothermic event with a maximum at about 321°C (e.g., when heated from approximately 25°C to approximately 350°C). In one embodiment, provided herein is a crystalline pomalidomide ({drug4b}) having a DSC thermogram comprising an onset of melting at about 319°C. In one embodiment, provided herein is a crystalline pomalidomide ({drug4b}) having a DSC thermogram indicative of a crystalline, unsolvaied material. In one embodiment, provided herein is a crystalline pomalidomide ({drug4b}) having a TGA thermograph corresponding substantially to the representative TGA thermogram depicted in Figure 17 of WO2013/126326. In one embodiment, a representative TGA thermogram of one embodiment of Form A is presented in Figure 17 of WO2013/126326. In certain embodiments, pro vided herein is a crystalline pomalidomide ({drug4b}) having a mass loss of less than approximately 1% of the total mass of the sample between the temperatures of approximately 25°C to approximately 200°C when heated from approximately 25°C to approximately 350°C. In certain embodiments, provided herein is a crystalline pomalidomide ({drug4b}) having a mass loss of less than about 0.001% of the total mass of the sample between the temperatures of approximately 25°C to approximately 150°C when heated from approximately 25°C to approximately 300°C. In certain embodiments, provided herein is a crystalline pomalidomide ({drug4b}) having a mass loss of less than 1%, less than 0.9%, less than 0.8%, less than 0.7%, less than 0.5%, less than 0.4%, less than 0.3%, less than 0.2%, less than 0.1%, less than 0.05%, less than 0.01%, less than 0.005%, or less than 0.001% of the total mass of the sample between the temperatures of approximately 25°C to approximately 200°C when heated from approximately 25°C to approximately 350°C. In one embodiment, provided herein is a. crystalline pomalidomide (e.g., Form A) that does not contain substantial amounts of solvent (e.g., water). In one embodiment, provided herein is a crystalline pomalidomide (e.g., Form A) that does not contain substantial amounts of solvent (e.g., water) in the crystal lattice. In certain embodiments, provided herein is a crystalline pomalidomide substantially free of solvent and water in the crystal lattice, as determined, e.g., via thermal analysis (such as, e.g., TG Analysis, TG-IR analysis, or TG-MS analysis), titration analysis for water content (such as, e.g., via volumetric or coulometric Karl Fischer titration), spectroscopic analysis (such as, e.g., NMRj, elemental analysis (such as, e.g., combustion analysis), or crystal structure analysis (such as, e.g., single-crystal X-ray diffraction). In certain embodiments, provided herein is an anhydrous crystal form comprising pomalidomide. In certain embodiments, provided herein is an unsolvated crystal form comprising pomalidomide. In one embodiment, provided herein is a crystalline pomalidomide ({drug4b}) having a structure corresponding substantially to the representative single-crystal X-ray structure depicted in Figures 19 and 20 of WO2013/126326. In one embodiment, provided herein is one embodiment of Form A having a structure corresponding substantially to the representative single- crystal X-ray structure depicted in Figures 19 and 20 of WO2013/126326. In one embodiment, the single X- ray data of a. crystalline pomalidomide provided herein may be used to simulate a representative XRPD pattern of crystalline pomalidomide, as depicted in Figure 21 of WO2013/126326. In one embodiment, provided herein is a crystalline pomalidomide ({drug4b}) characterized by one or more XRPD peaks (e.g., one, two, three, four, five, six, seven, eight, nine, or greater than nine peaks) selected from peaks located at the approximate positions as listed in Table 2.

Table 2:

| Peak No. | Approximate Peak Position (°2θ) | Relative Peak Intensity |
|---|---|---|
| C | 12.379 | 50.843 |
| I | 12.791 | 19.074 |
| D | 14.109 | 44.921 |
| H | 17.028 | 29.767 |
| B | 17.489 | 60.259 |
| G | 24.503 | 36.754 |
| F | 24.904 | 37.803 |
| A | 25.738 | 100.000 |
| E | 28.254 | 43.584 |

In one embodiment, provided herein is a crystalline pomalidomide ({drug4b}) having an IR spectrum, corresponding substantially to the representative IR spectrum depicted in Figure 22 of WO2013/126326. In one embodiment, provided herein is one embodiment of Form A having an IR spectrum corresponding substantially to the representative IR spectrum depicted in Figure 22 of WO2013/126326. In one embodiment, provided herein is a crystalline pomalidomide ({drug4b}) having a DVS isotherm plot corresponding substantially to the representative DVS isotherm plot depicted in Figures 23 and 24 of WO2013/126326. In one embodiment, provided lierein is one embodiment of Form A having a DVS isotherm plot corresponding substantially to the representative DVS isotherm plot depicted in Figures 23 and 24 of WO2013/126326. In certain embodiments, provided herein is an anhydrous crystal form comprising pomalidomide, characterized by one or more XRPD peaks as provided herein elsewhere. In certain embodiments, provided herein is an anhydrous crystal form comprising pomalidomide, having one or more representative XRPD patterns as provided herein elsewhere. In certain embodiments, provided herein is an unsolvated crystal form comprising pomalidomide, characterized by one or more XRPD peaks as provided herein elsewhere. In certain embodiments, provided herein is an unsolvated crystal form comprising pomalidomide, having one or more representative XRPD patterns as provided herein elsewhere. In certain embodiments, provided herein is a crystalline pomalidomide (e.g., Form A) that is substantially physically pure. For example, in certain embodiments, a crystalline pomalidomide provided herein (e.g., Form A) is substantially free of other solid forms, including other solid forms comprising pomalidomide (e.g., crystal forms or amorphous forms comprising pomalidomide), as determined, e.g., using methods of solid-state analysis including, but not limited to, diffraction analysis, thermal analysis, elemental combustion analysis and/or spectroscopic analysis. In certain embodiments, provided herein is a crystalline pomalidomide (e.g., Form A) that is substantially chemically pure. For example, in certain embodiments, a crystalline pomalidomide provided herein (e.g., Form A) is substantially free of other chemical compounds, as determined, e.g., using methods of chemical analysis including, but not limited to, mass spectrometry analysis, spectroscopic analysis, thermal analysis, elemental combustion analysis, and/or chromatographic analysis. In certain embodiments, a crystalline pomalidomide provided herein (e.g., Form A) is substantially free of solvent. In certain embodiments, a crystalline pomalidomide provided herein (e.g., Form A) is substantially free of water. In certain embodiments, provided herein is a crystalline pomalidomide (e.g., Form A) that is substantially chemically pure and substantially physically pure. In one embodiment, provided herein is a mixture comprising a crystalline pomalidomide (e.g., Form A) and an amorphous pomalidomide. In one embodiment, provided herein is a solid form of pomalidomide comprising a crystalline pomalidomide (e.g., Form A) and amorphous pomalidomide. In certain embodiment, provided herein is a solid form of pomalidomide comprising a crystalline pomalidomide (e.g., Form A) and amorphous pomalidomide, which is about 1%, about 2%, about 3%, about 4%, about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 96%, about 97%, about 98%, or about 99% by weight crystalline. In certain embodiment, provided herein is a solid form of pomalidomide comprising a crystalline pomalidomide (e.g., Form A) and amorphous pomalidomide, which is greater than about 1%, greater than about 2%, greater than about 3%, greater than about 4%, greater than about 5%, greater than about 10%, greater than about 15%, greater than about 20%, greater than about 25%, greater than about 30%, greater than about 35%, greater than about 40%, greater than about 45%, greater than about 50%, greater than about 55%, greater than about 60%, greater than about 65%, greater than about 70%, greater than about 75%, greater than about 80%, greater than about 85%, greater than about 90%, greater than about 95%, greater than about 96%, greater than about 97%, greater than about 98%, or greater than about 99% by weight crystalline. In certain embodiment, provided herein is a sol id form of pomal idomide comprising a crystalline pomalidomide (e.g.. Form A) and amorphous pomalidomide, which is greater than about 95% by weight

crystalline. In certain embodiment, provided herein is a solid form of pomalidomide comprising a crystalline pomalidomide (e.g., Form A) and amorphous pomalidomide, which is greater than about 97%> by weight crystalline. In certain embodiment, provided herein is a solid form of pomalidomide comprising a crystalline pomalidomide (e.g., Form A) and amorphous pomalidomide, which is greater than about 99% by weight crystalline. In certain embodiments, provided herein is a mixture of solid forms comprising a crystalline form provided herein (e.g., Form A) and one or more other crystalline form(s) of pomalidomide. In certain embodiments, provided herein is a mixture of solid forms comprising a crystalline form provided herein (e.g., Form A) and amorphous pomalidomide. In one embodiment, provided herein is a Form A that is greater than 50% by weight crystalline. In certain embodiments, Form A is greater than 50%, greater than 60%, greater than 70%, greater than 80%, greater than 85%, greater than 90%, greater than 95%, greater than 97%, greater than 98%», or greater than 99% by weight crystalline, In one embodiment, provided herein is a crystalline pomalidomide that is prepared by a method described herein. In one embodiment, provided herein is a. Form A that is prepared by a method described herein.

In certain embodiments, provided herein is pomalidomide in an amorphous form. In certain embodiments, provided herein are stable amorphous forms of pomalidomide (e.g., does not undergo significant physical form change after storage for a certain period of time or after formulation processing or handling). In certain embodiments, an amorphous form of pomalidomide can be obtained from certain aqueous and/or organic solvent systems, as exemplified herein elsewhere. In certain embodiments, an amorphous form of pomalidomide can be obtained by slurry methods, evaporation methods, cooling methods, and precipitation methods. In one embodiment, an amorphous form of pomalidomide provided herein is a stable amorphous form (e.g., does not undergo significant change in physical form after storage over a certain period of time, e.g., after 1, 2, 3, 4, 5, 6, 8, 10, 12, 16, 20, 24, 28, 32, 36, 40, 44, 48, 52, or more weeks, under conditions, including, but not limited to, humidity, heat, exposure to light, or combinations thereof; or after formulation processing or handling; as measured by a method for characterizing solid forms described herein). In one embodiment, provided herein is a solid form of pomalidomide that is substantially amorphous (i.e., an amorphous form), as indicated by, e.g., X-ray powder diffraction measurements. In one embodiment, a representative XRPD pattern of an amorphous form is provided in Figure 18 of WO2013/126326. In certain embodiments, an amorphous form of pomalidomide is characterized by no sharp or distinguishable XRPD peaks. In some embodiments, an amorphous form of pomalidomide is characterized by a broad hump, In some embodiments, an amorphous form of pomalidomide is characterized by a broad hump with a global maximum at approximately 26 degrees 20. In some embodiments, an amorphous form of pomalidomide is characterized by a broad hump with a. global maximum at approximately 26 degrees 20, and optionally a local maximum at approximately 12 degrees 20. In certain embodiments, an amorphous form of pomalidomide provided herein is substantially physically pure. For example, in certain embodiments, an amorphous form is substantially free from other solid forms, including other solid forms comprising pomalidomide (e.g., crystal forms), as determined, e.g., using methods of solid-state analysis including, but not limited to, diffraction analysis, thermal analysis, elemental combustion analysis and/or spectroscopic analysis. In certain embodiments, an amorphous form provided herein is substantially chemically pure. For example, in certain embodiments, an amorphous form provided herein is substantially free from other chemical compounds, as determined, e.g., using methods of chemical analysis including, but not limited to, mass spectrometry analysis, spectroscopic analysis, thermal analysis, elemental combustion analysis, and/or chromatographic analysis. In certain embodiments, an amorphous form provided herein is substantially free of water. In certain embodiments, an amorphous form provided herein is substantially free of solvent. In certain embodiments, an amorphous form provided herein is substantially chemically pure and substantially physically pure. In one embodiment, provided herein is a mixture comprising an amorphous form comprising pomalidomide and one or more solid form(s) comprising pomalidomide. In one embodiment, provided herein is a solid form of pomalidomide comprising amorphous pomalidomide and one or more crystal form(s) of pomalidomide. In certain embodiment, provided herein is a solid form of pomalidomide comprising amorphous pomalidomide, wherein the solid form is about 50%, about 55%, about 60%, about 65%, about 70%, about 75%, about 80%, about 85%, about 90%, about 95%, about 96%, about 97%, about 98%, or about 99% by weight amorphous (e.g., noncrystalline). In certain embodiment, provided herein is a solid form of pomalidomide comprising amorphous pomalidomide, wherein the solid form is greater than about 50%, greater than about 55%, greater than about 60%, greater than about 65%, greater than about 70%, greater than about 75%, greater than about 80%, greater than about 85%, greater than about 90%, greater than about 95%, greater than about 96%, greater than about 97%, greater than about 98%, or greater than about 99% by weight amorphous. In certain embodiment, provided herein is a solid form of pomalidomide comprising amorphous pomalidomide, wherein the solid form is greater than about 90%, greater than about 95%, greater than about 96%, greater than about 97%, greater than about 98%, or greater than about 99%> by weight amorphous. In certain embodiments, provided herein is a mixture of solid forms of pomalidomide comprising amorphous pomalidomide. In one embodiment, provided herein is an amorphous pomalidomide that is greater than 50% by weight amorphous. In certain embodiments, an amorphous pomalidomide provided herein is greater than 50%, greater than 60%, greater than 70%, greater than 80%, greater than 85%, greater than 90%, greater than 95%, greater than 97%, greater than 98%, or greater than 99% by weight amorphous. In one embodiment, provided herein is an amorphous form of pomalidomide that is prepared by a method described herein.

Provided herein are methods of treating, preventing, and/or managing various diseases or disorders using a solid form provided herein. In certain embodiments, provided are methods of treating, managing, and preventing various diseases and disorders, which comprise administering to a. patient in need of such treatment, prevention or management a therapeutically or propliylacticaily effective amount of a solid form provided herein. Examples of diseases and disorders are described herein, Examples of diseases or disorders include, but are not limited to: cancer, including hematologic cancer or solid tumor, for example, multiple myeloma, leukemia, lymphoma, sarcoma, prostate cancer, or lung cancer (e.g., small ceil lung cancer); scleroderma; amyloidosis; pain, for example, complex regional pain syndrome (CRPS); myelofibrosis; myeloproliferative disease, for example, MMM; myelodysplastic syndromes (MDS); diffuse systemic sclerosis; macular degeneration; a skin disease; a pulmonary disorder; an asbestos-related disorder; a parasitic disease; an immunodeficiency disorder; a CNS disorder; a CNS injury; atherosclerosis; hemoglobinopathy; anemia, for example, sickle cell anemia; an inflammatory disease; an autoimmune disease; a viral disease; a genetic disease; an allergic disease; a bacterial disease; an ocular neovascular disease; a choroidal neovascular disease; a retina neovascular disease; rubeosis; a sleep disorder; disorders associated with angiogenesis; and TNFa related disorders. Examples of cancer and precancerous conditions include, but are not limited to, those described in U.S. patent nos. 6,281,230 and 5,635,517 to Muller et ah, in various U.S. patent publications to Zeidis, including publication nos. 2004/0220144A1, published November 4, 2004 (Treatment of Myelodysplastic Syndrome); 2004/0029832A 1, published February 12, 2004 (Treatment of Various Types of Cancer); and 2004/0087546, published May 6, 2004 (Treatment of Myeloproliferative Diseases). Examples also include those described in WO 2004/103274, published December 2, 2004. All of these references are incorporated herein in their entireties by reference. Other examples of diseases or disorders include, but are not limited to, those described in U.S. Patent Nos. 5,712,291, 7,393,863, and 7,863,297; and U.S. Patent Application Publication Nos. 2005/0143420, 2006/0166932, 2006/0188475,2007/0048327, 2007/0066512, 2007/0155791, 2008/0051431, 2008/0317708, 2009/0087407,2009/0088410, 2009/0148853, 2009/0232776, 2009/0232796, 2009/0317385, 2010/0098657, 2010/0099711, and 2011/0184025; all of which are incorporated herein by reference in their entireties. In one embodiment is provided a method of treating, preventing and/or managing a disease provided herein, comprising administering to a patient in need of such treatment, prevention and/or management a therapeutically or prophylactically effective amount of a solid form of pomalidomide as described herein and a therapeutically or prophylactically effective amount of a second active agent. Examples of second active agents include, but are not limited to, cytokines, corticosteroids, ribonucleotide reductase inhibitors, platelet inhibitors, all-trans retinoic acids, kinase inhibitors, topoisomerase inhibitors, farnesyl transferase inhibitors, antisense oligonucleotides, vaccines, anti-cancer agents, anti-fungal agents, antiinflammatory agents, immunosuppressive or myelosuppressive agents, and conventional therapies for MPD (e.g., prednisone). Specific second active agents include, but are not limited to, 2-methoxyestradiol, telomestatin, inducers of apoptosis in mutiple myeloma cells (such as, for example, TRAIL), statins, semaxanib, cyclosporin, etanercept, doxycycline, bortezomib, oblimersen (Genasense*), remicade, docetaxel, celecoxib, rnelphalan, dexamethasone (Decadron®), steroids, gemcitabine, cisplatinum, temozolomide, etoposide, cyclophosphamide, temodar, carboplatin, procarbazine, gliadei, tamoxifen, topotecan, methotrexate, Ansa*', taxol, taxotere, fluorouracil, leucovorin, irinotecan, xeloda, CPT-11, interferon alpha, pegylated interferon alpha (e.g., PEG 1NT ON-A), capecitabine, cisplatin, thiotepa, fludarabine, carboplatin, liposomal daunorubicin, cytarabine, doxetaxoi, pacilitaxel, vinblastine, IL-2, GM-CSF, dacarbazine, vinorelbine, zoledronic acid, palmitronate, biaxin, busulphan, prednisone, bisphosphonate, arsenic trioxi.de, vincristine, doxorubicin (Doxif), pacfitaxel, ganciclovir, adriamycin, estramustine sodium phosphate (Emeyt®), sulindac, etoposide, and a mixture thereof. In one embodiment, specific second active agent is dexamethasone. Certain examples of cancer include, but are not limited to, cancers of the skin, such as melanoma; lymph node; breast; cervix; uterus; gastrointestinal tract; lung; ovary; prostate; colon; rectum; mouth; brain; head and neck; throat; testes; kidney; pancreas; bone; spleen; liver; bladder; larynx; nasal passages; and AIDS-related cancers. The solid forms are also useful for treating cancers of the blood and bone marrow, such a s multiple myeloma and acute and chronic leukeniias, for example, lymphoblastic, myelogenous, lymphocytic, and myelocytic leukeniias. The solid forms provided herein can be used for treating, preventing, or managing either primary or metastatic tumors. Other cancers include, but are not limited to, advanced malignancy, amyloidosis, neuroblastoma, meningioma, hemangiopericytoma, multiple brain metastases, glioblastoma multi forms, glioblastoma, brain stem glioma, poor prognosis malignant brain tumor, malignant glioma, recurrent malignant glioma, anaplastic astrocytoma, anaplastic oligodendroglioma, neuroendocrine tumor, rectal adenocarcinoma, Dukes C & D colorectal cancer, unresectable colorectal carcinoma, metastatic hepatocellular carcinoma, Kaposi's sarcoma, karotype acute myeloblastic leukemia, chronic lymphocytic leukemia (CLL), Hodgkin's lymphoma, non-Hodgkin's lymphoma, cutaneous T-Cell lymphoma, cutaneous B-Celi lymphoma, diffuse large B~ Cell lymphoma, low grade follicular lymphoma, metastatic melanoma, focalized melanoma (including, but not limited to, ocular melanoma), malignant mesothelioma, malignant pleural effusion mesothelioma syndrome, peritoneal carcinoma, papillary serous carcinoma, gynecologic sarcoma, soft tissue sarcoma, cutaneous vasculitis, Langerhans cell histiocytosis, leiomyosarcoma, fibrodysplasia ossificans progressive, hormone refractory prostate cancer, resected high-risk soft tissue sarcoma, unresectable hepatocellular carcinoma, Waldenstrom's macroglobulinemia, smoldering myeloma, indolent myeloma, fallopian tube cancer, androgen independent prostate cancer, androgen dependent stage IV

non-metastatic prostate cancer, hormone-insensitive prostate cancer, chemotherapy-insensitive prostate cancer, papillary thyroid carcinoma, follicular thyroid carcinoma, medullary thyroid carcinoma, and leiomyoma. In a specific embodiment, the cancer is metastatic. In another embodiment, the cancer is refractory or resistance to chemotherapy or radiation. In one embodiment is provided a method of treating, preventing, or managing myeloproliferative disease (MPD), comprising administering to a patient in need of such treatment, prevention, or management a therapeutically or prophyketieaily effective amount of a solid form of pomalidomide as described herein. The embodiment encompasses the treatment, prevention or management of specific sub-types of MPD such as, but not limited to, polycythemia rubra vera (PRV), primary t!irornoboeythemia (PT), myelofibrosis with myeloid metaplasia. (MMM) and agnogenic myeloid metaplasia (AMM). in one embodiment, MPD includes: polycythemia rubra vera (PRV), primary thromobocythemia (PT), and agnogenic myeloid metaplasia (AMM). In a specific embodiment, MPD excludes leukemia. In one embodiment, particular types of MPD are MMM, PRV, PT, and AMM. In one embodiment, a solid form of pomalidomide is administered to patients who are refractor}' to conventional treatments for myeloproliferative diseases as well as treatments using thalidomide. As used herein, the term "refractory" means the patient's response to a MPD treatment is not satisfactory by clinical standards, e.g., showing no or little improvement of symptoms or laboratory findings. In one embodiment is provided a method of reversing, reducing, or avoiding an adverse effect associated with the administration of an active agent used to treat MPD in a patient, suffering from MPD, comprising administering to a patient in need thereof a therapeutically or prophylactically effective amount of a solid form of pomalidomide as described herein. Examples of active agents include, but are not limited to, the second active agents described herein. Examples of adverse effects associated with active agents used to treat MPD include, but are not limited to: conversion to acute leukemia; severe myelosuppression; gastrointestinal toxicity such as, but not limited to, early and late-forming diarrhea and flatulence; gastrointestinal bleeding; nausea; vomiting; anorexia; leukopenia; anemia; neutropenia; asthenia; abdominal cramping; fever; pain; loss of body weight; dehydration; alopecia; dyspnea; insomnia; dizziness; mucositis; xerostomia; mucocutaneous lesions; and kidney failure. In one embodiment, provided herein is a method of treating, preventing, or managing MPD, comprising administering to a patient (e.g., a human) a solid form of pomalidomide as described herein, before, during, or after transplantation therapy, In one embodiment, provided herein are pharmaceutical compositions, single unit dosage forms, and kits, comprising a solid form of pomalidomide as described herein, a second active ingredient, and/or blood or cells for transplantation therapy. For example, a kit may comprise a solid form of pomalidomide as described herein, stem cells for transplantation, an immunosuppressive agent, and an antibiotic or other drug. In one embodiment, provided herein is a method of modulating the differentiation of CD34+ stem, precursor, or progenitor ceils to a predominantly erythroid lineage, comprising administering to a patient an effective amount of a solid form of pomalidomide as described herein. In one embodiment, provided herein is a method of modulating differentiation of a CD34+ cell to an erythroid lineage comprising differentiating said cell under suitable conditions and in the presence of pomalidomide. The CD34 f cell may be any stem, progenitor, or committed cell able to differentiate into an eiythroid cell. Such cells may be totipotent or pluripotent, or may be committed to a hematopoietic lineage. The CD34+ ceil may be derived from any source; in particular embodiments, "embryonic-like" stem cells derived from the placenta. For a description of such embryonic-like stem cells and methods of obtaining them, see U.S. application publication no. US 2003/0180269 A1, published September 25, 2003, which is incorporated by reference herein in its entirety. Other CD34 cells useful for the methods provided herein include stem cells obtained from any tissue (such as, for example, hematopoietic stem ceils or embryonic stem cells) and non-committed progenitor cells from any tissue. Such CD34+ cells may be heterologous or autologous with reference to the intended recipient, when such cells, the differentiation of which is modulated according to the methods provided herein, are used to treat anemia or a hemoglobinopath. Differentiation of the CD 34"' cells may typically take place over the course of 3-6 days. In in vitro assays in which CD34+ cells are cultured in the presence of pomalidomide, changes in gene expression indicating differentiation along an erythroid pathway may be evident by the third day of culture. In one embodiment, erytbroid-specific gene expression is significantly increased, and phenotypic characteristics of erythroid cells are present in the CD34+ cells by day 6 of culture. In one embodiment, therefore, CD34 cells may be cultured in vitro in the presence of pomalidomide, for a period of days sufficient for erythroid-speeific gene expression, particularly fetal hemoglobin gene expression, and/or cell characteristics to appear. In various embodiments, the CD34+ ceils may be cultured for 3, 6, 9, or 12 days, or more. A solid form of pomalidomide or a solution thereof may be introduced once at the start of culture, and culturing continued until differentiation is substantially complete, or for 3, 6, 9, 12 or more days. Alternatively, a solid form of pomalidomide or a solution thereof may be administered to a culture of CD341 cells a plurality of times during culture. The CD34" cells may be cultured and differentiated in the presence pomalidomide. In one embodiment, a solid form of pomalidomide maybe used as a solution at any concentration from 0.01 μM to 10 mM. In certain embodiments, the concentration is between 0.01 μM and 10 μM. In addition to differentiating CD34" cells in vitro, such cells may be differentiated within an individual, in vivo. In one embodiment, such an individual is a mammal, for example a human. As with in vitro differentiation of CD34; cells, CD34" cells within an individual may be differentiated by administration of a solid form of pomalidomide as described herein. Such administration may be in the form of a single dose. Alternatively, the individual may be administered a solid form of pomalidomide as described herein a plurality of times. Such administration may be performed, for example, over a period of 3, 6, 9, 12, or more

days. Where differentiation of CD34+ cells is to be accomplished in vivo, differentiation may be accomplished using pomalidomide alone, or a combination with a second active agent. For example, for an individual having a hemoglobinopathy such as sickle cell anemia or a thalassemia, who has a higher than normal level of SCF and/or erythropoietin, in vivo differentiation may be accomplished by administration of a solid form of pomalidomide as described herein. Conversely, where an individual suffers an anemia that is the result of, or is characterized by, a lower-than-normal level of erythropoietic cytokines (e.g., SCF or erythropoietin), such cytokines may be administered along with, or prior to, administration of a solid form of pomalidomide. For example, an individual suffering from chemotherapy-induced anemia may be administered one or more cytokines (e.g., a combination of SCF, Flt-3L, and/or IL-3) for, e.g., 3-6 days, followed by administration for, e.g., 3-6 days, of the solid form of pomalidomide, particularly with SCF and erythropoietin, in an amount sufficient to cause a detectable increase in fetal hemoglobin expression in CD34+ cells of said individual Alternatively, CD34+ cells may be contacted with one or more cytokines in vitro (e.g., SCF, Flt-3L, and/or IL-3) for, e.g., 3-6 days, followed by administration of the cells to an individual, along with SCF and erythropoietin in an amount sufficient to cause a detectable increase in fetal hemoglobin expression in the CD34+ cells. Such administration may be performed a single time or multiple times, and any one or more of such administrations may be accompanied by the administration of a solid form of pomalidomide, a second active agent, or a combination thereof. In one embodiment is provided a method of inducing one or more genes associated with or essential for erythropoiesis or hematopoiesis, comprising contacting an hematopoietic stem, progenitor or precursor cell with pomalidomide in the presence of erythropoietin and stem cell factor, wherein said pomalidomide is present in a sufficient amount to cause said hematopoietic stem, progenitor or precursor cell to express one or more genes encoding fetal hemoglobin. In a specific embodiment, said hematopoietic stem, progenitor or precursor cell is a CD34" cell. In another specific embodiment, said one or more genes associated with or essential for erythropoiesis or hematopoiesis are genes encoding Kruppel-like factor I erythroid; rhesus blood group-associated glycoprotein; glycophorin B; integrin alpha 2b; erythroid-assoeiated factor; giycophorin A; Kell blood group precursor; hemoglobin a2; solute carrier 4, anion exchanger; carbonic anhydrase hemoglobin γA; hemoglobin yG; hemoglobin εi ; or any combination of the foregoing. In some embodiments, the CD34 " cells are additionally differentiated, either in vivo or in vitro, in the presence of one or more cytokines. Cytokines useful to direct CD34 cells along an erythroid differentiation pathway include, but are not limited to, erythropoietin (Epo), TNFa, stem cell factor (SCF), Flt-3L, and granulocyte macrophage-colony stimulating factor (GM-CSF). Epo and SCF are known to be erythropoietic cytokines. Thus, in one embodiment, CD341' cells are differentiated in the presence of Epo or SCF. In another embodiment, the CD34+ cells are differentiated in the presence of Epo and SCF. In another embodiment, the CD34+ cells are differentiated in the presence of a combination of TNFa, SCF, Flt-3L, and/or GM-CSF. In another embodiment, said cells that are differentiated are one or more cells in cell culture. In another embodiment, said cells that are differentiated are cells within an individual. In an embodiment of in vitro differentiation, one or more of Epo, TNFa, SCF, Fit-3L and GM-CSF is contacted with pomalidomide. In an embodiment of w vivo differentiation, one or more of Epo, TNFa, SCF, FU-3L and GM-CSF is administered to an individual in the same treatment regimen a the solid form of pomalidomide as provided herein. The cytokines used in the methods provided herein may be naturally-occurring cytokines, or may be an artificial derivative or analog of the cytokines. For example, analogs or derivatives of erythropoietin that may be used in combination with a solid form or compound provided herein include, but are not limited to, Aranesp " and Darbopoietin. Cytokines used may be purified from natural sources or recombinantly produced. Examples of recombinant cytokines that may be used in the methods provided herein include filgrastim, or recombinant granulocyte-colony stimulating factor (G-CSF), which is sold in the United States under the trade name Neupogen® (Amgen, Thousand Oaks, CA); sargramostim, or recombinant GM-CSF, which is sold in the United States under the trade name Leukine© (Immunex, Seattle, WA); recombinant Epo, which is sold in the United States under the trade name Epogen® (Amgen, Thousand Oaks, CA); and methionyl stem cell factor (SCF), which is sold in the United States under the trade name AncestimrM. Recombinant and mutated forms of GM-CSF can be prepared as described in U.S. patent nos. 5,391,485; 5,393,870; and 5,229,496; all of which are incorporated herein by reference. Recombinant and mutated forms of G-CSF can be prepared as described in U.S. patent nos, 4,810,643; 4,999,291; 5,528,823; and 5,580,755; all of which are incorporated herein by reference. Other cytokines may be used which encourage the survival and/or proliferation of hematopoietic precursor ceils and immunologically active poietic cells in vitro or in vivo, or which stimulate the division and differentiation of committed erythroid progenitors in cells in vitro or in vivo. Such cytokines include, but are not limited to: interleukins, such as IL-2 (including recombinant IL-ii ("rIL2") and canarypox. IL -10, IL- 12, and IL- 18: interferons, such as interferon alfa~2a, interferon alfa-2b, interferon alfa-n 1, interferon alfa-n3, interferon beta-I a, and interferon gamma-I b; and G-C8F. When administered to a person having a hemoglobinopathy, a solid form of pomalidomide as described herein, particulariy in the presence of Epo, particularly in the presence of the combination of TNFa, SCF, Flt-3L and GM-CSF, or more particularly in the presence of Epo and SCF, induces the production of erythrocytes, and the production of fetal hemoglobin as well as the production of AHSP. As noted above, cytokines used may include purified or recombinant forms, or analogs or derivatives of specific cytokines. A solid form of pomalidomide as described herein may also be administered in conjunction with one or more second compounds known to have, or suspected of having, a beneficial

effect on a hemoglobinopathy. In this context, "beneficial effect" means any reduction of any symptom of a hemoglobinopathy or anemia. For example, with specific reference to the hemoglobinopathy sickle cell anemia, the second compound can be a compound, other than a pomalidomide or a derivative thereof, that is known or suspected to induce the production of fetal hemoglobin. Such compounds include hydroxyurea, and butyrates or butyrate derivatives. The second compound may also be a compound thai relaxes blood vessels, such as nitrous oxide, e.g., exogenously-applied or administered nitrous oxide. The second compound may also be a compound that binds directly to hemoglobin S, preventing it from assuming the sickle-inducing conformation. For example, the plant extract known as HEMOX1N™ (NIPRISAN™; see United States Patent No. 5,800,819), which is an extract of a mixture of about 12 to about 17 parts by weight of Piper guineense seeds, from about 15 to about 19 parts by weight of Pterocarpus osun stem, from about 12 to about 18 parts by weight of Eugenia caryophyllata fruit, and from about 25 to about 32 parts by weigh t of Sorghum bicolor leaves, and optionally 15-22 parts by weight potash, wherein the mixture is extracted with cold water, has antisickling activity. The second compound may also be a Gardos channel antagonist. Examples of Gardos channel antagonists include clotrimazole and triaryl methane derivatives. The second compound may also be one that reduces red blood cell adhesion, thereby reducing the amount of clotting pervasive in sickle cell anemia. Other hemoglobinopathies may be treated with a second compound known or suspected to be efficacious for the specific condition. For example, β thalassemia may additionally be treated with the second compound Deferoxamine, an iron chelator that helps prevent the buildup of iron in the blood, or folate (vitamin B9). Thalassemia or sickle ceil anemia may also be treated with protein C as the second compound (U.S. Patent No. 6,372,213). There is some evidence that herbal remedies can ameliorate symptoms of hemoglobinopathies, e.g., thalassemia; such remedies, and any of the specific active compounds contained therein, may also be used as a second compound in the method provided herein. See, e.g., Wu Zhikui ei al. "The Effect of Bushen Shengxue Fang on β-thalassemia at the Gene Level," Journal of Traditional Chinese Medicine 18(4): 300-303 (1998); U.S. Patent No. 6,538,023 "Therapeutic Uses of Green Tea Polyphenols for Sickle Cell Disease". Treatment of autoimmune hemolytic anemia can include corticosteroids as the second compound. Second compounds that are proteins may also be derivatives or analogs of other proteins. Such derivatives may include, but are not limited to, proteins that lack carbohydrate moieties normally present in their naturally occurring forms (e.g., nonglycosylated forms), pegylated derivatives, and fusion proteins, such as proteins formed by fusing IgG1 or IgG3 to the protein or active portion of the protein of interest. See, e.g., Penichei, MX. and Morrison, S.L., J. Immunol. Methods 248:91 - 101 (2001). Cytokines and/or other compounds potentially useful in the treatment of anemi or a hemoglobinopathy may be administered at the same time as pomalidomide or a derivative thereof. In this regard, the cytokines or other compounds maybe administered as formulations separate from a solid form of pomalidomide, or, where possible, may be compounded with a solid form of pomalidomide for administration as a single pharmaceutical composition. Alternatively, the cytokines, the other compounds, or both, may be administered separately from a solid form of pomalidomide used in the methods provided herein, and may follow the same or different dosing schedules. In one embodiment, a solid form of pomalidomide, cytokines, and/or any other compound useful to treat anemia, or a hemoglobinopathy, are administered at the same time, but in separate pharmaceutical formulations for flexibility in administration. In addition to the treatment combinations outlined herein, the treated individual may be given transfusions. Such transfusions may be of blood, for example matched blood, or of a blood substitute such as Hemospan™ or Hemospan™ PS (Sangart). In any of the treatment combinations described herein, the treated individual is eukaryotie. In one embodiment, the treated individual is a. mammal, for example a human. The methods described herein may be used to treat any anemia, including anemia resulting from a hemoglobinopathy. Hemoglobinopathies and anemias treatable by the methods provided herein may be genetic in origin, such as sickle-cell anemia, or thalassemias. The hemoglobinopathy may be due to a disease, such as cancer, including, but not limited to, cancers of the hematopoietic or lymphatic systems. Other conditions treatable using the methods provided herein include Hypersplenism, splenectomy, bowel resection, and bone marrow infiltration. The methods described herein may also be used to treat anemia resulting from the deliberate or accidental introduction of a poison, toxin, or drug. For example, anemias resulting from cancer chemotherapies may be treated using the methods and solid forms provided herein. As such, the methods described herein may be employed when anemia or a hemoglobinopathy is the primary condition to be treated, or is a secondary condition caused by an underlying disease or treatment regimen. In one embodiment, the diseases or disorders are various forms of ieukemias such as chronic lymphocytic leukemia, chronic myelocytic leukemia, acute lymphoblastic leukemia, acute myelogenous leukemia, and acute myeloblastic leukemia, including Ieukemias that are relapsed, refractory, or resistant, as disclosed in U.S. publication no. 2006/0030594, published February 9, 2006, which is incorporated in its entirety by reference. The term 'leukemia" refers malignant neoplasms of the blood-forming tissues. The leukemia includes, but is not limited to, chronic lymphocytic leukemia, chronic myelocytic leukemia, acute lymphoblastic leukemia, acute myelogenous leukemia, and acute myeloblastic leukemia. The leukemia can be relapsed, refractory or resistant to conventional therapy. The term "relapsed" refers to a situation where patients who have had a remission of leukemia after therapy have a return of leukemia cells in the marrow and a decrease in normal blood cells. The term "refractory or resistant" refers to a circumstance where patients, even after intensive treatment, have residual leukemia cells in their marrow. In another embodiment, the diseases or disorders are various types of lymphomas, including on-Hodgkin's lymphoma (NHL). The term "lymphoma" refers a

heterogenous group of neoplasms arising in the reticuloendothelial and lymphatic systems. "NHL" refers to malignant monoclonal proliferation of lymphoid cells in sites of the immune system, including lymph nodes, bone marrow, spleen, fiver, and gastrointestinal tract. Examples of NHL include, but are not limited to, mantle cell lymphoma (MCL), lymphocytic lymphoma of intermediate differentiation, intermediate lymphocytic lymphoma (ILL), diffuse poorly differentiated lymphocytic lymphoma (PDL), centrocytic lymphoma., diffuse small-cleaved cell lymphoma (DSCCL), follicular lymphoma, and any type of the mant le cell lymphomas that can be seen under the microscope (nodular, diffuse, bias tic and mentle zone lymphoma). Examples of diseases and disorders associated with, or characterized by, undesired angiogenesis include, but are not limited to, inflammatory diseases, autoimmune diseases, viral diseases, genetic diseases, allergic diseases, bacterial diseases, ocular neovascular diseases, choroidal neovascular diseases, retina neovascular diseases, and rubeosis (neovascularization of the angle). Specific examples of the diseases and disorders associated with, or characterized by, undesired angiogenesis include, but are not limited to, arthritis, endometriosis, Crohn’s disease, heart failure, advanced heart failure, renal impairment, endotoxemia, toxic shock syndrome, osteoarthritis, retrovirus replication, wasting, meningitis, silica- induced fibrosis, asbestos-induced fibrosis, veterinary disorder, malignancy-associated hypercalcemia, stroke, circulatory shock, periodontitis, gingivitis, macrocytic anemia, refractor}’ anemia, and 5q-deietion syndrome. Other disease or disorders treated, prevented, or managed include, but not limited to, viral, genetic, allergic, and autoimmune diseases. Specific examples include, but are not limited to, HIV, hepatitis, adult respiratory distress syndrome, bone resorption diseases, chronic pulmonary inflammatory diseases, dermatitis, cystic fibrosis, septic shock, sepsis, endotoxic shock, hemodynamic shock, sepsis syndrome, post ischemic reperfusion injur)’, meningitis, psoriasis, fibrotic disease, cachexia, graft versus host disease, graft rejection, autoimmune disease, rheumatoid spondylitis, Crohn’s disease, ulcerative colitis, inflammatory-bowel disease, multiple sclerosis, systemic lupus erythrematosus, ENL in leprosy, radiation damage, cancer, asthma, or hyperoxic alveolar injury. In certain embodiments, a solid form provided herein, or a composition comprising a solid form provided herein, is administered orally, parenterally, topically, or mucosaliy. Examples of such dosage forms can be found infra. In certain embodiments, a solid form provided herein, or a composition comprising a solid form provided herein, is administered at a. dosing frequency of once, twice, thrice, or four times daily. In certain embodiments, solid form provided herein, or a composition comprising a solid form provided herein, comprises pomalidomide in an amount of from about 0.1 to about 100 mg, from about 0.5 to about 50 mg, from, about 0.5 to about 25 mg, from about 1 mg to about 10 mg, from about 0.5 to about 5 mg, or from about 1 mg to about 5 mg. In certain embodiments, provided herein is a single unit dosage form suitable for oral administration to a human comprising: an amount equal to or greater than about 1, 2, 3, 4, or 5 mg of a solid form of pomalidomide provided herein; and a pharmaceutically acceptable excipient. In one embodiment, the amount of the active ingredient is about 0,5 mg.

In another embodiment, the amount of the active ingredient is about 1 mg. In another embodiment, the amount of the active ingredient is about 2 mg. In another embodiment, the amount of the active ingredient is about 4 mg. In one embodiment, the second active agent is administered intravenously or subcutaneously and once or twice daily, once every other day, once every week, once every two weeks, or once eveiy three weeks, in an amount of from about 1 to about 1000 mg, from about 5 to about 500 mg, from about 10 to about 350 mg, or from about 50 to about 200 mg. In one embodiment, the second active agent is administered orally and once or twice daily, once every other day, once every week, once even,’ two weeks, or once every three weeks, in an amount of from about 1 to about 1000 mg, from about 5 to about 500 mg, from about 10 to about 350 mg, from about 10 to about 200 mg, from about 10 to about 100 mg, or from about 20 to about 50 mg. In specific embodiments, the second active agent is administered once eveiy week in an amount of about 40 mg. The specific amount of the second active agent will depend on the specific agent used, the type of disease being treated or managed, the severity and stage of disease, and the amount(s) of compounds provided herein and any optional additional active agents concurrently administered to the patient. As discussed elsewhere herein, also encompassed is a method of reducing, treating and/or preventing adverse or undesired effects associated with conventional therapy including, but not limited to, surgery, chemotherapy, radiation therapy, hormonal therapy, biological therapy and immunotherapy. Compounds provided herein and other active ingredients can be administered to a patient prior to, during, or after the occurrence of the adverse effect associated with conventional therapy.

In certain embodiments, the prophylctic or therapeutic agents provided herein are cyclically administered to a patient. Cycling therapy involves the administration of an active agent for a period of time, followed by a rest (i.e., discontinuation of the administration) for a period of time, and repeating this sequential administration. Cycling therapy can reduce the development of resistance to one or more of the therapies, avoid or reduce the side effects of one of the therapies, and/or improve the efficacy of the treatment. Consequently, in one embodiment, a compound provided herein is administered daily in a single or divided doses in a four to six week cycle with a rest period of about a week or two weeks. Cycling therapy further allows the frequency, number, and length of dosing cycles to be increased. Thus, another embodiment encompasses the administration of a compound provided herein for more cycles than are typical when it is administered alone. In yet another embodiment, a compound provided herein is administered for a greater number of cycles than wo uld typically cause dose-limiting toxicity in a patient to whom a second active ingredient is not also being administered. In one embodiment, a compound provided herein is administered daily and continuously for three or four weeks at a dose of from about 0.1 mg to about 5 mg per day, followed by a rest of one or two weeks. In other

embodiments, the dose can be from about 1 mg to about 5 mg per day (e.g., 1, 2, 3, or 4 mg day), given on Days 1-21 of each 28-day cycle until disease progression, followed by a rest of 7 days on Days 22-28 of each 28-day cycle, for example, in patients with relapsed and refractory multiple myeloma who are refractory to their last myeloma therapy and have received at least 2 prior therapies that included lenaiidomide and bortezomib. In one embodiment, a compound provided herein and a second active ingredient are administered orally, with administration of the compound provided herein occurring 30 to 60 minutes prior to the second active ingredient, during a cycle of four to six weeks. In anoiher embodiment, the combination of a compound provided herein and a second active ingredient is administered by intravenous infusion over about 90 minutes every cycle. In one embodiment, a compound provided herein is administered at a dose of about 4 mg per day given on Days 1 -21, followed by a rest of 7 days on Days 22-28 of each 28-day cycle, alone or in combination with low dose dexamethasone (e.g., 40 mg/day given on Days 1, 8, 15 and 22 of each 28-day cycle), for example, in patients with relapsed and refractory multiple myeloma who are refractory to their last myeloma therapy and have received at least 2 prior therapies that included lenaiidomide and bortezomib.

Pharmaceutical compositions can be used in the preparation of single unit dosage forms comprising one or more solid forms provided herein. In one embodiment, provided herein are pharmaceutical compositions and dosage forms comprising one or more solid forms comprising a compound provided herein, or a p armaceutically acceptable salt, solvate (e.g., hydrate), stereoisomer, co-crystal, clathrate, or prodrug thereof. Pharmaceutical compositions and dosage forms provided herein can further comprise one or more pharmaceutically acceptable excipients or carriers. In some embodiments, pharmaceutical compositions and dosage forms provided herein can also comprise one or more additional active ingredients. Examples of optional second, or additional, active ingredients are disclosed herein elsewhere. In one embodiment, single unit dosage forms provided herein are suitable for oral, parenteral (e.g., subcutaneous, intravenous, bolus injection, intramuscular, or intraarterial), topical (e.g., eye drops or other ophthalmic preparations), mucosal (e.g., nasal, sublingual, vaginal, buccal, or rectal), or transdermal administration to a patient. Examples of dosage forms include, but are not limited to: tablets; caplets; capsules, such as soft elastic gelatin capsules or hard gelatin capsules; cachets; troches; lozenges; dispersions; suppositories; powders; aerosols (e.g., nasal sprays or inhalers); gels; liquid dosage forms suitable for oral or mucosal administration to a patient, including suspensions (e.g., aqueous or non-aqueous liquid suspensions, oil-in-water emulsions, or water-in-oil liquid emulsions), solutions, and elixirs; liquid dosage forms suitable for parenteral admini tration to a patient; eye drops or other ophthalmic preparations suitable for topical administration; and sterile solids (e.g., crystalline or amorphous solids) that can be reconstituted to provide liquid dosage forms suitable for parenteral administration to a patient. In one embodiment, the single dosage forms provided herein are tablets, caplets, or capsules comprising one or more solid forms provided herein. In one embodiment, the single dosage forms provided herein are tablets or capsules comprising one or more solid forms provided herein. The composition, shape, and type of dosage forms will typically vary depending on their use. For example, a dosage form used in the acute treatment of a disease may contain larger amounts of one or more of the active ingredients it comprises than a dosage form used in the chronic treatment of the same disease. Similarly, a parenteral dosage form may contain smaller amounts of one or more of the active ingredients it comprises than an oral dosage form used to treat the same disease. These and other ways in which specific dosage forms are used will vary from one another will be readily apparent to those skilled in the art. See, e.g., Remington’s Pharmaceutical Sciences, 18th ed., Mack Publishing, Easton PA (1990). In one embodiment, pharmaceutical compositions and dosage forms comprise one or more excipients or carriers. Suitable excipients are known to those skilled in the art of pharmacy, and non-limiting examples of suitable excipients are provided herein. Whether a particular excipient is suitable for incorporation into a pharmaceutical composition or dosage form depends on a variety of factors known in the art. including, but not limited to, the way in which the dosage form will be administered to a patient. For example, oral dosage forms such as tablets may contain excipients not suited for use in parenteral dosage forms. The suitability of a particular excipient may also depend on the specific active ingredients in the dosage form. For example, the decomposition of some active ingredients may be accelerated by some excipients such as lactose, or when exposed to water. Active ingredients that comprise primary or secondary amines are particularly susceptible to such accelerated decomposition. Consequently, in one embodiment, provided are pharmaceutical compositions and dosage forms that contain little, if any, lactose or other mono- or di-saccharides. As used herein, the term "lactose-free" means that the amount of lactose present, if any, is insufficient to substantially increase the degradation rate of an active ingredient. Lactose-free compositions provided herein can comprise excipients which are known in the art and are listed in the U.S. Pharmacopeia (USP) 25-NF20 (2002), which is incorporated herein in its entirety. Also provided are anhydrous pharmaceutical compositions and dosage forms comprising active ingredient(s), since water may facilitate the degradation of some compounds. Anhydrous pharmaceutical compositions and dosage forms can be prepared using anhydrous or low moisture containing ingredients and low moisture or low humidity conditions. An anhydrous pharmaceutical composition should be prepared and stored such that its anhydrous nature is maintained. Accordingly, in one embodiment, anhydrous compositions are packaged using materials known to prevent exposure to water such that they can be included in suitable formulary kits. Examples of suitable packaging include, but are not limited to, hermetically sealed foils, plastics, unit dose containers (e.g., vials), blister packs, and strip packs. Also provided are pharmaceutical compositions and dosage forms that comprise one or

more compounds that reduce the rate by which an active ingredient will decompose. Such compounds, which are referred to herein as "stabilizers," include, but are not limited to, antioxidants such as ascorbic acid, pH buffers, or salt buffers. Like the amounts and types of excipients, the amounts and specific types of active ingredients in a dosage form may differ depending on factors such as, but not limited to, the route by which it is to be administered to patients. In one embodiment, dosage forms comprise the active ingredient or solid form of pomalidomide provided herein in an amount of from about 0.10 to about 10 mg, or from about 0.10 to about 5 mg. In other embodiments, dosage forms comprise a compound provided herein in an amount of about 0.1, 0.2, 0.3, 0.4, 0.5, 0.6, 0.7, 0.8, 0.9, 1, 2, 2.5, 3, 4, or 5 mg. in other embodiments, dosage forms comprise a second active ingredient in an amount from about 1 mg to about 1000 mg, from about 5 mg to about 500 mg, from about 10 mg to about 350 mg, from about 5 mg to about 250 mg, from about 5 mg to about 100 mg, from about 10 mg to about 100 mg, from about 10 mg to about 50 mg, or from about 50 mg to about 200 mg. In one embodiment, the specific amount of the second active agent will depend on the specific agent used, the diseases or disorders being treated or managed, and the amount(s) of a compound provided herein, and any optional additional active agents concurrently administered to the patient. In particular embodiments, provided herein is a p armaceutical composition comprising a solid form of pomalidomide provided herein and a pharmaceutically acceptable excipient or carrier. In particular embodiments, provided herein is a pharmaceutical composition comprising a crystalline pomalidomide provided herein and a pharmaceutically acceptable excipient or carrier. In particular embodiments, provided herein is a pharmaceutical composition comprising an amorphous pomalidomide pro vided herein and a pharmaceutically acceptable excipient or carrier. Exemplar embodiments of formulations of pomalidomide are described in, for example, U.S. Patent Nos. 5,635,517, 6,335,349, 6,316,471, 6,476,052, 7,041,680, and 7,709,502; and U.S. Patent Application Publication o. 2011/0045064; the entireties of which are incorporated herein by reference.

Pharmaceutical compositions that are suitable for oral administration can be provided as discrete dosage forms, such as, but not limited to, tablets, fastmelts, chewabie tablets, capsules, pills, strips, troches, lozenges, pastilles, cachets, pellets, medicated chewing gum, bulk powders, effervescent or non-effervescent powders or granules, oral mists, solutions, emulsions, suspensions, wafers, sprinkles, elixirs, and syrups. In one embodiment, such dosage forms contain predetermined amounts of active ingredients, and may be prepared by methods of pharmacy known to those skilled in the art. See generally, Remingto's Pharmaceutical Sciences, 18th ed., Mack Publishing, Easton PA (1 90). As used herein, oral administration also includes buccal, lingual, and sublingual administration. In one embodiment, the oral dosage form provided herein is a tablet. In one embodiment, the oral dosage form provided herein is a capsule. In one embodiment, the oral dosage form provided herein is a caplet. In particular embodiments. In one embodiment, oral dosage forms provided herein are prepared by combining the active ingredients in an intimate admixture with one or more pharmaceutically acceptable earner or excipient, including, but not limited to, binders, fillers, diluents, disintegrants, wetting agents, lubricants, glidants, coloring agents, dye-migration inhibitors, sweetening agents, flavoring agents, emulsifying agents, suspending and dispersing agents, preservatives, solvents, non-aqueous liquids, organic acids, and sources of carbon dioxide, according to conventional pharmaceutical compounding techniques. Excipients can take a wide variety of forms depending on the form of preparation desired for administration. For example, excipients suitable for use in oral liquid or aerosol dosage forms include, but are not limited to, water, glycols, oils, alcohols, flavoring agents, preservatives, and coloring agents. Examples of excipients suitable for use in solid oral dosage forms (e.g., powders, tablets, capsules, and capiets) include, but are not limited to, starches, sugars, micro-crystalline cellulose, diluents, granulating agents, lubricants, binders, and disintegrating agents. In one embodiment, oral dosage forms are tablets or capsules, in which case solid excipients are employed. In specific embodiments, capsules comprising one or more solid forms of pomalidomide (e.g., Form A or an amorphous form) provided herein can be used for oral administration. In one embodiment, the total amount of pomalidomide in the capsule is about 1 mg, about 2 mg, about 3 mg, about 4 mg, or about 5 mg. In one embodiment, the total amount of pomalidomide in the capsule is about 1 mg, about 2 mg, or about 4 mg. In one embodiment, the total amount of pomalidomide in the capsule is about I mg or about 2 mg. Each capsule can contain pomalidomide as the active ingredient and one or more of the following inactive ingredients: mannitoi, pregelatinized starch and sodium stearyl fumarate. In specific embodiments, the 1 mg capsule shell can contain gelatin, titanium dioxide, FD&C blue 2, yellow iron oxide, white ink and black ink. In specific embodiments, the 2 mg capsule shell can contain gelatin, titanium dioxide, FD&C blue 2, yellow iron oxide, FD&C red 3 and white ink. In another embodiment, tablets can be coated by standard aqueous or nonaqueous techniques. Such dosage forms can be prepared by any of the methods of pharmacy. In general, pharmaceutical compositions and dosage forms are prepared by uniformly and intimately admixing the active ingredients with liquid carriers, finely divided solid carriers, or both, and then shaping the product into the desired presentation if necessary. In certain embodiments, the dosage form is a tablet, wherein the tablet is manufactured using standard, art-recognized tablet processing procedures and equipment. In certain embodiments, the method for forming the tablets is direct compression of a powdered, crystalline and/or granular composition comprising a solid form provided herein, alone or in combination with one or more excipients, such as, for example, carriers, additives, polymers, or the like. In certain embodiments, as an alternative to direct compression, the tablets may be prepared using wet granulation or dry granulation processes. In certain embodiments, the tablets are molded rather than compressed, starting with a moist or otherwise tractable material. In certain embodiments, compression and

granulation techniques are used. In certain embodiments, the dosage form is a capsule, wherein the capsules may be manufactured using standard, art-recognized capsule processing procedures and equipments. In certain embodiments, soft gelatin capsules may be prepared in which the capsules contain a mixture comprising a solid form provided herein and vegetable oil or non-aqueous, water miseible materials, such as, for example, polyethylene glycol and the like. In certain embodiments, hard gelatin capsules may be prepared containing granules of solid forms provided herein in combination with a solid pulverulent carrier, such as, for example, lactose, saccharose, sorbitol, mannitol, potato starch, corn starch, amylopectin, cellulose derivatives, or gelatin. In certain embodiments, a hard gelatin capsule shell may be prepared from a. capsule composition comprising gelatin and a small amount of plasticizer such as glycerol. In certain embodiments, as an alternative to gelatin, the capsule shell may be made of a carbohydrate material. In certain embodiments, the capsule composition may additionally include polymers, colorings, flavorings and opaeifiers as required. In certain embodiments, the capsule comprises TiPMC. Examples of excipients or carriers thai can be used in oral dosage forms provided herein include, but are not limited to, diluents (bulking agents), lubricants, disintegrants, fillers, stabilizers, surfactants, preservatives, coloring agents, flavoring agents, binding agents (binders), excipient supports, glidants, permeation enhancement excipients, plasticizers and the like, e.g., as known in the art. It will be understood by those in the art that some substances serve more than one purpose in a pharmaceutical composition. For instance, some substances are binders that help hold a tablet together after compression, yet are also disintegrants that help break the tablet apart once it reaches the target delivery site. Selection of excipients and amounts to use may be readily determined by the formulation scientist based upon experience and consideration of standard procedures and reference works available in the art. In certain embodiments, dosage forms provided herein comprise one or more binders. Binders may be used, e.g., to impart cohesive qualities to a tablet or a capsule, and thus ensure that the formulation remains intact after compression. Suitable binders include, but are not limited to, starch (including potato starch, corn starch, and pregelatinized starch), gelatin, sugars (including sucrose, glucose, dextrose and lactose), polyethylene glycol, propylene glycol, waxes, and natural and synthetic gums, e.g., acacia, sodium alginate, polyvinylpyrrolidone (PVP), celiulosic polymers (including hydroxypropyl cellulose (HPC), hydroxypropylmethylcellulose (HPMC), methyl cellulose, ethyl cellulose, hydroxyethyl cellulose (HEC), carboxymethyl cellulose and the like), veegum, carbomer (e.g., carbopol), sodium, dextrin, guar gum, hydrogenated vegetable oil, magnesium aluminum silicate, maltodextrin, polymethacryiates, povidone (e.g., KOLLIDON, PLASDO E), macrocrystalline cellulose, among others. Binding agents also include, e.g., acacia, agar, aiginic acid, cabomers, carrageenan, cellulose acetate phthalate, ceratonia, chitosan, confectioner's sugar, copovidone, dextrates, dextrin, dextrose, ethylceilulose, gelatin, glyceryl behenate, guar gum, hydroxyethyl cellulose, hydroxyethylmethyl cellulose, hydroxypropyl cellulose, hydroxypropyl starch, hypromeflose, inulin, lactose, magnesium aluminum silicate, maltodextrin, maltose, methylcellulose, poloxamer, polycarbophil, polydextrose, polyethylene oxide, polymethylacrylates, povidone, sodium alginate, sodium carboxymethylcellulose, starch, pregelatinized starch, stearic acid, sucrose, and zein. In one embodiment, the binding agent can be, relative to the weight of the dosage form, in an amount of from about 50% to about 99% w/w. In certain embodiments, a suitable amount of a particular binder is determined by one of ordinary skill in the art. Suitable forms of microery stall ine cellulose include, but are not limited to, the materials sold as AVICEL-PH- 101, AVICEL-PH-103 AVICEL RC-581, AVICEL-PH-105 (FMC Corporation, Marcus Hook, PA), and mixtures thereof. In one embodiment, a specific binder is a mixture of microcrystaHine cellulose and sodium carboxymetbyl cellulose sold as AVICEL RC-581. Suitable anhydrous or low moisture excipients or additives include AVICEL-PH- 103™ and Starch 100 LM. Examples of fillers suitable for use in the pharmaceutical compositions and dosage forms provided herein include, but are not limited to, talc, calcium carbonate (e.g., granules or powder), microcrystaHine cellulose, powdered cellulose, dextrates, kaolin, mannitoi, silicic acid, sorbitol, starch, pre-gelatinized starch, and mixtures thereof. The binder or filler in a pharmaceutical composition is, in one embodiment, present in from about 50 to about 99 weight percent of the pharmaceutical composition or dosage form. In certain embodiments, dosage forms provided herein comprise one or more diluents. Diluents may be used, e.g., to increase bulk so that a practical size tablet or capsule is ultimately provided. Suitable diluents include dicalcium phosphate, calcium sulfate, lactose, cellulose, kaolin, mannitoi, sodium chloride, dry starch, microcrystaHine cellulose (e.g., AVICEL), microfine cellulose, pregelitinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitoi, polymeihacrylates (e.g., EUDRAGIT), potassium chloride, sodium chloride, sorbitol and talc, among others. Diluents also include, e.g., ammonium alginate, calcium carbonate, calcium phosphate, calcium sulfate, cellulose acetate, compressible sugar, confectioner's sugar, dextrates, dextrin, dextrose, erythritol, ethylcellulose, fructose, fumaric acid, glyceryl palraitostearate, isomalt, kaolin, lacitol, lactose, mannitoi, magnesium carbonate, magnesium oxide, maltodextrin, maltose, medium-chain triglycerides, microcrystaHine cellulose, microcrystaHine siiieified cellulose, powered cellulose, polydextrose, polymethylacrylates, simethicone, sodium alginate, sodium chloride, sorbitol, starch, pregelatinized starch, sucrose, sulfobutylether-β-cyclodextrin, talc, tragaeanth, trehalose, and xylitol. Diluents may be used in amounts calculated to obtain a desired volume for a tablet or capsule. The amount of a diluent in the pharmaceutical compositions provided herein varies upon the type of formulation, and is readily discernible to those of ordinary skill in the art. Disintegrants may be used in the compositions to provide tablets or capsules that disintegrate when exposed to an aqueous

environment. Dosage forms that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients) may be used to form solid oral dosage forms. The amount of disintegrant used varies based upon the type of formulation, and is readily discernible to those of ordinary skill in the art. In one embodiment, pharmaceutical compositions comprise from about 0.5 to about 15 weight percent of disintegrant, or from about 1 to about 5 weight percent of disintegrant. Suitable disintegrants include, but are not limited to, agar; bentonite; celluloses, such as methyiceliulose and carboxymethylceilulose; wood products; natural sponge; cation-exchange resins; alginic acid; gums, such as guar gum and Veegum HV; citrus pulp; cross-linked celluloses, such as croscarmellose; cross-finked polymers, such as crospovidone; cross-linked starches; calcium carbonate; mierocrystalline cellulose, such as sodium starch glycolate; polacrilin potassium; starches, such as corn starch, potato starch, tapioca starch, and pre-gelatinized starch; clays; aligns; and mixtures thereof. The amount of a disintegrant in the pharmaceutical compositions provided herein varies upon the type of formulation, and is readily discernible to those of ordinary skill in the art. The amount of a disintegrant in the pharmaceutical compositions provided herein varies upon the type of formulation, and is readily discernible to those of ordinary skill in the art. Lubricants that can be used in pharmaceutical compositions and dosage forms include, but are not limited to, calcium stearate, magnesium stearate, mineral oil, light mineral oil, glycerin, sorbitol, mannitof, polyethylene glycol, other glycols, stearic acid, sodium lauryl sulfate, talc, hydrogenated vegetable oil (e.g., peanut oil, cottonseed oil, sunflower oil, sesame oil, olive oil, corn oil, and soybean oil), zinc stearate, ethyl lease, ethyl laureate, agar, and mixtures thereof. Additional lubricants include, for example, a syloid silica gel (AERGSIL200, manufactured by W.R. Grace Co. of Baltimore, MD), a coagulated aerosol of synthetic silica (marketed by Degussa Co. of Piano, TX), CAB-O-SIL (a pyrogenic silicon dioxide product sold by Cabot Co. of Boston, MA), and mixtures thereof. The pharmaceutical compositions provided herein may contain about 0.1 to about 5% by weight of a lubricant. Suitable glidants include, but are not limited to, colloidal silicon dioxide, CAB-O-SIL® (Cabot Co. of Boston, MA), and asbestos-free talc. Suitable coloring agents include, but are not limited to, any of the approved, certified, water soluble FD&C dyes, and water insoluble FD&C dyes suspended on alumina hydrate, and color lakes and mixtures thereof. A color lake is the combination by adsorption of a water-soluble dye to a hydrous oxide of a heavy metal, resulting in an insoluble form of the dye. Suitable flavoring agents include, but are not limited to, natural flavors extracted from plants, such as fruits, and synthetic blends of compounds which produce a pleasant taste sensation, such as peppermint and methyl salicylate. Suitable sweetening agents include, but are not limited to, sucrose, lactose, mannitol, syrups, glycerin, and artificial sweeteners, such as saccharin and aspartame. Suitable emulsifying agents include, but are not limited to, gelatin, acacia, tragacanth, bentonite, and surfactants, such as polyoxyethylene sorbitan monoofeate (TWEEN® 2.0), polyoxyethylene sorbitan monooieate 80 (TWEEN® 80), and triethanoiamine oleate. Suitable suspending and dispersing agents include, but are not limited to, sodium carboxymefhylcellulose, pectin, tragacanth, Veegum, acacia, sodium carbomethylcellulose, hydroxy-propyl methylcellulose, and polyvinylpyrrolidone. Suitable preservatives include, but are not limited to, glycerin, methyl and propylparaben, benzoic add, sodium benzoate and alcohol. Suitable wetting agents include, but are not limited to, propylene glycol monostearate, sorbitan monooieate, diethylene glycol monolaurate, and polyoxyethylene lauryl ether. Suitable solvents include, but are not limited to, glycerin, sorbitol, ethyl alcohol, and syrup. Suitable non-aqueous liquids utilized in emulsions include, but are not limited to, mineral oil and cottonseed oil. Suitable organic acids include, but are not limited to, citric and tartaric acid. Suitable sources of carbon dioxide include, but are not limited to, sodium bicarbonate and sodium carbonate. In one embodiment, a solid oral dosage form comprises a compound provided herein, and one or more excipients selector from anhydrous lactose, mi crocry stall ine cellulose, polyvinylpyrrolidone, stearic acid, colloidal anhydrous silica, and gelatin. In one embodiment, capsules comprise one or more solid forms of pomalidomide provided herein, and one or more of the following inactive ingredients: mannitoi, pregelatinized starch, sodium stearyl fumarate, gelatin, titanium dioxide, FD&C bine 2, yellow iron oxide, white ink, black ink, FD&C red 3, and a combination thereof. The pharmaceutical compositions provided herein for oral administration can be provided as compressed tablets, tablet triturates, chewable lozenges, rapidly-dissolving tablets, multiple compressed tablets, or enteric-coating tablets, sugar-coated, or film-coated tablets. Enteric-coated tablets are compressed tablets coated with substances that resist the action of stomach acid but dissolve or disintegrate in the intestine, thus protecting the active ingredients from the acidic environment of the stomach. Enteric -coatings include, but are not limited to, fatty acids, fats, phenyl salicylate, waxes, shellac, ammoniated shellac, and cellulose acetate phthalates. Sugar-coated tablets are compressed tablets surrounded by a sugar coating, which may be beneficial in covering up objectionable tastes or odors and in protecting the tablets from oxidation. Film-coated tablets are compressed tablets that are covered with a thin layer or film of a water-soluble material. Film coatings include, but are not limited to, hydroxyethylcelSulose, sodium carboxymethylcellulose, polyethylene glycol 4000, and cellulose acetate phthalate. Film coating imparts the same general characteristics as sugar coating. Multiple compressed tablets are compressed tablets made by more than one compression cycle, including layered tablets, and press-coated or dry-coated tablets, [00200] The tablet dosage forms can be prepared from the active ingredient in powdered, crystalline, or granular forms, alone or in combination with one or more carriers or excipients described herein, including binders, disintegrates, controlied-release polymers, lubricants, diluents, and/or colorants. Flavoring and sweetening agents are especially useful

in the formation of chewable tablets and lozenges. The pharmaceutical compositions provided herein for oral administration can be provided as soft or hard capsules, which can be made from gelatin, methylcelluiose, starch, or calcium alginate. The hard gelatin capsule, also known as the dry-filled capsule (DFC), consists of two sections, one slipping over the other, thus completely enclosing the active ingredient. The soft elastic capsule (SEC) is a soft, globular shell, such as a gelatin shell, which is plasticized by the addition of glycerin, sorbitol, or a similar polyol. The soft gelatin shells may contain a preservative to prevent the growth of microorganisms. Suitable preservatives are those as described herein, including methyl- and propylparabens, and sorbic acid. The liquid, semisolid, and solid dosage forms provided herein may be encapsulated in a capsule. Suitable liquid and semisolid dosage forms include solutions and suspensions in propylene carbonate, vegetable oils, or triglycerides. Capsules containing such solutions can be prepared as described in U.S. Pat. Nos. 4,328,245; 4,409,239; and 4,410,545. The capsules may also be coated as known by those of skill in the art in order to modify or sustain dissolution of the active ingredient. The pharmaceutical compositions provided herein for oral administration can be provided in liquid and semisolid dosage forms, including emulsions, solutions, suspensions, elixirs, and syrups. An emulsion is a two-phase system, in which one liquid is dispersed in the form of small globules throughout another liquid, which can be oil-in-water or water-in-oil. Emulsions may include a pharmaceutically acceptable nonaqueous liquid or solvent, emulsifying agent, and preservative. Suspensions may include a pharmaceutically acceptable suspending agent and preservative. Aqueous alcoholic solutions may include a pharmaceutically acceptable acetai, such as a di(lower alk 1) acetal of a lower alkyl aldehyde, e.g., acetaldehyde diethyl acetai; and a water-miscible solvent having one or more hydroxy! groups, such as propylene glycol and efhanol. Elixirs are clear, sweetened, and hydroalcoholic solutions. Syrups are concentrated aqueous solutions of a sugar, for example, sucrose, and may also contain a preservative. For a liquid dosage form, for example, a solution in a polyethylene glycol may be diluted with a sufficient quantity of a pharmaceutically acceptable liquid carrier, e.g., water, to be measured conveniently for administration. Other useful liquid and semisolid dosage forms include, but are not limited to, those containing the active ingredient(s) provided herein, and a dialkylated mono- or poiy-alkylene glycol, including, 1,2-dimethoxymethane, diglyme, triglyme, tetraglyme, polyethylene glyeol-350-dimethyl ether, polyethylene glycol-550-dimethyi ether, polyethylene glycol-750-dimetl yl ether, wherein 350, 550, and 750 refer to the approximate average molecular weight of the polyethylene glycol. These formulations can further comprise one or more antioxidants, such as butylated hydroxytoluene (BUT), butylated hydroxyanisole (BHA), propyl gallate, vitamin E, hydroquinone, hydroxycoumarins, ethanolamine, lecithin, cephalin, ascorbic acid, malic acid, sorbitol, phosphoric acid, bisulfite, sodium meiabisulfite, thiodipropionic acid and its esters, and dithiocarbamates. The pharmaceutical compositions provided herein for oral administration can be also provided in the forms of liposomes, micelles, microspheres, or nanosystems. Micellar dosage forms can be prepared as described in U.S. Pat. No. 6,350,458. The pharmaceutical compositions provided herein for oral administration can be provided as non-effervescent or effervescent, granules and powders, to be reconstituted into a liquid dosage form. Pharmaceutically acceptable carriers and excipients used in the non-effervescent granules or powders may include diluents, sweeteners, and wetting agents. Pharmaceutically acceptable carriers and excipients used in the effervescent granules or powders may include organic acids and a. source of carbon dioxide. Coloring and flavoring agen ts can be used in all of the above dosage forms. The pharmaceutical compositions provided herein for oral administration can be formulated as immediate or modified release dosage forms, including delayed-, sustained, pulsed-, controlled, targeted-, and programmed-release forms. Certain embodiments provided herein are illustrated by the following non-limiting examples. In one embodiment, pomalidomide may be synthesized using methods described in U.S. Patent Nos. 5,635,517, 6,335,349, 6,316,471, 6,476,052, 7,041,680, 7,709,502, and 7,994,327, all of which are incorporated herein in their entireties.

Pomalidomide Polymorph Preparations: A weighed sample of pomalidomide (about 100 mg) was treated with 2 mL of a test solvent. The solvents used were either reagent or HPLC grade. The resulting mixture was agitated for at least 24 hours at 25°C. When all of the solids were dissolved by visual inspection, the estimated solubilities were calculated. The solubility was estimated from these experiments based on the total volume of solvent used to give a solution. The actual solubility may be greater than those calculated due to the use of a large amount of solvent or to a slow rate of dissolution. If dissolution did not occur during the experiment, the solubility was measured gravimetrically. A known volume of filtrate was evaporated to dryness and the weight of the residue was measured. Equilibration and evaporation experiments were carried out by adding an excess of pomalidomide to 4 mL of a test solvent. The resulting mixture was agitated for at least 24 hours at 25°C and 50°C separately. Upon reaching equilibrium, the saturated solution was removed and allowed to evaporate slowly in an open vial under nitrogen at 25°C and 50°C respectively. The slurry resulting from the equilibration was filtered and dried in the air. Crystallizations using cooling methods were performed. The solid was dissolved in a solvent at an elevated temperature, approximately 80°C and allowed to cool to room temperature. Samples that did not crystallize at room temperature were placed in a refrigerator (0-5°C). Solids were isolated by decantation and allowed to dry in the air. Precipitation studies were carried out by solvent/anti-solvent combinations. The solid was dissolved in a solvent in which pomalidomide solubility was high, and then a selected solvent in which pomalidomide was highly insoluble (anti-solvent) was added to the solution. A precipitate formed immediately in some solvent/anti-solvent systems. If the precipitation did not occur immediately, the resulting mixture was

allowed to cool in a refrigerator until a precipitate formed. The precipitate was then isolated by decantation and allowed to dry in the air. All of the samples generated as described in the polymorph preparation study were analyzed by XRPD. X PD was conducted on a Thermo ARL X'TRA X-ray powder diffractometer using Cu Kcc radiation at 1.54 A. The instrument was equipped with a fine focus X-ray tube. The voltage and amperage of X-ray generator were set at 45 kV and 40 mA, respectively. The divergence slices were set at 4 mm and 2 mm and the measuring slices were set at 0.5 mm and 0.2 mm. Diffracted radiation was detected by peitier-cooled Si(Li) solid state detector. A theta-two theta continuous scan at 2.40°/min (0.5 sec/0.02c step) from 1.5° 20 to 40° 2.0 was used. A sintered alumina standard was used to check the peak position. DSC analyses were performed on a TA instrument Q1000. Indium was used as calibration standards. Approximately 2-5 mg of sample was placed into a DSC pan and the weight was accurately recorded. The sample was heated under nitrogen at a rate of 10°C/min, up to a final temperature of 350°C. Melting points were reported as extrapolated onset temperature. TG analyses were performed on a TA instrument Q500. Calcium oxalate was used for performance check. Approximately 10-25 mg of sample was placed on a pan, accurately weighed and loaded into the TG furnace. The sample was heated under nitrogen at a rate of 10°C/min, up to a final temperature of 350°C. Morphology and particle size analysis of the samples were carried out on an Olympus microscope. The instrument was calibrated with USP standards. Hygroscopicity was determined on a Surface Measurement Systems DVS. Typically a sample size of 10-50 mg was loaded into the DVS instrument sample pan and the sample was analyzed on a DVS automated sorption analyzer at 25°C. The relative humidity was increased in 10% increments from 0% to 95% RH. The relative humidity was then decreased in a similar mariner to accomplish a full adsosption/desorption cycle. Solubility of pomalidomide in various solvents at ambient temperature was determined and is shown in Table 3. Pomalidomide was found to be most soluble in THF (5.26 mg/mL) followed by acetone (1.99 mg/mL). It was also found to have low solubility in certain solvents (< 1 mg/mL), including heptane, n-butanol, n-butyl acetate, ethanol, methanol, ethyl acetate, methyl t-butyl ether, 2-propanol, toluene, and water.

Table 3; Solubility of Pomalidomide:

| Solvent | Solubility (mg/mL) |
|---|---|
| Acetone | 1.99 |
| Acetonitrile | 1.27 |
| n-Butanol | 0.08 |
| n-Butyl acetate | 0.10 |
| Absolute ethanol | 0.14 |
| Ethyl acetate | 0.03 |
| Heptane | 0.36 |
| Methylene chloride | 1.45 |
| Methyl ethyl ketone | 1.26 |
| Methanol | 0.41 |
| Methyl t-butyl ether | 1.03 |
| 2-Propanol | 0.32 |
| Tetrahydrofuran | 5.26 |
| Toluene | 0.36 |
| Water | 0.19 |
| Ethanol/Water (1:1) | 0.54 |

Various embodiments of Form A of pomalidomide were prepared from evaporation study at 50°C, in methyl ethyl ketone or in tetrahydrofiiran; slurry at 25°C in certain solvent (e.g., acetone, acetonitrile, n-butanol, n-butyl acetate, ethanol, ethyl acetate, heptane, methylene chloride, methyl ethyl ketone, methanol, methyl t-butyl ether, 2-propanoi, tetrahydrofiiran, toluene, water, or ethanol/water (1 : 1 )), or slurry at 50°C in certain solvent (e.g., acetone, acetonitrile, n-butanol, n-butyl acetate, ethanol, ethyl acetate, heptane, methyl ethyl ketone, methanol, 2-propanoi, tetrahydrofuran, toluene, water, or ethanol/water (1 : 1)). Crystalline material was also obtained from crystallization from solvent, such as acetomtrile, methyl ethyl ketone, or tetrahydrofuran, under conditions summarized in Table 4, to generate samples with XRPD patterns consistent with Form A.

Table 4: Crystallization of Pomalidomide

| Solvent | Method | XRPD Result |
|---|---|---|
| Acetonitrile | Dissolved at ~80 °C Cooled in refrigerator | Form A |
| Methyl Ethyl Ketone | Dissolved at 80 °C Cooled in refrigerator | Form A |
| Tetrahydrofuran | Dissolved at ~80 °C Cooled in refrigerator | Form A |

The results of precipitation studies are summarized in Table 5. When toluene, methyl t-butyl ether, or water was added to a pomalidomide in TITF solution at 60°C, clear solutions were obtained. After the solutions were cooled in a refrigerator, no crystalline materials precipitated. When heptane was added to a pomalidomide in THF solution at 60°C, a cloudy mixture was obtained. The mixture was then placed in a refrigerator. The crystalline material obtained after 24 hours was identifiedas one preparation of Form A by XRPD.

| Solvent | Anti-solvent | Ratio of Solvent and Anti-solvent | XRPD Result |
|---|---|---|---|
| Tetrahydrofuran | Heptane | 1:3 | Form A |
| Tetrahydrofuran | Toluene | 1:3 | |
| Tetrahydrofuran | Water | 1:3 | |
| Tetrahydrofuran | Methyl t-butyl ether | 1:3 | |

Pomalidomide Solid Form Preparations: Preparation of solid form of pomalidomide was conducted by equilibrating pomalidomide in 96 solvent compositions at 50°C and subjected to four conditions: slurry, evaporation, cooling, and precipitation. For the slurry experiment, approximately 3 mg of a solid sample of pomalidomide (e.g. Form A) was delivered to each position of the plate. Solvents were then added, as binary gradients consisting of main solvent and co-solvent, to give a total of 150 μl, in each of the 96 vials (see Table 6 for the exact composition of each vial). The sealed plate was then magnetically stirred at 50°C for 8 hours, then at 25°C for 8 hours. The lid of the crystal lize!" assembly was then removed, the residual solvents removed by wicking, and the samples dried in air. Each sample was analyzed by XRPD and birefringence. For each of the evaporation, precipitation, and cooling experiments, approximately 8 mg of a solid sample of pomalidomide (e.g., Form A) was delivered to each position of the plate. Solvents were added to the master plate, as binary gradients consisting of main solvent and co-solvent, to give a total volume of 800 μT in each of the 96 vials (see Table 7 for the exact composition of each vial). The master plate, containing pomalidomide and solvents, was then equilibrated by stirring for 2 hours at 50°C. Upon completion of the equilibration cycle, the supernatant was aspirated into a four-tipped needle assembly and transferred to a sealed parallel filtration assembly. The mixtures were forced through the filtration assembly using positive pressure and the filtrates were collected in an array of glass vials. The filtered solutions were subsequently aspirated into the four-tipped assembly and aliquots were dispensed to an array of glass vials for subsequent LC analysis to determine approximate concentrations of pomalidomide in each solvent composition at the beginning of the experiments (Table 8). Both the four-tipped needle assembly and filtration assembly were maintained at the equilibration temperature of 50°C throughout the entire transfer process.

Main Solvents

| | |
|---|---|
| Water | THF |
| Cyclohexane | Trichloroethane |

| | Main Solvent Volume (uL) | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| A | 0 | 24 | 48 | 72 | 96 | 120 | 130 | 104 | 78 | 52 | 26 | 0 |
| B | 0 | 24 | 48 | 72 | 96 | 120 | 130 | 104 | 78 | 52 | 26 | 0 |
| C | 0 | 24 | 48 | 72 | 96 | 120 | 150 | 120 | 90 | 60 | 30 | 0 |
| D | 0 | 24 | 48 | 72 | 96 | 120 | 150 | 120 | 90 | 60 | 30 | 0 |
| E | 0 | 30 | 60 | 90 | 120 | 150 | 150 | 120 | 90 | 60 | 30 | 0 |
| F | 0 | 30 | 60 | 90 | 120 | 150 | 150 | 120 | 90 | 60 | 30 | 0 |
| G | 0 | 26 | 52 | 78 | 104 | 130 | 130 | 104 | 78 | 52 | 26 | 0 |
| H | 0 | 26 | 52 | 78 | 104 | 130 | 130 | 104 | 78 | 52 | 26 | 0 |

| | | Co-solvent Volume (uL) | | | | | | | | | | | | |
|---|---|---|---|---|---|---|---|---|---|---|---|---|---|---|
| | | 1 | 2 | 3 | 4 | 5 | 6 | 7 | 8 | 9 | 10 | 11 | 12 | |
| Acetic acid | A | 150 | 126 | 102 | 78 | 54 | 30 | 26 | 46 | 72 | 98 | 124 | 150 | H2O |
| Ethanol | B | 150 | 126 | 102 | 78 | 54 | 30 | 26 | 46 | 72 | 98 | 124 | 150 | Toluene |
| 2-Propanol | C | 150 | 126 | 102 | 78 | 54 | 30 | 0 | 30 | 60 | 90 | 120 | 150 | 1-Butanol |
| Acetonitrile | D | 150 | 126 | 102 | 78 | 54 | 30 | 0 | 30 | 60 | 90 | 120 | 150 | n-Butyl acetate |
| 2-butanone | E | 150 | 120 | 90 | 60 | 30 | 0 | 0 | 30 | 60 | 90 | 120 | 150 | Trifluoroethanol |
| cyclopentyl methyl ether | F | 150 | 120 | 90 | 60 | 30 | 0 | 0 | 30 | 60 | 90 | 120 | 150 | Ethyl acetate |
| 1,2-Dimethoxyethane | G | 150 | 124 | 98 | 72 | 46 | 20 | 26 | 46 | 72 | 98 | 124 | 150 | 1,4-Dioxane |
| 1,2-Dichloroethane | H | 150 | 124 | 98 | 72 | 46 | 20 | 26 | 46 | 72 | 98 | 124 | 150 | nitromethane |

Table 6. Solvents, Co-Solvents, and Amounts Thereof for the Slurry Experiment

Main Solvents

| | |
|---|---|
| Water | THF |
| Cyclohexane | Trichloroethane |

| | Main Solvent Volume (uL) | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| A | 0 | 128 | 256 | 384 | 512 | 640 | 760 | 608 | 456 | 304 | 152 | 0 |
| B | 0 | 128 | 256 | 384 | 512 | 640 | 760 | 608 | 456 | 304 | 152 | 0 |
| C | 0 | 128 | 256 | 384 | 512 | 640 | 800 | 640 | 480 | 320 | 160 | 0 |
| D | 0 | 128 | 256 | 384 | 512 | 640 | 800 | 640 | 480 | 320 | 160 | 0 |
| E | 0 | 160 | 320 | 480 | 640 | 800 | 800 | 640 | 480 | 320 | 160 | 0 |
| F | 0 | 160 | 320 | 480 | 640 | 800 | 800 | 640 | 480 | 320 | 160 | 0 |
| G | 0 | 152 | 304 | 456 | 608 | 760 | 760 | 608 | 456 | 304 | 152 | 0 |
| H | 0 | 152 | 304 | 456 | 608 | 760 | 760 | 608 | 456 | 304 | 152 | 0 |

| | | Co-solvent Volume (uL) | | | | | | | | | | | | |
|---|---|---|---|---|---|---|---|---|---|---|---|---|---|---|
| | | 1 | 2 | 3 | 4 | 5 | 6 | 7 | 8 | 9 | 10 | 11 | 12 | |
| Acetic acid | A | 800 | 672 | 544 | 416 | 288 | 160 | 40 | 192 | 344 | 496 | 648 | 800 | H2O |
| Ethanol | B | 800 | 672 | 544 | 416 | 288 | 160 | 40 | 192 | 344 | 496 | 648 | 800 | Toluene |
| 2-Propanol | C | 800 | 672 | 544 | 416 | 288 | 160 | 0 | 160 | 320 | 480 | 640 | 800 | 1-Butanol |
| Acetonitrile | D | 800 | 672 | 544 | 416 | 288 | 160 | 0 | 160 | 320 | 480 | 640 | 800 | n-Butylacetate |
| 2-butanone | E | 800 | 640 | 480 | 320 | 160 | 0 | 0 | 160 | 320 | 480 | 640 | 800 | Trifluoroethanol |
| cyclopentyl methyl ether | F | 800 | 640 | 480 | 320 | 160 | 0 | 0 | 160 | 320 | 480 | 640 | 800 | Ethyl acetate |
| 1,2-Dimethoxyethane | G | 800 | 648 | 496 | 344 | 192 | 40 | 40 | 192 | 344 | 496 | 648 | 800 | 1,4-Dioxane |
| 1,2-Dichloroethane | H | 800 | 648 | 496 | 344 | 192 | 40 | 40 | 192 | 344 | 496 | 648 | 800 | nitromethane |

Table 7. Solvents, Co-Solveots, and Amounts Thereof for the Evaporation.

For the evaporation experiments, 200 µl., filtrate aliquots were dispensed into each well of an unsealed erystaliizer assembly and allowed to evaporate under ambient conditions. For the precipitation experiments, 100

| | 1 | 2 | 3 | 4 | 5 | 6 | 7 | 8 | 9 | 10 | 11 | 12 |
|---|---|---|---|---|---|---|---|---|---|---|---|---|
| A | 2.24 | 8.19 | 2.43 | 1.44 | 0.80 | 0.28 | 6.38 | 7.84 | 4.44 | 1.83 | 0.29 | 0.03 |
| B | 0.23 | 0.73 | 1.93 | 0.22 | 0.36 | 0.15 | 3.16 | 1.72 | 0.32 | 0.48 | 0.18 | ND |
| C | 0.62 | 1.41 | 1.87 | 0.48 | 0.47 | 0.26 | 3.71 | 2.97 | 1.84 | 1.09 | 0.49 | 0.34 |
| D | 1.53 | 3.57 | 3.45 | 2.53 | 1.34 | 0.49 | 3.73 | 2.31 | 1.83 | 0.36 | 1.16 | 0.87 |
| E | 1.80 | 0.99 | 2.32 | 0.13 | 0.02 | ND | 0.02 | 0.73 | 1.83 | 2.31 | 2.48 | 0.95 |
| F | 0.10 | 0.36 | 0.03 | 0.01 | ND | ND | 0.04 | 0.04 | 0.13 | 0.10 | 0.39 | 0.38 |
| G | 2.89 | 1.88 | 0.70 | 0.23 | 0.06 | ND | 0.03 | 0.41 | 0.85 | 2.37 | 5.18 | 5.59 |
| H | 0.30 | 0.24 | 0.11 | 0.04 | ND | ND | 0.05 | 0.40 | 0.35 | 1.50 | 1.36 | 2.03 |

Table 8. Approximate Measured Solubility of Pomalidomide at 50°C

Preparation and Characterization of Solid Forms of Pomalidomide: Preparations of Form A of Pomalidomide: In one embodiment, various preparations of Form A were obtained via crystallization in certain solvent systems. The pomalidomide starting material used in these experiments was first characterized by XRPD (Figure 2 of WO2013/126326) and DSC (Figure 11 of WO2013/126326). In one embodiment, 20 mg of pomalidomide was heated in 10 mL of nitromethane at 50°C with magnetic stirring for 2 hours. The resulting homogenous solution was allowed to slowly evaporate under ambient conditions. The isolated solid was characterized by XRPD (Figure 3 of WO2013/126326, one embodiment of Form A), DSC (Figure 13 of WO2013/126326, single endotherm at 318.6°C), and TGA (0.6 wt % lost up to 200°C), which was consistent with an unsolvated form. This experiment resulted in large crystals, as shown in the birefringence image of Figure 7 of WO2013/126326. In one embodiment, Form A was prepared from 20 nig of pomalidomide slurried in 0.8 mL of water and 0.2 niL of acetonitrile at 50°C with magnetic stirring for 20 hours. After cooling to room temperature, the solvent was removed by decanting and the isolated solid was further dried by wicking onto filter paper. The isolated solid was characterized by XRPD (FiG, 4, one embodiment of Form A), DSC (FIG, 14, single endotherm at 320°C), and TGA (Figure 17 of WO2013/126326, 0.3 wt % gained up to 200°C), which was consistent with an unsolvated form. The birefringence image of the resultant crystalline material is shown in Figure 8 of WO2013/126326. In one embodiment, Form A was prepared from 40 mg of pomalidomide slurried in 2 mL of acetonitrile at 80°C with magnetic stirring for 3 days. After cooling to room temperature, the solvent was removed by decanting and the isolated solid was further dried by wicking onto filter paper. The isolated solid was characterized by XRPD (Figure 1 of WO2013/126326, one embodiment of Form A) and DSC (Figure 10 of WO2013/12632, single endotherm at 320.1°C), which was consistent with an unsolvated form. In one embodiment, Form A was prepared from 40 mg of pomalidomide slurried in 2 mL of water at 100°C with magnetic stirring for 3 days. After cooling to room temperature, the solvent was removed by decanting and the isolated solid was further dried by wicking onto filter paper. The isolated solid was characterized by XRPD (Figure 5 of WO2013/126326, one embodiment of Form A) and DSC (Figure 15 of WO2013/126326, single endotherm at 320.5°C), which was consistent with an unsolvated form. In one embodiment. Form A was prepared from 100 mg of pomalidomide slurried in 4 mL of water and 1 mL of acetonitrile at 100°C with magnetic stirring for 20 hours. After cooling to room temperature, the solvent was removed by decanting and the isolated solid was further dried by wicking onto filter paper. The isolated solid was characterized by XRPD (FIG, 6, one embodiment of Form A), DSC (Figure 16 of WO2013/126326, single endotherm at 319.2°C), and TGA (0.3 wt. % gained up to 200°C), and was consistent with an unsolvated form. The birefringence image of the resultant crystalline material is shown in Figure 9 of WO2013/126326. In one embodiment, solid forms of pomalidomide comprising Form A may be prepared via crystallization in solvent systems, such as: water/ethanol, water/2-propanol, water/acetonitriie, cyclohexane/2-butanone, cyclohexane/cyclopentyl methyl ether, cyclohexane/1,2-dimethoxyethane, cyclohexane/1,2-dichloroethane, THF/toluene, THF/1-butanol, THF/n-butyl acetate, 1,1,2-trichloroethene/trifluoroeffimof, 1,1,2-tricholoethene/ethyl acetate, 1,1,2-trieholoethene/1,4-dioxane, and 1,1,2-tricholoethenelnitromethane. DSC cycling experiments were performed on certain samples comprising Form A to determine if thermal conversion or melt crystallization would generate a form change. Form A remained the only form detected after DSC cycling experiments (FK 12a and 12b). Figures 1-17 of WO2013/126326provide representative XRPD patterns, birefringence images, DSC thermograms, and TGA thermogram, of various preparations of Form A of pomalidomide.

Amorphous Forms of Pomalidomide: In certain embodiments, an amorphous form of pomalidomide can be obtained from certain aqueous and/or organic solvent systems, for example, water/acetic acid, THF/water, water/ethanol, THF/toluene, and water/2 -propanol, using a method described herein. In one embodiment, amorphous forms were prepared in the slurry experiment described in and Table 6, for example, including the following cells of the 96-well plate: A1, A 10, B3, C2, C3, C11, E4, F8, F10, F11, and G12. In one embodiment, amorphous forms were prepared in the evaporation experiment described in Section 6.2 and Table 4, for example, including the following cells of the 96-well plate: A11, A12, B6, C1-C3, C5, C6, C9-C11, D3, D4, D8, E1, E9, E11, and G12. In one embodiment, amorphous forms were prepared in the precipitation experiment described in Table 4, for example, including the following cells of the 96-well plate: A8, B7, C7, C8, D1-D5, D7, E1, G10, G12, and H11. In one embodiment, amorphous forms were prepared in the cooling experiment described in Table 4, for example, including the following cells of the 96-well plate: A10, A11, B2, B6, CI, C2, C6-C8, CIO, C 12, D2, D5, D6, E1, E2, E10, E12, and H12. Characterization Methods: X-ray powder diffraction (XRPD) data were obtained with a Bruker D8 Discover equipped with a xyz translation stage (with x, y, z travel of 100 mm, 150 mm, and 100 mm, respectively). The X-ray detector was a high-performance HI-STAR two-dimensional detector which was set to 30 cm from the center of the goniometer. At this distance, the detector had a typical FWHM of 0.15-0.2 degrees in 20, The X-ray generator was typically set to 40 kV and 40 mA. The data was collected in one frame with a typical data acquisition time of 3 minutes. The 20 range covered by the HI-STAR detector was from 4.5 to 39.5 degrees. The sample was typically oscillated in the y direction (perpendicular to the x-ray travel direction) with oscillation amplitude of ± 2-3 mm. Omega-scan (rocking the x-ray source and the detector synchronously) was also used occasionally to reduce preferred orientation in samples that were producing very spotty diffraction patterns. Crystals grown on Universal Substrate were analyzed either uncrushed or crashed. Epoch softw are was used to facilitate the translation of the stage to the elements of interest and a joystick to control translation and a knob to adj ust the Z height were used to focus the beam on the samples of interest. Epoch then stored the images and coordinates of each of the user specified locations to the database. Epoch was also used to control the data acquisition parameters, area plots, and 20 plots to the database as an XRPD experiment. Differential Scanning Calorimetry (DSC) analyses were made on a DSC 2920 differential scanning calorimeter by TA Instruments with a dual sample ceil. Samples (0.1 - 2 mg) were loaded in an aluminum pan with an aluminum lid set on top (not crimp sealed). The prepared samples and an inert, reference were placed in the device to measure the differential heat flow from 50 to 350°C at 10°C /minute. Universal analysis software from TA Instruments was used for data analysis. Thermogravimetric (TG) analyses were performed on a TGA 2950 Thermogravimetrix analyzer by TA Instruments, Samples (0.1 - 2 mg) were placed in an aluminum pan and placed in the device. The data were collected from 50 to 350°C at 10°C /minute. Birefringence microscopy measurements were made on an Inverted Microscope Axiovert 200M by Carl Zeiss Inc. equipped with a Bioprecision Inverted Stage from Ludi which had a total travel of 120 mm x 100 mm with a resolution of 0.2 μm, a repeatability of i μm ars accuracy of 6 μm, and was controlled by a MAC 5000 controller. The light source for the microscope system was a Hal 100W system. The light was passed through a polarizing filter, the sample, and then a second polarizing filter set perpendicular to the first so that only materials that rotate plane polarized light are observed. AxioVision controlled the microscope, camera, light, XY stage, s utter, and filter wheels. A 2.5X microscope objective was used to ensure that the entire area of each element on the Universal Substrate could be captured in a single image, A transiational sequence was set up to obtain birefringence images for all 96 elements. Epoch software interfaced with AxioVision software and controlled the acquisition of birefringence images and stored the images and intensities to the da.taba.se. A 10X microscope objective was used to obtain higher resolution images of selected samples. Higher magnification images of user selected samples were subsequently recorded and saved to the database using Epoch. The general characterization methods described herein are non-limiting, and are intended merely as examples of parameters, methods and techniques which can be used to analyze certain embodiments provided herein. Other standard parameters, methods and techniques for chemical, biological, physiological and solid-state analysis are contemplated herein as means of characterizing various embodiments provided herein. Additional Data on Form A of Pomalidomide: DSC and TGA: In one embodiment, TG and DSC thermograms of one preparation of Form A were obtained. In this preparation, Form A was found to lose about 0.13% volatiles before decomposition, indicative of an unsolvated material. The DSC thermogram exhibited an endotherm at about 317.60°C. Elemental Analysis: The results of the determined and the theoretical values of %carbon, %hydrogen and %nitrogen of one sample of pomalidomide are given in Table 9. The experimental results were consistent with theory.

| | **% Carbon** | **% Hydrogen** | **% Nitrogen** |
|---|---|---|---|
| **Theoretical values** | 57.14% | 4.06% | 15.38% |
| **Results** | 57.13% | 4.12% | 15.41% |

Single Crystal X-Ray Analysis: The molecular structure and representative solid state conformation of pomalidomide were determined by a single crystal X-ray study at 173 K. Single crystal intensities were measured dififractometrically using radiation wavelength of 1.54178 A. The structure is shown in Figure 19 of WO2013/126326. Figure 20 of WO2013/126326 shows a drawing of the packing along the a-axis where the dotted lines represent potential hydrogen bonding. The unit cell dimensions were determined to be a = 7.0046(2) A, b = 7.8130(2) A, c = 11.2541(4) A. Atomic coordinates are shown in Tables 10 and 12. Bond length (A) and bond angles (°) are shown in Tables 11 and 13. XRPD powder diffraction data was simulated using the single crystal intensity data using the program XPOW1. Key parameters for the calculation include the wavelength (used copper wavelength of 1,54 A), and the cell parameters retrieved from the final refinements. The simulated XRPD pattern is shown in Figure 21 of WO2013/126326, with peak position and

peak intensity values listed at the bottom of the Figure. Experimental: Pomalidomide was dissolved in warm DMF, and then placed in the refrigerator. The initial crystals were small, allowing these to sit in mother liquor produced larger crystals. A yellow needle crystal with dimensions of 0.26 x 0.14 x 0,07 mm was mounted on a Nylon loop using very small amount of paratone oil. Data were collected using a Bruker CCD (charge coupled device) based diffractometer equipped with an Oxford Cryostream low-temperature apparatus operating at 173 K. Data were measured using omega and phi scans of 0.5° per frame for 45 s. The total number of images was based on results from the program COSMO where redundancy was expected to be 4.0 and completeness to 100% out to 0.83 A. Cell parameters were retrieved using APEX II software and refined using SAINT on all observed reflections. Data reduction was performed using the SAINT software which corrects for Lp. Scaling and absorption corrections were applied using SADABS multi-scan technique. The structures are solved by the direct method using the SHELXS-97 program and refined by least squares method on F2, SHELXL-97, which were incorporated in SHELXTL-PC V 6, 10. The structure was solved in the space group Pi (# 2). All non-hydrogen atoms were refined anisotropically. Hydrogens were found by difference Fourier methods and refined isotropically. The crystal used for the diffraction study showed no decomposition during data collection. All drawings were done at 50% ellipsoids.

Table 10; Atomic coordinates (x 104) and equivalent isotropic displacement parameters ($A^2 \times 10^3$)

| | x | y | z | U(eq) |
|---|---|---|---|---|
| O(1) | 2909(3) | 9805(3) | 4402(2) | 31(1) |
| O(2) | 5972(3) | 5099(3) | 6544(2) | 40(1) |
| O(3) | 2202(3) | 898(3) | 6783(2) | 39(1) |
| O(4) | 3084(4) | 4673(3) | 9119(2) | 44(1) |
| N(1) | 4314(4) | 7461(3) | 5590(2) | 29(1) |
| N(2) | 2609(3) | 3134(3) | 7703(2) | 29(1) |
| N(3) | 2941(5) | 1983(5) | 11692(3) | 47(1) |
| C(1) | 2731(4) | 8311(4) | 5115(3) | 25(1) |
| C(2) | 860(4) | 7282(4) | 5491(3) | 34(1) |
| C(3) | 798(4) | 5775(4) | 6682(3) | 33(1) |
| C(4) | 2566(4) | 4624(4) | 6604(3) | 29(1) |
| C(5) | 4437(4) | 5718(4) | 6287(3) | 28(1) |
| C(6) | 2813(4) | 3265(4) | 8892(3) | 30(1) |
| C(7) | 2662(4) | 1450(4) | 9715(3) | 26(1) |
| C(8) | 2744(4) | 829(4) | 10995(3) | 30(1) |
| C(9) | 2585(4) | -1017(4) | 11510(3) | 35(1) |
| C(10) | 2369(4) | -2144(5) | 10794(3) | 36(1) |
| C(11) | 2253(4) | -1523(4) | 9513(3) | 33(1) |
| C(12) | 2411(4) | 288(4) | 9011(3) | 26(1) |
| C(13) | 2383(4) | 1368(4) | 7698(3) | 28(1) |

Table 11. Bond lengths [A] and angles [°].

| | |
|---|---|
| O(1)-C(1) | 1.216(3) |
| O(2)-C(5) | 1.211(3) |
| O(3)-C(13) | 1.211(4) |
| O(4)-C(6) | 1.222(4) |
| N(1)-C(5) | 1.378(4) |
| N(1)-C(1) | 1.379(4) |
| N(1)-H(1) | 0.85(4) |
| N(2)-C(13) | 1.395(4) |
| N(2)-C(6) | 1.400(4) |
| N(2)-C(4) | 1.448(4) |
| N(3)-C(8) | 1.371(4) |
| N(3)-H(3A) | 1.02(5) |
| N(3)-H(3B) | 0.88(5) |

(continued)

| | |
|---|---|
| C(1)-C(2) | 1.496(4) |
| C(2)-C(3) | 1.514(4) |
| C(2)-H(2A) | 0.98(4) |
| C(2)-H(2B) | 0.97(4) |
| C(3)-C(4) | 1.516(4) |
| C(3)-H(3C) | 0.95(3) |
| C(3)-H(3D) | 0.99(4) |
| C(4)-C(5) | 1.523(4) |
| C(4)-H(4) | 1.01(3) |
| C(6)-C(7) | 1.459(4) |
| C(7)-C(12) | 1.391(4) |
| C(7)-C(8) | 1.397(4) |
| C(8)-C(9) | 1.398(4) |
| C(9)-C(10) | 1.374(5) |
| C(9)-H(9) | 0.94(4) |
| C(10)-C(11) | 1.403(5) |
| C(10)-H(10) | 0.84(3) |
| C(11)-C(12) | 1.371(4) |
| C(11)-H(11) | 1.01(3) |
| C(12)-C(13) | 1.488(4) |
| C(5)-N(1)-C(1) | 128.0(3) |
| C(5)-N(1)-H(1) | 115(3) |
| C(1)-N(1)-H(1) | 117(3) |
| C(13)-N(2)-C(6) | 111.8(2) |
| C(13)-N(2)-C(4) | 122.8(3) |
| C(6)-N(2)-C(4) | 125.4(2) |
| C(8)-N(3)-H(3A) | 115(3) |
| C(8)-N(3)-H(3B) | 116(3) |
| H(3A)-N(3)-H(3B) | 122(4) |
| O(1)-C(1)-N(1) | 120.0(3) |
| O(1)-C(1)-C(2) | 123.2(3) |
| N(1)-C(1)-C(2) | 116.7(2) |
| C(1)-C(2)-C(3) | 113.3(2) |
| C(1)-C(2)-H(2A) | 108(2) |
| C(3)-C(2)-H(2A) | 107(2) |
| C(1)-C(2)-H(2B) | 109(2) |
| C(3)-C(2)-H(2B) | 113(2) |
| H(2A)-C(2)-H(2B) | 106(3) |
| C(2)-C(3)-C(4) | 110.5(3) |
| C(2)-C(3)-H(3C) | 109.7(19) |
| C(4)-C(3)-H(3C) | 109.7(19) |
| C(2)-C(3)-H(3D) | 107(2) |
| C(4)-C(3)-H(3D) | 111(2) |
| H(3C)-C(3)-H(3D) | 109(3) |
| N(2)-C(4)-C(3) | 114.0(3) |
| N(2)-C(4)-C(5) | 110.6(2) |
| C(3)-C(4)-C(5) | 111.9(3) |
| N(2)-C(4)-H(4) | 107.1(18) |
| C(3)-C(4)-H(4) | 108.6(18) |
| C(5)-C(4)-H(4) | 103.8(18) |
| O(2)-C(5)-N(1) | 120.9(3) |

(continued)

| | |
|---|---|
| O(2)-C(5)-C(4) | 123.5(3) |
| N(1)-C(5)-C(4) | 115.4(2) |
| O(4)-C(6)-N(2) | 123.6(3) |
| O(4)-C(6)-C(7) | 130.2(3) |
| N(2)-C(6)-C(7) | 106.2(2) |
| C(12)-C(7)-C(8) | 121.5(3) |
| C(12)-C(7)-C(6) | 108.7(2) |
| C(8)-C(7)-C(6) | 129.9(3) |
| N(3)-C(8)-C(7) | 121.3(3) |
| N(3)-C(8)-C(9) | 122.9(3) |
| C(7)-C(8)-C(9) | 115.9(3) |
| C(10)-C(9)-C(8) | 121.7(3) |
| C(10)-C(9)-H(9) | 121(2) |
| C(8)-C(9)-H(9) | 117(2) |
| C(9)-C(10)-C(11) | 122.5(3) |
| C(9)-C(10)-H(10) | 1.22(2) |
| C(11)-C(10)-H(10) | 116(2) |
| C(12)-C(11)-C(10) | 115.6(3) |
| C(12)-C(11)-H(11) | 119.5(18) |
| C(10)-C(11)-H(11) | 124.9(19) |
| C(11)-C(12)-C(7) | 122.8(3) |
| C(11)-C(12)-C(13) | 129.3(3) |
| C(7)-C(12)-C(13) | 107.9(2) |
| O(3)-C(13)-N(2) | 124.7(3) |
| O(3)-C(13)-C(12) | 129.8(3) |
| N(2)-C(13)-C(12) | 105.5(2) |

Table 12. Hydrogen coordinates (x $10^4$) and isotropic displacement parameters ($A^2$x$10^3$)

| | x | y | z | U(eq) |
|---|---|---|---|---|
| H(1) | 5370(60) | 8050(50) | 5390(40) | 50(11) |
| H(3A) | 3360(70) | 3240(70) | 11180(50) | 85(15) |
| H(3B) | 3260(70) | 1500(60) | 12440(50) | 76(14) |
| H(2A) | 700(50) | 6750(50) | 4820(40) | 44(10) |
| H(2B) | -200(60) | 8090(50) | 5540(40) | 56(11) |
| H(3C) | -340(50) | 5070(40) | 6810(30) | 30(8) |
| H(3D) | 750(50) | 6330(50) | 7380(40) | 53(11) |
| H(4) | 2590(40) | 4110(40) | 5870(30) | 33(8) |
| H(9) | 2650(50) | -1460(40) | 12370(30) | 39(9) |
| H(10) | 2310(40) | -3250(50) | 11090(30) | 27(8) |
| H(11) | 2090(50) | -2310(40) | 8960(30) | 33(8) |

Table 13. Torsion angles [°]

| | |
|---|---|
| C(5)-N(1)-C(1)-O(1) | -172.1(3) |
| C(5)-N(1)-C(1)-C(2) | 6.0(4) |
| O(1)-C(1)-C(2)-C(3) | -160.4(3) |
| N(1)-C(1)-C(2)-C(3) | 21.6(4) |
| C(1)-C(2)-C(3)-C(4) | -50.9(4) |
| C(13)-N(2)-C(4)-C(3) | 110.6(3) |

(continued)

| | |
|---|---|
| C(6)-N(2)-C(4)-C(3) | -67.2(4) |
| C(13)-N(2)-C(4)-C(5) | -122.2(3) |
| C(6)-N(2)-C(4)-C(5) | 60.0(4) |
| C(2)-C(3)-C(4)-N(2) | -179.3(3) |
| C(2)-C(3)-C(4)-C(5) | 54.2(4) |
| C(1)-N(1)-C(5)-O(2) | 173.0(3) |
| C(1I)-N(1)-C(5)-C(4) | -2.2(4) |
| N(2)-C(4)-C(5)-O(2) | 28.0(4) |
| C(3)-C(4)-C(5)-O(2) | 156.3(3) |
| N(2)-C(4)-C(5)-N(1) | -157.0(3) |
| C(3)-C(4)-C(5)-N(1) | -28.6(4) |
| C(13)-N(2)-C(6)-O(4) | 177.9(3) |
| C(4)-N(2)-C(6)-O(4) | -4.1(4) |
| C(13)-N(2)-C(6)-C(7) | -1.4(3) |
| C(4)-N(2)-C(6)-C(7) | 176.7(2) |
| O(4)-C(6)-C(7)-C(12) | -178.1(3) |
| N(2)-C(6)-C(7)-C(12) | 1.0(3) |
| O(4)-C(6)-C(7)-C(8) | 1.6(5) |
| N(2)-C(6)-C(7)-C(8) | -179.2(3) |
| C(12)-C(7)-C(8)-N(3) | -178.3(3) |
| C(6)-C(7)-C(8)-N(3) | 2.1(5) |
| C(12)-C(7)-C(8)-C(9) | 0.8(4) |
| C(6)-C(7)-C(8)-C(9) | -178.9(3) |
| N(3)-C(8)-C(9)-C(10) | 179.3(3) |
| C(7)-C(8)-C(9)-C(10) | 0.2(4) |
| C(8)-C(9)-C(10)-C(11) | -1.4(5) |
| C(9)-C(10)-C(11)-C(12) | 1.3(4) |
| C(10)-C(11)-C(12)-C(7) | -0.2(4) |
| C(10)-C(11)-C(12)-C(13) | 178.9(3) |
| C(8)-C(7)-C(12)-C(11) | -0.9(4) |
| C(6)-C(7)-C(12)-C(11) | 178.9(3) |
| C(8)-C(7)-C(12)-C(13) | 179.9(2) |
| C(6)-C(7)-C(12)-C(13) | -0.4(3) |
| C(6)-N(2)-C(13)-O(3) | -179.1(3) |
| C(4)-N(2)-C(13)-O(3) | 2.8(4) |
| C(6)-N(2)-C(13)-C(12) | 1.1(3) |
| C(4)-N(2)-C(13)-C(12) | -177.0(2) |
| C(11)-C(12)-C(13)-O(3) | 0.6(5) |
| C(7)-C(12)-C(13)-O(3) | 179.8(3) |
| C(11)-C(12)-C(13)-N(2) | -179.6(3) |
| C(7)-C(12)-C(13)-N(2) | -0.4(3) |

Infrared Spectrum ("FT-IR Spectrum): A representative FT-IR spectrum of pomalidomide was obtained as per USP <197K> in diffuse reflectance mode from KBr pellet. The FT-IR spectrum and its spectral assignments are shown in Figure 22 of WO2013/126326 and Table 14, respectively.

Table 14: Infrared Spectral Assignments of Pomalidomide

| Wave Number ($cm^{-1}$) | Functional Group |
|---|---|
| 3482, 3378 | Aniline N-H stretching |

(continued)

| Wave Number ($cm^{-1}$) | Functional Group |
|---|---|
| 3250 | Imide N-H stretch |
| 3115 | Ammatic C-H stretch |
| 2900 | Aliphatic C-H stretch |
| 1701, 1634 | Imide C-O stretching |
| 1595, 1480, 1409 | Aromatic C=C stretching |
| 1260 | Aniline C-N stretch |
| 470 - 1200 | Fingerprint region |

Hygroscopicity: Dynamic vapor sorption (DVS) analysis was carried out for two preparations of Form A using Surface Measurement Systems DVS. Representative DVS isotherm plots are presented in Figure 23 of WO2013/126326 and Figure 24 of WO2013/126326. In one study, the moisture uptake was less than about 0.14 % (wv'w) up to 95% relative humidity, indicative that pomalidomide Form A is not hygroscopic. In another study, Form A exhibited less than about 0.11% mass change over the dry mass when relative humidity was increased from 0 to 95%. At the end of desorption, the sample lost all of the weight it gained during the process of moisture sorption. After undergoing a full adsorption/desorption cycle, the sample had an XRPD pattern consistent with Form A X-Ray Powder Diffraction: XRPD of two preparations of Form A of pomalidomide was measured. The X-ray powder diffractograms are shown in Figure 25 and Figure 26 of WO2013/126326. Stability: The stability of Form A was demonstrated by exposing the sample to a 40°C/75% RH environment for four weeks. The physical properties of the exposed form A were unchanged as compared to at the start of the study. Form A also appeared to be stable in acetone, acetonitrile, ethanol, ethyl acetate, methyl ethyl ketone, THF, toluene, ethanoi-water (1 : 1) or water at 40°C for at least 4 weeks. Compression Test: A compression test for Form A was carried out by applying 2000-psi pressure to the sample for 1 minute and then analyzing the sample by XRPD. The XRPD pattern revealed that Form A was unchanged after compression (Figure 27 of WO2013/126326). Particle Size Distribution: In some embodiments, Form A was prepared as a white, irregular plate crystalline with a particle size D90 < 12 μιη. . Particle size distribution was determined for various lots of pomalidomide. Particle size data for representative lots are listed in Table 15.

Table 15: Particle Size Data for Pomalidomide Lots

| Lot Number | D (v, 0.5) | < 10μm | < 1μm |
|---|---|---|---|
| 1 | 11 | 46% | 0.0% |
| 2 | 15 | 31% | 0.0% |
| 3 | 17 | 31% | 0.0% |
| 4 | 11 | 43% | 0.0% |
| 5 | 9 | 57% | 0.0% |

Assays: TNFct Inhibition Assay in PBMC: Peripheral blood mononuclear cells (PBMC) from normal donors are obtained by Ficoll Hypaque (Pharmacia, Piscatawav, NJ, USA) density centnfugation. Cells are cultured in RPM 1640 (Life Technologies, Grand Island, NY, USA) supplemented with 10% AB+hirman serum (Gemini Bio-products, Woodland, CA, USA), 2 mM L~ glutamine, 100 U/ml penicillin, and 100 μg/ml streptomycin (Life Technologies). PBMC (2 x 105 cells) are plated in 96-well flat-bottom Costar tissue culture plates (Corning, NY, USA) in triplicate. Cells are stimulated with LPS (from Salmonella abortus equi, Sigma cat. no. L-1887, St. Louis, MO, USA) at 1 ng mL final in the absence or presence of compounds. Compounds provided herein are dissolved in DMSO (Sigma) and further dilutions are done in culture medium immediately before use. The final DMSO concentration in all assays can be about 0.25%. Compounds are added to cells 1 hour before LPS stimulation. Cells are then incubated for 18-20 hours at 37°C in 5 % $CO_2$, and supernatants are then collected, diluted with culture medium and assayed for TNFcc levels by ELISA (Endogen, Boston, MA, USA), IC5.3S are calculated using non-linear regression, sigmoidal dose-response, constraining the top to 100% and bottom to 0%, allowing variable slope (GraphPad Prism v3.02). IL-2 and MIP-3a Production by T Cells: PBMC are depleted of adherent monocytes by placing 1 x 108 PBMC in 10 ml complete medium (RPMI 1640 supplemented with 10% heat-inactivated fetal bovine serum, 2 mM L-glutamine, 100 U/ml penicillin, and 100 μg/ml streptomycin) per 10

cm tissue culture dish, in 37°C, 5 % C02 incubator for 30-60 minutes. The dish is rinsed with medium to remove all non-adherent PBMC. T cells are purified by negative selection using the following antibody (Pharmingen) and Dynabead (Dynai) mixture for every 1 x 108 non-adherent PBMC: 0.3 mi Sheep anti-mouse IgG beads, 15 μl anti-CD16, 15 μl anti-CD33, 15 μl anti-CD56, 0.23 ml anti-CD19 beads, 0.23 ml anti-HLA class II beads, and 56 μl anti-CD 14 beads. The cells and bead/antibody mixture is rotated end-over-end for 30-60 minutes at 4°C. Purified T cells are removed from beads using a Dynal magnet. Typical yield is about 50% T cells, 87-95% $CD3^+$ by flow cytometry. Tissue culture 96-well flat-bottom plates are coated with anti-CD3 antibody OKT3 at 5 ^ig/mi in PBS, 100 μl per well, incubated at 37°C for 3-6 hours, then washed four times with complete medium 100 μl/well just before T cells are added. Compounds are diluted to 20 times of final in a round bottom tissue culture 96-well plate. Final concentrations are about 10 μM to about 0.00064 μM. A 10 mM stock of compounds provided herein is diluted 1 :50 in complete for the first 20x dilution of 200 μM in 2 % DMSO and serially diluted 1 : 5 into 2 % DMSO. Compound is added at 10 μl per 200 μl culture, to give a final DMSO concentration of 0.1 %. Cultures are incubated at 37°C, 5 % $CO_2$ for 2-3 days, and supernatants analyzed for IL-2 and MTP-3a by ELISA (R&D Systems). IL-2 and MIP-3a levels are normalized to the amount produced in the presence of an amount of a compound provided herein, and EC50S calculated using nonlinear regression, sigmoidal dose-response, constraining the top to 100 % and bottom to 0 %, allowing variable slope (GraphPad Prism v3.02). Cell Proliferation Assay: Cell lines Namalwa, MUTZ-5, and UT-7 are obtained from the Deutsche Sammlung von Mikroorganismen und Zellkulturen GmbH (Braunschweig, Germany). The cell line KG-1 is obtained from the American Type Culture Collection (Manassas, VA, LISA). Cell proliferation as indicated by ^H-thymidine incorporation is measured in all cell lines as follows. Cells are plated in 96-well plates at 6000 cells per well in media. The cells are pre -treated with compounds at about 100, 10, 1, 0.1, 0.01, 0.001, 0.0001 and 0 μM in a final concentration of about 0.25 % DMSO in triplicate at 37°C in a humidified incubator at 5 % C02 for 72 hours. One microcurie of 'H-- thymidine (Amersham) is then added to each well, and cells are incubated again at 37°C in a humidified incubator at 5 % C02 for 6 hours. The cells are harvested onto UniFilter GF/C filter plates (Perkin Elmer) using a cell harvester (Tomtec), and the plates are allowed to dry overnight. Microscint 20 (Packard) (25 μl/well) is added, and plates are analyzed in TopCount NXT (Packard). Each wrelf is counted for one minute. Percent inhibition of cell proliferation is calculated by averaging all triplicates and normalizing to the DMSO control (0 % inhibition). Each compound is tested in each cell line in three separate experiments. Final \(' :,s are calculated using non-linear regression, sigmoidal dose-response, constraining the top to 100 % and bottom to 0 %, allowing variable slope. (GraphPad Prism v3.02). Immunoprecipitation and Immunoblot: Namalwa cells are treated with DMSO or an amount of a compound provided herein for 1 hour, then stimulated with 10 U/ml of Epo (R&D Systems) for 30 minutes. Cell lysates are prepared and either immunoprecipitated with Epo receptor Ab or separated immediately by SDS-PAGE. Immunoblots are probed with Akt, p ospo-Akt (Ser473 or Thr308), phospho-Gabl (Y627), Gabl, I S2, actin and IRF- 1 Abs and analyzed on a Storm 860 Imager using ImageQuant software (Molecular Dynamics). Cell Cycle Analysis: Cells are treated with DMSO or an amount of a compound provided herein overnight. Propidium iodide staining for cell cycle is performed using CycleTEST PLUS (Beet on Dickinson) according to manufacturer's protocol Following staining, cells are analyzed by a FACSCalibur flow cytometer using ModFit LT software (Becton Dickinson). Apoptosis Analysis: Cells are treated with DMSO or an amount of a compound provided herein at various time points, then washed with armexin-V wash buffer (BD Biosciences). Cells are incubated with annexin-V binding protein and propidiurn iodide (BD Biosciences) for 10 minutes. Samples are analyzed using flow cytometry. Luciferase Assay: Namalwa cells are transfected with 4 μg of API - luciferase (Stratagene) per 1 x 10° cells and 3 μl Lipofectamine 2000 (Invitrogen) reagent according to manufacturer's instructions. Six hours post-transfeciion, cells are treated with DMSO or an amount of a compound provided herein. Luciferase activity is assayed using luciferase lysis buffer and substrate (Promega) and measured using a luminometer (Turner Designs). The embodiments described above are intended to be merely exemplary, and those skilled in the art will recognize, or will be able to ascertain using no more than routine experimentation, numerous equivalents of specific compounds, materials, and procedures. All such equivalents are considered to be within the scope of the disclosure and are encompassed by the appended claims. All of the patents, patent applications and publications referred to herein are incorporated herein in their entireties. Citation or identification of any reference in this application is not an admission that such reference is available as prior art. The full scope of the disclosure is better understood with reference to the appended claims.

SPECIFIC INORMATION CONCERNING ASPECT (V) OF THE INVENTION

Aspect (v) of the invention relates to administration unit comprising solid form of 3-(4-nitro-1-oxoisoindolin-2-yl)piperidine-2,6-dione. The invention relates to solid forms of 3-(4-nitro-1-oxoisoindolin-2-yl)piperidine-2,6-dione, namely to solid form of 3-(4-nitro-1-oxoisoindolin-2-yl)piperidine-2,6-dione, which are useful for treating inflammatory diseases, autoimmune diseases, and cancer or for preparation of such medicaments for treating diseases. Solid form of 3-(4-nitro-1-oxoisoindolin-2-yl)piperidine-2,6-dione is described in WO2013/126394, which is incorporated by reference. As used herein, solid form of 3-(4-nitro-1-oxoisoindolin-2-yl)piperidine-2,6-dione are solid forms of 3-(4-nitro-1-oxoisoindolin-2-yl)piperidine-2,6-dione characterized by / obtained preferred as described in WO2013/126394, which is cited hereinafter:

Provided herein are solid forms of 3-(4-nitro-1-oxoisoindolin-2-yl)piperidine-2,6-dione, compositions comprising the solid forms, methods of making the solid forms and methods of their use for the preparation of 3-(4-amino-1-oxoisoindolin-2-yl)piperidine-2,6-dione and for the treatment of diseases and conditions including, but not limited to, inflammatory diseases, autoimmune diseases, and cancer.

Compounds may exist in different solid forms. The selection of a solid form of a pharmaceutical compound may affect a variety of physical and chemical properties, which may provide benefits or drawbacks in processing, formulation, stability and bioavailability, among other important pharmaceutical characteristics. Potential pharmaceutical solids include crystalline solids and amorphous solids. Amorphous solids are characterized by a lack of long-range structural order, whereas crystalline solids are characterized by structural periodicity. The desired class of pharmaceutical solid depends upon the specific application; amorphous solids are sometimes selected on the basis of, e.g., an enhanced dissolution profile, while crystalline solids may be desirable for properties such as, e.g., physical or chemical stability (see, e.g., S. R. Vippagunta et al., Adv. Drug. Deliv. Rev., (2001) 48:3-26; L. Yu, Adv. Drug. Deliv. Rev., (2001) 48:27-42). Whether crystalline or amorphous, potential solid forms of a pharmaceutical compound may include single-component and multiple-component solids. Single-component solids consist essentially of the pharmaceutical compound in the absence of other compounds. Variety among single-component crystalline materials may potentially arise from the phenomenon of polymorphism, wherein multiple three-dimensional arrangements exist for a particular pharmaceutical compound {see, e.g., S. R. Byrn et ah, Solid State Chemistry of Drugs, (1999) SSCI, West Lafayette). Additional diversity among the potential solid forms of a pharmaceutical compound may arise from the possibility of multiple-component solids. Crystalline solids comprising two or more ionic species are termed salts (see, e.g., Handbook of Pharmaceutical Salts: Properties, Selection and Use, P. H. Stahl and C. G. Wermuth, Eds., (2002), Wiley, Weinheim). Additional types of multiple-component solids that may potentially offer other property improvements for a pharmaceutical compound or salt thereof include, e.g., hydrates, solvates, co-crystals and clathrates, among others (see, e.g., S. R. Bym et ah, Solid State Chemistry of Drugs, (1999) SSCI, West Lafayette). Moreover, multiple-component crystal forms may potentially be susceptible to polymorphism, wherein a given multiple-component composition may exist in more than one three-dimensional crystalline arrangement. The discovery of solid forms is of great importance in the development of a safe, effective, stable and marketable pharmaceutical compound. U.S. Patent Nos. 5,635,517 and 6,281,230, both to Muller et al, disclose 3-(4-nitro-1-oxoisoindolin-2-yl)piperidine -2,6-dione, which is useful in preparing 3-(4-amino-1-oxoisoindolin-2-yl)piperidine -2,6-dione, which is useful in treating and preventing a wide range of diseases and conditions including, but not limited to, inflammatory diseases, autoimmune diseases, and cancer. New polymorphic forms of 3-(4-nitro-1-oxoisoindolin-2-yl)piperidine-2,6-dione can further the preparation of 3-(4-amino-1-oxoisoindolin-2-yl)piperidine -2,6-dione and can further the development of formulations for the treatment of these chronic illnesses, and may yield numerous formulation, manufacturing and therapeutic benefits.

The invention relates to 3-(4-nitro-1-oxoisoindolin-2-yl)piperidine-2,6-dione ({drug5}). In some embodiments, provided herein are solid forms of 3-(4-nitro-1-oxoisoindolin-2-yl)piperidine-2,6-dione ({drug5a}). In some embodiments, provided herein are polymorphs of the compound identified herein as forms A, B, and C. In some embodiments, provided herein are mixtures of these forms. In further embodiments, provided herein are methods of making, isolating and characterizing the polymorphs. In some embodiments, provided herein are pharmaceutical compositions and single unit dosage forms comprising a polymorph of 3-(4-nitro-1-oxoisoindolin-2-yl)piperidine-2,6-dione. In some embodiments, provided herein are methods for the treatment or prevention of a variety of diseases and disorders, which comprise administering to a patient in need of such treatment or prevention a therapeutically effective amount of a polymorph of 3-(4-nitro-1-oxoisoindolin-2-yl)piperidine-2,6-dione.

Figure 1 of WO2013/126394 provides a representative X-ray powder diffraction (XRPD) pattern of Form A ({drug5b}); Figure 2 of WO2013/126394 provides a representative Miniature Scanning Electron Microscope (Mini SEM) picture of Form A ({drug5b}); Figure 3 of WO2013/126394 provides a representative IR spectrum of Form A ({drug5b}); Figure 4 of WO2013/126394 provides a representative thermogravimetric analysis (TGA) curve and a representative differential scanning calorimeter (DSC) thermogram of Form A ({drug5b}); Figure 5 of WO2013/126394 provides a representative moisture sorption/desorption isotherm of Form A ({drug5b}); Figure 6 of WO2013/126394 provides a representative X-ray powder diffraction (XRPD) pattern of Form A ({drug5b}) after Dynamic Vapor Sorption (DVS); Figure 7 of WO2013/126394 provides a representative X-ray powder diffraction (XRPD) pattern of Form B ({drug5c}); Figure 8 of WO2013/126394 provides a representative Miniature Scanning Electron Microscope (Mini SEM) picture of Form B ({drug5c}); Figure 9 of WO2013/126394 provides a representative IR spectrum of Form B ({drug5c}); Figure 10 of WO2013/126394 provides a representative thermogravimetric analysis (TGA) curve and a representative differential scanning calorimeter (DSC) thermogram of Form B ({drug5c}); Figure 11 of WO2013/126394 provides a representative moisture sorption/desorption isotherm of Form B ({drug5c}); Figure 12 of WO2013/126394 provides a representative X-ray powder diffraction (XRPD) pattern of Form B ({drug5c}) after Dynamic Vapor Sorption (DVS); Figure 13 of WO2013/126394 provides a representative X-ray powder diffraction (XRPD) pattern of solid resulted from heating Form B ({drug5c}) at 220°C for 4 hours; Figure 14 of WO2013/126394 provides a representative X-ray powder diffraction (XRPD) pattern of Form C ({drug5d}); Figure 15 of WO2013/126394 provides a representative Miniature Scanning

Electron Microscope (Mini SEM) picture of Form C ({drug5d}); Figure 16 of WO2013/126394 provides a representative IR spectrum of Form C ({drug5d}); Figure 17 of WO2013/126394 provides a representative thermogravimetric analysis (TGA) curve and a representative differential scanning calorimeter (DSC) thermogram of Form C ({drug5d}); Figure 18 of WO2013/126394 provides a representative moisture sorption/desorption isotherm of Form C ({drug5d}); Figure 19 of WO2013/126394 provides a representative X-ray powder diffraction (XRPD) pattern of Form C ({drug5d}) after Dynamic Vapor Sorption (DVS); Figure 20 of WO2013/126394 provides a representative X-ray powder diffraction (XRPD) pattern of solid resulted from heating Form C ({drug5d}) at 220°C for 4 hours. Figure 21 of WO2013/126394 provides an X-ray powder diffraction (XRPD) stack plot of Forms A, B and C. Figure 22 of WO2013/126394 provides an IR overlay plot of Forms A, B and C. Figure 23 of WO2013/126394 provides an X-ray powder diffraction (XRPD) stack plot of four batches of Compound of formula (V). Figure 24 of WO2013/126394 provides an IR overlay plot of four batches of Compound of formula (V).

As used herein and unless otherwise indicated, the terms "treat," "treating" and "treatment" refer to the alleviation of a disease or disorder and/or at least one of its attendant symptoms. As used herein and unless otherwise indicated, the terms "prevent," "preventing" and "prevention" refer to the inhibition of a symptom of a disease or disorder or the disease itself. As used herein and unless otherwise specified, the terms "solid form" and related terms refer to a physical form which is not predominantly in a liquid or a gaseous state. Solid forms may be crystalline, amorphous or mixtures thereof. In particular embodiments, solid forms may be liquid crystals. A solid form may be a single component or multiple component solid form. A "single-component" solid form comprising a compound consists essentially of the compound. A "multiple-component" solid form comprising a compound comprises a significant quantity of one or more additional species, such as ions and/or molecules, within the solid form. For example, in particular embodiments, a crystalline multiple-component solid form comprising a compound further comprises one or more species non-covalently bonded at regular positions in the crystal lattice. As used herein and unless otherwise specified, the term "crystalline" and related terms used herein, when used to describe a substance, modification, material, component or product, unless otherwise specified, mean that the substance, modification, material, component or product is substantially crystalline as determined by X-ray diffraction. See, e.g., Remington: The Science and Practice of Pharmacy, 21st edition, Lippincott, Williams and Wilkins, Baltimore, MD (2005); The United States Pharmacopeia, 23rd edition, 1843-1844 (1995). As used herein and unless otherwise specified, the term "crystal forms" and related terms herein refer to solid forms that are crystalline. Crystal forms include single- component crystal forms and multiple-component crystal forms, and include, but are not limited to, polymorphs, solvates, hydrates, and other molecular complexes, as well as salts, solvates of salts, hydrates of salts, other molecular complexes of salts, and polymorphs thereof. In certain embodiments, a crystal form of a substance may be substantially free of amorphous forms and/or other crystal forms. In certain embodiments, a crystal form of a substance may contain less than about 1%, 2%, 3%, 4%, 5%, 6%, 7%, 8%, 9%, 10%, 15%, 20%, 25%, 30%, 35%, 40%, 45% or 50% of one or more amorphous forms and/or other crystal forms on a weight basis. In certain embodiments, a crystal form of a substance may be physically and/or chemically pure. In certain embodiments, a crystal form of a substance may be about 99%, 98%>, 97%, 96%, 95%, 94%, 93%), 92%), 91) or 90% physically and/or chemically pure. As used herein and unless otherwise specified, the terms "polymorphs", "polymorphic forms" and related terms herein, refer to two or more crystal forms that consist essentially of the same molecule, molecules or ions. Like different crystal forms, different polymorphs may have different physical properties such as, for example, melting temperatures, heats of fusion, solubilities, dissolution rates and/or vibrational spectra, as a result of the arrangement or conformation of the molecules and/or ions in the crystal lattice. The differences in physical properties may affect pharmaceutical parameters such as storage stability, compressibility and density (important in formulation and product manufacturing), and dissolution rate (an important factor in bioavailability). Differences in stability can result from changes in chemical reactivity (e.g., differential oxidation, such that a dosage form discolors more rapidly when comprised of one polymorph than when comprised of another polymorph) or mechanical changes (e.g., tablets crumble on storage as a kinetically favored polymorph converts to a thermodynamically more stable polymorph) or both (e.g., tablets of one polymorph are more susceptible to breakdown at high humidity). As a result of solubility/dissolution differences, in the extreme case, some solid-state transitions may result in lack of potency or, at the other extreme, toxicity. In addition, the physical properties may be important in processing (for example, one polymorph might be more likely to form solvates or might be difficult to filter and wash free of impurities, and particle shape and size distribution might be different between polymorphs). Polymorphs of a molecule can be obtained by a number of methods known in the art. Such methods include, but are not limited to, melt recrystallization, melt cooling, solvent recrystallization, desolvation, rapid evaporation, rapid cooling, slow cooling, vapor diffusion and sublimation. Polymorphs can be detected, identified, classified and characterized using well-known techniques such as, but not limited to, differential scanning calorimetry (DSC), thermogravimetry (TGA), X-ray powder diffractometry (XRPD), single crystal X-ray diffractometry, vibrational spectroscopy, solution calorimetry, solid state nuclear magnetic resonance (NMR), infrared (IR) spectroscopy, Raman spectroscopy, hot stage optical microscopy, scanning electron microscopy (SEM), electron crystallography and quantitative analysis, particle size analysis (PSA), surface area analysis, solubility, and rate of dissolution. As used herein to refer to the spectra or data presented in graphical form (e.g., XRPD, IR, Raman and NMR spectra), and unless otherwise indicated, the term "peak"

refers to a peak or other special feature that one skilled in the art would recognize as not attributable to background noise. The term "significant peaks" refers to peaks at least the median size (e.g., height) of other peaks in the spectrum or data, or at least 1.5, 2, or 2.5 times the median size of other peaks in the spectrum or data. As used herein and unless otherwise indicated, the term "substantially pure" when used to describe a polymorph of a compound means a solid form of the compound that comprises that polymorph and is substantially free of other polymorphs of the compound. A representative substantially pure polymorph comprises greater than about 80% by weight of one polymorphic form of the compound and less than about 20% by weight of other polymorphic forms of the compound, more preferably greater than about 90% by weight of one polymorphic form of the compound and less than about 10% by weight of the other polymorphic forms of the compound, even more preferably greater than about 95% by weight of one polymorphic form of the compound and less than about 5% by weight of the other polymorphic forms of the compound, and most preferably greater than about 97% by weight of one polymorphic forms of the compound and less than about 3% by weight of the other polymorphic forms of the compound. The term "substantially similar," when used herein in the context of comparing spectra such as, but not limited to, X-ray powder diffraction pattern or differential scanning calorimetry scan obtained for a solid form, means that two spectra share defining characteristics sufficient to differentiate them from a spectrum obtained for a different solid form. In certain embodiments, the term "substantially similar" means that two spectra are the same, i.e., visibly overlap. In certain embodiments, spectra or characterization data that are substantially similar to those of a reference crystalline form, amorphous form, or mixture thereof, is understood by those of ordinary skill in the art to correspond to the same crystalline form, amorphous form, or mixture thereof as the particular reference. In analyzing whether spectra or characterization data are substantially similar, a person of ordinary skill in the art understands that particular characterization data points may vary to a reasonable extent while still describing a given solid form, due to, for example, experimental error and routine sample-to-sample analysis.

Solid forms: In some embodiments, provided herein are solid forms comprising crystalline 3-(4-nitro-1-oxoisoindolin-2-yl)piperidine-2,6-dione, which is a compound of Formula (V) ({drug5})

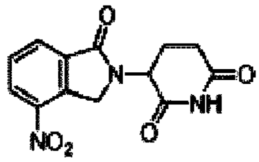

(V).

This compound can be prepared according to the methods described in U.S. Patent Nos. 6,281,230 and 5,635,517, the entireties of which are incorporated herein by reference. For example, the compound can be prepared by allowing 2,6-dioxopiperidin-3-ammonium chloride to react with methyl 2-bromomethyl-3-nitrobenzoate in dimethylformamide in the presence of triethylamine. The methyl 2-bromomethyl-3-nitrobenzoate in turn is obtained from the corresponding methyl ester of 3-nitro-ortho-toluic acid by conventional bromination with N-bromosuccinimide under the influence of light. The compound can be used for the preparation of 3-(4-amino-1-oxoisoindolin-2-yl)piperidine-2,6-dione through catalytic hydrogenation. Polymorphs of 3-(4-nitro-1-oxoisoindolin-2-yl)piperidine-2,6-dione can be obtained by techniques known in the art, including solvent recrystallization, desolvation, vapor diffusion, rapid evaporation, slow evaporation, rapid cooling and slow cooling. Polymorphs can be made by dissolving a weighed quantity of 3-(4-nitro-1-oxoisoindolin-2-yl)piperidine-2,6-dione in various solvents at elevated temperatures. The solutions of the compound can then be filtered and allowed to evaporate either in an open vial (for fast hot evaporation) or in a vial covered with aluminum foil containing pinholes (hot slow evaporation). Polymorphs can also be obtained from slurries. Polymorphs can be crystallized from solutions or slurries using several methods. For example, a solution created at an elevated temperature (e.g., 60°C) can be filtered quickly then allowed to cool to room temperature. Once at room temperature, the sample that did not crystallize can be moved to a refrigerator then filtered. Alternatively, the solutions can be crash cooled by dissolving the solid in a solvent at an increased temperature (e.g., 45-65°C) followed by cooling in a dry ice/solvent bath. In one embodiment, provided herein is Form A ({drug5b}) of 3-(4-nitro-1-oxoisoindolin-2-yl)piperidine-2,6-dione. Form A ({drug5b}) is an anhydrate, crystalline material. In another embodiment, provided herein is Form B ({drug5c}) of 3-(4-nitro-1-oxoisoindolin-2-yl)piperidine-2,6-dione. Form B ({drug5c}) is an anhydrate, crystalline material that is monotropically related to Form A ({drug5b}). In yet another embodiment, provided herein is Form C ({drug5d}) of 3-(4-nitro-1-oxoisoindolin-2-yl)piperidine-2,6-dione. Form C ({drug5d}) is an anhydrate, crystalline material that is monotropically related to Form A ({drug5b}). Each of these forms is discussed in detail below. In one embodiment, provided herein is a composition comprising amorphous 3-(4-nitro-1-oxoisoindolin-2-yl)piperidine-2,6-dione and crystalline 3-(4-nitro-1-oxoisoindolin-2-yl)piperidine-2,6-dione of Form A ({drug5b}), B and C. Specific compositions can comprise greater than 50, 75, 90 or 95 weight percent crystalline 3-(4-nitro-1-oxoisoindolin-2-yl)piperidine-2,6-dione. In another embodiment, provided herein is a composition comprising at least two crystalline forms of 3-(4-nitro-1-oxoisoindolin-2-yl)piperidine-2,6-dione (e.g., a mixture of polymorph forms A and C).

Form A ({drug5b}): Form A ({drug5b}) can be obtained using the experimental methods described in Example 6.1, provided below. A representative XRPD pattern of Form A ({drug5b}) is provided in Figure 1 of WO2013/126394. In certain embodiments, Form A ({drug5b}) of Compound of formula (V) is characterized by XRPD peaks, preferably significant peaks, located at, at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, or all of the following approximate positions: 10.30, 10.59, 12.80, 13.03, 14.85, 15.15, 16.40, 16.93, 18.39, 18.55, 20.71, 21.08, 21.28, 21.53, 22.08, 22.63, 24.91, 25.40, 25.78, 26.25, 28.70, 29.55, 30.02, 30.31, 30.69, 31.00, 31.56, 32.43, 33.17, 34.34, 35.78, 36.68, and 39.12 degrees 2θ, plus or minus 0.10. In some embodiments, Form A ({drug5b}) has at least 8, at least 9, or at least 10 peaks. In some embodiments, Form A ({drug5b}) has at least 10 peaks. In certain embodiments, Form A ({drug5b}) is characterized by peaks in an XRPD pattern located at 1, 2, 3, 4, 5, 6, or all of the following approximate peak positions: 10.30 ± 0.10, 15.15 ± 0.10,18.39 ± 0.10,21.28 ± 0.10,24.91 ± 0.10,26.25 ± 0.10, and 29.55 ± 0.10 degrees 2θ. In certain embodiments, Form A ({drug5b}) is characterized by two or more peaks selected from 10.30 ± 0.10, 15.15 ± 0.10, 18.39 ± 0.10, 21.28 ± 0.10, 24.91 ± 0.10, 26.25 ± 0.10, and 29.55 ± 0.10 degrees 2θ. In certain embodiments, Form A ({drug5b}) is characterized by three or more peaks selected from 10.30 ± 0.10, 15.15 ± 0.10, 18.39 ± 0.10, 21.28 ± 0.10, 24.91 ± 0.10, 26.25 ± 0.10, and 29.55 ± 0.10 degrees 2θ. In certain embodiments, Form A ({drug5b}) is characterized by four or more peaks selected from 10.30 ± 0.10, 15.15 ± 0.10, 18.39 ± 0.10, 21.28 ± 0.10, 24.91 ± 0.10, 26.25 ± 0.10, and 29.55 ± 0.10 degrees 2θ. In certain embodiments, Form A ({drug5b}) is characterized by five or more peaks selected from 10.30 ± 0.10,15.15 ± 0.10,18.39 ± 0.10,21.28 ± 0.10,24.91 ± 0.10, 26.25 ± 0.10, and 29.55 ± 0.10 degrees 2θ. In certain embodiments, Form A ({drug5b}) is characterized by six or more peaks selected from 10.30 ± 0.10, 15.15 ± 0.10, 18.39 ± 0.10,21.28 ± 0.10,24.91 ± 0.10, 26.25 ± 0.10, and 29.55 ± 0.10 degrees 2θ. In certain embodiments, Form A ({drug5b}) is characterized by all of the following peaks: 10.30 ± 0.10, 15.15 ± 0.10, 18.39 ± 0.10, 21.28 ± 0.10, 24.91 ± 0.10, 26.25 ± 0.10, and 29.55 ± 0.10 degrees 2θ. In certain embodiments, Form A ({drug5b}) is characterized by an XRPD pattern substantially similar to the XRPD pattern presented in Figure 1 of WO2013/126394. In certain embodiments, provided herein is a solid form of Compound of formula (V) having an XRPD pattern comprising peaks at approximately 10.30, 24.91, and 26.25 degrees 2θ. In further embodiments, the XRPD pattern further comprises peaks at approximately 15.15, 18.39,21.28, and 29.55 degrees 2θ. In even further embodiments, the XRPD pattern further comprises peaks at approximately 10.59, 13.03, 14.85, 16.40, 16.93, 18.55, 20.71, 22.08, 22.63, 25.40, and 25.78 degrees 2θ. In some embodiments, the XRPD peaks above (degrees 2θ peaks) are when analyzed using copper Ka radiation. In some embodiments, provided herein is a solid form of Compound of formula (V), which has an XRPD pattern comprising peaks at approximately 10.30,24.91, and 26.25 degrees 2θ when analyzed using copper Ka radiation. Form A ({drug5b}) has an irregular rod habit as shown in Figure 2 of WO2013/126394. Representative IR spectrum data of Form A ({drug5b}) is provided in Figure 3 of WO2013/126394. In certain embodiments, provided herein is a solid form of Compound of formula (V), wherein the solid form is characterized by an IR spectrum substantially similar to the IR spectrum presented in Figure 3 of WO2013/126394. Representative thermal characteristics of Form A ({drug5b}) are shown in Figure 4 of WO2013/126394. The TGA data shows minimal weight loss up to about 160°C. In certain embodiments, provided herein is a solid form of Compound of formula (V), wherein the solid form is characterized by a TGA thermogram substantially similar to the TGA thermogram presented in Figure 4 of WO2013/126394. The DSC thermogram shows only one major event at slightly above 300°C. These data indicate that Form A ({drug5b}) is an anhydrate. In certain embodiments, the temperature maximum at slightly above 300°C corresponds to decomposition of Compound of formula (V). In certain embodiments, provided herein is a solid form of Compound of formula (V), wherein the solid form is characterized by a DSC thermogram substantially similar to the DSC thermogram presented in Figure 4 of WO2013/126394. Representative moisture sorption and desorption data of Form A ({drug5b}) are plotted in Figure 5 of WO2013/126394. In certain embodiments, Form A ({drug5b}) exhibits a total mass change of 0.42% between 0% and 90%) relative humidity, suggesting Form A ({drug5b}) is not hygroscopic. In certain embodiments, provided herein is a solid form of Compound of formula (V), wherein the solid form is characterized by a DVS plot substantially similar to the DVS plot presented in Figure 5 of WO2013/126394. After undergoing the adsorption/desorption cycles, the XRPD pattern of Form A ({drug5b}) shows no change, as shown in Figure 6 of WO2013/126394. In certain embodiments, Form A ({drug5b}) is characterized by an XRPD pattern substantially similar to the XRPD pattern presented in Figure 6 of WO2013/126394. Form conversion studies show that Form A ({drug5b}) appears to be the most thermodynamically stable form of 3-(4-nitro-1-oxoisoindolin-2-yl)piperidine-2,6-dione discovered thus far, and no conversion from Form A ({drug5b}) to other forms has been observed. All of the combinations of the above embodiments are encompassed by this invention.

Form B ({drug5c}): Form B ({drug5c}) can be obtained using the experimental methods described in Example 6.1, provided below. A representative XRPD pattern of Form B ({drug5c}) is provided in Figure 7 of WO2013/126394. In certain embodiments, Form B ({drug5c}) of Compound of formula (V) is characterized by XRPD peaks, preferably significant peaks, located at, at least 1, at least 2, at least 3, at least 4, at least 5, at least 6, at least 7, at least 8, at least

9, at least 10, at least 11, at least 12, at least 13, at least 14, at least 15, at least 16, at least 17, at least 18, at least 19, at least 20, at least 21, at least 22, at least 23, at least 24, at least 25, or all of the following approximate positions: 10.32, 13.94, 14.63, 15.46, 15.72, 16.83,17.14, 18.82, 20.68, 22.48, 22.69, 24.27, 24.79, 25.86, 26.26, 26.99, 27.33, 27.69, 28.01, 29.45, 30.49, 30.56, 34.64, 36.33, 36.97, and 39.15 degrees 2θ, plus or minus 0.10. In some embodiments, Form B ({drug5c}) has at least 8, at least 9, or at least 10 peaks. In some embodiments, Form B ({drug5c}) has at least 10 peaks. In certain embodiments, Form B ({drug5c}) is characterized by peaks in an XRPD pattern located at 1, 2, 3, 4, 5, 6, 7, 8, or all of the following approximate peak positions: 10.32 ± 0.10, 13.94 ± 0.10, 14.63 ± 0.10, 15.72 ± 0.10, 17.14 ± 0.10, 18.82 ± 0.10, 20.68 ± 0.10, 22.69 ± 0.10, and 27.69 ± 0.10 degrees 2θ. In certain embodiments, Form B ({drug5c}) is characterized by two or more peaks selected from 10.32 ± 0.10,13.94 ± 0.10,14.63 ± 0.10,15.72 ± 0.10,17.14 ± 0.10,18.82 ± 0.10,20.68 ± 0.10, 22.69 ± 0.10, and 27.69 ± 0.10 degrees 2θ. In certain embodiments, Form B ({drug5c}) is characterized by three or more peaks selected from 10.32 ± 0.10, 13.94 ± 0.10, 14.63 ± 0.10, 15.72 ± 0.10, 17.14 ± 0.10, 18.82 ± 0.10, 20.68 ± 0.10, 22.69 ± 0.10, and 27.69 ± 0.10 degrees 2θ. In certain embodiments, Form B ({drug5c}) is characterized by four or more peaks selected from 10.32 ± 0.10, 13.94 ± 0.10, 14.63 ± 0.10, 15.72 ± 0.10,17.14 ± 0.10, 18.82 ± 0.10, 20.68 ± 0.10, 22.69 ± 0.10, and 27.69 ± 0.10 degrees 2θ. In certain embodiments, Form B ({drug5c}) is characterized by five or more peaks selected from 10.32 ± 0.10, 13.94 ± 0.10, 14.63 ± 0.10, 15.72 ± 0.10, 17.14 ± 0.10, 18.82 ± 0.10, 20.68 ± 0.10, 22.69 ± 0.10, and 27.69 ± 0.10 degrees 2θ. In certain embodiments, Form B ({drug5c}) is characterized by six or more peaks selected from 10.32 ± 0.10, 13.94 ± 0.10, 14.63 ± 0.10, 15.72 ± 0.10, 17.14 ± 0.10, 18.82 ± 0.10, 20.68 ± 0.10, 22.69 ± 0.10, and 27.69 ± 0.10 degrees 2θ. In certain embodiments, Form B ({drug5c}) is characterized by seven or more peaks selected from 10.32 ± 0.10,13.94 ± 0.10, 14.63 ± 0.10, 15.72 ± 0.10, 17.14 ± 0.10, 18.82 ± 0.10, 20.68 ± 0.10, 22.69 ± 0.10, and 27.69 ± 0.10 degrees 2θ. In certain embodiments, Form B ({drug5c}) is characterized by eight or more peaks selected from 10.32 ± 0.10, 13.94 ± 0.10, 14.63 ± 0.10, 15.72 ± 0.10, 17.14 ± 0.10, 18.82 ± 0.10, 20.68 ± 0.10, 22.69 ± 0.10, and 27.69 ± 0.10 degrees 2θ. In certain embodiments, Form B ({drug5c}) is characterized by all of the following peaks: 10.32 ± 0.10, 13.94 ± 0.10, 14.63 ± 0.10, 15.72 ± 0.10, 17.14 ± 0.10, 18.82 ± 0.10, 20.68 ± 0.10, 22.69 ± 0.10, and 27.69 ± 0.10 degrees 2θ. In certain embodiments, Form B ({drug5c}) is characterized by an XRPD pattern substantially similar to the XRPD pattern presented in Figure 7 of WO2013/126394. In certain embodiments, provided herein is a solid form of Compound of formula (V) having an XRPD pattern comprising peaks at approximately 13.94, 14.63, and 17.14 degrees 2θ. In further embodiments, the XRPD pattern further comprises peaks at approximately 10.32, 15.72, 18.82, 20.68, 22.69, and 27.69 degrees 2θ. In some embodiments, the XRPD peaks above (degrees 2θ peaks) are when analyzed using copper Ka radiation. In some embodiments, provided herein is a solid form of Compound of formula (V), which has an XRPD pattern comprising peaks at approximately 13.94, 14.63, and 17.14 degrees 2θ when analyzed using copper Ka radiation. Form B ({drug5c}) has an irregular rod habit as shown in Figure 8 of WO2013/126394. Representative IR spectrum data of Form B ({drug5c}) is provided in Figure 9 of WO2013/126394. In certain embodiments, provided herein is a solid form of Compound of formula (V), wherein the solid form is characterized by an IR spectrum substantially similar to the IR spectrum presented in Figure 9 of WO2013/126394. Representative thermal characteristics of Form B ({drug5c}) are shown in Figure 10 of WO2013/126394. The TGA data shows minimal weight loss up to about 150°C. In certain embodiments, provided herein is a solid form of Compound of formula (V), wherein the solid form is characterized by a TGA thermogram substantially similar to the TGA thermogram presented in Figure 10 of WO2013/126394. The DSC thermogram shows only one major event at slightly above 300°C. These data indicate that Form B ({drug5c}) is an anhydrate. In certain embodiments, the temperature maximum at slightly above 300°C corresponds to decomposition of Compound of formula (V). In certain embodiments, provided herein is a solid form of Compound of formula (V), wherein the solid form is characterized by a DSC thermogram substantially similar to the DSC thermogram presented in Figure 10 of WO2013/126394. Representative moisture sorption and desorption data of Form B ({drug5c}) are plotted in Figure 11 of WO2013/126394. In certain embodiments, Form B ({drug5c}) exhibits a total mass change of 0.39% between 0% and 90%) relative humidity, suggesting Form B ({drug5c}) is not hygroscopic. In certain embodiments, provided herein is a solid form of Compound of formula (V), wherein the solid form is characterized by a DVS plot substantially similar to the DVS plot presented in Figure 11 of WO2013/126394. After undergoing the adsorption/desorption cycles, the XRPD pattern of Form B ({drug5c}) shows no change, as shown in Figure 12 of WO2013/126394. In certain embodiments, Form B ({drug5c}) is characterized by an XRPD pattern substantially similar to the XRPD pattern presented in Figure 12 of WO2013/126394. Conversion studies show that Form B ({drug5c}) typically converts to Form A ({drug5b}) in acetonitrile at 80°C, in acetonitrile/water (2: 1) at 60°C, and in acetonitrile/water (1 : 1) at ambient temperature. Form B ({drug5c}) also converts to Form A ({drug5b}) at ambient temperature in other solvent systems such as acetone, water, acetone/water (1 :1), and acetonitrile/water/acetone (1 :1 :1). Form B ({drug5c}) also converts to Form A ({drug5b}) upon heating up to 220°C for 4 hours, which is evidenced by the XRPD pattern of the resulting solid, as shown in Figure 13 of WO2013/126394. These observations suggest that Form B ({drug5c}) is monotropically related to Form A ({drug5b}). In certain embodiments, Form A ({drug5b}) is characterized by an XRPD pattern substantially similar to the XRPD pattern presented in Figure 13 of WO2013/126394. All of the combinations of the above embodiments are encompassed by this invention.

Form C ({drug5d}): Form C ({drug5d}) can be obtained from recrystallization of 3-(4-nitro-1-oxoisoindolin-2-yl)piperidine-2,6-dione, using 1-methyl-2-pyrrolidinone (NMP) as primary solvent, with anti-solvent of acetonitrile at 80°C, acetonitrile/water (2: 1) at 60°C, or acetonitrile/water (1 : 1) at ambient temperature. A representative XRPD pattern of Form C ({drug5d}) is provided in Figure 14 of WO2013/126394. In certain embodiments, Form C ({drug5d}) of Compound of formula (V) is characterized by XRPD peaks, preferably significant peaks, located at, at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, or all of the following approximate positions: 10.25,13.01,15.40,17.13,18.62,20.56, 21.07, 21.96, 22.67, 24.84, 25.78, 26.17, 27.90, 29.50, 30.68, 31.04, 36.36, 37.89, and 39.05 degrees 2θ, plus or minus 0.10. In some embodiments, Form C ({drug5d}) has at least 8, at least 9, or at least 10 peaks. In some embodiments, Form C ({drug5d}) has at least 10 peaks. In certain embodiments, Form C ({drug5d}) is characterized by peaks in an XRPD pattern located at 1, 2, 3, 4, 5, 6, 7, or all of the following approximate peak positions: 10.25 ± 0.10, 13.01 ± 0.10, 15.40 ± 0.10, 20.56 ± 0.10, 21.07 ± 0.10, 24.84 ± 0.10, 25.78 ± 0.10, and 26.17 ± 0.10 degrees 2θ. In certain embodiments, Form C ({drug5d}) is characterized by two or more peaks selected from 10.25 ± 0.10, 13.01 ± 0.10, 15.40 ± 0.10, 20.56 ± 0.10, 21.07 ± 0.10, 24.84 ± 0.10, 25.78 ± 0.10, and 26.17 ± 0.10 degrees 2θ. In certain embodiments, Form C ({drug5d}) is characterized by three or more peaks selected from 10.25 ± 0.10, 13.01 ± 0.10, 15.40 ± 0.10, 20.56 ± 0.10, 21.07 ± 0.10, 24.84 ± 0.10, 25.78 ± 0.10, and 26.17 ± 0.10 degrees 2θ. In certain embodiments, Form C ({drug5d}) is characterized by four or more peaks selected from 10.25 ± 0.10,13.01 ± 0.10, 15.40 ± 0.10, 20.56 ± 0.10, 21.07 ± 0.10, 24.84 ± 0.10, 25.78 ± 0.10, and 26.17 ± 0.10 degrees 2θ. In certain embodiments, Form C ({drug5d}) is characterized by five or more peaks selected from 10.25 ± 0.10, 13.01 ± 0.10, 15.40 ± 0.10, 20.56 ± 0.10, 21.07 ± 0.10, 24.84 ± 0.10,25.78 ± 0.10, and 26.17 ± 0.10 degrees 2θ. In certain embodiments, Form C ({drug5d}) is characterized by six or more peaks selected from 10.25 ± 0.10, 13.01 ± 0.10, 15.40 ± 0.10, 20.56 ± 0.10, 21.07 ± 0.10, 24.84 ± 0.10, 25.78 ± 0.10, and 26.17 ± 0.10 degrees 2θ. In certain embodiments, Form C ({drug5d}) is characterized by seven or more peaks selected from 10.25 ± 0.10,13.01 ± 0.10, 15.40 ± 0.10, 20.56 ± 0.10, 21.07 ± 0.10, 24.84 ± 0.10, 25.78 ± 0.10, and 26.17 ± 0.10 degrees 2θ. In certain embodiments, Form C ({drug5d}) is characterized by all of the following peaks: 10.25 ± 0.10, 13.01 ± 0.10, 15.40 ± 0.10, 20.56 ± 0.10, 21.07 ± 0.10, 24.84 ± 0.10, 25.78 ± 0.10, and 26.17 f 0.10 degrees 2θ. In certain embodiments, Form C ({drug5d}) is characterized by an XRPD pattern substantially similar to the XRPD pattern presented in Figure 14 of WO2013/126394. In certain embodiments, provided herein is a solid form of Compound of formula (V) having an XRPD pattern comprising peaks at approximately 10.25, 15.40, and 26.17 degrees 2θ. In further embodiments, the XRPD pattern further comprises peaks at approximately 20.56, 21.07, and 25.78 degrees 2θ. In even further embodiments, the XRPD pattern further comprises peaks at approximately 13.01 and 24.84 degrees 2θ. In some embodiments, the XRPD peaks above (degrees 2θ peaks) are when analyzed using copper Ka radiation. In some embodiments, provided herein is a solid form of Compound of formula (V), which has an XRPD pattern comprising peaks at approximately 10.25, 15.40, and 26.17 degrees 2θ when analyzed using copper Ka radiation. Form C ({drug5d}) has an irregular block habit as shown in Figure 15 of WO2013/126394. Representative IR spectrum data of Form C ({drug5d}) is provided in Figure 16 of WO2013/126394. In certain embodiments, provided herein is a solid form of Compound of formula (V), wherein the solid form is characterized by an IR spectrum substantially similar to the IR spectrum presented in Figure 16 of WO2013/126394. Representative thermal characteristics of Form C ({drug5d}) are shown in Figure 17 of WO2013/126394. The TGA data shows weight loss of 0.12 wt% up to about 150°C. In certain embodiments, provided herein is a solid form of Compound of formula (V), wherein the solid form is characterized by a TGA thermogram substantially similar to the TGA thermogram presented in Figure 17 of WO2013/126394. The DSC thermogram shows only one major event at slightly above 300°C. These data indicate that Form C ({drug5d}) is an anhydrate. In certain embodiments, the temperature maximum at slightly above 300°C corresponds to decomposition of Compound of formula (V). In certain embodiments, provided herein is a solid form of Compound of formula (V), wherein the solid form is characterized by a DSC thermogram substantially similar to the DSC thermogram presented in Figure 17 of WO2013/126394. Representative moisture sorption and desorption data of Form C ({drug5d}) are plotted in Figure 18 of WO2013/126394. In certain embodiments, Form C ({drug5d}) exhibits a total mass change of 0.43% between 0% and 90%) relative humidity, suggesting Form C ({drug5d}) is not hygroscopic. In certain embodiments, provided herein is a solid form of Compound of formula (V), wherein the solid form is characterized by a DVS plot substantially similar to the DVS plot presented in Figure 18 of WO2013/126394. After undergoing the adsorption/desorption cycles, the XRPD pattern of Form C ({drug5d}) shows no change, as shown in Figure 19 of WO2013/126394. In certain embodiments, Form C ({drug5d}) is characterized by an XRPD pattern substantially similar to the XRPD pattern presented in Figure 19 of WO2013/126394. Form conversion studies show that Form C ({drug5d}) typically converts to Form A ({drug5b}) at ambient temperature in a solvent system such as NMP/acetone/water (1 :2.5:2.5). Form C ({drug5d}) also partially converts to Form A ({drug5b}) upon heating up to 220°C for 4 hours, which is evidenced by the XRPD pattern of the resulting solid, as shown in Figure 20 of WO2013/126394. These observations suggest that Form C ({drug5d}) is monotropically related to Form A ({drug5b}). In certain embodiments, Form A ({drug5b}) is characterized by an XRPD pattern substantially similar to the XRPD pattern presented in Figure 20 of WO2013/126394. All of the combinations of the above

embodiments are encompassed by this invention. An XRPD stack plot of crystalline Forms A, B and C is provided in Figure 21 of WO2013/126394. In certain embodiments, Form A ({drug5b}) is characterized by an XRPD pattern substantially similar to the XRPD pattern presented in Figure 21 of WO2013/126394 Form A ({drug5b}). In certain embodiments, Form B ({drug5c}) is characterized by an XRPD pattern substantially similar to the XRPD pattern presented in Figure 21 of WO2013/126394 Form B ({drug5c}). In certain embodiments, Form C ({drug5d}) is characterized by an XRPD pattern substantially similar to the XRPD pattern presented in Figure 21 of WO2013/126394 Form C ({drug5d}). An IR overlay plot of crystalline Forms A, B and C is provided in Figure 22 of WO2013/126394. In certain embodiments, Form A ({drug5b}) is characterized by an IR spectrum substantially similar to the IR spectrum presented in Figure 22 of WO2013/126394 Form A ({drug5b}). In certain embodiments, Form B ({drug5c}) is characterized by an IR spectrum substantially similar to the IR spectrum presented in Figure 22 of WO2013/126394 Form B ({drug5c}). In certain embodiments, Form C ({drug5d}) is characterized by an IR spectrum substantially similar to the IR spectrum presented in Figure 22 of WO2013/126394 Form C ({drug5d}).

Mixture of Forms: In some embodiments, provided herein are compositions comprising at least two crystalline forms of 3-(4-nitro-1-oxoisoindolin-2-yl)piperidine-2,6-dione. In some embodiments, the composition comprises two crystalline forms of 3-(4-nitro-1-oxoisoindolin-2-yl)piperidine-2,6-dione. In one embodiment, the composition comprises Form A ({drug5b}) and Form B ({drug5c}). In another embodiment, the composition comprises Form A ({drug5b}) and Form C ({drug5d}). In yet another embodiment, the composition comprises Form B ({drug5c}) and Form C ({drug5d}). The ratio of the two crystalline forms can be any ratio. In some embodiments, the ratio of the two crystalline forms is from about 5 : 1 to about 1 : 5. In some embodiments, the ratio of the two crystalline forms is from about 4: 1 to about 1 :4. In some embodiments, the ratio of the two crystalline forms is from about 3 : 1 to about 1 : 3. In some embodiments, the ratio of the two crystalline forms is from about 2 : 1 to about 1 : 2. In one embodiment, the ratio of the two crystalline forms is about 1 : 1. In another embodiment, the ratio of the two crystalline forms is about 2 : 1. In another embodiment, the ratio of the two crystalline forms is about 3 : 1. In another embodiment, the ratio of the two crystalline forms is about 4 : 1. In another embodiment, the ratio of the two crystalline forms is about 5 : 1. In some embodiments, the composition comprises three crystalline forms of 3-(4-nitro-1-oxoisoindolin-2-yl)piperidine-2,6-dione. In one embodiment, the composition comprises Form A ({drug5b}), Form B ({drug5c}), and Form C ({drug5d}). The ratio of the three crystalline forms can be any ratio. In one embodiment, the ratio of the three crystalline forms is about 1 : 1 : 1. In one embodiment, the composition comprises more Form A ({drug5b}) than any other crystalline form or forms. In another embodiment, the composition comprises more Form B ({drug5c}) than any other crystalline form or forms. In yet another embodiment, the composition comprises more Form C ({drug5d}) than any other crystalline form or forms. All of the combinations of the above embodiments are encompassed by this invention. Methods of Use: Polymorphs provided herein exhibit physical characteristics that are beneficial for drug manufacture, storage or use. All polymorphs provided herein have utility as pharmaceutically active ingredients or intermediates thereof. In some embodiments, provided herein are methods of treating and preventing a wide variety of diseases and conditions using polymorphs of 3-(4-nitro-1-oxoisoindolin-2-yl)piperidine-2,6-dione. In each of the methods, a therapeutically or prophylactically effective amount of the compound is administered to a patient in need of such treatment or prevention. Examples of such disease and conditions include, but are not limited to, diseases associated with undesired angiogenesis, cancer (e.g., solid and blood borne tumors), inflammatory diseases, autoimmune diseases, and immune diseases. Examples of cancers and pre-cancerous conditions include those described in U.S. Patent Nos. 6,281,230 and 5,635,517 to Muller et al.; 7,189,740 (Treatment of Myelodisplastic Syndrome), 7,968,569 (Treatment of Various Types of Cancer), and 7,563,810 (Treatment of Myeloproliferative Diseases) to Zeldis. Examples of other diseases and disorders that can be treated or prevented using compositions provided herein are described in U.S. Patent Nos. 6,235,756 and 6,114,335 to D'Amato; U.S. Application Publication Nos. 2005-0203142 Al (Treatment of Pain Syndrome) and 2004-0091455 Al (Treatment of Macular Degeneration) to Zeldis. The entirety of each of the patents and patent application publications cited herein is incorporated herein by reference. Depending on the disease to be treated and the subject's condition, polymorphs provided herein can be administered by oral, parenteral (e.g., intramuscular, intraperitoneal, intravenous, ICV, intracisternal injection or infusion, subcutaneous injection, or implantation), inhalation spray, nasal, vaginal, rectal, sublingual, or topical routes of administration and may be formulated, alone or together, in suitable dosage unit formulations containing conventional nontoxic pharmaceutically acceptable carriers, adjuvants and vehicles appropriate for each route of administration. Because individual polymorphs have different dissolution, stability, and other properties, the optimal polymorph used in methods of treatment may depend on the route of administration. For example, forms that are readily soluble in aqueous solutions are preferably used to provide liquid dosage forms, whereas forms that exhibit great thermal stability may be preferred in the manufacture of solid dosage forms (e.g., tablets and capsules). Although the physical characteristics of polymorphs can, in some cases, affect their bioavailability, amounts of the polymorphs that are therapeutically or prophylactically effective in the treatment of various disease and conditions can be readily determined by those of ordinary skill in the pharmacy or medical arts. In certain embodiments provided herein, a polymorph is administered orally and in a single or divided daily doses in an amount of from about 0.10 to about 150 mg/day, or from about 5 to about 25 mg/day. In other embodiments, a polymorph is administered every other day in an amount of from about 0.10 to about

150 mg/day, or from about 5 to about 25 mg/day.

In some embodiments, provided herein are methods of preparing 3-(4-amino-1-oxoisoindolin-2-yl)piperidine-2,6-dione, which comprise reduction of one or more polymorphs of 3-(4-nitro-1-oxoisoindolin-2-yl)piperidine-2,6-dione. Because individual polymorphs have different dissolution, stability, and other properties, the optimal polymorph used in methods of preparation of 3-(4-amino-1-oxoisoindolin-2-yl)piperidine-2,6-dione may depend on the reaction conditions. For example, the more stable form is often preferred for consistence of production and better physical properties.

Pharmaceutical Compositions and Dosage Form: In some embodiments, provided herein are pharmaceutical compositions and single unit dosage forms that can be used in methods of treatment and prevention, which comprise one or more polymorphs of 3-(4-nitro-1-oxoisoindolin-2-yl)piperidine-2,6-dione and optionally one or more excipients or diluents. Specific compositions and dosage forms are disclosed in the various patents and patent applications incorporated herein by reference. In one embodiment, a single dosage Form C ({drug5d})omprises a polymorph (e.g., Form A ({drug5b})) in an amount of about 5, 10, 25 or 50 mg. Pharmaceutical compositions can be used in the preparation of individual, single unit dosage forms. Pharmaceutical compositions and dosage forms provided herein comprise a compound provided herein, or a pharmaceutically acceptable salt, solvate, stereoisomer, or prodrug thereof. Pharmaceutical compositions and dosage forms provided herein can further comprise one or more excipients. Pharmaceutical compositions and dosage forms provided herein can also comprise one or more additional active ingredients. Single unit dosage forms provided herein are suitable for oral, mucosal (e.g., nasal, sublingual, vaginal, buccal, or rectal), parenteral (e.g., subcutaneous, intravenous, bolus injection, intramuscular, or intraarterial), topical (e.g., eye drops or other ophthalmic preparations), transdermal or transcutaneous administration to a patient. Examples of dosage forms include, but are not limited to: tablets; caplets; capsules, such as soft elastic gelatin capsules; cachets; troches; lozenges; dispersions; suppositories; powders; aerosols (e.g., nasal sprays or inhalers); gels; liquid dosage forms suitable for oral or mucosal administration to a patient, including suspensions (e.g., aqueous or non-aqueous liquid suspensions, oil-in- water emulsions, or a water-in-oil liquid emulsions), solutions, and elixirs; liquid dosage forms suitable for parenteral administration to a patient; eye drops or other ophthalmic preparations suitable for topical administration; and sterile solids (e.g., crystalline or amorphous solids) that can be reconstituted to provide liquid dosage forms suitable for parenteral administration to a patient. The composition, shape, and type of dosage forms provided herein will typically vary depending on their use. For example, a dosage form used in the acute treatment of a disease may contain larger amounts of one or more of the active ingredients it comprises than a dosage form used in the chronic treatment of the same disease. Similarly, a parenteral dosage form may contain smaller amounts of one or more of the active ingredients it comprises than an oral dosage form used to treat the same disease. These and other ways in which specific dosage forms provided herein will vary from one another will be readily apparent to those skilled in the art. See, e.g., Remington's Pharmaceutical Sciences, 18th ed., Mack Publishing, Easton PA (1990). Typical pharmaceutical compositions and dosage forms comprise one or more excipients. Suitable excipients are well known to those skilled in the art of pharmacy, and non-limiting examples of suitable excipients are provided herein. Whether a particular excipient is suitable for incorporation into a pharmaceutical composition or dosage form depends on a variety of factors well known in the art including, but not limited to, the way in which the dosage form will be administered to a patient. For example, oral dosage forms such as tablets may contain excipients not suited for use in parenteral dosage forms. The suitability of a particular excipient may also depend on the specific active ingredients in the dosage form. For example, the decomposition of some active ingredients may be accelerated by some excipients such as lactose, or when exposed to water. Active ingredients that comprise primary or secondary amines are particularly susceptible to such accelerated decomposition. Consequently, in some embodiments, provided herein are pharmaceutical compositions and dosage forms that contain little, if any, lactose other mono- or di-saccharides. As used herein, the term "lactose-free" means that the amount of lactose present, if any, is insufficient to substantially increase the degradation rate of an active ingredient. Lactose-free compositions provided herein can comprise excipients that are well known in the art and are listed, for example, in the U.S. Pharmacopeia (USP) 25-NF20 (2002). In general, lactose-free compositions comprise active ingredients, a binder/filler, and a lubricant in pharmaceutically compatible and pharmaceutically acceptable amounts. Preferred lactose-free dosage forms comprise active ingredients, microcrystalline cellulose, pre-gelatinized starch, and magnesium stearate. In some embodiments, provided herein are anhydrous pharmaceutical compositions and dosage forms comprising active ingredients, since water can facilitate the degradation of some compounds. For example, the addition of water (e.g., 5%) is widely accepted in the pharmaceutical arts as a means of simulating long-term storage in order to determine characteristics such as shelf-life or the stability of formulations over time. See, e.g., Jens T. Carstensen, Drug Stability: Principles & Practice, 2d. Ed., Marcel Dekker, NY, NY, 1995, pp. 379-80. In effect, water and heat accelerate the decomposition of some compounds. Thus, the effect of water on a formulation can be of great significance since moisture and/or humidity are commonly encountered during manufacture, handling, packaging, storage, shipment, and use of formulations. Anhydrous pharmaceutical compositions and dosage forms provided herein can be prepared using anhydrous or low moisture containing ingredients and low moisture or low humidity conditions. Pharmaceutical compositions and dosage forms that comprise lactose and at least one active ingredient that comprises a primary or secondary amine are preferably anhydrous if substantial contact with moisture and/or humidity during manufacturing, packaging, and/or storage is expected. An anhydrous pharmaceutical composition should be prepared and stored such that its anhydrous nature is maintained. Accordingly, anhydrous compositions are preferably packaged using materials known to prevent exposure to water such that they can be included in suitable formulary kits. Examples of suitable packaging include, but are not limited to, hermetically sealed foils, plastics, unit dose containers (e.g., vials), blister packs, and strip packs. In some embodiments, provided herein are pharmaceutical compositions and dosage forms that comprise one or more compounds that reduce the rate by which an active ingredient will decompose. Such compounds, which are referred to herein as "stabilizers," include, but are not limited to, antioxidants such as ascorbic acid, pH buffers, or salt buffers. Like the amounts and types of excipients, the amounts and specific types of active ingredients in a dosage form may differ depending on factors such as, but not limited to, the route by which it is to be administered to patients. However, typical dosage forms provided herein comprise a compound provided herein in an amount of from about 0.10 to about 500 mg. Typical dosage forms comprise a compound provided herein in an amount of about 0.1, 1, 2,5,7.5,10,12.5,15,17.5,20,25, 50, 100, 150, 200, 250, 300, 350, 400, 450, or 500 mg. Typical dosage forms comprise the second active ingredient in an amount of 1 to about 1000 mg, from about 5 to about 500 mg, from about 10 to about 350 mg, or from about 50 to about 200 mg. Of course, the specific amount of the second active agent will depend on the specific agent used, the type of cancer being treated or managed, and the amount(s) of a compound provided herein and any optional additional active agents concurrently administered to the patient.

Oral Dosage Forms: Pharmaceutical compositions provided herein that are suitable for oral administration can be presented as discrete dosage forms, such as, but are not limited to, tablets (e.g., chewable tablets), caplets, capsules, and liquids (e.g., flavored syrups). Such dosage forms contain predetermined amounts of active ingredients, and may be prepared by methods of pharmacy well known to those skilled in the art. See generally, Remington 's Pharmaceutical Sciences, 18th ed., Mack Publishing, Easton PA (1990). Typical oral dosage forms provided herein are prepared by combining the active ingredients in an intimate admixture with at least one excipient according to conventional pharmaceutical compounding techniques. Excipients can take a wide variety of forms depending on the form of preparation desired for administration. For example, excipients suitable for use in oral liquid or aerosol dosage forms include, but are not limited to, water, glycols, oils, alcohols, flavoring agents, preservatives, and coloring agents. Examples of excipients suitable for use in solid oral dosage forms (e.g., powders, tablets, capsules, and caplets) include, but are not limited to, starches, sugars, micro-crystalline cellulose, diluents, granulating agents, lubricants, binders, and disintegrating agents. Because of their ease of administration, tablets and capsules represent the most advantageous oral dosage unit forms, in which case solid excipients are employed. If desired, tablets can be coated by standard aqueous or nonaqueous techniques. Such dosage forms can be prepared by any of the methods of pharmacy. In general, pharmaceutical compositions and dosage forms are prepared by uniformly and intimately admixing the active ingredients with liquid carriers, finely divided solid carriers, or both, and then shaping the product into the desired presentation if necessary. For example, a tablet can be prepared by compression or molding. Compressed tablets can be prepared by compressing in a suitable machine the active ingredients in a free-flowing form such as powder or granules, optionally mixed with an excipient. Molded tablets can be made by molding in a suitable machine a mixture of the powdered compound moistened with an inert liquid diluent. Examples of excipients that can be used in oral dosage forms provided herein include, but are not limited to, binders, fillers, disintegrants, and lubricants. Binders suitable for use in pharmaceutical compositions and dosage forms include, but are not limited to, corn starch, potato starch, or other starches, gelatin, natural and synthetic gums such as acacia, sodium alginate, alginic acid, other alginates, powdered tragacanth, guar gum, cellulose and its derivatives (e.g., ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, (e.g., Nos. 2208, 2906, 2910), microcrystalline cellulose, and mixtures thereof. Suitable forms of microcrystalline cellulose include, but are not limited to, the materials sold as AVICEL-PH-101, AVICEL-PH-103 AVICEL RC-581, AVICEL-PH-105 (available from FMC Corporation, American Viscose Division, Avicel Sales, Marcus Hook, PA), and mixtures thereof. An specific binder is a mixture of microcrystalline cellulose and sodium carboxymethyl cellulose sold as AVICEL RC-581. Suitable anhydrous or low moisture excipients or additives include AVICEL-PH-103™ and Starch 1500 LM. Examples of fillers suitable for use in the pharmaceutical compositions and dosage forms disclosed herein include, but are not limited to, talc, calcium carbonate (e.g., granules or powder), microcrystalline cellulose, powdered cellulose, dextrates, kaolin, mannitol, silicic acid, sorbitol, starch, pre-gelatinized starch, and mixtures thereof. The binder or filler in pharmaceutical compositions provided herein is typically present in from about 50 to about 99 weight percent of the pharmaceutical composition or dosage form. Disintegrants are used in the compositions provided herein to provide tablets that disintegrate when exposed to an aqueous environment. Tablets that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients should be used to form solid oral dosage forms provided herein. The amount of disintegrant used varies based upon the type of formulation, and is readily discernible to those of ordinary skill in the art. Typical pharmaceutical compositions comprise from about 0.5 to about 15 weight percent of disintegrant, preferably from about 1 to about 5 weight percent of disintegrant. Disintegrants that can be used in pharmaceutical compositions and dosage forms provided herein include,

but are not limited to, agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, other starches, clays, other algins, other celluloses, gums, and mixtures thereof. Lubricants that can be used in pharmaceutical compositions and dosage forms provided herein include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (e.g., peanut oil, cottonseed oil, sunflower oil, sesame oil, olive oil, corn oil, and soybean oil), zinc stearate, ethyl oleate, ethyl laureate, agar, and mixtures thereof. Additional lubricants include, for example, a syloid silica gel (AEROSIL200, manufactured by W.R. Grace Co. of Baltimore, MD), a coagulated aerosol of synthetic silica (marketed by Degussa Co. of Piano, TX), CAB-O-SIL (a pyrogenic silicon dioxide product sold by Cabot Co. of Boston, MA), and mixtures thereof. If used at all, lubricants are typically used in an amount of less than about 1 weight percent of the pharmaceutical compositions or dosage forms into which they are incorporated. A solid oral dosage form provided herein comprises a compound provided herein, anhydrous lactose, microcrystalline cellulose, polyvinylpyrrolidone, stearic acid, colloidal anhydrous silica, and gelatin.

Controlled Release Dosage Forms: Active ingredients provided herein can be administered by controlled release means or by delivery devices that are well known to those of ordinary skill in the art. Examples include, but are not limited to, those described in U.S. Patent Nos.: 3,845,770; 3,916,899; 3,536,809; 3,598,123; and 4,008,719, 5,674,533, 5,059,595, 5,591,767, 5, 120,548, 5,073,543, 5,639,476, 5,354,556, and 5,733,566, each of which is incorporated herein by reference. Such dosage forms can be used to provide slow or controlled-release of one or more active ingredients using, for example, hydropropylmethyl cellulose, other polymer matrices, gels, permeable membranes, osmotic systems, multilayer coatings, microparticles, liposomes, microspheres, or a combination thereof to provide the desired release profile in varying proportions. Suitable controlled-release formulations known to those of ordinary skill in the art, including those described herein, can be readily selected for use with the active ingredients provided herein. In some embodiments, provided herein are single unit dosage forms suitable for oral administration such as, but not limited to, tablets, capsules, gelcaps, and caplets that are adapted for controlled-release. All controlled-release pharmaceutical products have a common goal of improving drug therapy over that achieved by their non-controlled counterparts. Ideally, the use of an optimally designed controlled-release preparation in medical treatment is characterized by a minimum of drug substance being employed to cure or control the condition in a minimum amount of time. Advantages of controlled-release formulations include extended activity of the drug, reduced dosage frequency, and increased patient compliance. In addition, controlled-release formulations can be used to affect the time of onset of action or other characteristics, such as blood levels of the drug, and can thus affect the occurrence of side (e.g., adverse) effects. Most controlled-release formulations are designed to initially release an amount of drug (active ingredient) that promptly produces the desired therapeutic effect, and gradually and continually release of other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled-release of an active ingredient can be stimulated by various conditions including, but not limited to, pH, temperature, enzymes, water, or other physiological conditions or compounds. Parenteral Dosage Forms: Parenteral dosage forms can be administered to patients by various routes including, but not limited to, subcutaneous, intravenous (including bolus injection), intramuscular, and intraarterial. Because their administration typically bypasses patients' natural defenses against contaminants, parenteral dosage forms are preferably sterile or capable of being sterilized prior to administration to a patient. Examples of parenteral dosage forms include, but are not limited to, solutions ready for injection, dry products ready to be dissolved or suspended in a pharmaceutically acceptable vehicle for injection, suspensions ready for injection, and emulsions. Suitable vehicles that can be used to provide parenteral dosage forms provided herein are well known to those skilled in the art. Examples include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate. Compounds that increase the solubility of one or more of the active ingredients disclosed herein can also be incorporated into the parenteral dosage forms provided herein. For example, cyclodextrin and its derivatives can be used to increase the solubility of an immunomodulatory compound provided herein and its derivatives. See, e.g., U.S. Patent No. 5,134,127, which is incorporated herein by reference.

Topical and Mucosal Dosage Forms: Topical and mucosal dosage forms provided herein include, but are not limited to, sprays, aerosols, solutions, emulsions, suspensions, eye drops or other ophthalmic preparations, or other forms known to one of skill in the art. See, e.g., Remington's Pharmaceutical Sciences, 16th and 18th eds., Mack Publishing, Easton PA (1980 & 1990); and Introduction to Pharmaceutical Dosage Forms, 4th ed., Lea & Febiger, Philadelphia (1985). Dosage forms suitable for treating mucosal tissues within the oral cavity can be formulated as mouthwashes or as oral gels. Suitable excipients (e.g., carriers and diluents) and other materials that can be used to provide topical and mucosal dosage forms provided herein are well known to those skilled in the pharmaceutical arts,

and depend on the particular tissue to which a given pharmaceutical composition or dosage form will be applied. With that fact in mind, typical excipients include, but are not limited to, water, acetone, ethanol, ethylene glycol, propylene glycol, butane- 1,3-diol, isopropyl myristate, isopropyl palmitate, mineral oil, and mixtures thereof to form solutions, emulsions or gels, which are non-toxic and pharmaceutically acceptable. Moisturizers or humectants can also be added to pharmaceutical compositions and dosage forms if desired. Examples of such additional ingredients are well known in the art. See, e.g., Remington's Pharmaceutical Sciences, 16th and 18th eds., Mack Publishing, Easton PA (1980 & 1990). The pH of a pharmaceutical composition or dosage form may also be adjusted to improve delivery of one or more active ingredients. Similarly, the polarity of a solvent carrier, its ionic strength, or tonicity can be adjusted to improve delivery. Compounds such as stearates can also be added to pharmaceutical compositions or dosage forms to advantageously alter the hydrophilicity or lipophilicity of one or more active ingredients so as to improve delivery. In this regard, stearates can serve as a lipid vehicle for the formulation, as an emulsifying agent or surfactant, and as a delivery-enhancing or penetration-enhancing agent. Different salts, hydrates or solvates of the active ingredients can be used to further adjust the properties of the resulting composition.

Synthesis of Form A ({drug5b}) and/or Form B ({drug5c}): A nitrogen-purged 1600 L Hastelloy reaction vessel was charged with methyl-2-bromomethyl-3-nitrobenzoate (61 kg, 1.0 mole eq.), racemic a-aminoglutarimide (36.6 kg, 1.0 mole eq.), and acetonitrile (478 kg). The mixture was stirred at ambient temperature and then cooled to 5-8°C. Sodium bicarbonate (46.7 kg, 2.5 mole eq.) was charged to the reaction vessel and the mixture was heated to reflux. The reaction vessel contents were vigorously stirred at reflux until the remaining brominated nitrobenzoate level is <3.0 mol% as determined by 1H-NMR. The reaction mixture was then cooled to 58-62°C. A hydrochloric acid solution was prepared from concentrated hydrochloric acid (12.9 kg) and purified water (355 L) in a nitrogen-purged, stirred 1000 L Hastelloy vessel and heated to 58-62°C. A portion of this hydrochloric acid solution (329 L) was added to the reaction mixture in the 1600 L vessel over at least 10 minutes while maintaining the temperature at 58-62°C. The mixture was stirred for approximately 30 minutes, then pH of the reaction mixture was checked. If the pH was >6, 6.1 L aliquots of hydrochloric acid were added, while maintaining the temperature at approximately 58-62°C, until the pH of the reaction mixture was <6. The mixture was then cooled to ambient temperature over approximately 2.5 hours and stirred for an additional hour. The solid was collected by vacuum/pressure filtration on an Oyster filter. The collected solid was washed on the Oyster filter with a mixture of acetone (76 kg) and purified water (90 kg), and de-liquored using vacuum pressure. The collected solid was washed with 147 kg of acetone and de-liquored using vacuum pressure. The solid was dried under vacuum in a VPD at <45°C until loss on drying is <0.5% to provide 3-(4-nitro-1-oxoisoindolin-2-yl)piperidine-2,6-dione. The yield of a recent lot was 79.5%.

Equilibration: Equilibration studies using single, binary and ternary combinations of batch solvents (water, acetone, and acetonitrile) were investigated to mimic key steps in manufacturing 3-(4-nitro-1-oxoisoindolin-2-yl)piperidine-2,6-dione. Experimental details of solvent systems and temperature used in equilibration studies are listed in Table 1. Materials of 3-(4-nitro-1-oxoisoindolin-2-yl)piperidine-2,6-dione and solvent or solvent mixtures were added to a glass vial to achieve free-flowing slurry and allowed to equilibrate at the specified temperature. After equilibration for 8 days, the solid phase was separated via filtration. The solid phase was then dried under air and analyzed by XRPD. List of Experimental Conditions for Equilibration:

| Solvent system | Temperature | Equilibration Time |
|---|---|---|
| Acetonitrile | 80 °C (or reflux) | 6 hours |
| Acetonitrile/water (2:1) | 60 °C | 6 hours |
| acetone | Ambient | 8 days |
| water | Ambient | 8 days |
| Acetone/water (1:1) | Ambient | 8 days |
| Acetonitrile/water (1:1) | Ambient | 8 days |
| Acetonitrile/water/acetone (1:1:1) | Ambient | 8 days |

A total of four batches of 3-(4-nitro-1-oxoisoindolin-2-yl)piperidine-2,6-dione were characterized by XRPD, DSC, TGA, ATR-FTIR and DVS, as described in section 6.5. The solid form represented by the batch 1 was designated as Form A ({drug5b}); the solid form represented by the batch 4 was designated as Form B ({drug5c}); the batches 2 and 3 were determined to be mixtures of Forms A and B, with Form B ({drug5c}) dominating the patterns. The equilibration experiments were performed using batches 2, 3 and 4, and the results are described in Table 2. Results for Equilibration Experiments:

| Solvent system | Temperature | Time | Starting Batch # | Form by XRPD |
|---|---|---|---|---|
| Acetonitrile | 80 °C (or reflux) | 6 hours | 2 (A + B) | A |
| Acetonitrile/water (2:1) | 60 °C | 6 hours | 2 (A + B) | A |
| acetone | Ambient | 8 days | 2 (A + B) | A |
| water | Ambient | 8 days | 2 (A + B) | A |
| Acetone/water (1:1) | Ambient | 8 days | 2 (A + B) | A |
| Acetonitrile/water (1:1) | Ambient | 8 days | 2 (A + B) | A |
| Acetonitrile/water/acetone (1:1:1) | Ambient | 8 days | 2 (A + B) | A |
| Acetonitrile | 80 °C (or reflux) | 6 hours | 3 (A + B) | A |
| Acetonitrile/water (2:1) | 60 °C | 6 hours | 3 (A + B) | A |
| acetone | Ambient | 8 days | 3 (A + B) | A |
| water | Ambient | 8 days | 3 (A + B) | A |
| Acetone/water (1:1) | Ambient | 8 days | 3 (A + B) | A |
| Acetonitrile/water (1:1) | Ambient | 8 days | 3 (A + B) | A |
| Acetonitrile/water/acetone (1:1:1) | Ambient | 8 days | 3 (A + B) | A |
| Acetonitrile | 80 °C (or reflux) | 6 hours | 4 (B) | A |
| | | | | |
| Acetonitrile/water (2:1) | 60 °C | 6 hours | 4 (B) | A |
| acetone | Ambient | 8 days | 4 (B) | A |
| water | Ambient | 8 days | 4 (B) | A |
| Acetone/water (1:1) | Ambient | 8 days | 4 (B) | A |
| Acetonitrile/water (1:1) | Ambient | 8 days | 4 (B) | A |
| Acetonitrile/water/acetone (1:1:1) | Ambient | 8 days | 4 (B) | A |

All solids isolated from these experiments afforded Form A ({drug5b}), suggesting Form A ({drug5b}) is more thermodynamically stable form. An XRPD stack plot of the four batches is provided in Figure 23 of WO2013/126394. In certain embodiments, Form A ({drug5b}) is characterized by an XRPD pattern substantialy similar to the XRPD pattern presented in Figure 23 of WO2013/126394 batch 1. In certain embodiments, Form B ({drug5c}) is characterized by an XRPD pattern substantially similar to the XRPD pattern presented in Figure 23 of WO2013/126394 batch 4. In certain embodiments, a solid form of Compound of formula (V) is characterized by an XRPD pattern substantially similar to the XRPD pattern presented in Figure 23 of WO2013/126394 batch 2. In certain embodiments, a solid form of Compound of formula (V) is characterized by an XRPD pattern substantially similar to the XRPD pattern presented in Figure 23 of WO2013/126394 batch 3. An IR overlay plot of the four batches is provided in Figure 24 of WO2013/126394. In certain embodiments, Form A ({drug5b}) is characterized by an IR spectrum substantially similar to the IR spectrum presented in Figure 24 of WO2013/126394 batch 1. In certain embodiments, Form B ({drug5c}) is characterized by an IR spectrum substantially similar to the IR spectrum presented in Figure 24 of WO2013/126394 batch 4. In certain embodiments, a solid form of Compound of formula (V) is characterized by an IR spectrum substantially similar to the IR spectrum presented in Figure 24 of WO2013/126394 batch 2. In certain embodiments, a solid form of Compound of formula (V) is characterized by an IR spectrum substantially similar to the IR spectrum presented in Figure 24 of WO2013/126394 batch 3. Recrystallization: Recrystallization of 3-(4-nitro-1-oxoisoindolin-2-yl)piperidine-2,6-dione was performed using 1-methyl-2-pyrrolidinone (NMP) as primary solvent. Solid of 3-(4-nitro-1-oxoisoindolin-2-yl)piperidine-2,6-dione were dissolved in NMP with help of heat and filtered. The solvent systems listed in Table 1 were used as anti-solvent at the corresponding temperature. The results are summarized in Table 3. Recrystallization Results usin NMP as Primar Solvent

| Anti-solvent | Temperature | Form by XRPD |
|---|---|---|
| Acetonitrile | 80 °C (or reflux) | C |
| Acetonitrile/water (2:1) | 60 °C | C |
| acetone | Ambient | B + C |
| water | Ambient | A + B + C |
| Acetone/water (1:1) | Ambient | B + C + peaks* |
| Acetone/water (1:1)** | Ambient | C |
| Acetonitrile/water (1:1) | Ambient | C |
| Acetonitrile/water (1:1)** | Ambient | C |
| Acetonitrile/water/acetone (1:1:1) | Ambient | B + C + peaks* |
| * Additional diffraction peaks were observed at 7.1, 9.6, and 14.2° 2θ, which may represent a unique crystalline form. ** Experiments were repeated to confirm solid form observation and to generate additional materials. | | |

Experiments from acetonitrile at 80°C, acetonitrile/water (2: 1) at 60°C, or acetonitrile/water (1 : 1) at ambient temperature afforded unique XRPD pattern, which was designated as Form C ({drug5d}). The other experiments afforded solids with mixture of forms. Solids from acetone/water (1 : 1) or acetonitrile/water/acetone (1 : 1 : 1) showed additional diffraction peaks at 7.1, 9.6, and 14.2° 2θ, which may represent another unique crystalline form. The anti-solvent recrystallization with acetonitrile/water (1 : 1) or acetone/water (1 : 1) at ambient were repeated to confirm the solid form observation and to generate additional materials for further characterization. The solid form the repeated experiment using acetonitrile/water (1 : 1) as antisolvent was confirmed to be Form C ({drug5d}). The attempt to regenerate the additional diffraction peaks using acetone/water (1 : 1) as antisolvent didn't generate a unique pattern, but afforded solid of Form C ({drug5d}).

Additional Conversion Experiments: Additional Form conversion studies were performed and the results are summarized in Table 4. Results from Additional Form conversion Experiments:

| Starting Form | Condition | XRPD Result |
|---|---|---|
| Form B | Heated to 220 °C for 4 hours | Form A |
| Form C | Heated to 220 °C for 4 hours | Form A+C |
| Form C | Slurry in NMP/Acetone/water at ambient for 5 days | Form A+C |
| Form C | Slurry in NMP/Acetone/water at ambient for 10 days | Form A+C |

Characterization: X-ray Powder Diffraction (XRPD): All of the solid samples generated in the polymorph screen were analyzed by XRPD. XRPD analysis was conducted on a PANalytical Empyrean or a Thermo ARL XTRA X-ray powder diffractometer using Cu Ka radiation at 1.54 A. The PANalytical Empyrean instrument was equipped with a fine focus X-ray tube. The voltage and amperage of the X-ray generator were set at 45 kV and 40 mA, respectively. The divergence slits were set at 1/16° and 1/8 °, and the receiving slits was set at 1/16°. Diffracted radiation was measured using a Pixel 2D detector. A theta-two theta continuous scan was set at step size 0.013 from 3° to 40° 2θ with sample spinning rate at 4. A sintered alumina standard was used to check the peak positions. The Thermo ARL X'TRA instrument was equipped with a fine focus X-ray tube. The voltage and amperage of the X-ray generator were set at 45 kV and 40 mA, respectively. The divergence slits were set at 4 mm and 2 mm and the measuring slits were set at 0.5 mm and 0.2 mm. Diffracted radiation was measured using a Peltier-cooled Si (Li) solid-state detector. A theta-two theta continuous scan at 2.40 min (0.5 sec/0.02° step) from 1.5° to 40° 2θ was used. A sintered alumina standard was used to check the peak positions. The peaks for Form A ({drug5b}), Form B ({drug5c}), and Form C ({drug5d}) XRPD patterns were identified. XRPD patterns are provided in Figure 1 of WO2013/126394 (Form A ({drug5b})), Figure 7 of WO2013/126394 (Form B ({drug5c})), and Figure 14 of WO2013/126394 (Form C ({drug5d})). Peak values and intensity values are provided in the table below. For samples with only one XRPD pattern and no other means to evaluate whether the sample provides a good approximation of the powder average, peak tables contain data identified only as "Prominent

Peaks". These peaks are a subset of the entire observed peak list. Prominent peaks are selected from observed peaks by identifying preferably non-overlapping, low-angle peaks, with strong intensity. Although peaks are labeled on diffraction patterns and/or listed in tables, for technical reasons, different rounding algorithms were used to round each peak to the nearest 0.1° or 0.01° 2θ, depending upon the instrument used to collect the data and/or the inherent peak resolution. The location of the peaks along the x-axis (° 2θ) were rounded to one or two significant figures after the decimal point based upon the above criteria. Peak position variabilities are given to within ±0.1° 2θ based upon recommendations outlined in the USP discussion of variability in x-ray powder diffraction.

Table 5. XRPD Peak Table of Form A ({drug5b}):

| No. | Pos. [°2Th.] | d-spacing [Å] | Height [cts] | Rel. Int. [%] |
|---|---|---|---|---|
| 1 | 10.30 | 8.59 | 14766.88 | 100.00 |
| 2 | 10.59 | 8.35 | 1959.09 | 13.27 |
| 3 | 12.80 | 6.91 | 867.96 | 5.88 |
| 4 | 13.03 | 6.80 | 2195.07 | 14.86 |
| 5 | 14.85 | 5.96 | 2980.16 | 20.18 |
| 6 | 15.15 | 5.85 | 4172.06 | 28.25 |
| 7 | 16.40 | 5.41 | 1529.30 | 10.36 |
| 8 | 16.93 | 5.24 | 2097.79 | 14.21 |
| 9 | 18.39 | 4.82 | 3837.08 | 25.98 |
| 10 | 18.55 | 4.78 | 3001.13 | 20.32 |
| 11 | 20.71 | 4.29 | 2092.99 | 14.17 |
| 12 | 21.08 | 4.22 | 844.00 | 5.72 |
| 13 | 21.28 | 4.18 | 3167.38 | 21.45 |
| 14 | 21.53 | 4.13 | 286.77 | 1.94 |
| | | | | |
| 15 | 22.08 | 4.03 | 2934.77 | 19.87 |
| 16 | 22.63 | 3.93 | 959.05 | 6.49 |
| 17 | 24.91 | 3.57 | 6432.72 | 43.56 |
| 18 | 25.40 | 3.51 | 2281.35 | 15.45 |
| 19 | 25.78 | 3.46 | 2798.30 | 18.95 |
| 20 | 26.25 | 3.39 | 11225.20 | 76.02 |
| 21 | 27.22 | 3.28 | 82.18 | 0.56 |
| 22 | 28.70 | 3.11 | 494.31 | 3.35 |
| 23 | 29.55 | 3.02 | 3625.21 | 24.55 |
| 24 | 30.02 | 2.98 | 350.35 | 2.37 |
| 25 | 30.31 | 2.95 | 378.60 | 2.56 |
| 26 | 30.69 | 2.91 | 567.71 | 3.84 |
| 27 | 31.00 | 2.88 | 1093.68 | 7.41 |
| 28 | 31.56 | 2.84 | 350.95 | 2.38 |
| 29 | 32.43 | 2.76 | 549.38 | 3.72 |
| 30 | 33.17 | 2.70 | 148.43 | 1.01 |
| | | | | |

(continued)

| No. | Pos. [°2Th.] | d-spacing [Å] | Height [cts] | Rel. Int. [%] |
|---|---|---|---|---|
| 31 | 34.34 | 2.61 | 527.70 | 3.57 |
| 32 | 34.96 | 2.57 | 93.08 | 0.63 |
| 33 | 35.78 | 2.51 | 405.55 | 2.75 |
| 34 | 36.68 | 2.45 | 350.54 | 2.37 |
| 35 | 37.95 | 2.37 | 131.97 | 0.89 |
| 36 | 38.30 | 2.35 | 115.07 | 0.78 |
| 37 | 39.12 | 2.30 | 621.07 | 4.21 |

Table 6. XRPD Peak Table of Form B ({drug5c})

| No. | Pos. [°2Th.] | d-spacing [Å] | Height [cts] | Rel. Int. [%] |
|---|---|---|---|---|
| 1 | 10.32 | 8.57 | 4126.10 | 10.64 |
| 2 | 12.54 | 7.06 | 87.15 | 0.22 |
| 3 | 13.94 | 6.35 | 38772.77 | 100.00 |
| 4 | 14.63 | 6.06 | 7910.54 | 20.40 |
| 5 | 15.46 | 5.73 | 2432.00 | 6.27 |
| 6 | 15.72 | 5.64 | 4052.98 | 10.45 |
| 7 | 16.83 | 5.27 | 1589.96 | 4.10 |
| 8 | 17.14 | 5.17 | 14501.45 | 37.40 |
| 9 | 18.82 | 4.72 | 3347.54 | 8.63 |
| 10 | 19.07 | 4.65 | 257.01 | 0.66 |
| 11 | 20.68 | 4.29 | 2889.94 | 7.45 |
| 12 | 20.99 | 4.23 | 383.02 | 0.99 |
| 13 | 21.35 | 4.16 | 232.00 | 0.60 |
| 14 | 21.72 | 4.09 | 324.95 | 0.84 |
| 15 | 22.14 | 4.02 | 326.51 | 0.84 |
| | | | | |
| 16 | 22.48 | 3.96 | 1210.79 | 3.12 |
| 17 | 22.69 | 3.92 | 2963.80 | 7.64 |
| 18 | 23.36 | 3.81 | 238.82 | 0.62 |
| 19 | 24.27 | 3.67 | 460.76 | 1.19 |
| 20 | 24.79 | 3.59 | 1654.72 | 4.27 |
| 21 | 25.46 | 3.50 | 274.53 | 0.71 |
| 22 | 25.86 | 3.45 | 710.64 | 1.83 |
| 23 | 26.26 | 3.39 | 2200.96 | 5.68 |
| 24 | 26.99 | 3.30 | 668.91 | 1.73 |
| 25 | 27.33 | 3.26 | 461.88 | 1.19 |
| 26 | 27.69 | 3.22 | 2960.01 | 7.63 |

(continued)

| No. | Pos. [°2Th.] | d-spacing [Å] | Height [cts] | Rel. Int. [%] |
|---|---|---|---|---|
| 27 | 28.01 | 3.19 | 1912.16 | 4.93 |
| 28 | 28.70 | 3.11 | 308.94 | 0.80 |
| 29 | 29.45 | 3.03 | 603.29 | 1.56 |
| 30 | 30.49 | 2.93 | 2202.15 | 5.68 |
| | | | | |
| 31 | 30.56 | 2.93 | 1746.82 | 4.51 |
| 32 | 31.21 | 2.86 | 261.71 | 0.67 |
| 33 | 32.12 | 2.78 | 359.23 | 0.93 |
| 34 | 33.13 | 2.70 | 127.53 | 0.33 |
| 35 | 33.66 | 2.66 | 123.91 | 0.32 |
| 36 | 34.64 | 2.59 | 413.08 | 1.07 |
| 37 | 35.35 | 2.54 | 106.99 | 0.28 |
| 38 | 35.73 | 2.51 | 251.84 | 0.65 |
| 39 | 36.33 | 2.47 | 569.91 | 1.47 |
| | | | | |
| 40 | 36.97 | 2.43 | 589.65 | 1.52 |
| 41 | 37.98 | 2.37 | 331.89 | 0.86 |
| 42 | 39.15 | 2.30 | 555.03 | 1.43 |

Table 7. XRPD Peak Table of Form C ({drug5d})

| No. | Pos. [°2Th.] | d-spacing [Å] | Height [cts] | Rel. Int. [%] |
|---|---|---|---|---|
| 1 | 10.25 | 8.63 | 3535.01 | 100.00 |
| 2 | 13.01 | 6.81 | 231.55 | 6.55 |
| 3 | 15.40 | 5.76 | 1939.24 | 54.86 |
| 4 | 17.13 | 5.18 | 82.49 | 2.33 |
| 5 | 18.62 | 4.77 | 78.79 | 2.23 |
| 6 | 20.56 | 4.32 | 597.87 | 16.91 |
| 7 | 21.07 | 4.22 | 401.69 | 11.36 |
| 8 | 21.96 | 4.05 | 101.65 | 2.88 |
| 9 | 22.67 | 3.92 | 109.31 | 3.09 |
| 10 | 23.38 | 3.81 | 32.12 | 0.91 |
| 11 | 24.84 | 3.58 | 271.03 | 7.67 |
| 12 | 25.78 | 3.46 | 655.67 | 18.55 |
| 13 | 26.17 | 3.41 | 1836.63 | 51.96 |
| 14 | 27.90 | 3.20 | 54.64 | 1.55 |
| | | | | |
| 15 | 29.50 | 3.03 | 147.04 | 4.16 |

(continued)

| No. | Pos. [°2Th.] | d-spacing [Å] | Height [cts] | Rel. Int. [%] |
|---|---|---|---|---|
| 16 | 30.68 | 2.91 | 165.02 | 4.67 |
| 17 | 31.04 | 2.88 | 90.21 | 2.55 |
| 18 | 35.56 | 2.52 | 33.01 | 0.93 |
| 19 | 36.36 | 2.47 | 45.85 | 1.30 |
| 20 | 37.89 | 2.37 | 49.90 | 1.41 |
| 21 | 39.05 | 2.31 | 137.81 | 3.90 |

Differential Scanning Calorimetry (DSC): DSC analyses were performed on a TA instrument Q2000 Differential Scanning Calorimeter. Indium was used as the calibration standard. Approximately 2-5 mg of sample was placed into a DSC pan. The sample was heated under nitrogen at a rate of 10°C/min, up to a final temperature of 350°C. Melting points were reported as the extrapolated onset temperatures. Thermogravimetric Analysis (TGA): TGA analyses were performed on a TA instrument Q5000 Thermogravimetric Analyzer. Calcium oxalate was used for a performance check. Approximately 2-10 mg of accurately weighed sample was placed on a pan and loaded into the TGA furnace. The sample was heated under nitrogen at a rate of 10°C/min, up to a final temperature of 300°C. Attenuated Total Reflection Fourier-Transform Infrared Analysis (ATR-FTIR): FTIR analyses were performed on the samples "as is" using an attenuated total reflection (ATR) attachment on a Nicolet 6700 FTIR spectrometer. After a background of ambient lab conditions was obtained, samples were placed on the ATR, compressed with the anvil, and spectrum acquired. Miniature Scanning Electron Microscope (Mini SEM) Morphology analysis of the samples was carried out on an Even Mini SEM. Small amounts of samples were dispersed on a sample holder, and then coating with gold viewed with 200x and 1000x magnification. Dynamic Vapor Sorption (DVS) Hygroscopicity was determined on a Surface Measurement Systems DVS. Typically a sample size of 5-30 mg was loaded into the DVS instrument sample pan and the sample was analyzed on a DVS automated sorption analyzer at 25°C. The relative humidity was increased from 0 % to 90 %RH at 10 %RH step. The relative humidity was then decreased in a similar manner to accomplish a full adsorption/desorption cycle. The entire scope of this invention is not limited by the specific examples described herein, but is more readily understood with reference to the appended claims.

SPECIFIC INORMATION CONCERNING ASPECT (VI) OF THE INVENTION

Aspect (vi) of the invention relates to an administration unit comprising crystalline form of a hemisulfate salt of compound (VI). The invention relates to a solid form of hemisulfate salt of compound (VI), namely to crystalline form of a hemisulfate salt of compound (VI), which is useful for treating migraine headaches, neurogenic vasodilation, neurogenic inflammation, thermal injury, circulatory shock, flushing associated with menopause, airway inflammatory diseases, or chronic obstructive pulmonary disease (COPD). Crystalline form of a hemisulfate salt of compound (VI) is described in WO2013/130402, which is incorporated by reference. Hemisulfate salt of compound (VI) can be described (cf. WO2013/130402) by a hemisulfate salt of Compound (VI):

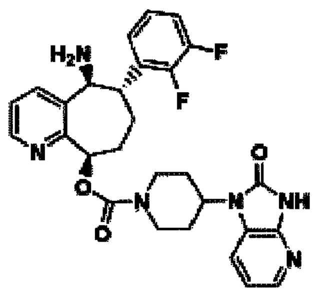

(VI)

As used herein, crystalline form of a hemisulfate salt of compound (VI) is a solid form of hemisulfate salt of compound (VI) characterized by / obtained preferred as described in WO2013/130402, which is cited hereinafter: N-(5S,6S,9R)-5-amino-6-(2,3-10-difluorophenyl)-6,7,8,9-tetrahydro-5H-cyclohepta[b]pyridin-9-yl-4-(2-oxo-2,3-dihydro-1H-imidazo[4,5-b]pyridin-1-yl)piperidine-1-carboxylate hemisulfate salt: Disclosed is a hemisulfate salt of N-(5S,6S,9R)-5-amino-6-(2,3-10-difluorophenyl)-6,7,8,9-tetrahydro-5H-cyclohepta[b]pyridin-9-yl-4-(2-oxo-2,3-dihydro-1H-imidazo[4,5-b]pyridin-1-yl)piperidine-1-carboxylate ({drug6a}), as well as crystalline forms thereof. Also disclosed are at least one pharmaceutical composition comprising the hemisulfate salt of N-(5S,6S,9R)-5-amino-6-(2,3-difluorophenyl)- 6,7,8,9-tetrahydro-5H-cyclohepta[b]pyridin-9-yl-4-(2-oxo-2,3-dihydro-1H-imidazo[4,5-b]pyridin-1-yl)piperidine-1-carboxylate and at least one method of using the hemisulfate salt of N-(5S,6S,9R)-5-amino-6-(2,3-difluorophenyl)-6,7,8,9- tetrahydro-5H-cyclohepta[b]pyridin-9-yl-4-(2-oxo-2,3-dihydro-1H-imidazo[4,5-b] pyridin-1-yl)piperidine-1-carboxylate in the treatment of a CGRP -related disorder, such as migraine headaches and asthma. The compound, N-(5S,6S,9R)-5-amino-6-(2,3-10-difluorophenyl)-6,7,8,9-tetrahydro-5H-cyclohepta[b]pyridin-9-yl-4-(2-oxo-2,3-dihydro-1H-imidazo[4,5-b]pyridin-1-yl)piperidine-1-carboxylate ({drug6})

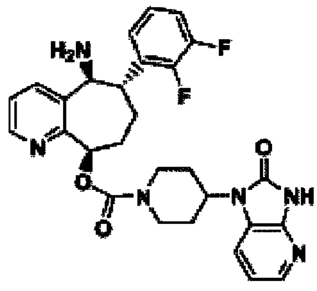

(VI)

and is referred to herein as "Compound (VI)". Compound (VI), processes to prepare Compound (VI), and methods of treatment employing Compound (VI) are disclosed in U.S. Patent Publication 2011/0251223 Al. This reference is assigned to the present assignee and is incorporated herein by reference in its entirety. The usefulness of an oral formulation is dependent upon, among other things, the degree to which the active agent is bioavailable and consistency in bioavailability among patients. The bioavailability of orally administered drugs is often affected by various factors including, for example, the solubility of the drug in the gastrointestinal tract, the stability of the drug in the gastrointestinal tract, and drug absorption in the gastrointestinal tract. Further, these factors may be affected by coadministration of other drugs and/or the intake of food, which may lead to variability in the bioavailability of orally administered drug. Furthermore, rapid in vivo dissolution of the active agent is also required to provide rapid treatment of conditions such as migraine headaches. The dissolution rate of Compound (VI) is dependent on the pH of the aqueous medium. Compound (VI) has a higher dissolution rate at pH values of 1 and 5 than at a pH value of 7. In the oral administration of Compound (VI), the dissolution rate and hence the bioavailability of Compound I can be affected by the pH of the stomach contents. The normal pH of the stomach is 1.2 to 1.8 according to C.J. Perigard, Clinical Analysis, Chapter 32, in Remington: The Science and Practice of Pharmacy 20th Edition, A.R. Gennaro, editor; 2000, Lippinocott Williams & Wilkins, Baltimore, MD. However, patients often take other medications that can raise the pH of the stomach, including antacids, proton pump inhibitors, and H2-receptor antagonists such as famotidine, which can lower the dissolution rate of Compound (VI). Typically, in preparing a pharmaceutical composition, a form of the active ingredient is sought that has a balance of desired properties, such as, for example, dissolution rate, solubility, bioavailability, and/or storage stability. For example, a form of the active ingredient is sought having sufficient stability, solubility, and bioavailability to prevent the sufficiently soluble and bioavailable form from converting during the manufacture, preparation, and/or storage of the pharmaceutical composition to another form having an undesirable solubility and/or bioavailability profile. For example, a form of the active ingredient is sought that is stable and has low hygroscopicity at ambient temperature and humidity conditions. In addition, a form of the active ingredient may also be sought that permits the active ingredient to be produced by a process that is amendable to large-scale production. In such a process, it is desirable that active ingredient is in a form that allows facile isolation and/or purification of the active ingredient, for example, by filtration, as well as easy drying. Further, as production economics are important, it is desirable to avoid the use of higher cost materials, whenever possible, in the preparation of the form. A hemisulfate salt of Compound (VI) has been found that surprisingly reduces the variability in the bioavailability of Compound (VI), provides consistency in bioavailability among patients, and/or increases the bioavailability of Compound (VI) to the patient. Further, a crystalline form of the hemisulfate salt of Compound (VI) has been found that surprisingly reduces the variability in the bioavailability of Compound (VI), provides consistency in bioavailability among patients, and/or increases the bioavailability of Compound (VI) to the patient. The hemisulfate salt of Compound (VI) and the crystalline form thereof, surprisingly afford a balance of properties sought in a pharmaceutical composition. The present invention is also directed to other important aspects. One aspect of the invention is a hemisulfate salt of Compound (VI):

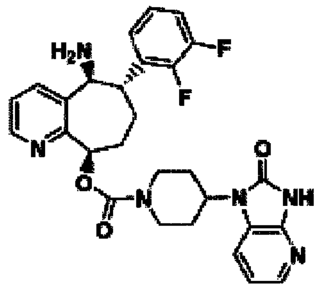

(VI)

The present invention also provides a crystalline form of the hemisulfate salt of Compound (VI). The present invention also provides pharmaceutical compositions comprising a pharmaceutically acceptable carrier and/or diluent; and the hemisulfate salt of Compound (VI). The present invention also provides a method of treating a disease or disorder associated with the activity of the CGRP receptor, the method comprising administering to a mammalian patient the hemisulfate salt of Compound (VI). The present invention also provides processes and intermediates for making the hemisulfate salt of Compound (VI), and/or crystalline forms thereof. The present invention also provides the hemisulfate salt of Compound (VI) for use in therapy. The present invention also provides the hemisulfate salt of Compound (VI) for the manufacture of a medicament for the treatment of migraine headaches, neurogenic vasodilation, neurogenic inflammation, thermal injury, circulatory shock, flushing associated with menopause, airway inflammatory diseases such as asthma, and chronic obstructive pulmonary disease (COPD). These and other features of the invention will be set forth in expanded form as the disclosure continues.

Figure 1 of WO2013/130402 shows the experimental (at room temperature) and simulated (room temperature) PXRD patterns (CuKa $\lambda$=1.5418A) of Form HI.5-1 ({drug6b}) of Example 1. Figure 2 of WO2013/130402 shows the differential scanning calorimetry profile of Form HI.5-1 ({drug6b}) of Example 1. Figure 3 of WO2013/130402 shows the thermogravimetric analysis profile of Form HI.5-1 ({drug6b}) of Example 1. Figure 4 of WO2013/130402 shows the charge polarized magic angle spinning (CPMAS) NMR spectrum of Form HI.5-1 ({drug6b}) of Example 1. Figure 5 of WO2013/130402 shows the moisture sorption isotherm for Example 1 at 25°C. (X) adsorption; (■) desorption. Figure 6 of WO2013/130402 shows the % dissolution versus time at a pH value of approximately 5 in fed state simulated intestinal fluid (FeSSIF) and a pH value of approximately 7 in fasted state simulated intestinal fluid (FaSSIF) for Compound (VI) as free base and the HCl salt of Compound (VI). Figure 7 of WO2013/130402 shows the % dissolution versus time at a pH value of approximately 1, a pH value of approximately 5 in fed state simulated intestinal fluid (FeSSIF), and a pH value of approximately 7 in fasted state simulated intestinal fluid (FaSSIF) for Compound (VI) as free base and the hemisulfate salt of Compound (VI). Figure 8 of WO2013/130402 shows plasma pharmacokinetics of Compound (VI), free base, in humans after oral administration, with and without pretreatment with famotidine (40 mg) two hours prior to administration of Compound (VI). Compound (VI) was administered at a dose of 150 mg. (•) Compound (VI) (nM); (♦) Compound (VI) with famotidine pretreatment (nM). The x-axis is time in minutes. Figure 9 of WO2013/130402 shows plasma pharmacokinetics of Compound (VI) in dogs after the oral administration of Compound (VI) and Example 1. Compound (VI) and Example 1 were orally administered at a dose of 150 mg (or equivalent). (♦) Compound (VI) (nM) and pretreatment with pentagastrin (6 $\mu$m/km); (■) Compound (VI) and famotidine (40 mg); (A) Example 1 (hemisulfate salt) and famotidine (40 mg). Figure 10 of WO2013/130402 shows the experimental (at room temperature) PXRD pattern (CuKa $\lambda$=1.5418A) of Form P22C of Example 1. Figure 11 of WO2013/130402 shows the experimental (at room temperature) PXRD pattern (CuKa $\lambda$=1.5418A) of Form P33 ({drug6c}) of Example 1. Figure 11 shows the experimental (at room temperature) PXRD pattern (CuKa $\lambda$=1.5418A) of Form P35 of Example 1.

The features and advantages of the invention may be more readily understood by those of ordinary skill in the art upon reading the following detailed description. It is to be appreciated that certain features of the invention that are, for clarity reasons, described above and below in the context of separate embodiments, may also be combined to form a single embodiment. Conversely, various features of the invention that are, for brevity reasons, described in the context of a single embodiment, may also be combined so as to form sub-combinations thereof. The names used herein to characterize a specific form, e.g., "HI.5-1", "P22C", "P33", and "P35", are merely identifiers that are to be interpreted in accordance with the characterization information presented herein and are not to be limited so as to exclude any other substance possessing similar or identical physical and chemical characteristics. The definitions set forth herein take precedence over definitions set forth in any patent, patent application, and/or patent application publication incorporated herein by reference. All numbers expressing quantities of ingredients, weight percentages, temperatures, and so forth that are preceded by the word "about" are to be understood as only approximations so that slight variations above and below the stated number may be used to achieve substantially the same results as the stated number. Accordingly,

unless indicated to the contrary, numerical parameters preceded by the word "about" are approximations that may vary depending upon the desired properties sought to be obtained. At the very least, and not as an attempt to limit the application of the doctrine of equivalents to the scope of the claims, each numerical parameter should at least be construed in light of the number of reported significant digits and by applying ordinary rounding techniques. All measurements are subject to experimental error and are within the spirit of the invention. The hemisulfate salt of Compound (VI) is, subsequent to its preparation, preferably isolated and purified to obtain a composition containing an amount by weight equal to or greater than 99%, preferably 99.5%, and more preferably, 99.9%, of the hemisulfate salt of Compound (VI)) ("substantially pure"), which is then used or formulated as described herein. Such "substantially pure" hemisulfate salt of Compound (VI) is also contemplated herein as part of the present invention. As used herein, "polymorphs" refer to crystalline forms having the same chemical structure but different spatial arrangements of the molecules and/or ions forming the crystals. As used herein, "amorphous" refers to a solid form of a molecule and/or ion that is not crystalline. An amorphous solid does not display a definitive X-ray diffraction pattern with sharp maxima. As used herein, the term "substantially pure crystalline form" means the crystalline form of Compound (VI) hemisulfate salt referred to contains at least about 90 wt.% of that form, based on the weight of the Compound (VI) hemisulfate salt. The term "at least about 90 wt.%," while not intending to limit the applicability of the doctrine of equivalents to the scope of the claims, includes, but is not limited to, for example, about 90, 90, about 91, 91, about 92, 92, about 93, 93, about 94, 94, about 95, 95, about 96, 96, about 97, 97, about 98, 98, about 99, 99, and about 100 wt. %, based on the weight of the Compound (VI) hemisulfate salt. The remainder of the Compound (VI) hemisulfate salt may comprise other Form(s) of the Compound (VI) hemisulfate salt including amorphous Compound (VI) hemisulfate salt and/or reaction impurities and/or processing impurities that arise, for example, when the hemisulfate salt is prepared and/or when the crystalline form is prepared. The presence of reaction impurities and/or processing impurities may be determined by analytical techniques known in the art, such as, for example, chromatography, nuclear magnetic resonance spectroscopy, mass spectrometry, and/or infrared spectroscopy. As used herein, the parameter "molecules/asymmetric unit" refers to the number of molecules of Compound (VI) in the asymmetric unit. As used herein, the unit cell parameter "molecules/unit cell" refers to the number of molecules of Compound (VI) in the unit cell. The invention is intended to include all isotopes of atoms occurring in the present compounds. Isotopes include those atoms having the same atomic number but different mass numbers. By way of general example and without limitation, isotopes of hydrogen include deuterium and tritium. Isotopes of carbon include 13C and 14C. Isotopically-labeled compounds of the invention can generally be prepared by conventional techniques known to those skilled in the art or by processes analogous to those described herein, using an appropriate isotopically-labeled reagent in place of the non-labeled reagent otherwise employed. Such compounds may have a variety of potential uses, for example as standards and reagents in determining biological activity. In the case of stable isotopes, such compounds may have the potential to favorably modify biological, pharmacological, or pharmacokinetic properties. The first aspect of the invention provides the hemisulfate salt of Compound (VI),

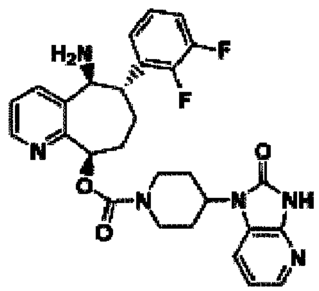

(VI).

The hemisulfate salt of Compound (VI) is an acid salt of Compound (VI) having a ratio of 0.5 H2SO4 molecule to each molecule of Compound (VI), and has the name: (5S,6S,9R)-5-amino-6-(2,3-dfluorophenyl)-6,7,8,9-tetrahydro-5H-cyclohepta[b] pyridin-9-yl 4-(2-oxo-2,3-dihydro- 1 H-imidazo[4,5-b]pyridin-1-yl)piperidine-1-carboxylate, hemisulfate salt. In one embodiment, the hemisulfate salt of Compound (VI) is provided as a sesquihydrate, having a ratio of 1.5 water molecules and 0.5 H2SO4 molecule for each molecule of Compound (VI). In one embodiment, the hemisulfate salt of Compound (VI) is provided as a crystalline form. In one embodiment, the hemisulfate salt of Compound (VI) is provided as a crystalline form, wherein the crystalline form is Form H I.5-1. This crystalline form has a ratio of 1.5 water molecules and 0.5 H2SO4 molecule for each molecule of Compound (VI).

In one embodiment, Form HI.5-1 ({drug6b}) is characterized by unit cell parameters substantially equal to the following:

Cell dimensions: a = 10.92 A, b = 33.04 A, c = 7.90 A, a = 90 degrees, β = 90 degrees, γ = 90 degrees, Space group: P2i2i2

Molecules of Compound (VI)/asymmetric unit: 1, Volume = 2851 A3, Density (calculated) = 1.423 g/cm3, wherein measurement of said crystalline form is at a temperature of about 25°C. In one embodiment, Form HI.5-1 ({drug6b}) is characterized by an observed powder x-ray diffraction pattern substantially in accordance with the pattern shown in Figure 1 of WO2013/130402. In one embodiment, Form HI.5-1 ({drug6b}) is characterized by a simulated powder x-ray diffraction pattern substantially in accordance with the pattern shown in Figure 1 of WO2013/130402. In one embodiment, Form HI.5-1 ({drug6b}) is characterized by a powder x-ray diffraction pattern (CuKa $\lambda$=1.5418A) comprising four or more, preferably five or more, 2Θ values selected from: 5.4±0.1, 8.6±0.1, 9.7±0.1, 12.4±0.1, 14.9±0.1, 17.6±0.1, 18.1.±01, 20.5±0.1, 21.4±0.1, and 22.0±0.1, wherein measurement of the crystalline form is at a temperature of about 25°C. In another embodiment, the Form HI.5-1 ({drug6b}) is characterized by a solid state nuclear magnetic resonance spectra (ssNMR) substantially in accordance with the spectra shown in Figure 4 of WO2013/130402. In one embodiment, Form HI.5-1 ({drug6b}) is characterized by a solid state nuclear resonance spectra comprising six or more, preferably seven or more peaks ($\delta$ (ppm) referenced to TMS) selected from: 26.6±0.1, 2IΛ±0Λ, 28.3±0.1, 30.7±0.1, 43.1±0.1, 45.9±0.1, 47.1±0.1, 52.0±0.1, 54.2±0.1, 72.5±0.1, 117.0±0.1, 117.7±0.1, 124.2±0.1, 125.2±0.1, 128.3±0.1, 130.3±0.1, 131.4±0.1, 134.1±0.1, 140.8±0.1, 144.7±0.1, 148.7±0.1, 149.8±0.1, 151.2±0.1, 153.4±0.1, 155.1±0.1, 155.6±0.1, and 156.7±0.1. In yet an even further embodiment, the Form HI.5-1 ({drug6b}) is characterized by fractional atomic coordinates substantially as listed in Table 1. Fractional Atomic Coordinates of Form HI.5-1 ({drug6b}) Calculated at 25°C. Atomic coordinates (x 104) of Non-hydrogen Atoms and Equivalent, Isotropic Displacement Parameters ($A^2$ x $10^3$):

| | x | y | z | U(eq) |
|---|---|---|---|---|
| C(1) | 7702(3) | 8678(1) | 5047(4) | 45(1) |
| C(2) | 7665(3) | 8376(1) | 6299(4) | 43(1) |
| C(3) | 9272(3) | 8790(1) | 6797(4) | 44(1) |
| C(4) | 6025(4) | 8432(1) | 3770(5) | 70(1) |
| C(5) | 5920(4) | 8128(1) | 4927(5) | 68(1) |
| C(6) | 6764(3) | 8087(1) | 6238(5) | 58(1) |
| C(7) | 9084(3) | 8223(1) | 8839(4) | 46(1) |
| C(8) | 9695(3) | 7827(1) | 8327(5) | 54(1) |
| C(9) | 10218(3) | 7622(1) | 9881(5) | 63(1) |
| C(10) | 8686(4) | 7945(1) | 11700(4) | 58(1) |
| C(11) | 8134(3) | 8159(1) | 10190(4) | 53(1) |
| C(12) | 8697(3) | 7218(1) | 11470(4) | 45(1) |
| C(13) | 7480(3) | 6816(1) | 6484(5) | 63(1) |
| | | | | |
| C(14) | 6737(4) | 6498(1) | 6034(5) | 66(1) |
| C(15) | 6572(3) | 6186(1) | 7183(5) | 56(1) |
| C(16) | 7163(3) | 6198(1) | 8726(4) | 45(1) |
| C(17) | 7940(3) | 6521(1) | 9039(4) | 46(1) |
| C(18) | 7080(3) | 5870(1) | 10058(4) | 47(1) |
| C(19) | 8201(3) | 5591(1) | 10055(4) | 48(1) |
| C(20) | 9403(3) | 5815(1) | 9660(5) | 54(1) |
| C(21) | 9708(3) | 6200(1) | 10646(5) | 54(1) |
| C(22) | 8709(3) | 6526(1) | 10646(4) | 47(1) |
| | | | | |

(continued)

| | x | y | z | U(eq) |
|---|---|---|---|---|
| C(23) | 8230(3) | 5323(1) | 11619(4) | 49(1) |
| C(24) | 8222(3) | 4904(1) | 11444(5) | 64(1) |
| C(25) | 8278(3) | 4654(2) | 12797(7) | 85(1) |
| C(26) | 8326(4) | 4790(2) | 14381(7) | 88(1) |
| C(27) | 8409(4) | 5198(2) | 14617(6) | 87(1) |
| C(28) | 8331(4) | 5461(1) | 13244(5) | 72(1) |
| N(1) | 8644(2) | 8451(1) | 7370(3) | 45(1) |
| N(2) | 8677(2) | 8920(1) | 5375(3) | 48(1) |
| N(3) | 6912(3) | 8717(1) | 3793(4) | 60(1) |
| N(4) | 9279(3) | 7571(1) | 11190(4) | 53(1) |
| N(5) | 8091(3) | 6835(1) | 7965(4) | 54(1) |
| N(6) | 5959(2) | 5617(1) | 9862(4) | 53(1) |
| O(1) | 10190(2) | 8933(1) | 7438(3) | 62(1) |
| O(2) | 7746(2) | 7179(1) | 12241(3) | 62(1) |
| O(3) | 9356(2) | 6904(1) | 10833(3) | 51(1) |
| O(4) | 3908(2) | 4985(1) | 7592(3) | 66(1) |
| O(5) | 4930(3) | 5364(1) | 5467(3) | 75(1) |
| S(1) | 5000 | 5000 | 6494(1) | 46(1) |
| O(1S) | 5000 | 5000 | 2134(5) | 82(1) |
| O(2S) | 3401(3) | 5784(1) | 8737(4) | 80(1) |
| F(1) | 8437(3) | 5844(1) | 13581(3) | 87(1) |
| F(2) | 8604(4) | 5343(1) | 16124(4) | 108(1) |
| F(1A) | 8206(8) | 4781(5) | 9853(10) | 114(6) |
| F(2A) | 8316(9) | 4256(2) | 12660(30) | 159(8) |
| *The difluorophenyl ring is found disordered in the crystal over two orientations (F1/F1A, F2/F2A) with occupancies of 0.817(5) and 0.183(5). | | | | |

Table 2: Fractional Atomic Coordinates of Form HI.5-1 ({drug6b}) Calculated at 25°C; Atomic coordinates (x $10^4$) of Hydrogen Atoms and Equivalent, Isotropic Displacement Parameters ($A^2$ x $10^3$):

| | x | y | z | U(eq) |
|---|---|---|---|---|
| H(4) | 5448 | 8444 | 2906 | 84 |
| | | | | |
| H(5) | 5274 | 7946 | 4840 | 82 |
| H(6) | 6720 | 7877 | 7021 | 69 |
| H(7) | 9727 | 8389 | 9355 | 55 |
| H(8A) | 9099 | 7650 | 7791 | 64 |
| H(8B) | 10345 | 7880 | 7520 | 64 |

(continued)

| | x | y | z | U(eq) |
|---|---|---|---|---|
| H(9A) | 10543 | 7359 | 9569 | 76 |
| H(9B) | 10887 | 7783 | 10327 | 76 |
| H(10A) | 9281 | 8121 | 12238 | 70 |
| H(10B) | 8047 | 7886 | 12517 | 70 |
| H(11A) | 7467 | 7998 | 9738 | 63 |
| H(11B) | 7803 | 8418 | 10541 | 63 |
| H(13) | 7567 | 7029 | 5727 | 76 |
| H(14) | 6353 | 6493 | 4984 | 80 |
| H(15) | 6064 | 5969 | 6916 | 67 |
| | | | | |
| H(18) | 7040 | 6002 | 11167 | 56 |
| H(19) | 8075 | 5406 | 9102 | 58 |
| H(20A) | 10071 | 5626 | 9831 | 65 |
| H(20B) | 9395 | 5884 | 8467 | 65 |
| H(21A) | 10450 | 6316 | 10175 | 65 |
| H(21B) | 9882 | 6126 | 11809 | 65 |
| H(22) | 8173 | 6486 | 11626 | 56 |
| H(24) | 8191 | 4792 | 10344 | 77 |
| H(25) | 8243 | 4381 | 12622 | 101 |
| H(26) | 8303 | 4613 | 15296 | 106 |
| H(2) | 8891 | 9125 | 4774 | 57 |
| H(6A) | 6054 | 5449 | 8992 | 80 |
| H(6B) | 5316 | 5777 | 9675 | 80 |
| H(6C) | 5836 | 5475 | 10803 | 80 |
| H(1SA) | 4864 | 5219 | 2659 | 123 |
| H(2SA) | 3380 | 5533 | 8525 | 120 |
| H(2SB) | 2905 | 5909 | 8107 | 120 |

In a still further embodiment, Form HI.5-1 ({drug6b}) is characterized by a DSC thermogram substantially in accordance with that shown in Figure 2 of WO2013/130402. In still another embodiment, the Form HI.5-1 ({drug6b}) is characterized by a TGA thermogram, wherein the Form HI.5-1 ({drug6b}) experiences a weight loss of approximately 4-5 weight% upon being heated to a temperature of about 200°C. In still an even further embodiment, the Form HI.5-1 ({drug6b}) exhibits a TGA thermogram substantially the same as shown in Figure 3 of WO2013/130402. In still yet another embodiment, Form HI. 5-1 is provided in a substantially pure crystalline form. In still yet an even further embodiment, the hemisulfate salt of Compound (VI) contains at least about 90 wt.%, preferably at least about 95 wt.%, and more preferably at least about 99 wt.%, of Form HI.5-1 ({drug6b}), based on the weight of the hemisulfate salt. In a still further embodiment, a substantially pure Form HI.5-1 ({drug6b}) has substantially pure phase homogeneity with less than about 10%, preferably less than about 5%, and more preferably less than about 2% of the total peak area of the experimentally measured PXRD pattern arising from peaks that are absent from the simulated PXRD pattern. Most preferably, the substantially pure Form HI.5-1 ({drug6b}) has substantially pure phase homogeneity with less than about 1% of the total peak area of the experimentally measured PXRD pattern arising from peaks that are absent from the simulated PXRD pattern. In another embodiment, the hemisulfate salt of Compound (VI) consists essentially of Form

HI.5-1 ({drug6b}). The crystalline form of this embodiment may comprise at least about 90 wt. %, preferably at least about 95 wt. %, and more preferably at least about 99 wt. %, based on the weight of the hemisulfate salt of Compound (VI). In yet another embodiment, a pharmaceutical composition comprises the hemisulfate salt of Compound (VI) in Form HI.5-1 ({drug6b}); and at least one pharmaceutically-acceptable carrier and/or diluent. In one embodiment, the hemisulfate salt of Compound (VI) is provided as a crystalline form, wherein the crystalline form is P22C. This crystalline form is a sesquihydrate having a ratio of 1.5 water molecules and 0.5 H2S04 molecule for each molecule of Compound (VI). In one embodiment, Form P22C is characterized by an observed powder x-ray diffraction pattern substantially in accordance with the pattern shown inFigure 10 of WO2013/130402. In one embodiment, the hemisulfate salt of Compound (VI) is provided as a monohydrate, having a ratio of one water molecule and 0.5 $H_2SO_4$ molecule for each molecule of Compound (VI). In one embodiment, the hemisulfate salt of Compound (VI) is provided as a crystalline form, wherein the crystalline form is Form P33 ({drug6c}). This crystalline form has a ratio of one water molecule and 0.5 H2SO4 molecule for each molecule of Compound (VI). In one embodiment, Form P33 ({drug6c}) is characterized by an observed powder x-ray diffraction pattern substantially in accordance with the pattern shown in Figure 11 of WO2013/130402. Compound (VI) is suitable as a CGRP receptor antagonist and is useful in the treatment of CGRP related disorders including migraine headaches, neurogenic vasodilation, neurogenic inflammation, thermal injury, circulatory shock, flushing associated with menopause, airway inflammatory diseases such as asthma, and chronic obstructive pulmonary disease (COPD). Calcitonin gene-related peptide (CGRP) is a naturally occurring 37-amino-acid peptide first identified in 1982 (Amara, S. G. et al, Science 1982, 298, 240-244). Two forms of the peptide are expressed (aCGRP and CGRP) which differ by one and three amino acids in rats and humans, respectively. The peptide is widely distributed in both the peripheral (PNS) and central nervous system (CNS), principally localized in sensory afferent and central neurons, and displays a number of biological effects, including vasodilation. When released from the cell, CGRP binds to specific cell surface G protein-coupled receptors and exerts its biological action predominantly by activation of intracellular adenylate cyclase (Poyner, D. R. et al, Br J Pharmacol 1992, 105, 441-7; Van Valen, F. et al, Neurosci Lett 1990,119,195-8.). Two classes of CGRP receptors, CGRP1 and CGRP2, have been proposed based on the antagonist properties of the peptide fragment CGRP(8-37) and the ability of linear analogues of CGRP to activate CGRP2 receptors (Juaneda, C. et al. TiPS 2000, 21, 432 438). However, there is lack of molecular evidence for the CGRP2 receptor (Brain, S. D. et al, TiPS 2002, 23, 51-53). The CGRP1 receptor has three components: (i) a 7 transmembrane calcitonin receptor-like receptor (CRLR); (ii) the single transmembrane receptor activity modifying protein type one (RAMP 1); and (iii) the intracellular receptor component protein (RCP) (Evans B. N. et al., J Biol Chem. 2000, 275, 31438-43). RAMP1 is required for transport of CRLR to the plasma membrane and for ligand binding to the CGRP -receptor (McLatchie, L. M. et al, Nature 1998, 393, 333-339). RCP is required for signal transduction (Evans B. N. et al, J Biol Chem. 2000, 275, 31438-43). There are known species-specific differences in binding of small molecule antagonists to the CGRP-receptor with typically greater affinity seen for antagonism of the human receptor than for other species (Brain, S. D. et al, TiPS 2002, 23, 51-53). The amino acid sequence of RAMP 1 determines the species selectivity, in particular, the amino acid residue Trp74 is responsible for the phenotype of the human receptor (Mallee et al. J Biol Chem 2002, 277, 14294-8). Inhibitors at the receptor level to CGRP are postulated to be useful in pathophysiologic conditions where excessive CGRP receptor activation has occurred. Some of these include neurogenic vasodilation, neurogenic inflammation, migraine, cluster headache and other headaches, thermal injury, circulatory shock, menopausal flushing, and asthma. CGRP receptor activation has been implicated in the pathogenesis of migraine headache (Edvinsson L. CNS Drugs 2001 ; 15(10):745-53; Williamson, D. J. Microsc. Res. Tech. 2001, 53, 167-178.; Grant, A. D. Brit. J. Pharmacol. 2002,135, 356-362.). Serum levels of CGRP are elevated during migraine (Goadsby PJ, et al. Ann Neurol 1990;28: 183-7) and treatment with antimigraine drugs returns CGRP levels to normal coincident with alleviation of headache (Gallai V. et al. Cephalalgia 1995; 15: 384-90). Migraineurs exhibit elevated basal CGRP levels compared to controls (Ashina M, et al, Pain 2000, 86(1-2): 133-8.2000). Intravenous CGRP infusion produces lasting headache in migraineurs (Lassen LH, et al. Cephalalgia 2002 Feb;22(1):54-61). Preclinical studies in dog and rat report that systemic CGRP blockade with the peptide antagonist CGRP(8-37) does not alter resting systemic hemodynamics nor regional blood flow (Shen, Y-T. et al, J Pharmacol Exp Ther 2001, 298, 551-8). Thus, CGRP-receptor antagonists may present a novel treatment for migraine that avoids the cardiovascular liabilities of active vasoconstriction associated with non-selective 5-HT1B/1D agonists, 'triptans' (e.g., sumatriptan). CGRP antagonists have shown efficacy in human clinical trials. See Davis CD, Xu C. Curr Top Med Chem. 2008 8(16): 1468-79; Benemei S, Nicoletti P, Capone JG, Geppetti P. Curr Opin Pharmacol. 2009 9(1):9-14. Epub 2009 Jan 20; Ho TW, Ferrari MD, Dodick DW, Galet V, Kost J, Fan X, Leibensperger H, Froman S, Assaid C, Lines C, Koppen H, Winner PK. Lancet. 2008 372:2115. Epub 2008 Nov 25; Ho TW, Mannix LK, Fan X, Assaid C, Furtek C, Jones CJ, Lines CR, Rapoport AM; Neurology 2008 70: 1304. Epub 2007 Oct 3.

Pharmaceutical Compositions and Methods of Treatment: The hemisulfate salt of Compound (VI) inhibits the CGRP receptor. As such, the hemisulfate salt of Compound (VI) is useful for treating conditions or disorders associated with aberrant CGRP levels or where modulating CGRP levels may have therapeutic benefit. Accordingly, another aspect of the invention is a pharmaceutical composition comprising the hemisulfate salt of Compound (VI) with a pharmaceutically acceptable adjuvant, carrier, or diluent. One embodiment provides a pharmaceutical composition comprising a hemisulfate salt of Compound (VI) sesquihydrate with a pharmaceutically acceptable adjuvant, carrier, or diluent. One embodiment provides a pharmaceutical composition comprising a crystalline form of the hemisulfate salt of Compound (VI) with a pharmaceutically acceptable adjuvant, carrier, or diluent. One embodiment provides a pharmaceutical composition comprising a crystalline form of the hemisulfate salt of Compound (VI) sesquihydrate with a pharmaceutically acceptable adjuvant, carrier, or diluent. One embodiment provides a pharmaceutical composition comprising Form HI.5-1 ({drug6b}) of the hemisulfate salt of Compound (VI) with a pharmaceutically acceptable adjuvant, carrier, or diluent. Compounds are generally given as pharmaceutical compositions comprised of a therapeutically effective amount of the hemisulfate salt of Compound (VI), and a pharmaceutically acceptable carrier, and may contain conventional excipients. A therapeutically effective amount is the amount needed to provide a meaningful patient benefit as determined by practitioners in that art. Pharmaceutically acceptable carriers are those conventionally known carriers having acceptable safety profiles. Compositions encompass all common solid and liquid forms including capsules, tablets, lozenges, and powders as well as liquid suspensions, syrups, elixirs, and solutions. Solid compositions may by formed in timed or sustained released formulations. Compositions are made using common formulation techniques and conventional excipients (such as binding and wetting agents) and vehicles (such as water and alcohols). Solid compositions are normally formulated in dosage units providing from about 1 to about 1000 mg of the active ingredient per dose. Some examples of solid dosage units are 0.1 mg, 1 mg, 10 mg, 100 mg, 500 mg, and 1000 mg. Liquid compositions are generally in a unit dosage range of 1-100 mg/mL. Some examples of liquid dosage units are 0.1 mg/mL, 1 mg/mL, 10 mg/mL, 25 mg/mL, 50 mg/mL, and 100 mg/mL. In one embodiment, an oral dosage form provides 70 to 750 mg of Compound (VI) as the hemisulfate salt of Compound (VI). Included in this embodiment are oral dosage forms having 70 mg, 80 mg, 90 mg, 100 mg, 150 mg, 200 mg, 250 mg, 300 mg, 500 mg, and 750 mg of the Compound (VI) as the hemisulfate salt of Compound (VI). In one embodiment, an oral dosage form provides 70 to 750 mg of Compound (VI) as Form HI.5-1 ({drug6b}) of the hemisulfate salt of Compound (VI). Included in this embodiment are oral dosage forms having 70 mg, 80 mg, 90 mg, 100 mg, 150 mg, 200 mg, 250 mg, 300 mg, 500 mg, and 750 mg of the Compound (VI) as Form HI.5-1 ({drug6b}) of the hemisulfate salt of Compound (VI). In one embodiment, the hemisulfate salt of Compound (VI) is administered once a day. Suitable doses include 70 mg, 80 mg, 90 mg, 100 mg, 150 mg, 200 mg, 250 mg, 300 mg, 500 mg, and 750 mg of the Compound (VI) as Form HI.5-1 ({drug6b}) of the hemisulfate salt of Compound (VI). In one embodiment, the hemisulfate salt of Compound (VI) is administered twice a day. Suitable doses include 70 mg, 80 mg, 90 mg, 100 mg, 150 mg, 200 mg, 250 mg, 300 mg, 500 mg, and 750 mg of the Compound (VI) as Form HI.5-1 ({drug6b}) of the hemisulfate salt of Compound (VI). The invention encompasses all conventional modes of administration including oral, parenteral, intranasal, sublingual, and transdermal methods. Typically, the daily dose will be 0.01-100 mg/kg body weight daily. Generally, more compound is required orally and less parenterally. The specific dosing regimen, however, should be determined by a physician using sound medical judgment. Inhibitors at the receptor level to CGRP are postulated to be useful in pathophysiologic conditions where excessive CGRP receptor activation has occurred. Some of these include neurogenic vasodilation, neurogenic inflammation, migraine, cluster headache and other headaches, thermal injury, circulatory shock, menopausal flushing, and asthma. CGRP receptor activation has been implicated in the pathogenesis of migraine headache (Edvinsson L. CNS Drugs 2001, 15(10),745-53; Williamson, D. J. Microsc. Res. Tech. 2001, 53, 167-178.; Grant, A. D. Brit. J. Pharmacol. 2002, 135, 356-362.). Serum levels of CGRP are elevated during migraine (Goadsby P. J. et al. Ann. Neurol. 1990,28,183-7) and treatment with antimigraine drugs returns CGRP levels to normal coincident with alleviation of headache (Gallai V. et al. Cephalalgia 1995, 15, 384-90). Migraineurs exhibit elevated basal CGRP levels compared to controls (Ashina M. et al, Pain 2000, 86(1-2), 133-8). Intravenous CGRP infusion produces lasting headache in migraineurs (Lassen L.H. et al. Cephalalgia. 2002, 22(1), 54-61). Preclinical studies in dog and rat report that systemic CGRP blockade with the peptide antagonist CGRP(8-37) does not alter resting systemic hemodynamics nor regional blood flow (Shen, Y-T. et al. J. Pharmacol. Exp. Ther. 2001, 298, 551-8). Thus, CGRP -receptor antagonists may present a novel treatment for migraine that avoids the cardiovascular liabilities of active vasoconstriction associated with non-selective 5-HT1B/1D agonists, "triptans" (e.g., sumatriptan). Another aspect of the invention is a method of inhibiting the CGRP receptor comprising contacting the CGRP receptor with the hemisulfate salt of Compound (VI). One embodiment provides a method of inhibiting the CGRP receptor comprising contacting the CGRP receptor with the hemisulfate salt of Compound (VI) sesquihydrate. One embodiment provides a method of inhibiting the CGRP receptor comprising contacting the CGRP receptor with a crystalline hemisulfate salt of Compound (VI) sesquihydrate. One embodiment provides a method of inhibiting the CGRP receptor comprising contacting the CGRP receptor with Form HI.5-1 ({drug6b}) of the hemisulfate salt of Compound (VI). Another aspect of the invention is a method for treating conditions associated with aberrant levels of CGRP comprising the administration of a therapeutically effective amount of the hemisulfate salt of Compound (VI) to a patient.

Another aspect of the invention is the use of the hemisulfate salt of Compound (VI) in the manufacture of a medicament for the treatment of conditions related to aberrant levels of CGRP. Another aspect of the invention is a method of treating migraine or headache. Another aspect of the invention relates to a method of treating inflammation (particularly neurogenic inflammation), pain, thermal injury, circulatory shock, diabetes, Reynaud's syndrome, peripheral arterial insufficiency, subarachnoid/ cranial hemorrhage, tumor growth, flushing associated with menopause and other

conditions the treatment of which can be effected by the antagonism of the CGRP receptor by the administration of pharmaceutical compositions comprising the hemisulfate salt of Compound (VI) as defined herein. Another aspect of the invention relates to methods selected from the group consisting of (a) immune regulation in gut mucosa (b) protective effect against cardiac anaphylactic injury (c) stimulating or preventing interleukin-1b(IL-1b)-stimulation of bone resorption (d) modulating expression of NK1 receptors in spinal neurons and (e) airway inflammatory diseases and chronic obstructive pulmonary disease including asthma. See (a) Calcitonin Receptor-Like Receptor Is Expressed on Gastrointestinal Immune Cells. Hagner, Stefanie; Knauer, Jens; Haberberger, Rainer; Goeke, Burkhard; Voigt, Karlheinz; McGregor, Gerard Patrick. Institute of Physiology, Philipps University, Marburg, Germany. Digestion (2002), 66(4), 197-203 ; (b) Protective effects of calcitonin gene-related peptide-mediated evodiamine on guinea-pig cardiac anaphylaxis. Rang, Wei-Qing; Du, Yan-Hua; Hu, Chang-Ping; Ye, Feng; Tan, Gui-Shan; Deng, Han-Wu; Li, Yuan-Jian. School of Pharmaceutical Sciences, Department of Pharmacology, Central South University, Xiang-Ya Road 88, Changsha, Hunan, Naunyn-Schmiedeberg's Archives of Pharmacology (2003), 367(3), 306-311; (c) The experimental study on the effect calcitonin gene-related peptide on bone resorption mediated by interleukin-1. Lian, Kai; Du, Jingyuan; Rao, Zhenyu; Luo, Huaican. Department of Orthopedics, Xiehe Hospital, Tongji Medical College, Huazhong University of Science and Technology, Wuhan, Peop. Rep. China. Journal of Tongji Medical University (2001), 21(4), 304-307, (d) Calcitonin gene-related Peptide regulates expression of neurokininl receptors by rat spinal neurons. Seybold VS, McCarson KE, Mermelstein PG, Groth RD, Abrahams LG. J. Neurosci. 2003 23 (5): 1816-1824. Department of Neuroscience, University of Minnesota, Minneapolis, Minnesota 55455, and Department of Pharmacology, Toxicology, and Therapeutics, University of Kansas Medical Center, Kansas City, Kansas 66160 (e) Attenuation of antigen-induced airway hyperresponsiveness in CGRP-deficient mice. Aoki-Nagase, Tomoko; Nagase, Takahide; Oh-Hashi, Yoshio; Shindo, Takayuki; Kurihara, Yukiko; Yamaguchi, Yasuhiro; Yamamoto, Hiroshi; Tomita, Tetsuji; Ohga, Eijiro; Nagai, Ryozo; Kurihara, Hiroki; Ouchi, Yasuyoshi. Department of Geriatric Medicine, Graduate School of Medicine, University of Tokyo, Tokyo, Japan. American Journal of Physiology (2002), 283(5,Pt. 1), L963-L970; (f) Calcitonin gene-related peptide as inflammatory mediator. Springer, Jochen; Geppetti, Pierangelo; Fischer, Axel; Groneberg, David A. Charite Campus - Virchow, Department of Pediatric Pneumology and Immunology, Division of Allergy Research, Humboldt-University Berlin, Berlin, Germany. Pulmonary Pharmacology & Therapeutics (2003), 16(3), 121-130; and (g) Pharmacological targets for the inhibition of neurogenic inflammation. Helyes, Zsuzsanna; Pinter, Erika; Nemeth, Jozsef; Szolcsanyi, Janos. Department of Pharmacology and Pharmacotherapy, Faculty of Medicine, University of Pecs, Pecs, Hung. Current Medicinal Chemistry: Anti-Inflammatory & Anti-Allergy Agents (2003), 2(2), 191-218. Another aspect of this invention relates to a method of treatment using combinations of the hemisulfate salt of Compound (VI) with one or more agents selected from the group consisting of COX-2 inhibitors, NSAIDS, aspirin, acetaminophen, triptans, ergotamine and caffeine for the treatment of migraine. "Migraine," "headache," and related terms are as understood by medical practitioners. Migraine encompasses all classes of migraine including common, classic, cluster, fulgurating, hemiplegic, opthalmoplegic, and opthomalmic. By "therapeutically effective amount" is meant an amount that when administered either alone, or in combination with an additional therapeutic agent is effective to prevent, suppress, and/or ameliorate a disease and/or condition and/or the progression of a disease and/or condition. "Patient" means a person who may benefit from treatment as determined by medical practitioners.

The invention is further defined in the following Examples. It should be understood that the Examples are given by way of illustration only. From the above discussion and the Examples, one skilled in the art can ascertain the essential characteristics of the invention, and without departing from the spirit and scope thereof, can make various changes and modifications to adapt the invention to various uses and conditions. As a result, the invention is not limited by the illustrative examples set forth hereinbelow, but rather is defined by the claims appended hereto.

ABBREVIATIONS: DMSO dimethylsulfoxide, EDTA ethylenediaminetetraacetic acid, eq equivalent(s), ESI electrospray ionization mass spectroscopy, g gram(s), h hour(s), L liter(s), LCMS liquid chromatography mass spectrometry, M molar, mg milligram(s), min minute(s), mL milliliter(s), mmol millimole(s), MS mass spectrometry, N normal, NaHMDS sodium bis(trimethylsilyl)amide, NCS N-chlorosuccinimide, NMR nuclear magnetic resonance spectroscopy, RT retention time, ssNMR solid state nuclear magnetic resonance, TBAF tetrabutylammonium fluoride, TFA trifluoroacetic acid, THF tetrahydrofuran, TIPSO triisopropylsilyloxy, TMS tetramethylsilane, μL microliter(s), °C degrees Celsius.

Proton magnetic resonance (1H NMR) spectra were recorded on a Bruker AC AC 500. All spectra were determined in the solvents indicated and chemical shifts are reported in δ units downfield from the internal standard tetramethylsilane (TMS) and interproton coupling constants are reported in Hertz (Hz). Splitting patterns are designated as follows: s, singlet; d, doublet; t, triplet; q, quartet; m, multiplet; br, broad peak. Low resolution mass spectra (MS) and the apparent molecular (MH+) or (M-H)+ was determined on a Micromass platform. Elemental analyses are reported as percent by weight. The products were purified by Preparative HPLC using the column YMC S5 ODS (30 x 100 mm) at a flow rate of 40.0 mL/min and gradient time of 8.0 min. starting from solvent composition of 40% methanol-60% water-0.1% TFA and ending with solvent composition 95% methanol-5% water-0.1% TFA. The products were analyzed by a HPLC instrument using an XTERA column (3.0 x 50 mm S7) starting from solvent A (10% methanol - 90% water - 0.1% trifluoroacetic acid (TFA)) and reaching solvent B (10% water - 90% methanol -0.1% TFA) over a gradient time

of 2 min. The flow rate is 5 mL/min. and retention time (Rf) of product was measured at 220 nm wavelength.

INTERMEDIATE 1: (6S,9R)-6-(2,3-Difluorophenyl)-9-hydroxy-6,7,8,9-tetrahydro-5H- cyclohepta[b]pyridin-5 -one

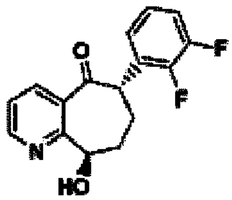

(Int. 1)

In a 250 mL round-bottom flask was dissolved (9R)-6-(2,3-difluorophenyl)-9- (triisopropylsilyloxy)-6,7,8,9-tetrahydro-5H-cyclohepta[b]pyridin-5-one (0.218 g, 0.49 mmol) in tetrahydrofuran (5 mL) to give a colorless solution. After cooling to - 15°C (ice-methanol bath) under nitrogen, TBAF (0.490 mL, 0.490 mmol) was added, and the resulting bright yellow solution was stirred at -15°C for 1h. It was quenched with sodium bicarbonate solution and diluted with ethyl acetate. The layers were separated and the aqueous layer was extracted with ethyl acetate. The combined organic layers was washed with brine, dried and concentrated to give a tan oil. Flash column chromatography (25 g silica gel column) up to 100% ethyl acetate/hexane afforded the desired product (112mg, 62%). 1HNMR (400 MHz, CHLOROFORM-d) δ ppm 8.53 (dd, J=4.91, 1.64 Hz, 1 H) 7.85 (dd, J=7.68, 1.64 Hz, 1 H) 7.34 (dd, J=7.68, 4.91 Hz, 1 H) 7.00-7.16 (m, 3 H) 5.32 (s, 1 H) 4.94-5.04 (m, 1 H) 4.48 (dd, J=11.83, 3.02 Hz, 1 H) 2.14-2.48 (m, 4 H); 19F NMR (376 MHz, CHLOROFORM-d) δ ppm -138.24-138.07 (m, 1 F) - 140.70-140.50 (m, 1 F).

INTERMEDIATE 2: (5S,6S,9R)-6-(2,3-difluorophenyl)-9-(triisopropylsilyloxy)-6,7,8,9-tetrahydro-5H- cyclohepta[b]-pyridin-5-ol.

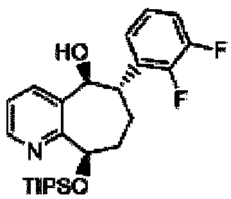

(Int-2)

Lithium borohydride (0.982 g, 45.1 mmol) was added to a cyclopentyl methyl ether (30 mL) solution of (6S,9R)-6-(2,3-difluorophenyl)-9-(triisopropylsilyloxy)-6,7,8,9-tetrahydro-5H-cyclohepta[b]pyridin-5-one (5.0224 g, 11.27 mmol) at 0°C under N2. The reaction mixture was stirred at 0°C for 2 hours and then an addition 4 hours at room temperature. The reaction was quenched by adding methanol. The reaction mixture was stirred for 0.5 hour. The solvent was mostly removed via vacuum and the crude material was taken up in ethyl acetate, which was washed with water three times. Flash column by ethyl acetate in hexane from 0 to 10% gave the desired product (3.28g, 65%).

INTERMEDIATE 3: (5R,6S,9R)-5-chloro-6-(2,3-difluorophenyl)-9-(triisopropylsilyloxy)-6,7,8,9- tetrahydro-5H-cyclohepta[b]pyridine

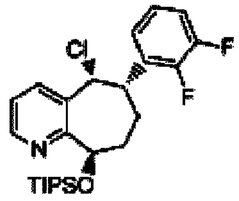

(Int.3)

In an oven-dried 250 mL round-bottom flask was suspended NCS (0.751 g, 5.62 mmol) in tetrahydrofuran (15 mL). Triphenylphosphine (1.475 g, 5.62 mmol) was added. After stirring under nitrogen for 5min, (5S,6S,9R)-6-(2,3-difluorophenyl)-9-(triisopropylsilyloxy)-6,7,8,9-tetrahydro-5H-cyclohepta[b]pyridin- 5-ol (1.007 g, 2.250 mmol) was added in one portion to the gray suspension. The resulting reddish suspension was stirred at room temperature. The solids gradually dissolved to give a tan solution. After 5h, LCMS indicated complete conversion. Tetrahydrofuran was removed in vacuo and the remaining red oil was directly purified by ISCO (240g silica column) up to 60% ethyl acetate/hexane. Pure ethyl acetate eluted the non polar component and the product was eluted by 10% methanol (with 2.0M NH4OH) in methylene chloride. The product fractions were combined and re-purified by FCC up to 50% ethyl acetate/hexane to afford the desired product as a colorless oil (869mg, 83%). MS(ESI)[M+H+] = 466.22; 1H NMR (400 MHz, CHLOROFORM-d) δ ppm 8.55 (d, J=3.53 Hz, 1 H) 7.63 (br. s., 1 H) 7.20 (dd, J=7.68, 4.91 Hz, 1 H) 7.01-7.15 (m, 1 H) 6.90-7.01 (m, 1 H) 6.66-6.90 (m, 1 H) 5.55-5.85 (m, 1 H) 5.40-5.56 (m, 1 H) 3.96-4.33 (m, 1 H) 2.33 (br. s., 3 H) 2.09-2.20 (m, 1 H) 1.14-1.23 (m, 3 H) 1.04-1.14 (m, 9 H) 1.01 (d, J=7.30 Hz, 9 H).

INTERMEDIATE 4: (5S,6S,9R)-5-azido-6-(2,3-difluorophenyl)-9-(triisopropylsilyloxy)-6,7,8,9-tetrahydro-5H-cyclohepta-[b]pyridine

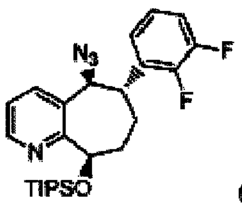

(Int.4)

In a 100 mL round-bottom flask was dissolved (5R,6S,9R)-5-chloro-6-(2,3-difluorophenyl)-9-(triisopropylsilyloxy)-6,7,8,9-tetrahydro-5H-cyclohepta[b]pyridine (566 mg, 1.214 mmol) in dimethylformamide (5 mL) to give a colorless solution. Sodium azide (474 mg, 7.29 mmol) was added, and the mixture was stirred at room temperature under nitrogen for 2.5h. LCMS indicated only partial reaction. The mixture was heated at 50°C overnight. After 15h, LCMS indicated complete conversion with some elimination product. The mixture was diluted with water and ethyl acetate. The layers were separated. The organic layer was washed with brine, dried, and concentrated to give a colorless oil. The crude product was carried onto the next reaction without further purification and characterization. Smaller scale purification afforded an analytical sample: MS(ESI)[M+H+] = 473.27; 1H NMR (400 MHz, CHLOROFORM-d) δ ppm 8.52-8.63 (m, 1 H) 7.75 (d, J=7.81 Hz, 1 H) 7.23-7.36 (m, 1 H) 6.95-7.17 (m, 2 H) 6.89 (br. s., 1 H) 5.28 (d, J=4.03 Hz, 1 H) 4.90 (d, J=9.07 Hz, 1 H) 3.79 (t, J=9.44 Hz, 1 H) 1.86-2.23 (m, 4 H) 1.16-1.30 (m, 3 H) 0.98-1.15 (m, 18 H); 19F NMR (376 MHz, CHLOROFORM-d) δ ppm -137.68-137.36 (m, 1 F) -141.78-141.54 (m, 1 F).

INTERMEDIATE 5: (5S,6S,9R)-5-azido-6-(2,3-difluorophenyl)-6,7,8,9-tetrahydro-5H- cyclohepta[b]pyridin-9-ol

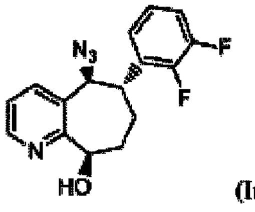

(Int-5)

In a 100 mL round-bottom flask was dissolved (5S,6S,9R)-5-azido-6-(2,3-difluorophenyl)-9-(triisopropylsilyloxy)-6,7,8,9-tetrahydro-5H-cyclohepta[b]pyridine (0.732 g, 1.549 mmol) (crude) in tetrahydrofuran (8 mL) to give a colorless solution. TBAF (1.859 mL, 1.859 mmol) was added, and the resulting light yellow solution was stirred at room temperature for 1.5h. LCMS indicated complete conversion. Tetrahydrofuran was removed and the residue was diluted with water and ethyl acetate. The layers were separated. The organic layer was washed with brine, dried, and concentrated to give a light yellow oil. Purification by FCC up to 60% ethyl acetate/hexane afforded the desired product (crude weight: 480mg) as a colorless oil. Smaller scale purification afforded an analytical sample: MS(ESI)[M+H+] = 317.22; ¾ NMR (400 MHz, CHLOROFORM-d) δ ppm 8.51 (dd, J=4.91, 1.38 Hz, 1 H) 7.99 (d, J=7.30 Hz, 1 H) 7.35 (dd, J=7.81, 5.04 Hz, 1 H) 7.06-7.20 (m, 2 H) 6.94-7.05 (m, 1 H) 5.91 (br. s., 1 H) 5.03 (d, J=10.32 Hz, 1 H) 4.92 (dd, J=11.21, 2.39 Hz, 1 H) 2.84-3.02 (m, 1 H) 2.37-2.49 (m, 1 H) 2.25-2.36 (m, 1 H) 2.07-2.17 (m, J=14.38, 4.94, 3.05, 3.05 Hz, 1 H) 1.40-1.64 (m, 1 H); 13C NMR (101 MHz, CHLOROFORM-if) δ ppm 158.48 (s, 1 C) 152.19-149.87 (dd, J=13.10 and 221Hz, 1 C) 149.72-147.42 (dd, J=13.87 and 219 Hz, 1 C) 146.16 (s, 3 C) 133.67 (s, 2 C) 133.23 (s, 1 C) 132.66 (d, J=10.79 Hz, 1 C) 124.43 (dd, J=6.94, 3.85 Hz, 2 C) 123.84 (br. s., 1 C) 122.89 (s, 2 C) 115.98 (d, J=17.73 Hz, 2 C) 70.94 (s, 3 C) 65.67 (s, 1 C) 45.43 (br. s., 1 C) 35.71 (s, 3 C) 33.45 (s, 2C); 19F NMR (376 MHz, CHLOROFORM-d) δ ppm -137.55-137.20 (m, 1 F) -142.28-141.89 (m, 1 F).

INTERMEDIATE 6: (5S,6S,9R)-5-azido-6-(2,3-difluorophenyl)-6,7,8,9-tetrahydro-5H- cyclohepta[b]pyridin-9-yl 4-(2-oxo-2,3-dihydro-1H-imidazo[4,5-b]pyridin-1-yl)piperidine-1-carboxylate

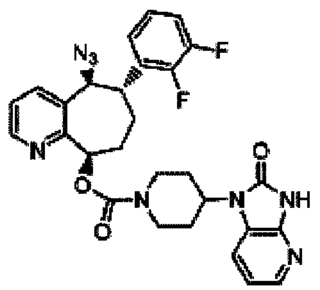

(Int.6)

In a 100 mL round-bottom flask was dissolved (5S,6S,9R)-5-azido-6-(2,3-difluorophenyl)-6,7,8,9-tetrahydro-5H-cyclohepta[b]pyridin-9-ol (0.490 g, 1.549 mmol) (azeotroped with dry benzene) and 4-nitrophenyl 4-(2-oxo-2,3-dihydro-1H-imidazo[4,5-b]pyridin-1-yl)piperidine-1-carboxylate (0.713 g, 1.859 mmol) in dimethylformamide (8 mL) to give a light yellow suspension under nitrogen. After cooling to -15°C (ice-methanol bath), NaHMDS (4.18 mL, 4.18 mmol) was added dropwise. The resulting tan solution was stirred under nitrogen at -10°C to 0°C for 2h and at room temperature for 2h. LCMS showed complete conversion. The reaction was quenched with sodium bicarbonate solution. The mixture was diluted with ethyl acetate. The layers were separated and the aqueous layer was extracted with ethyl acetate. The combined organic layers were washed with water, brine, dried with sodium sulfate, and concentrated to give a tan oil. Purification by FCC up to 8% methanol/methylene chloride afforded the desired product (major peak, 632mg, 73% for 3 steps) as a light yellow foam. MS(ESI)[M+H+] = 561.27; 1H NMR (400 MHz, CHLOROFORM-d) δ ppm 11.50 (br. s., 1 H) 8.58 (d, J=3.78 Hz, 1 H) 8.11 (d, J=5.04 Hz, 1 H) 7.91 (d, J=7.30 Hz, 1 H) 7.33 (br. s., 2 H) 7.07-7.19 (m, 2 H) 6.92-7.06 (m, 2 H) 6.10 (d, J=9.32 Hz, 1 H) 5.23 (d, J=10.07 Hz, 1 H) 4.26-4.84 (m, 3 H) 2.46-3.34 (m, 4 H) 2.20-2.43 (m, 3 H) 2.01-2.13 (m, 1 H) 1.94 (d, J=12.34 Hz, 3 H); 19F NMR (376 MHz, CHLOROFORM-d) δ ppm -137.30-137.01 (m, 1 F) -142.32-142.03 (m, 1 F).

COMPOUND (VI): (5S"6S,9R)-5-amino-6-(2,3-difluorophenyl)-6,7,8,9-tetrahydro-5H-cyclohepta[b]pyridin-9-yl 4-(2-oxo-2,3-dihydro-1H-imidazo[4,5-b]pyridin-1-yl)piperidine-1-carboxylate

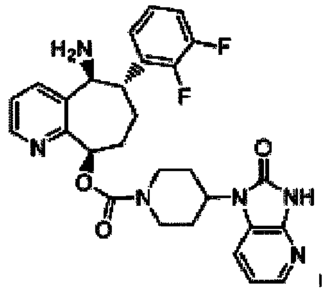

I

In a 100 mL round-bottom flask was dissolved (55"65',9R)-5-azido-6-(2,3-difluorophenyl)-6,7,8,9-tetrahydro-5H-cyclohepta[b]pyridin-9-yl 4-(2-oxo-2,3-dihydro-1H-imidazo[4,5-b]pyridin-1-yl)piperidine-1-carboxylate (620 mg, 1.106 mmol) (Intermediate 6) in tetrahydrofuran (5 mL) to give a colorless solution. Trimethylphosphine (3.32 mL, 3.32 mmol, 1.0 M in toluene) was added. The mixture was stirred at room temperature. After 2h, LCMS showed no starting material. Water (0.080 mL, 4.42 mmol) was added, and the mixture was stirred for another 3h. LCMS showed complete conversion to the desired product. Volatile components were removed in vacuo and the residue was directly purified by FCC up to 10% methanol in methylene chloride to afford the product (5 lOmg, 85%) as a white solid. MS(ESI)[M+H+] = 535.23; 1 H NMR (400 MHz, CHLOROFORM-d) $\delta$ ppm 10.39 (br. s., 1 H) 8.52 (d, J=3.78 Hz, 1 H) 8.09 (d, J=5.04 Hz, 2 H) 7.46 (br. s., 1 H) 7.26-7.38 (m, 1 H) 7.06-7.20 (m, 3 H) 6.94-7.05 (m, 1 H) 6.06-6.23 (m, 1 H) 4.31-4.78 (m, 4 H) 4.05 (spt, J=6.13 Hz, 1 H) 2.57-3.25 (m, 3 H) 2.17-2.38 (m, 3 H) 1.42-2.04 (m, 6 H); 19F NMR (376 MHz, CHLOROFORM-d) $\delta$ ppm -136.90 (br. s., 1 F) -142.48-142.21 (m, 1 F). High Throughput Salt Screening for Crystalline Salts of Compound (VI): High throughput crystallization was employed to screen for the formation of crystalline salts of Compound (VI). The screening examined acid type, acid level (equivalents), and/or type of crystallization solvent. Each plate contained 96 well plates (8 rows of 12 columns per plate). A solution was prepared by dissolving 400 mg of Compound (VI) in a mixture of 36 ml THF and 4 mL H2O. The solution (12.5 mL) was transferred to 24 vials. To each vial was added 0.25 M EtOH stock solutions of the following acids:

| | | |
|---|---|---|
| 1 eq. acetic acid | 1 eq. L-lactic acid | 2 eq. succinic acid |
| 1 eq. benzoic acid | 1 eq. maleic acid | 0.5 eq. sulfuric acid |
| 1 eq. benzenesulfonic acid | 1 eq. L-malic acid | 1. eq. sulfuric acid |
| 1 eq. citric acid | 1 eq. methanesulfonic acid | 1 eq. sulfuric acid |
| 1 eq. fumaric acid | 2 eq. methanesulfonic acid | 2 eq. sulfuric acid |
| 2 eq. fumaric acid | 1 eq. phosphoric acid | 1 eq. D-tartaric acid |
| 1 eq. hydrochloric acid | 2 eq. phosphoric acid | 1 eq. L-tartaric acid |
| 2 eq. hydrochloric acid | 1 eq. succinic acid | 2 eq. L-tartaric acid |

The content of each vial was transferred to 12 crystallization wells and evaporated to dryness. Upon evaporation, each well was charged with 100 $\mu$l of solvent using a robotic liquid handler. The following crystallization solvents were tested: methyl isobutyl ketone (MIBK), ethyl acetate, toluene, THF, acetonitrile, acetone, isopropanol, ethanol, methanol, 1,2-dichloroethylene, isopropanol/water (50:50), and water. Next, the plates were sealed with Teflon septa and subjected to temperature cycling. The plates were maintained at 50°C for 10 hours and then allowed to cool to room temperature over a period of 14 hours. After the heating/cooling cycle, the contents of the wells were characterized by birefringent imaging. Perceived crystalline hits were further characterized by PXRD analysis. Crystalline salt formation was not observed for Compound (VI) in the presence of acetic acid, benzoic acid benzenesulfonic acid, L-lactic acid, maleic acid, L-malic acid, phosphoric acid, and succinic acid. Crystalline salt formation was observed for Compound (VI) in the presence of citric acid, fumaric acid, hydrochloric acid, methanesulfonic acid, sulfuric acid, D-tartaric acid, and L-tartaric acid, in at least one solvent. The properties of the crystalline salts of Compound (VI) were characterized further. The results of the salt screen and crystalline salt characterization are shown in Table 3:

| Acid | Equiv. | Observations |
|---|---|---|
| Acetic acid | 1 | No crystalline salt formation. |

(continued)

| Acid | Equiv. | Observations |
|---|---|---|
| Benzoic acid | 1 | No crystalline salt formation. |
| Benzenesulfonic acid | 1 | No crystalline salt formation. |
| Citric acid | 1 | Crystalline salt; hygroscopic at ambient temperature and relative humidity conditions. PXRD patterns of slurry and dried material did not match. |
| Fumaric acid | 1, 2 | Crystalline salt; hygroscopic at ambient temperature and relative humidity conditions; samples contained about 6 wt. % water at >25% relative humidity. |
| Hydrochloric acid | 1, 2 | Crystalline salt; crystals were hygroscopic at ambient temperatures and relative humidity conditions; crystalline salt samples included multiple hydration states and multiple crystalline forms. Solid state NMR indicated that prepared samples contained mixture of crystalline phases. |
| L-lactic acid | 1 | No crystalline salt formation. |
| Maleic acid | 1 | No crystalline salt formation. |
| L-malic acid | 1 | No crystalline salt formation. |
| Methanesulfonic acid | 1, 2 | Crystalline salt obtained in salt screen. Unable to scale-up preparation of crystalline salt for >40 mg samples. Scale-up yielded mixture of amorphous salt and free base. |
| Phosphoric acid | 1, 2 | No crystalline salt formation observed in high throughput screening. Phosphate salt was |
| | | |
| | | isolated in manual salt screening study, but converted to free base in an EtOH/water slurry. |
| Succinic acid | 1, 2 | No crystalline salt formation. |
| Sulfuric acid | 0.5, 1, 2 | Crystalline hemisulfate salt obtained at 0.5, 1, and 2 equivalents $H_2SO_4$; crystallized reproducibly with high purity and yield; chemically stable; physically stable; low/non-hygroscopic at ambient temperatures and relative humidity conditions. PXRD patterns of slurry and dried material matched. Solid state NMR indicated single phase. |
| D-tartaric acid | 1 | Crystalline salt; non-naturally occurring stereoisomer; expensive material. |
| | | |
| L-tartaric acid | 1, 2 | Crystalline salt; different PXRD patterns for slurry and dried phases; heated drying of slurry needed to remove solvent efficiently for larger scale process; however, heating results in partial loss of crystallinity and non-reproducibility of drying process |

EXAMPLE 1: (5S,6S,9R)-5-Amino-6-(2,3-difluorophenyl)-6,7,8,9-tetrahydro-5H- cyclohepta[b]pyridin-9-yl 4-(2-oxo-2,3-dihydro-1H-imidazo[4,5-b]pyridin-1-yl) piperidine-1-carboxylate, Hemisulfate Salt: Preparation from Ethanol/Water Solution:

Compound (VI) (1 g) was dissolved in 17 mL of ethanol and water (3: 1) at 70°C (Solution A). Separately, 52 of 96% H2SO4 (0.5 equiv.) was dissolved in 8 mL of ethanol and water (3 : 1) at room temperature (Solution B). Next, 30 mg of seeds were added to the Solution A. Solution B was added to the seeded Solution A over a period of 2 hours with a syringe pump. The resulting slurry was stirred at 70°C for 1 hour and cooled to 20°C over 90 min. The slurry was allowed to stir at room temperature overnight. The slurry was filtered. The wet cake was washed with 8 mL of EtOH:water solution (3 : 1), and dried at 30°C in a vacuum oven overnight to afford 1.01 g (88.4 mole %) of Example 1 as a crystalline solid. GADDS showed that crystalline solid was in Form H-1.5. Preparation from Tetrahydrofuran/Water Solution: Compound (VI) (1 g) was dissolved in 10 mL of THF and water (4: 1) at 50°C (Solution A). Separately, 52 μE of 96% H2S04 (0.5 equiv.) was dissolved in 10 mL of THF at room temperature (Solution B). Next, 0.5 mL of Solution B was added to

Solution A, followed by the addition of 20 mg of seeds. The solution changed into a thin slurry. The remaining quantity of Solution B was to the slurry over a period of 2 hours with a syringe pump. The slurry was stirred at 50°C for 1 hour and then allowed to cool to 20°C over a period of 1 hour. The slurry was stirred at room temperature overnight. The slurry was filtered. The wet cake was washed 8 mL of THF:water=3 : 1, and dried at 30°C in a vacuum oven overnight to afford 1.06 g (92.8 mole %) of Example 1 as a crystalline solid. GADDS showed that crystalline solid was in Form H-1.5.

Stability Study: The solid state stability of Example Iwas tested by exposing samples to various temperature and relative humidity conditions for periods of 1, 2, and 4 weeks. The % potency (% pot.) and the % total impurities (% total imp.) are shown in Table 4. The results indicate that the crystalline Form HI.5-1 ({drug6b}) of the hemisulfate salt of Compound (VI) is stable under the tested storage conditions as indicated by no significant increase in total impurity levels and/or no decrease in potency after four weeks of storage.

Table 4: Solid State Stability of Hemisulfate Salt, Form HI.5-1 ({drug6b}):

| | Initial | | 1 week | | 2 weeks | | 4 weeks | |
|---|---|---|---|---|---|---|---|---|
| | % pot. | % total imp. | % pot. | % total imp. | % pot. | % total imp. | % pot. | % total imp. |
| Refrigerated | 98.5 | 0.27 | 98.0 | 0.29 | 96.5 | 0.27 | 105.7 | 0.27 |
| 25 RH/ 60 °C | | | 94.4 | 0.27 | 96.8 | 0.27 | 96.9 | 0.27 |
| | | | | | | | | |
| 25 RH/ 60 °C | | | 95.1 | 0.27 | 98.5 | 0.27 | 97.0 | 0.27 |
| 25 RH/ 60 °C | | | 101.6 | 0.28 | 102.6 | 0.27 | 110.0 | 0.27 |
| HIL/UV | | | 100.7 | 0.27 | 96.9 | 0.27 | - | - |

% pot. = % potency
% total imp. = % total impurities
HIL/UV: high-intensity light/ultraviolet

The moisture sorption isotherm for Example 1 is shown in Figure 5 of WO2013/130402. Example 1 has moisture sorption weight gains of 0.8 weight % and 2.8 weight % between 25% and 75% relative humidity and 5% and 95% relative humidity, respectively. These results indicated that the hemisulfate salt of Compound (VI) is low or non-hygroscopic under the tested conditions Form HI.5-1 ({drug6b}). Table 5 shows characteristic PXRD diffraction peak positions (degrees $2\Theta \pm 0.1$) measured at about 25°C for Example 1, based on a high quality pattern collected with a diffractometer (CuKa) with a spinning capillary with $2\Theta$ calibrated with a NIST other suitable standard. PXRD Peak Positions (degrees $2\Theta \pm 0.1$):

| | |
|---|---|
| 5.4 | 17.6 |
| 8.6 | 18.1 |
| 9.7 | 20.5 |
| 12.4 | 21.4 |
| 14.9 | 22.0 |

Table 6 shows characteristic solid state NMR peak positions $\delta$ (ppm) for Example 1, referenced to TMS. ssNMR peak positions - 8 (ppm):

| | | |
|---|---|---|
| 26.6 | 72.5 | 140.8 |
| 27.1 | 117.0 | 144.7 |
| 28.3 | 117.7 | 148.7 |
| 30.7 | 124.2 | 149.8 |
| 43.1 | 125.2 | 151.2 |
| 45.9 | 128.3 | 153.4 |

(continued)

| | | |
|---|---|---|
| 47.1 | 130.3 | 155.1 |
| 52.0 | 131.4 | 155.6 |
| 54.2 | 134.1 | 156.7 |

Other Crystalline Forms of Example 1: Form P22C: prepared by heating Form HI.5-1 ({drug6b}) at 60°C for 2 hours or at 75°C for 5 minutes. Water activity studies between Form HI.5-1 ({drug6b}) and Form P22C showed that Form HI.5-1 ({drug6b}) is more stable at > 23% relative humidity. Form P33 ({drug6c}): Form HI.5-1 ({drug6b}) converted to Form P33 ({drug6c}) between 50°C and 75°C in variable temperature PXRD experiment. Also observed after Form HI.5-1 ({drug6b}) was heated to 105°C for 5 minutes, or prepared by slurring dry powder Form HI.5-1 ({drug6b}) in dry EtOH or IP Ac. Elemental analysis indicated that Form P33 ({drug6c}) is a hemisulfate monohydrate. Solid state NMR indicated that Form P33 ({drug6c}) was a single phase. Water activity studies between Form HI.5-1 ({drug6b}) and Form P33 ({drug6c}) showed that Form HI.5-1 ({drug6b}) is more stable at > 23% relative humidity. Form P35: Prepared from slurry of Form HI.5-1 ({drug6b}) in dry MeOH under molecular sieves (7% RH). Converted to Form P33 ({drug6c}) when dried at 60°C. Stability in Water Slurry: An aqueous slurry of Example 1 was prepared and stored at room temperature. After two days, there was no significant chemical degradation; and no change in the PXRD pattern, which indicated that the crystalline Form HI.5-1 ({drug6b}) was stable in the aqueous slurry. No significant changes were observed in the thermogravimetric scan and the differential scanning calorimetry scan, and in the PXRD. The hemisulfate salt of Compound (VI) has been compared to other salts of Compound (VI) and has been found to be especially advantageous. The hemisulfate salt of Compound (VI) has the surprising advantage of providing a salt that is physically stable and chemically stable compared to other salts of Compound (VI). Further, the hemisulfate salt had the surprising advantage of being provided in a stable crystalline form, Form HI.5-1 ({drug6b}). For example, the hemisulfate salt of Compound (VI) was reproducibly prepared as a crystalline form, had low hygroscopicity, and did not readily change crystalline form or hydration state in response to changes in relative humidity and/or temperature. In contrast, the citric acid salt, fumaric acid salt, hydrochloric acid salt, methanesulfonic acid salt, phosphoric acid salt, and L-tartaric acid salt were hygroscopic at ambient temperature and relative humidity conditions, resulting is weight changes, hydration state changes, and/or phase changes. Crystalline salt formation was not observed for Compound (VI) in the presence of acetic acid, benzoic acid, benzenesulfonic acid, L-lactic acid, maleic acid, L-malic acid, and succinic acid in the high throughput salt screening. Further, the preparation of the hemisulfate salt did not require use of an expensive material such as D-tartaric acid.

Biological Methods: In vitro pharmacology: Tissue Culture. SK-N-MC cells were grown at 37°C in 5% CO2 as a monolayer in medium consisting of MEM with Earle's salts and L-glutamine (Invitrogen) supplemented with 10% fetal bovine serum (Invitrogen). Membrane Preparation: Crude membranes were prepared from SK-N-MC cells expressing CGRP receptors. The cells were rinsed twice with phosphate-buffered saline (155 mM aCl, 3.3 mM a2HP04, 1.1 mM KHYDROGENP04, pH 7.4), and incubated for 5-10 min. at 4°C in hypotonic lysis buffer consisting of 10 mM Tris (pH 7.4) and 5 mM EDTA. The cells were transferred from plates to polypropylene tubes (16 x 100 mm) and homogenized using a polytron. Homogenates were centrifuged at 32,000 x g for 30 min. The pellets were resuspended in cold hypotonic lysis buffer with 0.1 % mammalian protease inhibitor cocktail (Sigma) and assayed for protein concentration. The SK-N-MC homogenate was aliquoted and stored at -80°C. Radioligand Binding Assay: Compound (VI) was solubilized and carried through serial dilutions using 100% DMSO. Aliquots from the compound serial dilutions were further diluted 25 fold into assay buffer (50 mM Tris-Cl pH 7.5, 5 mM MgCl2, 0.005% Triton X-100) and transferred (volume 50 μl) into 96 well assay plates. [125I]-CGRP (GE Healthcare or Perkin-Elmer) was diluted to 72 pM in assay buffer and a volume of 50 μl was added to each well. SK-N-MC membranes were thawed, diluted in assay buffer with fresh 0.1% mammalian protease inhibitor cocktail (Sigma), and re-homogenized. SK-N-MC homogenate (7 μg/well) was added in a volume of 100 μl. The assay plates were then incubated at room temperature for 2 h. Assays were stopped by addition of excess cold wash buffer (50 mM Tris-Cl pH 7.5, 0.1% BSA) immediately followed by filtration over glass fiber filters (Whatman GF/B) previously soaked in 0.5% PEL Non-specific binding was defined with 1 μM beta-CGRP (Bachem). Protein bound radioactivity was determined using a gamma or scintillation counter. The resulting data was analyzed using a four parameter competitive binding equation (XLfit v2.0) and the IC50 was defined as the concentration of Compound (VI) required to displace 50% of radioligand binding. Final assay concentration of [125I]-CGRP was 18 pM. The mean Kd for [125I]-CGRP is 25.4 pM. Compound (VI) was evaluated in at least two separate experiments. In this study, the Human CGRP Receptor IC50 value of Compound (VI) was 0.04 nM.

In Vivo Pharmacokinetic Studies: An in vivo study was conducted comparing the pharmacokinetics of the free base Compound (VI) in humans pretreated with 40 mg of famotidine with non-pretreated humans. Compound (VI) in human EDTA plasma was analyzed using liquid-liquid extraction with uHPLC-MS/MS detection on a Triple Quad 5500 mass spectrometer. The method utilized stable isotope labeled [13C2, D4]-Compound (VI) as the internal standard.

After the addition of 50 μl, of 100 ng/mL [13C2, D4]-Compound (VI) in MeOH:water (20/80) and of 50 μl, 1M NH40Ac containing 4% acetic acid buffer solution to 0.100 mL of each study sample, quality control (QC) sample, and calibration standard, the samples were extracted with 600 μï^ methyl tert-butyl ether (MTBE) by shaking for 15 min. A 450 μï^ portion of the organic layer was removed and evaporated to dryness. The residue was reconstituted in 200 μl^ of the reconstitution solution (30% acetonitrile in 10 mM NH40Ac with 0.01% acetic acid). All liquid transfer steps were performed using a Perkin Elmer JANUS Mini® liquid handler except for the addition of the internal standard solution. A 10 μï^ aliquot of the extracted sample was injected to uHPLC-MS/MS system. The uHPLC was performed on a LEAP 4X Ultra uHPLC system with LEAP HTC PAL autosampler. Mobile phase A contained 10 mM NH40Ac and 0.01% acetic acid in ACN/water (10:90), and mobile phase B contained 10 mM NH40Ac and 0.01% acetic acid in ACN/water (90: 10). Chromatographic separation was achieved on an Acquity® uHPLC BEH C18 column (1.7 μιη. 2.1 x 50 mm) with an isocratic elution from 0 -1.5 min consisted of 28% Mobile Phase B for analysis of Compound (VI), then with a gradient elution consisted of a linear increase from 28% B to 100% B in 0.1 min, then maintaining it at 100% B for 1.1 min for washing out the column. The gradient was then returned to 28% B within 0.1 min, and maintained at 28% for 0.9 min with a total run time of 3.7 min. The flow rate was 0.6 mL/min and column temperature was maintained at 60°C condition. Detection was accomplished using an AB Sciex Triple Quad 5500 mass spectrometer in positive ESI with turbo ion spray ionization and using multiple reaction monitoring (MRM) mode. The MRM transitions were m/z 535→256 for Compound (VI) and m/z 541→256 for [13C2, D4]-Compound (VI). Data acquisition and quantiation were performed using AB Sciex Analyst® 1.5.1 software. The standard curve, which ranged from 0.500 - 500 ng/ml for Compound (VI), was fitted to a 1/x2 weighted linear regression model. During sample analysis, four levels of analytical quality control (QC) samples representing low, geometric-mean, medium, and high concentrations of Compound (VI) prepared in human EDTA plasma were analyzed in 4 replicates at each concentration level for each analytical run. The results from these QC samples were used to accept or reject the analytical runs containing study samples based on the acceptance criteria established a priori for the analysis of Compound (VI) in human EDTA plasma. The results of this study are show in Table 7 and Figure 8 of WO2013/130402. A significant reduction in AUC and Cmax of Compound (VI) was observed in humans pretreated with 40 mg of famotidine compared to non-pretreated humans.

Table 7:

| Dose | 150 mg Compound (I) | 150 mg Compound (I) with 40 mg Famotidine |
|---|---|---|
| $C_{max}$ (ng/mL) | 991 (85%) | 259 (35%) |
| $T_{max}$ Median | 2.5 | 3 |
| (min-max) | (0.75-3.0) | (2.0-4.0) |
| AUC inf (ng*hr/mL) | 7197 (75%) | 3058 (27%) |
| Half Life (hr) | 12.04 (29%) | 12.6 (36%) |
| Cl/F (L/hr) | 20.84 (75%) | 49 (28%) |
| F ($AUC_{inf}$) | - | 42.5% (96%) |
| F ($C_{max}$) | - | 26.2% (130%) |

An in vivo study was conducted comparing the pharmacokinetics of the free base Compound (VI) and the hemisulfate salt of Compound (VI) in dogs pretreated with famotidine or pentagastrin. Capsules were prepared containing either 150 mg of Compound (VI) as the free base or as the hemisulfate salt of Compound (VI):1. Compound (VI) free base capsules: 50 wt. % Compound (VI), 42 wt. % microcrystalline cellulose, 3 wt. % croscarmellose sodium, 4 wt. % Klucel EXF hydroxypropylcellulose, 0.5 wt. % magnesium stearate, 0.5 wt. % colloidal silicon dioxide; 2. Compound (VI) hemisulfate salt capsules: 57% Example 1 (hemisulfate salt of Compound (VI), in crystalline form HI.5-1), 40% microcrystalline cellulose, 3% Croscarmellose sodium. Four male dogs (10 kg) were treated according to the following three treatment protocols : Treatment 1 : pretreatment with pentagastrin (6 μg/kg, IP) several hours prior to the oral administration of Compound (VI) free base capsule. Treatment 2: pretreatment with 40 mg famotidine orally, three hours prior to the oral administration of Compound (VI) free base capsule. Treatment 3: pretreatment with 40 mg famotidine orally, three hours prior to the oral administration of Compound (VI) hemisulfate salt capsule. Blood samples were collected at 0, 0.5, 1, 2, 4, 8, and 24 hours after administration of the Compound (VI) free base capsule or Compound (VI) hemisulfate salt capsules and stored in EDTA tubes. Compound (VI) in dog EDTA plasma was analyzed using liquid-liquid extraction and uHPLC - MS/MS detection on a Triple Quad 5500 mass spectrometer. An aliquot of 0.050 mL of dog EDTA plasma was used for the assay. The method utilized stable isotope labeled [13C2, D4]-Compound (VI) as the internal standard. After the addition of 50 μl, of 200 ng/mL [C2, D4]-Compound (VI) in MeOH:water (20/80) and of 50 μl. 1M NH40Ac

containing 4% acetic acid buffer solution to 0.050 mL of each study sample, quality control (QC) sample, and calibration standard, the samples were extracted with 600 μï^ methyl tert-butyl ether (MTBE) by shaking for 15 min. A 450 μï^ portion of the organic layer was removed and evaporated to dryness. The residue was reconstituted in 300 μl, of the reconstitution solution (30% acetonitrile in 10 mM NH40Ac with 0.01% acetic acid). All liquid transfer steps were performed using a Perkin Elmer JANUS Mini® liquid handler except for the addition of the internal standard solution. A 5 μï^ aliquot of the extracted sample was injected to uHPLC-MS/MS system. The uHPLC was performed on a LEAP 4X Ultra uHPLC system with LEAP HTC PAL autosampler. Mobile phase A contained 10 mM NH4OAc and 0.01% acetic acid in ACN/water (10:90), and mobile phase B contained 10 mM NH4OAc and 0.01% acetic acid in ACN/water (90: 10). Chromatographic separation was achieved on an Acquity® uHPLC BEH C18 column (1.7 μiη, 2.1 x 50 mm) with an isocratic elution from 0 -1.5 min consisted of 28% Mobile Phase B for analysis of Compound (VI), then with a gradient elution consisted of a linear increase from 28% B to 100% B in 0.1 min, then maintaining it at 100% B for 1.1 min for washing out the column. The gradient was then returned to 28% B within 0.1 min, and maintained at 28% for 0.9 min with a total run time of 3.7 min. The flow rate was 0.6 mL/min and column temperature was maintained at 60°C condition. Detection was accomplished using an AB Sciex Triple Quad 5500 mass spectrometer in positive ESI with turbo ion spray ionization and using multiple reaction monitoring (MRM) mode. The MRM transitions were m/z 535→256 for Compound (VI) and m/z 541→256 for [13C2, D4]-Compound (VI). Data acquisition and quantification were performed using AB Sciex Analyst® 1.5.1 software. The standard curve, which ranged from 3.00 to 3000 ng/mL for Compound (VI), was fitted to a1/x2 weighted linear regression model. During sample analysis, four levels of analytical quality control (QC) samples representing low, geometric-mean, medium, and high concentrations of Compound (VI) prepared in dog EDTA plasma were analyzed in 4 replicates at each concentration level for each analytical run. The results from these QC samples were used to accept or reject the analytical runs containing study samples based on the acceptance criteria established a priori for the analysis of Compound (VI) in dog EDTA plasma. The results of this study are show in Table 8 and Figure 9 of WO2013/130402. A significant reduction in AUC and Cmax was observed in famotidine pre-treated dogs (high stomach pH) after treatment with the free base Compound (VI) compared to pentagastrin pre-treated dogs (low stomach pH). Dosing of Example 1, the hemisulfate sesquihydrate salt of Compound (VI), in famotidine pre-treated dogs showed a much lower reduction in AUC and Cmax. In this particular study, the hemisulfate salt of Compound (VI) provided a Cmax value of 2596 ng/mL, an AUCo-24hr of 12473 ng-h/mL, and 34.73 % bioavailability when administered after pretreatment with famotidine. In contrast, in a similar test, Compound (VI) free base provided a Cmax value of 245 ng/mL, an AUCo-24hr of 1762 ng-h/mL, and 4.54 % bioavailability when administered after pretreatment with famotidine.

Table 8: Pharmacokinetic Parameters for Dosing in Dogs of 150 mg of Compound (VI) as Hemisulfate salt or Free base:

| | $C_{max}$ (ng/mL) | | $T_{max}$ (h) | $AUC_{0-24hr}$ (ng·h/mL) | | BA (%) | | CV (%) |
|---|---|---|---|---|---|---|---|---|
| | Mean | Std Dev | | Mean | Std Dev | Mean | Std Dev | |
| Compound (T) + pentagastrin | 8156 | 4423 | 0.75 | 38796 | 15407 | 100 | - | 39.71 |
| Compound (T) + famotidine | 245 | 95 | 2 | 1762 | 392 | 4.54 | 1.01 | 22.23 |
| Example 1 + famotidine | 2596 | 409 | 1 | 13473 | 2098 | 34.73 | 5.41 | 15.57 |

The hemisulfate salt of Compound (VI) has been compared to Compound (VI) free base and has been found to be especially advantageous. The hemisulfate salt of Compound (VI) has the surprising advantage of reducing the variability in the bioavailability of Compound (VI) and/or increasing the bioavailability of Compound (VI) to the patient. Based on these in vitro and in vivo data, it is expected that the hemisulfate will provide a significant advantage of consistency in bioavailability among patients over the free form. To illustrate, the hemisulfate form provides surprisingly enhanced bioavailability in a patient population that is dosed with medicines that can raise the pH of stomach acids, such as antacids, proton pump inhibitors, or H2-receptor antagonists. Single Crystal Data (LVL): Data were collected on a Bruker-Nonius CAD4 serial diffractometer. Unit cell parameters were obtained through least-squares analysis of the experimental diffractometer settings of 25 high-angle reflections. Intensities were measured using Cu Ka radiation (λ = 1.5418 A) at a constant temperature with the Θ-2Θ variable scan technique and were corrected only for Lorentz-polarization factors. Background counts were collected at the extremes of the scan for half of the time of the scan. Alternately, single crystal data were collected on a Bruker-Nonius Kappa CCD 2000 system using Cu Ka radiation (λ = 1.5418 A). Indexing and processing of the measured intensity data were carried out with the HKL2000 software package (Otwinowski, Z & Minor, W. (1997) in Macromolecular Crystallography, eds. Carter, W.C. Jr. & Sweet, R.M. (Academic, NY), Vol. 276, pp307-326) in the Collect program suite. (Collect Data collection and processing user interface: Collect: Data collection software, R. Hooft, Nonius B.V., 1998). Alternately, single crystal data were collected on a Bruker-AXS APEX2 CCD system using Cu Ka radiation (λ = 1.5418 A). Indexing and processing of the measured intensity data were carried out with the APEX2 software package/program suite (APEX2 Data collection and processing user interface:

APEX2 User Manual, v1.27; BRUKER AXS, inc., Madison, WI). When indicated, crystals were cooled in the cold stream of an Oxford cryo system (Oxford Cryosystems Cryostream cooler: J. Cosier and A.M. Glazer, J. Appl. Cryst, 1986, 19, 105) during data collection. The structures were solved by direct methods and refined on the basis of observed reflections using either the SDP software package (SDP, Structure Determination Package, Enraf-Nonius, Bohemia NY) with minor local modifications or the crystallographic packages MAXUS (maXus solution and refinement software suite: S. Mackay, C.J. Gilmore, C. Edwards, M. Tremayne, N. Stewart, K. Shankland. maXus: a computer program for the solution and refinement of crystal structures from diffraction data) or SHELXTL (Sheldrick, GM. 1997, SHELXTL. Structure Determination Programs. Version 5.10 or greater, Bruker AXS, Madison, Wisconsin). The derived atomic parameters (coordinates and temperature factors) were refined through full matrix least-squares. The function minimized in the refinements was∑w(|F0| - |FC|)2- R is defined as∑ ‖F0| - |Fc‖/∑ |F0| while Rw = [∑w(|F0| - |FC|)V∑W |F0|] where w is an appropriate weighting function based on errors in the observed intensities. Difference maps were examined at all stages of refinement. Hydrogens were introduced in idealized positions with isotropic temperature factors, but no hydrogen parameters were varied. Single Crystal Data (WFD): A Bruker SMART 2K CCD diffractometer equipped with graphite-monochromated Cu Ka radiation, ($\lambda$ = 1.54056 A) was used to collect diffraction data at the room temperature. A full data set was collected using the $\omega$ scan mode over the 20 range with a crystal-to-detector distance of 4.98 cm. An empirical absorption correction utilized the SADABS routine associated with the diffractometer (Bruker AXS. 1998, SMART and SATNTPLUS. Area Detector Control and Integration Software, Bruker AXS, Madison, Wisconsin, USA). The final unit cell parameters were determined using the entire data set. All structures were solved by direct methods and refined by the full-matrix least-squares techniques, using the SHELXTL software package (Sheldrick, GM. 1997, SHELXTL. Structure Determination Programs. Version 5.10, Bruker AXS, Madison, Wisconsin, USA.). The function minimized in the refinements was∑W(|F0| -|FC|)2. R is defined as∑ ‖F0| - |Fc‖/∑ |F0| while Rw = [∑w(|F0| - |FC|)2/∑W |F0|2]1/2, where w is an appropriate weighting function based on errors in the observed intensities. Difference Fourier maps were examined at all stages of refinement. All non-hydrogen atoms were refined with anisotropic thermal displacement parameters. The hydrogen atoms associated with hydrogen bonding were located in the final difference Fourier maps while the positions of the other hydrogen atoms were calculated from an idealized geometry with standard bond lengths and angles. They were assigned isotropic temperature factors and included in structure factor calculations with fixed parameters. PXRD (Philips): About 200 mg were packed into a Philips powder X-ray diffraction (PXRD) sample holder. The sample was tranferred to a Philips MPD unit (45 KV, 40 mA, Cu Ka). Data were collected at room temperature in the 2 to 32 2-theta range (continuous scanning mode, scanning rate 0.03 degrees/sec, auto divergence and anti scatter slits, receiving slit: 0.2 mm, sample spinner : ON) PXRD (GADDS-NB). X-ray powder diffraction (PXRD) data were obtained using a Bruker C2 GADDS. The radiation was Cu Ka (40 KV, 40mA). The sample-detector distance was 15 cm. Powder samples were placed in sealed glass capillaries of 1mm or less in diameter; the capillary was rotated during data collection. Data were collected for $3<2\Theta<35°$ with a sample exposure time of at least 1000 seconds. The resulting two-dimensional diffraction arcs were integrated to create a traditional 1 -dimensional PXRD pattern with a step size of 0.02 degrees 20 in the range of 3 to 35 degrees 20. DSC (open pan): Differential scanning calorimetry (DSC) experiments were performed in a TA Instruments™ model Q2000, Q1000 or 2920. The sample (about 2-6 mg) was weighed in an aluminum pan and recorded accurately recorded to a hundredth of a milligram, and transferred to the DSC. The instrument was purged with nitrogen gas at 50mL/min. Data were collected between room temperature and 300°C at 10°C/min heating rate. The plot was made with the endothermic peaks pointing down. TGA (open pan): Thermal gravimetric analysis (TGA) experiments were performed in a TA Instruments™ model Q500 or 2950. The sample (about 10-30 mg) was placed in a platinum pan previously tared. The weight of the sample was measured accurately and recorded to a thousand of a milligram by the instrument. The furnace was purged with nitrogen gas at 1OOmL/min. Data were collected between room temperature and 300°C at 10°C/min heating rate. Solid-State Nuclear Magnetic Resonance (SSNMR): All solid-state C-13 NMR measurements were made with a Bruker AV-400, 400 MHz NMR spectrometer. High resolution spectra were obtained using high-power proton decoupling and the TPPM pulse sequence and ramp amplitude cross-polarization (RAMP-CP) with magic-angle spinning (MAS) at approximately 12 kHz (A.E. Bennett et al, J. Chem. Phys., 1995, 103, 6951),(G. Metz, X. Wu and S.O. Smith, J. Magn. Reson. A,. 1994, 110, 219-227). Approximately 70 mg of sample, packed into a canister-design zirconia rotor was used for each experiment. Chemical shifts ($\delta$) were referenced to external adamantane with the high frequency resonance being set to 38.56 ppm (W.L. Earl and D.L. VanderHart, J. Magn. Reson., 1982, 48, 35-54). VTI (dry on): Moisture sorption isotherms were collected in a VTI SGA-100 Symmetric Vapor Analyzer using approximately 10 mg of sample. The sample was dried at 60°C until the loss rate of 0.0005 wt %/min was obtained for 10 minutes. The sample was tested at 25°C and 3 or 4, 5, 15, 25, 35, 45, 50, 65, 75, 85, and 95% RH. Equilibration at each RH was reached when the rate of 0.0003 wt%/min for 35 minutes was achieved or a maximum of 600 minutes. It will be evident to one skilled in the art that the present disclosure is not limited to the foregoing illustrative examples, and that it can be embodied in other specific forms without departing from the essential attributes thereof. It is therefore desired that the examples be considered in all respects as illustrative and not restrictive, reference being made to the appended claims, rather than to the foregoing examples, and all changes which come within the meaning and range of equivalency of the claims are therefore intended to be embraced therein.

Very preferred embidoments are the claims of WO2013/130402 that are repeated as items 1 to 10 hereinafter:

1. A hemisulfate salt of Compound (VI):

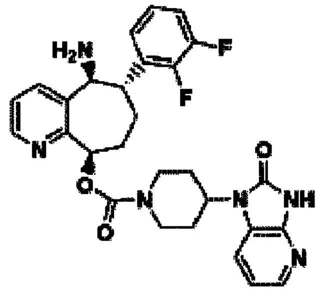

2. The hemisulfate salt of Compound (VI) according to item 1, wherein said Compound (VI) is crystalline.
3. The hemisulfate salt of Compound (VI) according to item 1, wherein said salt Compound (VI) is a sesquihydrate.
4. The hemisulfate salt of Compound (VI) according to item 3, wherein said salt comprises crystalline Form HI.5-1 ({drug6b}).
5. The hemisulfate salt of Compound (VI) according to item 4, wherein said Form HL5-1 ({drug6b}) is characterized by one or more of the following:

a) unit cell parameters substantially equal to the following: Cell dimensions: a = 10.92 A, b 33.04 A, c 7.90 A, a 90 degrees, β 90 degrees, y 90 degrees, Space group: P2i2i2, Molecules of Compound (VI)/asymmetric unit: 1, Volume = 2851 A3, Density (calculated) = 1.423 g/cm3, wherein measurement of said crystalline form is at a temperature of about 25°C;
b) an observed powder x-ray diffraction pattern substantially in accordance with the pattern shown in Figure 1 of WO2013/130402;
c) a simulated powder x-ray diffraction pattern substantially in accordance with the pattern shown in Figure 1 of WO2013/130402;
d) a powder x-ray diffraction pattern (CuKa λ=1.5418A) comprising four or more 20 values selected from: 5.4±0.1, 8.6±0.1, 9.7±0.1, 12.4±0.1, 14.9±0.1, 17.6±0.1, 18.1±0.1, 20.5±0.1, 21.4±0.1, and 22.0±0.1, wherein measurement of the crystalline form is at a temperature of about 25°C; and/or
e) a solid state nuclear resonance spectra comprising six or more peaks (δ (ppm) referenced to TMS) selected from: 26.6±0.1, 27.1±0.1, 28.3±0.1, 30.7±0.1, 43.1±0.1, 45.9±0.1, 47.1±0.1, 52.0±0.1, 54.2±0.1, 72.5±0.1, 117.0±0.1, 117.7±0.1, 124.2±0.1, 125.2±0.1, 128.3±0.1, 130.3±0.1, 131.4±0.1, 134.1±0.1, 140.8±0.1, 144.7±0.1, 148.7±0.1, 149.8±0.1, 151.2±0.1, 153.4±0.1, 155.1±0.1, 155.6±0.1, and 156.7±0.1.

6. A pharmaceutical composition comprising said hemisulfate salt of Compound (VI) according to item 1 ; and a pharmaceutically acceptable carrier of diluent.
7. The pharmaceutical composition according to item 6, wherein said hemisulfate salt of Compound (VI) is a sesquihydrate.
8. The pharmaceutical composition according to item 7, wherein said hemisulfate salt of Compound (VI) comprises crystalline Form HI.5-1 ({drug6b}).
9. The use of the hemisulfate salt of Compound (VI) according to item 1 in the manufacture of a medicament for the treatment of a CGRP related disorder.
10. The use according to item 9, wherein said disorder is migraine headaches, neurogenic vasodilation, neurogenic inflammation, thermal injury, circulatory shock, flushing associated with menopause, airway inflammatory diseases, or chronic obstructive pulmonary disease (COPD).

The invention relates to an administration unit comprising crystalline form of a hemisulfate salt of compound (VI). The administration unit according to the invention is useful for treating various diseases and disorders which include but are not limited to migraine headaches, neurogenic vasodilation, neurogenic inflammation, thermal injury, circulatory shock, flushing associated with menopause, airway inflammatory diseases, or chronic obstructive pulmonary disease (COPD).

SPECIFIC INORMATION CONCERNING ASPECT (VII) OF THE INVENTION

Aspect (vii) of the invention relates to an administration unit comprising crystalline solvates form of N-[2,4-bis(1,1-dimethylethyl)-5-hydroxyphenyl]-1,4-dihydro-4-oxoquinoline-3-carboxamide. The invention relates to a solid form of N-[2,4-bis(1,1-dimethylethyl)-5-hydroxyphenyl]-1,4-dihydro-4-oxoquinoline-3-carboxamide, namely to crystalline solvates form of N-[2,4-bis(1,1-dimethylethyl)-5-hydroxyphenyl]-1,4-dihydro-4-oxoquinoline-3-carboxamide ({drug7a}), which is useful for treating cystic fibrosis, hereditary emphysema, COPD, or dry-eye disease. Crystalline solvates form of N-[2,4-bis(1,1-dimethylethyl)-5-hydroxyphenyl]-1,4-dihydro-4-oxoquinoline-3-carboxamideis described in WO2013/158121, which is incorporated by reference. Crystalline solvates form of N-[2,4-bis(1,1-dimethylethyl)-5-hydroxyphenyl]-1,4-dihydro-4-oxoquinoline-3-carboxamidecan be selected from Forms D, E, F, G, H, I, J, K, L, M, N, O, P, Q, R, S, T, W, or Hydrate B, respectively. N-[2,4-bis(1,1-dimethylethyl)-5-hydroxyphenyl]-1,4-dihydro-4-oxoquinoline-3-carboxamidecan ({drug7}) be described (cf. WO2013/15812 by Compound (VII):

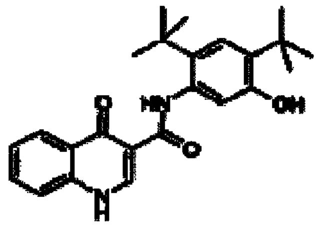

(VII)

As used herein, crystalline solvates form of N-[2,4-bis(1,1-dimethylethyl)-5-hydroxyphenyl]-1,4-dihydro-4-oxoquinoline-3-carboxamideis a solid form of N-[2,4-bis(1,1-dimethylethyl)-5-hydroxyphenyl]-1,4-dihydro-4-oxoquinoline-3-carboxamide characterized by / obtained preferred as described in WO2013/158121, which is cited hereinafter: The present invention relates to solid state forms, for example, crystalline solvate forms of N-[2,4-bis(1,1-dimethylethyl)-5-hydroxyphenyl]-1,4-dihydro-4-oxoquinoline-3-carboxamide (Compound (VII)), which is a modulator of cystic fibrosis transmembrane conductance regulator ("CFTR"). The invention also relates to pharmaceutical compositions including crystalline forms of N-(4-(7-azabicyclo[2.2.1]heptan-7-yl)-2-(trifluoromethyl)phenyl)-4-oxo-5-(trifluoromethyl)-1,4-dihydroquinoline-3-carboxamide, and methods therewith.

Cystic fibrosis (CF) is a recessive genetic disease that affects approximately 30,000 children and adults in the United States and approximately 30,000 children and adults in Europe. Despite progress in the treatment of CF, there is no cure. CF is caused by mutations in the cystic fibrosis transmembrane conductance regulator (CFTR) gene that encodes an epithelial chloride ion channel responsible for aiding in the regulation of salt and water absorption and secretion in various tissues. Small molecule drugs, known as potentiators that increase the probability of CFTR channel opening represent one potential therapeutic strategy to treat CF. Potentiators of this type are disclosed in WO 2006/002421, which is herein incorporated by reference in its entirety. Another potential therapeutic strategy involves small molecule drugs known as CF correctors that increase the number and function of CFTR channels. Correctors of this type are disclosed in WO 2005/075435, which are herein incorporated by reference in their entirety. Specifically, CFTR is a cAMP/ATP-mediated anion channel that is expressed in a variety of cells types, including absorptive and secretory epithelia cells, where it regulates anion flux across the membrane, as well as the activity of other ion channels and proteins. In epithelia cells, normal functioning of CFTR is critical for the maintenance of electrolyte transport throughout the body, including respiratory and digestive tissue. CFTR is composed of approximately 1480 amino acids that encode a protein made up of a tandem repeat of transmembrane domains, each containing six transmembrane helices and a nucleotide binding domain. The two transmembrane domains are linked by a large, polar, regulatory (R)-domain with multiple phosphorylation sites that regulate channel activity and cellular trafficking. The gene encoding CFTR has been identified and sequenced (See Gregory, R. J. et al. (1990) Nature 347:382-386; Rich, D. P. et al. (1990) Nature 347:358-362), (Riordan, J. R. et al. (1989) Science 245:1066-1073). A defect in this gene causes mutations in CFTR resulting in cystic fibrosis ("CF"), the most common fatal genetic disease in humans. Cystic fibrosis affects approximately one in every 2,500 infants in the United States. Within the general United States population, up to 10 million people carry a single copy of the defective gene without apparent ill effects. In contrast, individuals with two copies of the CF associated gene suffer from the debilitating and fatal effects of CF, including chronic lung disease. In patients with CF, mutations in CFTR endogenously expressed in respiratory epithelia leads to reduced apical anion secretion causing an imbalance in ion and fluid transport. The resulting decrease in anion transport contributes to enhanced mucus accumulation in the lung and the accompanying microbial infections that ultimately cause death in CF patients. In addition to respiratory disease, CF patients typically suffer from gastrointestinal problems and pancreatic insufficiency that, if left untreated, results in death. In addition, the majority of males with cystic fibrosis are infertile and fertility is decreased

among females with cystic fibrosis. In contrast to the severe effects of two copies of the CF associated gene, individuals with a single copy of the CF associated gene exhibit increased resistance to cholera and to dehydration resulting from diarrhea - perhaps explaining the relatively high frequency of the CF gene within the population. Sequence analysis of the CFTR gene of CF chromosomes has revealed a variety of disease causing mutations (Cutting, G. R. et al. (1990) Nature 346:366-369; Dean, M. et al. (1990) Cell 61:863:870; and erem, B-S. et al. (1989) Science 245:1073-1080; Kerem, B-S et al. (1990) Proc. Natl. Acad. Sci. USA 87:8447-8451). To date, greater than 1000 disease causing mutations in the CF gene have been identified (http://www.genet.sickkids.on.ca/cftr/app). The most prevalent mutation is a deletion of phenylalanine at position 508 of the CFTR amino acid sequence, and is commonly referred to as AF508-CFTR. This mutation occurs in approximately 70% of the cases of cystic fibrosis and is associated with a severe disease. The deletion of residue 508 in AF508-CFTR prevents the nascent protein from folding correctly. This results in the inability of the mutant protein to exit the ER, and traffic to the plasma membrane. As a result, the number of channels present in the membrane is far less than observed in cells expressing wild-type CFTR. In addition to impaired trafficking, the mutation results in defective channel gating. Together, the reduced number of channels in the membrane and the defective gating lead to reduced anion transport across epithelia leading to defective ion and fluid transport. (Quinton, P. M. (1990), FASEB J. 4: 2709-2727). Studies have shown, however, that the reduced numbers of AF508-CFTR in the membrane are functional, albeit less than wild-type CFTR. (Dalemans et al. (1991), Nature Lond. 354: 526-528; Denning et al., supra; Pasyk and Foskett (1995), J. Cell. Biochem. 270: 12347-50). In addition to AF508-CFTR, other disease causing mutations in CFTR that result in defective trafficking, synthesis, and/or channel gating could be up- or down-regulated to alter anion secretion and modify disease progression and/or severity. Although CFTR transports a variety of molecules in addition to anions, it is clear that this role (the transport of anions) represents one element in an important mechanism of transporting ions and water across the epithelium. The other elements include the epithelial Na+ channel, ENaC, Na+/2C17K+ co-transporter, Na+-K+-ATPase pump and the basolateral membrane K+ channels, that are responsible for the uptake of chloride into the cell. These elements work together to achieve directional transport across the epithelium via their selective expression and localization within the cell. Chloride absorption takes place by the coordinated activity of ENaC and CFTR present on the apical membrane and the Na+-K+-ATPase pump and Cl" ion channels expressed on the basolateral surface of the cell. Secondary active transport of chloride from the luminal side leads to the accumulation of intracellular chloride, which can then passively leave the cell via Cl" channels, resulting in a vectorial transport. Arrangement of Na+/2C17K+ co-transporter, Na+-K+- ATPase pump and the basolateral membrane K+ channels on the basolateral surface and CFTR on the luminal side coordinate the secretion of chloride via CFTR on the luminal side. Because water is probably never actively transported itself, its flow across epithelia depends on tiny transepithelial osmotic gradients generated by the bulk flow of sodium and chloride. As discussed above, it is believed that the deletion of residue 508 in AF508-CFTR prevents the nascent protein from folding correctly, resulting in the inability of this mutant protein to exit the ER, and traffic to the plasma membrane. As a result, insufficient amounts of the mature protein are present at the plasma membrane and chloride transport within epithelial tissues is significantly reduced. In fact, this cellular phenomenon of defective ER processing of ABC transporters by the ER machinery has been shown to be the underlying basis not only for CF disease, but for a wide range of other isolated and inherited diseases. N-[2,4-bis(1,1-dimethylethyl)-5-hydroxyphenyl]-1,4-dihydro-4-oxoquinoline-3-carboxamide (Compound (VII)) is a potent and selective CFTR potentiator of wild-type and mutant (including e.g., AF508, R1 17H, and G551D) forms of human CFTR. N-[2,4-bis(1,1-dimethylethyl)-5-hydroxyphenyl]-1,4-dihydro-4-oxoquinoline-3-carboxamide is useful for treatment of adult patients with cystic fibrosis and at least one G551D-CFTR allele. Accordingly, there is a need for stable bioavailable pharmaceutical compositions of N-[2,4-bis(1,1-dimethylethyl)-5-hydroxyphenyl]-1,4-dihydro-4-oxoquinoline-3-carboxamide useful for treating patients suffering from CF and methods of administering the same.

Solid forms of N-[2,4-bis(1,1-dimethylethyl)-5-hydroxyphenyl]-1,4-dihydro-4-oxoquinoline-3-carboxamide (Compound (VII)), which are crystalline solvates are described herein.

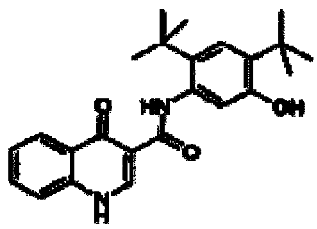

(VII).

The crystalline solvates forms of Compound (VII) (or "crystalline solvates"), disclosed herein are designated as Forms D, E, F, G, H, I, J, K, L, M, N, O, P, Q, R, S, T, W, or Hydrate B, respectively. The present invention is also directed to processes for making crystalline solvates of compound 1 designated as Forms D, E, F, G, H, I, J, K, L, M,

N, O, P, Q, R, S, T, W, or Hydrate B, respectively. The present invention is further directed to pharmaceutical compositions comprising a crystalline solvate of Compound (VII) selected from Forms D, E, F, G, H, I, J, K, L, M, N, O, P, Q, R, S, T, W, or Hydrate B, respectively, and a pharmaceutically acceptable carrier or excipient. The invention is further directed to therapeutic methods for treating CFTR-mediated diseases such as cystic fibrosis.

Figure 1 of WO2013/158121 is an XRPD pattern of Form XX of Compound (VII). Figure 2 of WO2013/158121 is an XRPD pattern of Form D ({drug7b}) of Compound (VII). Figure 3 of WO2013/158121 is a thermogravimetric trace of Form D ({drug7b}) of Compound (VII). Figure 4 of WO2013/158121 is an XRPD pattern of Form E ({drug7c}) of Compound (VII). Figure 5 of WO2013/158121 is a thermogravimetric trace of Form E ({drug7c}) of Compound (VII). Figure 6 of WO2013/158121 is an XRPD pattern of Form F ({drug7d}) of Compound (VII). Figure 7 of WO2013/158121 is a thermogravimetric trace of Form F ({drug7d}) of Compound (VII). Figure 8 of WO2013/158121 is an XRPD pattern of Form G ({drug7e}) of Compound (VII). Figure 9 of WO2013/158121 is a thermogravimetric trace of Form G ({drug7e}) of Compound (VII). Figure 10 of WO2013/158121 is an XRPD pattern of Form H ({drug7f}) of Compound (VII). Figure 11 of WO2013/158121 is a thermogravimetric trace of Form H({drug7f}) of Compound (VII). Figure 12 of WO2013/158121 is an XRPD pattern of Form I ({drug7g}) of Compound (VII). Figure 13 of WO2013/158121 is a thermogravimetric trace of Form I ({drug7g}) of Compound (VII). Figure 14 of WO2013/158121 is an XRPD pattern of Form J ({drug7h}) of Compound (VII). Figure 15 of WO2013/158121 is a thermogravimetric trace of Form J ({drug7h}) of Compound (VII). Figure 16 of WO2013/158121 is an XRPD pattern of Form K ({drug7i}) of Compound (VII). Figure 17 of WO2013/158121 is a thermogravimetric trace of Form K ({drug7i}) of Compound (VII). Figure 18 of WO2013/158121 is an XRPD pattern of Form L({drug7j}) of Compound (VII). Figure 19 of WO2013/158121 is a thermogravimetric trace of Form L ({drug7j}) of Compound (VII). Figure 20 of WO2013/158121 is an XRPD pattern of Form M ({drug7k}) of Compound (VII). Figure 21 of WO2013/158121 is a thermogravimetric trace of Form M ({drug7k}) of Compound (VII). Figure 22 of WO2013/158121 is an XRPD pattern of Form N ({drug71}) of Compound (VII). Figure 23 of WO2013/158121 is a thermogravimetric trace of Form N ({drug71}) of Compound (VII). Figure 24 of WO2013/158121 is an XRPD pattern of Form O ({drug7m}) of Compound (VII). Figure 25 of WO2013/158121 is a thermogravimetric trace of Form O ({drug7m}) of Compound (VII). Figure 26 of WO2013/158121 is an XRPD pattern of Form P ({drug7n}) of Compound (VII). Figure 27 of WO2013/158121 is a thermogravimetric trace of Form P ({drug7n}) of Compound (VII). Figure 28 of WO2013/158121 is an XRPD pattern of Form Q ({drug7o}) of Compound (VII). Figure 29 of WO2013/158121 is a thermogravimetric trace of Form Q ({drug7o}) of Compound (VII). Figure 30 of WO2013/158121 is an XRPD pattern of Form R ({drug7p}) of Compound (VII). Figure 31 of WO2013/158121 is a thermogravimetric trace of Form R ({drug7p}) of Compound (VII). Figure 32 of WO2013/158121 is an XRPD pattern of Form S ({drug7q}) of Compound (VII). Figure 33 of WO2013/158121 is a thermogravimetric trace of Form S ({drug7q}) of Compound (VII). Figure 34 of WO2013/158121 is an XRPD pattern of Form T ({drug7r}) of Compound (VII). Figure 35 of WO2013/158121 is a thermogravimetric trace of Form T ({drug7r}) of Compound (VII). Figure 36 of WO2013/158121 is an XRPD pattern of Hydrate B ({drug7t}) of Compound (VII). Figure 37 of WO2013/158121 is a thermogravimetric trace of Hydrate B ({drug7t}) of Compound (VII). Figure 38 of WO2013/158121 is an XRPD pattern of Form W ({drug7s}) of Compound (VII). Figure 39 of WO2013/158121 is a thermogravimetric trace of Form W ({drug7s}) of Compound (VII).

Definitions: As used herein, the following definitions shall apply unless otherwise indicated. The term "ABC-transporter" as used herein means an ABC-transporter protein or a fragment thereof comprising at least one binding domain, wherein said protein or fragment thereof is present in vivo or in vitro. The term "binding domain" as used herein means a domain on the ABC-transporter that can bind to a modulator. See, e.g., Hwang, T. C. et ah, J. Gen. Physiol. (1998): 777(3), 477-90. The term "CFTR" as used herein means cystic fibrosis transmembrane conductance regulator or a mutation thereof capable of regulator activity, including, but not limited to, AF508 CFTR, R1 17H CFTR, and G551D CFTR (see, e.g., http://www.genet.sickkids.on.ca/cftr/, for CFTR mutations). The term "modulating" as used herein means increasing or decreasing by a measurable amount. The term "treatment", as used herein, unless otherwise indicated, means the treatment or prevention of a CFTR related disorder as provided in the methods described herein, including curing, reducing the symptoms of or slowing the progress of said disorder. The terms "treat" and "treating" are defined in accord the foregoing term "treatment". The term "normal CFTR" or "normal CFTR function" as used herein means wild-type like CFTR without any impairment due to environmental factors such as smoking, pollution, or anything that produces inflammation in the lungs. The term "reduced CFTR" or "reduced CFTR function" as used herein means less than normal CFTR or less than normal CFTR function. The term "crystalline" refers to compounds or compositions where the structural units are arranged in fixed geometric patterns or lattices, so that crystalline solids have rigid long range order. The structural units that constitute the crystal structure can be atoms, molecules, or ions. Crystalline solids show definite melting points. "Solvate" as in the phrase "crystalline solvate" is a crystalline solid containing either stoichiometric or nonstoichiometric amounts of a solvent incorporated within the crystal structure. For example, if the incorporated solvent is water, the solvates are also commonly known as hydrates.

Crystalline Solvate Forms: The present invention is directed to crystalline solvates of N-[2,4-bis(1,1-dimethylethyl)-5-hydroxyphenyl]-1,4-dihydro-4-oxoquinoline-3-carboxamide (Compound (VII)) having the structural formula:

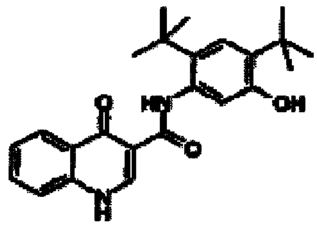

Compound (VII).

In one aspect, the invention includes a crystalline solvate of Compound (VII), wherein the crystalline solvate is designated as a solid form selected from the group consisting of Form D ({drug7b}), Form E ({drug7c}), Form F ({drug7d}), Form G ({drug7e}), Form H ({drug7f}), Form I ({drug7g}), Form J ({drug7h}), Form K ({drug7i}), Form L ({drug7j}), Form M ({drug7k}), Form N ({drug71}), Form O ({drug7m}), Form P ({drug7n}), Form Q ({drug7o}), Form R ({drug7p}), Form S ({drug7q}), Form T ({drug7r}), Form W ({drug7s}), and Hydrate B ({drug7t}).

In another aspect, the invention provides a crystalline solvate Form D ({drug7b}) of Compound (VII), which has an XRPD pattern as depicted in Figure 2 of WO2013/158121. This crystalline solvate form is an acetonitrile solvate, or an acetonitrile/water 75/25 solvate. In a further embodiment of this aspect, crystalline solvate Form D ({drug7b}) is characterized by one or more peaks selected from the group consisting of 5.6 ± 0.2 degrees, 6.0 ± 0.2 degrees, 7.8 ± 0.2 degrees, 13.5 ± 0.2 degrees, 14.1 ± 0.2 degrees, 14.7 ± 0.2 degrees, and 16.2 ± 0.2 degrees on a 2Θ scale in an X-ray powder diffraction pattern. In a further embodiment, the X-ray powder diffraction pattern is obtained using Cu K alpha radiation. In a further embodiment of this aspect, crystalline solvate Form D ({drug7b}) is characterized by a peak at 5.6 ± 0.2 degrees, a peak at 6.0 ± 0.2 degrees, a peak at 7.8 ± 0.2 degrees, a peak at 13.5 ± 0.2 degrees, a peak at 14.1 ± 0.2 degrees, a peak at 14.7 ± 0.2 degrees, and a peak at 16.2 ± 0.2 degrees on a 20 scale in an X-ray powder diffraction pattern. In a further embodiment, the X-ray powder diffraction pattern is obtained using Cu K alpha radiation. A thermogravimetric trace of Form D ({drug7b}) is provided as Figure 3 of WO2013/158121.

In another aspect, the invention provides a crystalline solvate Form E ({drug7c}) of Compound (VII), which has an XRPD pattern as depicted in Figure 4 of WO2013/158121. This crystalline solvate form is a methylethyl ketone (MEK) solvate, a MEK/water 99/1 solvate, MEK/water 90/10 solvate, or a MEK water 80/20 solvate. In a further embodiment of this aspect, crystalline solvate Form E ({drug7c}) is characterized by one or more peaks selected from the group consisting of 8.0 ± 0.2 degrees, 8.6 ± 0.2 degrees, 10.1 ± 0.2 degrees, 11.0 ± 0.2 degrees, 11.9 ± 0.2 degrees, 16.5 ± 0.2 degrees, 17.1 ± 0.2 degrees, and 18.1 ± 0.2 degrees on a 20 scale in an X-ray powder diffraction pattern. In a further embodiment, the X-ray powder diffraction pattern is obtained using Cu K alpha radiation. In a further embodiment of this aspect, crystalline solvate Form E ({drug7c}) is characterized by a peak at 8.0 ± 0.2 degrees, a peak at 8.6 ± 0.2 degrees, a peak at 10.1 ± 0.2 degrees, a peak at 11.0 ± 0.2 degrees, a peak at 11.9 ± 0.2 degrees, a peak at 16.5 ± 0.2 degrees, a peak at 17.1 ± 0.2 degrees, and a peak at 18.1 ± 0.2 degrees on a 20 scale in an X-ray powder diffraction pattern. In a further embodiment, the X-ray powder diffraction pattern is obtained using Cu K alpha radiation. In a further embodiment of this aspect, crystalline solvate Form E ({drug7c}) is characterized by one or more peaks selected from the group consisting of 6.8 ± 0.2 degrees, 8.0 ± 0.2 degrees, 8.6 ± 0.2 degrees, 10.1 ± 0.2 degrees, 10.7 ± 0.2 degrees, 11.0 ± 0.2 degrees, 11.9 ± 0.2 degrees, 12.9 ± 0.2 degrees, 14.0 ± 0.2 degrees, 15.9 ± 0.2 degrees, 16.5 ± 0.2 degrees, 17.1 ± 0.2 degrees, 18.1 ± 0.2 degrees, 20.2 ± 0.2 degrees, 21.1 ± 0.2 degrees, 23.8 ± 0.2 degrees, 24.3 ± 0.2 degrees, 24.7 ± 0.2 degrees, 25.5 ± 0.2 degrees, and 26.6 ± 0.2 degrees on a 20 scale in an X-ray powder diffraction pattern. In a further embodiment, the X-ray powder diffraction pattern is obtained using Cu K alpha radiation. In a further embodiment of this aspect, crystalline solvate Form E ({drug7c}) is characterized by a peak at 6.8 ± 0.2 degrees, a peak at 8.0 ± 0.2 degrees, a peak at 8.6 ± 0.2 degrees, a peak at 10.1 ± 0.2 degrees, a peak at 10.7 ± 0.2 degrees, a peak at 11.0 ± 0.2 degrees, a peak at 11.9 ± 0.2 degrees, a peak at 12.9 ± 0.2 degrees, a peak at 14.0 ± 0.2 degrees, a peak at 15.9 ± 0.2 degrees, a peak at 16.5 ± 0.2 degrees, a peak at 17.1 ± 0.2 degrees, a peak at 18.1 ± 0.2 degrees, a peak at 20.2 ± 0.2 degrees, a peak at 21.1 ± 0.2 degrees, a peak at 23.8 ± 0.2 degrees, a peak at 24.3 ± 0.2 degrees, a peak at 24.7 ± 0.2 degrees, a peak at 25.5 ± 0.2 degrees, and a peak at 26.6 ± 0.2 degrees on a 20 scale in an X-ray powder diffraction pattern. In a further embodiment, the X-ray powder diffraction pattern is obtained using Cu K alpha radiation. A thermogravimetric trace of Form E ({drug7c}) is provided as Figure 5 of WO2013/158121.

In another aspect, the invention provides a crystalline solvate Form F ({drug7d}) of Compound (VII), which has an XRPD pattern as depicted in Figure 6 of WO2013/158121. This crystalline solvate form is an acetonitrile/water 75/25 solvate. In one embodiment of this aspect, Form F ({drug7d}) is characterized by one or more peaks selected from the group consisting of 7.2 ± 0.2 degrees, 7.8 ± 0.2 degrees, 9.4 ± 0.2 degrees, 10.7 ± 0.2 degrees, 12.1 ± 0.2 degrees, 12.8 ± 0.2 degrees, 14.1 ± 0.2 degrees, and 14.7 ± 0.2 degrees on a 20 scale in an X-ray powder diffraction pattern. In a further embodiment, the X-ray powder diffraction pattern is obtained using Cu K alpha radiation. In one embodiment

of this aspect, Form F ({drug7d}) is characterized by a peak at 7.2 ± 0.2 degrees, a peak at 7.8 ± 0.2 degrees, a peak at 9.4 ± 0.2 degrees, a peak at 10.7 ± 0.2 degrees, a peak at 12.1 ± 0.2 degrees, a peak at 12.8 ± 0.2 degrees, a peak at 14.1 ± 0.2 degrees, and a peak at 14.7 ± 0.2 degrees on a 20 scale in an X-ray powder diffraction pattern. In a further embodiment, the X-ray powder diffraction pattern is obtained using Cu K alpha radiation. In one embodiment of this aspect, Form F ({drug7d}) is characterized by one or more peaks selected from the group consisting of 7.2 ± 0.2 degrees, 7.8 ± 0.2 degrees, 9.4 ± 0.2 degrees, 10.7 ± 0.2 degrees, 11.7 ± 0.2 degrees, 12.1 ± 0.2 degrees, 12.8 ± 0.2 degrees, 13.2 ± 0.2 degrees, 14.1 ± 0.2 degrees, 14.7 ± 0.2 degrees 15.6 ± 0.2 degrees, 17.0 ± 0.2 degrees, 18.3 ± 0.2 degrees, 18.9 ± 0.2 degrees, 20.0 ± 0.2 degrees, 20.6 ± 0.2 degrees, 21.3 ± 0.2 degrees, 25.5 ± 0.2 degrees, 26.0 ± 0.2 degrees, and 27.4 ± 0.2 degrees on a 20 scale in an X-ray powder diffraction pattern. In a further embodiment, the X-ray powder diffraction pattern is obtained using Cu K alpha radiation. In one embodiment of this aspect, Form F ({drug7d}) is characterized by a peak at 7.2 ± 0.2 degrees, a peak at 7.8 ± 0.2 degrees, a peak at 9.4 ± 0.2 degrees, a peak at 10.7 ± 0.2 degrees, a peak at 11.7 ± 0.2 degrees, a peak at 12.1 ± 0.2 degrees, a peak at 12.8 ± 0.2 degrees, a peak at 13.2 ± 0.2 degrees, a peak at 14.1 ± 0.2 degrees, a peak at 14.7 ± 0.2 degrees a peak at 15.6 ± 0.2 degrees, a peak at 17.0 ± 0.2 degrees, a peak at 18.3 ± 0.2 degrees, a peak at 18.9 ± 0.2 degrees, a peak at 20.0 ± 0.2 degrees, a peak at 20.6 ± 0.2 degrees, a peak at 21.3 ± 0.2 degrees, a peak at 25.5 ± 0.2 degrees, a peak at 26.0 ± 0.2 degrees, and a peak at 27.4 ± 0.2 degrees on a 20 scale in an X-ray powder diffraction pattern. In a further embodiment, the X-ray powder diffraction pattern is obtained using Cu K alpha radiation. A thermogravimetric trace of Form F ({drug7d}) is provided as Figure 7 of WO2013/158121.

In another aspect, the invention provides a crystalline solvate Form G ({drug7e}) of Compound (VII), which has an XRPD pattern as depicted in Figure 8 of WO2013/158121. This crystalline solvate form is an isopropyl acetate solvate. In one embodiment of this aspect, Form G ({drug7e}) is characterized by one or more peaks selected from the group consisting of 6.3 ± 0.2 degrees, 7.3 ± 0.2 degrees, and 12.4 ± 0.2 degrees on a 20 scale in an X-ray powder diffraction pattern. In a further embodiment, the X-ray powder diffraction pattern is obtained using Cu K alpha radiation. In one embodiment of this aspect, Form G ({drug7e}) is characterized by a peak at 6.3 ± 0.2 degrees, a peak at 7.3 ± 0.2 degrees, and a peak at 12.4 ± 0.2 degrees on a 20 scale in an X-ray powder diffraction pattern. In a further embodiment, the X-ray powder diffraction pattern is obtained using Cu K alpha radiation. A thermogravimetric trace of Form G ({drug7e}) is provided as Figure 9 of WO2013/158121.

In another aspect, the invention provides a crystalline solvate Form H ({drug7f}) of Compound (VII), which has an XRPD pattern as depicted in Figure 10 of WO2013/158121. This crystalline solvate form is an isopropyl acetate/water 95/5 solvate. In a further embodiment of this aspect, crystalline solvate Form H ({drug7f}) is characterized by one or more peaks selected from the group consisting of 7.9 ± 0.2 degrees, 9.9 ± 0.2 degrees, 10.4 ± 0.2 degrees, 11.6 ± 0.2 degrees, 12.1 ± 0.2 degrees, 14.8 ± 0.2 degrees, 16.0 ± 0.2 degrees, and 17.9 ± 0.2 degrees on a 20 scale in an X-ray powder diffraction pattern. In a further embodiment, the X-ray powder diffraction pattern is obtained using Cu K alpha radiation. In a further embodiment of this aspect, crystalline solvate Form H ({drug7f}) is characterized by a peak at 7.9 ± 0.2 degrees, a peak at 9.9 ± 0.2 degrees, a peak at 10.4 ± 0.2 degrees, a peak at II.6 ± 0.2 degrees, a peak at 12.1 ± 0.2 degrees, a peak at 14.8 ± 0.2 degrees, a peak at 16.0 ± 0.2 degrees, and a peak at 17.9 ± 0.2 degrees on a 20 scale in an X-ray powder diffraction pattern. In a further embodiment, the X-ray powder diffraction pattern is obtained using Cu K alpha radiation. In a further embodiment of this aspect, crystalline solvate Form H ({drug7f}) is characterized by one or more peaks selected from the group consisting of 5.9 ± 0.2 degrees, 7.9 ± 0.2 degrees, 9.9 ± 0.2 degrees, 10.4 ± 0.2 degrees, 10.9 ± 0.2 degrees, 11.6 ± 0.2 degrees, 12.1 ± 0.2 degrees, 13.2 ± 0.2 degrees, 13.8 ± 0.2 degrees, 14.8 ± 0.2 degrees, 16.0 ± 0.2 degrees, 17.4 ± 0.2 degrees, 17.9 ± 0.2 degrees, 19.6 ± 0.2 degrees, 20.6 ± 0.2 degrees, 21.6 ± 0.2 degrees, 22.5 ± 0.2 degrees, 23.8 ± 0.2 degrees, 24.8 ± 0.2 degrees, and 26.9 ± 0.2 degrees on a 20 scale in an X-ray powder diffraction pattern. In a further embodiment, the X-ray powder diffraction pattern is obtained using Cu K alpha radiation. In a further embodiment of this aspect, crystalline solvate Form H ({drug7f}) is characterized by a peak at 5.9 ± 0.2 degrees, a peak at 7.9 ± 0.2 degrees, a peak at 9.9 ± 0.2 degrees, a peak at 10.4 ± 0.2 degrees, a peak at 10.9 ± 0.2 degrees, a peak at 11.6 ± 0.2 degrees, a peak at 12.1 ± 0.2 degrees, a peak at 13.2 ± 0.2 degrees, a peak at 13.8 ± 0.2 degrees, a peak at 14.8 ± 0.2 degrees, a peak at 16.0 ± 0.2 degrees, a peak at 17.4 ± 0.2 degrees, a peak at 17.9 ± 0.2 degrees, a peak at 19.6 ± 0.2 degrees, a peak at 20.6 ± 0.2 degrees, a peak at 21.6 ± 0.2 degrees, a peak at 22.5 ± 0.2 degrees, a peak at 23.8 ± 0.2 degrees, a peak at 24.8 ± 0.2 degrees, and a peak at 26.9 ± 0.2 degrees on a 20 scale in an X-ray powder diffraction pattern. In a further embodiment, the X-ray powder diffraction pattern is obtained using Cu K alpha radiation. A thermogravimetric trace of Form H ({drug7f}) is provided as Figure 11 of WO2013/158121.

In another aspect, the invention provides a crystalline solvate Form I ({drug7g}) of Compound (VII), which has an XRPD pattern as depicted in Figure 12 of WO2013/158121. This crystalline solvate form is a MEK solvate. In a further embodiment of this aspect, crystalline solvate Form I ({drug7g}) is characterized by one or more peaks selected from the group consisting of 6.8 ± 0.2 degrees, 8.4 ± 0.2 degrees, 9.4 ± 0.2 degrees, 10.2 ± 0.2 degrees, 11.4 ± 0.2 degrees, 17.1 ± 0.2 degrees, 17.8 ± 0.2 degrees, and 18.3 ± 0.2 degrees on a 20 scale in an X-ray powder diffraction pattern. In a further embodiment, the X-ray powder diffraction pattern is obtained using Cu K alpha radiation. In a further

embodiment of this aspect, crystalline solvate Form I ({drug7g}) is characterized by a peak at 6.8 ± 0.2 degrees, a peak at 8.4 ± 0.2 degrees, a peak at 9.4 ± 0.2 degrees, a peak at 10.2 ± 0.2 degrees, a peak at 11.4 ± 0.2 degrees, a peak at 17.1 ± 0.2 degrees, a peak at 17.8 ± 0.2 degrees, and a peak at 18.3 ± 0.2 degrees on a 20 scale in an X-ray powder diffraction pattern. In a further embodiment, the X-ray powder diffraction pattern is obtained using Cu K alpha radiation. In a further embodiment of this aspect, crystalline solvate Form I ({drug7g}) is characterized by one or more peaks selected from the group consisting of 5.8 ± 0.2 degrees, 6.8 ± 0.2 degrees, 7.2 ± 0.2 degrees, 8.4 ± 0.2 degrees, 8.8 ± 0.2 degrees, 9.4 ± 0.2 degrees, 10.2 ± 0.2 degrees, 11.4 ± 0.2 degrees, 12.7 ± 0.2 degrees, 13.7 ± 0.2 degrees, 14.8 ± 0.2 degrees, 15.7 ± 0.2 degrees, 17.1 ± 0.2 degrees, 17.8 ± 0.2 degrees, 18.3 ± 0.2 degrees, 18.8 ± 0.2 degrees, 20.2 ± 0.2 degrees, 21.3 ± 0.2 degrees, 23.9 ± 0.2 degrees, and 25.2 ± 0.2 degrees, on a 20 scale in an X-ray powder diffraction pattern. In a further embodiment, the X-ray powder diffraction pattern is obtained using Cu K alpha radiation. In a further embodiment of this aspect, crystalline solvate Form I ({drug7g}) is characterized by a peak at 5.8 ± 0.2 degrees, a peak at 6.8 ± 0.2 degrees, a peak at 7.2 ± 0.2 degrees, a peak at 8.4 ± 0.2 degrees, a peak at 8.8 ± 0.2 degrees, a peak at 9.4 ± 0.2 degrees, a peak at 10.2 ± 0.2 degrees, a peak at 11.4 ± 0.2 degrees, a peak at 12.7 ± 0.2 degrees, a peak at 13.7 ± 0.2 degrees, a peak at 14.8 ± 0.2 degrees, a peak at 15.7 ± 0.2 degrees, a peak at 17.1 ± 0.2 degrees, a peak at 17.8 ± 0.2 degrees, a peak at 18.3 ± 0.2 degrees, a peak at 18.8 ± 0.2 degrees, a peak at 20.2 ± 0.2 degrees, a peak at 21.3 ± 0.2 degrees, a peak at 23.9 ± 0.2 degrees, and a peak at 25.2 ± 0.2 degrees, on a 20 scale in an X-ray powder diffraction pattern. In a further embodiment, the X-ray powder diffraction pattern is obtained using Cu K alpha radiation. A thermogravimetric trace of Form I ({drug7g}) is provided as Figure 13 of WO2013/158121.

In another aspect, the invention provides a crystalline solvate Form J ({drug7h}) of Compound (VII), which has an XRPD pattern as depicted in Figure 14 of WO2013/158121. This crystalline solvate form is a MEK/water 99/1 solvate. In one embodiment of this aspect, Form J ({drug7h}) is characterized by one or more peaks selected from the group consisting of 6.0 ± 0.2 degrees, 7.9 ± 0.2 degrees, 8.8 ± 0.2 degrees, 9.9 ± 0.2 degrees, 11.8 ± 0.2 degrees, 17.7 ± 0.2 degrees, 18.0 ± 0.2 degrees, and 18.5 ± 0.2 degrees on a 20 scale in an X-ray powder diffraction pattern. In a further embodiment, the X-ray powder diffraction pattern is obtained using Cu alpha radiation. In one embodiment of this aspect, Form J ({drug7h}) is characterized by a peak at 6.0 ± 0.2 degrees, a peak at 7.9 ± 0.2 degrees, a peak at 8.8 ± 0.2 degrees, a peak at 9.9 ± 0.2 degrees, a peak at 11.8 ± 0.2 degrees, a peak at 17.7 ± 0.2 degrees, a peak at 18.0 ± 0.2 degrees, and a peak at 18.5 ± 0.2 degrees on a 20 scale in an X-ray powder diffraction pattern. In a further embodiment, the X-ray powder diffraction pattern is obtained using Cu K alpha radiation. In one embodiment of this aspect, Form J ({drug7h}) is characterized by one or more peaks selected from the group consisting of 6.0 ± 0.2 degrees, 6.8 ± 0.2 degrees, 7.9 ± 0.2 degrees, 8.4 ± 0.2 degrees, 8.8 ± 0.2 degrees, 9.9 ± 0.2 degrees, 11.8 ± 0.2 degrees, 12.6 ± 0.2 degrees, 13.6 ± 0.2 degrees, 15.7 ± 0.2 degrees, 16.7 ± 0.2 degrees, 17.7 ± 0.2 degrees, 18.0 ± 0.2 degrees, 18.5 ± 0.2 degrees, 19.3 ± 0.2 degrees, 20.7 ± 0.2 degrees, 22.6 ± 0.2 degrees, 26.8 ± 0.2 degrees, and 29.4 ± 0.2 degrees on a 20 scale in an X-ray powder diffraction pattern. In a further embodiment, the X-ray powder diffraction pattern is obtained using Cu K alpha radiation. In one embodiment of this aspect, Form J ({drug7h}) is characterized by a peak at 6.0 ± 0.2 degrees, a peak at 6.8 ± 0.2 degrees, a peak at 7.9 ± 0.2 degrees, a peak at 8.4 ± 0.2 degrees, a peak at 8.8 ± 0.2 degrees, a peak at 9.9 ± 0.2 degrees, a peak at 11.8 ± 0.2 degrees, a peak at 12.6 ± 0.2 degrees, a peak at 13.6 ± 0.2 degrees, a peak at 15.7 ± 0.2 degrees, a peak at 16.7 ± 0.2 degrees, a peak at 17.7 ± 0.2 degrees, a peak at 18.0 ± 0.2 degrees, a peak at 18.5 ± 0.2 degrees, a peak at 19.3 ± 0.2 degrees, a peak at 20.7 ± 0.2 degrees, a peak at 22.6 ± 0.2 degrees, a peak at 26.8 ± 0.2 degrees, and a peak at 29.4 ± 0.2 degrees on a 20 scale in an X-ray powder diffraction pattern. In a further embodiment, the X-ray powder diffraction pattern is obtained using Cu K alpha radiation. A thermogravimetric trace of Form J ({drug7h}) is provided as Figure 15 of WO2013/158121.

In another aspect, the invention provides a crystalline solvate Form K ({drug7i}) of Compound (VII), which has an XRPD pattern as depicted in Figure 16 of WO2013/158121. This crystalline solvate form is a methylethyl ketone (MEK) solvate, a MEK/water 99/1 solvate, MEK/water 90/10 solvate, or a MEK water 80/20 solvate. In a further embodiment of this aspect, crystalline solvate Form K ({drug7i}) is characterized by one or more peaks selected from the group consisting of 8.0 ± 0.2 degrees, 8.5 ± 0.2 degrees, 10.0 ± 0.2 degrees, 11.8 ± 0.2 degrees, 15.4 ± 0.2 degrees, 15.9 ± 0.2 degrees, 16.9 ± 0.2 degrees, and 18.0 ± 0.2 degrees on a 20 scale in an X-ray powder diffraction pattern. In a further embodiment, the X-ray powder diffraction pattern is obtained using Cu K alpha radiation. In a further embodiment of this aspect, crystalline solvate Form K ({drug7i}) is characterized by a peak at 8.0 ± 0.2 degrees, a peak at 8.5 ± 0.2 degrees, a peak at 10.0 ± 0.2 degrees, a peak at I l.8 ± 0.2 degrees, a peak at 15.4 ± 0.2 degrees, a peak at 15.9 ± 0.2 degrees, a peak at 16.9 ± 0.2 degrees, and a peak at 18.0 ± 0.2 degrees on a 20 scale in an X-ray powder diffraction pattern. In a further embodiment, the X-ray powder diffraction pattern is obtained using Cu alpha radiation. In a further embodiment of this aspect, crystalline solvate Form K ({drug7i}) is characterized by one or more peaks selected from the group consisting of 8.0 ± 0.2 degrees, 8.5 ± 0.2 degrees, 10.0 ± 0.2 degrees, 11.8 ± 0.2 degrees, 12.3 ± 0.2 degrees, 14.2 ± 0.2 degrees, 14.8 ± 0.2 degrees, 15.4 ± 0.2 degrees, 15.9 ± 0.2 degrees, 16.9 ± 0.2 degrees, 18.0 ± 0.2 degrees, 18.5 ± 0.2 degrees, 19.8 ± 0.2 degrees, 20.2 ± 0.2 degrees, 20.7 ± 0.2 degrees, 21.0 ± 0.2 degrees,

21.5 ± 0.2 degrees, 22.7 ± 0.2 degrees, and 29.5 ± 0.2 degrees on a 2θ scale in an X-ray powder diffraction pattern. In a further embodiment, the X-ray powder diffraction pattern is obtained using Cu K alpha radiation. In a further embodiment of this aspect, crystalline solvate Form K ({drug7i}) is characterized by a peak at 8.0 ± 0.2 degrees, a peak at 8.5 ± 0.2 degrees, a peak at 10.0 ± 0.2 degrees, a peak at 11.8 ± 0.2 degrees, a peak at 12.3 ± 0.2 degrees, a peak at 14.2 ± 0.2 degrees, a peak at 14.8 ± 0.2 degrees, a peak at 15.4 ± 0.2 degrees, a peak at 15.9 ± 0.2 degrees, a peak at 16.9 ± 0.2 degrees, a peak at 18.0 ± 0.2 degrees, a peak at 18.5 ± 0.2 degrees, a peak at 19.8 ± 0.2 degrees, a peak at 20.2 ± 0.2 degrees, a peak at 20.7 ± 0.2 degrees, a peak at 21.0 ± 0.2 degrees, a peak at 21.5 ± 0.2 degrees, a peak at 22.7 ± 0.2 degrees, and a peak at 29.5 ± 0.2 degrees on a 2θ scale in an X-ray powder diffraction pattern. In a further embodiment, the X-ray powder diffraction pattern is obtained using Cu K alpha radiation. A thermogravimetric trace of Form K ({drug7i}) is provided as Figure 17 of WO2013/158121.

In another aspect, the invention provides a crystalline solvate Form L ({drug7j}) of Compound (VII), which has an XRPD pattern as depicted in Figure 18 of WO2013/158121. This crystalline solvate form is an isopropyl acetate/water 95/5 solvate. In a further embodiment of this aspect, crystalline solvate Form L ({drug7j}) is characterized by one or more peaks selected from the group consisting of 7.9 ± 0.2 degrees, 10.4 ± 0.2 degrees, 10.9 ± 0.2 degrees, 12.1 ± 0.2 degrees, 14.8 ± 0.2 degrees, 16.0 ± 0.2 degrees, 18.6 ± 0.2 degrees, and 20.5 ± 0.2 degrees on a 2θ scale in an X-ray powder diffraction pattern. In a further embodiment, the X-ray powder diffraction pattern is obtained using Cu K alpha radiation. In a further embodiment of this aspect, crystalline solvate Form L ({drug7j}) is characterized by a peak at 7.9 ± 0.2 degrees, a peak at 10.4 ± 0.2 degrees, a peak at 10.9 ± 0.2 degrees, a peak at 12.1 ± 0.2 degrees, a peak at 14.8 ± 0.2 degrees, a peak at 16.0 ± 0.2 degrees, a peak at 18.6 ± 0.2 degrees, and a peak at 20.5 ± 0.2 degrees on a 2θ scale in an X-ray powder diffraction pattern. In a further embodiment, the X-ray powder diffraction pattern is obtained using Cu K alpha radiation. In a further embodiment of this aspect, crystalline solvate Form L ({drug7j}) is characterized by one or more peaks selected from the group consisting of 7.9 ± 0.2 degrees, 10.0 ± 0.2 degrees, 10.4 ± 0.2 degrees, 10.9 ± 0.2 degrees, 12.1 ± 0.2 degrees, 13.2 ± 0.2 degrees, 13.8 ± 0.2 degrees, 14.8 ± 0.2 degrees, 16.0 ± 0.2 degrees, 16.9 ± 0.2 degrees, 17.4 ± 0.2 degrees, 18.6 ± 0.2 degrees, 19.7 ± 0.2 degrees, 20.5 ± 0.2 degrees, 21.6 ± 0.2 degrees, 22.1 ± 0.2 degrees, 22.5 ± 0.2 degrees, 24.8 ± 0.2 degrees, and 25.2 ± 0.2 degrees on a 2θ scale in an X-ray powder diffraction pattern. In a further embodiment, the X-ray powder diffraction pattern is obtained using Cu K alpha radiation. In a further embodiment of this aspect, crystalline solvate Form L ({drug7j}) is characterized by a peak at 7.9 ± 0.2 degrees, a peak at 10.0 ± 0.2 degrees, a peak at 10.4 ± 0.2 degrees, a peak at 10.9 ± 0.2 degrees, a peak at 12.1 ± 0.2 degrees, a peak at 13.2 ± 0.2 degrees, a peak at 13.8 ± 0.2 degrees, a peak at 14.8 ± 0.2 degrees, a peak at 16.0 ± 0.2 degrees, a peak at 16.9 ± 0.2 degrees, a peak at 17.4 ± 0.2 degrees, a peak at 18.6 ± 0.2 degrees, a peak at 19.7 ± 0.2 degrees, a peak at 20.5 ± 0.2 degrees, a peak at 21.6 ± 0.2 degrees, a peak at 22.1 ± 0.2 degrees, a peak at 22.5 ± 0.2 degrees, a peak at 24.8 ± 0.2 degrees, and a peak at 25.2 ± 0.2 degrees on a 2θ scale in an X-ray powder diffraction pattern. In a further embodiment, the X-ray powder diffraction pattern is obtained using Cu K alpha radiation. A thermogravimetric trace of Form L ({drug7j}) is provided as Figure 19 of WO2013/158121.

In another aspect, the invention provides a crystalline solvate Form M ({drug7k}) of Compound (VII), which has an XRPD pattern as depicted in Figure 20 of WO2013/158121. This crystalline solvate form is a methylethyl ketone (MEK) solvate, or a MEK/water 99/1 solvate. In a further embodiment of this aspect, crystalline solvate Form M ({drug7k}) is characterized by one or more peaks selected from the group consisting of 5.2 ± 0.2 degrees, 6.0 ± 0.2 degrees, 6.9 ± 0.2 degrees, 10.7 ± 0.2 degrees, 12.3 ± 0.2 degrees, 17.6 ± 0.2 degrees, 18.3 ± 0.2 degrees, and 21.1 ± 0.2 degrees on a 2θ scale in an X-ray powder diffraction pattern. In a further embodiment, the X-ray powder diffraction pattern is obtained using Cu K alpha radiation. In a further embodiment of this aspect, crystalline solvate Form M ({drug7k}) is characterized by a peak at 5.2 ± 0.2 degrees, a peak at 6.0 ± 0.2 degrees, a peak at 6.9 ± 0.2 degrees, a peak at 10.7 ± 0.2 degrees, a peak at 12.3 ± 0.2 degrees, a peak at 17.6 ± 0.2 degrees, a peak at 18.3 ± 0.2 degrees, and a peak at 21.1 ± 0.2 degrees on a 2θ scale in an X-ray powder diffraction pattern. In a further embodiment, the X-ray powder diffraction pattern is obtained using Cu K alpha radiation. In a further embodiment of this aspect, crystalline solvate Form M ({drug7k}) is characterized by one or more peaks selected from the group consisting of 5.2 ± 0.2 degrees, 6.0 ± 0.2 degrees, 6.9 ± 0.2 degrees, 8.0 ± 0.2 degrees, 8.9 ± 0.2 degrees, 10.7 ± 0.2 degrees, 12.3 ± 0.2 degrees, 13.0 ± 0.2 degrees, 13.5 ± 0.2 degrees, 14.4 ± 0.2 degrees, 16.8 ± 0.2 degrees, 17.3 ± 0.2 degrees, 17.6 ± 0.2 degrees, 18.3 ± 0.2 degrees, 18.7 ± 0.2 degrees, 19.8 ± 0.2 degrees, 20.4 ± 0.2 degrees, 21.1 ± 0.2 degrees, 22.0 ± 0.2 degrees, and 23.4 ± 0.2 degrees, on a 2θ scale in an X-ray powder diffraction pattern. In a further embodiment, the X-ray powder diffraction pattern is obtained using Cu K alpha radiation. In a further embodiment of this aspect, crystalline solvate Form M ({drug7k}) is characterized by a peak at 5.2 ± 0.2 degrees, a peak at 6.0 ± 0.2 degrees, a peak at 6.9 ± 0.2 degrees, a peak at 8.0 ± 0.2 degrees, a peak at 8.9 ± 0.2 degrees, a peak at 10.7 ± 0.2 degrees, a peak at 12.3 ± 0.2 degrees, a peak at 13.0 ± 0.2 degrees, a peak at 13.5 ± 0.2 degrees, a peak at 14.4 ± 0.2 degrees, a peak at 16.8 ± 0.2 degrees, a peak at 17.3 ± 0.2 degrees, a peak at 17.6 ± 0.2 degrees, a peak at 18.3 ± 0.2 degrees, a peak at 18.7 ± 0.2 degrees, a peak at 19.8 ± 0.2 degrees, a peak at 20.4 ± 0.2 degrees, a peak at 21.1 ± 0.2 degrees, a peak at 22.0 ± 0.2 degrees, and a peak at 23.4 ± 0.2 degrees, on a 2θ scale in an X-ray powder diffraction pattern. In a further embodiment, the X-ray powder diffraction pattern is obtained using Cu K alpha radiation. A thermogravimetric

trace of Form M ({drug7k}) is provided as Figure 21 of WO2013/158121.

In another aspect, the invention provides a crystalline solvate Form N ({drug71}) of Compound (VII), which has an XRPD pattern as depicted in Figure 22 of WO2013/158121. This crystalline solvate form is a MEK/water 90/10 solvate or a MEK/water 80/20 solvate. In a further embodiment of this aspect, crystalline solvate Form N ({drug71}) is characterized by one or more peaks selected from the group consisting of 8.2 ± 0.2 degrees, 9.7 ± 0.2 degrees, 10.6 ± 0.2 degrees, 11.5 ± 0.2 degrees, 12.4 ± 0.2 degrees, 16.6 ± 0.2 degrees, 17.6 ± 0.2 degrees, and 23.8 ± 0.2 degrees on a 20 scale in an X-ray powder diffraction pattern. In a further embodiment, the X-ray powder diffraction pattern is obtained using Cu K alpha radiation. In a further embodiment of this aspect, crystalline solvate Form N ({drug71}) is characterized by a peak at 8.2 ± 0.2 degrees, a peak at 9.7 ± 0.2 degrees, a peak at 10.6 ± 0.2 degrees, a peak at 11.5 ± 0.2 degrees, a peak at 12.4 ± 0.2 degrees, a peak at 16.6 ± 0.2 degrees, a peak at 17.6 ± 0.2 degrees, and a peak at 23.8 ± 0.2 degrees on a 20 scale in an X-ray powder diffraction pattern. In a further embodiment, the X-ray powder diffraction pattern is obtained using Cu K alpha radiation. In a further embodiment of this aspect, crystalline solvate Form N ({drug71}) is characterized by one or more peaks selected from the group consisting of 6.4 ± 0.2 degrees, 7.6 ± 0.2 degrees, 8.2 ± 0.2 degrees, 9.1 ± 0.2 degrees, 9.7 ± 0.2 degrees, 10.2 ± 0.2 degrees, 10.6 ± 0.2 degrees, 11.5 ± 0.2 degrees, 12.4 ± 0.2 degrees, 14.1 ± 0.2 degrees, 15.4 ± 0.2 degrees, 16.6 ± 0.2 degrees, 17.0 ± 0.2 degrees, 17.6 ± 0.2 degrees, 18.8 ± 0.2 degrees, 21.4 ± 0.2 degrees, 23.3 ± 0.2 degrees, 23.8 ± 0.2 degrees, 24.2 ± 0.2 degrees, and 25.7 ± 0.2 degrees on a 20 scale in an X-ray powder diffraction pattern. In a further embodiment, the X-ray powder diffraction pattern is obtained using Cu K alpha radiation. In a further embodiment of this aspect, crystalline solvate Form N ({drug71}) is characterized by a peak at 6.4 ± 0.2 degrees, a peak at 7.6 ± 0.2 degrees, a peak at 8.2 ± 0.2 degrees, a peak at 9.1 ± 0.2 degrees, a peak at 9.7 ± 0.2 degrees, a peak at 10.2 ± 0.2 degrees, a peak at 10.6 ± 0.2 degrees, a peak at 11.5 ± 0.2 degrees, a peak at 12.4 ± 0.2 degrees, a peak at 14.1 ± 0.2 degrees, a peak at 15.4 ± 0.2 degrees, a peak at 16.6 ± 0.2 degrees, a peak at 17.0 ± 0.2 degrees, a peak at 17.6 ± 0.2 degrees, a peak at 18.8 ± 0.2 degrees, a peak at 21.4 ± 0.2 degrees, a peak at 23.3 ± 0.2 degrees, a peak at 23.8 ± 0.2 degrees, a peak at 24.2 ± 0.2 degrees, and a peak at 25.7 ± 0.2 degrees on a 20 scale in an X-ray powder diffraction pattern. In a further embodiment, the X-ray powder diffraction pattern is obtained using Cu K alpha radiation. A thermogravimetric trace of Form N ({drug71}) is provided as Figure 23 of WO2013/158121.

In another aspect, the invention provides a crystalline solvate Form O ({drug7m}) of Compound (VII), which has an XRPD pattern as depicted in Figure 24 of WO2013/158121. This crystalline solvate form is a methylethyl ketone (MEK) solvate, or a MEK/water 99/1 solvate. In a further embodiment of this aspect, crystalline solvate Form O ({drug7m}) is characterized by one or more peaks selected from the group consisting of 5.7 ± 0.2 degrees, 8.5 ± 0.2 degrees, 9.5 ± 0.2 degrees, 11.4 ± 0.2 degrees, 15.3 ± 0.2 degrees, 17.2 ± 0.2 degrees, 18.9 ± 0.2 degrees, and 22.2 ± 0.2 degrees on a 2Θ scale in an X-ray powder diffraction pattern. In a further embodiment, the X-ray powder diffraction pattern is obtained using Cu K alpha radiation. In a further embodiment of this aspect, crystalline solvate Form O ({drug7m}) is characterized by a peak at 5.7 ± 0.2 degrees, a peak at 8.5 ± 0.2 degrees, a peak at 9.5 ± 0.2 degrees, a peak at II.4 ± 0.2 degrees, a peak at 15.3 ± 0.2 degrees, a peak at 17.2 ± 0.2 degrees, a peak at 18.9 ± 0.2 degrees, and a peak at 22.2 ± 0.2 degrees on a 20 scale in an X-ray powder diffraction pattern. In a further embodiment, the X-ray powder diffraction pattern is obtained using Cu K alpha radiation. In a further embodiment of this aspect, crystalline solvate Form O ({drug7m}) is characterized by one or more peaks selected from the group consisting of 5.7 ± 0.2 degrees, 7.5 ± 0.2 degrees, 8.5 ± 0.2 degrees, 9.5 ± 0.2 degrees, 11.4 ± 0.2 degrees, 13.2 ± 0.2 degrees, 15.3 ± 0.2 degrees, 16.3 ± 0.2 degrees, 17.2 ± 0.2 degrees, 17.5 ± 0.2 degrees, 18.0 ± 0.2 degrees, 18.9 ± 0.2 degrees, 20.3 ± 0.2 degrees, 20.6 ± 0.2 degrees, 22.2 ± 0.2 degrees, 23.1 ± 0.2 degrees, 23.5 ± 0.2 degrees, and 26.4 ± 0.2 degrees on a 20 scale in an X-ray powder diffraction pattern. In a further embodiment, the X-ray powder diffraction pattern is obtained using Cu K alpha radiation. In a further embodiment of this aspect, crystalline solvate Form O ({drug7m}) is characterized by a peak at 5.7 ± 0.2 degrees, a peak at 7.5 ± 0.2 degrees, a peak at 8.5 ± 0.2 degrees, a peak at 9.5 ± 0.2 degrees, a peak at 11.4 ± 0.2 degrees, a peak at 13.2 ± 0.2 degrees, a peak at 15.3 ± 0.2 degrees, a peak at 16.3 ± 0.2 degrees, a peak at 17.2 ± 0.2 degrees, a peak at 17.5 ± 0.2 degrees, a peak at 18.0 ± 0.2 degrees, a peak at 18.9 ± 0.2 degrees, a peak at 20.3 ± 0.2 degrees, a peak at 20.6 ± 0.2 degrees, a peak at 22.2 ± 0.2 degrees, a peak at 23.1 ± 0.2 degrees, a peak at 23.5 ± 0.2 degrees, and a peak at 26.4 ± 0.2 degrees on a 20 scale in an X-ray powder diffraction pattern. In a further embodiment, the X-ray powder diffraction pattern is obtained using Cu K alpha radiation. A thermogravimetric trace of Form O ({drug7m}) is provided as Figure 25 of WO2013/158121.

In another aspect, the invention provides a crystalline solvate Form P ({drug7n}) of Compound (VII), which has an XRPD pattern as depicted in Figure 26 of WO2013/158121. This crystalline solvate form is a MEK/water 90/10 solvate or a MEK/water 80/20 solvate. In a further embodiment of this aspect, crystalline solvate Form P ({drug7n}) is characterized by one or more peaks selected from the group consisting of 6.1 ± 0.2 degrees, 7.4 ± 0.2 degrees, 8.2 ± 0.2 degrees, 9.1 ± 0.2 degrees, 11.5 ± 0.2 degrees, 12.3 ± 0.2 degrees, 16.7 ± 0.2 degrees, and 17.7 ± 0.2 degrees on a 20 scale in an X-ray powder diffraction pattern. In a further embodiment, the X-ray powder diffraction pattern is obtained using Cu K alpha radiation. In a further embodiment of this aspect, crystalline solvate Form P ({drug7n}) is characterized by a peak at 6.1 ± 0.2 degrees, a peak at 7.4 ± 0.2 degrees, a peak at 8.2 ± 0.2 degrees, a peak at 9.1 ± 0.2 degrees,

a peak at 11.5 ± 0.2 degrees, a peak at 12.3 ± 0.2 degrees, a peak at 16.7 ± 0.2 degrees, and a peak at 17.7 ± 0.2 degrees on a 2θ scale in an X-ray powder diffraction pattern. In a further embodiment, the X-ray powder diffraction pattern is obtained using Cu K alpha radiation. In a further embodiment of this aspect, crystalline solvate Form P ({drug7n}) is characterized by one or more peaks selected from the group consisting of 6.1 ± 0.2 degrees, 7.4 ± 0.2 degrees, 8.2 ± 0.2 degrees, 9.1 ± 0.2 degrees, 9.7 ± 0.2 degrees, 10.6 ± 0.2 degrees, 10.9 ± 0.2 degrees, 11.5 ± 0.2 degrees, 12.3 ± 0.2 degrees, 16.1 ± 0.2 degrees, 16.7 ± 0.2 degrees, 17.0 ± 0.2 degrees, 17.7 ± 0.2 degrees, 18.9 ± 0.2 degrees, 21.9 ± 0.2 degrees, 23.3 ± 0.2 degrees, 23.8 ± 0.2 degrees, 24.9 ± 0.2 degrees, and 25.8 ± 0.2 degrees on a 2θ scale in an X-ray powder diffraction pattern. In a further embodiment, the X-ray powder diffraction pattern is obtained using Cu K alpha radiation. In a further embodiment of this aspect, crystalline solvate Form P ({drug7n}) is characterized by a peak at 6.1 ± 0.2 degrees, a peak at 7.4 ± 0.2 degrees, a peak at 8.2 ± 0.2 degrees, a peak at 9.1 ± 0.2 degrees, a peak at 9.7 ± 0.2 degrees, a peak at 10.6 ± 0.2 degrees, a peak at 10.9 ± 0.2 degrees, a peak at 11.5 ± 0.2 degrees, a peak at 12.3 ± 0.2 degrees, a peak at 16.1 ± 0.2 degrees, a peak at 16.7 ± 0.2 degrees, a peak at 17.0 ± 0.2 degrees, a peak at 17.7 ± 0.2 degrees, a peak at 18.9 ± 0.2 degrees, a peak at 21.9 ± 0.2 degrees, a peak at 23.3 ± 0.2 degrees, a peak at 23.8 ± 0.2 degrees, a peak at 24.9 ± 0.2 degrees, and a peak at 25.8 ± 0.2 degrees on a 2θ scale in an X-ray powder diffraction pattern. In a further embodiment, the X-ray powder diffraction pattern is obtained using Cu K alpha radiation. A thermogravimetric trace of Form P ({drug7n}) is provided as Figure 27 of WO2013/158121.

In another aspect, the invention provides a crystalline solvate Form Q ({drug7o}) of Compound (VII), which has an XRPD pattern as depicted in Figure 28 of WO2013/158121. This crystalline solvate form is a MEK/water 80/20 solvate. In one embodiment of this aspect, Form Q ({drug7o}) is characterized by one or more peaks selected from the group consisting of 6.3 ± 0.2 degrees, 7.6 ± 0.2 degrees, 8.4 ± 0.2 degrees, 11.1 ± 0.2 degrees, 12.5 ± 0.2 degrees, 16.4 ± 0.2 degrees, 16.9 ± 0.2 degrees, and 18.0 ± 0.2 degrees on a 2θ scale in an X-ray powder diffraction pattern. In a further embodiment, the X-ray powder diffraction pattern is obtained using Cu K alpha radiation. In one embodiment of this aspect, Form Q ({drug7o}) is characterized by a peak at 6.3 ± 0.2 degrees, a peak at 7.6 ± 0.2 degrees, a peak at 8.4 ± 0.2 degrees, a peak at 11.1 ± 0.2 degrees, a peak at 12.5 ± 0.2 degrees, a peak at 16.4 ± 0.2 degrees, a peak at 16.9 ± 0.2 degrees, and a peak at 18.0 ± 0.2 degrees on a 2θ scale in an X-ray powder diffraction pattern. In a further embodiment, the X-ray powder diffraction pattern is obtained using Cu K alpha radiation. In one embodiment of this aspect, Form Q ({drug7o}) is characterized by one or more peaks selected from the group consisting of 6.3 ± 0.2 degrees, 7.6 ± 0.2 degrees, 8.4 ± 0.2 degrees, 11.1 ± 0.2 degrees, 11.7 ± 0.2 degrees, 12.5 ± 0.2 degrees, 14.7 ± 0.2 degrees, 16.4 ± 0.2 degrees, 16.9 ± 0.2 degrees, 17.3 ± 0.2 degrees, 18.0 ± 0.2 degrees, 18.8 ± 0.2 degrees, 20.6 ± 0.2 degrees, 21.0 ± 0.2 degrees, 22.2 ± 0.2 degrees, 24.1 ± 0.2 degrees, 25.1 ± 0.2 degrees, and 27.4 ± 0.2 degrees on a 2θ scale in an X-ray powder diffraction pattern. In a further embodiment, the X-ray powder diffraction pattern is obtained using Cu K alpha radiation. In one embodiment of this aspect, Form Q ({drug7o}) is characterized by a peak at 6.3 ± 0.2 degrees, a peak at 7.6 ± 0.2 degrees, a peak at 8.4 ± 0.2 degrees, a peak at 11.1 ± 0.2 degrees, a peak at 11.7 ± 0.2 degrees, a peak at 12.5 ± 0.2 degrees, a peak at 14.7 ± 0.2 degrees, a peak at 16.4 ± 0.2 degrees, a peak at 16.9 ± 0.2 degrees, a peak at 17.3 ± 0.2 degrees, a peak at 18.0 ± 0.2 degrees, a peak at 18.8 ± 0.2 degrees, a peak at 20.6 ± 0.2 degrees, a peak at 21.0 ± 0.2 degrees, a peak at 22.2 ± 0.2 degrees, a peak at 24.1 ± 0.2 degrees, a peak at 25.1 ± 0.2 degrees, and a peak at 27.4 ± 0.2 degrees on a 2θ scale in an X-ray powder diffraction pattern. In a further embodiment, the X-ray powder diffraction pattern is obtained using Cu K alpha radiation. A thermogravimetric trace of Form Q ({drug7o}) is provided as Figure 29 of WO2013/158121.

In another aspect, the invention provides a crystalline solvate Form R ({drug7p}) of Compound (VII), which has an XRPD pattern as depicted in Figure 30 of WO2013/158121. This crystalline form is an acetonitrile solvate. In a further embodiment of this aspect, crystalline solvate Form R ({drug7p}) is characterized by one or more peaks selected from the group consisting of 6.7 ± 0.2 degrees, 7.7 ± 0.2 degrees, 13.2 ± 0.2 degrees, 15.1 ± 0.2 degrees, and 17.8 ± 0.2 degrees on a 2θ scale in an X-ray powder diffraction pattern. In a further embodiment, the X-ray powder diffraction pattern is obtained using Cu K alpha radiation. In a further embodiment of this aspect, crystalline solvate Form R ({drug7p}) is characterized by a peak at 6.7 ± 0.2 degrees, a peak at 7.7 ± 0.2 degrees, a peak at 13.2 ± 0.2 degrees, a peak at 15.1 ± 0.2 degrees, and a peak at 17.8 ± 0.2 degrees on a 2θ scale in an X-ray powder diffraction pattern. In a further embodiment, the X-ray powder diffraction pattern is obtained using Cu K alpha radiation. A thermogravimetric trace of Form R ({drug7p}) is provided as Figure 31 of WO2013/158121.

In another aspect, the invention provides a crystalline solvate Form S ({drug7q}) of Compound (VII), which has an XRPD pattern as depicted in Figure 32 of WO2013/158121. This crystalline solvate form is a MEK/water 80/20 solvate. In a further embodiment of this aspect, crystalline solvate Form S ({drug7q}) is characterized by one or more peaks selected from the group consisting of 4.8 ± 0.2 degrees, 7.7 ± 0.2 degrees, 8.2 ± 0.2 degrees, 9.6 ± 0.2 degrees, 11.6 ± 0.2 degrees, 15.1 ± 0.2 degrees, 18.3 ± 0.2 degrees, and 23.6 ± 0.2 degrees on a 2θ scale in an X-ray powder diffraction pattern. In a further embodiment, the X-ray powder diffraction pattern is obtained using Cu K alpha radiation. In a further embodiment of this aspect, crystalline solvate Form S ({drug7q}) is characterized by a peak at 4.8 ± 0.2 degrees, a peak at 7.7 ± 0.2 degrees, a peak at 8.2 ± 0.2 degrees, a peak at 9.6 ± 0.2 degrees, a peak at 11.6 ± 0.2

degrees, a peak at 15.1 ± 0.2 degrees, a peak at 18.3 ± 0.2 degrees, and a peak at 23.6 ± 0.2 degrees on a 2θ scale in an X-ray powder diffraction pattern. In a further embodiment, the X-ray powder diffraction pattern is obtained using Cu K alpha radiation. In a further embodiment of this aspect, crystalline solvate Form S ({drug7q}) is characterized by one or more peaks selected from the group consisting of 4.8 ± 0.2 degrees, 5.7 ± 0.2 degrees, 7.7 ± 0.2 degrees, 8.2 ± 0.2 degrees, 8.6 ± 0.2 degrees, 9.6 ± 0.2 degrees, 11.3 ± 0.2 degrees, 11.6 ± 0.2 degrees, 12.3 ± 0.2 degrees, 13.9 ± 0.2 degrees, 15.1 ± 0.2 degrees, 15.5 ±0.2 degrees, 15.9 ± 0.2 degrees, 16.5 ± 0.2 degrees, 17.6 ± 0.2 degrees, 18.3 ± 0.2 degrees, 19.4 ± 0.2 degrees, 19.7 ± 0.2 degrees, 20.7 ± 0.2 degrees, and 23.6 ± 0.2 degrees on a 2θ scale in an X-ray powder diffraction pattern. In a further embodiment, the X-ray powder diffraction pattern is obtained using Cu K alpha radiation. In a further embodiment of this aspect, crystalline solvate Form S ({drug7q}) is characterized by a peak at 4.8 ± 0.2 degrees, a peak at 5.7 ± 0.2 degrees, a peak at 7.7 ± 0.2 degrees, a peak at 8.2 ± 0.2 degrees, a peak at 8.6 ± 0.2 degrees, a peak at 9.6 ± 0.2 degrees, a peak at 11.3 ± 0.2 degrees, a peak at 11.6 ± 0.2 degrees, a peak at 12.3 ± 0.2 degrees, a peak at 13.9 ± 0.2 degrees, a peak at 15.1 ± 0.2 degrees, a peak at 15.5 ± 0.2 degrees, a peak at 15.9 ± 0.2 degrees, a peak at 16.5 ± 0.2 degrees, a peak at 17.6 ± 0.2 degrees, a peak at 18.3 ± 0.2 degrees, a peak at 19.4 ± 0.2 degrees, a peak at 19.7 ± 0.2 degrees, a peak at 20.7 ± 0.2 degrees, and a peak at 23.6 ± 0.2 degrees on a 2θ scale in an X-ray powder diffraction pattern. In a further embodiment, the X-ray powder diffraction pattern is obtained using Cu alpha radiation. A thermogravimetric trace of Form S ({drug7q}) is provided as Figure 33 of WO2013/158121.

In another aspect, the invention provides a crystalline solvate Form T ({drug7r}) of Compound (VII), which has an XRPD pattern as depicted in Figure 34 of WO2013/158121. This crystalline solvate form is an isopropyl acetate/water 95/5 solvate. In a further embodiment of this aspect, crystalline solvate Form T ({drug7r}) is characterized by one or more peaks selected from the group consisting of 4.9 ± 0.2 degrees, 8.3 ± 0.2 degrees, 9.8 ± 0.2 degrees, 11.6 ± 0.2 degrees, 18.1 ± 0.2 degrees, 18.7 ± 0.2 degrees, 21.1 ± 0.2 degrees, and 24.0 ± 0.2 degrees on a 2θ scale in an X-ray powder diffraction pattern. In a further embodiment, the X-ray powder diffraction pattern is obtained using Cu K alpha radiation. In a further embodiment of this aspect, crystalline solvate Form T ({drug7r}) is characterized by a peak at 4.9 ± 0.2 degrees, a peak at 8.3 ± 0.2 degrees, a peak at 9.8 ± 0.2 degrees, a peak at 11.6 ± 0.2 degrees, a peak at 18.1 ± 0.2 degrees, a peak at 18.7 ± 0.2 degrees, a peak at 21.1 ± 0.2 degrees, and a peak at 24.0 ± 0.2 degrees on a 2θ scale in an X-ray powder diffraction pattern. In a further embodiment, the X-ray powder diffraction pattern is obtained using Cu K alpha radiation (λ = 1.5418 A). In a further embodiment of this aspect, crystalline solvate Form T ({drug7r}) is characterized by one or more peaks selected from the group consisting of 4.9 ± 0.2 degrees, 7.8 ± 0.2 degrees, 8.3 ± 0.2 degrees, 8.5 ± 0.2 degrees, 9.8 ± 0.2 degrees, 11.6 ± 0.2 degrees, 14.2 ± 0.2 degrees, 15.2 ± 0.2 degrees, 15.7 ± 0.2 degrees, 17.3 ± 0.2 degrees, 18.1 ± 0.2 degrees, 18.7 ± 0.2 degrees, 19.9 ± 0.2 degrees, 21.1 ± 0.2 degrees, 21.3 ± 0.2 degrees, 23.0 ± 0.2 degrees, 23.7 ± 0.2 degrees, 24.0 ± 0.2 degrees, 25.0 ± 0.2 degrees, and 25.6 ± 0.2 degrees on a 2θ scale in an X-ray powder diffraction pattern. In a further embodiment, the X-ray powder diffraction pattern is obtained using Cu K alpha radiation. In a further embodiment of this aspect, crystalline solvate Form T ({drug7r}) is characterized by a peak at 4.9 ± 0.2 degrees, a peak at 7.8 ± 0.2 degrees, a peak at 8.3 ± 0.2 degrees, a peak at 8.5 ± 0.2 degrees, a peak at 9.8 ± 0.2 degrees, a peak at 11.6 ± 0.2 degrees, a peak at 14.2 ± 0.2 degrees, a peak at 15.2 ± 0.2 degrees, a peak at 15.7 ± 0.2 degrees, a peak at 17.3 ± 0.2 degrees, a peak at 18.1 ± 0.2 degrees, a peak at 18.7 ± 0.2 degrees, a peak at 19.9 ± 0.2 degrees, a peak at 21.1 ± 0.2 degrees, a peak at 21.3 ± 0.2 degrees, a peak at 23.0 ± 0.2 degrees, a peak at 23.7 ± 0.2 degrees, a peak at 24.0 ± 0.2 degrees, a peak at 25.0 ± 0.2 degrees, and a peak at 25.6 ± 0.2 degrees on a 2θ scale in an X-ray powder diffraction pattern. In a further embodiment, the X-ray powder diffraction pattern is obtained using Cu K alpha radiation. A thermogravimetric trace of Form T ({drug7r}) is provided as Figure 35 of WO2013/158121. A single crystal was obtained for Form T ({drug7r}), which has the following unit cell dimensions: Space Group: C2/c, Cell Lengths: a = 32.5478(13), b = 22.5036(9), c = 22.5540(9), Cell Angles: a = 90.00, β = 112.725(2), γ = 90.00, Cell Volume: 15237.1 A.

In another aspect, the invention provides a crystalline solvate of Compound (VII) designated as Hydrate B ({drug7t}), which has an XRPD pattern as depicted in Figure 36 of WO2013/158121. In a further embodiment of this aspect, Hydrate B ({drug7t}) is characterized by one or more peaks selected from the group consisting of 4.9 ± 0.2 degrees, 6.0 ± 0.2 degrees, 7.2 ± 0.2 degrees, 8.9 ± 0.2 degrees, 10.1 ± 0.2 degrees, 10.7 ± 0.2 degrees, 11.3 ± 0.2 degrees, and 11.9 ± 0.2 degrees on a 2θ scale in an X-ray powder diffraction pattern. In a further embodiment, the X-ray powder diffraction pattern is obtained using Cu K alpha radiation. In a further embodiment of this aspect, Hydrate B ({drug7t}) is characterized by a peak at 4.9 ± 0.2 degrees, a peak at 6.0 ± 0.2 degrees, a peak at 7.2 ± 0.2 degrees, a peak at 8.9 ± 0.2 degrees, a peak at 10.1 ± 0.2 degrees, a peak at 10.7 ± 0.2 degrees, a peak at 11.3 ± 0.2 degrees, and a peak at 11.9 ± 0.2 degrees on a 2θ scale in an X-ray powder diffraction pattern. In a further embodiment, the X-ray powder diffraction pattern is obtained using Cu K alpha radiation. A thermogravimetric trace of Hydrate B ({drug7t}) is provided as Figure 37 of WO2013/158121.

In another aspect, the invention provides a crystalline solvate of Compound (VII) designated as Form W ({drug7s}), which has an XRPD pattern as depicted in Figure 38 of WO2013/158121. In a further embodiment of this aspect, Form W ({drug7s}) is characterized by one or more peaks selected from the group consisting of 5.5 ± 0.2

degrees, 10.9 ± 0.2 degrees, 12.1 ± 0.2 degrees, 13.3 ± 0.2 degrees, 14.6 ± 0.2 degrees, 17.2 ± 0.2 degrees, 18.8 ± 0.2 degrees, and 21.8 ± 0.2 degrees on a 2θ scale in an X-ray powder diffraction pattern. In a further embodiment, the X-ray powder diffraction pattern is obtained using Cu K alpha radiation. In a further embodiment of this aspect, Form W ({drug7s}) is characterized by a peak at 5.5 ± 0.2 degrees, a peak at 10.9 ± 0.2 degrees, a peak at 12.1 ± 0.2 degrees, a peak at 13.3 ± 0.2 degrees, a peak at 14.6 ± 0.2 degrees, a peak at 17.2 ± 0.2 degrees, a peak at 18.8 ± 0.2 degrees, and a peak at 21.8 ± 0.2 degrees on a 2θ scale in an X-ray powder diffraction pattern. In a further embodiment, the X-ray powder diffraction pattern is obtained using Cu K alpha radiation. In a further embodiment of this aspect, Form W ({drug7s}) is characterized by one or more peaks selected from the group consisting of 5.5 ± 0.2 degrees, 5.9 ± 0.2 degrees, 7.6 ± 0.2 degrees, 10.9 ± 0.2 degrees, 12.1 ± 0.2 degrees, 13.3 ± 0.2 degrees, 14.6 ± 0.2 degrees, 17.2 ± 0.2 degrees, 18.8 ± 0.2 degrees, 21.8 ± 0.2 degrees, 22.5 ± 0.2 degrees, 23.2 ± 0.2 degrees, 23.7 ± 0.2 degrees, 25.0 ± 0.2 degrees, and 25.6 ± 0.2 degrees on a 2θ scale in an X-ray powder diffraction pattern. In a further embodiment, the X-ray powder diffraction pattern is obtained using Cu K alpha radiation. In a further embodiment of this aspect, Form W ({drug7s}) is characterized by a peak at 5.5 ± 0.2 degrees, a peak at 5.9 ± 0.2 degrees, a peak at 7.6 ± 0.2 degrees, a peak at 10.9 ± 0.2 degrees, a peak at 12.1 ± 0.2 degrees, a peak at 13.3 ± 0.2 degrees, a peak at 14.6 ± 0.2 degrees, a peak at 17.2 ± 0.2 degrees, a peak at 18.8 ± 0.2 degrees, a peak at 21.8 ± 0.2 degrees, a peak at 22.5 ± 0.2 degrees, a peak at 23.2 ± 0.2 degrees, a peak at 23.7 ± 0.2 degrees, a peak at 25.0 ± 0.2 degrees, and a peak at 25.6 ± 0.2 degrees on a 2θ scale in an X-ray powder diffraction pattern. In a further embodiment, the X-ray powder diffraction pattern is obtained using Cu K alpha radiation. A thermogravimetric trace of Form W ({drug7s}) is provided as Figure 39 of WO2013/158121.

Processes for Making Crystalline Solvate Forms:

In another aspect, the invention provides a process for making a crystalline solvate Form D ({drug7b}) of Compound (VII) comprising: a) dissolving Form XX of Compound (VII) in at least one solvent to form a mixture; b) heating the mixture until the solid has dissolved; c) precipitating Form D ({drug7b}) by cooling the mixtures; and d) isolating the Form D ({drug7b}). In one embodiment of this aspect, at least one of the solvents is acetonitrile. In another embodiment of this aspect, the solvent is acetonitrile/water 75/25.

In another aspect, the invention provides a process for making a crystalline solvate Form E ({drug7c}) of Compound (VII) comprising: a) dissolving Form XX of Compound (VII) in at least one solvent to form a mixture; b) heating the mixture until the solid has dissolved; c) precipitating Form E ({drug7c}) by cooling the mixtures; and d) isolating the Form E ({drug7c}). In one embodiment of this aspect, at least one of the solvents is MEK. In another embodiment of this aspect, the solvent is MEK/water. In a further embodiment of this aspect, the solvent is MEK/water 99/1. In a further embodiment of this aspect, the solvent is MEK/water 90/10. In a further embodiment of this aspect, the solvent is MEK/water 80/20. In another aspect, the invention provides a process for making a crystalline solvate Form E ({drug7c}) of Compound (VII) comprising: a) slurrying Form XX of Compound (VII) in acetonitrile containing water to form a mixture; b) removing the residual solid from the mixture by filtration; c) removing the solvent by rapid evaporation; and d) isolating the Form E ({drug7c}). In one embodiment of this aspect, the solvent is MEK/water 90/10. In a further embodiment of this aspect, the solvent is MEK/water 80/20. In another aspect, the invention provides a process for making a crystalline solvate Form E ({drug7c}) of Compound (VII) comprising: a) slurrying amorphous Compound (VII) in isopropyl acetate containing water to form a mixture; b) removing the residual solid from the mixture by filtration; c) removing the solvent by rapid evaporation; and d) isolating the Form E ({drug7c}). In one embodiment of this aspect, the solvent is MEK/water 90/10. In a further embodiment of this aspect, the solvent is MEK/water 80/20.

In another aspect, the invention provides a process for making a crystalline solvate Form F ({drug7d}) of Compound (VII) comprising: a) slurrying Form XX of Compound (VII) in acetonitrile containing water to form a mixture; b) removing the residual solvent from the mixture by filtration; c) removing the solvent by rapid evaporation; and d) isolating the Form F ({drug7d}). In one embodiment of this aspect, the solvent is acetonitrile water 75/25.

In another aspect, the invention provides a process for making a semi-crystalline solvate Form G ({drug7e}) of Compound (VII) comprising: a) slurrying Form XX of Compound (VII) in isopropyl acetate to form a mixture; b) removing the residual solid from the mixture by filtration; c) removing the solvent by rapid evaporation; and d) isolating the Form G ({drug7e}). In one embodiment of this aspect, the solvent is isopropyl acetate.

In another aspect, the invention provides a process for making a crystalline solvate Form H ({drug7f}) of Compound (VII) comprising: a) slurrying Form XX of Compound (VII) in isopropyl acetate containing water to form a mixture; b) removing the residual solid from the mixture by filtration; c) removing the solvent by rapid evaporation; and d) isolating the Form H ({drug7f}). In one embodiment of this aspect, the solvent is isopropyl acetate/water 95/5.

In another aspect, the invention provides a process for making a crystalline solvate Form I ({drug7g}) of Compound (VII) comprising: a) slurrying Form XX of Compound (VII) in methylethyl ketone to form a mixture; b) removing the residual solid from the mixture by filtration; c) removing the solvent by rapid evaporation; and d) isolating the Form I ({drug7g}).

In another aspect, the invention provides a process for making a crystalline solvate Form J ({drug7h}) of Compound (VII) comprising: a) slurrying Form XX of Compound (VII) in methylethyl ketone containing water to form a mixture; b) removing the residual solid from the mixture by filtration; c) removing the solvent by rapid evaporation; and d) isolating the Form J ({drug7h} In one embodiment of this aspect, the solvent is MEK/water 99/1.

In another aspect, the invention provides a process for making a crystalline solvate Form K ({drug7i}) of Compound (VII) comprising: a) slurrying Form XX of Compound (VII) in methylethyl ketone containing 0 percent to 30 percent water to form a mixture; b) removing the residual solid from the mixture by filtration; c) adding an antisolvent to the mixture to induce precipitation; and d) isolating the Form K ({drug7i}). In one embodiment of this aspect, at least one of the solvents is MEK. In another embodiment of this aspect, the solvent is MEK/water. In a further embodiment of this aspect, the solvent is MEK/water 99/1. In a further embodiment of this aspect, the solvent is MEK/water 90/10. In a further embodiment of this aspect, the solvent is MEK/water 80/20. When the solvent is MEK or MEK/water, the antisolvent is a non-polar hydrocarbon solvent such as pentane hexane, heptane, octane, nonane, or the like. More particularly, the antisolvent is n-hexane.

In another aspect, the invention provides a process for making a crystalline solvate Form L ({drug7j}) of Compound (VII) comprising: a) slurrying amorphous Compound (VII) in isopropyl acetate containing water at 25°C for 6 hours to 4 weeks to form a mixture; b) removing the residual solvent from the mixture by filtration; c) removing the solvent by rapid evaporation; and d) isolating the Form L ({drug7j}). In one embodiment of this aspect, the solvent is isopropyl acetate/water 95/5. In another embodiment of this aspect, the process occurs from 12 hours to 3 weeks. In another embodiment of this aspect, the process occurs from 24 hours to 2 weeks.

In another aspect, the invention provides a process for making a crystalline solvate Form M ({drug7k}) of Compound (VII) comprising: a) at 25°C, slurrying amorphous Compound (VII) in methylethyl ketone containing 0 percent to 1 percent water for 12 hours to a month to form a mixture; b) removing the residual solid from the mixture by filtration; and c) isolating the Form M ({drug7k}). In one aspect of this embodiment, the mixture further comprises one or more polymeric additives. In another embodiment of this aspect, the additives are selected from hydroxypropyl methyl cellulose (HPMC) and sodium lauryl sulfate (SLS).

In another aspect, the invention provides a process for making a crystalline solvate Form N ({drug7l}) of Compound (VII) comprising: a) at 25°C, slurrying amorphous Compound (VII) in methylethyl ketone containing 10 percent to 20 percent water for 12 hours to a month to form a mixture; b) removing the residual solid from the mixture by filtration; and c) isolating the Form N ({drug7l)). In one aspect of this embodiment, the mixture further comprises one or more polymeric additives. In another embodiment of this aspect, the additives are selected from HPMC and SLS.

In another aspect, the invention provides a process for making a crystalline solvate Form O ({drug7m}) of Compound (VII) comprising: a) at 70°C, slurrying amorphous Compound (VII) in methylethyl ketone containing 0 percent to 1 percent water for 6 hours to 4 weeks to form a mixture; b) heating the mixture at approximately 70°C for 1 to 30 hours; c) removing the residual solid from the mixture by filtration; and d) isolating the Form O ({drug7m}). In one aspect of this embodiment, the mixture further comprises one or more polymeric additives. In another embodiment of this aspect, the additives are HPMC and SLS. In another embodiment of this aspect, the solvent is MEK. When the solvent is MEK, the slurry is heated at 70°C for from 12 hours to 3 weeks, more preferably from 24 hours to 2 weeks. In another embodiment of this aspect, the solvent is MEK/H20 99/1. When the solvent is MEK/H20 99/1, the slurry is heated at 70°C for from 6 hours to 1 week, more preferably from 12 hours to 24 hours.

In another aspect, the invention provides a process for making a crystalline solvate Form P ({drug7n}) of Compound (VII) comprising: a) at 70°C, slurrying amorphous Compound (VII) in methylethyl ketone containing 10 percent to 20 percent water for 12 to 24 hours to form a mixture; b) heating the mixture at approximately 70°C for 1 to 30 hours; c) removing the residual solid from the mixture by filtration; and d) isolating the Form P ({drug7n}). In one aspect of this embodiment, the mixture further comprises one or more polymeric additives. In another embodiment of this aspect, the additives are HPMC and SLS.

In another aspect, the invention provides a process for making a crystalline solvate Form Q ({drug7o}) of Compound (VII) comprising: a) at 70°C, slurrying amorphous Compound (VII) in methylethyl ketone containing 10 percent to 20 percent water for 2 days to 3 weeks to form a mixture; b) heating the mixture at approximately 70°C for 1 to 30 hours; c) removing the residual solid from the mixture by filtration; and d) isolating the Form Q ({drug7o}). In one aspect of this embodiment, the mixture further comprises one or more polymeric additives. In another embodiment of this aspect, the additives are HPMC and SLS.

In another aspect, the invention provides a process for making a crystalline solvate Form R ({drug7p}) of Compound (VII) comprising: a) drying the Form D ({drug7b}) of Compound (VII) in a vacuum oven at a temperature between room temperature and 45°C; and b) isolating the Form R ({drug7p}).

In another aspect, the invention provides a process for making a crystalline solvate Form S ({drug7q}) of Compound (VII) comprising: a) drying Form K ({drug7i}) of Compound (VII) in a vacuum oven at a temperature between room temperature and 45°C; and b) isolating the Form R ({drug7p}).

In another aspect, the invention provides a process for making a crystalline solvate Form T ({drug7r}) of Com-

pound (VII) comprising: a) slurrying Form XX of Compound (VII) in methylethyl ketone containing 0 percent to 30 percent water to form a mixture; b) removing the residual solid from the mixture by filtration; c) adding an antisolvent to the mixture to induce precipitation; and d) isolating the Form T ({drug7r}).

In another aspect, the invention provides a process for making a crystalline solvate Hydrate B ({drug7t}) of Compound (VII). In one embodiment of this aspect, the process for making a crystalline solvate Hydrate B ({drug7t}) comprises: a) slurrying amorphous Compound (VII) in in simulated fluids (FeSSIF); b) removing the residual solid from the mixture by filtration; c) isolating the Hydrate Form B. In another embodiment of this aspect, the process for making a crystalline solvate Hydrate B ({drug7t}) comprises: a) heating a mixture of amorphous Compound (VII) and water; b) removing the residual solid from the mixture by filtration; c) isolating the Hydrate Form B. In a further embodiment, the ratio of amorphous Compound (VII) and water is about 1:1. In another aspect, the invention provides a crystalline solvate of Compound (VII), wherein the solvent is a polar solvent, more preferably a polar aprotic solvent. The polar aprotic solvent is acetonitrile, MEK, or isopropyl acetate. In another aspect, the invention provides a crystalline solvate of Compound (VII), wherein the solvent is a polar solvent, more preferably a polar aprotic solvent, optionally additionally comprising water. In another aspect, the invention provides a crystalline solvate of Compound (VII), wherein the solvent is selected from the group consisting of acetonitrile, acetonitrile/water, isopropyl acetate, isopropyl acetate/water, methylethyl ketone, and methylethyl ketone/water. In one aspect of this embodiment, the solvent is acetonitrile. In another aspect of this embodiment, the solvent is acetonitrile/water 75/25. In another aspect of this embodiment, the solvent is isopropyl acetate. In another aspect of this embodiment, the solvent is isopropyl acetate/water 95/5. In another aspect of this embodiment, the solvent is MEK. In another aspect of this embodiment, the solvent is MEK/water 99/1. In another aspect of this embodiment, the solvent is MEK/water 90/10. In another aspect of this embodiment, the solvent is MEK/water 80/20. In one embodiment of this aspect, the solvent is acetonitrile or acetonitrile/water and the crystalline solvate is Form D ({drug7b}), Form F ({drug7d}), or Form R ({drug7p}), as described above. In another embodiment of this aspect, the solvent is isopropyl acetate or isopropyl acetate/water and the crystalline solvate, which is Form G ({drug7e}), Form H ({drug7f}), Form L ({drug7j}), or Form T ({drug7r}). In a further embodiment of this aspect, the solvent is methylethyl ketone or methylethyl ketone/water and the crystalline solvate is Form E ({drug7c}), Form I ({drug7g}), Form J ({drug7h}), Form K ({drug7i}), Form M ({drug7k}), Form N ({drug7l}), Form O ({drug7m}), Form P ({drug7n}), or Form S ({drug7q}).

In another aspect, the invention provides a process for making a crystalline solvate of Compound (VII) comprising: a) dissolving Form XX of Compound (VII) in at least one solvent to form a mixture; b) heating the mixture until the solid has dissolved; c) precipitating the crystalline solvate by cooling the mixtures; and d) isolating the crystalline solvate. In one embodiment of this aspect, the solvent is acetonitrile or acetonitrile/water and the crystalline solvate is Form D ({drug7b}). In another embodiment of this aspect, the solvent is methylethyl ketone or methylethyl ketone/water and the crystalline solvate is Form E ({drug7c}). In another aspect, the invention provides a process for making a crystalline solvate of Compound (VII) comprising: a) dissolving Form XX of Compound (VII) in at least one solvent to form a mixture; b) removing the residual solvent from the mixture by filtration; c) removing the solvent by rapid evaporation; and d) isolating the crystalline solvate. In one embodiment of this aspect, the solvent is acetonitrile/water and the crystalline solvate is Form F

({drug7d}). In another embodiment of this aspect, the solvent is isopropyl acetate and the crystalline solvate is Form G ({drug7e}). In another embodiment of this aspect, the solvent isopropyl acetate/water and the crystalline solvate is Form H ({drug7f}). In another embodiment of this aspect, the solvent methylethyl ketone and the crystalline solvate is Form I ({drug7g}). In another embodiment of this aspect, the solvent is methylethyl ketone/water and the crystalline solvate is Form J ({drug7h}) or Form E ({drug7c}). In another aspect, the invention provides a process for making a crystalline solvate of Compound (VII) comprising: a) slurrying amorphous of Compound (VII) in at least one solvent; b) removing the residual solid from the mixture by filtration; c) adding an antisolvent to the mixture to induce precipitation; and d) isolating the crystalline solvate. In one embodiment of this aspect, the solvent is methylethyl ketone containing 0 to 30 percent water and the crystalline solvate is Form K ({drug7i}). In another embodiment of this aspect, the solvent is isopropyl acetate/water and the crystalline solvate is Form T ({drug7r}). In another aspect, the invention provides a process for making a crystalline solvate of Compound (VII) comprising: a) slurrying amorphous Compound (VII) in at least one solvent; b) removing the residual solvent from the mixture by filtration; c) removing the solvent by rapid evaporation; and d) isolating the crystalline solvate. In one embodiment of this aspect, the solvent is isopropyl acetate containing water and the crystalline solvate is Form L ({drug7j}). In another aspect, the invention provides a process for making a crystalline solvate of Compound (VII) comprising: a) slurrying at room temperature amorphous Compound (VII) in at least one solvent further in the presence of HPMC and sodium lauryl sulfate to form a mixture; b) removing the residual solid from the mixture by filtration; c) allowing the crystalline solvate to precipitate from the solvent; and d) isolating the crystalline solvate. In one embodiment of this aspect, the crystalline solvate is Form M ({drug7k}) or Form N ({drug7l}). In another aspect, the invention provides a process for making a crystalline solvate of Compound (VII) comprising: a) slurrying at 70°C amorphous Compound (VII) in at least one solvent further in the presence of HPMC and sodium lauryl sulfate to form a mixture; b) removing the residual solid from the mixture by filtration; c) allowing the crystalline solvate to precipitate from the solvent; and d) isolating the crystalline solvate. In one embodiment of this

aspect, the crystalline solvate is solvate is Form O ({drug7m}), Form P ({drug7n}), or Form Q ({drug7o}). In another aspect, the invention provides a process for making a crystalline solvate of Compound (VII) comprising: a) drying solvate Form Compound (VII) in a vacuum oven at a temperature between room temperature and 45°C; and b) isolating the crystalline solvate. In one aspect of this embodiment, the solvate form used in step (a) is Form D ({drug7b}) and the solvate form isolated is Form R ({drug7p}). In another aspect of this embodiment, the solvate form used in step (a) is Form K ({drug7i}) and the solvate form isolated is Form S ({drug7q}).

In another aspect, the invention provides crystalline solvate Form D ({drug7b}) of Compound (VII) prepared by the process comprising a) dissolving Form XX of Compound (VII) in at least one solvent to form a mixture; b) heating the mixture until the solid has dissolved; c) precipitating Form D ({drug7b}) by cooling the mixtures; and d) isolating the Form D ({drug7b}). In one embodiment of this aspect, at least one of the solvents is acetonitrile. In another embodiment of this aspect, the solvent is acetonitrile/water 75/25.

In another aspect, the invention provides crystalline solvate Form E ({drug7c}) of Compound (VII) prepared by the process comprising: a) dissolving Form XX of Compound (VII) in at least one solvent to form a mixture; b) heating the mixture until the solid has dissolved; c) precipitating Form E ({drug7c}) by cooling the mixtures; and d) isolating the Form E ({drug7c}). In one embodiment of this aspect, at least one of the solvents is MEK. In another embodiment of this aspect, the solvent is MEK/water. In a further embodiment of this aspect, the solvent is MEK/water 99/1. In a further embodiment of this aspect, the solvent is MEK/water 90/10. In a further embodiment of this aspect, the solvent is MEK/water 80/20. In another aspect, the invention provides crystalline solvate Form E ({drug7c}) of Compound (VII) prepared by the process comprising: a) slurrying Form XX of Compound (VII) in acetonitrile containing water to form a mixture; b) removing the residual solid from the mixture by filtration; c) removing the solvent by rapid evaporation; and d) isolating the Form E ({drug7c}). In one embodiment of this aspect, the solvent is MEK/water 90/10. In a further embodiment of this aspect, the solvent is MEK/water 80/20. In another aspect, the invention provides crystalline solvate Form E ({drug7c}) of Compound (VII) prepared by the process comprising: a) slurrying amorphous Compound (VII) in isopropyl acetate containing water to form a mixture; b) removing the residual solid from the mixture by filtration; c) removing the solvent by rapid evaporation; and d) isolating the Form E ({drug7c}). In one embodiment of this aspect, the solvent is MEK water 90/10. In a further embodiment of this aspect, the solvent is MEK/water 80/20.

In another aspect, the invention provides crystalline solvate Form F ({drug7d}) of Compound (VII) prepared by the process comprising: a) slurrying Form XX of Compound (VII) in acetonitrile containing water to form a mixture; b) removing the residual solvent from the mixture by filtration; c) removing the solvent by rapid evaporation; and d) isolating the Form F ({drug7d}). In one embodiment of this aspect, the solvent is acetonitrile water 75/25.

In another aspect, the invention provides crystalline solvate Form G ({drug7e}) of Compound (VII) prepared by the process comprising: a) slurrying Form XX of Compound (VII) in isopropyl acetate to form a mixture; b) removing the residual solid from the mixture by filtration; c) removing the solvent by rapid evaporation; and d) isolating the Form G ({drug7e}). In one embodiment of this aspect, the solvent is isopropyl acetate.

In another aspect, the invention provides crystalline solvate Form H ({drug7f}) of Compound (VII) prepared by the process comprising: a) slurrying Form XX of Compound (VII) in isopropyl acetate containing water to form a mixture; b) removing the residual solid from the mixture by filtration; c) removing the solvent by rapid evaporation; and d) isolating the Form H ({drug7f}). In one embodiment of this aspect, the solvent is isopropyl acetate/water 95/5. .In another aspect, the invention provides crystalline solvate Form I ({drug7g}) of Compound (VII) prepared by the process comprising: a) slurrying Form XX of Compound (VII) in methylethyl ketone to form a mixture; b) removing the residual solid from the mixture by filtration; c) removing the solvent by rapid evaporation; and d) isolating the Form I ({drug7g}).

In another aspect, the invention provides crystalline solvate Form J ({drug7h}) of Compound (VII) prepared by the process comprising: a) slurrying Form XX of Compound (VII) in methylethyl ketone containing water to form a mixture; b) removing the residual solid from the mixture by filtration; c) removing the solvent by rapid evaporation; and d) isolating the Form J ({drug7h}). In one embodiment of this aspect, the solvent is MEK/water 99/1.

In another aspect, the invention provides crystalline solvate Form K ({drug7i}) of Compound (VII) prepared by the process comprising: a) slurrying Form XX of Compound (VII) in methylethyl ketone containing 0 percent to 30 percent water to form a mixture; b) removing the residual solid from the mixture by filtration; c) adding an antisolvent to the mixture to induce precipitation; and d) isolating the Form K ({drug7i}). In one embodiment of this aspect, at least one of the solvents is MEK. In another embodiment of this aspect, the solvent is MEK/water. In a further embodiment of this aspect, the solvent is MEK water 99/1. In a further embodiment of this aspect, the solvent is MEK/water 90/10. In a further embodiment of this aspect, the solvent is MEK water 80/20. When the solvent is MEK or MEK, the antisolvent is a non-polar hydrocarbon solvent such as pentane hexane, heptane, octane, nonane, or the like. More particularly, the antisolvent is n-hexane.

In another aspect, the invention provides crystalline solvate Form L ({drug7j}) of Compound (VII) prepared by the process: comprising: a) slurrying amorphous Compound (VII) in isopropyl acetate containing water at 25°C for 6 hours to 4 weeks to form a mixture; b) removing the residual solvent from the mixture by filtration; c) removing the solvent by rapid evaporation; and d) isolating the Form L ({drug7j}). In one embodiment of this aspect, the solvent is isopropyl

acetate/water 95/5. In another embodiment of this aspect, the process occurs from 12 hours to 3 weeks. In another embodiment of this aspect, the process occurs from 24 hours to 2 weeks.

In another aspect, the invention provides crystalline solvate Form M ({drug7k}) of Compound (VII) prepared by the process comprising: a) at 25°C, slurrying amorphous Compound (VII) in methylethyl ketone containing 0 percent to 1 percent water for 12 hours to a month to form a mixture; b) removing the residual solid from the mixture by filtration; and c) isolating the Form M ({drug7k}).

In one aspect of this embodiment, the mixture further comprises one or more polymeric additives. In another embodiment of this aspect, the additives are selected from hydroxypropyl methyl cellulose (HPMC) and sodium lauryl sulfate (SLS).

In another aspect, the invention provides crystalline solvate Form N ({drug7l}) of Compound (VII) prepared by the process comprising: a) at 25°C, slurrying amorphous Compound (VII) in methylethyl ketone containing 10 percent to 20 percent water for 12 hours to a month to form a mixture; b) removing the residual solid from the mixture by filtration; and c) isolating the Form N ({drug7l}). In one aspect of this embodiment, the mixture further comprises one or more polymeric additives. In another embodiment of this aspect, the additives are selected from HPMC and SLS.

In another aspect, the invention provides crystalline solvate Form O ({drug7m}) of Compound (VII) prepared by the process comprising: a) at 70°C, slurrying amorphous Compound (VII) in methylethyl ketone containing 0 percent to 1 percent water for 6 hours to 4 weeks to form a mixture; b) heating the mixture at approximately 70°C for 1 to 30 hours; c) removing the residual solid from the mixture by filtration; and d) isolating the Form O ({drug7m}). In one aspect of this embodiment, the mixture further comprises one or more polymeric additives. In another embodiment of this aspect, the additives are HPMC and SLS. In another embodiment of this aspect, the solvent is MEK. When the solvent is MEK, the slurry is heated at 70°C for from 12 hours to 3 weeks, more preferably from 24 hours to 2 weeks. In another embodiment of this aspect, the solvent is MEK/H20 99/1. When the solvent is MEK/H20 99/1, the slurry is heated at 70°C for from 6 hours to 1 week, more preferably from 12 hours to 24 hours.

In another aspect, the invention provides crystalline solvate Form P ({drug7n}) of Compound (VII) prepared by the process comprising: a) at 70°C, slurrying amorphous Compound (VII) in methylethyl ketone containing 10 percent to 20 percent water for 12 to 24 hours to form a mixture; b) heating the mixture at approximately 70°C for 1 to 30 hours; c) removing the residual solid from the mixture by filtration; and d) isolating the Form P ({drug7n}). In one aspect of this embodiment, the mixture further comprises one or more polymeric additives. In another embodiment of this aspect, the additives are HPMC and SLS.

In another aspect, the invention provides crystalline solvate Form Q ({drug7o}) of Compound (VII) prepared by the process comprising: a) at 70°C, slurrying amorphous Compound (VII) in methylethyl ketone containing 10 percent to 20 percent water for 2 days to 3 weeks to form a mixture; b) heating the mixture at approximately 70°C for 1 to 30 hours; c) removing the residual solid from the mixture by filtration; and d) isolating the Form Q ({drug7o}). In one aspect of this embodiment, the mixture further comprises one or more polymeric additives. In another embodiment of this aspect, the additives are HPMC and SLS.

In another aspect, the invention provides crystalline solvate Form R ({drug7p}) of Compound (VII) prepared by the process comprising: a) drying the Form D ({drug7b}) of Compound (VII) in a vacuum oven at a temperature between room temperature and 45°C; and b) isolating the Form R ({drug7p}).

In another aspect, the invention provides crystalline solvate Form S ({drug7q}) of Compound (VII) prepared by the process comprising: a) drying Form K ({drug7i}) of Compound (VII) in a vacuum oven at a temperature between room temperature and 45°C; and b) isolating the Form R ({drug7p}).

In another aspect, the invention provides crystalline solvate Form T ({drug7r}) of Compound (VII) prepared by the process comprising: a) slurrying Form XX of Compound (VII) in methylethyl ketone containing 0 percent to 30 percent water to form a mixture; b) removing the residual solid from the mixture by filtration; c) adding an antisolvent to the mixture to induce precipitation; and d) isolating the Form T ({drug7r}).

Uses, Formulation and Administration: Pharmaceutically acceptable compositions: In one aspect of the present invention, pharmaceutically acceptable compositions are provided, wherein these compositions comprise Forms D, E, F, G, H, I, J, K, L, M, N, O, P, Q, R, S, T, W, or Hydrate B as described herein, and optionally comprise a pharmaceutically acceptable carrier, adjuvant or vehicle. In certain embodiments, these compositions optionally further comprise one or more additional therapeutic agents. As described above, the pharmaceutically acceptable compositions of the present invention additionally comprise a pharmaceutically acceptable carrier, adjuvant, or vehicle, which, as used herein, includes any and all solvents, diluents, or other liquid vehicle, dispersion or suspension aids, surface active agents, isotonic agents, thickening or emulsifying agents, preservatives, solid binders, lubricants and the like, as suited to the particular dosage Form D ({drug7b})esired. Remington's Pharmaceutical Sciences, Sixteenth Edition, E. W. Martin (Mack Publishing Co., Easton, Pa., 1980) discloses various carriers used in formulating pharmaceutically acceptable compositions and known techniques for the preparation thereof. Except insofar as any conventional carrier medium is incompatible with the compounds of the invention, such as by producing any undesirable biological effect or otherwise interacting in a deleterious manner with any other component(s) of the pharmaceutically acceptable composition, its use is contemplated to be within the scope of this invention. Some examples of materials which can serve as pharmaceutically acceptable carriers include, but are not limited to, ion exchangers, alumina, aluminum stearate, lecithin, serum proteins, such as human serum albumin, buffer substances such as phosphates, glycine, sorbic acid, or potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, polyacrylates, waxes, polyethylene-polyoxypropylene-block polymers, wool fat, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such a propylene glycol or polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

Uses of Compounds and Pharmaceutically Acceptable Compositions: In one aspect, the invention provides a method of treating or lessening the severity of a disease in a patient comprising administering to said patient Form D, E, F, G, H, I, J, K, L, M, N, O, P, Q, R, S, T, W, or Hydrate B, or any combination of these forms, as described herein, and said disease is selected from cystic fibrosis, asthma, smoke induced COPD, chronic bronchitis, rhinosinusitis, constipation, pancreatitis, pancreatic insufficiency, male infertility caused by congenital bilateral absence of the vas deferens (CBAVD), mild pulmonary disease, idiopathic pancreatitis, allergic bronchopulmonary aspergillosis (ABPA), liver disease, hereditary emphysema, hereditary hemochromatosis, coagulation-fibrinolysis deficiencies, such as protein C deficiency, Type 1 hereditary angioedema, lipid processing deficiencies, such as familial hypercholesterolemia, Type 1 chylomicronemia, abetalipoproteinemia, lysosomal storage diseases, such as I-cell disease/pseudo-Hurler, mucopolysaccharidoses, Sandhof/Tay-Sachs, Crigler-Najjar type II, polyendocrinopathy/ hyperinsulinemia, Diabetes mellitus, Laron dwarfism, myeloperoxidase deficiency, primary hypoparathyroidism, melanoma, glycanosis CDG type 1, congenital hyperthyroidism, osteogenesis imperfecta, hereditary hypofibrinogenemia, ACT deficiency, Diabetes insipidus (DI), neurohypophyseal DI, nephrogenic DI, Charcot-Marie Tooth syndrome, Pelizaeus-Merzbacher disease, neurodegenerative diseases such as Alzheimer's disease, Parkinson's disease, amyotrophic lateral sclerosis, progressive supranuclear palsy, Pick's disease, several polyglutamine neurological disorders such as Huntington's, spinocerebellar ataxia type I, spinal and bulbar muscular atrophy, dentatorubral pallidoluysian atrophy, and myotonic dystrophy, as well as spongiForm E ({drug7c})ncephalopathies, such as hereditary Creutzfeldt- Jakob disease (due to prion protein processing defect), Fabry disease, Straussler-Scheinker syndrome, COPD, dry-eye disease, or Sjogren's disease, Osteoporosis, Osteopenia, bone healing and bone growth (including bone repair, bone regeneration, reducing bone resorption and increasing bone deposition), Gorham's Syndrome, chloride channelopathies such as myotonia congenita (Thomson and Becker forms), Bartter's syndrome type III, Dent's disease, epilepsy, hyperekplexia, lysosomal storage disease, Angelman syndrome, and Primary Ciliary Dyskinesia (PCD), a term for inherited disorders of the structure and/or function of cilia, including PCD with situs inversus (also known as Kartagener syndrome), PCD without situs inversus and ciliary aplasia. In some embodiments, the method includes treating or lessening the severity of cystic fibrosis in a patient comprising administering to said patient Form D, E, F, G, H, I, J, K, L, M, N, O, P, Q, R, S, T, W, or Hydrate B, or any combination of these forms, as described herein. In certain embodiments, the patient possesses mutant forms of human CFTR. In other embodiments, the patient possesses one or more of the following mutations AF508, R1 17H, and G551D of human CFTR. In one embodiment, the method includes treating or lessening the severity of cystic fibrosis in a patient possessing the AF508 mutation of human CFTR comprising administering to said patient Form D, E, F, G, H, I, J, K, L, M, N, O, P, Q, R, S, T, W, or Hydrate B, or any combination of these forms, as described herein. In one embodiment, the method includes treating or lessening the severity of cystic fibrosis in a patient possessing the G551D mutation of human CFTR comprising administering to said patient Form D, E, F, G, H, I, J, K, L, M, N, O, P, Q, R, S, T, W, or Hydrate B, or any combination of these forms, as described herein. In one embodiment, the method includes treating or lessening the severity of cystic fibrosis in a patient possessing the AF508 mutation of human CFTR on at least one allele comprising administering to said patient Form D, E, F, G, H, I, J, K, L, M, N, O, P, Q, R, S, T, W, or Hydrate B, or any combination of these forms, as described herein. In one embodiment, the method includes treating or lessening the severity of cystic fibrosis in a patient possessing the AF508 mutation of human CFTR on both alleles comprising administering to said patient Form D, E, F, G, H, I, J, K, L, M, N, O, P, Q, R, S, T, W, or Hydrate B, or any combination of these forms, as described herein. In one embodiment, the method includes treating or lessening the severity of cystic fibrosis in a patient possessing the G55 ID mutation of human CFTR on at least one allele comprising administering to said patient Form D, E, F, G, H, I, J, K, L, M, N, O, P, Q, R, S, T, W, or Hydrate B, or any combination of these forms, as described herein. In one embodiment, the method includes treating or lessening the severity of cystic fibrosis in a patient possessing the G551D mutation of human CFTR on both alleles comprising administering to said patient Form D, E, F, G, H, I, J, K, L,

M, N, O, P, Q, R, S, T, W, or Hydrate B, or any combination of these forms, as described herein. In yet another aspect, the present invention provides a method of treating or lessening the severity of a condition, disease, or disorder implicated by CFTR mutation. In certain embodiments, the present invention provides a method of treating a condition, disease, or disorder implicated by a deficiency of the CFTR activity, the method comprising administering a composition comprising forms D, E, F, G, H, I, J, K, L, M, N, O, P, Q, R, S, T, W, or Hydrate B, or any combination of these forms as described herein, to a subject, preferably a mammal, in need thereof. In certain embodiments, the present invention provides a method of treating diseases associated with reduced CFTR function due to mutations in the gene encoding CFTR or environmental factors (e.g., smoke). These diseases include, cystic fibrosis, chronic bronchitis, recurrent bronchitis, acute bronchitis, male infertility caused by congenital bilateral absence of the vas deferens (CB AVD), female infertility caused by congenital absence of the uterus and vagina (CAUV), idiopathic chronic pancreatitis (ICP), idiopathic recurrent pancreatitis, idiopathic acute pancreatitis, chronic rhinosinusitis, primary sclerosing cholangitis, allergic bronchopulmonary aspergillosis, diabetes, dry eye, constipation, allergic bronchopulmonary aspergillosis (ABPA), bone diseases (e.g., osteoporosis), and asthma, comprising administering to said patient Form D, E, F, G, H, I, J, K, L, M, N, O, P, Q, R, S, T, W, or Hydrate B, or any combination of these forms, as described herein. In certain embodiments, the present invention provides a method for treating diseases associated with normal CFTR function. These diseases include, chronic obstructive pulmonary disease (COPD), chronic bronchitis, recurrent bronchitis, acute bronchitis, rhinosinusitis, constipation, pancreatitis including chronic pancreatitis, recurrent pancreatitis, and acute pancreatitis, pancreatic insufficiency, male infertility caused by congenital bilateral absence of the vas deferens (CBAVD), mild pulmonary disease, idiopathic pancreatitis, liver disease, hereditary emphysema, gallstones, gastroesophageal reflux disease, gastrointestinal malignancies, inflammatory bowel disease, constipation, diabetes, arthritis, osteoporosis, and osteopenia, comprising administering to said patient Form D, E, F, G, H, I, J, K, L, M, N, O, P, Q, R, S, T, W, or Hydrate B, or any combination of these forms, as described herein. In certain embodiments, the present invention provides a method for treating diseases associated with normal CFTR function including hereditary hemochromatosis, coagulation-fibrinolysis deficiencies, such as protein C deficiency, Type 1 hereditary angioedema, lipid processing deficiencies, such as familial hypercholesterolemia, Type 1 chylomicronemia, abetalipoproteinemia, lysosomal storage diseases, such as I-cell disease/pseudo-Hurler, mucopolysaccharidoses, Sandhof/Tay-Sachs, Crigler-Najjar type II, polyendocrinopathy/hyperinsulinemia, Diabetes mellitus, Laron dwarfism, myeloperoxidase deficiency, primary hypoparathyroidism, melanoma, glycanosis CDG type 1, congenital hyperthyroidism, osteogenesis imperfecta, hereditary hypofibrinogenemia, ACT deficiency, Diabetes insipidus (DI), neurohypophyseal DI, nephrogenic DI, Charcot-Marie Tooth syndrome, Pelizaeus-Merzbacher disease, neurodegenerative diseases such as Alzheimer's disease, Parkinson's disease, amyotrophic lateral sclerosis, progressive supranuclear palsy, Pick's disease, several polyglutamine neurological disorders such as Huntington's, spinocerebellar ataxia type I, spinal and bulbar muscular atrophy, dentatorubral pallidoluysian atrophy, and myotonic dystrophy, as well as spongiForm E ({drug7c})ncephalopathies, such as hereditary Creutzfeldt-Jakob disease (due to prion protein processing defect), Fabry disease, Straussler-Scheinker syndrome, Gorham's Syndrome, chloride channelopathies, myotonia congenita (Thomson and Becker forms), Bartter's syndrome type III, Dent's disease, epilepsy, hyperekplexia, lysosomal storage disease, Angelman syndrome, Primary Ciliary Dyskinesia (PCD), PCD with situs inversus (also known as Kartagener syndrome), PCD without situs inversus and ciliary aplasia, or Sjogren's disease, comprising the step of administering to said mammal an effective amount of. Forms D, E, F, G, H, I, J, K, L, M, N, O, P, Q, R, S, T, W, or Hydrate B, or any combination of these forms, as described herein. According to an alternative preferred embodiment, the present invention provides a method of treating cystic fibrosis comprising the step of administering to said mammal a composition comprising the step of administering to said mammal an effective amount of a composition comprising Forms D, E, F, G, H, I, J, K, L, M, N, O, P, Q, R, S, T, W, or Hydrate B, or any combination of these forms, described herein. According to the invention an "effective amount" of Forms D, E, F, G, H, I, J, K, L, M, N, O, P, Q, R, S, T, W, or Hydrate B, any combination of these forms, or a pharmaceutically acceptable composition thereof is that amount effective for treating or lessening the severity of one or more of the diseases, disorders or conditions as recited above. Forms D, E, F, G, H, I, J, K, L, M, N, O, P, Q, R, S, T, W, or Hydrate B, or any combination of these forms, or a pharmaceutically acceptable composition thereof may be administered using any amount and any route of administration effective for treating or lessening the severity of one or more of the diseases, disorders or conditions as recited above. In certain embodiments, Forms D, E, F, G, H, I, J, K, L, M, N, O, P, Q, R, S, T, W, or Hydrate B, any combination of these forms, or a pharmaceutically acceptable composition thereof is useful for treating or lessening the severity of cystic fibrosis in patients who exhibit residual CFTR activity in the apical membrane of respiratory and non-respiratory epithelia. The presence of residual CFTR activity at the epithelial surface can be readily detected using methods known in the art, e.g., standard electrophysiological, biochemical, or histochemical techniques. Such methods identify CFTR activity using in vivo or ex vivo electrophysiological techniques, measurement of sweat or salivary Cl" concentrations, or ex vivo biochemical or histochemical techniques to monitor cell surface density. Using such methods, residual CFTR activity can be readily detected in patients heterozygous or homozygous for a variety of different mutations, including patients homozygous or heterozygous for the most common mutation, AF508. In another embodiment, Forms D, E, F, G, H, I, J,, L, M, N, O, P, Q, R, S, T, W, or Hydrate B, or any combination of these forms, described herein or a pharmaceutically

acceptable composition thereof, is useful for treating or lessening the severity of cystic fibrosis in patients who have residual CFTR activity induced or augmented using pharmacological methods or gene therapy. Such methods increase the amount of CFTR present at the cell surface, thereby inducing a hitherto absent CFTR activity in a patient or augmenting the existing level of residual CFTR activity in a patient. In one embodiment, Forms D, E, F, G, H, I, J, K, L, M, N, O, P, Q, R, S, T, W, or Hydrate B, or any combination of these forms, described herein, or a pharmaceutically acceptable composition thereof, is useful for treating or lessening the severity of cystic fibrosis in patients within certain genotypes exhibiting residual CFTR activity, e.g., class III mutations (impaired regulation or gating), class IV mutations (altered conductance), or class V mutations (reduced synthesis) (Lee R. Choo-Kang, Pamela L., Zeitlin, Type I, II, III, IV, and V cystic fibrosis Transmembrane Conductance Regulator Defects and Opportunities of Therapy; Current Opinion in Pulmonary Medicine 6:521 - 529,2000). Other patient genotypes that exhibit residual CFTR activity include patients homozygous for one of these classes or heterozygous with any other class of mutations, including class I mutations, class II mutations, or a mutation that lacks classification. In one embodiment, Forms D, E, F, G, H, I, J, K, L, M, N, O, P, Q, R, S, T, W, or Hydrate B, or any combination of these forms described herein or a pharmaceutically acceptable composition thereof, is useful for treating or lessening the severity of cystic fibrosis in patients within certain clinical phenotypes, e.g., a moderate to mild clinical phenotype that typically correlates with the amount of residual CFTR activity in the apical membrane of epithelia. Such phenotypes include patients exhibiting pancreatic insufficiency or patients diagnosed with idiopathic pancreatitis and congenital bilateral absence of the vas deferens, or mild lung disease. The exact amount required will vary from subject to subject, depending on the species, age, and general condition of the subject, the severity of the infection, the particular agent, its mode of administration, and the like. The compounds of the invention are preferably formulated in dosage unit Form F ({drug7d})or ease of administration and uniformity of dosage. The expression "dosage unit form" as used herein refers to a physically discrete unit of agent appropriate for the patient to be treated. It will be understood, however, that the total daily usage of the compounds and compositions of the present invention will be decided by the attending physician within the scope of sound medical judgment. The specific effective dose level for any particular patient or organism will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed, and like factors well known in the medical arts. The term "patient", as used herein, means an animal, preferably a mammal, and most preferably a human. The pharmaceutically acceptable compositions of this invention can be administered to humans and other animals orally, rectally, parenterally, intracistemally, intravaginally, intraperitoneally, topically (as by powders, ointments, drops or patch), bucally, as an oral or nasal spray, or the like, depending on the severity of the infection being treated. In certain embodiments, the compounds of the invention may be administered orally or parenterally at dosage levels of about 0.01 mg/kg to about 50 mg/kg and preferably from about 0.5 mg/kg to about 25 mg/kg, of subject body weight per day, one or more times a day, to obtain the desired therapeutic effect.

Liquid dosage forms for oral administration include, but are not limited to, pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents. Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables. The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use. In order to prolong the effect of a compound of the present invention, it is often desirable to slow the absorption of the compound from subcutaneous or intramuscular injection. This may be accomplished by the use of a liquid suspension of crystalline or amorphous material with poor water solubility. The rate of absorption of the compound then depends upon its rate of dissolution that, in turn, may depend upon crystal size and crystalline form. Alternatively, delayed absorption of a parenterally administered compound form is accomplished by dissolving or suspending the compound in an oil vehicle. Injectable depot forms are made by forming microencapsule matrices of the compound in

biodegradable polymers such as polylactide-polyglycolide. Depending upon the ratio of compound to polymer and the nature of the particular polymer employed, the rate of compound release can be controlled. Examples of other. biodegradable polymers include poly(orthoesters) and poly(anhydrides). Depot injectable formulations are also prepared by entrapping the compound in liposomes or microemulsions that are compatible with body tissues. Compositions for rectal or vaginal administration are preferably suppositories which can be prepared by mixing the compounds of this invention with suitable non-irritating excipients or carriers such as cocoa butter, polyethylene glycol or a suppository wax which are solid at ambient temperature but liquid at body temperature and therefore melt in the rectum or vaginal cavity and release the active compound. Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents. Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes. Solid compositions of a similar type may also be employed as fillers in soft and hard-filled gelatin capsules using such excipients as lactose or milk sugar as well as high molecular weight polethylene glycols and the like. The active compounds can also be in microencapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes. Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, eardrops, and eye drops are also contemplated as being within the scope of this invention. Additionally, the present invention contemplates the use of transdermal patches, which have the added advantage of providing controlled delivery of a compound to the body. Such dosage forms are prepared by dissolving or dispensing the compound in the proper medium. Absorption enhancers can also be used to increase the flux of the compound across the skin. The rate can be controlled by either providing a rate controlling membrane or by dispersing the compound in a polymer matrix or gel. It will also be appreciated that the Forms D, E, F, G, H, I, J, K, L, M, N, O, P, Q, R, S, T, W, or Hydrate B, or any combination thereof described herein or a pharmaceutically acceptable composition thereof can be employed in combination therapies, that is, Forms D, E, F, G, H, I, J, K, L, M, N, O, P, Q, R, S, T, W, or Hydrate B, or any combination thereof described herein or a pharmaceutically acceptable composition thereof, can be administered concurrently with, prior to, or subsequent to, one or more other desired therapeutics or medical procedures. The particular combination of therapies (therapeutics or procedures) to employ in a combination regimen will take into account compatibility of the desired therapeutics and/or procedures and the desired therapeutic effect to be achieved. It will also be appreciated that the therapies employed may achieve a desired effect for the same disorder (for example, an inventive compound may be administered concurrently with another agent used to treat the same disorder), or they may achieve different effects (e.g., control of any adverse effects). As used herein, additional therapeutic agents that are normally administered to treat or prevent a particular disease, or condition, are known as "appropriate for the disease, or condition, being treated." In one embodiment, the additional agent is selected from a mucolytic agent, bronchodilator, an anti-biotic, an anti-infective agent, an anti-inflammatory agent, a CFTR modulator other than a compound of the present invention, or a nutritional agent. In one embodiment, the additional agent is an antibiotic. Exemplary antibiotics useful herein include tobramycin, including tobramycin inhaled powder (TIP), azithromycin, aztreonam, including the aerosolized form of aztreonam, amikacin, including liposomal formulations thereof, ciprofloxacin, including formulations

thereof suitable for administration by inhalation, levoflaxacin, including aerosolized formulations thereof, and combinations of two antibiotics, e.g., fosfomycin and tobramycin. In another embodiment, the additional agent is a mucolyte. Exemplary mucolytes useful herein includes Pulmozyme®. In another embodiment, the additional agent is a bronchodilator. Exemplary bronchodilators include albuterol, metaprotenerol sulfate, pirbuterol acetate, salmeterol, or tetrabuline sulfate. In another embodiment, the additional agent is effective in restoring lung airway surface liquid. Such agents improve the movement of salt in and out of cells, allowing mucus in the lung airway to be more hydrated and, therefore, cleared more easily. Exemplary such agents include hypertonic saline, denufosol tetrasodium ([[(3S, 5R)-5-(4-amino-2-oxopyrimidin-1-yl)-3-hydroxyoxolan-2-yl]methoxy-hydroxyphosphoryl] [[[(2R,3S,4R,5R)-5-(2,4-dioxopyrimidin-1-yl)-3,4-dihydroxyoxolan-2-yl]methoxy-hydroxy-phosphoryl]oxy-hydroxyphosphoryl] hydrogen phosphate), or bronchitol (inhaled formulation of mannitol). In another embodiment, the additional agent is an anti-inflammatory agent, i.e., an agent that can reduce the inflammation in the lungs. Exemplary such agents useful herein include ibuprofen, docosahexanoic acid (DHA), sildenafil, inhaled glutathione, pioglitazone, hydroxychloroquine, or simavastatin. In another embodiment, the additional agent reduces the activity of the epithelial sodium channel blocker (ENaC) either directly by blocking the channel or indirectly by modulation of proteases that lead to an increase in ENaC activity (e.g., seine proteases, channel-activating proteases). Exemplary such agents include camostat (a trypsin-like protease inhibitor), QAU145, 552-02, GS-9411, INO-4995, Aerolytic, and amiloride. Additional agents that reduce the activity of the epithelial sodium channel blocker (ENaC) can be found, for example in PCT Publication No. WO2009/074575, the entire contents of which are incorporated herein in their entirety. Amongst other diseases described herein, combinations of CFTR modulators, such as Forms D, E, F, G, H, I, J, K, L, M, N, O, P, Q, R, S, T, W, or Hydrate B, and agents that reduce the activity of ENaC are used for treating Liddle's syndrome, an inflammatory or allergic condition including cystic fibrosis, primary ciliary dyskinesia, chronic bronchitis, chronic obstructive pulmonary disease, asthma, respiratory tract infections, lung carcinoma, xerostomia and keratoconjunctivitis sire, respiratory tract infections (acute and chronic; viral and bacterial) and lung carcinoma. Combinations of CFTR modulators, such as Forms D, E, F, G, H, I, J, K, L, M, N, O, P, Q, R, S, T, W, or Hydrate B, and agents that reduce the activity of ENaC are also useful for treating diseases mediated by blockade of the epithelial sodium channel also include diseases other than respiratory diseases that are associated with abnormal fluid regulation across an epithelium, perhaps involving abnormal physiology of the protective surface liquids on their surface, e.g., xerostomia (dry mouth) or keratoconjunctivitis sire (dry eye). Furthermore, blockade of the epithelial sodium channel in the kidney could be used to promote diuresis and thereby induce a hypotensive effect. Asthma includes both intrinsic (non-allergic) asthma and extrinsic (allergic) asthma, mild asthma, moderate asthma, severe asthma, bronchitic asthma, exercise-induced asthma, occupational asthma and asthma induced following bacterial infection. Treatment of asthma is also to be understood as embracing treatment of subjects, e.g., of less than 4 or 5 years of age, exhibiting wheezing symptoms and diagnosed or diagnosable as "wheezy infants", an established patient category of major medical concern and now often identified as incipient or early-phase asthmatics. (For convenience this particular asthmatic condition is referred to as "wheezy-infant syndrome".) Prophylactic efficacy in the treatment of asthma will be evidenced by reduced frequency or severity of symptomatic attack, e.g., of acute asthmatic or bronchoconstrictor attack, improvement in lung function or improved airways hyperreactivity. It may further be evidenced by reduced requirement for other, symptomatic therapy, i.e., therapy for or intended to restrict or abort symptomatic attack when it occurs, e.g., anti-inflammatory (e.g., cortico-steroid) or bronchodilatory. Prophylactic benefit in asthma may, in particular, be apparent in subjects prone to "morning dipping". "Morning dipping" is a recognized asthmatic syndrome, common to a substantial percentage of asthmatics and characterized by asthma attack, e.g., between the hours of about 4-6 am, i.e., at a time normally substantially distant from any previously administered symptomatic asthma therapy. Chronic obstructive pulmonary disease includes chronic bronchitis or dyspnea associated therewith, emphysema, as well as exacerbation of airways hyperreactivity consequent to other drug therapy, in particular, other inhaled drug therapy. In some embodiments, the combinations of CFTR modulators, such as Forms D, E, F, G, H, I, J, K, L, M, N, O, P, Q, R, S, T, W, or Hydrate B, and agents that reduce the activity of ENaC are useful for the treatment of bronchitis of whatever type or genesis including, e.g., acute, arachidic, catarrhal, croupus, chronic or phthinoid bronchitis. In another embodiment, the additional agent is a CFTR modulator other than Form XX, Forms D, E, F, G, H, I, J, K, L, M, N, O, P, Q, R, S, T, W, or Hydrate B, i.e., an agent that has the effect of modulating CFTR activity. Exemplary such agents include ataluren ("PTC124®"; 3-[5-(2-fluorophenyl)-1,2,4-oxadiazol-3-yl]benzoic acid), sinapultide, lancovutide, depelestat (a human recombinant neutrophil elastase inhibitor), cobiprostone (7-{(2R, 4aR, 5R, 7aR)-2-[(3S)-1,1-difluoro-3-methylpentyl]-2-hydroxy-6-oxooctahydrocyclopenta[b]pyran-5-yl}heptanoic acid), or (3-(6-(1-(2,2-difluorobenzo[d][1,3]dioxol-5-yl) cyclopropanecarboxamido)-3-methylpyridin-2-yl)benzoic acid. In another embodiment, the additional agent is (3-(6-(1-(2,2-difluorobenzo[d][1,3]dioxol-5-yl) cyclopropanecarboxamido)-3-methylpyridin-2-yl)benzoic acid. In another embodiment, the additional agent is a nutritional agent. Exemplary such agents include pancrelipase (pancreating enzyme replacement), including Pancrease®, Pancreacarb®, Ultrase®, or Creon®, Liprotomase® (formerly Trizytek®), Aquadeks®, or glutathione inhalation. In one embodiment, the additional nutritional agent is pancrelipase. In one embodiment, the additional agent is a CFTR modulator other than a compound of the present invention. The amount of additional therapeutic agent present in the compositions of this invention will be no more than the amount that would normally be administered in a composition

comprising that therapeutic agent as the only active agent. Preferably the amount of additional therapeutic agent in the presently disclosed compositions will range from about 50 % to 100 % of the amount normally present in a composition comprising that agent as the only therapeutically active agent. Forms D, E, F, G, H, I, J, K, L, M, N, O, P, Q, R, S, T, W, or Hydrate B, or any combination thereof described herein or a pharmaceutically acceptable composition thereof, may also be incorporated into compositions for coating an implantable medical device, such as prostheses, artificial valves, vascular grafts, stents and catheters. Accordingly, the present invention, in another aspect, includes a composition for coating an implantable device comprising a compound of the present invention as described generally above, and in classes and subclasses herein, and a carrier suitable for coating said implantable device. In still another aspect, the present invention includes an implantable device coated with a composition comprising a compound of the present invention as described generally above, and in classes and subclasses herein, and a carrier suitable for coating said implantable device. Suitable coatings and the general preparation of coated implantable devices are described in US Patents 6,099,562; 5,886,026; and 5,304,121. The coatings are typically biocompatible polymeric materials such as a hydrogel polymer, polymethyldisiloxane, polycaprolactone, polyethylene glycol, polylactic acid, ethylene vinyl acetate, and mixtures thereof. The coatings may optionally be further covered by a suitable topcoat of fluorosilicone, polysaccarides, polyethylene glycol, phospholipids or combinations thereof to impart controlled release characteristics in the composition. Another aspect of the invention relates to modulating CFTR activity in a biological sample or a patient (e.g., in vitro or in vivo), which method comprises administering to the patient, or contacting said biological sample with Forms D, E, F, G, H, I, J, K, L, M, N, O, P, Q, R, S, T, W, or Hydrate B, or any combination thereof described herein or a pharmaceutically acceptable composition thereof. The term "biological sample", as used herein, includes, without limitation, cell cultures or extracts thereof; biopsied material obtained from a mammal or extracts thereof; and blood, saliva, urine, feces, semen, tears, or other body fluids or extracts thereof. Modulation of CFTR in a biological sample is useful for a variety of purposes that are known to one of skill in the art. Examples of such purposes include, but are not limited to, the study of CFTR in biological and pathological phenomena; and the comparative evaluation of new modulators of CFTR. In yet another embodiment, a method of modulating activity of an anion channel in vitro or in vivo, is provided comprising the step of contacting said channel with Forms D, E, F, G, H, I, J, K, L, M, N, O, P, Q, R, S, T, W, or Hydrate B, or any combination thereof described herein or a pharmaceutically acceptable composition thereof. In preferred embodiments, the anion channel is a chloride channel or a bicarbonate channel. In other preferred embodiments, the anion channel is a chloride channel. According to an alternative embodiment, the present invention provides a method of increasing the number of functional CFTR in a membrane of a cell, comprising the step of contacting said cell with Forms D, E, F, G, H, I, J, K, L, M, N, O, P, Q, R, S, T, W, or Hydrate B, or any combination thereof described herein or a pharmaceutically acceptable composition thereof. According to another preferred embodiment, the activity of the CFTR is measured by measuring the transmembrane voltage potential. Means for measuring the voltage potential across a membrane in the biological sample may employ any of the known methods in the art, such as optical membrane potential assay or other electrophysiological methods. The optical membrane potential assay utilizes voltage-sensitive FRET sensors described by Gonzalez and Tsien (Se^ Gonzalez, J. E. and R. Y. Tsien (1995) "Voltage sensing by fluorescence resonance energy transfer in single cells." Biophys J69(4): 1272-80, and Gonzalez, J. E. and R. Y. Tsien (1997); "Improved indicators of cell membrane potential that use fluorescence resonance energy transfer" Chem Biol 4(4): 269-77) in combination with instrumentation for measuring fluorescence changes such as the Voltage/Ion Probe Reader (VIPR) (Seei Gonzalez, J. E., K. Oades, et al. (1999) "Cell-based assays and instrumentation for screening ion-channel targets" Drug Discov Today 4(9): 431-439). These voltage sensitive assays are based on the change in fluorescence resonant energy transfer (FRET) between the membrane-soluble, voltage-sensitive dye, DiSBAC2(3), and a fluorescent phospholipid, CC2-DMPE, which is attached to the outer leaflet of the plasma membrane and acts as a FRET donor. Changes in membrane potential (Vm) cause the negatively charged DiSBAC2(3) to redistribute across the plasma membrane and the amount of energy transfer from CC2-DMPE changes accordingly. The changes in fluorescence emission can be monitored using VIPR™ II, which is an integrated liquid handler and fluorescent detector designed to conduct cell-based screens in 96- or 384-well microtiter plates. In another aspect the present invention provides a kit for use in measuring the activity of CFTR or a fragment thereof in a biological sample in vitro or in vivo comprising (i) a composition comprising Forms D, E, F, G, H, I, J, K, L, M, N, O, P, Q, R, S, T, W, or Hydrate B, or any combination thereof or any of the above embodiments; and (ii) instructions for a) contacting the composition with the biological sample and b) measuring activity of said CFTR or a fragment thereof. In one embodiment, the kit further comprises instructions for a) contacting an additional composition with the biological sample; b) measuring the activity of said CFTR or a fragment thereof in the presence of said additional compound, and c) comparing the activity of the CFTR in the presence of the additional compound with the density of the CFTR in the presence of Forms D, E, F, G, H, I, J, K, L, M, N, O, P, Q, R, S, T, W, or Hydrate B, or any combination thereof described herein. In preferred embodiments, the kit is used to measure the density of CFTR. In another aspect, the invention provides a kit for use in measuring the activity of CFTR or a fragment thereof in a biological sample in vitro or in vivo, comprising: (i) a composition comprising crystalline solvate Form D, E, F, G, H, I, J, K, L, M, N, O, P, Q, R, S, T, W, or Hydrate B, or combinations thereof; (ii) instructions for: (a) contacting the composition with the biological sample; (b) measuring activity of said CFTR or a fragment thereof. In one

embodiment, the kit further comprises instructions for: i. contacting an additional composition with the biological sample; ii. measuring the activity of said CFTR, or a fragment thereof, in the presence of said additional compound; and iii. comparing the activity of the CFTR, or fragment thereof, in the presence of the additional compound with the density of CFTR, or fragment thereof, in the presence of a crystalline solvate Form D, E, F, G, H, I, J, K, L, M, N, O, P, Q, R, S, T, W, or Hydrate B, or combination thereof. In another embodiment, the step of comparing the activity of said CFTR, or fragment thereof, provides a measure of the density of said CFTR, or fragment thereof In order that the invention described herein may be more fully understood, the following examples are set forth. It should be understood that these examples are for illustrative purposes only and are not to be construed as limiting this invention in any manner.

Examples: Polymorphic screening of Compound (VII) was conducted within the process crystallization (acetonitrile, isopropyl acetate, water) and spray-drying (methyl ethyl ketone, water) solvent systems in order to understand its polymorphic behavior under process relevant conditions. Materials and Methods: Crystalline Compound (VII) of Form XX was used in the evaporation, antisolvent addition and crash cooling experiments described below. Amorphous Compound (VII) or neat amorphous Compound (VII) was used for slurry experiments in acetonitrile / water and isopropyl acetate/ water solvent systems as well as in methyl ethyl ketone (MEK)/ water solvent systems. Spray-dried dispersion containing amorphous Compound (VII) in the presence of the polymeric additives that include hypromellose acetate succinate (HPMCAS), and sodium lauryl sulfate (SLS) Fischer Scientific, was used for slurry experiments in the MEK/water spray-drying solvent systems. Preparation of Crystalline Compound (VII) of Form XX:

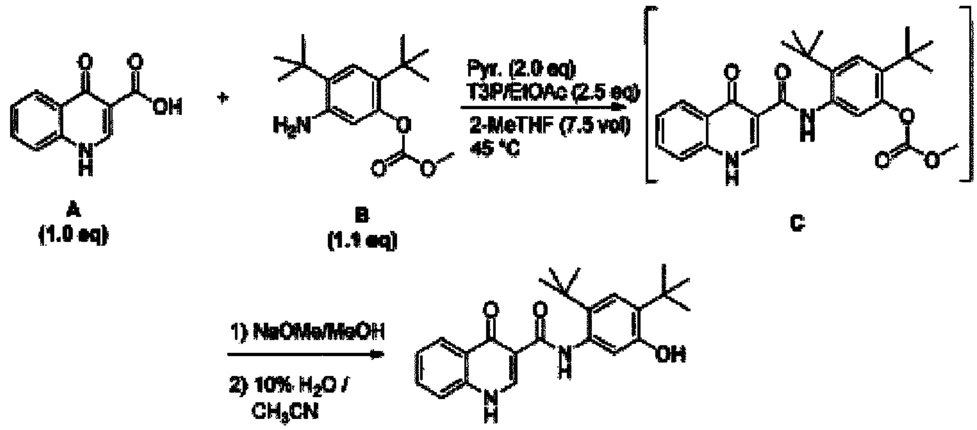


A (1.0 eq)

B (1.1 eq)

C

Compound (VII), Form XX

Compound A (1.0 eq.) and Compound B (1.1 eq.) were charged to a reactor. 2-MeTHF (4.0 vol., relative to Compound A) was added followed by T3P® 50% solution in EtOAc (2.5 eq.). The T3P charge vessel was washed with 2-MeTHF (3.5 vol.). Pyridine (2.0 eq.) was then charged. The resulting suspension was heated to 45.0 to 50.0°C and held at this temperature for 15 hours. A sample was taken and checked for completion by HPLC. Once complete, the resulting mixture was cooled to 20.0°C +/- 5.0°C. 2-MeTHF was charged (12.5 vol.) to dilute the mixture. The reaction mixture was washed with water (10.0 vol.) 3 times. 2-MeTHF was charged to bring the total volume of reaction to 40.0 vol. (-16.5 vol. charged). Residual water was removed by continuous distillation at 35.0°C +/- 5°C from 40 vol. to 30 vol. with 2-MeTHF until in-process control testing using the Karl Fisher method shows the water content to be no more than 1.0% w/w. The solution was cooled to 20.0°C +/- 5.0°C. To this solution was charged NaOMe/MeOH (1.7 equiv) to perform the hydrolysis of the carbonate. The reaction was stirred for no less than 1.0 hours, and checked for completion by HPLC. Once complete, the reaction was quenched with 1 N HCl / $H_2O$ (10.0 vol.), and washed with 0.1 N HCl (10.0 vol.). The organic solution was polish filtered to remove any particulates and placed in a second flask. The filtered solution was concentrated at 25.0°C +/- 5.0°C under reduced pressure to 20 vol. C¾CN was added to 40 vol. and the solution concentrated at 25.0°C +/- 5.0°C to 20 vol. The addition of C¾CN and concentration was repeated 2 more times for a total of 3 additions of CH3CN and 4 concentrations to 20 vol. After the final concentration to 20 vol., 16.0 vol. of $CH_3CN$ was charged followed by 4.0 vol. of H20 to make a final concentration of 40 vol. of 10% $H_2O/CH_3CN$ relative to Compound A. This slurry was refluxed for 5 hours. The slurry was cooled to 20.0°C +/- 5°C and filtered. The cake was washed with CH3CN (5 vol.) 2 times. The resulting solid was dried in a vacuum oven at 50.0°C +/- 5.0°C until a constant weight is attained.

Preparation of Amorphous Compound (VII) (also referred to as Neat Amorphous Compound (VII)): The following suspension was prepared by stirring Compound (VII), Form XX into 90% MEK/10% water according to Table A:

| (MEK/Water-90/10) | Weight (g) |
|---|---|
| MEK | 162.00 |
| Water | 18.00 |
| Compound 1, Form XX | 20.00 |
| Total Solution Weight | 200.00 |
| Solids Loading | 20.00 |

Spray drying was performed on a Buchi Mini Spray Dryer B-290 with dehumidifier B-296 and Inert Loop B-295 using the parameters used in Table B. Spray Drying Parameters:

| | |
|---|---|
| INLET Temperature | 150°C |
| OUTLET Temperature | 60°C |
| Nitrogen Pressure | 120 psi |
| Aspirator | 100 % |
| Pump Rate | 25 % |
| Nozzle Cleaner Setting | 0 |
| Rotameter | 40 mm |
| Filter Pressure | 11 mbar |
| Condenser Temperature | 5°C |
| Run Time | 37 min. |

The system was saturated with solvent that was to be sprayed, and inlet and outlet temperatures were allowed to equilibrate before spray drying.

Preparation of Spray Dried Dispersion (SDD) Containing Amorphous Compound (VII): A solvent system of MEK and DI water, formulated according to the ratio 90 wt% MEK / 10 wt% DI water, was heated to a temperature of 20 - 30°C in a reactor, equipped with a portable agitator and thermal circuit. Into this solvent system, hypromellose acetate succinate polymer (HPMCAS)(HG grade), SLS, and N-[2,4-Bis(1,1-dimethylethyl)-5-hydroxyphenyl]-1,4-dihydro-4-oxoquinoline-3-carboxamide were added according to the ratio 19.5 wt% hypromellose acetate succinate / 0.5 wt% SLS / 80 wt% N-[2,4-Bis(1,1-dimethylethyl)-5-hydroxyphenyl]-1,4-dihydro-4-oxoquinoline-3-carboxamide. The resulting mixture contained 12.5 wt% solids. The actual amounts of ingredients and solvents used to generate this mixture are recited in Table C, below: Solid Spray Dispersion Ingredients:

| | Units | Batch |
|---|---|---|
| N-[2,4-Bis(1,1-dimethylethyl)-5-hydroxyphenyl]-1,4 dihydro-4-oxoquinoline-3-carboxamide (Form XX) | Kg | 24.00 |
| HPMCAS | Kg | 5.850 |
| SLS | Kg | 0.1500 |
| **Total Solids** | **Kg** | **30.00** |
| | | |
| MEK | Kg | 189.0 |
| Water | Kg | 21.00 |
| **Total Solvents** | **Kg** | **210.0** |
| **Total Spray Solution Weight** | **Kg** | **240.0** |

The mixture was maintained at a temperature of 22-26°C and mixed until it was substantially homogenous and all components were substantially dissolved. A spray drier, Niro Production Minor Spray Dryer, fitted with pressure nozzle (Spraying Systems Maximum Free Passage (MFP) SK series nozzle having orifice size 72 and core size number 16), was used in closed cycle mode (i.e., with recirculation of the drying gas), following the dry spray process parameters recited in Table C2, below. The spray nozzle was situated approximately 5 cm from the top of the spray drying vessel. The solution was manually agitated during spray drying using a HDPE spatula. Dry spray process parameters:

| **Parameter** | **Target Value** |
|---|---|
| Feed Pressure | 30-44 bar |
| Feed Flow Rate | 15 - 21 Kg/hr |
| Inlet Temperature | 78 - 84 °C |
| Outlet Temperature | 45 - 47°C |
| Vacuum Dryer Temperature (Drying Time) | 60 °C (+/-5 °C) and 80 °C (+/-5 °C) (see below) |
| Vacuum Drying Time | 112 hours total |

A high efficiency cyclone separated the wet product from the spray gas and solvent vapors. The wet product was transferred to a tray vacuum dryer for drying to reduce residual solvents to a level of less than about 5000 ppm. During post-drying, the jacket temperature was kept at 60°C for the first 8 hours and then increased to 80°C. XPRD Analysis: The XRPD patterns were acquired with either a Bruker D8 Discover or Bruker D8 Advance diffractometer. The Bruker D8 Advance system was used to characterize the starting materials and the results of the slurry experiments in the MEK/water solvent system in the presence of the polymeric additives that include hyromellose acetate succinate (HPMCAS), and sodium lauryl sulfate (SLS) Fischer Scientific. The Bruker D8 Discover system was used for all other XRPD acquisitions. All XRPD diffractograms were evaluated using DIFFRACplus released 2006, EVA version 12.0 revision 0 software. Bruker D8 Advance System: The XRPD patterns were recorded at room temperature in reflection mode using a Bruker D8 Advance diffractometer equipped with a sealed tube Cu source and a Vantec PSD detector (Bruker AXS, Madison, WI). The X-ray generator was operating at a voltage of 40 kV and a current of 40 mA. The powder sample was placed in a silicon or PMM holder. The data were recorded in a Θ-Θ scanning mode over the range of 4°-45° 20 with a step size of 0.014° and a dwell time of Is per step. Fixed divergence slits of 0.2 mm were used. Bruker D8 Discover System: The XRPD patterns were acquired at room temperature in reflection mode using a Bruker D8 Discover diffractometer equipped with a sealed tube source and a Hi-Star area detector (Bruker AXS, Madison, WI). The X-Ray generator was operating at a voltage of 40 kV and a current of 35 mA. The powder sample was placed in an aluminum holder. Two frames were registered with an exposure time of 120 s each. The data were subsequently integrated over the range of 4°-40° 20 with a step size of 0.02° and merged into one continuous pattern. Thermogravimetric Analysis (TGA): TGA was conducted on a TA Instruments model Q5000 V3.8 thermogravimetric analyzer. Approximately 1-4 mg of solid sample was placed in a platinum sample pan and heated in a 90 mL/min nitrogen stream at 10°C/min to 300°C. All thermograms were analyzed using TA Instruments Universal Analysis 2000 software V4.4A. Single-crystal analysis: Single crystal diffraction was performed on a Bruker APEX II CCD diffractometer, with Cu Kct radiation by using single crystals picked from mother liquors and mounted on glass fibers. Oscillation photos were taken around ω axis at 4 φ angles. The data were indexed, integrated, and scaled with APEX software. The structures were solved and refined with the SHELX-TL package. The data collection was performed at a temperature of 100 Kelvin.

Polymorph screening: A polymorph screen was conducted in the methylethyl ketone (MEK)/ water solvent systems, as well as in the acetonitrile (ACN), ACN/water and isopropyl acetate solvent systems. Polymorph screening techniques included crash cooling, solvent evaporation, antisolvent addition, and slurry experiments. A range of solvent ratios were investigated for each technique and are listed in Tables 1 A, IB, 9, 12 and 14. Slurry experiments were performed on an IKA RCT hot plate with magnetic stirring set to 710 rpm. The temperature was controlled with an IKA ETS-D5 thermocouple. Solvent volumes were measured and transferred with Gibson Pipetteman volumetric pipettes. New forms that were identified as solvates were subjected to desolvation under two potential conditions: (1) drying in a vacuum oven at 40°C and (2) sitting undisturbed under ambient conditions in a fume hood. Physical characterization of all forms proceeded by X-ray powder diffraction (XRPD) and thermogravimetric analysis (TGA).

<u>Experiment 1:</u> Crash Cooling: A saturated solution of Compound (VII), Form XX was made by preparing 1 mL slurries using acetonitrile (ACN), ACN- water (75:25), methylethyl ketone (MEK), or MEK-water (99:1, 90:10, or 80:20) in vials. After 24 hours in capped HPLC vials, the slurries were then filtered to remove residual solids. Approximately 2 additional milligrams of Compound (VII) was added to each vial containing a saturated solution. Next, the vials were heated to 70°C with stirring until all of the material was completely dissolved. Immediately after, the samples were

submerged into an ice bath. The product, which precipitated as a white powder within 1 minute, was isolated by filtration and analyzed by XRPD and TGA.

The results of the crash cooling experiments are listed in Table 1 A. Crash Cooling Results:

| Solvent System | Solvate Polymorph |
|---|---|
| Acetonitrile | Form D |
| Acetonitrile/$H_2O$ | Form D |
| MEK | Form E |
| MEK/$H_2O$ (99:1) | Form E |
| MEK/$H_2O$ (90:10) | Form E |
| MEK/$H_2O$ (80:20) | Form E |
| Water | not soluble |

A white powder precipitated from the ACN and ACN/water samples within a few seconds of submerging the heated samples in an ice bath. XRPD analysis of the ACN and ACN/Water samples showed the same unique powder patterns indicating that both solvent systems produce isostructural forms. TGA showed a weight loss of 1.3% upon heating to 200°C (Figure 3 of WO2013/158121). Additionally, the weight loss began immediately upon heating in the TGA (at 27°C), indicating that the solvent is likely loosely bound in the lattice. As a consequence the precipitate was determined to be a new form of Compound (VII), designated as Form D ({drug7b}), on the basis of its unique XRPD pattern. Form D ({drug7b}) is likely a solvate based on the TGA data. The peak list for Form D ({drug7b}) is provided in Table 2. In some embodiments, Form D ({drug7b}) is characterized by one or more peaks in an XRPD spectrum selected from the peaks listed in Table 2. Form D ({drug7b}) Peak List:

| Peak Number | Angle (2-theta ± 0.2) | Intensity (%) |
|---|---|---|
| 1 | 5.6 | 100.0 |
| 2 | 6.0 | 41.5 |
| 3 | 7.8 | 9.4 |
| 4 | 13.5 | 6.8 |
| 5 | 14.1 | 7.5 |
| 6 | 14.7 | 6.9 |
| 7 | 16.2 | 9.7 |

A white powder also precipitated from MEK and MEK/water solvent systems within a few seconds of submerging the heated samples in an ice bath. XRPD analysis showed the same unique powder patterns (see, e.g., Figure 4 of WO2013/158121) for the MEK and all MEK/water ratio samples, indicating that all four solvent systems produce the same isostructural form upon crash cooling regardless of water concentration. TGA of the precipitate showed a 4.8% weight loss upon heating to 200°C (Figure 5 of WO2013/158121). The precipitate was thus determined to be a new form of Compound (VII), designated Form E ({drug7c}), on the basis of its unique XRPD pattern. Form E ({drug7c}) is likely a solvate based on the TGA data. The peak list for Form E ({drug7c}) is provided in Table 3. In some embodiments, Form E ({drug7c}) is characterized by one or more peaks in an XRPD spectrum selected from the peaks listed in Table 3.

Table 3. Form E ({drug7c}) Peak List:

| Peak Number | Angle (2-theta ± 0.2) | Intensity (%) |
|---|---|---|
| 1 | 6.8 | 32.4 |
| 2 | 8.0 | 33.4 |
| 3 | 8.6 | 34.1 |
| 4 | 10.1 | 61.4 |
| 5 | 10.7 | 61.0 |
| 6 | 11.0 | 100.0 |
| 7 | 11.9 | 41.8 |
| 8 | 12.9 | 27.1 |

(continued)

| Peak Number | Angle (2-theta ± 0.2) | Intensity (%) |
|---|---|---|
| 9 | 14.0 | 29.1 |
| 10 | 15.9 | 31.6 |
| 11 | 16.5 | 35.2 |
| 12 | 17.1 | 34.1 |
| 13 | 18.1 | 39.6 |
| 14 | 20.2 | 19.7 |
| 15 | 21.1 | 11.4 |
| 16 | 23.8 | 17.3 |
| 17 | 24.3 | 23.9 |
| 18 | 24.7 | 22.8 |
| 19 | 25.5 | 20.9 |
| 20 | 26.6 | 25.7 |

Experiment 2: Solvent Evaporation

A saturated solution was made by slurrying Compound (VII), Form XX in 5 mL of solvent (ACN, isopropyl acetate (IP Ac), or MEK) or 1 mL of solvent for the MEK/Water solvent system. After 24 hours, the residual solids were removed by filtration. The saturated solutions were then subjected to rapid solvent evaporation by blowing nitrogen (N2) gas over the samples with a Pierce Model 18780 Reacti-Vap evaporating unit for four hours. The product precipitated as a white powder and the form was analyzed by XRPD and TGA. The results of the Solvent Evaporation experiment are listed in Table IB.

Table IB: Solvent Evaporation Results:

| Solvent System | Solvent Evaporation |
|---|---|
| Acetonitrile/$H_2O$ | Form F |
| IPAc | Form G Semi-crystalline |
| IPAc/$H_2O$ | Form H |
| MEK | Form I |
| MEK/$H_2O$ (99:1) | Form J |
| MEK/$H_2O$ (90:10) | Form E |
| MEK/$H_2O$ (80:20) | Form E |
| Water | not soluble |

A white powder precipitated from the ACN and ACN/water samples upon solvent evaporation. XRPD analysis of the powder collected from the ACN sample is consistent with the powder pattern of neat crystalline Compound, Form XX (Figure 1 of WO2013/158121). The powder isolated from the ACN/water sample yielded a unique XRPD pattern indicating precipitation of a new form (Figure 6 of WO2013/158121). TGA showed a 3.1% weight loss upon heating to 200°C, consistent with solvate formation (Figure 7 of WO2013/158121). The precipitate from the ACN/water solvent system evaporation was determined to be a new form of Compound (VII), designated Form F ({drug7d}), on the basis of its unique XRPD pattern. Form F ({drug7d}) is likely a solvate based on the TGA data. The peak list for Form F ({drug7d}) is provided in Table 4. In some embodiments, Form F ({drug7d}) is characterized by one or more peaks in an XRPD spectrum selected from the peaks listed in Table 4. Form F ({drug7d}) Peak List:

| Peak Number | Angle (2-theta ± 0.2) | Intensity (%) |
|---|---|---|
| 1 | 7.2 | 55,3 |
| 2 | 7.8 | 56.5 |
| 3 | 9.4 | 100.0 |

(continued)

| Peak Number | Angle (2-theta ± 0.2) | Intensity (%) |
|---|---|---|
| 4 | 10.7 | 50.4 |
| 5 | 11.7 | 52.6 |
| 6 | 12.1 | 65.0 |
| 7 | 12.8 | 56.4 |
| 8 | 13.2 | 55.7 |
| 9 | 14.1 | 75.6 |
| 10 | 14.7 | 63.1 |
| 11 | 15.6 | 38.5 |
| 12 | 17.0 | 41.4 |
| 13 | 18.3 | 39.3 |
| 14 | 18.9 | 42.7 |
| 15 | 20.0 | 27.1 |
| 16 | 20.6 | 30.0 |
| 17 | 21.3 | 20.6 |
| 18 | 25.5 | 39.5 |
| 19 | 26.0 | 33.8 |
| 20 | 27.4 | 32.4 |

A white powder precipitated from the IP Ac and IP Ac/water samples upon solvent evaporation. Each sample produced a separate unique XRPD pattern indicating that different forms of Compound (VII) were produced from IP Ac and water saturated IP Ac upon solvent evaporation (Figures 8 and 10 of WO2013/158121). TGA showed a 2.3% weight loss upon heating the form precipitated from IP Ac to 200°C, which is attributed to loss of solvent (Figure 9 of WO2013/158121). TGA of the form precipitated from water saturated IP Ac resulted in an 11.6% weight loss upon heating to 200°C, which is attributed to loss of solvent and a further 1.8% step-wise weight loss between 200°C and 250°C (Figure 11 of WO2013/158121). As a consequence, each of the unique forms were determined to be new forms of Compound (VII) designated as Form G ({drug7e}) from IP Ac and Form H ({drug7f}) from IP Ac/water on the basis of the unique XRPD patterns. Form G ({drug7e}) and Form H ({drug7f}) are likely solvates based on the TGA results. The peak list for Form G ({drug7e}) is provided in Table 5. In some embodiments, Form G ({drug7e}) is characterized by one or more peaks in an XRPD spectrum selected from the peaks listed in Table 5. Form G ({drug7e}) Peak List:

| Peak Number | Angle (2-theta ± 0.2) | Intensity (%) |
|---|---|---|
| **1** | 6.3 | 69.6 |
| 2 | 7.3 | 100.0 |
| 3 | 12.4 | 79.1 |

The peak list for Form H ({drug7f}) is provided in Table 6. In some embodiments, Form H ({drug7f}) is characterized by one or more peaks in an XRPD spectrum selected from the peaks listed in Table 6.

Table 6. Form H ({drug7f}) Peak List:

| Peak Number | Angle (2-theta ± 0.2) | Intensity (%) |
|---|---|---|
| 1 | 5.9 | 20.1 |
| 2 | 7.9 | 64.1 |
| 3 | 9.9 | 100.0 |
| 4 | 10.4 | 69.7 |
| 5 | 10.9 | 38.4 |
| 6 | 11.6 | 43.3 |
| 7 | 12.1 | 50.4 |
| 8 | 13.2 | 35.9 |
| 9 | 13.8 | 37.3 |
| 10 | 14.8 | 66.8 |
| 11 | 16.0 | 52.8 |

(continued)

| Peak Number | Angle (2-theta ± 0.2) | Intensity (%) |
|---|---|---|
| 12 | 17.4 | 38.1 |
| 13 | 17.9 | 40.6 |
| 14 | 19.6 | 34.5 |
| 15 | 20.6 | 33.1 |
| 16 | 21.6 | 14.1 |
| 17 | 22.5 | 17.2 |
| 18 | 23.8 | 17.7 |
| 19 | 24.8 | 35.7 |
| 20 | 26.9 | 25.3 |

A white powder precipitated from the MEK and MEK/water solvent systems upon solvent evaporation. XRPD analysis of the powder collected from the MEK/Water (90:10) and MEK/Water (80:20) samples yielded a powder pattern that matches the powder pattern of Form E ({drug7c}) (Figure 4 of WO2013/158121). Isostructural forms are thus produced from crash cooling Compound (VII) from MEK and MEK/water and rapid solvent evaporation from MEK/water (90:10) and MEK water (80:20). The powder harvested from the MEK and MEK/water (99: 1) samples produced two unique XRPD patterns, indicating the precipitation of two new forms (figures 12 and 14). TGA showed a 6.2% weight loss upon heating the form precipitated from MEK out to 250°C, which is attributed to loss of solvent (Figure 13 of WO2013/158121). TGA of the form precipitated from MEK/water (99:1) shows a 3.5% weight loss upon heating to 200°C, and a further 3.7% loss between 200°C and 275°C, both losses of which are attributed to loss of solvent (Figure 15 of WO2013/158121). As a consequence, the forms precipitated from MEK and MEK/Water (99:1) were determined to be new forms of Compound (VII), designated as Form I ({drug7g}) from MEK and Form J ({drug7h}) from MEK/water (99:1) on the basis of the unique XRPD patterns. Form I ({drug7g}) and Form J ({drug7h}) are likely solvates based on the TGA results. The peak list for Form I ({drug7g}) is provided in Table 7. In some embodiments, Form I ({drug7g}) is characterized by one or more peaks in an XRPD spectrum selected from the peaks listed in Table 7. Form I ({drug7g}) Peak List:

| Peak Number | Angle (2-theta ± 0.2) | Intensity (%) |
|---|---|---|
| 1 | 5.8 | 49.0 |
| 2 | 6.8 | 92.8 |
| 3 | 7.2 | 47.5 |
| 4 | 8.4 | 100.0 |
| 5 | 8.8 | 51.2 |
| 6 | 9.4 | 49.4 |
| 7 | 10.2 | 54.8 |
| 8 | 11.4 | 50.2 |
| 9 | 12.7 | 41.3 |
| 10 | 13.7 | 48.3 |
| 11 | 14.8 | 44.8 |
| 12 | 15.7 | 45.4 |
| 13 | 17.1 | 53.6 |
| 14 | 17.8 | 54.2 |
| 15 | 18.3 | 64.3 |
| 16 | 18.8 | 61.4 |
| 17 | 20.2 | 27.4 |
| 18 | 21.3 | 25.8 |
| 19 | 23.9 | 21.2 |
| 20 | 25.2 | 30.4 |

The peak list for Form J ({drug7h}) is provided in Table 8. In some embodiments, Form J ({drug7h}) is characterized by one or more peaks in an XRPD spectrum selected from the peaks listed in Table 8.

Table 8. Form J ({drug7h}) Peak List:

| Peak Number | Angle (2-theta ± 0.2) | Intensity (%) |
|---|---|---|
| 1 | 6.0 | 89.0 |
| 2 | 6.8 | 37.7 |
| 3 | 7.9 | 78.6 |
| 4 | 8.4 | 42.3 |
| 5 | 8.8 | 100.0 |
| 6 | 9.9 | 85.4 |
| 7 | 11.8 | 93.8 |
| 8 | 12.6 | 31.3 |
| 9 | 13.6 | 41.9 |
| 10 | 15.7 | 73.9 |
| 11 | 16.7 | 41.0 |
| 12 | 17.7 | 93.7 |
| 13 | 18.0 | 95.0 |
| 14 | 18.5 | 78.4 |
| 15 | 19.3 | 73.7 |
| 16 | 20.7 | 50.7 |
| 17 | 22.6 | 23.3 |
| 18 | 26.8 | 44.7 |
| 19 | 29.4 | 22.5 |

Experiment 3: Antisolvent Addition: A saturated solution of Compound (VII), Form XX was made by slurrying either in 5 mL of solvent in the ACN, IP Ac, and MEK solvent systems, 2 mL total solvent for IP Ac/water, or 1 mL total solvent for the MEK/water solvent systems. After 24 hours, the residual solids were removed by filtration 20 mL of antisolvent was then quickly added to the saturated solutions to induce precipitation. Hexane was used as the antisolvent in the MEK and IP Ac solvent systems, and MTBE was used as the antisolvent in the ACN solvent systems, as a consequence of the immiscibility between ACN and hexane. The product precipitated as a white powder and the form was analyzed by XRPD and TGA. The results of rapid antisolvent addition into saturated solutions of Compound (VII) are listed in Table 9.

Table 9. Antisolvent Addition:

| Solvent System | Antisolvent Addition |
|---|---|
| IPAc/$H_2O$ | Form T |
| MEK | Form K |
| MEK/$H_2O$ (99:1) | Form K |
| MEK/$H_2O$ (90:10) | Form K |
| MEK/$H_2O$ (80:20) | Form K |
| Water | not soluble |

A precipitate was not initially obtained from hexane antisolvent addition into a saturated solution of Compound (VII) in IP Ac. A precipitate was not initially obtained from hexane antisolvent addition into a saturated solution of Compound **(VI)** in IP Ac/water. The clear solution was allowed to stand in a capped vial for several days after which clear rod shaped single crystals grew at the bottom of the vial. The single crystal structure solution showed a centrosymmetric structure with space group C2/c. Disordered solvent was apparent within pockets and propagated through channels in the structure. The degree of disorder was such that no attempt was made to assign the atom identities and so the solvent was simply modeled as disordered electron density. TGA of the single crystals resulted in a 0.4% weight loss upon heating to 100°C with a further 4.8% weight loss between 100°C and 200°C as a result of desolvation (Figure 35 of WO2013/158121). Simulation of the XRPD pattern from the single crystal data resulted in a unique pattern (Figure 34 of WO2013/158121). As a consequence the form that was obtained as single crystals from hexane addition to a saturated solution of Compound (VII) in IP Ac/Water was ultimately designated as Form T ({drug7r}) on the basis of the single crystal structure solution and TGA data. The peak list for Form T ({drug7r}) is provided in Table 10. In some embodiments, Form T ({drug7r}) is characterized by one or more peaks in an XRPD spectrum selected from the peaks listed in Table 10. Form T ({drug7r}) Peak List:

| Peak Number | Angle (2-theta ± 0.2) | Intensity (%) |
|---|---|---|
| 1.0 | 4.9 | 100.0 |
| 2.0 | 7.8 | 6.3 |
| 3.0 | 8.3 | 17.3 |
| 4.0 | 8.5 | 17.2 |
| 5.0 | 9.8 | 29.8 |
| 6.0 | 11.6 | 16.7 |
| 7.0 | 14.2 | 7.4 |
| 8.0 | 15.2 | 10.8 |
| 9.0 | 15.7 | 10.8 |
| 10.0 | 17.3 | 7.4 |
| 11.0 | 18.1 | 11.1 |
| 12.0 | 18.7 | 17.0 |
| 13.0 | 19.9 | 10.3 |
| 14.0 | 21.1 | 12.3 |
| 15.0 | 21.3 | 10.4 |
| 16.0 | 23.0 | 6.8 |
| 17.0 | 23.7 | 15.5 |
| 18.0 | 24.0 | 25.8 |
| 19.0 | 25.0 | 4.4 |
| 20.0 | 25.6 | 5.6 |

A white powder instantly precipitated from the MEK and MEK/water solvent systems upon hexane addition. XRPD analysis of the precipitate collected from the MEK, MEK/Water (99:1), MEK/Water (90:10) and MEK/Water (8020) resulted in the same unique powder pattern for all four solvent system samples (See, e.g., Figure 16 of WO2013/158121). All four solvent systems produced the same isostructural form upon antisolvent addition, regardless of water concentration. TGA of the precipitate showed a 13.4% weight loss upon heating to 175°C, attributable to loss of solvent and a further 1.3% weight loss between 175°C and 200°C (Figure 17 of WO2013/158121). As a consequence, the form obtained from antisolvent addition into saturated solutions of Compound (VII) in the MEK and MEK/Water solvent systems was determined to be a new form of Compound (VII) designated Form K ({drug7i}) on the basis of its unique XRPD pattern. Form K ({drug7i}) is likely a solvate judging from TGA. The peak list for Form K ({drug7i}) is provided in Table 11. In some embodiments, Form K ({drug7i}) is characterized by one or more peaks in an XRPD spectrum selected from the peaks listed in Table 11. Form K ({drug7i}) Peak List:

| Peak Number | Angle (2-theta ± 0.2) | Intensity (%) |
|---|---|---|
| 1 | 8.0 | 21.2 |
| 2 | 8.5 | 11.1 |
| 3 | 10.0 | 100.0 |
| 4 | 11.8 | 24.1 |
| 5 | 12.3 | 7.1 |
| 6 | 14.2 | 11.1 |
| 7 | 14.8 | 6.9 |
| 8 | 15.4 | 15.5 |
| 9 | 15.9 | 11.9 |
| 10 | 16.9 | 11.7 |
| 11 | 18.0 | 16.7 |
| 12 | 18.5 | 8.7 |
| 13 | 19.8 | 7.8 |
| 14 | 20.2 | 3.5 |
| 15 | 20.7 | 3.7 |

(continued)

| Peak Number | Angle (2-theta ± 0.2) | Intensity (%) |
|---|---|---|
| 16 | 21.0 | 4.1 |
| 17 | 21.5 | 2.5 |
| 18 | 22.7 | 1.3 |
| 19 | 29.5 | 5.7 |

Experiment 4: Slurry Experiments with Neat Amorphous Compound (VII): The slurry experiments were carried out using neat amorphous compound 1 in 1 mL of total solvent in capped HPLC vials at 25°C and 70°C for either 24 hours or 2 weeks. The XRPD patterns of the resulting solids were acquired with either a Bruker D8 Discover or Bruker D8 Advance diffractometer. The results of neat Compound (VII) slurry experiments are listed in Table 12. Slurry Experiments Using Neat, Amorphous Compound (VII):

| Solvent System | Slurry 24 hours 25°C | Slurry 2 weeks 25°C | Slurry 24 hours 70°C | Slurry 2 weeks 70°C |
|---|---|---|---|---|
| Acetonitrile | Form XX | Form XX | Form XX | Form XX |
| Acetonitrile/$H_2O$ | Form XX | Form XX | Form XX | Form XX |
| IPAc | Form XX | Form XX | Form XX | Form XX |
| IPAc/$H_2O$ | Form L | Form L | Form XX | Form XX |
| MEK | Form XX | Form XX | Form XX | Form XX |
| MEK/$H_2O$ (99:1) | Form XX | Form XX | Form XX | Form XX |
| MEK/$H_2O$ (90:10) | Form E | Form E | Form XX | Form XX |
| MEK/$H_2O$ (80:20) | Form E | Form E | Form XX | Form XX |
| Water | Hydrate | Hydrate | Hydrate | Form XX |

A white powder was collected by filtration from 24 hour and 2 week slurry experiments conducted at 25°C and 70°C in ACN and ACN/water. XRPD analysis of the samples results in peaks consistent with neat crystalline Form XX. When neat amorphous Compound (VII) was slurried for 24 hours and 2 weeks in ACN and ACN/water at 25°C and 70°C neat Form XX was produced. A white powder was collected by filtration from 24 hour and 2 week slurry experiments conducted at 25°C and 70°C in IP Ac and IP Ac/water. XRPD analysis of the powder collected from the IP Ac and IP Ac/water samples slurried at 70°C and IP Ac slurried at 25°C results in peaks consistent with neat crystalline Form XX. When neat amorphous Compound (VII) was slurried for 24 hours and 2 weeks in IP Ac and IP Ac/water at 70°C and in IP Ac for 24 hours and 2 weeks at 25°C, neat Form XX was produced. The powder harvested from samples slurried in IP Ac/water at 25°C for 24 hours and 2 weeks resulted in the same unique XRPD pattern for these two samples, indicating the precipitation of isostructural forms. TGA of the precipitate showed a 14.1% weight loss upon heating to 200°C, attributable to loss of solvent with a further 0.8% weight loss between 200°C and 250°C (Figure 19 of WO2013/158121). The powder harvested at 24 hours and 2 weeks after slurrying neat amorphous Compound (VII) in IP Ac/water at 25°C and 70°C is determined to be a new form of Compound (VII) designated Form L ({drug7j}) (Figure 18 of WO2013/158121). Form L ({drug7j}) is likely a solvate based on the TGA results. The peak list for Form L ({drug7j}) is provided in Table 13. In some embodiments, Form L ({drug7j}) is characterized by one or more peaks in an XRPD spectrum selected from the peaks listed in Table 13. Form L ({drug7j}) Peak List:

| Peak Number | Angle (2-theta ± 0.2) | Intensity (%) |
|---|---|---|
| 1 | 7.9 | 63.6 |
| 2 | 10.0 | 77.1 |
| 3 | 10.4 | 100.0 |
| 4 | 10.9 | 57.5 |
| 5 | 12.1 | 94.1 |
| 6 | 13.2 | 36.0 |
| 7 | 13.8 | 41.1 |
| 8 | 14.8 | 52.9 |

(continued)

| Peak Number | Angle (2-theta ± 0.2) | Intensity (%) |
|---|---|---|
| 9 | 16.0 | 49.6 |
| 10 | 16.9 | 27.2 |
| 11 | 17.4 | 31.7 |
| 12 | 18.6 | 63.6 |
| 13 | 19.7 | 35.9 |
| 14 | 20.5 | 49.8 |
| 15 | 21.6 | 21.2 |
| 16 | 22.1 | 21.1 |
| 17 | 22.5 | 32.0 |
| 18 | 24.8 | 35.3 |
| 19 | 25.2 | 34.0 |

A white powder was collected by filtration from the 24 hour and 2 week slurry experiments conducted at 25°C and 70°C in MEK, MEK/water (99:1), MEK/water (90:10) and MEK/water (80:20). XRPD analysis of the powder collected from MEK/Water (90:10) and MEK/Water (80:20) samples at 25°C after 24 hours and 2 weeks yielded a powder pattern that matched the powder pattern of Form E ({drug7c}), indicating the precipitation of isostructural forms. The powder collected from MEK/water (90:10) and MEK/water (80:20) slurried at 70°C yielded XRPD patterns consistent with neat crystalline Form XX. Slurrying of neat amorphous Compound (VII) in MEK/water (90:10) and MEK/water (80:20) for 24 hours and 2 weeks produced Form XX at 70°C and Form E ({drug7c}) at 25°C. In contrast, the powder collected from slurrying neat amorphous Compound (VII) in the solvent systems with lower water content- MEK and MEK water (99:1)-produced only neat crystalline Form XX as determined from their XRPD patterns at 25°C and 70°C after 24 hours and 2 weeks. A white powder was collected by filtration from 24 hour and 2 week slurry experiments conducted at 25°C and 70°C in water. XRPD analysis of the powder collected from samples slurried at 25°C for 24 hours and 2 weeks in water resulted in powder patterns consistent with the known form of Hydrate A of Compound (VII). XRPD analysis of the powder collected from samples slurried at 70°C demonstrated Compound (VII), Hydrate A formation after 24 hours and Compound (VII), Form XX after 2 weeks. When neat amorphous Compound (VII) was slurried in water at 25°C, Hydrate A was formed. When it was slurried at 70°C, Hydrate A was observed after 24 hours and Form XX was observed after 2 weeks.

<u>Experiment 5:</u> Slurry Experiments with Compound (VII) Spray-Dried Dispersion in the Presence of HPMC-AS and SLS.

Spray dried dispersion containing amorphous Compound (VII), along with the polymeric additives HPMC-AS (Hovione) and sodium lauryl sulfate (SLS, Fischer Scientific), was used for the slurry experiments in ACN/water and IP Ac/water MEK/water solvent system in the methyl ethyl ketone/water solvent systems. The experiments were carried out in 1 mL of total solvent in capped HPLC vials at 25°C and 70°C for either 24 hours or 2 weeks. The XRPD patterns of the resulting solids were acquired with either a Bruker D8 Discover or Bruker D8 Advance diffractometer. The results of these slurry experiments with HPMC-AS and SLS are listed in Table 14. Slurry Experiments with Spray Dried Dispersion Containing Amorphous Compound (VII) in the Presence of the Polymeric Additives HPMC-AS and SLS:

| Solvent System | Slurry 24 hours 25°C | Slurry 2 weeks 25°C | Slurry 24 hours 70°C | Slurry 2 weeks 70°C |
|---|---|---|---|---|
| MEK | Form M | Form M | Form O | Form O |
| MEK/H2O (99:1) | Form M | Form M | Form O | Form XX |
| MEK/H2O (90:1.0) | Form N | Form N | Form P | Form XX |
| MEK/H2O (80;20) | Form N | Form N | Form P | Form Q |

A white powder was collected by filtration from the 24 hour and 2 week slurry experiments conducted at 25°C and 70°C in MEK, MEK/water (99:1), MEK/water (90:10) and MEK/water (80:20). Analysis of the powder collected from samples slurried in MEK and MEK/water (99:1) at 2 5°C for 24 hours and 2 weeks yielded the same unique XRPD pattern for all four samples, indicating the synthesis of isostructural forms (Figure 20 of WO2013/158121). TGA of the precipitate showed a 1.2% weight loss upon heating to 100°C and a further 11.3% weight loss upon further heating

between 100°C and 200°C, attributable to loss of solvent (Figure 21 of WO2013/158121). As such, the powder collected from samples slurried in MEK and MEK/water (99:1) at 25°C for 24 hours and 2 weeks is determined to be a new form of Compound (VII) designated Form M ({drug7k}). Form M ({drug7k}) is likely a solvate based on the TGA results. The peak list for Form M ({drug7k}) is provided in Table 15. In some embodiments, Form M ({drug7k}) is characterized by one or more peaks in an XRPD spectrum selected from the peaks listed in Table 15. Form M ({drug7k}) Peak List:

| Peak Number | Angle (2-theta ± 0.2) | Intensity (%) |
|---|---|---|
| 1 | 5.2 | 77.3 |
| 2 | 6.0 | 55.5 |
| 3 | 6.9 | 100.0 |
| 4 | 8.0 | 33.4 |
| 5 | 8.9 | 40.1 |
| 6 | 10.7 | 93.6 |
| 7 | 12.3 | 53.6 |
| 8 | 13.0 | 33.7 |
| 9 | 13.5 | 29.2 |
| 10 | 14.4 | 36.2 |
| 11 | 16.8 | 35.1 |
| 12 | 17.3 | 43.2 |
| 13 | 17.6 | 47.3 |
| 14 | 18.3 | 55.0 |
| 15 | 18.7 | 53.8 |
| 16 | 19.8 | 35.9 |
| 17 | 20.4 | 28.6 |
| 18 | 21.1 | 40.5 |
| 19 | 22.0 | 40.5 |
| 20 | 23.4 | 39.1 |

Increasing the water content in the solvent system to MEK/water (90: 10) and MEK/water (80:20) in slurries carried out at 25°C produced a different Form F ({drug7d})rom MEK and MEK/ Water (99: 1) as judged from XRPD analysis at 24 hour and 2 week time points. The same XRPD pattern was produced from MEK/water (90:10) and MEK/water (80:20) slurries after 24 hours and 2 weeks at 25°C, indicating the precipitation of new isostructural forms (Figure 22 of WO2013/158121). TGA of the precipitate resulted in a 1.0% weight loss upon heating to 75°C and a further 3.7% weight loss upon heating between 100°C and 200°C (Figure 23 of WO2013/158121). As a consequence, the powder collected from samples slurried in MEK/water (90:10) and MEK/water (80:20) at 25°C for 24 hours and 2 weeks was determined to be a new form of Compound (VII) designated Form N ({drug71}). Form N ({drug71}) is likely a solvate based on the TGA results. The peak list for Form N ({drug71}) is provided in Table 16. In some embodiments, Form N ({drug71}) is characterized by one or more peaks in an XRPD spectrum selected from the peaks listed in Table 16. Form N ({drug71}) Peak List:

| Peak Number | Angle (2-theta ± 0.2) | Intensity (%) |
|---|---|---|
| 1 | 6.4 | 20.4 |
| 2 | 7.6 | 26.5 |
| 3 | 8.2 | 56.8 |
| 4 | 9.1 | 27.2 |
| 5 | 9.7 | 63.3 |
| 6 | 10.2 | 47.3 |
| 7 | 10.6 | 100.0 |
| 8 | 11.5 | 48.2 |
| 9 | 12.4 | 41.6 |
| 10 | 14.1 | 18.4 |
| 11 | 15.4 | 23.1 |
| 12 | 16.6 | 49.2 |

(continued)

| Peak Number | Angle (2-theta ± 0.2) | Intensity (%) |
|---|---|---|
| 13 | 17.0 | 37.3 |
| 14 | 17.6 | 35.1 |
| 15 | 18.8 | 31.9 |
| 16 | 21.4 | 26.0 |
| 17 | 23.3 | 30.8 |
| 18 | 23.8 | 34.5 |
| 19 | 24.2 | 17.0 |
| 20 | 25.7 | 21.8 |

Slurry experiments conducted at 70°C on a spray dried dispersion containing amorphous Compound (VII) in the presence of HPMC-AS and SLS yielded different results from those conducted at 25°C, as provided in Table 12. Analysis of the powder diffraction patterns of spray dried dispersion containing amorphous Compound (VII) slurried in MEK at 70°C showed the same unique XRPD pattern at the 24 hour and 2 week time points, indicating precipitation of the same new form (Figure 24 of WO2013/158121). TGA of the precipitate showed a weight loss of 0.8% upon heating to 150°C and a further 2.0% weight loss upon heating between 150°C and 200°C (Figure 25 of WO2013/158121). The powder collected from the 24 hour and 2 week slurries at 70°C in MEK was determined to be a new form of Compound (VII) designated Form O ({drug7m}). The peak list for Form O ({drug7m}) is provided in Table 17. In some embodiments, Form O ({drug7m}) is characterized by one or more peaks in an XRPD spectrum selected from the peaks listed in Table 17. Form O ({drug7m}) Peak List

| Peak Number | Angle (2-theta ± 0.2) | Intensity (%) |
|---|---|---|
| 1 | 5.7 | 100.0 |
| 2 | 7.5 | 41.1 |
| 3 | 8.5 | 88.1 |
| 4 | 9.5 | 62.5 |
| 5 | 11.4 | 67.9 |
| 6 | 13.2 | 22.7 |
| 7 | 15.3 | 55.0 |
| 8 | 16.3 | 21.5 |
| 9 | 17.2 | 64.5 |
| 10 | 17.5 | 63.2 |
| 11 | 18.0 | 42.3 |
| 12 | 18.9 | 52.3 |
| 13 | 20.3 | 43.4 |
| 14 | 20.6 | 25.4 |
| 15 | 22.2 | 68.3 |
| 16 | 23.1 | 15.7 |
| 17 | 23.5 | 17.7 |
| 18 | 26.4 | 31.1 |

XRPD of a spray dried dispersion containing amorphous Compound (VII) with HPMC-AS and SLS slurried in MEK/water (99:1) at 70°C yielded a diffraction pattern consistent with Form O ({drug7m}) after 24 hours and a diffraction pattern consistent with Form XX after 2 weeks. XRPD of a spray dried dispersion containing amorphous Compound (VII) slurried in MEK/water (90:10) at 70°C yielded a unique diffraction pattern after 24 hours, indicating the synthesis of a new form of Compound (VII) (Figure 26 of WO2013/158121), designated Form P ({drug7n}) and a diffraction pattern consistent with Form XX after 2 weeks. TGA of Form P ({drug7n}) showed a 2.4% weight loss upon heating to 100°C with a further 4.7% weight loss upon heating between 100°C and 200°C, indicating that Form P ({drug7n}) is likely a solvate (Figure 27 of WO2013/158121). The peak list for Form P ({drug7n}) is provided in Table 18. In some embodiments,

Form P ({drug7n}) is characterized by one or more peaks in an XRPD spectrum selected from the peaks listed in Table 18. Form P ({drug7n}) Peak List:

| Peak Number | Angle (2-theta ± 0.2) | Intensity (%) |
|---|---|---|
| 1 | 6.1 | 38.9 |
| 2 | 7.4 | 27.9 |
| 3 | 8.2 | 81.7 |
| 4 | 9.1 | 26.4 |
| 5 | 9.7 | 21.9 |
| 6 | 10.6 | 23.8 |
| 7 | 10.9 | 23.8 |
| 8 | 11.5 | 34.1 |
| 9 | 12.3 | 100.0 |
| 10 | 16.1 | 19.7 |
| 11 | 16.7 | 57.9 |
| 12 | 17.0 | 45.2 |
| 13 | 17.7 | 30.9 |
| 14 | 18.9 | 16.0 |
| 15 | 21.9 | 16.7 |
| 16 | 23.3 | 12.7 |
| 17 | 23.8 | 11.9 |
| 18 | 24.9 | 10.3 |
| 19 | 25.8 | 16.2 |

XRPD of a spray dried dispersion containing amorphous Compound (VII) with HPMC-AS and SLS slurried in MEK/water (80:20) at 70°C for 24 hours yielded a diffraction pattern consistent with Form P ({drug7n}) and a unique diffraction pattern after 2 weeks (Figure 28 of WO2013/158121), indicating the synthesis of a new form of Compound (VII) designated Form Q ({drug7o}). TGA of Form Q ({drug7o}) showed a 1.1% weight loss upon heating to 100°C, with a further 2.4% weight loss upon heating between 100°C and 200°C, indicating that Form Q ({drug7o}) is likely a solvate (Figure 29 of WO2013/158121). The peak list for Form Q ({drug7o}) is provided in Table 19. In some embodiments, Form Q ({drug7o}) is characterized by one or more peaks in an XRPD spectrum selected from the peaks listed in Table 19. Form Q ({drug7o}) Peak List:

| Peak Number | Angle (2-theta ± 0.2) | Intensity (%) |
|---|---|---|
| 1 | 6.3 | 34.6 |
| 2 | 7.6 | 100.0 |
| 3 | 8.4 | 25.9 |
| 4 | 11.1 | 15.4 |
| 5 | 11.7 | 9.4 |
| 6 | 12.5 | 79.0 |
| 7 | 14.7 | 14.5 |
| 8 | 16.4 | 14.8 |
| 9 | 16.9 | 21.5 |
| 10 | 17.3 | 12.9 |
| 11 | 18.0 | 79.6 |
| 12 | 18.8 | 11.0 |
| 13 | 20.6 | 11.8 |
| 14 | 21.0 | 13.0 |
| 15 | 22.2 | 11.1 |
| 16 | 24.1 | 10.7 |
| 17 | 25.1 | 11.7 |
| 18 | 27.4 | 8.4 |

Experiment 6: Desolvation: New forms of Compound (VII) obtained in the polymorph screen suspected of being solvates were subjected to further form investigation through attempted desolvation. Forms D, E, F, G, H, I, J, K, L and Hydrate A were left to stand open to the atmosphere under ambient conditions and or were placed in a vacuum oven. Forms E, F, I, J and Hydrate A yielded the same XRPD patterns after standing under ambient conditions and after sitting in the vacuum oven. Form G ({drug7e}) became x-ray amorphous after standing in the vacuum oven for one month. Form H ({drug7f}) produced the same Form H ({drug7f}) pattern after standing under ambient conditions for one month but converted to a semi-crystalline form after standing in the vacuum oven for one month. Form L ({drug7j}) produced the same Form L ({drug7j}) pattern after standing under ambient conditions for two weeks but converted to x-ray amorphous after standing in the vacuum oven for 24 hours. Forms D and K produced new crystalline XRPD patterns following desolvation. XRPD analysis of Form D ({drug7b}), after standing under ambient conditions and standing in the vacuum oven for approximately 1 month, showed a unique diffraction pattern, indicating that Form D ({drug7b}) had converted to a new crystalline form of Compound (VII) designated Form R ({drug7p}) (Figure 30 of WO2013/158121). TGA of Form R ({drug7p}) showed a 2.1% weight loss upon heating to 200°C, indicating that Form R ({drug7p}) had retained some solvent (Figure 31 of WO2013/158121). The peak list for Form R ({drug7p}) is provided in Table 20. In some embodiments, Form R ({drug7p}) is characterized by one or more peaks in an XRPD spectrum selected from the peaks listed in Table 20. Form R ({drug7p}) Peak List:

| Peak Number | Angle (2-theta ± 0.2) | Intensity (%) |
|---|---|---|
| 1 | 6.7 | 100.0 |
| 2 | 7.7 | 15.6 |
| 3 | 13.2 | 23.0 |
| 4 | 15.1 | 16.3 |
| 5 | 17.8 | 13.9 |

XRPD analysis of Form K ({drug7i}) after standing under ambient conditions and standing in the vacuum oven for 2 months showed a unique diffraction pattern, indicating that Form K ({drug7i}) had converted to a new crystalline form of Compound (VII) designated Form S ({drug7q}) (Figure 32 of WO2013/158121). TGA of Form S ({drug7q}) showed three separate weight losses. Form S ({drug7q}) underwent a 1.2% weight loss upon heating to 150°C and a further 0.7% weight loss upon heating between 150°C and 200°C, and finally a 1.0% weight loss upon heating between 200°C and 250°C, indicating that Form S ({drug7q}) has retained some solvent (Figure 33 of WO2013/158121). The peak list for Form S ({drug7q}) is provided in Table 21. In some embodiments, Form S ({drug7q}) is characterized by one or more peaks in an XRPDspectrum selected from the peaks listed in Table 21. Form S ({drug7q}) Peak List:

| Peak Number | Angle (2-theta ± 0.2) | Intensity (%) |
|---|---|---|
| 1.0 | 4.8 | 78.1 |
| 2.0 | 5.7 | 7.9 |
| 3.0 | 7.7 | 13.7 |
| 4.0 | 8.2 | 46.7 |
| 5.0 | 8.6 | 11.3 |
| 6.0 | 9.6 | 100.0 |
| 7.0 | 11.3 | 10.4 |
| 8.0 | 11.6 | 35.4 |
| 9.0 | 12.3 | 5.9 |
| 10.0 | 13.9 | 8.7 |
| 11.0 | 15.1 | 11.0 |
| 12.0 | 15.5 | 8.1 |
| 13.0 | 15.9 | 5.4 |
| 14.0 | 16.5 | 6.6 |
| 15.0 | 17.6 | 9.8 |
| 16.0 | 18.3 | 14.6 |
| 17.0 | 19.4 | 8.3 |
| 18.0 | 19.7 | 8.8 |
| 19.0 | 20.7 | 6.3 |
| 20.0 | 23.6 | 11.6 |

Hydrate B ({drug7t}) is a hydrated form of Compound (VII) made from a slurry of amorphous Compound (VII) in simulated fluids (FeSSIF). The XRPD pattern for Hydrate B ({drug7t}) is given inFigure 36 of WO2013/158121. A thermogravimetric analysis spectrum (TGA) of Hydrate B ({drug7t}) is shown in Figure 37 of WO2013/158121. The peak list for Hydrate B ({drug7t}) is provided in Table 22. In some embodiments, Hydrate B ({drug7t}) is characterized by one or more peaks in an XRPD spectrum selected from the peaks listed in Table 22. Hydrate B ({drug7t}) Peak List

| Peak Number | Angle (2-theta $\pm$ 0.2) | Intensity (%) |
|---|---|---|
| 1 | 4.9 | 100.0 |
| 2 | 6.0 | 25.6 |
| 3 | 7.2 | 12.9 |
| 4 | 8.9 | 15.2 |
| 5 | 10.1 | 12.8 |
| 6 | 10.7 | 11.5 |
| 7 | 11.3 | 11.5 |
| 8 | 11.9 | 26.2 |

Form W ({drug7s}) is a solvated Form from 10% water/ 90% acetonitrile. Form W ({drug7s}) is prepared by crystallization/ slurry conversion of Compound (VII), Form XX in 90% acetonitrile/ 10% water below 10 degrees Celsius. The XRPD pattern for Form W ({drug7s}) is shown in Figure 38 of WO2013/158121. A thermogravimetric analysis spectrum (TGA) of Form W ({drug7s}) overlayed with a Differential Scanning Calorimetry plot (DSC) of Form W ({drug7s}) is shown in Figure 39 of WO2013/158121. The peak list for Form W ({drug7s}) is provided in Table 23. Form W ({drug7s}) Peak List

| Peak Number | Angle (2-theta $\pm$ 0.2) | Intensity (%) |
|---|---|---|
| 1 | 5.5 | 100.0 |
| 2 | 5.9 | 5.4 |
| 3 | 7.6 | 4.6 |
| 4 | 10.9 | 5.3 |
| 5 | 12.1 | 7.0 |
| 6 | 13.3 | 6.2 |
| 7 | 14.6 | 7.2 |
| 8 | 17.2 | 5.3 |
| 9 | 18.8 | 4.9 |
| 10 | 21.8 | 4.7 |
| 11 | 22.5 | 4.5 |
| 12 | 23.2 | 4.3 |
| 13 | 23.7 | 4.3 |
| 14 | 25.0 | 4.7 |
| 15 | 25.6 | 4.6 |

SINGLE CRYSTAL ANALYSIS OF FORM T: Single crystal analysis was done on a crystal of Form T ({drug7r}) as described above. The unit cell dimensions and space group were found to be: Space Group: C2/c, Cell Lengths: a = 32.5478(13), b = 22.5036(9), c = 22.5540(9), Cell Angles: a = 90.00, $\beta$ = 112.725(2), $\gamma$ = 90.00, Cell Volume: 15237.1 A ASSAYS: A. PROTOCL 1: Assays for Detecting and Measuring AF508-CFTR Potentiation Properties of Compounds: Membrane potential optical methods for assaying AF508-CFTR modulation properties of compounds: The assay utilizes fluorescent voltage sensing dyes to measure changes in membrane potential using a fluorescent plate reader (e.g., FLIPR III, Molecular Devices, Inc.) as a readout for increase in functional AF508-CFTR in NIH 3T3 cells. The driving force for the response is the creation of a chloride ion gradient in conjunction with channel activation by a single liquid addition step after the cells have previously been treated with compounds and subsequently loaded with a voltage sensing dye. Identification of Potentiator Compounds: To identify potentiators of AF508-CFTR, a double-addition HTS assay format was developed. This HTS assay utilizes fluorescent voltage sensing dyes to measure changes in membrane potential on the FLIPR III as a measurement for increase in gating (conductance) of AF508 CFTR in temperature-corrected AF508 CFTR NIH 3T3 cells. The driving force for the response is a CI" ion gradient in conjunction with channel activation with forskolin in a single liquid addition step using a fluorescent plate reader such as FLIPR III after the cells

have previously been treated with potentiator compounds (or DMSO vehicle control) and subsequently loaded with a redistribution dye. Solutions: Bath Solution #1: (in mM) NaCl 160, KCl 4.5, CaCl2 2, MgC12 1, HEPES 10, pH 7.4 with NaOH. Chloride-free bath solution: Chloride salts in Bath Solution #1 (above) are substituted with gluconate salts. Cell Culture: NIH3T3 mouse fibroblasts stably expressing AF508-CFTR are used for optical measurements of membrane potential. The cells are maintained at 37°C in 5% C02 and 90 % humidity in Dulbecco's modified Eagle's medium supplemented with 2 mM glutamine, 10 % fetal bovine serum, 1 X NEAA, β-ME, 1 X pen/strep, and 25 mM HEPES in 175 cm2 culture flasks. For all optical assays, the cells were seeded at-20,000/well in 384- well matrigel-coated plates and cultured for 2 hrs at 37°C before culturing at 27°C for 24 hrs for the potentiator assay. For the correction assays, the cells are cultured at 27°C or 37°C with and without compounds for 16 - 24 hours. Electrophysiological Assays for assaying AF508-CFTR modulation properties of compounds. Ussing Chamber Assay: Ussing chamber experiments were performed on polarized airway epithelial cells expressing AF508-CFTR to further characterize the AF508-CFTR modulators identified in the optical assays. Non-CF and CF airway epithelia were isolated from bronchial tissue, cultured as previously described (Galietta, L.J.V., Lantero, S., Gazzolo, A., Sacco, O., Romano, L., Rossi, G.A., & Zegarra-Moran, O. (1998) In Vitro Cell. Dev. Biol. 34,478-481), and plated onto Costar® Snapwell™ filters that were precoated with NIH3T3 -conditioned media. After four days the apical media was removed and the cells were grown at an air liquid interface for >14 days prior to use. This resulted in a monolayer of fully differentiated columnar cells that were ciliated, features that are characteristic of airway epithelia. Non-CF HBE were isolated from non-smokers that did not have any known lung disease. CF-HBE were isolated from patients homozygous for AF508-CFTR. HBE grown on Costar® Snapwell™ cell culture inserts were mounted in an Ussing chamber (Physiologic Instruments, Inc., San Diego, CA), and the transepithelial resistance and short-circuit current in the presence of a basolateral to apical Cl" gradient (Isc) were measured using a voltage-clamp system (Department of Bioengineering, University of Iowa, IA). Briefly, HBE were examined under voltage-clamp recording conditions (Vh0id = 0 mV) at 37°C. The basolateral solution contained (in mM) 145 NaCl, 0.83 K2HP04, 3.3 KH2P04, 1.2 MgCl2, 1.2 CaCl2, 10 Glucose, 10 HEPES (pH adjusted to 7.35 with NaOH) and the apical solution contained (in mM) 145 NaGluconate, 1.2 MgCl2, 1.2 CaCl2, 10 glucose, 10 HEPES (pH adjusted to 7.35 with NaOH). Identification of Potentiator Compounds: Typical protocol utilized a basolateral to apical membrane Cl" concentration gradient. To set up this gradient, normal ringers was used on the basolateral membrane, whereas apical NaCl was replaced by equimolar sodium gluconate (titrated to pH 7.4 with NaOH) to give a large Cl" concentration gradient across the epithelium. Forskolin (10 μM) and all test compounds were added to the apical side of the cell culture inserts. The efficacy of the putative AF508-CFTR potentiators was compared to that of the known potentiator, genistein. Patch-clamp Recordings: Total Cl" current in AF508-NIH3T3 cells was monitored using the perforated-patch recording configuration as previously described (Rae, J., Cooper, K., Gates, P., & Watsky, M. (1991) J. Neurosci. Methods 37, 15-26). Voltage-clamp recordings were performed at 22°C using an Axopatch 200B patch-clamp amplifier (Axon Instruments Inc., Foster City, CA). The pipette solution contained (in mM) 150 N-methyl-D-glucamine (NMDG)-Cl, 2 MgCl2, 2 CaCl2, 10 EGTA, 10 HEPES, and 240 μg/mL amphotericin-B (pH adjusted to 7.35 with HCl). The extracellular medium contained (in mM) 150 NMDG-C1, 2 MgCl2, 2 CaCl2, 10 HEPES (pH adjusted to 7.35 with HCl). Pulse generation, data acquisition, and analysis were performed using a PC equipped with a Digidata 1320 AID interface in conjunction with Clampex 8 (Axon Instruments Inc.). To activate AF508-CFTR, 10 μM forskolin and 20 μM genistein were added to the bath and the current-voltage relation was monitored every 30 sec. Identification of Potentiator Compounds: The ability of AF508-CFTR potentiators to increase the macroscopic AF508-CFTR Cl" current (IAFSOS) in NIH3T3 cells stably expressing AF508-CFTR was also investigated using perforated-patch-recording techniques. The potentiators identified from the optical assays evoked a dose-dependent increase in IApsos with similar potency and efficacy observed in the optical assays. In all cells examined, the reversal potential before and during potentiator application was around -30 mV, which is the calculated Eci (-28 mV). Cell Culture:NIH3T3 mouse fibroblasts stably expressing AF508-CFTR are used for whole-cell recordings. The cells are maintained at 37°C in 5% C02 and 90 % humidity in Dulbecco's modified Eagle's medium supplemented with 2 mM glutamine, 10 % fetal bovine serum, 1 X NEAA, β-ME, 1 X pen/strep, and 25 mM HEPES in 175 cm2 culture flasks. For whole-cell recordings, 2,500 - 5,000 cells were seeded on poly-L-lysine-coated glass coverslips and cultured for 24 - 48 hrs at 27°C before use to test the activity of potentiators; and incubated with or without the correction compound at 37°C for measuring the activity of correctors. Single-channel recordings: Gating activity of wt-CFTR and temperature-corrected AF508-CFTR expressed in NIH3T3 cells was observed using excised inside-out membrane patch recordings as previously described (Dalemans, W., Barbry, P., Champigny, G., Jallat, S., Dott, K., Dreyer, D., Crystal, R.G., Pavirani, A., Lecocq, J-P., Lazdunski, M. (1991) Nature 354, 526 - 528) using an Axopatch 200B patch-clamp amplifier (Axon Instruments Inc.). The pipette contained (in mM): 150 NMDG, 150 aspartic acid, 5 CaCl2, 2 MgCl2, and 10 HEPES (pH adjusted to 7.35 with Tris base). The bath contained (in mM): 150 NMDG-C1, 2 MgCl2, 5 EGTA, 10 TES, and 14 Tris base (pH adjusted to 7.35 with HCl). After excision, both wt- and AF508-CFTR were activated by adding 1 mM Mg-ATP, 75 nM of the catalytic subunit of cAMP-dependent protein kinase (PKA; Promega Corp. Madison, WI), and 10 mM NaF to inhibit protein phosphatases, which prevented current rundown. The pipette potential was maintained at 80 mV. Channel activity was analyzed from membrane patches containing < 2 active channels. The maximum number of simultaneous openings determined the number of active channels during the

course of an experiment. To determine the single-channel current amplitude, the data recorded from 120 sec of AF508-CFTR activity was filtered "off-line" at 100 Hz and then used to construct all-point amplitude histograms that were fitted with multigaussian functions using Bio-Patch Analysis software (Bio-Logic Comp. France). The total microscopic current and open probability (P0) were determined from 120 sec of channel activity. The P0 was determined using the Bio-Patch software or from the relationship P0 = I i(N), where I = mean current, i = single-channel current amplitude, and N = number of active channels in patch. Cell Culture: NIH3T3 mouse fibroblasts stably expressing AF508-CFTR are used for excised-membrane patch-clamp recordings. The cells are maintained at 37°C in 5% C02 and 90 % humidity in Dulbecco's modified Eagle's medium supplemented with 2 mM glutamine, 10 % fetal bovine serum, 1 X NEAA, β-ME, 1 X pen/strep, and 25 mM HEPES in 175 cm2 culture flasks. For single channel recordings, 2,500 - 5,000 cells were seeded on poly-L-lysine-coated glass coverslips and cultured for 24 - 48 hrs at 27°C before use.

Activity of the Compound (VII): Compounds of the invention are useful as modulators of ATP binding cassette transporters. Table 1-3 below illustrates the EC50 and relative efficacy of certain embodiments in Table 1. In Table 1-3 below, the following meanings apply. EC50: "+++" means <10 uM; "++" means between lOuM to 25 uM; "+" means between 25 uM to 60uM. % Efficacy: "+" means < 25%; "++" means between 25% to 100%; "+++" means > 100%. TABLE 1-3:

| Cmpd # | EC50 (um) | % Activity |
|---|---|---|
| 1 | +++ | ++ |

B. PROTOCOL 2: Assays for Detecting and Measuring AF508-CFTR Potentiation Properties of Compounds: Membrane potential optical methods for assaying AF508-CFTR modulation properties of compounds: The assay utilizes fluorescent voltage sensing dyes to measure changes in membrane potential using a fluorescent plate reader (e.g., FLIPR III, Molecular Devices, Inc.) as a readout for increase in functional AF508-CFTR in NIH 3T3 cells. The driving force for the response is the creation of a chloride ion gradient in conjunction with channel activation by a single liquid addition step after the cells have previously been treated with compounds and subsequently loaded with a voltage sensing dye. Identification of Potentiator Compounds: To identify potentiators of AF508-CFTR, a double-addition HTS assay format was developed. This HTS assay utilizes fluorescent voltage sensing dyes to measure changes in membrane potential on the FLIPR III as a measurement for increase in gating (conductance) of AF508 CFTR in temperature-corrected AF508 CFTR NIH 3T3 cells. The driving force for the response is a Cl" ion gradient in conjunction with channel activation with forskolin in a single liquid addition step using a fluorescent plate reader such as FLIPR III after the cells have previously been treated with potentiator compounds (or DMSO vehicle control) and subsequently loaded with a redistribution dye. Solutions: Bath Solution #1 : (in mM) NaCl 160, KCl 4.5, CaCl2 2, MgCl2 1, HEPES 10, pH 7.4 with NaOH. Chloride-free bath solution: Chloride salts in Bath Solution #1 (above) are substituted with gluconate salts. Cell Culture: NIH3T3 mouse fibroblasts stably expressing AF508-CFTR are used for optical measurements of membrane potential. The cells are maintained at 37°C in 5% C02 and 90 % humidity in Dulbecco's modified Eagle's medium supplemented with 2 mM glutamine, 10 % fetal bovine serum, 1 X NEAA, β-ME, 1 X pen/strep, and 25 mM HEPES in 175 cm2 culture flasks. For all optical assays, the cells were seeded at -20,000/well in 384-well matrigel-coated plates and cultured for 2 hrs at 37°C before culturing at 27°C for 24 hrs. for the potentiator assay. For the correction assays, the cells are cultured at 27°C or 37°C with and without compounds for 16 - 24 hours. Electrophysiological Assays for assaying AF508-CFTR modulation properties of compounds. Ussing Chamber Assay: Ussing chamber experiments were performed on polarized airway epithelial cells expressing AF508-CFTR to further characterize the AF508-CFTR modulators identified in the optical assays. Non-CF and CF airway epithelia were isolated from bronchial tissue, cultured as previously described (Galietta, L.J.V., Lantero, S., Gazzolo, A., Sacco, O., Romano, L., Rossi, G.A., & Zegarra-Moran, O. (1998) In Vitro Cell. Dev. Biol. 34,478-481), and plated onto Costar® Snapwell™ filters that were precoated with NIH3T3-conditioned media. After four days the apical media was removed and the cells were grown at an air liquid interface for >14 days prior to use. This resulted in a monolayer of fully differentiated columnar cells that were ciliated, features that are characteristic of airway epithelia. Non-CF HBE were isolated from non-smokers that did not have any known lung disease. CF-HBE were isolated from patients homozygous for AF508-CFTR. HBE grown on Costar® Snapwell™ cell culture inserts were mounted in an Ussing chamber (Physiologic Instruments, Inc., San Diego, CA), and the transepithelial resistance and short-circuit current in the presence of a basolateral to apical Cl" gradient (Isc) were measured using a voltage-clamp system (Department of Bioengineering, University of Iowa, IA). Briefly, HBE were examined under voltage-clamp recording conditions (Vhoid = 0 mV) at 37°C. The basolateral solution contained (in mM) 145 NaCl, 0.83 K2HP04, 3.3 KH2P04, 1.2 MgCl2, 1.2 CaCl2, 10 Glucose, 10 HEPES (pH adjusted to 7.35 with NaOH) and the apical solution contained (in mM) 145 NaGluconate, 1.2 MgCl2, 1.2 CaCl2, 10 glucose, 10 HEPES (pH adjusted to 7.35 with NaOH). Identification of Potentiator Compounds: Typical protocol utilized a basolateral to apical membrane Cl" concentration gradient. To set up this gradient, normal ringers was used on the basolateral membrane, whereas

apical NaCl was replaced by equimolar sodium gluconate (titrated to pH 7.4 with NaOH) to give a large CI" concentration gradient across the epithelium. Forskolin (10 μM) and all test compounds were added to the apical side of the cell culture inserts. The efficacy of the putative AF508-CFTR potentiators was compared to that of the known potentiator, genistein. Patch-clamp Recordings: Total CI" current in AF508-NIH3T3 cells was monitored using the perforated-patch recording configuration as previously described (Rae, J., Cooper, K., Gates, P., & Watsky, M. (1991) J. Neurosci. Methods 37, 15-26). Voltage-clamp recordings were performed at 22°C using an Axopatch 200B patch-clamp amplifier (Axon Instruments Inc., Foster City, CA). The pipette solution contained (in mM) 150 N-methyl-D-glucamine (NMDG)-Cl, 2 MgCl2, 2 CaCl2, 10 EGTA, 10 HEPES, and 240 μ^ηύ, amphotericin-B (pH adjusted to 7.35 with HCl). The extracellular medium contained (in mM) 150 NMDG-C1, 2 MgCl2, 2 CaCl2, 10 HEPES (pH adjusted to 7.35 with HCl). Pulse generation, data acquisition, and analysis were performed using a PC equipped with a Digidata 1320 A/D interface in conjunction with Clampex 8 (Axon Instruments Inc.). To activate AF508-CFTR, 10 μM forskolin and 20 μM genistein were added to the bath and the current-voltage relation was monitored every 30 sec. Identification of Potentiator Compounds: The ability of AF508-CFTR potentiators to increase the macroscopic AF508-CFTR CI" current (IAFSOS) in NIH3T3 cells stably expressing AF508-CFTR was also investigated using perforated-patch-recording techniques. The potentiators identified from the optical assays evoked a dose-dependent increase in IApsos with similar potency and efficacy observed in the optical assays. In all cells examined, the reversal potential before and during potentiator application was around -30 mV, which is the calculated Eci (-28 mV). Cell Culture: NIH3T3 mouse fibroblasts stably expressing AF508-CFTR are used for whole-cell recordings. The cells are maintained at 37°C in 5% C02 and 90 % humidity in Dulbecco's modified Eagle's medium supplemented with 2 mM glutamine, 10 % fetal bovine serum, 1 X NEAA, β-ME, 1 X pen/strep, and 25 mM HEPES in 175 cm2 culture flasks. For whole-cell recordings, 2,500 - 5,000 cells were seeded on poly-L-lysine-coated glass coverslips and cultured for 24 - 48 hrs at 27°C before use to test the activity of potentiators; and incubated with or without the correction compound at 37°C for measuring the activity of correctors. Single-channel recordings: Gating activity of wt-CFTR and temperature-corrected AF508-CFTR expressed in NIH3T3 cells was observed using excised inside-out membrane patch recordings as previously described (Dalemans, W., Barbry, P., Champigny, G., Jallat, S., Dott, K., Dreyer, D., Crystal, R.G., Pavirani, A., Lecocq, J-P., Lazdunski, M. (1991) Nature 354, 526 - 528) using an Axopatch 200B patch-clamp amplifier (Axon Instruments Inc.). The pipette contained (in mM): 150 NMDG, 150 aspartic acid, 5 CaCl2, 2 MgCl2, and 10 HEPES (pH adjusted to 7.35 with Tris base). The bath contained (in mM): 150 NMDG-C1, 2 MgCl2, 5 EGTA, 10 TES, and 14 Tris base (pH adjusted to 7.35 with HCl). After excision, both wt- and AF508-CFTR were activated by adding 1 mM Mg-ATP, 75 nM of the catalytic subunit of cAMP-dependent protein kinase (PKA; Promega Corp. Madison, WI), and 10 mM NaF to inhibit protein phosphatases, which prevented current rundown. The pipette potential was maintained at 80 mV. Channel activity was analyzed from membrane patches containing < 2 active channels. The maximum number of simultaneous openings determined the number of active channels during the course of an experiment. To determine the single-channel current amplitude, the data recorded from 120 sec of AF508-CFTR activity was filtered "off-line" at 100 Hz and then used to construct all-point amplitude histograms that were fitted with multigaussian functions using Bio-Patch Analysis software (Bio-Logic Comp. France). The total microscopic current and open probability (P0) were determined from 120 sec of channel activity. The P0 was determined using the Bio-Patch software or from the relationship P0 = I i(N), where I = mean current, i = single-channel current amplitude, and N = number of active channels in patch. Cell Culture: NIH3T3 mouse fibroblasts stably expressing AF508-CFTR are used for excised-membrane patch-clamp recordings. The cells are maintained at 37°C in 5% C02 and 90 % humidity in Dulbecco's modified Eagle's medium supplemented with 2 mM glutamine, 10 % fetal bovine serum, 1 X NEAA, β-ME, 1 X pen/strep, and 25 mM HEPES in 175 cm2 culture flasks. For single channel recordings, 2,500 - 5,000 cells were seeded on poly-L-lysine-coated glass coverslips and cultured for 24 - 48 hrs at 27°C before use.

The following clauses 1 to 36 describe additional embodiments and/or subject matters of the present invention:

1. A process for making a crystalline solvate form of Compound (VII), selected from Form D ({drug7b}) or Form E ({drug7c}) comprising: a) dissolving Form XX of Compound (VII) in at least one solvent to form a mixture; b) heating the mixture until the solid has dissolved; c) precipitating the solid form by cooling the mixtures; and d) isolating the solid form.
2. The process of clause 1, wherein at least one of the solvents is acetonitrile.
3. The process of clause 1, wherein the solvent is a mixture of MEK and water, wherein the mixture is 99% MEK and 1% water, 90% MEK and 10% water, or 80% MEK and 20% water.
4. A process for making a crystalline solvate form of Compound (VII), selected from Form F ({drug7d}) or Form E ({drug7c}) comprising: a) slurrying Form XX of Compound (VII) in acetonitrile optionally containing water to form a mixture; b) removing residual solid from the mixture by filtration; c) removing the solvent by rapid evaporation; and d) isolating the solid form.
5. The process of clause 4, wherein the solvent is acetonitrile.
6. A process for making a crystalline solvate Form G ({drug7e}) of Compound (VII) comprising: a) slurrying Form XX of Compound (VII) in isopropyl acetate to form a mixture; b) removing the residual solid from the mixture by

filtration; c) removing the solvent by rapid evaporation; and d) isolating the Form G ({drug7e}).

7. A process for making a crystalline solvate Form H ({drug7f}) of Compound (VII) comprising: a) slurrying Form XX of Compound (VII) in isopropyl acetate containing water to form a mixture; b) removing the residual solid from the mixture by filtration; c) removing the solvent by rapid evaporation; and d) isolating the Form H ({drug7f}).

8. The process of clause 7, wherein, the solvent is isopropyl acetate/water 95/5.

9. A process for making a crystalline solvate Form I ({drug7g}) of Compound (VII) comprising: a) slurrying Form XX of Compound (VII) in methylethyl ketone to form a mixture; b) removing the residual solid from the mixture by filtration; c) removing the solvent by rapid evaporation; and d) isolating the Form I ({drug7g}).

10. A process for making a crystalline solvate Form J ({drug7h}) of Compound (VII) comprising: a) slurrying Form XX of Compound (VII) in methylethyl ketone containing water to form a mixture; b) removing the residual solid from the mixture by filtration; c) removing the solvent by rapid evaporation; and d) isolating the Form J ({drug7h}).

11. The process of clause 10, wherein the solvent comprises MEK in an amount of about 99% and water in an amount of about 1%, MEK in an amount of about 90% and water in an amount of about 10%, or MEK in an amount of about 80% and water in an amount of about 20%.

12. A process for making a crystalline solvate Form K ({drug7i}) of Compound (VII) comprising: a) slurrying Form XX of Compound (VII) in methylethyl ketone containing 0 percent to 30 percent water to form a mixture; b) removing the residual solid from the mixture by filtration; c) adding an antisolvent to the mixture to induce precipitation; and d) isolating the Form K({drug7i}).

13. The process of clause 12, wherein the antisolvent is hexane.

14. A process for making a crystalline solvate Form L ({drug7j}) of Compound (VII) comprising: a) slurrying amorphous Compound (VII) in isopropyl acetate containing water to form a mixture; b) removing the residual solid from the mixture by filtration; c) removing the solvent by rapid evaporation; and d) isolating the Form L ({drug7j}).

15. The process of clause 14, wherein the solvent comprises isopropyl acetate in an amount of about 95% and water in an amount of about 5%.

16. A process for making a crystalline solvate Form M ({drug7k}) of Compound (VII) comprising: a) at 25°C, slurrying amorphous Compound (VII) in methylethyl ketone containing 0 percent to 1 percent water for 12 hours to a month to form a mixture; b) removing the residual solid from the mixture by filtration; and c) isolating the Form M ({drug7k}).

17. The process of clause 16, wherein the mixture further comprises one or more polymeric additives selected from HPMC and sodium lauryl sulfate.

18. A process for making a crystalline solvate Form N ({drug71}) of Compound (VII) comprising: a) at 25°C, slurrying amorphous Compound (VII) in methylethyl ketone containing 10 percent to 20 percent water for 12 hours to a month to form a mixture; b) removing the residual solid from the mixture by filtration; and c) isolating the Form N ({drug71}).

19. The process of clause 18, wherein the mixture further comprises one or more polymeric additives selected from HPMC and sodium lauryl sulfate.

20. A. process for making a crystalline solvate Form O ({drug7m}) of Compound (VII) comprising: a) at 70°C, slurrying amorphous Compound (VII) in methylethyl ketone containing 0 percent to 1 percent water for 12 hours to 24 hours to form a mixture; b) heating the mixture at approximately 70°C for 1 to 30 hours; c) removing the residual solid from the mixture by filtration; and d) isolating the Form O ({drug7m}).

21. The process of clause 20, wherein the mixture further comprises one or more polymeric additives selected from HPMC and sodium lauryl sulfate.

22. A process for making a crystalline solvate Form P ({drug7n}) of Compound (VII) comprising: a) at 70°C, slurrying amorphous Compound (VII) in methylethyl ketone containing 10 percent to 20 percent water for 12 to 24 hours to form a mixture; b) heating the mixture at approximately 70°C for 1 to 30 hours; c) removing the residual solid from the mixture by filtration; and d) isolating the Form P ({drug7n}).

23. The process of clause 22, wherein the mixture further comprises one or more polymeric additives selected from HPMC and sodium lauryl sulfate.

24. A process for making a crystalline solvate Form Q ({drug7o}) of Compound (VII) comprising: a) at 70°C, slurrying amorphous Compound (VII) in methylethyl ketone containing 10 percent to 20 percent water for 2 days to 3 weeks to form a mixture; b) heating the mixture at approximately 70°C for 1 to 30 hours; c) removing the residual solid from the mixture by filtration; and d) isolating the Form Q ({drug7o}).

25. The process of clause 24, wherein the mixture further comprises one or more polymeric additives selected from HPMC and sodium lauryl sulfate.

26. A process for making a crystalline solvate Form R ({drug7p}) of Compound (VII) comprising: a) drying the Form D ({drug7b}) of Compound (VII) in a vacuum oven at a temperature between room temperature and 45°C; and b) isolating the Form R({drug7p}).

27. A process for making a crystalline solvate Form S ({drug7q}) of Compound (VII) comprising: a) drying Form K ({drug7i}) of Compound (VII) in a vacuum oven at a temperature between room temperature and 45°C; and d) isolating the Form S ({drug7q}).

28. A process for making a crystalline solvate Form T ({drug7r}) of Compound (VII) comprising: a) slurrying Form XX of Compound (VII) in methylethyl ketone containing 0 percent to 30 percent water to form a mixture; b) removing the residual solid from the mixture by filtration; c) adding an antisolvent to the mixture to induce precipitation; and d) isolating the Form T({drug7r}).
29. A crystalline solvate of Compound (VII), wherein the solvent is selected from the group consisting of acetonitrile, acetonitrile/water, isopropyl acetate, isopropyl acetate/water, methylethyl ketone, and methylethyl ketone/water.
30. The crystalline solvate of clause 29, wherein the solvent is acetonitrile or acetonitrile/water and the crystalline solvate is Form D ({drug7b}), Form F ({drug7d}), or Form R ({drug7p}),.
31. The crystalline solvate of clause 29, wherein the solvent is isopropyl acetate or isopropyl acetate/water and the crystalline solvate is Form G ({drug7e}), Form H ({drug7f}), Form L ({drug7j}), or Form T ({drug7r}).
32. The crystalline solvate of clause 29, wherein the solvent is methylethyl ketone or methylethyl ketone/water and the crystalline solvate is Form E ({drug7c}), Form I ({drug7g}), Form J ({drug7h}), Form K ({drug7i}), Form M ({drug7k}), Form N ({drug71}), Form O ({drug7m}), Form P ({drug7n}), or Form S ({drug7q}).
33. A kit for use in measuring the activity of CFTR or a fragment thereof in a biological sample in vitro or in vivo, comprising: (i) a composition comprising crystalline solvate Form D, E, F, G, H, I, J, K, L, M, N, O, P, Q, R, S, T, W, or Hydrate B, or combinations thereof, according to claim i ; (ii) instructions for: (a) contacting the composition with the biological sample; (b) measuring activity of said CFTR or a fragment thereof.
34. The kit of clause 33, further comprising instructions for: i. contacting an additional composition with the biological sample; ii. measuring the activity of said CFTR, or a fragment thereof, in the presence of said additional compound; and iii. comparing the activity of the CFTR, or fragment thereof, in the presence of the additional compound with the density of CFTR, or fragment thereof, in the presence of a crystalline solvate Form D, E, F, G, H, I, J, K, L, M, N, O, P, Q, R, S, T, W, or Hydrate B, or combination thereof.
35. The kit of clause 34, wherein the step of comparing the activity of said CFTR, or fragment thereof, provides a measure of the density of said CFTR, or fragment thereof. A process for making a crystalline solvate Form W ({drug7s}) of Compound (VII) comprising: a) slurrying Form XX of Compound (VII) in acetonitrile containing 10% water to form a mixture. b) removing the residual solid from the mixture by filtration; and c) isolating Form W ({drug7s}).
36. A process for making a crystalline solvate Hydrate B ({drug7t}) of Compound (VII) comprising: a) slurrying neat amorphous Compound (VII) in FeSSIF to form a mixture; b) removing the residual solid from the mixture by filtration; and c) isolating Hydrate B ({drug7t}).

The invention relates to an administration unit comprising crystalline solvates form of N-[2,4-bis(1,1-dimethylethyl)-5-hydroxyphenyl]-1,4-dihydro-4-oxoquinoline-3-carboxamide. The administration unit according to the invention is useful for treating various diseases and disorders which include but are not limited to cystic fibrosis, hereditary emphysema, COPD, or dry-eye disease.

SPECIFIC INORMATION CONCERNING ASPECT (VIII) OF THE INVENTION

Aspect (viii) of the invention relates to an administration unit comprising solid form of (S)-4-amino-N-(1-(4-chlorophenyl)-3-hydroxypropyl)-1-(7H-pyrrolo[2,3-d]pyrimidin-4-yl)piperidine-4-carboxamide. The invention relates to a form of (S)-4-amino-N-(1-(4-chlorophenyl)-3-hydroxypropyl)-1-(7H-pyrrolo[2,3-d]pyrimidin-4-yl)piperidine-4-carboxamide ({drug8}), namely to solid form of (S)-4-amino-N-(1-(4-chlorophenyl)-3-hydroxypropyl)-1-(7H-pyrrolo[2,3-d]pyrimidin-4-yl)piperidine-4-carboxamide ({drug8a}), which is useful for treating cancer, breast cancer, prostate cancer or gastric cancer. solid form of (S)-4-amino-N-(1-(4-chlorophenyl)-3-hydroxypropyl)-1-(7H-pyrrolo[2,3-d]pyrimidin-4-yl)piperidine-4-carboxamide is described in WO2013/156772, which is incorporated by reference. (S)-4-amino-N-(1-(4-chlorophenyl)-3-hydroxypropyl)-1-(7H-pyrrolo[2,3-d]pyrimidin-4-yl)piperidine-4-carboxamide can be described (cf. WO2013/156772 ) by "Compound (VIII)", and is alternatively known as "AZD5363".

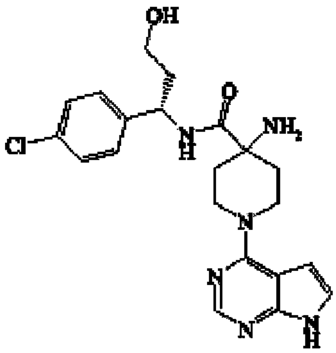

(VIII)

As used herein, solid form of (S)-4-amino-N-(1-(4-chlorophenyl)-3-hydroxypropyl)-1-(7H-pyrrolo[2,3-d]pyrimidin-4-yl)piperidine-4-carboxamide is a solid form of (S)-4-amino-N-(1-(4-chlorophenyl)-3-hydroxypropyl)-1-(7H-pyrrolo[2,3-d]pyrimidin-4-yl)piperidine-4-carboxamide, namely form B or form C, as characterized by / obtained preferred as described in WO2013/156772, which is cited hereinafter: The present invention discloses certain new solid state forms of (S)-4-amino-N-(1-(4-chlorophenyl)-3-hydroxypropyl)-1-(7H-pyrrolo[2,3-d]pyrimidin-4-yl)piperidine-4-carboxamide, processes for preparing such forms, pharmaceutical compositions comprising them, and the use of such forms in therapy.

In the formulation of drug compositions, it is important for the drug substance to be in a form in which it can be conveniently handled and processed. This is of importance, not only from the point of view of obtaining a commercially viable manufacturing process, but also from the point of view of subsequent manufacture of pharmaceutical formulations (e.g. oral dosage forms such as tablets) comprising the active compound. The different physical properties of the crystalline forms with respect to each other and with respect to the non-crystalline state may influence markedly the chemical and pharmaceutical processing of a compound, particularly when the compound is prepared or used on an industrial scale. Further, in the manufacture of oral drug compositions, it is important that a reliable and reproducible plasma concentration profile of drug is provided following administration to a patient. Inter-patient variability in the absorption profile of a drug within the stomach, intestine or bloodstream can have an effect on drug safety and efficacy. Chemical stability, solid state stability and "shelf life" of the active ingredients are also very important factors. The drug substance, and compositions containing it, should be capable of being effectively stored over appreciable periods of time, without exhibiting a significant change in the active component’ s physico-chemical characteristics (e.g. its chemical composition, density, hygroscopicity and solubility). Moreover, it is also important to be able to provide drug in a form which is as chemically pure as possible. Amorphous materials may present problems in this regard. For example, such materials are typically difficult to handle and to formulate, provide for unreliable solubility, and are often found to be unstable and chemically impure. The skilled person will appreciate that, if a drug can be readily obtained in a crystalline form that is also stable, one of more of the above problems may be solved. Thus, in the manufacture of commercially viable, and pharmaceutically acceptable, drug compositions, it is important, wherever possible, to provide drug in a crystalline, and stable, form. It is to be noted, however, that this goal is not always achievable. Indeed, typically, it is not possible to predict, from molecular structure alone, what the crystallisation behaviour of a compound (either alone or in the form of a salt) will be. This can only be determined empirically. WO2009/047563, teaches a novel group of bicyclic heterocycles which may be useful in the treatment or prevention of a disease or medical condition mediated through protein kinase B (PKB, also known as AKT). WO2009/047563 further discloses a specific bicyclic heterocycle identified therein as (S)-4-amino-N-(1-(4-chlorophenyl)-3-hydroxypropyl)-1-(7H-pyrrolo[2,3-d]pyrimidin-4-yl)piperidine-4-carboxamide (Example 9). This compound is designated herein as "Compound (VIII)", and is alternatively known as "AZD5363".

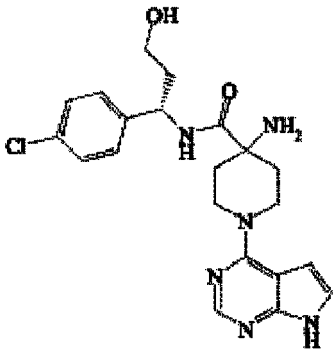

Compound (VIII)

Compound (VIII) has been shown to exhibit potent activity against all 3 mammalian isoforms of the AKT enzyme - with an IC50 of 3nM against AKT1, an IC50 of 7nM against AKT2 and an IC50 of 7nM against AKT3. Compound (VIII) is currently being developed as a potential new drug for the treatment of several different forms of cancer, either as a monotherapy or as part of a combination therapy. WO2009/047563 further discloses three proesses for the preparation of Compound (VIII) - Example 9 itself and alternative routes 1 and 2 for Example 9. "Example 9 alternative route 1" includes a slurry of Compound (VIII) in ethyl acetate and the other two processes isolate Compound (VIII) as a solid by evaporating fractions eluted from a column. Three historical batches of Compound (VIII) from a compound collection which were synthesised using a procedure identical to, or substantially similar to, one of these three processes have been analysed (by XRD) and identified all three as being a semi-crystalline form, designated as "Form A ({drug8b})". It has been found that Compound (VIII) can be obtained in a number of different solid forms. Two of these new solid forms are referred to hereafter as "Form B ({drug8c})" and "Form C ({drug8d})". Form B ({drug8c}) is crystalline and Form C ({drug8d}) is semi-crystalline in nature. Analysis by XRD has determined that these forms are new forms and that Form A ({drug8b}) did not contain any Form B ({drug8c}) or C material. Thus in the first aspect of the invention, there is provided Compound (VIII) in crystalline form. In an alternative aspect of the invention, Compound (VIII) in crystalline form is in the form of Form B ({drug8c}). By "crystalline" we include greater than 80% crystalline, particularly greater than 90%), and more particularly greater than 95%. Most preferably "crystalline" is greater than 98%). By "semi-crystalline" we include greater than 5% but less than 80%> crystalline. The degree (%) of crystallinity may be determined by the skilled person using X-ray powder diffraction (XRPD). Other techniques, such as solid state MR, FT-IR, Raman spectroscopy, differential scanning calorimetry (DSC) and microcalorimetry, may also be used. The crystalline form of the invention can have improved properties, for example stability, for example when compared with Compound (VIII) prepared as described in WO2009/047563. According to a further aspect of the invention, there is thus provided a stable crystalline form of Compound (VIII). In particular this stable crystalline form of Compound (VIII) is Form B ({drug8c}). It has been found that a slurry of Form A ({drug8b}) in a suitable organic solvent, for example acetonitrile stirred for an appropriate length of time, for example 3 days, yields Form B ({drug8c}). Form B ({drug8c}) can also be obtained by stirring a slurry of Form C ({drug8d}) in a suitable organic solvent such as acetone or isopropyl alcohol (IP A) for an appropriate amount of time. In view of these conversions over time of other Forms to Form B ({drug8c}), it has been concluded that Form B ({drug8c}) is most likely to be the thermodynamically most stable form. It is therefore predicted that Form B ({drug8c}) has beneficial properties when compared to other forms of Compound (VIII) for example in terms of its stability and therefore its propensity to convert or partially convert into other less desirable solid forms. This makes Form B ({drug8c}) potentially advantageous for assuring a longer product shelf-life and minimising any inter-patient variability and intra-patient variability of Compound (VIII) absorption. The term "stability" as defined herein includes chemical stability and/or solid state stability. By "chemical stability", we include that the respective compounds can be stored in an isolated form, or in the form of a formulation in which it is provided in admixture with pharmaceutically acceptable carriers, diluents or adjuvants (e.g. in an oral dosage form, such as tablet, capsule etc.), under normal storage conditions, with a limited degree of chemical degradation or decomposition. By "solid state stability", we include that the respective compounds can be stored in an isolated solid form, or in the form of a solid formulation in which it is provided in admixture with pharmaceutically acceptable carriers, diluents or adjuvants (e.g. in an oral dosage form, such as tablet, capsule etc.), under normal storage conditions, with an insignificant degree of solid state transformation (e.g. crystallisation, recrystallisation, solid state phase transition, hydration, dehydration, solvation or desolvation). Examples of "normal storage conditions" include temperatures of between minus 80°C and plus 50°C (particularly between 0°C and 40°C and more particularly room temperatures, such as 15°C to 30°C), pressures of between 0.1 and 2 bars (particularly at atmospheric pressure), relative humidities of between 5 and 95% (particularly 10 to 75%), and/or exposure to 460 lux of UV/visible light, for prolonged periods (i.e. greater than or equal to six months). Under such conditions, the crystalline forms of the invention may be found to be less than 15%, more particularly less than 10%, and especially less than 5%, chemically

degraded/decomposed, or solid state transformed, as appropriate. The skilled person will appreciate that the above-mentioned upper and lower limits for temperature, pressure and relative humidity represent extremes of normal storage conditions, and that certain combinations of these extremes will not be experienced during normal storage (e.g. a temperature of 50°C and a pressure of 0.1 bar). According to a further aspect of the invention, there is provided a process for the production of Compound (VIII) in crystalline form in the form of Form B ({drug8c}), which comprises stirring a slurry of Compound (VIII), particularly Compound (VIII) in the form of Form A ({drug8b}) or Form C ({drug8d}), more particularly Form A ({drug8b}), in a suitable solvent such as acetone or acetonitrile, particularly acetonitrile, followed by filtering and drying. In such a process it is important to leave the slurry to stir for a sufficient period of time in order to achieve optimum conversion to Form B ({drug8c}). The length of time may also depend on the temperature of the slurry. If the slurry is at 50°C acceptable conversion yields may be achieved if the reaction is stirred for at least 3 days. According to a further aspect, there is provided a process for the production of Compound (VIII) in the form of Form C ({drug8d}), which comprises stirring a slurry of Compound (VIII), particularly Compound (VIII) in the form of Form A ({drug8b}), in methanol followed by filtering and drying. In such a process it is important to leave the slurry to stir for a sufficient period of time in order to achieve full conversion to Form C ({drug8d}). The length of time may also depend on the temperature of the slurry. If the slurry is at room temperature acceptable conversion yields may be achieved if the reaction is stirred for at least 3 days. In an alternative aspect of the invention, Compound (VIII) in crystalline form is in the form of Form B ({drug8c}) and is substantially free of other Forms. In an alternative aspect of the invention, Compound (VIII) in crystalline form is in the form of Form B ({drug8c}) and is substantially free of Form A ({drug8b}). In an alternative aspect of the invention, Compound (VIII) in crystalline form is in the form of Form B ({drug8c}) and is substantially free of Form C ({drug8d}). In an alternative aspect of the invention, Compound (VIII) in crystalline form is in the form of Form B ({drug8c}) and is substantially free of Form A ({drug8b}) and Form C ({drug8d}). In an alternative aspect, Compound (VIII) is in the form of Form C ({drug8d}) and is substantially free of other Forms. In an alternative aspect, Compound (VIII) is in the form of Form C ({drug8d}) and is substantially free of Form A ({drug8b}). In an alternative aspect, Compound (VIII) is in the form of Form C ({drug8d}) and is substantially free of Form B ({drug8c}). In an alternative aspect, Compound (VIII) is in the form of Form C ({drug8d}) and is substantially free of Form A ({drug8b}) and Form B ({drug8c}). The term "substantially free" refers to less than 10% of another Form or Forms, preferably less than 5%. Further information on the processes of the invention and the products obtainable there from are described in the Examples herein. Crystalline forms of the invention may be isolated using techniques which are well known to those skilled in the art, for example decanting, filtering or centrifuging. Crystalline forms of the invention may be dried using standard techniques. It will be appreciated by the skilled person that drying temperature and drying time may affect the solid state properties of compounds that are in the form of solvates (e.g. desolvation may occur at certain temperatures and/or reduced pressure). The crystalline forms of the invention may be readily characterised using X-ray powder diffraction (XRPD) methods, for example as described hereinafter. Standard DSC and TGA techniques may also be used. ("TGA" = Thermogravimetric analysis). Forms A, B and C of Compound (VIII) can be distinguished by reference to their onset of melting, powder X-ray diffraction patterns and/or single crystal X-ray data. In all of the claims, aspects and embodiments recited herein the peaks of the X-ray diffraction patterns are measured using CuKa radiation (i.e. X-rays with 1.54A wavelength).

Form A ({drug8b}) of Compound (VIII) is characterised in providing an X-ray powder diffraction pattern substantially as shown in Figure 1 of WO2013/156772. Ten X-Ray powder diffraction peaks (obtained using 1.54A X-rays, i.e. CuKa radiation) are shown in Table A:

| **Angle (2θ):** | 14.3 | 3.1 | 19.6 | 18.9 | 23.9 | 25.9 | 15.5 | 16.5 | 17.4 | 9.2 |
|---|---|---|---|---|---|---|---|---|---|---|
| **Intensity (%):** | 100 | 39.7 | 67.2 | 64.9 | 55.2 | 49.4 | 40.8 | 28.2 | 32.2 | 17.8 |

Table A: Ten X-Ray Powder Diffraction peaks for Form A ({drug8b}) of Compound (VIII). Accordingly Form A ({drug8b}) of Compound (VIII) has an X-ray powder diffraction pattern substantially the same as the X-ray powder diffraction pattern shown in Figure 1 of WO2013/156772. Form A ({drug8b}) of Compound (VIII) provides X-ray powder diffraction patterns substantially the same as the X-ray powder diffraction patterns shown in Figure 1 of WO2013/156772and has ten peaks [angle 2-theta (20) values] shown in Table A. It will be understood that the 2-theta values of the X-ray powder diffraction pattern may vary slightly from one machine to another or from one sample to another, and so the values quoted are not to be construed as absolute. DSC analysis of Form A ({drug8b}) of Compound (VIII) shows a melting endotherm with an onset of 155.2°C (Figure 2 of WO2013/156772).

Form B ({drug8c}) of Compound (VIII): When Form B ({drug8c}) of Compound (VIII) was prepared by the method described hereinafter in 'Example and analysed using a Bruker D8 X-ray powder diffractometer, the X-ray powder diffraction pattern of Figure 3.1 of WO2013/156772was obtained (using 1.54A X-rays, i.e. CuKa radiation). Accordingly, Form B ({drug8c}) of Compound (VIII) may be characterised in providing at least one of the following 2Θ values measured using CuKa radiation: 15.0 and 19.2. Form B ({drug8c}) may be characterised in providing an X-ray powder diffraction

pattern substantially as shown in Figure 3.1 of WO2013/156772. Based on the X-ray diffraction pattern as shown in Figure 3.1 of WO2013/156772, ten X- Ray powder diffraction peaks (using 1.54A X-rays, i.e. CuKa radiation) relating to Form B ({drug8c}) are shown in Table B-1:

| Angle (2θ): | 15.0 | 19.2 | 12.3 | 10.0 | 17.1 | 124.4 | 16.4 | 26.0 | 15.5 | 23.9 |
|---|---|---|---|---|---|---|---|---|---|---|
| Intensity (%): | 100 | 57.7 | 54.7 | 36.0 | 32.7 | 31.0 | 22.1 | 21.8 | 21.2 | 19.0 |

Table B-1: Ten X-Ray Diffraction peaks for Form B ({drug8c}) (based on Figure 3.1 of WO2013/156772): When Form B ({drug8c}) of Compound (VIII) was prepared by the method described hereinafter in 'Example 3' and analysed using a

PANalytical CUB IX PRO X-ray powder diffractometer, where an improved signal-to-noise ratio was also achieved, the X-ray diffraction pattern of Figure 3.2 of WO2013/156772was obtained (using 1.54A X-rays, i.e. CuKa radiation). Accordingly, Form B ({drug8c}) may be characterised in providing an X-ray powder diffraction pattern substantially as shown in Figure 3.2 of WO2013/156772. Based on the X-ray diffraction pattern as shown in Figure 3.2 of WO2013/156772, ten X-ray powder diffraction peaks (using 1.54 A X-rays, i.e. CuKa radiation) relating to Form B ({drug8c}) are shown in Table B-2:

| Angle (2θ): | 10.0 | 5.0 | 15.0 | 19.2 | 17.1 | 12.3 | 24.4 | 30.2 | 32.3 | 23.3 |
|---|---|---|---|---|---|---|---|---|---|---|
| Intensity (%): | 100 | 57.8 | 48.7 | 24 | 14.1 | 13.8 | 12.4 | 11.4 | 9.8 | 8.2 |

Table B-2: Ten X-Ray Diffraction peaks for Form B ({drug8c}) (based on Figure 3.2 of WO2013/156772). According to the present invention there is provided a crystalline form, Form B ({drug8c}) of Compound (VIII), which has an X-ray powder diffraction pattern with at least one specific peak at about 2-theta = 15.0°. According to the present invention there is provided a crystalline form, Form B ({drug8c}) of Compound (VIII), which has an X-ray powder diffraction pattern with at least one specific peak at about 2-theta =19.2°. According to the present invention there is provided a crystalline form, Form B ({drug8c}) of Compound (VIII), which has an X-ray powder diffraction pattern with at least one specific peak at about 2-theta = 12.3°. According to the present invention there is provided a crystalline form, Form B ({drug8c}) of Compound (VIII), which has an X-ray powder diffraction pattern with at least two specific peaks at about 2-theta = 15.0° and 19.2°.

According to the present invention there is provided a crystalline form, Form B ({drug8c}) of Compound (VIII), which has an X-ray powder diffraction pattern with at least three specific peaks at about 2-theta =12.3°, 15.0° and 19.2°. According to the present invention there is provided a crystalline form, Form B ({drug8c}) of Compound (VIII), which has an X-ray powder diffraction pattern with specific peaks at about 2-theta = 10.0, 12.3, 15.0, 17.1, 19.2 and 24.4°. According to the present invention there is provided a crystalline form, Form B ({drug8c}) of Compound (VIII), which has an X-ray powder diffraction pattern with specific peaks at about 2-theta = 15.0, 19.2, 12.3, 10.0, 17.1, 24.4, 16.4, 26.0, 15.5 and 23.9°.

According to the present invention there is provided a crystalline form, Form B ({drug8c}) of Compound (VIII), which has an X-ray powder diffraction pattern with specific peaks at about 2-theta = 10.0, 5.0, 15.0, 19.2, 17.1, 12.3, 24.4, 30.2, 32.2 and 23.3°. According to the present invention there is provided crystalline form, Form B ({drug8c}) of Compound (VIII), which has an X-ray powder diffraction pattern substantially the same as the X-ray powder diffraction pattern shown in Figure 3.1 of WO2013/156772. According to the present invention there is provided crystalline form, Form B ({drug8c}) of Compound (VIII), which has an X-ray powder diffraction pattern substantially the same as the X-ray powder diffraction pattern shown in Figure 3.2 of WO2013/156772. According to the present invention there is provided crystalline form, Form B ({drug8c}) of Compound (VIII), which has an X-ray powder diffraction pattern with at least one specific peak at 2-theta = 15.0° plus or minus 0.2° 2-theta. According to the present invention there is provided a crystalline form, Form B ({drug8c}) of Compound (VIII), which has an X-ray powder diffraction pattern with at least one specific peak at 2-theta =19.2° plus or minus 0.2° 2-theta. According to the present invention there is provided a crystalline form, Form B ({drug8c}) of Compound (VIII), which has an X-ray powder diffraction pattern with at least one specific peak at 2-theta =12.3° plus or minus 0.2° 2-theta. According to the present invention there is provided a crystalline form, Form B ({drug8c}) of Compound (VIII), which has an X-ray powder diffraction pattern with at least two specific peaks at 2-theta =15.0° and 19.2° wherein said values may be plus or minus 0.2° 2-theta. According to the present invention there is provided a crystalline form, Form B ({drug8c}) of Compound (VIII), which has an X-ray powder diffraction pattern with at least three specific peaks at 2-theta = 12.3°, 15.0° and 19.2° wherein said values may be plus or minus 0.2° 2-theta. According to the present invention there is provided a crystalline form, Form B ({drug8c}) of Compound (VIII), which has an X-ray powder diffraction pattern with specific peaks at 2-theta = 10.0, 12.3, 15.0, 17.1, 19.2 and 24.4°

wherein said values may be plus or minus 0.2° 2-theta. According to the present invention there is provided a crystalline form, Form B ({drug8c}) of Compound (VIII), which has an X-ray powder diffraction pattern with specific peaks at 2-theta = 15.0, 19.2, 12.3, 10.0, 17.1, 24.4, 16.4, 26.0, 15.5 and 23.9° wherein said values may be plus or minus 0.2° 2-theta. According to the present invention there is provided a crystalline form, Form B ({drug8c}) of Compound (VIII), which has an X-ray powder diffraction pattern with specific peaks at 2-theta = 10.0, 5.0, 15.0, 19.2, 17.1, 12.3, 24.4, 30.2, 32.2 and 23.3° wherein said values may be plus or minus 0.2° 2-theta. According to the present invention there is provided a crystalline form, Form B ({drug8c}) of Compound (VIII), which has an X-ray powder diffraction pattern with at least one specific peak at 2-theta = 15.0°. According to the present invention there is provided a crystalline form, Form B ({drug8c}) of Compound (VIII), which has an X-ray powder diffraction pattern with at least one specific peak at 2-theta = 19.2°. According to the present invention there is provided a crystalline form, Form B ({drug8c}) of Compound (VIII), which has an X-ray powder diffraction pattern with at least one specific peak at 2-theta = 12.3°.

According to the present invention there is provided a crystalline form, Form B ({drug8c}) of Compound (VIII), which has an X-ray powder diffraction pattern with at least two specific peaks at 2-theta = 15.0° and 19.2°. According to the present invention there is provided a crystalline form, Form B ({drug8c}) of Compound (VIII), which has an X-ray powder diffraction pattern with at least three specific peaks at 2-theta = 12.3°, 15.0° and 19.2°. According to the present invention there is provided a crystalline form, Form B ({drug8c}) of Compound (VIII), which has an X-ray powder diffraction pattern with specific peaks at 2-theta = 10.0, 12.3, 15.0, 17.1, 19.2 and 24.4°. According to the present invention there is provided crystalline form, Form B ({drug8c}) of Compound (VIII), which has an X-ray powder diffraction pattern with specific peaks at 2-theta = 15.0, 19.2, 12.3, 10.0, 17.1, 24.4, 16.4, 26.0, 15.5 and 23.9°. According to the present invention there is provided a crystalline form, Form B ({drug8c}) of Compound (VIII), which has an X-ray powder diffraction pattern with specific peaks at 2-theta = 10.0, 5.0, 15.0, 19.2, 17.1, 12.3, 24.4, 30.2, 32.2 and 23.3°. According to the present invention there is provided crystalline form, Form B ({drug8c}) of Compound (VIII), which has an X-ray powder diffraction pattern as shown in Figure 3.1 of WO2013/156772. According to the present invention there is provided crystalline form, Form B ({drug8c}) of Compound (VIII), which has an X-ray powder diffraction pattern as shown in Figure 3.2 of WO2013/156772. DSC analysis of Form B ({drug8c}) of Compound (VIII) [prepared by the method of Example 1, below] shows a melting endotherm with an onset of 162.3°C and a peak at 167.1° C (Figure 4 of WO2013/156772). DSC analysis of Form B ({drug8c}) of Compound (VIII) [prepared by the method of Example 3, below] shows a melting endotherm with an onset of 168.5°C and a peak at 171.0°C (Figure 7 of WO2013/156772). Accordingly, the Form B ({drug8c}) prepared by the method of Example 1 has a slightly lower melting point from the Form B ({drug8c}) as prepared by Example 3. It is speculated that this small difference in melting point arises because the Form B ({drug8c}) as prepared by the method of Example 3 is even more highly crystalline than the Form B ({drug8c}) as produced by the method of Example 1. Thus DSC analysis shows Form B ({drug8c}) of Compound (VIII) may be a high melting solid with an onset of melting at about 162.3° C and a peak at about 167.1°C. Equally, DSC analysis shows Form B ({drug8c}) of Compound (VIII) may be a high melting solid with an onset of 168.5°C and a peak at 171.0°C. Accordingly, in any embodiment, aspect or claim herein, the Form B ({drug8c}) of Compound (VIII) has a melting point peak (as measured by DSC) within the range from 165°C to 173°C. Form B ({drug8c}) of Compound (VIII) provides X-ray powder diffraction patterns substantially the same as the X-ray powder diffraction patterns shown in Figures 3.1 and 3.2 of WO2013/156772and has the ten (angle 2-theta values) shown in Tables B-1 and B-2.

It will be understood that the 2- theta values of the X-ray powder diffraction pattern may vary slightly from one machine to another or from one sample to another, and so the values quoted are not to be construed as absolute. Indeed, such variation is evident in Figures 3.1 and 3.2 of WO2013/156772, and the corresponding Tables B-1 and B-2.

Form C ({drug8d}) of Compound (VIII) is characterised in providing at least one of the following 2Θ values measured using CuKa radiation: 23.2 and 16.2. Compound (VIII) Form C ({drug8d}) is characterised in providing an X-ray powder diffraction pattern, substantially as shown in Figure 5 of WO2013/156772. Ten X-Ray powder diffraction peaks (using 1.54A X-rays, i.e. CuKa radiation) are shown in Table C:

| **Angle (2θ):** | 23.2 | 16.2 | 15.2 | 11.6 | 24.1 | 19.3 | 17.5 | 21.7 | 20.5 | 25.0 |
|---|---|---|---|---|---|---|---|---|---|---|
| **Intensity (%):** | 100 | 67.9 | 53.1 | 51.2 | 49.8 | 45.2 | 43.2 | 41.8 | 39.9 | 34.1 |

Table C: Ten X-Ray Powder Diffraction peaks for Form C ({drug8d}) of Compound (VIII). Accordingly there is provided Form C ({drug8d}) of Compound (VIII), which has an X-ray powder diffraction pattern with at least one specific peak at about 2-theta = 23.2°. Accordingly there is provided Form C ({drug8d}) of Compound (VIII), which has an X-ray powder diffraction pattern with at least one specific peak at about 2-theta = 16.2°. Accordingly there is provided Form C ({drug8d}) of Compound (VIII), which has an X-ray powder diffraction pattern with at least two specific peaks at about 2-theta = 23.2° and 16.2°. Accordingly there is provided Form C ({drug8d}) of Compound (VIII), which has an X-ray powder diffraction pattern with specific peaks at about 2-theta = 23.2, 16.2, 15.2, 11.6, 24.1, 19.3, 17.5, 21.7, 20.5 and

25.0°. Accordingly there is provided Form C ({drug8d}) of Compound (VIII) which has an X-ray powder diffraction pattern substantially the same as the X-ray powder diffraction pattern shown in Figure 5 of WO2013/156772. Accordingly there is provided Form C ({drug8d}) of Compound (VIII), which has an X-ray powder diffraction pattern with at least one specific peak at 2-theta = 23.2° plus or minus 0.2° 2-theta. Accordingly there is provided Form C ({drug8d}) of Compound (VIII), which has an X-ray powder diffraction pattern with at least one specific peak at 2-theta = 16.2° plus or minus 0.2° 2-theta. Accordingly there is provided Form C ({drug8d}) of Compound (VIII), which has an X-ray powder diffraction pattern with at least two specific peaks at 2-theta = 23.2° and 16.2° wherein said values may be plus or minus 0.2° 2-theta. Accordingly there is provided Form C ({drug8d}) of Compound (VIII), which has an X-ray powder diffraction pattern with specific peaks at 2-theta = 23.2, 16.2, 15.2, 11.6, 24.1, 19.3, 17.5, 21.7, 20.5 and 25.0° wherein said values may be plus or minus 0.2° 2-theta. Accordingly there is provided Form C ({drug8d}) of Compound (VIII), which has an X-ray powder diffraction pattern with at least one specific peak at 2-theta = 23.2°. Accordingly there is provided Form C ({drug8d}) of Compound (VIII), which has an X-ray powder diffraction pattern with at least one specific peak at 2-theta = 16.2°. Accordingly there is provided Form C ({drug8d}) of Compound (VIII), which has an X-ray powder diffraction pattern with at least two specific peaks at 2-theta = 15.0° and 19.2°. Accordingly there is provided Form C ({drug8d}) of Compound (VIII), which has an X-ray powder diffraction pattern with specific peaks at 2-theta = 23.2, 16.2, 15.2, 11.6, 24.1, 19.3, 17.5, 21.7, 20.5 and 25.0°. Accordingly there is provided Form C ({drug8d}) of Compound (VIII), which has an X-ray powder diffraction pattern as shown in Figure 5 of WO2013/156772. DSC analysis of Form C ({drug8d}) of Compound (VIII) shows a broad endotherm with an onset at 41.7°C and a peak at 67.2°C followed by a subsequent sharp endotherm with an onset at 142.7°C and a peak at 149.2°C followed by a small endotherm with an onset of 161.6°C and a peak at 164.5° C (Figure 6 of WO2013/156772). Form C ({drug8d}) of Compound (VIII) provides X-ray powder diffraction patterns substantially the same as the X-ray powder diffraction patterns shown in Figure 5 of WO2013/156772and has the ten (angle 2-theta values) shown in Table C. It will be understood that the 2-theta values of the X-ray powder diffraction pattern may vary slightly from one machine to another or from one sample to another, and so the values quoted are not to be construed as absolute.

It is known that an X-ray powder diffraction pattern may be obtained which has one or more measurement errors depending on measurement conditions (such as equipment or machine used). In particular, it is generally known that intensities in an X-ray powder diffraction pattern may fluctuate depending on measurement conditions. Therefore it should be understood that the Forms of the present invention are not limited to the crystals that provide X-ray powder diffraction patterns identical to the X-ray powder diffraction pattern shown in the Figures of WO2013/156772, and any crystals providing X-ray powder diffraction patterns substantially the same as those shown in the Figures of WO2013/156772 fall within the scope of the present invention. A person skilled in the art of X-ray powder diffraction is able to judge the substantial identity of X-ray powder diffraction patterns. Persons skilled in the art of X-ray powder diffraction will realise that the relative intensity of peaks can be affected by, for example, grains above 30$\mu$m in size and non-unitary aspect ratios, which may affect analysis of samples. The skilled person will also realise that the position of reflections can be affected by the precise height at which the sample sits in the diffractometer and the zero calibration of the diffractometer. The surface planarity of the sample may also have a small effect. Hence the diffraction pattern data presented are not to be taken as absolute values. (Jenkins, R & Snyder, R.L. 'Introduction to X-Ray Powder Diffractometry' John Wiley & Sons 1996; Bunn, C.W. (1948), Chemical Crystallography, Clarendon Press, London; Klug, H. P. & Alexander, L. E. (1974), X-Ray Diffraction Procedures). Generally, a measurement error of a diffraction angle in an X-ray powder diffractogram is approximately plus or minus 0.2° 2-theta, and such degree of a measurement error should be taken into account when considering the X-ray powder diffraction pattern in the Figures of WO2013/156772 and when reading the Tables. Furthermore, it should be understood that intensities might fluctuate depending on experimental conditions and sample preparation (preferred orientation). According to a further aspect of the invention there is provided a pharmaceutical composition, which comprises Compound (VIII) in crystalline form, as defined hereinbefore in association with a pharmaceutically-acceptable diluent or carrier. The compositions of the invention may be in a form suitable for oral use (for example as tablets, lozenges, hard or soft capsules, aqueous or oily suspensions, emulsions, dispersible powders or granules, syrups or elixirs), for topical use (for example as creams, ointments, gels, or aqueous or oily solutions or suspensions), for administration by inhalation (for example as a finely divided powder or a liquid aerosol), for administration by insufflation (for example as a finely divided powder) or for parenteral administration (for example as a sterile aqueous or oily solution for intravenous, subcutaneous, intramuscular or intramuscular dosing or as a suppository for rectal dosing). The compositions of the invention may be obtained by conventional procedures using conventional pharmaceutical excipients, well known in the art. Thus, compositions intended for oral use may contain, for example, one or more colouring, sweetening, flavouring and/or preservative agents. A suitable formulation of Compound (VIII) (in the following context also referred to as Compound (I)) in crystalline form is that where the compound is filled into a white hypromellose (FIPMC) hard capsule with no other excipients. The strength of the drug product can range from 5 to 165 mg. The composition of this capsule is as follows:

| Component | Quantity (per unit) | Function |
|---|---|---|
| Compound (I) | 5-165 mg (Note A) | Drug substance |
| White HPMC hard capsule (Note B) | 1 (size 0) | Capsule shell |

Note A: The quantity of Compound (VIII) drug substance filled in the capsule is corrected for the potency of the batch being used. Note B: These FIPMC -based capsule shells contain hypromellose, carrageenan, potassium chloride, titanium dioxide and carnauba wax. Each of these ingredients meet USP/NF, Ph Eur and JP or JPE standards. An alternative suitable formulation of Compound (VIII) in crystalline form, (particularly Form B ({drug8c})) is a tablet formulation, particularly a film-coated tablet formulation. An example of a suitable film-coated tablet composition containing Compound (VIII) is described below:

| **Substance (tablet core)** | **wt% within tablet core** |
|---|---|
| Form B of Compound (I) | 60 |
| Microcrystalline cellulose | 25.88 |
| Mannitol | 8.63 |
| Croscamellose sodium | 4 |
| Magnesium stearate | 1.5 (0.5 + 1.0) Note C |
| **Substance (film coating)** | **wt% relative to tablet core** |
| Opadry™ II film coating | 3 |

Note C: As described in more detail below, the 0.5wt% magnesium stearate is used in an intragranular context, while the remaining 1.0 wt% is used in an extragranular context Various sizes of tablets can be manufactured from a granule (described below) using conventional mixing, dry granulation, compression and film coating processes, according to Good Manufacturing Practice standards. For example, tablets containing from 50 mg to 500 mg of Compound (VIII) may be prepared using the above-mentioned composition using the methods described herein. Granule preparation: Form B ({drug8c}) of Compound (VIII), microcrystalline cellulose, mannitol, croscarmellose sodium and intragranular magnesium stearate were mixed in a blender at 16 revolutions/minute for 5 minutes to achieve a uniform distribution of the Compound (VIII) within the mixture. This mixture was then fed through a roller compactor to produce a ribbon which was milled and passed through 1mm screen to achieve uniform particle size of granule. Tablet core preparation: The remaining extragranular magnesium stearate was added to the granules and the mixture was blended at 30 revolutions/minute for 1 minute. This mixture was then compressed into tablet cores using conventional tabletting equipment using standard concave punches to achieve the desired tablet sizes. Film-coating of tablet cores: The compressed tablet cores were coated with an aqueous (purified water) suspension containing the Opadry™ II film coating components, using a perforated drum coater. The Opadry™ II film-coating material is available from Colorcon™ whose website is www.colorcon.com. In one embodiment of the invention there is provided a pharmaceutical composition which comprises Compound (VIII) in crystalline form as described herein (particularly as Form B ({drug8c})), in association with microcrystalline cellulose, mannitol, croscarmellose sodium and magnesium stearate. In one embodiment there is provided a pharmaceutical tablet (i.e. suitable for oral administration to a human patient) which comprises from 50 to 500 mg of Compound (VIII) in crystalline form (particularly as Form B ({drug8c})) as described herein, in association with one or more pharmaceutically acceptable excipients. In one embodiment there is provided a pharmaceutical tablet (i.e. suitable for oral administration to a human patient) which comprises from 0.5 to 2% by weight of magnesium stearate, from 2 to 5% by weight of croscarmellose sodium, from 15 to 60% by weight of Compound (VIII) in crystalline form (particularly as Form B ({drug8c})) as described herein, microcrystalline cellulose and mannitol, wherein the relative weights of microcrystalline cellulose and mannitol within the pharmaceutical tablet composition are in a ratio of between 3 : 1 and 1 : 1. In one embodiment there is provided a pharmaceutical tablet (i.e. suitable for oral administration to a human patient) which comprises from 0.5 to 2% by weight of magnesium stearate, from 2 to 5% by weight of croscarmellose sodium, from 15 to 60% by weight of Compound (VIII) in crystalline form (particularly as Form B ({drug8c})) as described herein, microcrystalline cellulose and mannitol, wherein the relative weights of microcrystalline cellulose and mannitol within the tablet are in a ratio of from 3 : 1 to 1 : 1 and wherein the amount of Compound (VIII) in crystalline form within the tablet is from 50 to 500 mg.

In any aspect, embodiment or claim referring to a tablet in this specification, the amount of Compound (VIII) in

crystalline form within the tablet may be from 50 to 500mg. In one embodiment there is provided a pharmaceutical tablet (i.e. suitable for oral administration to a human patient) which comprises greater than 55% by weight of Compound (VIII) in crystalline form as described herein (particularly as Form B ({drug8c})). In one embodiment there is provided a pharmaceutical tablet (i.e. suitable for oral administration to a human patient) which comprises 50-70% by weight of Compound (VIII) in crystalline form as described herein (particularly as Form B ({drug8c})). Such tablet may comprise microcrystalline cellulose (particularly between 20 and 30% by weight of the total tablet). Such tablet may comprise mannitol (particularly between 5 and 12% by weight of the total tablet). Such tablet may comprise croscarmellose sodium (particularly between 2 and 5% by weight of the total tablet). Such tablet may comprise magnesium stearate (particularly between 0.5 and 2% by weight of the total tablet). Such tablet may include a film-coating around the core of the tablet (particularly where the film coating comprises 1 to 5% by weight of the total tablet). Compound (VIII) in crystalline form will normally be administered to a warm-blooded animal at a unit dose within the range 5-5000 mg/m2 body area of the animal, i.e. approximately 0.1-100 mg/kg, and this normally provides a therapeutically-effective dose. A unit dose form such as a tablet or capsule will usually contain, for example 1-500 mg of active ingredient. Particular daily doses could be 400mg b.i.d for monotherapy and 320mg b.i.d (continuous) or 360mg b.i.d (intermittent) for combination with another chemo-therapeutic. However the daily dose will necessarily be varied depending upon the host treated, the particular route of administration, and the severity of the illness being treated. Accordingly the practitioner who is treating any particular patient may determine the optimum dosage. In the context of the present specification, the term "therapy" also includes "prophylaxis" unless there are specific indications to the contrary. The terms "therapeutic" and "therapeutically" should be construed accordingly. As used herein, the term "treatment" is intended to have its normal everyday meaning of dealing with a disease in order to entirely or partially relieve one, some or all of its symptoms, or to correct or compensate for the underlying pathology. As used herein, the term "prophylaxis" is intended to have its normal everyday meaning and includes primary prophylaxis to prevent the development of the disease and secondary prophylaxis whereby the disease has already developed and the patient is temporarily or permanently protected against exacerbation or worsening of the disease or the development of new symptoms associated with the disease. As a result of their PKB inhibitory activity, Compound (VIII) in crystalline form is expected to be useful in the treatment of diseases or medical conditions mediated alone or in part by PKB activity, for example cancer. The types of cancers which may be susceptible to treatment using Compound (VIII) in crystalline form of the present invention include, but are not limited to, ovarian cancer, cervical cancer, colorectal cancer, breast cancer, pancreatic cancer, glioma, glioblastoma, melanoma, prostate cancer, leukaemia, lymphoma, Non-Hodgkins lymphoma, gastric cancer, lung cancer, hepatocellular cancer, gastric cancer, gastrointestinal stromal tumour (GIST), glioma, thyroid cancer, bile duct cancer, endometrial cancer, renal cancer, anaplastic large cell lymphoma, acute myeloid leukaemia (AML), multiple myeloma, melanoma and mesothelioma. Breast cancer, and more specifically luminal breast cancer, may be particularly susceptible to treatment using compounds of the present invention. In particular Compound (VIII) in crystalline form may be useful in the treatment of breast cancer, including oestrogen receptor positive breast cancer, prostate cancer including castrate resistant prostate cancer and metastatic castrate resistant prostate cancer and gastric cancer. In one aspect of the invention Compound (VIII) in crystalline form may be useful in the treatment of breast cancer particularly oestrogen receptor positive breast cancer. In another aspect of the invention Compound (VIII) in crystalline form may be useful in the treatment of prostate cancer in particular castrate resistant prostate cancer. In a further aspect of the invention Compound (VIII) in crystalline form may be useful in the treatment of prostate cancer in particular metastatic castrate resistant prostate cancer. In another aspect of the invention Compound (VIII) in crystalline form may be useful in the treatment of gastric cancer. It is envisaged that for the methods of treatment of cancer mentioned herein, Compound (VIII) in crystalline form will be administered to a mammal, more particularly a human being. Similarly, for the uses of Compound (VIII) in crystalline form for the treatment of cancer mentioned herein, it is envisaged that Compound (VIII) in crystalline form will be administered to a mammal, more particularly a human being. According to another aspect of the invention, there is therefore provided Compound (VIII) in crystalline form as defined hereinbefore, for use as a medicament. According to a further aspect of the invention, there is provided Compound (VIII) in crystalline form as defined hereinbefore for use in the treatment of a disease mediated through PKB. According to a further aspect of the invention, there is provided Compound (VIII) in crystalline form as defined hereinbefore for use in the treatment of cancer. According to a further aspect of the invention, there is provided Compound (VIII) in crystalline form as defined hereinbefore for use in the treatment of breast cancer, including oestrogen receptor positive breast cancer, prostate cancer including castrate resistant prostate cancer and metastatic castrate resistant prostate cancer and gastric cancer. According to a further aspect of the invention, there is provided the use of Compound (VIII) in crystalline form as defined hereinbefore for the preparation of a medicament for the treatment of a disease mediated through PKB. According to a further aspect of the invention, there is provided the use of Compound (VIII) in crystalline form as defined hereinbefore for the preparation of a medicament for the treatment of cancer. According to a further aspect of the invention, there is provided the use of Compound (VIII) in crystalline form as defined hereinbefore for the preparation of a medicament for the treatment of breast cancer, including oestrogen receptor positive breast cancer, prostate cancer including castrate resistant prostate cancer and metastatic castrate resistant prostate cancer and gastric cancer. According to a further aspect of the invention, there is provided a method of treating a human suffering

from a disease in which inhibition of PKB is beneficial, comprising the steps of administering to a person in need thereof of a therapeutically effective amount of Compound (VIII) in crystalline form as defined hereinbefore. In one embodiment of the invention there is provided a method of treating cancer which comprises the steps of administering to a person in need thereof of a therapeutically effective amount of Compound (VIII) in crystalline form as defined hereinbefore. In one embodiment of the invention there is provided a method of treating breast cancer, including oestrogen receptor positive breast cancer, prostate cancer including castrate resistant prostate cancer and metastatic castrate resistant prostate cancer and gastric cancer which comprises the steps of administering to a person in need thereof of a therapeutically effective amount of Compound (VIII) in crystalline form as defined hereinbefore. In any embodiment, aspect or claim where "cancer" is mentioned, the cancer may be breast cancer. In any embodiment, aspect or claim where "cancer" is mentioned, the cancer may be oestrogen receptor positive breast cancer. In any embodiment, aspect or claim where "cancer" is mentioned, the cancer may be prostate cancer. In any embodiment, aspect or claim where "cancer" is mentioned, the cancer may be castrate resistant prostate cancer. In any embodiment, aspect or claim where "cancer" is mentioned, the cancer may be metastatic castrate resistant prostate cancer. In any embodiment, aspect or claim where "cancer" is mentioned, the cancer may be gastric cancer. The cancer treatment defined hereinbefore may be applied as a sole therapy or may involve, in addition to the compound of the invention, conventional surgery or radiotherapy or chemotherapy. Such chemotherapy may include a combination comprising Compound (VIII) in crystalline form with an androgen receptor signalling modulator selected from: • MDV-3100 (4-{3-[4-cyano-3-(trifluoromethyl)-phenyl]-5,5-dimethyl-4-oxo-2-thioxoimidazolidin-1-yl}-2-fluoro-N-methylbenzamide); • AZD3514 (1-{4-[2-(4-{ 1-[3- (trifluoromethyl)-7,8-dihydro[1,2,4]triazolo[4,3-£]pyrid-azin-6-yl]piperidin-4-yl }phenoxy)ethyl]piperazin-1-yl }ethanone); • abiraterone, or an ester prodrug thereof ((3P)-17-(pyridin-3-yl)androsta-5, 16-dien-3-ol "abiraterone", or "abiraterone acetate"); and • bicalutamide (N-[4-cyano-3-(trifluoromethyl)-phenyl]-3-[(4-fluorophenyl)-sulfonyl]-2-hydroxy-2-methylpropanamide); or a pharmaceutically acceptable salt thereof. MDV-3100 is alternatively known as "enzalutamide". Such chemotherapy may also include a combination comprising Compound (VIII) in crystalline form and a taxane, particularly a taxane selected from docetaxel and paclitaxel. Herein, where the term "combination" is used it is to be understood that this refers to simultaneous, separate or sequential administration. In one aspect of the invention "combination" refers to simultaneous administration. In another aspect of the invention "combination" refers to separate administration. In a further aspect of the invention "combination" refers to sequential administration. Where the administration is sequential or separate, the delay in administering the second component should not be such as to lose the beneficial and / or synergistic effect of the combination. In one embodiment of the invention there is provided a method of treating prostate cancer including castrate resistant prostate cancer and metastatic castrate resistant prostate cancer which comprises the steps of administering to a person in need thereof of a therapeutically effective amount of Compound (VIII) in crystalline form as defined hereinbefore in combination with an androgen receptor signalling modulator selected from: • MDV-3100 (4-{3-[4-cyano-3-(trifluoromethyl)-phenyl]-5,5-dimethyl-4-oxo-2-thioxoimidazolidin-1-yl}-2-fluoro-N-methylbenzamide); • AZD3514 (1-{4-[2-(4-{ 1-[3-(trifluoromethyl)-7,8-dihydro[1,2,4]triazolo[4,3-£]pyrid-azin-6-yl]piperidin-4-yl }phenoxy)ethyl]piperazin-1-yl} ethanone); • abiraterone, or an ester prodrug thereof ((3P)-17-(pyridin-3-yl)androsta-5, 16-dien-3-ol "abiraterone", or "abiraterone acetate"); and • bicalutamide (N-[4-cyano-3-(trifluoromethyl)-phenyl]-3-[(4-fluorophenyl)-sulfonyl]-2-hydroxy-2-methylpropanamide); or a pharmaceutically acceptable salt thereof. According to a further aspect of the invention, there is provided the use of Compound (VIII) in crystalline form as defined hereinbefore in combination with an androgen receptor signalling modulator selected from: • MDV-3100 (4-{3-[4-cyano-3-(trifluoromethyl)-phenyl]-5,5-dimethyl-4-oxo-2-thioxoimidazolidin-1-yl}-2-fluoro-N-methylbenzamide); • AZD3514 (1-{4-[2-(4-{ 1-[3-(trifluoromethyl)-7,8-dihydro[1,2,4]triazolo[4,3-£]pyrid-azin-6-yl]piperidin-4-yl }phenoxy)ethyl]piperazin-1-yl } ethanone); • abiraterone, or an ester prodrug thereof ((3P)-17-(pyridin-3-yl)androsta-5, 16-dien-3-ol "abiraterone", or "abiraterone acetate"); and • bicalutamide (N-[4-cyano-3-(trifluoromethyl)-phenyl]-3-[(4-fluorophenyl)-sulfonyl]-2-hydroxy-2-methylpropanamide); or a pharmaceutically acceptable salt thereof; for the preparation of a medicament for the treatment of prostate cancer including castrate resistant prostate cancer and metastatic castrate resistant prostate cancer. According to a further aspect of the invention, there is provided Compound (VIII) in crystalline form as defined hereinbefore in combination with an androgen receptor signalling modulator selected from: • MDV-3100 (4-{3-[4-cyano-3-(trifluoromethyl)-phenyl]-5,5-dimethyl-4-oxo-2-thioxoimidazolidin-1-yl}-2-fluoro-N-methylbenzamide); • AZD3514 (1-{4-[2-(4-{1-[3-(trifluoromethyl)-7,8-dihydro[1,2,4]triazolo[4,3-£]pyrid-azin-6-yl]piperidin-4-yl }phenoxy)ethyl]piperazin-1-yl } ethanone); • abiraterone, or an ester prodrug thereof ((3P)-17-(pyridin-3-yl)androsta-5, 16-dien-3-ol "abiraterone", or "abiraterone acetate"); and • bicalutamide (N-[4-cyano-3-(trifluoromethyl)-phenyl]-3-[(4-fluorophenyl)-sulfonyl]-2-hydroxy-2-methylpropanamide);

or a pharmaceutically acceptable salt thereof; for use in the treatment of prostate cancer including castrate resistant prostate cancer and metastatic castrate resistant prostate cancer. In a further embodiment of the invention there is provided a method of treating breast cancer, including oestrogen receptor positive breast cancer, which comprises the steps of administering to a person in need thereof of a therapeutically effective amount of Compound (VIII) in crystalline form as defined hereinbefore in combination with a taxane, particularly a taxane selected from docetaxel and paclitaxel. According to a further aspect of the invention, there is provided the use of Compound (VIII) in crystalline form as defined

hereinbefore in combination with a taxane, particularly a taxane selected from docetaxel and paclitaxel for the preparation of a medicament for the treatment of breast cancer, including oestrogen receptor positive breast cancer. According to a further aspect of the invention, there is provided Compound (VIII) in crystalline form as defined hereinbefore in combination with a taxane, particularly a taxane selected from docetaxel and paclitaxel for use in the treatment of breast cancer, including oestrogen receptor positive breast cancer. In one embodiment the taxane is docetaxel. In another embodiment the taxane is paclitaxel.

Figure 1 of WO2013/156772: X-ray powder diffraction pattern - Form A ({drug8b}) of Compound (VIII). Figure 2 of WO2013/156772: DSC thermogram - Form A ({drug8b}) of Compound (VIII). Figure 3.1 of WO2013/156772: X-ray powder diffraction pattern 1 - Form B ({drug8c}) of Compound (VIII). Figure 3.2 of WO2013/156772: X-ray powder diffraction pattern 2 - Form B ({drug8c}) of Compound (VIII). Figure 4 of WO2013/156772: DSC thermogram 1 - Form B ({drug8c}) of Compound (VIII). Figure 5 of WO2013/156772: X-ray powder diffraction pattern - Form C ({drug8d}) of Compound (VIII). Figure 6 of WO2013/156772: DSC thermogram - Form C ({drug8d}) of Compound (VIII). Figure 7 of WO2013/156772: DSC thermogram 2 - Form B ({drug8c}) of Compound (VIII).

Details of Techniques Used: A Siemens D5000 X-Ray Powder Diffractometer was used to obtain X-ray diffraction data on Form A ({drug8b}) of Compound (VIII). The X-ray powder diffraction spectra were determined by mounting a sample of the crystalline material on a Siemens single silicon crystal (SSC) wafer mount and spreading out the sample into a thin layer with the aid of a microscope slide. The sample was spun at 30 revolutions per minute (to improve counting statistics) and irradiated with X-rays generated by a copper long-fine focus tube operated at 40kV and 40mA with a wavelength of 1.54A (i.e. CuKa radiation). The collimated X-ray source was passed through an automatic variable divergence slit set at V20 and the reflected radiation directed through a 2mm antiscatter slit and a 0.2mm detector slit. The sample was exposed for 1 second per 0.02 degree 2-theta increment (continuous scan mode) over the range 2 degrees to 40 degrees 2-theta in theta-theta mode. The running time was 31 minutes and 41 seconds. The instrument was equipped with a scintillation counter as detector. Control and data capture was by means of a Dell Optiplex 686 NT 4.0 Workstation operating with Diffract+ software. A Bruker D8 X-ray powder diffractometer was used to obtain X-ray diffraction data on Forms B & C of Compound (VIII). The X-ray diffraction pattern of Figure 3.1 of WO2013/156772 (but not Figure 3.2 of WO2013/156772) for Form B ({drug8c}) was obtained using this diffractometer by mounting a sample of the crystalline material on a Bruker single silicon crystal (SSC) wafer mount and spreading out the sample into a thin layer with the aid of a microscope slide. The sample was spun at 30 revolutions per minute (to improve counting statistics) and irradiated with X-rays generated by a copper long-fine focus tube operated at 40kV and 40mA with a wavelength of 1.54A (i.e. CuKa radiation). The collimated X-ray source was passed through a fixed divergence slit. The sample was exposed for 0.2 seconds per 0.014° 2-theta increment (continuous scan mode) over the range 2 degrees to 40 degrees 2-theta in theta-theta mode. The running time was approximately 9 minutes and 3 seconds. The instrument was equipped with a Position sensitive detector. Control and data capture was by means of a Dell Optiplex 686 NT 4.0 Workstation operating with Diffrac+ software. A PANalytical CUB IX PRO X-ray powder diffractometer was used to analyse Form B ({drug8c}) of Compound (VIII). The X-ray diffraction pattern of Figure 3.2 of WO2013/156772 (but not Figure 3.1 of WO2013/156772) for Form B ({drug8c}) was obtained using this diffractometer by mounting a sample of the crystalline material on a single silicon crystal (SSC) wafer mount and spreading out the sample into a thin layer. The sample was spun at 30 revolutions per minute (to improve counting statistics) and irradiated with X-rays generated by a copper long-fine focus tube operated at 45kV and 40mA with a wavelength of 1.54A (i.e. CuKa radiation). The sample was exposed for 25 seconds per 0.025° 2-theta increment (continuous scan mode) over the range 2 degrees to 40 degrees 2-theta in theta-theta mode using an X'celerator detector (active length 2.55° 20). A TA Instruments Q 1000' Differential Scanning Calorimeter was used to analyse Forms A, B and C of Compound (VIII). Typically less than 5mg of material (contained in a standard aluminium pan fitted with a lid) was heated from 25-300°C at a constant heating rate of 10°C/minute. A purge gas using nitrogen was used - flow rate 50mL per minute. Any crystal form that provides a XRPD diffractogram, Raman/IR spectrum, SSNMR spectrum or DSC thermogram substantially identical to those disclosed herein, fall within the scope of the present disclosures. One skilled in the art will have the ability to determine substantial identities of diffractograms, spectra and thermograms.

<u>Reference Example 1:</u> Preparation of Form A ({drug8b}): WO 2009/047563 discloses three processes for the preparation of Compound (VIII) -Example 9 and alternative routes 1 and 2. "Example 9 alternative route 1" includes a slurry of Compound (VIII) in ethyl acetate, the other two processes isolate Compound (VIII) as a solid by evaporating fractions of a column. Three historical batches of Compound (VIII) from our compound collection synthesised using a procedure identical to, or substantially similar to, one of these three processes were analysed by XRD and all three identified as being a semi-crystalline form, designated as Form A ({drug8b}), that had a melting point of 155.2°C (onset).

<u>Example 1:</u> Preparation of Form B ({drug8c}): Approximately 20mg of Compound (VIII) Form A ({drug8b}) was placed in a vial with a magnetic stirrer bar, and approximately 2mL of acetonitrile added. The vial was then sealed tightly with a cap and left to stir on a magnetic stirrer plate. After 3 days, the sample was removed from the plate, the cap taken off and the slurry left to dry under ambient conditions before it was analysed by XRPD and DSC. This form (Form B ({drug8c})) was determined to be crystalline by XRPD. This material had a melting point of 162.3°C (onset). An X-ray

powder diffractogram of Form B ({drug8c}) prepared by this method is shown in Figure 3.1 of WO2013/156772.

Example 2: Preparation of Form C ({drug8d}): Approximately 20mg of Compound (VIII) Form A ({drug8b}) was placed in a vial with a magnetic flea, and approximately 2mL of methanol added, the vial was then sealed tightly with a cap and left to stir on a magnetic stirrer plate. After 3 days, the sample was removed from the plate, the cap taken off and the slurry left to dry under ambient conditions before it was analysed by XRPD and DSC. This form (Form C ({drug8d})) was determined to be semi-crystalline by XRPD. This material had a melting point of 142.7°C (onset) and a peak at about 149.2°C, followed by a further melting endotherm with an onset of 161.6°C and a peak at 164.5°C.

Example 3: Alternative preparation of Form B ({drug8c}): The initially produced Form A ({drug8b}) of Compound (VIII) maybe converted to Form B ({drug8c}) using the following process: Compound (VIII) is mixed with 7-8 relative volumes of absolute ethanol and the mixture is then heated to 70-75°C under reflux. The mixture is then filtered to remove undissolved particulate matter and the filtrate is cooled to 60-65°C. A small amount of previously prepared seed (e.g. 0.5wt% of Form B ({drug8c}) of Compound (VIII)) is then added to the mixture. The fluid surrounding the reaction vessel is then cooled to - 10°C at a cooling rate of 0.3°C/minute and then the mixture is stirred for a further 8-12 hours before the resulting solid is isolated by filtration. This wet solid is then dried under vacuum at a temperature of 60-65°C to provide Form B ({drug8c}) of Compound (VIII). An X-ray powder diffractogram of Form B ({drug8c}) prepared by this method is shown in Figure 3.2 of WO2013/156772. DSC analysis of Form B ({drug8c}) of Compound (VIII) as prepared by this method, shows a melting endotherm with an onset of 168.5°C and a peak at 171.0°C (Figure 7 of WO2013/156772). For the avoidance of doubt, one "relative volume" means that 1 mL of a liquid is used be used per 1 g of compound.

The invention relates to an administration unit comprising solid form of (S)-4-amino-N-(1-(4-chlorophenyl)-3-hydroxypropyl)-1-(7H-pyrrolo[2,3-d]pyrimidin-4-yl)piperidine-4-carboxamide.

PREFERRED EMBODIMENTS CONCERNING ASPECTS (I), (II), (III), (IV), (V), (VI), (VII) AND (VIII) OF THE INVENTION

The invention relates to an administration unit comprising (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}.

Preferably, the administration unit according to the invention comprises a therapeutically effective amount of (i) {drug1}, (ii) {drug2}, (iii) {drug3}, (iv) {drug4}, (v) {drug5}, (vi) {drug6}, (vii) {drug7}, or (viii) {drug8}, wherein at least 10 % by weight of said therapeutically effective amount of (i) {drug1}, (ii) {drug2}, (iii) {drug3}, (iv) {drug4}, (v) {drug5}, (vi) {drug6}, (vii) {drug7}, or (viii) {drug8} are present as (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}. Preferably, at least 20 % by weight, or at least 30 % by weight, or at least 40 % by weight, or at least 50 % by weight, or at least 60 % by weight, or at least 70 % by weight, or at least 80 % by weight, or at least 90 % by weight of said therapeutically effective amount of (i) {drug1}, (ii) {drug2}, (iii) {drug3}, (iv) {drug4}, (v) {drug5}, (vi) {drug6}, (vii) {drug7}, or (viii) {drug8} are present as (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}. Preferably, at least 95 % by weight, or at least 96 % by weight, or at least 97 % by weight, or at least 98 % by weight, or at least 99 % by weight, or at least 99.5 % by weight, or at least 99.8 % by weight, or at least 99.9 % by weight of said therapeutically effective amount of (i) {drug1}, (ii) {drug2}, (iii) {drug3}, (iv) {drug4}, (v) {drug5}, (vi) {drug6}, (vii) {drug7}, or (viii) {drug8} are present as (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}. The total content of (i) {drug1}, (ii) {drug2}, (iii) {drug3}, (iv) {drug4}, (v) {drug5}, (vi) {drug6}, (vii) {drug7}, or (viii) {drug8} as well as the relative content of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} can be

determined by standard analysis known to the skilled artisan. Suitable methods include but are not limited to thermal analysis, HPLC and XRPD.

In a preferred embodiment of the administration unit according to the invention, not more than 90 % by weight of said therapeutically effective amount of (i) {drug1}, (ii) {drug2}, (iii) {drug3}, (iv) {drug4}, (v) {drug5}, (vi) {drug6}, (vii) {drug7}, or (viii) {drug8} are amorphous. Preferably, not more than 80 % by weight, or not more than 70 % by weight, or not more than 60 % by weight, or not more than 50 % by weight, or not more than 40 % by weight, or not more than 30 % by weight, or not more than 20 % by weight, or not more than 10 % by weight of said therapeutically effective amount of (i) {drug1}, (ii) {drug2}, (iii) {drug3}, (iv) {drug4}, (v) {drug5}, (vi) {drug6}, (vii) {drug7}, or (viii) {drug8} are amorphous. Preferably, not more than 5 % by weight, or not more than 4 % by weight, or not more than 3 % by weight, or not more than 2 % by weight, or not more than 1 % by weight, or not more than 0.5 % by weight, or not more than 0.2 % by weight, or not more than 0.1 % by weight of said therapeutically effective amount of (i) {drug1}, (ii) {drug2}, (iii) {drug3}, (iv) {drug4}, (v) {drug5}, (vi) {drug6}, (vii) {drug7}, or (viii) {drug8} are amorphous.

The total content of (i) {drug1}, (ii) {drug2}, (iii) {drug3}, (iv) {drug4}, (v) {drug5}, (vi) {drug6}, (vii) {drug7}, or (viii) {drug8} as well as the relative content of amorphous (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} can be determined by standard analysis known to the skilled artisan. Suitable methods include but are not limited to thermal analysis, HPLC and XRPD (see e.g. K.D. Harris, Powder diffraction crystallography of molecular solids, Top Curr Chem., 2012, 315:133-77).

In a preferred embodiment of the administration unit according to the invention, not more than 90 % by weight of said therapeutically effective amount of (i) {drug1}, (ii) {drug2}, (iii) {drug3}, (iv) {drug4}, (v) {drug5}, (vi) {drug6}, (vii) {drug7}, or (viii) {drug8} are polymorphs of (i) {drug1}, (ii) {drug2}, (iii) {drug3}, (iv) {drug4}, (v) {drug5}, (vi) {drug6}, (vii) {drug7}, or (viii) {drug8} other than (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}. Preferably, not more than 80 % by weight, or not more than 70 % by weight, or not more than 60 % by weight, or not more than 50 % by weight, or not more than 40 % by weight, or not more than 30 % by weight, or not more than 20 % by weight, or not more than 10 % by weight of said therapeutically effective amount of (i) {drug1}, (ii) {drug2}, (iii) {drug3}, (iv) {drug4}, (v) {drug5}, (vi) {drug6}, (vii) {drug7}, or (viii) {drug8} are polymorphs of (i) {drug1}, (ii) {drug2}, (iii) {drug3}, (iv) {drug4}, (v) {drug5}, (vi) {drug6}, (vii) {drug7}, or (viii) {drug8} other than (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}. Preferably, not more than 5 % by weight, or not more than 4 % by weight, or not more than 3 % by weight, or not more than 2 % by weight, or not more than 1 % by weight, or not more than 0.5 % by weight, or not more than 0.2 % by weight, or not more than 0.1 % by weight of said therapeutically effective amount of (i) {drug1}, (ii) {drug2}, (iii) {drug3}, (iv) {drug4}, (v) {drug5}, (vi) {drug6}, (vii) {drug7}, or (viii) {drug8} are polymorphs of (i) {drug1}, (ii) {drug2}, (iii) {drug3}, (iv) {drug4}, (v) {drug5}, (vi) {drug6}, (vii) {drug7}, or (viii) {drug8} other than (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}.

The total content of (i) {drug1}, (ii) {drug2}, (iii) {drug3}, (iv) {drug4}, (v) {drug5}, (vi) {drug6}, (vii) {drug7}, or (viii) {drug8} as well as the relative content of polymorphs other than (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} can be determined by standard analysis known to the skilled artisan. Suitable methods include but are not limited to thermal analysis, HPLC and XRPD (see e.g. N. Chieng, T. Rades, J.Aaltonen, An overview of recent studies on the analysis of pharmaceutical polymorphs, J Pharm Biomed Anal. 2011, 25:55(4):618-44; R. Hilfiker, Polymorphism, Wiley-VCH, 1st ed. 2006; H.G. Brittain, Polymorphism in Pharmaceutical Solids (Drugs and the Pharmaceutical Sciences), Informa Healthcare, 2nd ed. 2009).

In a preferred embodiment of the administration unit according to the invention, therapeutically effective amount of (i) {drug1}, (ii) {drug2}, (iii) {drug3}, (iv) {drug4}, (v) {drug5}, (vi) {drug6}, (vii) {drug7}, or (viii) {drug8} is 0.1 μg to 2 g of (i) {drug1}, (ii) {drug2}, (iii) {drug3}, (iv) {drug4}, (v) {drug5}, (vi) {drug6}, (vii) {drug7}, or (viii) {drug8}. Preferably, therapeutically effective amount of (i) {drug1}, (ii) {drug2}, (iii) {drug3}, (iv) {drug4}, (v) {drug5}, (vi) {drug6}, (vii) {drug7}, or (viii) {drug8} is 1 μg to 2.5 μg of (i) {drug1}, (ii) {drug2}, (iii) {drug3}, (iv) {drug4}, (v) {drug5}, (vi) {drug6}, (vii) {drug7}, or (viii) {drug8}, or 2.5 μg to 5 μg of (i) {drug1}, (ii) {drug2}, (iii) {drug3}, (iv) {drug4}, (v) {drug5}, (vi) {drug6}, (vii) {drug7}, or (viii) {drug8}, or 5 μg to 10 μg of (i) {drug1}, (ii) {drug2}, (iii) {drug3}, (iv) {drug4}, (v) {drug5}, (vi) {drug6}, (vii) {drug7}, or (viii) {drug8}, or 10 μg to 25 μg of (i) {drug1}, (ii) {drug2}, (iii) {drug3}, (iv) {drug4}, (v) {drug5}, (vi) {drug6}, (vii) {drug7}, or (viii) {drug8}, or 25 μg to 50 μg of (i) {drug1}, (ii) {drug2}, (iii) {drug3}, (iv) {drug4}, (v) {drug5}, (vi) {drug6}, (vii) {drug7}, or (viii) {drug8}, or 50 μg to 100 μg of (i) {drug1}, (ii) {drug2}, (iii) {drug3}, (iv) {drug4}, (v) {drug5}, (vi) {drug6}, (vii) {drug7}, or (viii) {drug8}, or 100 μg to 250 μg of (i) {drug1}, (ii) {drug2}, (iii) {drug3}, (iv) {drug4}, (v) {drug5}, (vi) {drug6}, (vii) {drug7}, or (viii) {drug8}, or 250 μg to 500 μg of (i) {drug1}, (ii) {drug2}, (iii) {drug3}, (iv) {drug4}, (v) {drug5}, (vi) {drug6}, (vii) {drug7}, or (viii) {drug8}, or 500 μg to 1 mg of (i) {drug1}, (ii) {drug2}, (iii) {drug3}, (iv) {drug4}, (v) {drug5}, (vi) {drug6}, (vii) {drug7}, or (viii) {drug8}, or 1 mg to 2.5 mg of (i) {drug1}, (ii) {drug2}, (iii) {drug3}, (iv) {drug4}, (v) {drug5}, (vi) {drug6}, (vii) {drug7}, or (viii) {drug8}, or 2.5 mg to 5 mg of (i) {drug1}, (ii) {drug2}, (iii) {drug3}, (iv) {drug4}, (v) {drug5}, (vi) {drug6}, (vii) {drug7}, or (viii) {drug8}, or 5 mg to 10 mg of (i) {drug1}, (ii) {drug2}, (iii) {drug3}, (iv) {drug4}, (v) {drug5}, (vi) {drug6}, (vii) {drug7}, or (viii) {drug8}, or 10 mg to 25 mg of (i) {drug1}, (ii) {drug2}, (iii) {drug3}, (iv) {drug4}, (v) {drug5}, (vi) {drug6}, (vii) {drug7}, or (viii) {drug8}, or 25 mg to 50 mg of (i) {drug1}, (ii) {drug2}, (iii) {drug3}, (iv) {drug4}, (v) {drug5}, (vi) {drug6}, (vii) {drug7}, or (viii) {drug8}, or 50 mg to 100 mg of (i) {drug1}, (ii) {drug2}, (iii) {drug3}, (iv) {drug4}, (v) {drug5}, (vi) {drug6}, (vii) {drug7}, or (viii) {drug8}, or 100 mg to 250 mg of (i) {drug1}, (ii) {drug2}, (iii) {drug3}, (iv) {drug4}, (v) {drug5}, (vi) {drug6}, (vii) {drug7}, or (viii) {drug8}, or 250 mg to 500 mg of (i) {drug1}, (ii) {drug2}, (iii) {drug3}, (iv) {drug4}, (v) {drug5}, (vi) {drug6}, (vii) {drug7}, or (viii) {drug8}, or 500 mg to 1 g of (i) {drug1}, (ii) {drug2}, (iii) {drug3}, (iv) {drug4}, (v) {drug5}, (vi) {drug6}, (vii) {drug7}, or (viii) {drug8}, or 1 g to 1.25 g of (i) {drug1}, (ii) {drug2}, (iii) {drug3}, (iv) {drug4}, (v) {drug5}, (vi) {drug6}, (vii) {drug7}, or (viii) {drug8}, or 1.25 g to 1.5 g of (i) {drug1}, (ii) {drug2}, (iii) {drug3}, (iv) {drug4}, (v) {drug5}, (vi) {drug6}, (vii) {drug7}, or (viii) {drug8}, or 1.5 g to 1.75 g of (i) {drug1}, (ii) {drug2}, (iii) {drug3}, (iv) {drug4}, (v) {drug5}, (vi) {drug6}, (vii) {drug7}, or (viii) {drug8}, or 1.75 g to 2 g of (i) {drug1}, (ii) {drug2}, (iii) {drug3}, (iv) {drug4}, (v) {drug5}, (vi) {drug6}, (vii) {drug7}, or (viii) {drug8}.

Preferably, the administration unit according to the invention comprises 0.1 μg to 2 g of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}. Preferably, it comprises 1 μg to 2.5 μg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 2.5 μg to 5 μg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 5 μg to 10 μg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 10 μg to 25 μg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 25 μg to 50 μg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 50 μg to 100 μg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g},

{drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 100 $\mu$g to 250 $\mu$g of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 250 $\mu$g to 500 $\mu$g of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 500 $\mu$g to 1 mg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 1 mgto 2.5 mg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 2.5 mg to 5 mg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 5 mg to 10 mg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 10 mg to 25 mg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 25 mg to 50 mg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 50 mg to 100 mg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 100 mg to 250 mg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 250 mg to 500 mg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 500 mg to 1 g of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 1 g to 1.25 g of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a},

{drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 1.25 g to 1.5 g of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f} , {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 1.5 g to 1.75 g of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 1.75 g to 2 g of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}.

Preferably, the administration unit according to the invention contains (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} as substantially the only form of (i) {drug1}, (ii) {drug2}, (iii) {drug3}, (iv) {drug4}, (v) {drug5}, (vi) {drug6}, (vii) {drug7}, or (viii) {drug8}, i.e. preferably contains neither non-crystalline forms of (i) {drug1}, (ii) {drug2}, (iii) {drug3}, (iv) {drug4}, (v) {drug5}, (vi) {drug6}, (vii) {drug7}, or (viii) {drug8} nor crystalline forms other than (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}.

The administration unit according to the invention and its constituents, i.e. (i) {drug1}, (ii) {drug2}, (iii) {drug3}, (iv) {drug4}, (v) {drug5}, (vi) {drug6}, (vii) {drug7}, or (viii) {drug8} and (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, respectively, as well as pharmaceutical excipients, can be characterized by analytic methods that are known to the skilled artisan and described in Ph. Eur. and USP (see e.g. H.G. Brittain, S.J. Bodanowich, D.E. Bugay, J. DeVoncentis, G. Lewen, A.W. Newman, Physical characterization of pharmaceutical solids, Pharm Res. 1991, 8(8):963-73; D.E. Bugay, Characterization of the solid-state: spoectroscopic techniques, Adv Drug Deliv Rev., 2001, 48(1):43-65; P.A. Tishmack, D.E. Bugay, S.R. Byrn, Solid-state nuclear magnetic resonance spectroscopy-pharmaceutical application, J Pharm Sci, 2003, 92(3):441-74; C.J. Lee, C.J. Strachan, P.J. Manson, T. Rades, Characterization of the bulk properties of pharmaceutical solids using nonlinear optics-a review, J Pharm Pharmacol., 2007, 59(2):241-50); R.A. Storey, Solid State Characterization of Pharmaceuticals, Wiley-Blackwell, 2011).

In a preferred embodiment, the administration unit according to the invention contains (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} in form of particles, wherein the particle size distribution of X90 is about 500 $\mu$m or less, i.e. a particle size distribution (preferably by volume) such that at least 90 % of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} has a particle size smaller than 500 $\mu$m. Preferably, for (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b},

{drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} the particle size distribution of X90 is about 480 μm or less, or about 460 μm or less, or about 440 μm or less, or about 420 μm or less, or about 400 μm or less, or about 380 μm or less, or about 360 μm or less, or about 340 μm or less, or about 320 μm or less, or about 300 μm or less, or about 280 μm or less, or about 260 μm or less, or about 240 μm or less, or about 220 μm or less, or about 200 μm or less, or about 180 μm or less, or about 160 μm or less, or about 140 μm or less, or about 120 μm or less, or about 100 μm or less, or about 80 μm or less, or about 60 μm or less, or about 40 μm or less, or about 20 μm or less.

In a preferred embodiment, the administration unit according to the invention contains (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} in form of particles, wherein the particle size distribution of X50 is about 400 μm or less, i.e. a particle size distribution (preferably by volume) such that at least 50 % of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} has a particle size smaller than 400 μm. Preferably, for (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} the particle size distribution of X50 is about 380 μm or less, or about 360 μm or less, or about 340 μm or less, or about 320 μm or less, or about 300 μm or less, or about 280 μm or less, or about 260 μm or less, or about 240 μm or less, or about 220 μm or less, or about 200 μm or less, or about 180 μm or less, or about 160 μm or less, or about 140 μm or less, or about 120 μm or less, or about 100 μm or less, or about 80 μm or less, or about 70 μm or less, or about 60 μm or less, or about 50 μm or less. Preferably, for (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} the particle size distribution of X50 is about 45 μm or less, or about 40 μm or less, or about 35 μm or less, or about 30 μm or less, or about 25 μm or less, or about 20 μm or less, or about 15 μm or less, or about 10 μm or less.

According to USP, the following parameters may be defined based on the cumulative distribution. QR(X) = cumulative distribution of particles with a dimension less than or equal to X [μm] where the subscript R reflects the distribution type: 0 Number, 1 Length, 2 Area, 3 Volume. Therefore, by definition: QR(X) = 0.90 when X = X90; QR(X) = 0.50 when X = X50; and QR(X) = 0.10 when X = X10.

In preferred embodiments, the administration unit according to the invention contains (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} in form of particles having a cumulative distribution by volume basis Q3(X) (X in μm) of Q3(355) <0.50; or Q3(180) <0.50 and Q3(355) ≥0.50; or Q3(125) <0.50 and Q3(180) ≥0.50; or Q3(125) ≥0.50.

In preferred embodiments, the administration unit according to the invention contains (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} in form of particles having a cumulative distribution by volume basis Q3(X) (X in μm) of Q3(268) <0.50 and Q3(355) ≥0.50; or Q3(180) <0.50 and Q3(268) ≥0.50; or Q3(153) <0.50 and Q3(180) ≥0.50; or Q3(125) <0.50 and Q3(153) ≥0.50; or Q3(111) <0.50 and Q3(125) ≥0.50; or Q3(98) <0.50 and Q3(111) ≥0.50; or Q3(98) ≥0.50.

In preferred embodiments, the administration unit according to the invention contains (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k},

{drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} in form of particles having a cumulative distribution by volume basis Q3(X) (X in μm) of Q3(312) <0.50 and Q3(355) ≥0.50; or Q3(268) <0.50 and Q3(312) ≥0.50; or Q3(224) <0.50 and Q3(268) ≥0.50; or Q3(180) <0.50 and Q3(224) ≥0.50; or Q3(167) <0.50 and Q3(180) ≥0.50; or Q3(153) <0.50 and Q3(167) ≥0.50; or Q3(139) <0.50 and Q3(153) ≥0.50; or Q3(125) <0.50 and Q3(139) ≥0.50; or Q3(111) <0.50 and Q3(125) ≥0.50; or Q3(98) <0.50 and Q3(111) ≥0.50; or Q3(84) <0.50 and Q3(98) ≥0.50; or Q3(84) ≥0.50.

The indicated particle size properties are determined by laser-diffraction method, in particular low angle laser light scattering, i.e. Fraunhofer diffraction. Alternatively, the particle size properties can be also determined by microscopy (e.g. electron microscopy or scanning electron microscopy). The powder fineness is preferably determined in accordance with USP35 <811>. The results of the particle size distribution determined by different techniques can be correlated with one another.

Preferably, the administration unit according to the invention is solid, semisolid or liquid.

Preferably, the administration unit according to the invention is for administration once daily, or twice daily, or thrice daily, or four times daily, or five times daily, or six times daily.

Preferably, the administration unit according to the invention is monolithic or multiparticulate.

In a preferred embodiment of the administration unit according to the invention, (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} is homogeneously distributed over the administration unit.

Excipient composition and homogeneity can be determined by standard analysis known to the skilled artisan. Suitable methods include but are not limited to near-infrared chemical imaging.

In another preferred embodiment of the administration unit according to the invention, (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} is not homogeneously distributed over the administration unit.

In a preferred embodiment, the administration unit according to the invention provides controlled release of (i) {drug1}, (ii) {drug2}, (iii) {drug3}, (iv) {drug4}, (v) {drug5}, (vi) {drug6}, (vii) {drug7}, or (viii) {drug8}. Preferably, the administration unit according to the invention comprises a controlled release matrix or a controlled release coating.

In a preferred embodiment, under in vitro conditions (paddle apparatus, 75 rpm, 900 mL artificial gastric juice, pH 1.2) the administration unit according to the invention has released after 1 hour not more than 90 % of (i) {drug1}, (ii) {drug2}, (iii) {drug3}, (iv) {drug4}, (v) {drug5}, (vi) {drug6}, (vii) {drug7}, or (viii) {drug8}. Preferably, the administration unit according to the invention has released after 1 hour not more than 80 % of (i) {drug1}, (ii) {drug2}, (iii) {drug3}, (iv) {drug4}, (v) {drug5}, (vi) {drug6}, (vii) {drug7}, or (viii) {drug8}, or not more than 70 % of (i) {drug1}, (ii) {drug2}, (iii) {drug3}, (iv) {drug4}, (v) {drug5}, (vi) {drug6}, (vii) {drug7}, or (viii) {drug8}, or not more than 60 % of (i) {drug1}, (ii) {drug2}, (iii) {drug3}, (iv) {drug4}, (v) {drug5}, (vi) {drug6}, (vii) {drug7}, or (viii) {drug8}, or not more than 50 % of (i) {drug1}, (ii) {drug2}, (iii) {drug3}, (iv) {drug4}, (v) {drug5}, (vi) {drug6}, (vii) {drug7}, or (viii) {drug8}, or not more than 40 % of (i) {drug1}, (ii) {drug2}, (iii) {drug3}, (iv) {drug4}, (v) {drug5}, (vi) {drug6}, (vii) {drug7}, or (viii) {drug8}, or not more than 30 % of (i) {drug1}, (ii) {drug2}, (iii) {drug3}, (iv) {drug4}, (v) {drug5}, (vi) {drug6}, (vii) {drug7}, or (viii) {drug8}, or not more than 20 % of (i) {drug1}, (ii) {drug2}, (iii) {drug3}, (iv) {drug4}, (v) {drug5}, (vi) {drug6}, (vii) {drug7}, or (viii) {drug8}, or not more than 10 % of (i) {drug1}, (ii) {drug2}, (iii) {drug3}, (iv) {drug4}, (v) {drug5}, (vi) {drug6}, (vii) {drug7}, or (viii) {drug8}.

In a preferred embodiment, under in vitro conditions (paddle apparatus, 75 rpm, 900 mL artificial gastric juice, pH 1.2) the administration unit according to the invention has released after 2 hours not more than 90 % of (i) {drug1}, (ii) {drug2}, (iii) {drug3}, (iv) {drug4}, (v) {drug5}, (vi) {drug6}, (vii) {drug7}, or (viii) {drug8}. Preferably, the administration unit according to the invention has released after 2 hours not more than 80 % of (i) {drug1}, (ii) {drug2}, (iii) {drug3}, (iv) {drug4}, (v) {drug5}, (vi) {drug6}, (vii) {drug7}, or (viii) {drug8}, or not more than 70 % of (i) {drug1}, (ii) {drug2}, (iii) {drug3}, (iv) {drug4}, (v) {drug5}, (vi) {drug6}, (vii) {drug7}, or (viii) {drug8}, or not more than 60 % of (i) {drug1}, (ii) {drug2}, (iii) {drug3}, (iv) {drug4}, (v) {drug5}, (vi) {drug6}, (vii) {drug7}, or (viii) {drug8}, or not more than 50 % of (i) {drug1}, (ii) {drug2}, (iii) {drug3}, (iv) {drug4}, (v) {drug5}, (vi) {drug6}, (vii) {drug7}, or (viii) {drug8}, or not more than 40 % of (i) {drug1}, (ii) {drug2}, (iii) {drug3}, (iv) {drug4}, (v) {drug5}, (vi) {drug6}, (vii) {drug7}, or (viii) {drug8}, or not more than 30 % of (i) {drug1}, (ii) {drug2}, (iii) {drug3}, (iv) {drug4}, (v) {drug5}, (vi) {drug6}, (vii) {drug7}, or (viii) {drug8}, or not more than 20 % of (i) {drug1}, (ii) {drug2}, (iii) {drug3}, (iv) {drug4}, (v) {drug5}, (vi) {drug6}, (vii) {drug7}, or (viii) {drug8}, or not more than 10 % of (i) {drug1}, (ii) {drug2}, (iii) {drug3}, (iv) {drug4}, (v) {drug5}, (vi) {drug6}, (vii) {drug7}, or (viii) {drug8}.

In a preferred embodiment, under in vitro conditions (paddle apparatus, 75 rpm, 900 mL artificial gastric juice, pH 1.2) the administration unit according to the invention has released after 4 hours not more than 90 % of (i) {drug 1},

(ii) {drug2}, (iii) {drug3}, (iv) {drug4}, (v) {drug5}, (vi) {drug6}, (vii) {drug7}, or (viii) {drug8}. Preferably, the administration unit according to the invention has released after 4 hours not more than 80 % of (i) {drug1}, (ii) {drug2}, (iii) {drug3}, (iv) {drug4}, (v) {drug5}, (vi) {drug6}, (vii) {drug7}, or (viii) {drug8}, or not more than 70 % of (i) {drug1}, (ii) {drug2}, (iii) {drug3}, (iv) {drug4}, (v) {drug5}, (vi) {drug6}, (vii) {drug7}, or (viii) {drug8}, or not more than 60 % of (i) {drug1}, (ii) {drug2}, (iii) {drug3}, (iv) {drug4}, (v) {drug5}, (vi) {drug6}, (vii) {drug7}, or (viii) {drug8}, or not more than 50 % of (i) {drug1}, (ii) {drug2}, (iii) {drug3}, (iv) {drug4}, (v) {drug5}, (vi) {drug6}, (vii) {drug7}, or (viii) {drug8}, or not more than 40 % of (i) {drug1}, (ii) {drug2}, (iii) {drug3}, (iv) {drug4}, (v) {drug5}, (vi) {drug6}, (vii) {drug7}, or (viii) {drug8}, or not more than 30 % of (i) {drug1}, (ii) {drug2}, (iii) {drug3}, (iv) {drug4}, (v) {drug5}, (vi) {drug6}, (vii) {drug7}, or (viii) {drug8}, or not more than 20 % of (i) {drug1}, (ii) {drug2}, (iii) {drug3}, (iv) {drug4}, (v) {drug5}, (vi) {drug6}, (vii) {drug7}, or (viii) {drug8}, or not more than 10 % of (i) {drug1}, (ii) {drug2}, (iii) {drug3}, (iv) {drug4}, (v) {drug5}, (vi) {drug6}, (vii) {drug7}, or (viii) {drug8}.

In a preferred embodiment, under in vitro conditions (paddle apparatus, 75 rpm, 900 mL artificial gastric juice, pH 1.2) the administration unit according to the invention has released after 8 hours not more than 90 % of (i) {drug 1}, (ii) {drug2}, (iii) {drug3}, (iv) {drug4}, (v) {drug5}, (vi) {drug6}, (vii) {drug7}, or (viii) {drug8}. Preferably, the administration unit according to the invention has released after 8 hours not more than 80 % of (i) {drug1}, (ii) {drug2}, (iii) {drug3}, (iv) {drug4}, (v) {drug5}, (vi) {drug6}, (vii) {drug7}, or (viii) {drug8}, or not more than 70 % of (i) {drug1}, (ii) {drug2}, (iii) {drug3}, (iv) {drug4}, (v) {drug5}, (vi) {drug6}, (vii) {drug7}, or (viii) {drug8}, or not more than 60 % of (i) {drug1}, (ii) {drug2}, (iii) {drug3}, (iv) {drug4}, (v) {drug5}, (vi) {drug6}, (vii) {drug7}, or (viii) {drug8}, or not more than 50 % of (i) {drug1}, (ii) {drug2}, (iii) {drug3}, (iv) {drug4}, (v) {drug5}, (vi) {drug6}, (vii) {drug7}, or (viii) {drug8}, or not more than 40 % of (i) {drug1}, (ii) {drug2}, (iii) {drug3}, (iv) {drug4}, (v) {drug5}, (vi) {drug6}, (vii) {drug7}, or (viii) {drug8}, or not more than 30 % of (i) {drug1}, (ii) {drug2}, (iii) {drug3}, (iv) {drug4}, (v) {drug5}, (vi) {drug6}, (vii) {drug7}, or (viii) {drug8}, or not more than 20 % of (i) {drug1}, (ii) {drug2}, (iii) {drug3}, (iv) {drug4}, (v) {drug5}, (vi) {drug6}, (vii) {drug7}, or (viii) {drug8}, or not more than 10 % of (i) {drug1}, (ii) {drug2}, (iii) {drug3}, (iv) {drug4}, (v) {drug5}, (vi) {drug6}, (vii) {drug7}, or (viii) {drug8}.

In another preferred embodiment, the administration unit according to the invention provides immediate release of (i) {drug1}, (ii) {drug2}, (iii) {drug3}, (iv) {drug4}, (v) {drug5}, (vi) {drug6}, (vii) {drug7}, or (viii) {drug8}.

In a preferred embodiment, under in vitro conditions (paddle apparatus, 75 rpm, 900 mL artificial gastric juice, pH 1.2) the administration unit according to the invention has released after 0.5 hours at least 10 % of (i) {drug1}, (ii) {drug2}, (iii) {drug3}, (iv) {drug4}, (v) {drug5}, (vi) {drug6}, (vii) {drug7}, or (viii) {drug8}. Preferably, the administration unit according to the invention has released after 0.5 hours at least 20 % of (i) {drug1}, (ii) {drug2}, (iii) {drug3}, (iv) {drug4}, (v) {drug5}, (vi) {drug6}, (vii) {drug7}, or (viii) {drug8}, or at least 30 % of (i) {drug1}, (ii) {drug2}, (iii) {drug3}, (iv) {drug4}, (v) {drug5}, (vi) {drug6}, (vii) {drug7}, or (viii) {drug8}, or at least 40 % of (i) {drug1}, (ii) {drug2}, (iii) {drug3}, (iv) {drug4}, (v) {drug5}, (vi) {drug6}, (vii) {drug7}, or (viii) {drug8}, or at least 50 % of (i) {drug1}, (ii) {drug2}, (iii) {drug3}, (iv) {drug4}, (v) {drug5}, (vi) {drug6}, (vii) {drug7}, or (viii) {drug8}, or at least 60 % of (i) {drug1}, (ii) {drug2}, (iii) {drug3}, (iv) {drug4}, (v) {drug5}, (vi) {drug6}, (vii) {drug7}, or (viii) {drug8}, or at least 70 % of (i) {drug1}, (ii) {drug2}, (iii) {drug3}, (iv) {drug4}, (v) {drug5}, (vi) {drug6}, (vii) {drug7}, or (viii) {drug8}, or at least 80 % of (i) {drug1}, (ii) {drug2}, (iii) {drug3}, (iv) {drug4}, (v) {drug5}, (vi) {drug6}, (vii) {drug7}, or (viii) {drug8}, or at least 90 % of (i) {drug1}, (ii) {drug2}, (iii) {drug3}, (iv) {drug4}, (v) {drug5}, (vi) {drug6}, (vii) {drug7}, or (viii) {drug8}. Preferably, the administration unit according to the invention has a total weight of 10 mg to 3 g. Preferably, the administration unit according to the invention has a total weight of 10 to 25 mg, or 25 mg to 50 mg, or 50 mg to 100 mg, or 100 mg to 250 mg, or 250 mg to 500 mg, or 500 mg to 1000 mg, or 1000 mg to 2500 mg.

In a preferred embodiment, the administration unit according to the invention is for systemic or topical administration. In another preferred embodiment, the administration unit according to the invention is for parenteral administration. Preferably, the administration unit according to the invention is for oral, buccal, opthalmic, nasal, rectal, transmucosal, or intestinal administration. Preferably, it is for oral administration.

Preferably, the administration unit according to the invention is selected from the group consisting of tablets, capsules, effervescent tablets, orodispersible tablets, dragees, sachets, drops, suspensions, and powders. Preferably, it is a tablet. Preferably, the administration unit according to the invention is round or oblong; and/or is planar or biconvex.

In a preferred embodiment, the administration unit according to the invention has a round cross-section with a diameter of 1 mm to 20 mm. Preferably, the diameter of the round cross-section is 1 mm to 3 mm, or 3 mm to 5 mm, or 5 mm to 8 mm, or 8 mm to 10 mm, or 10 mm to 13 mm, or 13 mm to 15 mm, or 15 mm to 18 mm, or 18 mm to 20 mm.

In a preferred embodiment, the administration unit according to the invention is oblong and has an aspect ratio of at least 1.1:1. Preferably, the aspect ratio is at least 4:3, or at least 21/2:1, or at least 3:2, or at least 16:10, or at least □:1, or at least 5:3, or at least 16:9.

In a preferred embodiment, the administration unit according to the invention has been manufactured by direct compression. In another preferred embodiment, the administration unit according to the invention has been manufactured by granulation and subsequent compression of granules. Preferably, granulation is wet granulation or dry granulation.

Preferably, the administration unit according to the invention has been compacted at a pressure of 10 MPa to 10,000 MPa. Preferably, the administration unit according to the invention has been compacted at a pressure of 10 MPa to 25 MPa, or 25 MPa to 50 MPa, or 50 MPa to 100 MPa, or 100 MPa to 250 MPa, or 250 MPa to 500 MPa, or 500 MPa

to 1000 MPa, or 1000 MPa to 2500 MPa, or 2500 MPa to 5000 MPa, or 5000 MPa to 10,000 MPa.

In a preferred embodiment, the administration unit according to the invention comprises a film coating. Preferably, the film coating is enteric. Suitable enteric coating materials are known to the skilled artisan and include but are not limited to methyl acrylate-methacrylic acid copolymers, cellulose acetate succinate, hydroxy propyl methyl cellulose phthalate, hydroxy propyl methyl cellulose acetate succinate (hypromellose acetate succinate), polyvinyl acetate phthalate (PVAP), methyl methacrylate-methacrylic acid copolymers, sodium alginate and stearic acid.

In a preferred embodiment of the administration unit according to the invention, the film coating comprises a cellulose ether (e.g. hydroxypropylmethyl cellulose) or a synthetic polymer (e.g. polyvinyl alcohol).

In a preferred embodiment of the administration unit according to the invention, the film coating has an average thickness of 0.01 μm to 0.03 μm, or 0.03 to 0.05 μm, or 0.05 to 0.1 μm, or 0.1 to 0.25 μm, or 0.25 to 0.5 μm, or 0.5 to 1.0 μm, or 1.0 μm to 2.5 μm, or 2.5 μm to 5.0 μm, or 5.0 μm to 10 μm, or 10 μm to 25 μm, or 25 μm to 50 μm, or 50 μm to 100 μm, or 100 μm to 250 μm, or 250 μm to 500 μm, or 500 μm to 1000 μm.

In a preferred embodiment, the administration unit according to the invention has a tablet porosity of not more than 90 %. Preferably, it has a tablet porosity of not more than 80 %, or not more than 70 %, or not more than 60 %, or not more than 50 %, or not more than 40 %, or not more than 30 %, or not more than 20 %, or not more than 10 %. Preferably, it has a tablet porosity of not more than 5 %, or not more than 4 %, or not more than 3 %, or not more than 2 %, or not more than 1 %, or not more than 0.5 %, or not more than 0.2 %, or not more than 0.1 %.

The most common strength test in pharmaceutical applications is the diametral compression test, which is used to calculate the radial tensile strength of a tablet (Fell, J.T., Newton, J.M., 1970. Determination of tablet strength by diametral compression test. J. Pharm. Sci. 59, 688-691). In order to calculate the radial tensile strength, the stress conditions have to be such that the tablet fails in tension. During radial tensile strength measurements, the fracture occurs through a predetermined diametral cross section of the tablet. Therefore, the radial tensile strength is likely to reflect the average strength of tablet rather than the strength of the weakest plane in the tablet.

In a preferred embodiment, the administration unit according to the invention has a radial tensile strength of 0.1 to 100 MPa. Preferably, it has a radial tensile strength of 0.1 MPa to 0.3 MPa, or 0.3 MPa to 0.5 MPa, or 0.5 MPa to 1.0 MPa, or 1.0 MPa to 2.5 MPa, or 2.5 MPa to 5.0 MPa, or 5.0 MPa to 10 MPa, or 10 MPa to 25 MPa, or 25 MPa to 50 MPa, or 50 MPa to 100 MPa.

Another method for determining mechanical strength is to measure the axial tensile strength. The force necessary to break the tablet is obtained by pulling the tablet parallel to the applied force during the formation of the tablet and this force is then used to calculate the axial tensile strength (Nyström, C., Malmqvist, K., Mazur, J., Alex, W., Hölzer, A.W., 1978. Measurement of axial and radial tensile strength of tablets and their relation to capping. Acta Pharm. Suec. 15, 226-232). During axial tensile strength measurements, the fracture will occur through the weakest plane in the tablet. Consequently, this method allows detection of capping tendencies in a tablet.

In a preferred embodiment, the administration unit according to the invention has an axial tensile strength of 0.1 to 100 MPa. Preferably, it has an axial tensile strength of 0.1 MPa to 0.3 MPa, or 0.3 MPa to 0.5 MPa, or 0.5 MPa to 1.0 MPa, or 1.0 MPa to 2.5 MPa, or 2.5 MPa to 5.0 MPa, or 5.0 MPa to 10 MPa, or 10 MPa to 25 MPa, or 25 MPa to 50 MPa, or 50 MPa to 100 MPa.

In a preferred embodiment, the administration unit according to the invention has an average pore size of 0.001 μm to 1000 μm. Preferably, it has an average pore size of 0.001 μm to 0.003 μm, or 0,003 μm to 0.005 μm, or 0.005 μm to 0.01 μm, or 0.01 μm to 0.025 μm, or 0.025 μm to 0.05 μm, or 0.05 μm to 0.1 μm, or 0.1 μm to 0.25 μm, or 0.25 μm to 0.5 μm, or 0.5 μm to 1 μm, or 1 μm to 2.5 μm, or 2.5 μm to 5 μm, or 5 μm to 10 μm, or 10 μm to 25 μm, or 25 μm to 50 μm, or 50 μm to 100 μm, or 100 μm to 250 μm, or 250 μm to 500 μm, or 500 μm to 1000 μm.

The pore size distribution of a tablet may be assessed by methods that are known to the skilled artisan and include but are not limited to gas adsorption (e.g. Stanley-Wood, N.G., Johansson, M.E., 1980. Variation of intra-and inter-particle porosity with degree of compaction. Analyst 105, 1104-1112; Westermarck, S., Juppo, A.M., Kervinen, L., Yliruusi, J., 1998. Pore structure and surface area of mannitol powder, granules and tablets determined with mercury porosimetry and nitrogen adsorption. Eur. J. Pharm. Biopharm. 46, 61-86) or mercury porosimetry (e.g. Stanley-Wood, N.G., Johansson, M.E., 1980. Variation of intra-and inter-particle porosity with degree of compaction. Analyst 105, 1104-1112; Juppo, A.M., 1996. Relationship between breaking force and pore structure of lactose, glucose and mannitol tablets. Int. J. Pharm.127, 95-102; Westermarck, S., Juppo, A.M., Kervinen, L., Yliruusi, J., 1998. Pore structure and surface area of mannitol powder, granules and tablets determined with mercury porosimetry and nitrogen adsorption. Eur. J. Pharm. Biopharm. 46, 61-86). These techniques are complementary, in that mercury porosimetry can be used to measure larger pores (the lower size limit is about 0.003 μm in diameter) while gas adsorption allows measurement of smaller pores. For further details it is also referred to S. Lowell et al., Characterization of Porous Solids and Powders: Surface Area, Pore Size and Density (Particle Technology Series), Springer, 2010.

In a preferred embodiment, the administration unit according to the invention has a pore size distribution such that at least 10 % of the pores have a pore size of not more than 0.1 μm. Preferably, the pore size distribution is such that at least 20 %, or at least 30 %, or at least 40 %, or at least 50 %, or at least 60 %, or at least 70 %, or at least 80

%, or at least 90 % of the pores have a pore size of not more than 0.1 μm.

In another preferred embodiment, the administration unit according to the invention has a pore size distribution such that at least 10 % of the pores have a pore size of not less than 0.1 μm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not less than 0.1 μm.

In preferred embodiment, the administration unit according to the invention has a pore size distribution such that at least 10 % of the pores have a pore size of not more than 1 μm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not more than 1 μm.

In another preferred embodiment, the administration unit according to the invention has a pore size distribution such that at least 10 % of the pores have a pore size of not less than 1 μm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not less than 1 μm.

In a preferred embodiment, the administration unit according to the invention has a pore size distribution such that at least 10 % of the pores have a pore size of not more than 10 μm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not more than 10 μm.

In another preferred embodiment, the administration unit according to the invention has a pore size distribution such that at least 10 % of the pores have a pore size of not less than 10 μm. Preferably, the pore size distribution is such that at least 20 %, or at least 30 %, or at least 40 %, or at least 50 %, or at least 60 %, or at least 70 %, or at least 80 %, or at least 90 % of the pores have a pore size of not less than 10 μm.

In a preferred embodiment, the administration unit according to the invention has a water content of not more than 5 % by weight, relative to the total weight of the tablet. Preferably, it has a water content of not more than 4 % by weight, or not more than 3 % by weight, or not more than 2 % by weight, or not more than 1 % by weight, or not more than 0.5 % by weight, or not more than 0.1 % by weight, relative to the total weight of the tablet.

In a preferred embodiment, the administration unit according to the invention has a true density of 0.60 to 1.40 g cm-3. Preferably, it has a true density of 0.60 to 0.65 g cm-3, or 0.65 to 0.70 g cm-3, or 0.70 to 0.75 g cm-3, or 0.75 to 0.80 g cm-3, or 0.80 to 0.85 g cm-3, or 0.85 to 0.90 g cm-3, or 0.90 to 0.95 g cm-3, or 0.95 to 1.00 g cm-3, or 1.00 to 1.05 g cm-3, or 1.05 to 1.10 g cm-3, or 1.10 to 1.15 g cm-3, or 1.15 to 1.20 g cm-3, or 1.20 to 1.25 g cm-3, or 1.25 to 1.30 g cm-3, or 1.30 to 1.35 g cm-3, or 1.35 to 1.40 g cm-3.

In a preferred embodiment, the administration unit according to the invention has an apparent density of 0.60 to 1.40 g cm-3. Preferably, it has an apparent density of 0.60 to 0.65 g cm-3, or 0.65 to 0.70 g cm-3, or 0.70 to 0.75 g cm-3, or 0.75 to 0.80 g cm-3, or 0.80 to 0.85 g cm-3, or 0.85 to 0.90 g cm-3, or 0.90 to 0.95 g cm-3, or 0.95 to 1.00 g cm-3, or 1.00 to 1.05 g cm-3, or 1.05 to 1.10 g cm-3, or 1.10 to 1.15 g cm-3, or 1.15 to 1.20 g cm-3, or 1.20 to 1.25 g cm-3, or 1.25 to 1.30 g cm-3, or 1.30 to 1.35 g cm-3, or 1.35 to 1.40 g cm-3.

In a preferred embodiment, the administration unit according to the invention disintegrates within 6000 seconds. Preferably, it disintegrates within 5500 seconds, or within 5000 seconds, or within 4500 seconds, or within 4000 seconds, or within 3500 seconds, or within 3000 seconds, or within 2500 seconds, or within 2000 seconds, or within 1500 seconds, or within 1000 seconds, or within 750 seconds, or within 500 seconds, or within 250 seconds.

In a preferred embodiment, the administration unit according to the invention is a capsule. Preferably, it comprises a multitude of particulates comprising (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}. Preferably, it comprises at least 2 particulates, or at least 3 particulates, or at least 4 particulates, or at least 5 particulates, or at least 6 particulates, or at least 7 particulates, or at least 8 particulates, or at least 9 particulates, or at least 10 particulates.

In a preferred embodiment of the administration unit according to the invention, the capsule material comprises hard gelatine. In a preferred embodiment, the administration unit according to the invention comprises at least 2 different types of particulates that differ from each other in at least one property selected from the group consisting of nature of excipients, content of excipients, content of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, content of (i) {drug1}, (ii) {drug2}, (iii) {drug3}, (iv) {drug4}, (v) {drug5}, (vi) {drug6}, (vii) {drug7}, or (viii) {drug8}, water content, total weight, size, and shape.

In a preferred embodiment, the administration unit according to the invention passes the storage stability test

according to Ph. Eur. under accelerated storage conditions at 40°C and 75 % rel. humidity. Preferably, it has a storage stability for at least 3 months, or at least 4 months, or at least 5 months, or at least 6 months, or at least 7 months, or at least 8 months, or at least 9 months, or at least 10 months, or at least 11 months, or at least 12 months according to Ph. Eur. under accelerated storage conditions at 40°C and 75 % rel. humidity.

In a preferred embodiment, the administration unit according to the invention provides the peak plasma level within 0.1 hour to 36 hours after administration. Preferably, it provides the peak plasma level within 0.1 hour to 1 hour, or 1 hour to 2 hours, or 2 hours to 3 hours, or 3 hours to 4 hours, or 4 hours to 5 hours, or 5 hours to 6 hours, or 6 hours to 7 hours, or 7 hours to 8 hours, or 8 hours to 9 hours, or 9 hours to 10 hours, or 10 hours to 11 hours, or 11 hours to 12 hours, or 12 hours to 13 hours, or 13 hours to 14 hours, or 14 hours to 15 hours, or 15 hours to 16 hours, or 16 hours to 17 hours, or 17 hours to 18 hours, or 18 hours to 19 hours, or 19 hours to 20 hours, or 20 hours to 21 hours, or 21 hours to 22 hours, or 22 hours to 23 hours, or 23 hours to 24 hours, after administration.

Preferably, the administration unit according to the invention comprises one or more excipients independently selected from the group consisting of antiadherents, binders, disintegrants, fillers, diluents, flavors, colors, lubricants, glidants, sorbents, surfactants, preservatives and sweeteners.

In a preferred embodiment of the administration unit according to the invention, the antiadherent is selected from the group consisting of silicon dioxide, talc, talc waxes, stearates (e.g. magnesium, calcium and sodium), stearic acid, stearowet, boric acid, sodium chloride, DL-leucine, sodium oleate, sodium benzoate, sodium acetate, sodium lauryl sulfate, and magnesium lauryl sulfate. Magnesium stearate is particularly preferred.

In a preferred embodiment of the administration unit according to the invention, the binder is selected from the group consisting of saccharides and their derivatives; proteins; gums; silicates; and synthetic polymers. Preferably, the binder is a saccharide or a derivative thereof selected from the group consisting of disaccharides (e.g. sucrose, glucose or lactose); polysaccharides and their derivatives (e.g. starch, pregelatinized starch, cellulose, microcrystalline cellulose, polysaccharide acids, cellulose ethers [e.g. methyl cellulose, ethyl cellulose, hydroxyethyl cellulose, hydroxypropyl cellulose, hydroxypropylmethyl cellulose or carboxymethylcellulose sodium]); alginic acid and salts thereof (e.g. sodium alginate); and sugar alcohols (e.g. xylitol, sorbitol or maltitol); or wherein the binder is a protein selected from gelatin; or wherein the binder is a gum (e.g. acacia gum, guar gum, or tragacanth gum); or wherein the binder is a silicate (e.g. magnesium aluminum silicate or bentonite); or wherein the binder is a synthetic polymer selected from the group consisting of polyvinylpyrrolidone, polyvinylpyrrolidone-vinylacetate copolymer, polymethacrylate polymer, polyvinylalcohol, and polyethylene glycol.

In a preferred embodiment of the administration unit according to the invention, the disintegrant is selected from the group consisting of cross-linked polymers (e.g. cross-linked polyvinylpyrrolidone [crospovidone] or cross-linked sodium carboxymethyl cellulose [croscarmellose sodium]), starches (e.g. corn or potato), pregelatinized starch, modified starch (e.g. sodium starch glycolate), algenic acid, alginates (e.g. sodium alginate), carboxymethylcellulose, microcrystalline cellulose, clays (e.g. magnesium aluminum silicate), and gums (e.g. agar, guar, locust bean, karaya, pectin, or tragacanth gum).

In a preferred embodiment of the administration unit according to the invention, the filler (or diluent) is selected from the group consisting of plant cellulose, microcrystalline cellulose, inorganic phosphates (e.g. dibasic calcium phosphate or tricalcium phosphate), inorganic carbonates such as calcium carbonate, inorganic sulfates such as calcium sulfate dihydrate, sugars and sugar alcohols (e.g. lactose, glucose, inositol, mannitol, sorbitol, sucrose, dextrose, maltodextrins, and fructose), magnesium stearate, calcium lactate trihydrate, starch (e.g. corn, wheat, maize, potato or rice starch), and modified starch (e.g. carboxymethyl starch).

In a preferred embodiment of the administration unit according to the invention, the lubricant is hydrophilic or hydrophobic. Preferably, the lubricant is selected from the group consisting of talc, silica, stearin, fatty acids (e.g. stearic acid) or fatty acid derivatives, metal stearates (e.g. magnesium stearate, calcium stearate, or zinc stearate), glyceryl monostearate, glyceryl palmitostearate, glyceryl behenate, sodium lauryl sulfate, magnesium lauryl sulfate, sodium stearyl fumarate, hydrogenated vegetable oil, polyalkylene glycols (e.g. polyethylene glycol), starch, light mineral oil, sodium benzoate, and sodium chloride.

In a preferred embodiment of the administration unit according to the invention, the glidant is selected from the group consisting of fumed silica, cornstarch, talc, and magnesium carbonate.

In a preferred embodiment of the administration unit according to the invention, the surfactant is selected from the group consisting of sodium lauryl sulfate, polyethylene-polypropylene glycol co-polymers, poly(oxyethylene)-poly(oxypropylene)-block-copolymers (e.g. poloxamers).

In a preferred embodiment of the administration unit according to the invention, the preservative is selected from the group consisting of antioxidants (e.g. vitamin A, vitamin C, vitamin E, retinyl palmitate, selenium, ascorbyl palmitate, sodium ascorbate), amino acids (e.g. cysteine or methionine), citric acid and its salts (e.g. sodium citrate), synthetic preservatives (e.g. parabens such as methyl paraben or propyl paraben; butylated hydroxyanisole, butylated hydroxytoluene, monothioglycerol, propyl gallate), hypophosphorous acid, sodium bisulfite, sodium formaldehyde sulfoxylate, and sodium metabisulfite.

In a preferred embodiment, the administration unit according to the invention passes the friability test according to Ph. Eur. Preferably, the weight loss in the friability test according to Ph. Eur. is not more than 2.0 % by weight, or not more than 1.5 % by weight, or not more than 1.0 % by weight, or not more than 0.5 % by weight, or not more than 0.4 % by weight, or not more than 0.3 % by weight, or not more than 0.2 % by weight, or not more than 0.1 % by weight.

In a preferred embodiment, the administration unit according to the invention passes the test uniformity of content of single-dose preparations according to Ph. Eur.

In a preferred embodiment, the administration unit according to the invention does not comprise any active pharmaceutical ingredient other than (i) {drug1}, (ii) {drug2}, (iii) {drug3}, (iv) {drug4}, (v) {drug5}, (vi) {drug6}, (vii) {drug7}, or (viii) {drug8}.

In a preferred embodiment, the administration unit according to the invention is for storage not above 50°C. Preferably, it is for storage not above 25°C. In a preferred embodiment, the administration unit according to the invention is not identical with any of the administration units that are individualized in the experimental section of WO2013/088335, WO2013/087546, WO2013/109514, WO2013/126326, WO2013/126394, WO2013/130402, WO2013/15812 and WO2013/156772, respectively, which is incorporated by reference.

The invention also relates to the administration unit according to the invention for use in the treatment of a disorder or a disease in a mammal. Preferably, the mammal is a human. Preferably, the mammal is an adult.

Preferably, the mammal does not suffer from liver damage and/or kidney damage.

Preferably, the mammal is not pregnant.

Preferably, the mammal is not allergic against (i) {drug1}, (ii) {drug2}, (iii) {drug3}, (iv) {drug4}, (v) {drug5}, (vi) {drug6}, (vii) {drug7}, or (viii) {drug8}.

In a preferred embodiment, the administration unit according to the invention is administered orally with a liquid. Preferably, the liquid is water, milk, juice or lemonade.

In a preferred embodiment of the administration unit according to the invention, the administered daily dose of (i) {drug1}, (ii) {drug2}, (iii) {drug3}, (iv) {drug4}, (v) {drug5}, (vi) {drug6}, (vii) {drug7}, or (viii) {drug8} is 0.0001 mg/kg body weight to 100 mg/kg body weight. Preferably, the administered daily dose of (i) {drug1}, (ii) {drug2}, (iii) {drug3}, (iv) {drug4}, (v) {drug5}, (vi) {drug6}, (vii) {drug7}, or (viii) {drug8} is 0.0001 mg/kg body weight to 0.0003 mg/kg body weight, or 0.0003 mg/kg body weight to 0.0005 mg/kg body weight, or 0.0005 mg/kg body weight to 0.001 mg/kg body weight, or 0.001 mg/kg body weight to 0.0025 mg/kg body weight, or 0.0025 mg/kg body weight to 0.005 mg/kg body weight, or 0.005 mg/kg body weight to 0.01 mg/kg body weight, or 0.01 mg/kg body weight to 0.025 mg/kg body weight, or 0.025 mg/kg body weight to 0.05 mg/kg body weight, or 0.05 mg/kg body weight to 0.1 mg/kg body weight, or 0.1 mg/kg body weight to 0.25 mg/kg body weight, or 0.25 mg/kg body weight to 0.5 mg/kg body weight, or 0.5 mg/kg body weight to 1 mg/kg body weight, or 1 mg/kg body weight to 2.5 mg/kg body weight, or 2.5 mg/kg body weight to 5 mg/kg body weight, or 5 mg/kg body weight to 10 mg/kg body weight, or 10 mg/kg body weight to 25 mg/kg body weight, or 25 mg/kg body weight to 50 mg/kg body weight, or 50 mg/kg body weight to 100 mg/kg body weight.

In a preferred embodiment of the administration unit according to the invention, the administered daily dose of (i) {drug1}, (ii) {drug2}, (iii) {drug3}, (iv) {drug4}, (v) {drug5}, (vi) {drug6}, (vii) {drug7}, or (viii) {drug8} is 0.001 mg to 5000 mg. Preferably, the administered daily dose of (i) {drug1}, (ii) {drug2}, (iii) {drug3}, (iv) {drug4}, (v) {drug5}, (vi) {drug6}, (vii) {drug7}, or (viii) {drug8} is 0.001 mg to 0.003 mg, or 0.003 mg to 0.005 mg, or 0.005 mg to 0.01 mg, or 0.01 mg to 0.025 mg, or 0.025 mg to 0.05 mg, or 0.05 mg to 0.1 mg, or 0.1 mg to 0.25 mg, or 0.25 mg to 0.5 mg, or 0.5 mg to 1 mg, or 1 mg to 2.5 mg, or 2.5 mg to 5 mg, or 5 mg to 10 mg, or 10 mg to 25 mg, or 25 mg to 50 mg, or 50 mg to 100 mg, or 100 mg to 250 mg, or 250 mg to 500 mg, or 500 mg to 1000 mg, or 1000 mg to 1250 mg, or 1250 mg to 1500 mg, or 1500 mg to 1750 mg, or 1750 mg to 2000 mg.

The invention also relates to a packaging comprising one or more administration units according to the invention.

In a preferred embodiment, the packaging according to the invention comprises a material selected from the group consisting of paper, cardboard, paperboard, metal foil and plastic foil.

In a preferred embodiment, the packaging according to the invention is a blister packaging.

A preferred blister packaging includes but is not limited to PVC-blister, PVDC-blister, PVC/PVDC-blister, moisture-proof packaging material such as aluminium foil blister pack, alu/alu blister, transparent or opaque polymer blister with pouch.

An alternative packaging according to the invention is a polypropylene tube, glass bottle and HDPE bottle optionally containing a child-resistant feature. The primary packaging material may comprise a desiccant such as molecular sieve or silica gel to improve chemical stability of (i) {drug1}, (ii) {drug2}, (iii) {drug3}, (iv) {drug4}, (v) {drug5}, (vi) {drug6}, (vii) {drug7}, or (viii) {drug8}. Opaque packaging such as colored blister materials, tubes, brown glass bottles or the like can be used to prolong shelflife of (i) {drug1}, (ii) {drug2}, (iii) {drug3}, (iv) {drug4}, (v) {drug5}, (vi) {drug6}, (vii) {drug7}, or (viii) {drug8} by reduction of photodegradation.

In a preferred embodiment, the packaging according to the invention comprises a multitude of at least 2, or at least 3, or at least 4, or at least 5, or at least 6, or at least 7, or at least 8, or at least 9, or at least 10 administration units according to the invention.

In a preferred embodiment of the packaging according to the invention, the administration units do not substantially differ in at least one property selected from the group consisting of nature of excipients, content of excipients, content of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, content of (i) {drug1}, (ii) {drug2}, (iii) {drug3}, (iv) {drug4}, (v) {drug5}, (vi) {drug6}, (vii) {drug7}, or (viii) {drug8}, water content, total weight, release profile, disintegration time, size, shape, porosity, apparent density, true density, average pore size, pore size distribution, axial tensile strength, radial tensile strength, friability, and storage stability. Preferably, the administration units differ in said at least one property by not more than 5 %, or not more than 4 %, or not more than 3 %, or not more than 2 %, or not more than 1 %, or not more than 0.5 %, or not more than 0.1 %. Preferably, all administration units are substantially identical.

The invention also relates to the packaging according to the invention for use in the treatment of a disorder or a disease, wherein one or more administration units of said multitude are administered on a first day, and one or more administration units of said multitude are administered on a second day following said first day.

In a preferred embodiment of the packaging according to the invention, one or more administration units of said multitude are administered on one or more subsequent days following said second day. Preferably, the time interval between the administration of an administration unit and the administration of a subsequent administration unit is not more than 48 hours. Preferably, the time interval is not more than 36 hours, or not more than 24 hours, or not more than 12 hours, or not more than 8 hours, or not more than 6 hours, or not more than 4 hours.

In a preferred embodiment of the packaging according to the invention, the administration units are administered on not more than 30 subsequent days. Preferably, the administration units are administered on not more than 25 subsequent days, or on not more than 20 subsequent days, or on not more than 14 subsequent days, or on not more than 10 subsequent days, or on not more than 7 subsequent days, or on not more than 3 subsequent days.

In a preferred embodiment of the packaging according to the invention, the administered daily dose of (i) {drug1}, (ii) {drug2}, (iii) {drug3}, (iv) {drug4}, (v) {drug5}, (vi) {drug6}, (vii) {drug7}, or (viii) {drug8} is 0.0001 mg/kg body weight to 100 mg/kg body weight. Preferably, the administered daily dose of (i) {drug1}, (ii) {drug2}, (iii) {drug3}, (iv) {drug4}, (v) {drug5}, (vi) {drug6}, (vii) {drug7}, or (viii) {drug8} is 0.0001 mg/kg body weight to 0.0003 mg/kg body weight, or 0.0003 mg/kg body weight to 0.0005 mg/kg body weight, or 0.0005 mg/kg body weight to 0.001 mg/kg body weight, or 0.001 mg/kg body weight to 0.0025 mg/kg body weight, or 0.0025 mg/kg body weight to 0.005 mg/kg body weight, or 0.005 mg/kg body weight to 0.01 mg/kg body weight, or 0.01 mg/kg body weight to 0.025 mg/kg body weight, or 0.025 mg/kg body weight to 0.05 mg/kg body weight, or 0.05 mg/kg body weight to 0.1 mg/kg body weight, or 0.1 mg/kg body weight to 0.25 mg/kg body weight, or 0.25 mg/kg body weight to 0.5 mg/kg body weight, or 0.5 mg/kg body weight to 1 mg/kg body weight, or 1 mg/kg body weight to 2.5 mg/kg body weight, or 2.5 mg/kg body weight to 5 mg/kg body weight, or 5 mg/kg body weight to 10 mg/kg body weight, or 10 mg/kg body weight to 25 mg/kg body weight, or 25 mg/kg body weight to 50 mg/kg body weight, or 50 mg/kg body weight to 100 mg/kg body weight.

In a preferred embodiment of the packaging according to the invention, the administered daily dose of (i) {drug1}, (ii) {drug2}, (iii) {drug3}, (iv) {drug4}, (v) {drug5}, (vi) {drug6}, (vii) {drug7}, or (viii) {drug8} is 0.001 mg to 5000 mg. Preferably, the administered daily dose of (i) {drug1}, (ii) {drug2}, (iii) {drug3}, (iv) {drug4}, (v) {drug5}, (vi) {drug6}, (vii) {drug7}, or (viii) {drug8} is 0.001 mg to 0.003 mg, or 0.003 mg to 0.005 mg, or 0.005 mg to 0.01 mg, or 0.01 mg to 0.025 mg, or 0.025 mg to 0.05 mg, or 0.05 mg to 0.1 mg, or 0.1 mg to 0.25 mg, or 0.25 mg to 0.5 mg, or 0.5 mg to 1 mg, or 1 mg to 2.5 mg, or 2.5 mg to 5 mg, or 5 mg to 10 mg, or 10 mg to 25 mg, or 25 mg to 50 mg, or 50 mg to 100 mg, or 100 mg to 250 mg, or 250 mg to 500 mg, or 500 mg to 1000 mg, or 1000 mg to 1250 mg, or 1250 mg to 1500 mg, or 1500 mg to 1750 mg, or 1750 mg to 2000 mg.

In a preferred embodiment, the packaging according to the invention does not comprise any administration unit comprising an active pharmaceutical ingredient other than (i) {drug1}, (ii) {drug2}, (iii) {drug3}, (iv) {drug4}, (v) {drug5}, (vi) {drug6}, (vii) {drug7}, or (viii) {drug8}.

The invention also relates to a method for manufacturing an administration unit according to the invention or a packaging according to the invention, comprising the steps:

(a) providing a quantity of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} that exceeds the dose of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i},

{drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} to be contained in a single administration unit by a factor of at least 10;

(b) mixing the quantity of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} with one or more pharmaceutical excipients;

(c) dividing the mixture obtained in step (b) in fractions each containing the dose of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} to be contained in a single administration unit;

(d) optionally, forming the fractions obtained in step (c) to administration units; and

(e) optionally, packing the fractions obtained in step (c) and the administration units obtained in step (d), respectively.

In a preferred embodiment of the method according to the invention, in step (a) the quantity of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} exceeds the dose of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} to be contained in a single administration unit by a factor of at least 25, or at least 50, or at least 100, or at least 250, or at least 500, or at least 1000, or at least 2500, or at least 5000, or at least 10,000.

The invention also relates to particles of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} having a particle size distribution X90 of about 500 $\mu$m or less, i.e. a particle size distribution (preferably by volume) such that at least 90 % of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} has a particle size smaller than 500 $\mu$m. Preferably, for (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} the particle size distribution of X90 is about 480 $\mu$m or less, or about 460 $\mu$m or less, or about 440 $\mu$m or less, or about 420 $\mu$m or less, or about 400 $\mu$m or less, or about 380 $\mu$m or less, or about 360 $\mu$m or less, or about 340 $\mu$m or less, or about 320 $\mu$m or less, or about 300 $\mu$m or less, or about 280 $\mu$m or less, or about 260 $\mu$m or less, or about 240 $\mu$m or less, or about 220 $\mu$m or less, or about 200 $\mu$m or less, or about 180 $\mu$m or less, or about 160 $\mu$m or less, or about 140 $\mu$m or less, or about 120 $\mu$m or less, or about 100 $\mu$m or less, or about 80 $\mu$m or less, or about 60 $\mu$m or less, or about 40 $\mu$m or less, or about 20 $\mu$m or less.

In a preferred embodiment, the particles of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} according to the invention have a particle size distribution X50 of about 400 $\mu$m or less, i.e. a particle size distribution (preferably by volume) such that at least 50 % of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c},

or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} has a particle size smaller than 400 μm. Preferably, for (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} the particle size distribution of X50 is about 380 μm or less, or about 360 μm or less, or about 340 μm or less, or about 320 μm or less, or about 300 μm or less, or about 280 μm or less, or about 260 μm or less, or about 240 μm or less, or about 220 μm or less, or about 200 μm or less, or about 180 μm or less, or about 160 μm or less, or about 140 μm or less, or about 120 μm or less, or about 100 μm or less, or about 80 μm or less, or about 70 μm or less, or about 60 μm or less, or about 50 μm or less. Preferably, for (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} the particle size distribution of X50 is about 45 μm or less, or about 40 μm or less, or about 35 μm or less, or about 30 μm or less, or about 25 μm or less, or about 20 μm or less, or about 15 μm or less, or about 10 μm or less.

In preferred embodiments, the particles of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} have a cumulative distribution by volume basis Q3(X) (X in μm) of Q3(355) <0.50; or Q3(180) <0.50 and Q3(355) ≥0.50; or Q3(125) <0.50 and Q3(180) ≥0.50; or Q3(125) ≥0.50.

In preferred embodiments, the particles of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} have a cumulative distribution by volume basis Q3(X) (X in μm) of Q3(268) <0.50 and Q3(355) ≥0.50; or Q3(180) <0.50 and Q3(268) ≥0.50; or Q3(153) <0.50 and Q3(180) ≥0.50; or Q3(125) <0.50 and Q3(153) ≥0.50; or Q3(111) <0.50 and Q3(125) ≥0.50; or Q3(98) <0.50 and Q3(111) ≥0.50; or Q3(98) ≥0.50.

In preferred embodiments, the particles of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} have a cumulative distribution by volume basis Q3(X) (X in μm) of Q3(312) <0.50 and Q3(355) ≥0.50; or Q3(268) <0.50 and Q3(312) ≥0.50; or Q3(224) <0.50 and Q3(268) ≥0.50; or Q3(180) <0.50 and Q3(224) ≥0.50; or Q3(167) <0.50 and Q3(180) ≥0.50; or Q3(153) <0.50 and Q3(167) ≥0.50; or Q3(139) <0.50 and Q3(153) ≥0.50; or Q3(125) <0.50 and Q3(139) ≥0.50; or Q3(111) <0.50 and Q3(125) ≥0.50; or Q3(98) <0.50 and Q3(111) ≥0.50; or Q3(84) <0.50 and Q3(98) ≥0.50; or Q3(84) ≥0.50.

The indicated particle size properties are determined by laser-diffraction method, in particular low angle laser light scattering, i.e. Fraunhofer diffraction. Alternatively, the particle size properties can be also determined by microscopy (e.g. electron microscopy or scanning electron microscopy). The powder fineness is preferably determined in accordance with USP35 <811>. The results of the particle size distribution determined by different techniques can be correlated with one another. Suitable milling and grinding techniques that are suitable for obtaining a specific particle size distribution are known to the skilled artisan (see e.g. N. Rasenack, B.W. Müller, Micron-size drug particles: common and novel micronization techniques, Pharm Dev Technol., 2004, 9(1):1-13; A. Martin, M.J. Cocero, Micronization processes with supercritical fluids: fundamentals and mechanisms, Adv Drug Deliv Rev. 2008, 60(3):339-50; S.C. Gad, Pharmaceutical Manufacturing Handbook: Production and Processes (Pharmaceutical Development Series), Wiley Interscience, 1st ed. 2008; A.J. Hickey, Pharmaceutical Process Engineering (Drugs and the Pharmaceutical Sciences), Informa Healthcare, 2nd ed. 2009; B. Nickerson, Sample Preparation of Pharmaceutical Dosage Forms: Challenges and Strategies for Sample Preparation and Extraction, Springer, 1st ed. 2011; D. Dragoman, Optical Characteriaztion of Solids, Springer, 1st ed. 2010; and D. Schulze; Powders and Bulk Solids: Behavior, Characterization, Storage and Flow, Springer, 2008).

PREFERRED EMBODIMENTS OF THE INVENTION ACCORDING TO ITEMS 1 TO 137

1. An administration unit comprising (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or(viii) {drug8a}, {drug8b}, {drug8c}, or {drug8d}.
2. The administration unit according to item 1, comprising a therapeutically effective amount of (i) {drug1}, (ii) {drug2}, (iii) {drug3}, (iv) {drug4}, (v) {drug5}, (vi) {drug6}, (vii) {drug7}, or (viii) {drug8}, wherein at least 10 % by weight of said therapeutically effective amount of (i) {drug1}, (ii) {drug2}, (iii) {drug3}, (iv) {drug4}, (v) {drug5}, (vi) {drug6}, (vii) {drug7}, or (viii) {drug8} are present as (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}.
3. The administration unit according to item 2, wherein at least 20 % by weight, or at least 30 % by weight, or at least 40 % by weight, or at least 50 % by weight, or at least 60 % by weight, or at least 70 % by weight, or at least 80 % by weight, or at least 90 % by weight of said therapeutically effective amount of (i) {drug1}, (ii) {drug2}, (iii) {drug3}, (iv) {drug4}, (v) {drug5}, (vi) {drug6}, (vii) {drug7}, or (viii) {drug8} are present as (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}.
4. The administration unit according to any of items 2 to 3, wherein at least 95 % by weight, or at least 96 % by weight, or at least 97 % by weight, or at least 98 % by weight, or at least 99 % by weight, or at least 99.5 % by weight, or at least 99.8 % by weight, or at least 99.9 % by weight of said therapeutically effective amount of (i) {drug1}, (ii) {drug2}, (iii) {drug3}, (iv) {drug4}, (v) {drug5}, (vi) {drug6}, (vii) {drug7}, or (viii) {drug8} are present as (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}.
5. The administration unit according to any of items 2 to 4, wherein not more than 90 % by weight of said therapeutically effective amount of (i) {drug1}, (ii) {drug2}, (iii) {drug3}, (iv) {drug4}, (v) {drug5}, (vi) {drug6}, (vii) {drug7}, or (viii) {drug8} are amorphous.
6. The administration unit according to item 5, wherein not more than 80 % by weight, or not more than 70 % by weight, or not more than 60 % by weight, or not more than 50 % by weight, or not more than 40 % by weight, or not more than 30 % by weight, or not more than 20 % by weight, or not more than 10 % by weight of said therapeutically effective amount of (i) {drug1}, (ii) {drug2}, (iii) {drug3}, (iv) {drug4}, (v) {drug5}, (vi) {drug6}, (vii) {drug7}, or (viii) {drug8} are amorphous.
7. The administration unit according to item 5 or 6, wherein not more than 5 % by weight, or not more than 4 % by weight, or not more than 3 % by weight, or not more than 2 % by weight, or not more than 1 % by weight, or not more than 0.5 % by weight, or not more than 0.2 % by weight, or not more than 0.1 % by weight of said therapeutically effective amount of (i) {drug1}, (ii) {drug2}, (iii) {drug3}, (iv) {drug4}, (v) {drug5}, (vi) {drug6}, (vii) {drug7}, or (viii) {drug8} are amorphous.
8. The administration unit according to any of items 2 to 7, wherein not more than 90 % by weight of said therapeutically effective amount of (i) {drug1}, (ii) {drug2}, (iii) {drug3}, (iv) {drug4}, (v) {drug5}, (vi) {drug6}, (vii) {drug7}, or (viii) {drug8} are polymorphs of (i) {drug1}, (ii) {drug2}, (iii) {drug3}, (iv) {drug4}, (v) {drug5}, (vi) {drug6}, (vii) {drug7}, or (viii) {drug8} other than (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}.
9. The administration unit according to item 8, wherein not more than 80 % by weight, or not more than 70 % by weight, or not more than 60 % by weight, or not more than 50 % by weight, or not more than 40 % by weight, or not

more than 30 % by weight, or not more than 20 % by weight, or not more than 10 % by weight of said therapeutically effective amount of (i) {drug1}, (ii) {drug2}, (iii) {drug3}, (iv) {drug4}, (v) {drug5}, (vi) {drug6}, (vii) {drug7}, or (viii) {drug8} are polymorphs of (i) {drug1}, (ii) {drug2}, (iii) {drug3}, (iv) {drug4}, (v) {drug5}, (vi) {drug6}, (vii) {drug7}, or (viii) {drug8} other than (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}.

10. The administration unit according to item 8 or 9, wherein not more than 5 % by weight, or not more than 4 % by weight, or not more than 3 % by weight, or not more than 2 % by weight, or not more than 1 % by weight, or not more than 0.5 % by weight, or not more than 0.2 % by weight, or not more than 0.1 % by weight of said therapeutically effective amount of (i) {drug1}, (ii) {drug2}, (iii) {drug3}, (iv) {drug4}, (v) {drug5}, (vi) {drug6}, (vii) {drug7}, or (viii) {drug8} are polymorphs of (i) {drug1}, (ii) {drug2}, (iii) {drug3}, (iv) {drug4}, (v) {drug5}, (vi) {drug6}, (vii) {drug7}, or (viii) {drug8} other than (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}.

11. The administration unit according to any of items 2 to 10, wherein therapeutically effective amount of (i) {drug1}, (ii) {drug2}, (iii) {drug3}, (iv) {drug4}, (v) {drug5}, (vi) {drug6}, (vii) {drug7}, or (viii) {drug8} is 0.1 μg to 2 g of (i) {drug1}, (ii) {drug2}, (iii) {drug3}, (iv) {drug4}, (v) {drug5}, (vi) {drug6}, (vii) {drug7}, or (viii) {drug8}.

12. The administration unit according to item 11, wherein therapeutically effective amount of (i) {drug1}, (ii) {drug2}, (iii) {drug3}, (iv) {drug4}, (v) {drug5}, (vi) {drug6}, (vii) {drug7}, or (viii) {drug8} is 1 μg to 2.5 μg of (i) {drug1}, (ii) {drug2}, (iii) {drug3}, (iv) {drug4}, (v) {drug5}, (vi) {drug6}, (vii) {drug7}, or (viii) {drug8}, or 2.5 μg to 5 μg of (i) {drug1}, (ii) {drug2}, (iii) {drug3}, (iv) {drug4}, (v) {drug5}, (vi) {drug6}, (vii) {drug7}, or (viii) {drug8}, or 5 μg to 10 μg of (i) {drug1}, (ii) {drug2}, (iii) {drug3}, (iv) {drug4}, (v) {drug5}, (vi) {drug6}, (vii) {drug7}, or (viii) {drug8}, or 10 μg to 25 μg of (i) {drug1}, (ii) {drug2}, (iii) {drug3}, (iv) {drug4}, (v) {drug5}, (vi) {drug6}, (vii) {drug7}, or (viii) {drug8}, or 25 μg to 50 μg of (i) {drug1}, (ii) {drug2}, (iii) {drug3}, (iv) {drug4}, (v) {drug5}, (vi) {drug6}, (vii) {drug7}, or (viii) {drug8}, or 50 μg to 100 μg of (i) {drug1}, (ii) {drug2}, (iii) {drug3}, (iv) {drug4}, (v) {drug5}, (vi) {drug6}, (vii) {drug7}, or (viii) {drug8}, or 100 μg to 250 μg of (i) {drug1}, (ii) {drug2}, (iii) {drug3}, (iv) {drug4}, (v) {drug5}, (vi) {drug6}, (vii) {drug7}, or (viii) {drug8}, or 250 μg to 500 μg of (i) {drug1}, (ii) {drug2}, (iii) {drug3}, (iv) {drug4}, (v) {drug5}, (vi) {drug6}, (vii) {drug7}, or (viii) {drug8}, or 500 μg to 1 mg of (i) {drug1}, (ii) {drug2}, (iii) {drug3}, (iv) {drug4}, (v) {drug5}, (vi) {drug6}, (vii) {drug7}, or (viii) {drug8}, or 1 mg to 2.5 mg of (i) {drug1}, (ii) {drug2}, (iii) {drug3}, (iv) {drug4}, (v) {drug5}, (vi) {drug6}, (vii) {drug7}, or (viii) {drug8}, or 2.5 mg to 5 mg of (i) {drug1}, (ii) {drug2}, (iii) {drug3}, (iv) {drug4}, (v) {drug5}, (vi) {drug6}, (vii) {drug7}, or (viii) {drug8}, or 5 mg to 10 mg of (i) {drug1}, (ii) {drug2}, (iii) {drug3}, (iv) {drug4}, (v) {drug5}, (vi) {drug6}, (vii) {drug7}, or (viii) {drug8}, or 10 mg to 25 mg of (i) {drug1}, (ii) {drug2}, (iii) {drug3}, (iv) {drug4}, (v) {drug5}, (vi) {drug6}, (vii) {drug7}, or (viii) {drug8}, or 25 mg to 50 mg of (i) {drug1}, (ii) {drug2}, (iii) {drug3}, (iv) {drug4}, (v) {drug5}, (vi) {drug6}, (vii) {drug7}, or (viii) {drug8}, or 50 mg to 100 mg of (i) {drug1}, (ii) {drug2}, (iii) {drug3}, (iv) {drug4}, (v) {drug5}, (vi) {drug6}, (vii) {drug7}, or (viii) {drug8}, or 100 mg to 250 mg of (i) {drug1}, (ii) {drug2}, (iii) {drug3}, (iv) {drug4}, (v) {drug5}, (vi) {drug6}, (vii) {drug7}, or (viii) {drug8}, or 250 mg to 500 mg of (i) {drug1}, (ii) {drug2}, (iii) {drug3}, (iv) {drug4}, (v) {drug5}, (vi) {drug6}, (vii) {drug7}, or (viii) {drug8}, or 500 mg to 1 g of (i) {drug1}, (ii) {drug2}, (iii) {drug3}, (iv) {drug4}, (v) {drug5}, (vi) {drug6}, (vii) {drug7}, or (viii) {drug8}, or 1 g to 1.25 g of (i) {drug1}, (ii) {drug2}, (iii) {drug3}, (iv) {drug4}, (v) {drug5}, (vi) {drug6}, (vii) {drug7}, or (viii) {drug8}, or 1.25 g to 1.5 g of (i) {drug1}, (ii) {drug2}, (iii) {drug3}, (iv) {drug4}, (v) {drug5}, (vi) {drug6}, (vii) {drug7}, or (viii) {drug8}, or 1.5 g to 1.75 g of (i) {drug1}, (ii) {drug2}, (iii) {drug3}, (iv) {drug4}, (v) {drug5}, (vi) {drug6}, (vii) {drug7}, or (viii) {drug8}, or 1.75 g to 2 g of (i) {drug1}, (ii) {drug2}, (iii) {drug3}, (iv) {drug4}, (v) {drug5}, (vi) {drug6}, (vii) {drug7}, or (viii) {drug8}.

13. The administration unit according to any of items 1 to 12, comprising 0.1 μg to 2 g of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}.

14. The administration unit according to item 13, comprising 1 μg to 2.5 μg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b},

{drug8c}, or {drug8d}, or 2.5 μg to 5 μg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 5 μg to 10 μg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 10 μg to 25 μg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 25 μg to 50 μg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 50 μg to 100 μg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 100 μg to 250 μg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 250 μg to 500 μg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 500 μg to 1 mg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 1 mg to 2.5 mg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 2.5 mg to 5 mg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 5 mg to 10 mg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 10 mg to 25 mg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 25 mg to 50 mg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c};

(v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 50 mg to 100 mg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 100 mg to 250 mg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 250 mg to 500 mg of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 500 mg to 1 g of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 1 g to 1.25 g of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 1.25 g to 1.5 g of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 1.5 g to 1.75 g of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, or 1.75 g to 2 g of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}.

15. The administration unit according to any of items 1 to 14, which is solid, semisolid or liquid.

16. The administration unit according to any of items 1 to 15, which is for administration once daily, or twice daily, or thrice daily, or four times daily, or five times daily, or six times daily.

17. The administration unit according to any of items 1 to 16, which is monolithic or multiparticulate.

18. The administration unit according to any of items 1 to 17, wherein (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} is homogeneously distributed over the administration unit.

19. The administration unit according to any of items 1 to 17, wherein (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} is not homogeneously distributed over the administration unit.

20. The administration unit according to any of items 1 to 19, providing controlled release of (i) {drug1}, (ii) {drug2},

(iii) {drug3}, (iv) {drug4}, (v) {drug5}, (vi) {drug6}, (vii) {drug7}, or (viii) {drug8}.

21. The administration unit according to item 20, comprising a controlled release matrix or a controlled release coating.

22. The administration unit according to any of items 1 to 19, providing immediate release of (i) {drug1}, (ii) {drug2}, (iii) {drug3}, (iv) {drug4}, (v) {drug5}, (vi) {drug6}, (vii) {drug7}, or (viii) {drug8}.

23. The administration unit according to any of items 1 to 22, having a total weight of 10 mg to 3 g.

24. The administration unit according to item 23, having a total weight of 10 to 25 mg, or 25 mg to 50 mg, or 50 mg to 100 mg, or 100 mg to 250 mg, or 250 mg to 500 mg, or 500 mg to 1000 mg, or 1000 mg to 2500 mg.

25. The administration unit according to any of items 1 to 24, which is for systemic or topical administration.

26. The administration unit according to any of items 1 to 25, which is for parenteral administration.

27. The administration unit according to any of items 1 to 26, which is for oral, buccal, opthalmic, nasal, rectal, transmucosal, or intestinal administration.

28. The administration unit according to any of items 1 to 27, which is for oral administration.

29. The administration unit according to item 28, which is selected from the group consisting of tablets, capsules, effervescent tablets, orodispersible tablets, dragees, sachets, drops, suspensions, and powders.

30. The administration unit according to any of items 1 to 29, which is a tablet.

31. The administration unit according to item 30, which is round or oblong; and/or which is planar or biconvex.

32. The administration unit according to any of items 30 to 31, having been manufactured by direct compression.

33. The administration unit according to any of items 30 to 31, having been manufactured by granulation and subsequent compression of granules.

34. The administration unit according to item 33, wherein granulation is wet granulation or dry granulation.

35. The administration unit according to any of items 30 to 34, having been compacted at a pressure of 10 MPa to 10,000 MPa.

36. The administration unit according to item 35, having been compacted at a pressure of 10 MPa to 25 MPa, or 25 MPa to 50 MPa, or 50 MPa to 100 MPa, or 100 MPa to 250 MPa, or 250 MPa to 500 MPa, or 500 MPa to 1000 MPa, or 1000 MPa to 2500 MPa, or 2500 MPa to 5000 MPa, or 5000 MPa to 10,000 MPa.

37. The administration unit according to any of items 30 to 36, comprising a film coating.

38. The administration unit according to item 37, wherein the film coating is enteric.

39. The administration unit according to any of items 37 to 38, wherein the film coating comprises a cellulose ether (e.g. hydroxypropylmethyl cellulose) or a synthetic polymer (e.g. polyvinyl alcohol).

40. The administration unit according to any of items 37 to 39, wherein the film coating has an average thickness of 0.01 to 1000 μm.

41. The administration unit according to item 40, wherein the film coating has an average thickness of 0.01 μm to 0.03 μm, or 0.03 to 0.05 μm, or 0.05 to 0.1 μm, or 0.1 to 0.25 μm, or 0.25 to 0.5 μm, or 0.5 to 1.0 μm, or 1.0 μm to 2.5 μm, or 2.5μ m to 5.0 μm, or 5.0 μm to 10 μm, or 10 μm to 25 μm, or 25 μm to 50 μm, or 50 μm to 100 μm, or 100 μm to 250 μm, or 250 μm to 500 μm, or 500 μm to 1000 μm.

42. The administration unit according to any of items 30 to 41, having a tablet porosity of not more than 90%.

43. The administration unit according to item 42, having a tablet porosity of not more than 80 %, or not more than 70 %, or not more than 60 %, or not more than 50 %, or not more than 40 %, or not more than 30 %, or not more than 20 %, or not more than 10 %.

44. The administration unit according to item 42 to 43, having a tablet porosity of not more than 5 %, or not more than 4 %, or not more than 3 %, or not more than 2 %, or not more than 1 %, or not more than 0.5 %, or not more than 0.2 %, or not more than 0.1 %.

45. The administration unit according to any of items 30 to 44, having a radial tensile strength of 0.1 to 100 MPa.

46. The administration unit according to item 45, having a radial tensile strength of 0.1 MPa to 0.3 MPa, or 0.3 MPa to 0.5 MPa, or 0.5 MPa to 1.0 MPa, or 1.0 MPa to 2.5 MPa, or 2.5 MPa to 5.0 MPa, or 5.0 MPa to 10 MPa, or 10 MPa to 25 MPa, or 25 MPa to 50 MPa, or 50 MPa to 100 MPa.

47. The administration unit according to any of items 30 to 46, having an axial tensile strength of 0.1 to 100 MPa.

48. The administration unit according to item 47, having an axial tensile strength of 0.1 MPa to 0.3 MPa, or 0.3 MPa to 0.5 MPa, or 0.5 MPa to 1.0 MPa, or 1.0 MPa to 2.5 MPa, or 2.5 MPa to 5.0 MPa, or 5.0 MPa to 10 MPa, or 10 MPa to 25 MPa, or 25 MPa to 50 MPa, or 50 MPa to 100 MPa.

49. The administration unit according to any of items 30 to 48, having an average pore size of 0.001 μm to 1000 μm.

50. The administration unit according to item 49, having an average pore size of 0.001 μm to 0.003 μm, or 0,003 μm to 0.005 μm, or 0.005 μm to 0.01 μm, or 0.01 μm to 0.025 μm, or 0.025 μm to 0.05 μm, or 0.05 μm to 0.1 μm, or 0.1 μm to 0.25 μm, or 0.25 μm to 0.5 μm, or 0.5 μm to 1 μm, or 1 μm to 2.5 μm, or 2.5 μm to 5 μm, or 5 μm to 10 μm, or 10 μm to 25 μm, or 25 μm to 50 μm, or 50 μm to 100 μm, or 100 μm to 250 μm, or 250 μm to 500 μm, or 500 μm to 1000 μm.

51. The administration unit according to any of items 30 to 50, having a pore size distribution such that at least 10 % of the pores have a pore size of not more than 0.1 μm.

52. The administration unit according to item 51, having a pore size distribution such that at least 20 %, or at least 30 %, or at least 40 %, or at least 50 %, or at least 60 %, or at least 70 %, or at least 80 %, or at least 90 % of the pores have a pore size of not more than 0.1 μm.

53. The administration unit according to any of items 30 to 52, having a pore size distribution such that at least 10 % of the pores have a pore size of not less than 0.1 μm.

54. The administration unit according to item 53, having a pore size distribution such that at least 20 %, or at least 30 %, or at least 40 %, or at least 50 %, or at least 60 %, or at least 70 %, or at least 80 %, or at least 90 % of the pores have a pore size of not less than 0.1 μm.

55. The administration unit according to any of items 30 to 54, having a pore size distribution such that at least 10 % of the pores have a pore size of not more than 1 μm.

56. The administration unit according to item 55, having a pore size distribution such that at least 20 %, or at least 30 %, or at least 40 %, or at least 50 %, or at least 60 %, or at least 70 %, or at least 80 %, or at least 90 % of the pores have a pore size of not more than 1 μm.

57. The administration unit according to any of items 30 to 56, having a pore size distribution such that at least 10 % of the pores have a pore size of not less than 1 μm.

58. The administration unit according to item 57, having a pore size distribution such that at least 20 %, or at least 30 %, or at least 40 %, or at least 50 %, or at least 60 %, or at least 70 %, or at least 80 %, or at least 90 % of the pores have a pore size of not less than 1 μm.

59. The administration unit according to any of items 30 to 58, having a pore size distribution such that at least 10 % of the pores have a pore size of not more than 10 μm.

60. The administration unit according to item 59, having a pore size distribution such that at least 20 %, or at least 30 %, or at least 40 %, or at least 50 %, or at least 60 %, or at least 70 %, or at least 80 %, or at least 90 % of the pores have a pore size of not more than 10 μm.

61. The administration unit according to any of items 30 to 60, having a pore size distribution such that at least 10 % of the pores have a pore size of not less than 10 μm.

62. The administration unit according to item 61, having a pore size distribution such that at least 20 %, or at least 30 %, or at least 40 %, or at least 50 %, or at least 60 %, or at least 70 %, or at least 80 %, or at least 90 % of the pores have a pore size of not less than 10 μm.

63. The administration unit according to any of items 30 to 62, having a water content of not more than 5 % by weight, relative to the total weight of the tablet.

64. The administration unit according to item 63, having a water content of not more than 4 % by weight, or not more than 3 % by weight, or not more than 2 % by weight, or not more than 1 % by weight, or not more than 0.5 % by weight, or not more than 0.1 % by weight, relative to the total weight of the tablet.

65. The administration unit according to any of items 30 to 64, having a true density of 0.60 to 1.40 g cm-3.

66. The administration unit according to item 65, having a true density of 0.60 to 0.65 g cm-3, or 0.65 to 0.70 g cm-3, or 0.70 to 0.75 g cm-3, or 0.75 to 0.80 g cm-3, or 0.80 to 0.85 g cm-3, or 0.85 to 0.90 g cm-3, or 0.90 to 0.95 g cm-3, or 0.95 to 1.00 g cm-3, or 1.00 to 1.05 g cm-3, or 1.05 to 1.10 g cm-3, or 1.10 to 1.15 g cm-3, or 1.15 to 1.20 g cm-3, or 1.20 to 1.25 g cm-3, or 1.25 to 1.30 g cm-3, or 1.30 to 1.35 g cm-3, or 1.35 to 1.40 g cm-3.

67. The administration unit according to any of items 30 to 66, having an apparent density of 0.60 to 1.40 g cm-3.

68. The administration unit according to item 67, having an apparent density of 0.60 to 0.65 g cm-3, or 0.65 to 0.70 g cm-3, or 0.70 to 0.75 g cm-3, or 0.75 to 0.80 g cm-3, or 0.80 to 0.85 g cm-3, or 0.85 to 0.90 g cm-3, or 0.90 to 0.95 g cm-3, or 0.95 to 1.00 g cm-3, or 1.00 to 1.05 g cm-3, or 1.05 to 1.10 g cm-3, or 1.10 to 1.15 g cm-3, or 1.15 to 1.20 g cm-3, or 1.20 to 1.25 g cm-3, or 1.25 to 1.30 g cm-3, or 1.30 to 1.35 g cm-3, or 1.35 to 1.40 g cm-3.

69. The administration unit according to any of items 30 to 68, disintegrating within 6000 seconds.

70. The administration unit according to item 69, disintegrating within 5500 seconds, or within 5000 seconds, or within 4500 seconds, or within 4000 seconds, or within 3500 seconds, or within 3000 seconds, or within 2500 seconds, or within 2000 seconds, or within 1500 seconds, or within 1000 seconds, or within 750 seconds, or within 500 seconds, or within 250 seconds.

71. The administration unit according to any of items 1 to 29, which is a capsule.

72. The administration unit according to item 71, comprising a multitude of particulates comprising (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or(viii) {drug8a}, {drug8b}, {drug8c}, or {drug8d}.

73. The administration unit according to any of items 71 to 72, comprising at least 2 particulates, or at least 3 particulates, or at least 4 particulates, or at least 5 particulates, or at least 6 particulates, or at least 7 particulates, or at least 8 particulates, or at least 9 particulates, or at least 10 particulates.

74. The administration unit according to any of items 71 to 73, wherein the capsule material comprises hard gelatine.
75. The administration unit according to any of items 71 to 74, comprising at least 2 different types of particulates that differ from each other in at least one property selected from the group consisting of nature of excipients, content of excipients, content of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, content of (i) {drug1}, (ii) {drug2}, (iii) {drug3}, (iv) {drug4}, (v) {drug5}, (vi) {drug6}, (vii) {drug7}, or (viii) {drug8}, water content, total weight, size, and shape.
76. The administration unit according to any of items 1 to 75, passing the storage stability test according to Ph. Eur. under accelerated storage conditions at 40°C and 75 % rel. humidity.
77. The administration unit according to item 76, having a storage stability for at least 3 months, or at least 4 months, or at least 5 months, or at least 6 months, or at least 7 months, or at least 8 months, or at least 9 months, or at least 10 months, or at least 11 months, or at least 12 months according to Ph. Eur. under accelerated storage conditions at 40°C and 75 % rel. humidity.
78. The administration unit according to any of items 1 to 77, providing the peak plasma level within 0.1 hour to 36 hours after administration.
79. The administration unit according to item 78, providing the peak plasma level within 0.1 hour to 1 hour, or 1 hour to 2 hours, or 2 hours to 3 hours, or 3 hours to 4 hours, or 4 hours to 5 hours, or 5 hours to 6 hours, or 6 hours to 7 hours, or 7 hours to 8 hours, or 8 hours to 9 hours, or 9 hours to 10 hours, or 10 hours to 11 hours, or 11 hours to 12 hours, or 12 hours to 13 hours, or 13 hours to 14 hours, or 14 hours to 15 hours, or 15 hours to 16 hours, or 16 hours to 17 hours, or 17 hours to 18 hours, or 18 hours to 19 hours, or 19 hours to 20 hours, or 20 hours to 21 hours, or 21 hours to 22 hours, or 22 hours to 23 hours, or 23 hours to 24 hours, after administration.
80. The administration unit according to any of items 1 to 79, comprising one or more excipients independently selected from the group consisting of antiadherents, binders, disintegrants, fillers, flavors, colors, lubricants, glidants, sorbents, surfactants, preservatives and sweeteners.
81. The administration unit according to item 80, wherein the antiadherent is selected from the group consisting of silicon dioxide, talc, talc waxes, stearates (e.g. magnesium, calcium or sodium), stearic acid, stearowet, boric acid, sodium chloride, DL-leucine, sodium oleate, sodium benzoate, sodium acetate, sodium lauryl sulfate, and magnesium lauryl sulfate.
82. The administration unit according to any of items 80 to 81, wherein the binder is selected from the group consisting of saccharides and their derivatives; proteins; gums; silicates; and synthetic polymers.
83. The administration unit according to item 82, wherein the binder is a saccharide or a derivative thereof selected from the group consisting of disaccharides (e.g. sucrose, glucose or lactose); polysaccharides and their derivatives (e.g. starch, pregelatinized starch, cellulose, microcrystalline cellulose, polysaccharide acids, cellulose ethers [e.g. methyl cellulose, ethyl cellulose, hydroxyethyl cellulose, hydroxypropyl cellulose, hydroxypropylmethyl cellulose or carboxymethylcellulose sodium]); alginic acid and salts thereof (e.g. sodium alginate); and sugar alcohols (e.g. xylitol, sorbitol or maltitol); or wherein the binder is a protein selected from gelatin; or wherein the binder is a gum (e.g. acacia gum, guar gum, or tragacanth gum); or wherein the binder is a silicate (e.g. magnesium aluminum silicate or bentonite); or wherein the binder is a synthetic polymer selected from the group consisting of polyvinylpyrrolidone, polyvinylpyrrolidonevinylacetate copolymer, polymethacrylate polymer, polyvinylalcohol, and polyethylene glycol.
84. The administration unit according to any of items 80 to 83, wherein the disintegrant is selected from the group consisting of cross-linked polymers (e.g. cross-linked polyvinylpyrrolidone [crospovidone] or cross-linked sodium carboxymethyl cellulose [croscarmellose sodium]), starches (e.g. corn or potato), pregelatinized starch, modified starch (e.g. sodium starch glycolate), algenic acid, alginates (e.g. sodium alginate), carboxymethylcellulose, microcrystalline cellulose, clays (e.g. magnesium aluminum silicate), and gums (e.g. agar, guar, locust bean, karaya, pectin, or tragacanth gum).
85. The administration unit according to any of items 80 to 84, wherein the filler is selected from the group consisting of plant cellulose, microcrystalline cellulose, inorganic phosphates (e.g. dibasic calcium phosphate or tricalcium phosphate), inorganic carbonates such as calcium carbonate, inorganic sulfates such as calcium sulfate dihydrate, sugars and sugar alcohols (e.g. lactose, glucose, inositol, mannitol, sorbitol, sucrose, dextrose, maltodextrins, and fructose), magnesium stearate, calcium lactate trihydrate, starch (e.g. corn, wheat, maize, potato or rice starch), and modified starch (e.g. carboxymethyl starch).
86. The administration unit according to any of items 80 to 85, wherein the lubricant is hydrophilic or hydrophobic.
87. The administration unit according to item 86, wherein the lubricant is selected from the group consisting of talc, silica, stearin, fatty acids (e.g. stearic acid) or fatty acid derivatives, metal stearates (e.g. magnesium stearate,

calcium stearate, or zinc stearate), glyceryl monostearate, glyceryl palmitostearate, glyceryl behenate, sodium lauryl sulfate, magnesium lauryl sulfate, sodium stearyl fumarate, hydrogenated vegetable oil, polyalkylene glycols (e.g. polyethylene glycol), starch, light mineral oil, sodium benzoate, and sodium chloride.

88. The administration unit according to any of items 80 to 87, wherein the glidant is selected from the group consisting of fumed silica, cornstarch, talc, and magnesium carbonate.

89. The administration unit according to any of items 80 to 88, wherein the preservative is selected from the group consisting of antioxidants (e.g. vitamin A, vitamin C, vitamin E, retinyl palmitate, selenium, ascorbyl palmitate, sodium ascorbate), amino acids (e.g. cysteine or methionine), citric acid and its salts (e.g. sodium citrate), synthetic preservatives (e.g. parabens such as methyl paraben or propyl paraben; butylated hydroxyanisole, butylated hydroxytoluene, monothioglycerol, propyl gallate), hypophosphorous acid, sodium bisulfite, sodium formaldehyde sulfoxylate, and sodium metabisulfite.

90. The administration unit according to any of items 1 to 89, passing the friability test according to Ph. Eur.

91. The administration unit according to item 90, wherein the weight loss in the friability test according to Ph. Eur. is not more than 2.0 % by weight, or not more than 1.5 % by weight, or not more than 1.0 % by weight, or not more than 0.5 % by weight, or not more than 0.4 % by weight, or not more than 0.3 % by weight, or not more than 0.2 % by weight, or not more than 0.1 % by weight.

92. The administration unit according to any of items 1 to 91, passing the test uniformity of content of single-dose preparations according to Ph. Eur.

93. The administration unit according to any of items 1 to 92, not comprising any active pharmaceutical ingredient other than (i) {drug1}, (ii) {drug2}, (iii) {drug3}, (iv) {drug4}, (v) {drug5}, (vi) {drug6}, (vii) {drug7}, or(viii) {drug8}.

94. The administration unit according to any of items 1 to 93, which is for storage not above 50°C.

95. The administration unit according to item 94, which is for storage not above 25°C.

96. The administration unit according to any of items 1 to 95, with the proviso that the administration unit is not identical with any of the administration units that are individualized in the experimental section of WO2013/088335, WO2013/087546, WO2013/109514, WO2013/126326, WO2013/126394, WO2013/130402, WO2013/158121, and WO2013/156772, respectively.

97. The administration unit according to any of items 1 to 96 for use in the treatment of a disorder or a disease in a mammal.

98. The administration unit according to item 97, wherein the mammal is a human.

99. The administration unit according to any of items 97 to 98, wherein the mammal is an adult.

100. The administration unit according to any of items 97 to 99 wherein the mammal does not suffer from liver damage.

101. The administration unit according to any of items 97 to 100, wherein the mammal does not suffer from kidney damage.

102. The administration unit according to any of items 97 to 101, wherein the mammal is not pregnant.

103. The administration unit according to any of items 97 to 102, wherein the mammal is not allergic against (i) {drug1}, (ii) {drug2}, (iii) {drug3}, (iv) {drug4}, (v) {drug5}, (vi) {drug6}, (vii) {drug7}, or (viii) {drug8}.

104. The administration unit according to any of items 97 to 103, which is administered orally with a liquid.

105. The administration unit according to item 104, wherein the liquid is water, milk, juice or lemonade.

106. The administration unit according to any of items 97 to 105, wherein the administered daily dose of (i) {drug1}, (ii) {drug2}, (iii) {drug3}, (iv) {drug4}, (v) {drug5}, (vi) {drug6}, (vii) {drug7}, or (viii) {drug8} is 0.0001 mg/kg body weight to 100 mg/kg body weight.

107. The administration unit according to item 106, wherein the administered daily dose of (i) {drug1}, (ii) {drug2}, (iii) {drug3}, (iv) {drug4}, (v) {drug5}, (vi) {drug6}, (vii) {drug7}, or (viii) {drug8} is 0.0001 mg/kg body weight to 0.0003 mg/kg body weight, or 0.0003 mg/kg body weight to 0.0005 mg/kg body weight, or 0.0005 mg/kg body weight to 0.001 mg/kg body weight, or 0.001 mg/kg body weight to 0.0025 mg/kg body weight, or 0.0025 mg/kg body weight to 0.005 mg/kg body weight, or 0.005 mg/kg body weight to 0.01 mg/kg body weight, or 0.01 mg/kg body weight to 0.025 mg/kg body weight, or 0.025 mg/kg body weight to 0.05 mg/kg body weight, or 0.05 mg/kg body weight to 0.1 mg/kg body weight, or 0.1 mg/kg body weight to 0.25 mg/kg body weight, or 0.25 mg/kg body weight to 0.5 mg/kg body weight, or 0.5 mg/kg body weight to 1 mg/kg body weight, or 1 mg/kg body weight to 2.5 mg/kg body weight, or 2.5 mg/kg body weight to 5 mg/kg body weight, or 5 mg/kg body weight to 10 mg/kg body weight, or 10 mg/kg body weight to 25 mg/kg body weight, or 25 mg/kg body weight to 50 mg/kg body weight, or 50 mg/kg body weight to 100 mg/kg body weight.

108. The administration unit according to any of items 97 to 107, wherein the administered daily dose of (i) {drug1}, (ii) {drug2}, (iii) {drug3}, (iv) {drug4}, (v) {drug5}, (vi) {drug6}, (vii) {drug7}, or (viii) {drug8} is 0.001 mg to 5000 mg.

109. The administration unit according to item 108, wherein the administered daily dose of (i) {drug1}, (ii) {drug2}, (iii) {drug3}, (iv) {drug4}, (v) {drug5}, (vi) {drug6}, (vii) {drug7}, or (viii) {drug8} is 0.001 mg to 0.003 mg, or 0.003 mg to 0.005 mg, or 0.005 mg to 0.01 mg, or 0.01 mg to 0.025 mg, or 0.025 mg to 0.05 mg, or 0.05 mg to 0.1 mg, or 0.1 mg to 0.25 mg, or 0.25 mg to 0.5 mg, or 0.5 mg to 1 mg, or 1 mg to 2.5 mg, or 2.5 mg to 5 mg, or 5 mg to 10 mg,

or 10 mg to 25 mg, or 25 mg to 50 mg, or 50 mg to 100 mg, or 100 mg to 250 mg, or 250 mg to 500 mg, or 500 mg to 1000 mg, or 1000 mg to 1250 mg, or 1250 mg to 1500 mg, or 1500 mg to 1750 mg, or 1750 mg to 1999 mg.

110. A packaging comprising one or more administration units according to any of items 1 to 109.

111. The packaging according to item 110, comprising a material selected from the group consisting of paper, cardboard, paperboard, metal foil and plastic foil.

112. The packaging according to any of items 110 to 111, which is a blister packaging.

113. The packaging according to any of items 110 to 112, comprising a multitude of at least 2, or at least 3, or at least 4, or at least 5, or at least 6, or at least 7, or at least 8, or at least 9, or at least 10 administration units according to any of items 1 to 109.

114. The packaging according to item 113, wherein the administration units do not substantially differ in at least one property selected from the group consisting of nature of excipients, content of excipients, content of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d}, content of (i) {drug1}, (ii) {drug2}, (iii) {drug3}, (iv) {drug4}, (v) {drug5}, (vi) {drug6}, (vii) {drug7}, or (viii) {drug8}, water content, total weight, release profile, disintegration time, size, shape, porosity, apparent density, true density, average pore size, pore size distribution, axial tensile strength, radial tensile strength, friability, and storage stability.

115. The packaging according to item 114, wherein the administration units differ in said at least one property by not more than 5 %, or not more than 4 %, or not more than 3 %, or not more than 2 %, or not more than 1 %, or not more than 0.5 %, or not more than 0.1 %.

116. The packaging according to any of items 113 to 115, wherein all administration units are substantially identical.

117. The packaging according to any of items 113 to 116 for use in the treatment of a disorder or a disease, wherein one or more administration units of said multitude are administered on a first day, and one or more administration units of said multitude are administered on a second day following said first day.

118. The packaging according to item 117, wherein one or more administration units of said multitude are administered on one or more subsequent days following said second day.

119. The packaging according to any of items 117 to 118, wherein the time interval between the administration of an administration unit and the administration of a subsequent administration unit is not more than 48 hours.

120. The packaging according to item 119, wherein the time interval is not more than 36 hours, or not more than 24 hours, or not more than 12 hours, or not more than 8 hours, or not more than 6 hours, or not more than 4 hours.

121. The packaging according to any of items 117 to 120, wherein the administration units are administered on not more than 30 subsequent days.

122. The packaging according to item 121, wherein the administration units are administered on not more than 25 subsequent days, or on not more than 20 subsequent days, or on not more than 14 subsequent days, or on not more than 10 subsequent days, or on not more than 7 subsequent days, or on not more than 3 subsequent days.

123. The packaging according to any of items 117 to 122, wherein the administered daily dose of (i) {drug1}, (ii) {drug2}, (iii) {drug3}, (iv) {drug4}, (v) {drug5}, (vi) {drug6}, (vii) {drug7}, or (viii) {drug8} is 0.0001 mg/kg body weight to 100 mg/kg body weight.

124. The packaging according to item 123, wherein the administered daily dose of (i) {drug1}, (ii) {drug2}, (iii) {drug3}, (iv) {drug4}, (v) {drug5}, (vi) {drug6}, (vii) {drug7}, or (viii) {drug8} is 0.0001 mg/kg body weight to 0.0003 mg/kg body weight, or 0.0003 mg/kg body weight to 0.0005 mg/kg body weight, or 0.0005 mg/kg body weight to 0.001 mg/kg body weight, or 0.001 mg/kg body weight to 0.0025 mg/kg body weight, or 0.0025 mg/kg body weight to 0.005 mg/kg body weight, or 0.005 mg/kg body weight to 0.01 mg/kg body weight, or 0.01 mg/kg body weight to 0.025 mg/kg body weight, or 0.025 mg/kg body weight to 0.05 mg/kg body weight, or 0.05 mg/kg body weight to 0.1 mg/kg body weight, or 0.1 mg/kg body weight to 0.25 mg/kg body weight, or 0.25 mg/kg body weight to 0.5 mg/kg body weight, or 0.5 mg/kg body weight to 1 mg/kg body weight, or 1 mg/kg body weight to 2.5 mg/kg body weight, or 2.5 mg/kg body weight to 5 mg/kg body weight, or 5 mg/kg body weight to 10 mg/kg body weight, or 10 mg/kg body weight to 25 mg/kg body weight, or 25 mg/kg body weight to 50 mg/kg body weight, or 50 mg/kg body weight to 100 mg/kg body weight.

125. The packaging according to any of items 117 to 124, wherein the administered daily dose of (i) {drug1}, (ii) {drug2}, (iii) {drug3}, (iv) {drug4}, (v) {drug5}, (vi) {drug6}, (vii) {drug7}, or (viii) {drug8} is 0.001 mg to 5000 mg.

126. The packaging according to item 125, wherein the administered daily dose of (i) {drug1}, (ii) {drug2}, (iii) {drug3}, (iv) {drug4}, (v) {drug5}, (vi) {drug6}, (vii) {drug7}, or (viii) {drug8} is 0.001 mg to 0.003 mg, or 0.003 mg to 0.005 mg, or 0.005 mg to 0.01 mg, or 0.01 mg to 0.025 mg, or 0.025 mg to 0.05 mg, or 0.05 mg to 0.1 mg, or 0.1 mg to 0.25 mg, or 0.25 mg to 0.5 mg, or 0.5 mg to 1 mg, or 1 mg to 2.5 mg, or 2.5 mg to 5 mg, or 5 mg to 10 mg, or 10 mg to 25 mg, or 25 mg to 50 mg, or 50 mg to 100 mg, or 100 mg to 250 mg, or 250 mg to 500 mg, or 500 mg to 1000 mg,

or 1000 mg to 1250 mg, or 1250 mg to 1500 mg, or 1500 mg to 1750 mg, or 1750 mg to 2000 mg.

127. The packaging according to any of items 117 to 126, not comprising any administration unit comprising an active pharmaceutical ingredient other than (i) {drug1}, (ii) {drug2}, (iii) {drug3}, (iv) {drug4}, (v) {drug5}, (vi) {drug6}, (vii) {drug7}, or (viii) {drug8}.

128. A method for manufacturing an administration unit according to any of items 1 to 109 or a packaging according to any of items 110 to 127, comprising the steps: (a) providing a quantity of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} that exceeds the dose of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} to be contained in a single administration unit by a factor of at least 10; (b) mixing the quantity of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} with one or more pharmaceutical excipients; (c) dividing the mixture obtained in step (b) in fractions each containing the dose of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} to be contained in a single administration unit; (d) optionally, forming the fractions obtained in step (c) to administration units; and (e) optionally, packing the fractions obtained in step (c) and the administration units obtained in step (d), respectively.

129. The method according to item 133, wherein in step (a) the quantity of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} exceeds the dose of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i), {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} to be contained in a single administration unit by a factor of at least 25, or at least 50, or at least 100, or at least 250, or at least 500, or at least 1000, or at least 2500, or at least 5000, or at least 10,000.

130. Particles of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or(viii) {drug8a}, {drug8b}, {drug8c}, or {drug8d} having a particle size distribution X90 of 500 μm or less.

131. The particles of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} according to item 130, having a particle size distribution X90 of 480 μm or less, or 460 μm or less, or 440 μm or less, or 420 μm or less, or 400 μm or less, of 380 μm or less, or 360 μm or less, or 340 μm or less, or 320 μm or less, or 300 μm or less, of 280 μm or less, or 260 μm or less, or 240 μm or less, or 220 μm or less, or 200 μm or less, of 180 μm or less, or 160 μm or less, or 140 μm or less, or 120 μm or less, or 100 μm or less, or 80 μm or less, or 60 μm or less, or 40 μm or less, or 20 μm or less.

132. The particles of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b},

{drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} according to any of items 130 to 131, having a particle size distribution X50 of 400 μm or less.

133. The particles of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} according to item 132, having a particle size distribution X50 of 380 μm or less, or 360 μm or less, or 340 μm or less, or 320 μm or less, or 300 μm or less, of 280 μm or less, or 260 μm or less, or 240 μm or less, or 220 μm or less, or 200 μm or less, 180 μm or less, or 160 μm or less, or 140 μm or less, or 120 μm or less, or 100 μm or less, or 80 μm or less, or 70 μm or less, or 60 μm or less, or 50 μm or less.

134. The particles of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} according to item 133, having a particle size distribution X50 of 45 μm or less, or 40 μm or less, or 35 μm or less, or 30 μm or less, or 25 μm or less, or 20 μm or less, or 15 μm or less, or 10 μm or less.

135. The particles of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} according to any of items 130 to 134, having a cumulative distribution by volume basis Q3(X) (X in μm) of Q3(355) <0.50; or Q3(180) <0.50 and Q3(355) ≥0.50; or Q3(125) <0.50 and Q3(180) ≥0.50; or Q3(125) ≥0.50.

136. The particles of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} according to any of items 130 to 135, having a cumulative distribution by volume basis Q3(X) (X in μm) of Q3(268) <0.50 and Q3(355) ≥0.50; or Q3(180) <0.50 and Q3(268) ≥0.50; or Q3(153) <0.50 and Q3(180) ≥0.50; or Q3(125) <0.50 and Q3(153) ≥0.50; or Q3(111) <0.50 and Q3(125) ≥0.50; or Q3(98) <0.50 and Q3(111) ≥0.50; or Q3(98) ≥0.50.

137. The particles of (i) {drug1a}, {drug1b}, or {drug1c}; (ii) {drug2a}; (iii) {drug3a}, {drug3b}, {drug3c}, {drug3d}, {drug3e}, {drug3f}, {drug3g}, {drug3h}, {drug3i}, or {drug3j}; (iv) {drug4a}, {drug4b}, or {drug4c}; (v) {drug5a}, {drug5b}, {drug5c}, or {drug5d}; (vi) {drug6a}, {drug6b}, or {drug6c}; (vii) {drug7a}, {drug7b}, {drug7c}, {drug7d}, {drug7e}, {drug7f}, {drug7g}, {drug7h}, {drug7i}, {drug7j}, {drug7k}, {drug7l}, {drug7m}, {drug7n}, {drug7o}, {drug7p}, {drug7q}, {drug7r}, {drug7s}, or {drug7t}; or (viii) {drug8a}, {drug8b}, {drug8c}, or {drug8d} according to any of items 130 to 136, having a cumulative distribution by volume basis Q3(X) (X in μm) of Q3(312) <0.50 and Q3(355) ≥0.50; or Q3(268) <0.50 and Q3(312) ≥0.50; or Q3(224) <0.50 and Q3(268) ≥0.50; or Q3(180) <0.50 and Q3(224) ≥0.50; or Q3(167) <0.50 and Q3(180) ≥0.50; or Q3(153) <0.50 and Q3(167) ≥0.50; or Q3(139) <0.50 and Q3(153) ≥0.50; or Q3(125) <0.50 and Q3(139) ≥0.50; or Q3(111) <0.50 and Q3(125) ≥0.50; or Q3(98) <0.50 and Q3(111) ≥0.50; or Q3(84) <0.50 and Q3(98) ≥0.50; or Q3(84) ≥0.50.

## Claims

1. A packaging comprising one or more administration units comprising a solid form of 4-amino-2-(2,6-dioxopiperidine-3-yl)isoindoline-1,3-dione having an X-ray powder diffreaction pattern comprising peaks at approximately 12, 17, and 26 degrees 2Theta.

2. The packaging according to claim 1, wherein the X-ray powder diffreaction pattern further comprises peaks at 14, 18, and 24 degrees 2Theta.

3. The packaging according to claim 1 or 2, wherein the X-ray powder diffreaction pattern comprises peaks at 12.1, 14.0, 17.4, 18.4, 24.4, and 25.6 degrees 2Theta.

**4.** The packaging according to claim 1, wherein the one or more administration unit comprise an amorphous form of 4-amino-2-(2,6-dioxopiperidine-3-yl)isoindoline-1,3-dione.

**5.** The packaging according to any of claims 1 to 4, comprising a material selected from the group consisting of paper, cardboard, paperboard, metal foil and plastic foil.

**6.** The packaging according to any of claims 1 to 5, wherein the administration unit is solid, semisolid or liquid.

**7.** The packaging according to any of claims 1 to 6, wherein the administration unit is for administration once daily, or twice daily, or thrice daily, or four times daily, or five times daily, or six times daily.

**8.** The packaging according to any of claims 1 to 7, wherein the administration unit provides the peak plasma level within 0.1 hour to 36 hours after administration.

**9.** The packaging according to any of claims 1 to 8, comprising a multitude of at least 2, or at least 3, or at least 4, or at least 5, or at least 6, or at least 7, or at least 8, or at least 9, or at least 10 administration units according to any of claims 1 to 4.

**10.** The packaging according to any of claims 1 to 9, wherein all administration units are substantially identical.

**11.** The packaging according to any of claims 1 to 10, wherein the administration unit is for oral, buccal, opthalmic, nasal, rectal, transmucosal, or intestinal administration.

**12.** The packaging according to any of claims 9 to 11 for use in the treatment of a disorder or a disease, wherein one or more administration units of said multitude are administered on a first day, and one or more administration units of said multitude are administered on a second day following said first day.

**13.** The packaging according to claim 12, wherein one or more administration units of said multitude are administered on one or more subsequent days following said second day.

**14.** The packaging according to any of claims 12 to 13, wherein the time interval between the administration of an administration unit and the administration of a subsequent administration unit is not more than 48 hours.

**15.** The packaging according to any of claims 1 to 14, wherein the administration unit is for use in the treatment of a disorder or a disease in a mammal.

Europäisches Patentamt
European Patent Office
Office européen des brevets

# EUROPEAN SEARCH REPORT

Application Number
EP 14 17 2555

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 2011/224440 A1 (GE CHUANSHENG [US] ET AL) 15 September 2011 (2011-09-15)<br>* paragraphs [0003] - [0005], [0007], [0008], [0030], [0031]; examples 3,13-17 * | 1<br>1-15 | INV.<br>A61K31/45 |
| Y | US 2013/143923 A1 (GORE VINAYAK GOVIND [IN] ET AL) 6 June 2013 (2013-06-06)<br>* paragraphs [0016], [0035], [0071], [0072], [0085], [0092], [0093] * | 1-15 | |
| Y | US 2013/059889 A1 (KONAKANCHI DURGA PRASAD [IN] ET AL) 7 March 2013 (2013-03-07)<br>* paragraphs [0002] - [0019], [0032], [0033] * | 1-15 | |
| X<br>Y | EP 0 925 294 A1 (CELGENE CORP [US]) 30 June 1999 (1999-06-30)<br>* example 14 * | 1<br>1-15 | |
| X,D,P | WO 2013/126326 A1 (CELGENE CORP [US]) 29 August 2013 (2013-08-29)<br>* the whole document * | 1-15 | TECHNICAL FIELDS SEARCHED (IPC)<br>A61K |

The present search report has been drawn up for all claims

1

| Place of search | Date of completion of the search | Examiner |
|---|---|---|
| Munich | 20 October 2014 | Engl, Brigitte |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 17 2555

This annex lists the patent family members relating to the patent documents cited in the above-mentioned European search report. The members are as contained in the European Patent Office EDP file on
The European Patent Office is in no way liable for these particulars which are merely given for the purpose of information.

20-10-2014

| Patent document cited in search report | Publication date | Patent family member(s) | Publication date |
|---|---|---|---|
| US 2011224440 A1 | 15-09-2011 | AU 2006265019 A1 | 11-01-2007 |
| | | BR PI0612803 A2 | 02-10-2012 |
| | | CA 2612612 A1 | 11-01-2007 |
| | | CA 2823088 A1 | 11-01-2007 |
| | | CN 101253163 A | 27-08-2008 |
| | | CN 102643267 A | 22-08-2012 |
| | | CR 9697 A | 26-06-2008 |
| | | DK 1907373 T3 | 04-02-2013 |
| | | DK 2380887 T3 | 07-10-2013 |
| | | EC SP088154 A | 26-03-2008 |
| | | EP 1907373 A1 | 09-04-2008 |
| | | EP 2380887 A1 | 26-10-2011 |
| | | ES 2402204 T3 | 29-04-2013 |
| | | ES 2430545 T3 | 21-11-2013 |
| | | HK 1121447 A1 | 21-09-2012 |
| | | HR P20130102 T1 | 31-03-2013 |
| | | HR P20130966 T1 | 22-11-2013 |
| | | IL 188330 A | 30-09-2013 |
| | | IL 214892 A | 28-11-2013 |
| | | JP 5366544 B2 | 11-12-2013 |
| | | JP 5543555 B2 | 09-07-2014 |
| | | JP 2008544993 A | 11-12-2008 |
| | | JP 2013014599 A | 24-01-2013 |
| | | KR 20080039392 A | 07-05-2008 |
| | | KR 20130029442 A | 22-03-2013 |
| | | NZ 565309 A | 31-03-2011 |
| | | PE 01032007 A1 | 06-02-2007 |
| | | PE 01512010 A1 | 05-03-2010 |
| | | PT 1907373 E | 18-02-2013 |
| | | PT 2380887 E | 18-09-2013 |
| | | RS 52704 B | 30-08-2013 |
| | | RS 53030 B | 30-04-2014 |
| | | SI 1907373 T1 | 29-03-2013 |
| | | SI 2380887 T1 | 31-12-2013 |
| | | US 2007004920 A1 | 04-01-2007 |
| | | US 2011224440 A1 | 15-09-2011 |
| | | US 2014256950 A1 | 11-09-2014 |
| | | WO 2007005972 A1 | 11-01-2007 |
| | | ZA 200800828 A | 26-08-2009 |
| US 2013143923 A1 | 06-06-2013 | AU 2011263493 A1 | 10-01-2013 |
| | | CA 2801835 A1 | 15-12-2012 |
| | | CN 103068812 A | 24-04-2013 |
| | | EP 2580205 A1 | 17-04-2013 |
| | | JP 2013528206 A | 08-07-2013 |
| | | US 2013143923 A1 | 06-06-2013 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 14 17 2555

This annex lists the patent family members relating to the patent documents cited in the above-mentioned European search report. The members are as contained in the European Patent Office EDP file on
The European Patent Office is in no way liable for these particulars which are merely given for the purpose of information.

20-10-2014

| Patent document cited in search report | Publication date | Patent family member(s) | Publication date |
|---|---|---|---|
| | | WO 2011154739 A1 | 15-12-2011 |
| US 2013059889 A1 | 07-03-2013 | CA 2793312 A1 | 15-09-2011 |
| | | EP 2545043 A1 | 16-01-2013 |
| | | KR 20130038232 A | 17-04-2013 |
| | | US 2013059889 A1 | 07-03-2013 |
| | | US 2014121245 A1 | 01-05-2014 |
| | | WO 2011111053 A1 | 15-09-2011 |
| EP 0925294 A1 | 30-06-1999 | AT 229521 T | 15-12-2002 |
| | | AT 460409 T | 15-03-2010 |
| | | AT 500240 T | 15-03-2011 |
| | | AT 530542 T | 15-11-2011 |
| | | AU 715779 B2 | 10-02-2000 |
| | | CA 2261762 A1 | 29-01-1998 |
| | | CA 2560523 A1 | 29-01-1998 |
| | | CA 2624949 A1 | 29-01-1998 |
| | | CN 1239959 A | 29-12-1999 |
| | | CZ 302378 B6 | 20-04-2011 |
| | | CZ 304569 B6 | 09-07-2014 |
| | | CZ 9900202 A3 | 16-06-1999 |
| | | DE 69717831 D1 | 23-01-2003 |
| | | DE 69717831 T2 | 28-08-2003 |
| | | DE 122007000079 I1 | 28-02-2008 |
| | | DK 0925294 T3 | 07-04-2003 |
| | | DK 1285916 T3 | 26-04-2010 |
| | | EP 0925294 A1 | 30-06-1999 |
| | | EP 1285916 A1 | 26-02-2003 |
| | | EP 2070920 A1 | 17-06-2009 |
| | | EP 2305663 A1 | 06-04-2011 |
| | | ES 2187805 T3 | 16-06-2003 |
| | | ES 2339425 T3 | 20-05-2010 |
| | | ES 2372577 T3 | 24-01-2012 |
| | | FI 990101 A | 19-03-1999 |
| | | HK 1021819 A1 | 18-07-2003 |
| | | HK 1050893 A1 | 05-11-2010 |
| | | HK 1132502 A1 | 19-08-2011 |
| | | HK 1143360 A1 | 07-09-2012 |
| | | JP 4065567 B2 | 26-03-2008 |
| | | JP 2001503384 A | 13-03-2001 |
| | | KR 20050032629 A | 07-04-2005 |
| | | LU 91359 I2 | 06-11-2007 |
| | | NL 300291 I1 | 01-11-2007 |
| | | NZ 333903 A | 28-02-2000 |
| | | PL 195916 B1 | 30-11-2007 |
| | | PL 332867 A1 | 25-10-1999 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 14 17 2555

This annex lists the patent family members relating to the patent documents cited in the above-mentioned European search report. The members are as contained in the European Patent Office EDP file on
The European Patent Office is in no way liable for these particulars which are merely given for the purpose of information.

20-10-2014

| Patent document cited in search report | Publication date | Patent family member(s) | Publication date |
|---|---|---|---|
| | | PT 925294 E | 30-04-2003 |
| | | PT 1285916 E | 05-04-2010 |
| | | PT 2070920 E | 31-03-2011 |
| | | PT 2177517 E | 10-11-2011 |
| | | SK 9199 A3 | 12-07-1999 |
| | | WO 9803502 A1 | 29-01-1998 |
| WO 2013126326 A1 | 29-08-2013 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 13173120 A
- EP 13173118 A
- EP 13178100 A
- EP 13178101 A
- EP 13182294 A
- EP 13182299 A
- EP 13183449 A
- EP 13190172 A
- EP 13190171 A
- WO 2013088335 A
- WO 2013087546 A
- WO 2013109514 A
- WO 2013126326 A
- WO 2013126394 A
- WO 2013130402 A
- WO 2013158121 A
- WO 2013156772 A
- WO 2011051894 A
- WO 9630355 A
- WO 2005028462 A
- WO 2009115655 A
- WO 2007024945 A1
- WO 2007024945 A
- US 5635517 A
- US 6316471 B
- US 6476052 B
- US 7393863 B
- US 7629360 B
- US 7863297 B
- US 20050143420 A
- US 20060166932 A
- US 20060188475 A
- US 20070048327 A
- US 20070066512 A
- US 20070155791 A
- US 20080051431 A
- US 20080317708 A
- US 20090087407 A
- US 20090088410 A
- US 200901317385 A
- US 20090148853 A
- US 20090232776 A
- US 20090232796 A
- US 20100098657 A
- US 20100099711 A
- US 20110184025 A
- US 6335349 B
- US 7041680 B
- US 7709502 B
- US 7994327 B
- US 20060178402 A
- US 20110224440 A
- US 61500053 A
- US 6281230 B
- US 5635517 B, Muller
- WO 20040220144 A1
- WO 20040029832 A1
- WO 20040087546 A
- WO 2004103274 A
- US 5712291 A
- US 20090317385 A
- US 20030180269 A1
- US 5391485 A
- US 5393870 A
- US 5229496 A
- US 4810643 A
- US 4999291 A
- US 5528823 A
- US 5580755 A
- US 5800819 A
- US 6372213 B
- US 6538023 B
- US 20060030594 A
- US 20110045064 A
- US 4328245 A
- US 4409239 A
- US 4410545 A
- US 6350458 B
- WO 201312632 A
- US 6281230 A, Muller

- US 7189740 B
- US 7968569 B
- US 7563810 B
- US 6235756 B
- US 6114335 A, D'Amato
- US 20050203142 A1
- US 20040091455 A
- US 3845770 A
- US 3916899 A
- US 3536809 A
- US 3598123 A
- US 4008719 A
- US 5674533 A
- US 5059595 A
- US 5591767 A
- US 5120548 A
- US 5073543 A
- US 5639476 A
- US 5354556 A
- US 5733566 A
- US 5134127 A
- US 20110251223 A1
- WO 201315812 A
- WO 2006002421 A
- WO 2005075435 A
- WO 2009074575 A
- US 6099562 A
- US 5886026 A
- US 5304121 A
- WO 2009047563 A

**Non-patent literature cited in the description**


- **GOODMAN C et al.** *Health Policy Plan.,* November 2007, vol. 22 (6), 393-403
- **SOW PS et al.** *Int J Infect Dis.,* June 2002, vol. 6 (2), 108-12
- **GHEBRE-SELLASSIE, I. ; C. MARTIN.** Pharmaceutical Extrusion Technology. Marcel Dekker, 2003
- **LACHMAN et al.** The Theory and Practice of Industrial Pharmacy. Lea & Febiger, 1986
- **AMAR L. et al.** *Hypertension,* September 2010, vol. 56 (5), 831-838
- **S. R. VIPPAGUNTA.** *Adv. Drug. Deliv. Rev.,* 2001, vol. 48, 3-26
- **L. Y-A.** *Adv. Drug. Deliv. Rev.,* 2001, vol. 48, 27-42
- **S. R. BYRN.** Solid State Chemistry of Drugs. SSCI, 1999
- **S. R. CHCMBURKAR et al.** *Org. Process Res. Dev.,* 2000, vol. 4, 413-417
- Handbook of Pharmaceutical Salts: Properties, Selection and Use. Wiley, 2002
- **8. R. BYM.** Solid State Chemistry of Drugs. SSCI, 1999
- Remington 's Pharmaceutical Sciences. Mack Publishing, 1990, vol. 173
- The United States Pharmacopeia. 1995, 1843-1844
- **S. M. BERGE et al.** *J. Pharm. Set,* 1977, vol. 66, 1-19
- **WU ZHIKUI.** The Effect of Bushen Shengxue Fang on β-thalassemia at the Gene Level. *Journal of Traditional Chinese Medicine,* 1998, vol. 18 (4), 300-303
- **PENICHEI, MX. ; MORRISON, S.L.** *J. Immunol. Methods,* 2001, vol. 248, 91-101
- Remington's Pharmaceutical Sciences. Mack Publishing, 1990
- Remingto's Pharmaceutical Sciences. Mack Publishing, 1990
- **S. R. VIPPAGUNTA et al.** *Adv. Drug. Deliv. Rev.,* 2001, vol. 48, 3-26
- **L. YU.** *Adv. Drug. Deliv. Rev.,* 2001, vol. 48, 27-42
- **S. R. BYM.** Solid State Chemistry of Drugs. SSCI, 1999
- Remington: The Science and Practice of Pharmacy. Lippincott, Williams and Wilkins, 2005
- *U.S. Pharmacopeia (USP) 25-NF20,* 2002
- **JENS T. CARSTENSEN.** Drug Stability: Principles & Practice. Marcel Dekker, 1995, 379-80
- Remington 's Pharmaceutical Sciences. Mack Publishing, 1990
- Remington's Pharmaceutical Sciences. Mack Publishing, 1980
- Introduction to Pharmaceutical Dosage Forms. Lea & Febiger, 1985
- Clinical Analysis. **C.J. PERIGARD.** Remington: The Science and Practice of Pharmacy. Lippinocott Williams & Wilkins, 2000
- **AMARA, S. G. et al.** *Science,* 1982, vol. 298, 240-244
- **POYNER, D. R. et al.** *Br J Pharmacol,* 1992, vol. 105, 441-7
- **VAN VALEN, F. et al.** *Neurosci Lett,* 1990, vol. 119, 195-8
- **JUANEDA, C. et al.** *TiPS,* 2000, vol. 21, 432-438
- **BRAIN, S. D. et al.** *TiPS,* 2002, vol. 23, 51-53
- **EVANS B. N. et al.** *J Biol Chem.,* 2000, vol. 275, 31438-43
- **MCLATCHIE, L. M. et al.** *Nature,* 1998, vol. 393, 333-339
- **MALLEE et al.** *J Biol Chem,* 2002, vol. 277, 14294-8
- **EDVINSSON L.** *CNS Drugs,* 2001, vol. 15 (10), 745-53
- **WILLIAMSON, D.** *J. Microsc. Res. Tech.,* 2001, vol. 53, 167-178

- **GRANT, A. D.** *Brit. J. Pharmacol.,* 2002, vol. 135, 356-362
- **GOADSBY PJ et al.** *Ann Neurol,* 1990, vol. 28, 183-7
- **GALLAI V. et al.** *Cephalalgia,* 1995, vol. 15, 384-90
- **ASHINA M et al.** *Pain,* 2000, vol. 86 (1-2), 133-8
- **LASSEN LH et al.** *Cephalalgia,* February 2002, vol. 22 (1), 54-61
- **SHEN, Y-T. et al.** *J Pharmacol Exp Ther,* 2001, vol. 298, 551-8
- **DAVIS CD ; XU C.** *Curr Top Med Chem.,* 2008, vol. 8 (16), 1468-79
- **BENEMEI S, NICOLETTI P ; CAPONE JG ; GEPPETTI P.** *Curr Opin Pharmacol.,* 20 January 2009, vol. 9 (1), 9-14
- **HO TW ; FERRARI MD ; DODICK DW ; GALET V ; KOST J ; FAN X ; LEIBENSPERGER H ; FROMAN S ; ASSAID C ; LINES C.** *Lancet,* 25 November 2008, vol. 372, 2115
- **HO TW ; MANNIX LK ; FAN X ; ASSAID C ; FURTEK C ; JONES CJ ; LINES CR ; RAPOPORT AM.** *Neurology,* 03 October 2007, vol. 70, 1304
- **GRANT, A.** *D. Brit. J. Pharmacol.,* 2002, vol. 135, 356-362
- **GOADSBY P. J. et al.** *Ann. Neurol,* 1990, vol. 28, 183-7
- **ASHINA M. et al.** *Pain,* 2000, vol. 86 (1-2), 133-8
- **LASSEN L.H. et al.** *Cephalalgia,* 2002, vol. 22 (1), 54-61
- **SHEN, Y-T. et al.** *J. Pharmacol. Exp. Ther.,* 2001, vol. 298, 551-8
- **HAGNER, STEFANIE ; KNAUER, JENS ; HABERBERGER, RAINER ; GOEKE, BURKHARD ; VOIGT, KARLHEINZ ; MCGREGOR, GERARD PATRICK.** Calcitonin Receptor-Like Receptor Is Expressed on Gastrointestinal Immune Cells. *Digestion,* 2002, vol. 66, 197-203
- **RANG, WEI-QING ; DU, YAN-HUA ; HU, CHANG-PING ; YE, FENG ; TAN, GUI-SHAN ; DENG, HAN-WU ; LI, YUAN-JIAN.** Protective effects of calcitonin gene-related peptide-mediated evodiamine on guinea-pig cardiac anaphylaxis. *School of Pharmaceutical Sciences, Department of Pharmacology,* 2003, vol. 367, 306-311
- Department of Orthopedics, Xiehe Hospital, Tongji Medical College. **LIAN, KAI ; DU, JINGYUAN ; RAO, ZHENYU ; LUO, HUAICAN.** Journal of Tongji Medical University. Huazhong University of Science and Technology, 2001, vol. 21, 304-307
- Calcitonin gene-related Peptide regulates expression of neurokininl receptors by rat spinal neurons. **SEYBOLD VS ; MCCARSON KE ; MERMELSTEIN PG ; GROTH RD ; ABRAHAMS LG.** J. Neurosci. Department of Neuroscience, vol. 23, 1816-1824
- Attenuation of antigen-induced airway hyperresponsiveness in CGRP-deficient mice. **AOKI-NAGASE, TOMOKO ; NAGASE, TAKAHIDE ; OH-HASHI, YOSHIO ; SHINDO, TAKAYUKI ; KURIHARA, YUKIKO ; YAMAGUCHI, YASUHIRO ; YAMAMOTO, HIROSHI ; TOMITA, TETSUJI ; OHGA, EIJIRO ; NAGAI, RYOZO.** American Journal of Physiology. Department of Geriatric Medicine, 2002, vol. 283, L963-L970
- Calcitonin gene-related peptide as inflammatory mediator. **SPRINGER, JOCHEN ; GEPPETTI, PIERANGELO ; FISCHER, AXEL ; GRONEBERG, DAVID A.** Pulmonary Pharmacology & Therapeutics. Charite Campus - Virchow, 2003, vol. 16, 121-130
- Pharmacological targets for the inhibition of neurogenic inflammation. **HELYES, ZSUZSANNA ; PINTER, ERIKA ; NEMETH, JOZSEF ; SZOLCSANYI, JANOS.** Current Medicinal Chemistry: Anti-Inflammatory & Anti-Allergy Agents. Department of Pharmacology and Pharmacotherapy, 2002, vol. 2, 191-218
- **OTWINOWSKI, Z ; MINOR, W.** Macromolecular Crystallography. Academic, 1997, vol. 276, 307-326
- **R. HOOFT ; NONIUS B.V.** *Collect Data collection and processing user interface: Collect: Data collection software,* 1998
- Oxford Cryosystems Cryostream cooler. **J. COSIER ; A.M. GLAZER.** J. Appl. Cryst. 1986, vol. 19, 105
- **S. MACKAY ; C.J. GILMORE ; C. EDWARDS ; M. TREMAYNE ; N. STEWART ; K. SHANKLAND.** *maXus: a computer program for the solution and refinement of crystal structures from diffraction data*
- **SHELDRICK, GM.** SHELXTL. *Structure Determination Programs,* 1997
- **BRUKER AXS.** *SMART and SATNTPLUS. Area Detector Control and Integration Software,* 1998
- **SHELDRICK, GM.** SHELXTL. *Structure Determination Programs,* 1997
- **A.E. BENNETT et al.** *J. Chem. Phys,* 1995, vol. 103, 6951
- **G. METZ ; X. WU ; S.O. SMITH.** *J. Magn. Reson. A,* 1994, vol. 110, 219-227
- **W.L. EARL ; D.L. VANDERHART.** *J. Magn. Reson.,* 1982, vol. 48, 35-54
- **GREGORY, R. J. et al.** *Nature,* 1990, vol. 347, 382-386
- **RICH, D. P. et al.** *Nature,* 1990, vol. 347, 358-362
- **RIORDAN, J. R. et al.** *Science,* 1989, vol. 245, 1066-1073
- **CUTTING, G. R. et al.** *Nature,* 1990, vol. 346, 366-369
- **DEAN, M. et al.** *Cell,* 1990, vol. 61

- **EREM, B-S. et al.** *Science,* 1989, vol. 245, 1073-1080
- **KEREM, B-S et al.** *Proc. Natl. Acad. Sci. USA,* 1990, vol. 87, 8447-8451
- **QUINTON, P. M.** *FASEB J.,* 1990, vol. 4, 2709-2727
- **DALEMANS et al.** *Nature Lond,* 1991, vol. 354, 526-528
- **PASYK ; FOSKETT.** *J. Cell. Biochem.,* 1995, vol. 270, 12347-50
- **HWANG, T. C.** *J. Gen. Physiol.,* 1998, vol. 777 (3), 477-90
- **E. W. MARTIN.** Remington's Pharmaceutical Sciences. Mack Publishing Co, 1980
- **SE^ GONZALEZ, J. E. ; R. Y. TSIEN.** Voltage sensing by fluorescence resonance energy transfer in single cells. *Biophys J,* 1995, vol. 69 (4), 1272-80
- **GONZALEZ, J. E. ; R. Y. TSIEN.** Improved indicators of cell membrane potential that use fluorescence resonance energy transfer. *Chem Biol,* 1997, vol. 4 (4), 269-77
- **SEEI GONZALEZ, J. E. ; K. OADES et al.** Cell-based assays and instrumentation for screening ion-channel targets. *Drug Discov Today,* 1999, vol. 4 (9), 431-439
- **GALIETTA, L.J.V. ; LANTERO, S. ; GAZZOLO, A. ; SACCO, O. ; ROMANO, L. ; ROSSI, G.A. ; ZEGARRA-MORAN, O.** *In Vitro Cell. Dev. Biol.,* 1998, vol. 34, 478-481
- **RAE, J. ; COOPER, K. ; GATES, P. ; WATSKY, M.** *J. Neurosci. Methods,* 1991, vol. 37, 15-26
- **DALEMANS, W. ; BARBRY, P. ; CHAMPIGNY, G. ; JALLAT, S. ; DOTT, K. ; DREYER, D. ; CRYSTAL, R.G. ; PAVIRANI, A. ; LECOCQ, J-P. ; LAZDUNSKI, M.** *Nature,* 1991, vol. 354, 526-528
- **RAE, J. ; COOPER, K. ; GATES, P. ; WATSKY, M.** *J. Neurosci. Methods,* 1991, vol. 37, 15-26
- **JENKINS, R ; SNYDER, R.L.** Introduction to X-Ray Powder Diffractometry. John Wiley & Sons, 1996
- **BUNN, C.W.** Chemical Crystallography. Clarendon Press, 1948
- **KLUG, H. P. ; ALEXANDER, L. E.** *X-Ray Diffraction Procedures,* 1974
- **K.D. HARRIS.** Powder diffraction crystallography of molecular solids. *Top Curr Chem.,* 2012, vol. 315, 133-77
- **N. CHIENG ; T. RADES ; J.AALTONEN.** An overview of recent studies on the analysis of pharmaceutical polymorphs. *J Pharm Biomed Anal.,* 2011, vol. 55 (4), 618-44
- **R. HILFIKER.** Polymorphism. Wiley-VCH, 2006
- **H.G. BRITTAIN.** Polymorphism in Pharmaceutical Solids. Informa Healthcare, 2009
- **H.G. BRITTAIN ; S.J. BODANOWICH ; D.E. BUGAY ; J. DEVONCENTIS ; G. LEWEN ; A.W. NEWMAN.** Physical characterization of pharmaceutical solids. *Pharm Res.,* 1991, vol. 8 (8), 963-73
- **D.E. BUGAY.** Characterization of the solid-state: spoectroscopic techniques. *Adv Drug Deliv Rev.,* 2001, vol. 48 (1), 43-65
- **P.A. TISHMACK ; D.E. BUGAY ; S.R. BYRN.** Solid-state nuclear magnetic resonance spectroscopy-pharmaceutical application. *J Pharm Sci,* 2003, vol. 92 (3), 441-74
- **C.J. LEE ; C.J. STRACHAN ; P.J. MANSON ; T. RADES.** Characterization of the bulk properties of pharmaceutical solids using nonlinear optics-a review. *J Pharm Pharmacol.,* 2007, vol. 59 (2), 241-50
- **R.A. STOREY.** Solid State Characterization of Pharmaceuticals. Wiley-Blackwell, 2011
- **FELL, J.T. ; NEWTON, J.M.** Determination of tablet strength by diametral compression test. *J. Pharm. Sci,* 1970, vol. 59, 688-691
- **NYSTRÖM, C. ; MALMQVIST, K. ; MAZUR, J. ; ALEX, W. ; HÖLZER, A.W.** Measurement of axial and radial tensile strength of tablets and their relation to capping. *Acta Pharm. Suec,* 1978, vol. 15, 226-232
- **STANLEY-WOOD, N.G. ; JOHANSSON, M.E.** Variation of intra-and inter-particle porosity with degree of compaction. *Analyst,* 1980, vol. 105, 1104-1112
- **WESTERMARCK, S. ; JUPPO, A.M. ; KERVINEN, L. ; YLIRUUSI, J.** Pore structure and surface area of mannitol powder, granules and tablets determined with mercury porosimetry and nitrogen adsorption. *Eur. J. Pharm. Biopharm,* 1998, vol. 46, 61-86
- **JUPPO, A.M.** Relationship between breaking force and pore structure of lactose, glucose and mannitol tablets. *Int. J. Pharm.,* 1996, vol. 127, 95-102
- **S. LOWELL et al.** Characterization of Porous Solids and Powders: Surface Area, Pore Size and Density. Springer, 2010
- **N. RASENACK ; B.W. MÜLLER.** Micron-size drug particles: common and novel micronization techniques. *Pharm Dev Technol.,* 2004, vol. 9 (1), 1-13
- **A. MARTIN ; M.J. COCERO.** Micronization processes with supercritical fluids: fundamentals and mechanisms. *Adv Drug Deliv Rev.,* 2008, vol. 60 (3), 339-50
- **S.C. GAD.** Pharmaceutical Manufacturing Handbook: Production and Processes (Pharmaceutical Development Series). Wiley Interscience, 2008
- **A.J. HICKEY.** Pharmaceutical Process Engineering (Drugs and the Pharmaceutical Sciences). Informa Healthcare, 2009

- **B. NICKERSON.** Sample Preparation of Pharmaceutical Dosage Forms: Challenges and Strategies for Sample Preparation and Extraction. Springer, 2011
- **D. DRAGOMAN.** Optical Characteriaztion of Solids. Springer, 2010
- **D. SCHULZE.** Powders and Bulk Solids: Behavior, Characterization, Storage and Flow. Springer, 2008